# EUROPEAN PATENT SPECIFICATION

(11) **EP 2 906 237 B1**
(45) Date of publication and mention of the grant of the patent: **15.04.2020**
(21) Application number: 13816831.5
(22) Date of filing: 12.07.2013
(51) Int. Cl.: C07K 16/46, C07K 16/28, A61K 39/395, C07K 14/76

(54) **MULTIVALENT HETEROMULTIMER SCAFFOLD DESIGN AND CONSTRUCTS**
ENTWURF UND KONSTRUKTE FÜR EIN MULTIVALENTES HETEROMULTIMERES GERÜST
CONCEPTION D'ÉCHAFAUDAGES HÉTÉROMULTIMÈRES ET PRODUITS DE RECOMBINAISON

(30) Priority: 13.07.2012 US 201261671640 P; 05.09.2012 US 201261697245 P; 30.01.2013 US 201361758701 P
(43) Date of publication of application: 19.08.2015
(73) Proprietor: Zymeworks, Inc., Vancouver, British Columbia V6H 3V9 (CA)
(72) Inventor: DIXIT, Surjit, Bhimarao, Vancouver, Bristish Columbia V6H 3V9 (CA); D'ANGELO, Igor Edmondo, Paolo, Vancouver, Bristish Columbia V6H 3V9 (CA); SANCHES, Mario, Vancouver Bristish Columbia V6H 3V9 (CA); NG, Gordon Yiu Kon, Vancouver Bristish Columbia V6H 3V9 (CA)
(74) Representative: Elkington and Fife LLP
(86) International application number: PCT/US2013/050408
(87) International publication number: WO 2014/012082

(56) References cited:
- WO-A1-2012/116453
- WO-A2-03/060071
- WO-A2-2010/092135
- WO-A2-2010/118169
- US-A1- 2005 186 664
- US-A1- 2011 287 009
- US-A1- 2012 244 577
- TAFELMEYER P ET AL: "Transforming a (beta/alpha)8-Barrel Enzyme into a Split-Protein Sensor through Directed Evolution", CHEMISTRY AND BIOLOGY, CURRENT BIOLOGY, LONDON, GB, vol. 11, no. 5, 1 May 2004 (2004-05-01), pages 681-689, XP025916620, ISSN: 1074-5521 [retrieved on 2004-05-25]
- DOCKAL ET AL.: 'Five recombinant fragments of human serum albumin-Tools for the characterization of the warfarin binding site' PROTEIN SCIENCE vol. 9, no. ISS. 8, 2000, pages 1455 - 1456, XP055186742
- F F An ET AL: "Fragments of Bovine Serum Albumin Produced by Limited Proteolysis: Complementary Behavior of Two Large Fragments1", , 1 January 1976 (1976-01-01), XP55385286, Retrieved from the Internet: URL:http://pubs.acs.org/doi/pdf/10.1021/bi 00669a028 [retrieved on 2017-06-26]
- KRATZ ET AL: "Albumin as a drug carrier: Design of prodrugs, drug conjugates and nanoparticles", JOURNAL OF CONTROLLED RELEASE, ELSEVIER, AMSTERDAM, NL, vol. 132, no. 3, 18 December 2008 (2008-12-18), pages 171-183, XP025714816, ISSN: 0168-3659, DOI: 10.1016/J.JCONREL.2008.05.010 [retrieved on 2008-11-29]

## Description

### Field of Invention

The field of the invention is the rational design of a scaffold for custom development of biotherapeutics.

### Description of Related Art

In the realm of therapeutic proteins, antibodies with their multivalent target binding features are excellent scaffolds for the design of drug candidates. Advancing these features further, designed bispecific antibodies and other fused multispecific therapeutics exhibit dual or multiple target specificities and an opportunity to create drugs with novel modes of action. The development of such multivalent and multispecific therapeutic proteins with favorable pharmacokinetics and functional activity has been a challenge.

Human serum albumin (HSA, or HA), a protein of 585 amino acids in its mature form is responsible for a significant proportion of the osmotic pressure of serum and also functions as a carrier of endogenous and exogenous ligands. The role of albumin as a carrier molecule and its stable nature are desirable properties for use as a carrier and transporter of polypeptides in vivo.

Human serum albumin possesses many desirable characteristics. HSA is found throughout the body, but more specifically in the interstitial space and in blood at serum concentrations of 40 g/L which is equivalent to 0.7 mM (Yeh et al., Proc. Natl. Acad. Sci. USA, 89:1904-1908 (1992)). HSA is considered to be the most abundant protein of the serum and is responsible for maintaining osmolarity. HSA has favorable pharmacokinetic properties and is cleared very slowly by the liver and kidney displaying in vivo half-lives up to several weeks (Yeh et al., Proc. Natl. Acad. Sci. USA, 89:1904-1908 (1992); Waldmann, T. A., Albumin Structure, Function and Uses, pp. 255-273 (1977); Sarav et al., J Am Soc Nephrol 20:1941-1952(2009)). HSA lacks enzymatic activity and antigenicity thereby eliminating potentially undesirable side effects. HSA acts as a carrier for endogenous as well as exogenous ligands. HSA is also known to penetrate and be retained in the interstitium of tumors (see Elsadek and Kratz, J. Control. Release (2012) 157:4-28). Albumin has been proposed as a drug carrier for therapeutic proteins (see Kratz, J. Control. Release (2008) 132:171-183) Combined, these features can be extended, at least partially, onto albumin based fusion protein. The poor pharmacokinetic properties displayed by therapeutic proteins can then be circumvented.

### SUMMARY OF THE INVENTION

An object of the present invention is to provide multivalent heteromultimer scaffolds and methods of designing same.

In one aspect of the invention there is provided heteromultimer comprising: a first polypeptide construct that comprises a first transporter polypeptide and at least one first cargo molecule, optionally wherein the first cargo molecule is a cargo polypeptide, optionally wherein the first polypeptide construct comprises two first cargo polypeptides; and a second polypeptide construct that comprises a second transporter polypeptide; and wherein said first and second transporter polypeptides are obtained by segmentation of an albumin polypeptide having the sequence of SEQ ID NO:1 at a segmentation site, and wherein the location of the segmentation site is:
(a) between residues 339 and 340 of SEQ ID NO: 1; or
(b) between residues 300 and 301 of SEQ ID NO:1; or
(c) between residues 364 and 365 of SEQ ID NO: 1; or
(d) between residues 441 and 442 of SEQ ID NO: 1; or
(e) between residues 171 and 172 of SEQ ID NO:1; or
(f) between residues 281 and 282 of SEQ ID NO: 1; or
(g) after residue 83 and before residue 85 of SEQ ID NO:1 and residue 84 is deleted;
wherein the numbering of residues begins with the first residue after the signal sequence, and wherein said first transporter polypeptide is different from said second transporter polypeptide, and said transporter polypeptides self-assemble to form a quasi-native structure of the monomeric form of said albumin polypeptide.

Another aspect described herein is a heteromultimer comprising: a first polypeptide construct that comprises (i) a first transporter polypeptide; and (ii) at least one first cargo polypeptide that is an antigen-binding polypeptide construct that binds to CD3, CD19, CD20, HER2 or HER3, and a second polypeptide construct that comprises (iii) a second transporter polypeptide; and (iv) at least one second cargo polypeptide wherein each of said first and second transporter polypeptide comprises an amino acid sequence with at least 90% identity to a segment of an albumin polypeptide; and wherein said first and second transporter polypeptides are obtained by segmentation of said albumin polypeptide at a segmentation site, wherein said transporter polypeptides self-assemble to form a quasi-native structure of the monomeric form of said albumin polypeptide.

In another aspect of the invention there is provided a host cell comprising nucleic acid encoding a heteromultimer of the invention.

In another aspect of the invention there is provided a pharmaceutical composition comprising a heteromultimer of the invention and an adjuvant.

In another aspect of the invention there is provided a method of treating cancer comprising providing to a patient in need thereof an effective amount of the pharmaceutical composition of the invention.

In another aspect of the invention there is provided a method of inhibiting growth of a tumor, comprising contacting the tumor with an effective amount of a heteromultimer the invention.

In another aspect of the invention there is provided a method of shrinking a tumor, comprising contacting the tumor with an effective amount of a heteromultimer of the invention.

Another aspect described herein is a method of designing self-associating polypeptides from a protein of interest comprising: segmenting said protein at at-least one segmentation site to obtain at least two polypeptide segments such that said polypeptide segments self-assemble to form a heteromultimer, wherein said heteromultimer forms a quasi-native monomeric structure of said protein, the method comprising the steps of selecting at least one loop of the protein of interest that has a high solvent accessible surface area and limited contact with the rest of the structure of said protein, and introducing one segmentation site per selected loop, resulting in a complementary interface between the at least two polypeptide segments, wherein the interface is apolar, extensive and interdigitate.

In another aspect there is provided a heteromultimer designed by a method of the invention.

In another aspect there is provided a therapeutic scaffold comprising a heteromultimer designed by a method of the invention.

Provided herein are multifunctional heteromultimers of the invention and methods to design them. In certain embodiments are heteromultimers, each heteromultimer comprising: at least a first polypeptide construct that comprises at least one cargo molecule, and a first transporter polypeptide; and at least a second polypeptide construct that comprises at least one cargo molecule and a second transporter polypeptide; wherein the transporter polypeptides are derived by segmentation of a protein such that said transporter polypeptides self-assemble to form a quasi-native structure of said protein or analog thereof. In certain embodiments, at least one cargo molecule is a drug, or a therapeutic agent. In certain embodiment more the one cargo molecule of the same nature is present on the transporter polypeptide. In certain embodiments, at least one cargo molecule is a biomolecule. In an embodiment, the at least one biomolecule is a DNA, RNA, PNA or polypeptide. In an embodiment, at least one cargo molecule is a polypeptide. In certain embodiments, each transporter polypeptide is unstable and preferentially forms a heteromultimer with at least one other transporter polypeptide. In certain embodiments, each transporter polypeptide is stable and preferentially forms a heteromultimer with at least one other transporter polypeptide. In certain embodiments, the heteromultimerization interface comprises at least one disulfide bond. In certain embodiments, the heteromultimerization interface does not comprise a disulfide bond.

In certain embodiments is a heteromultimer that comprises: at least two polypeptide constructs, each polypeptide construct comprising at least one cargo polypeptide attached to a transporter polypeptide, wherein said first and second transporter polypeptides are obtained by segmentation of an albumin polypeptide having the sequence of SEQ ID NO: 1 at a segmentation site, and wherein the location of the segmentation site is:
(a) between residues 339 and 340 of SEQ ID NO: 1; or
(b) between residues 300 and 301 of SEQ ID NO:1; or
(c) between residues 364 and 365 of SEQ ID NO: 1; or
(d) between residues 441 and 442 of SEQ ID NO: 1; or
(e) between residues 171 and 172 of SEQ ID NO:1; or
(f) between residues 281 and 282 of SEQ ID NO: 1; or
(g) after residue 83 and before residue 85 of SEQ ID NO:1 and residue 84 is deleted;
wherein the numbering of residues begins with the first residue after the signal sequence,
and wherein said first transporter polypeptide is different from said second transporter polypeptide, and said transporter polypeptides self-assemble to form a quasi-native structure of the monomeric form of said albumin polypeptide. In certain embodiments, the heteromultimer is a heterodimer. In an embodiment, the heteromultimer is bispecific. In an embodiment, the heteromultimer is multispecific. In certain embodiments, the heteromultimer is bivalent. In an embodiment the heteromultimer is multivalent. In an embodiment, the heteromultimer is multifunctional.

In some aspects described herein, the transporter polypeptides are derivatives of an annexin protein. In an aspect, the transporter polypeptides are derived from different annexin proteins. In certain aspects, the transporter polypeptides are derived from the same annexin protein. In an aspect, at least one transporter polypeptide is derived from Annexin A1 or lipocortin I. In certain aspects of the heteromultimer, all transporter polypeptides are derived from Annexin A1 of SEQ ID NO: 14. In certain aspects of the heteromultimer, at least one transporter polypeptides is derived from a sequence homologous to SEQ ID NO: 14. In an aspect, at least one transporter polypeptide is derived from Annexin A2 or annexin II. In certain aspects of the heteromultimer, all transporter polypeptides are derived from Annexin A2 or lipocortin II. In an aspect, at least one transporter polypeptide is derived from Annexin like protein. In certain embodiments of the heteromultimer, all transporter polypeptides are derived from Annexin like protein. In an embodiment, at least one transporter polypeptide is derived from the group comprising Annexin A1-Annexin A7. In an embodiment of the heteromultimer described herein, all transporter polypeptides are derived from the group comprising Annexin A1-Annexin A7. SEQ ID No.-14. In certain aspects, the first annexin based transporter polypeptide has a sequence comprising SEQ ID NO: 15, and the second annexin based transporter polypeptide has a sequence comprising SEQ ID NO: 16.

In some aspects described herein, the transporter polypeptides are derivatives of transferrin. In an aspect, at least one transporter polypeptide is derived from transferrin. In certain aspects of the heteromultimer, at least one transporter polypeptides are derived from transferrin of SEQ ID NO: 19 or analog thereof. In certain aspects of the heteromultimer, at least one transporter polypeptide is derived from a polypeptide seuquence homologous to the transferrin. In certain aspects of the heteromultimer described herein, at least one transporter polypeptide is derived from apo-transferrin. In certain aspects, the first transferrin based transporter polypeptide has a sequence comprising SEQ ID NO: 15 and the second transferrin based transporter polypeptide has a sequence comprising SEQ ID NO: 16.

In certain embodiments of the heteromultimer, at least one cargo molecule is a cargo polypeptide. In an embodiment of the heteromultimer described herein, all cargo molecules are cargo polypeptides. In certain embodiments, the cargo polypeptides are therapeutic proteins or fragments or variants thereof. In certain embodiments, the cargo polypeptides are antigens or fragments or variants thereof. In certain embodiments, the cargo polypeptides are antigen receptors or fragments or variants thereof. In some embodiments, the cargo polypeptide is an antibody, an antibody domain, a ligand or a receptor that binds a target polypeptide. In some embodiments, at least one cargo polypeptide is fused to the transporter polypeptide. In certain embodiments, at least one cargo polypeptide is attached to the N-terminus of the transporter polypeptide. In some embodiments, at least one cargo polypeptide is attached to the C-terminus of the transporter polypeptide. In some embodiments, at least one cargo polypeptide is chemically linked to the transporter polypeptide. In some embodiments of the heteromultimers described herein, at least one cargo polypeptide comprises GLP-1 or fragment or variant thereof. In some embodiments, at least one cargo polypeptide comprises glucagon or fragment or variant thereof. In an embodiment, at least one cargo polypeptide comprises an EGF-A like domain.

Described herein are heteromultimers, each heteromultimer comprising: at least a first polypeptide construct that comprises at least one cargo polypeptide and a first transporter polypeptide; and at least a second polypeptide construct that comprises at least one cargo polypeptide and a second transporter polypeptide. In certain embodiments, the heteromultimer is a heterodimer. In an embodiment, the heteromultimer is multispecific. In an embodiment, the heteromultimer is bispecific. In certain embodiments of the heteromultimer, the transporter polypeptides are derivatives of the same protein. In certain embodiments, the transporter polypeptides are derivatives of albumin. In certain embodiments of the hetermultimer described herein, the transporter polypeptides are derived from human serum albumin of SEQ ID No. 1. In certain aspects, the transporter polypeptides are derivatives of an annexin. In an aspect, the transporter polypeptides are derivatives of Annexin A2. In some aspects, the transporter polypeptides are derivatives of transferrin.

In certain embodiments, are heteromultimers, each heteromultimer comprising: at least a first polypeptide construct that comprises at least one cargo polypeptide and a first transporter polypeptide comprising a first segment of human serum albumin; and at least a second polypeptide construct that comprises at least one cargo polypeptide and a second transporter polypeptide comprising a second segment of human serum albumin; wherein said transporter polypeptides self-assemble to form a quasi-native structure of albumin or analog thereof. In certain embodiments, the first and second segments of human serum albumin are from non-overlapping regions of the protein.

In certain aspects described herein are heteromultimers, each heteromultimer comprising: at least a first polypeptide construct that comprises at least one cargo polypeptide and a first transporter polypeptide comprising a sequence of SEQ ID NO:2; and at least a second polypeptide construct that comprises at least one cargo polypeptide, and a second transporter polypeptide comprising a sequence of SEQ ID NO: 3. In certain aspects, are heteromultimers, each heteromultimer comprising: at least a first polypeptide construct that comprises at least one cargo polypeptide and a first transporter polypeptide comprising a sequence of SEQ ID NO:8; and at least a second polypeptide construct that comprises at least one cargo polypeptide and a second transporter polypeptide comprising a sequence of SEQ ID NO: 10. In certain aspects of the hetermultimer described herein, at least one transporter polypeptide is derived from alloalbumins. In certain aspects, both transporter polypeptides are derived from alloalbumins. In certain aspects, all transporter polypeptides are derivatives of the same alloalbumin. In some other aspects, the transporter polypeptides are derivatives of different alloalbumins. In some aspects, each transporter polypeptide is an alloalbumin derivative based on an alloalbumin selected from Table 2. In certain aspects, the first polypeptide construct comprises two cargo polypeptides. In some aspects, the second polypeptide construct comprises two cargo polypeptides. In some aspect, at least one of the polypeptide constructs is engineered by introducing mutations. In certain aspects, the introduced mutations improve the functionality of the polypeptide construct as compared to the non-mutated form of the construct. In certain aspects the introduced mutations improve one or more of the stability, half-life and heteromultimer formation of the transporter polypeptide.

Described herein is a heteromultimer comprising: at least a first polypeptide construct that comprises (i) a first transporter polypeptide; and (ii) at least one cargo polypeptide and at least a second polypeptide construct that comprises (iii) a second transporter polypeptide and (iv) at least one cargo polypeptide; wherein said transporter polypeptides are derived from a protein by segmentation of said protein, each transporter polypeptide comprising an amino acid sequence with at least 90% identity to a segment of said protein, and wherein said transporter polypeptides self-assemble to form a quasi-native structure of said protein.

In certain aspects is a heteromultimer described herein, wherein the transporter polypeptides are derived from a protein by segmentation of said protein, each transporter polypeptide comprising an amino acid sequence with at least 95% identity to a segment of said protein.

In certain aspects is a heteromultimer described herein, wherein the transporter polypeptides are derived from a protein by segmentation of said protein, each transporter polypeptide comprising an amino acid sequence with at least 99% identity to a segment of said protein.

In certain aspetcs described herein, the heteromultimer is a heterodimer. In some aspects, at least one transporter polypeptide is not derived from an antibody. In exemplary aspects, each transporter polypeptide is an albumin derivative. In some aspects, at least one of said first and second transporter polypeptides comprise at least one mutation of an amino acid residue to cystine such that said cysteine forms a disulfide bond with a cysteine residue on another transporter polypeptide. In certain aspects, said first and second transporter polypeptide comprise at least one mutation of an amino acid residue to cystine such that said cysteine forms a disulfide bond with a cysteine residue on another transporter polypeptide. In some aspects are provided heteromutlimers wherein each transporter polypeptide is an albumin derivative, the first transporter polypeptide comprising at least one mutation selected from A194C, L198C, W214C, A217C, L331C and A335C. In some aspects, the second transporter polypeptide is an elbumin derivative comprising at least one mutation selected from L331C, A335C, V343C, L346C, A350C, V455C, and N458C.

In some aspects provided are heteromultimers described herein, wherein said first transporter polypeptide has a sequence comprising SEQ ID NO:35 or analog or variant thereof, and wherein said second transporter polypeptide has a sequence comprising SEQ ID NO:36 or analog or variant thereof. In some aspects, said first transporter polypeptide has a sequence comprising SEQ ID NO:37 or analog or variant thereof, and wherein said second transporter polypeptide has a sequence comprising SEQ ID NO:38 or analog or variant thereof. In certain embodiments, said first transporter polypeptide has a sequence comprising SEQ ID NO:39 or analog or variant thereof, and wherein said second transporter polypeptide has a sequence comprising SEQ ID NO:40 or analog or variant thereof. In exemplary embodiments, said first transporter polypeptide has a sequence comprising SEQ ID NO:41 or analog or variant thereof, and wherein said second transporter polypeptide has a sequence comprising SEQ ID NO:42 or analog or variant thereof. In certain embodiments, said first transporter polypeptide has a sequence comprising SEQ ID NO:43 or analog or variant thereof, and wherein said second transporter polypeptide has a sequence comprising SEQ ID NO:44 or analog or variant thereof. In an embodiment, said first transporter polypeptide has a sequence comprising SEQ ID NO:45 or analog or variant thereof, and wherein said second transporter polypeptide has a sequence comprising SEQ ID NO:46 or analog or variant thereof. In one embodiment, said first transporter polypeptide has a sequence comprising SEQ ID NO:47 or analog or variant thereof, and wherein said second transporter polypeptide has a sequence comprising SEQ ID NO:48 or analog or variant thereof. In certain embodiments, said first transporter polypeptide has a sequence comprising SEQ ID NO:49 or analog or variant thereof, and wherein said second transporter polypeptide has a sequence comprising SEQ ID NO:50 or analog or variant thereof. In certain embodiments, said first transporter polypeptide has a sequence comprising SEQ ID NO:51, and wherein said second transporter polypeptide has a sequence comprising SEQ ID NO:52

In certain embodiments of the heteromultimer described herein, at least one cargo polypeptide binds a target antigen, and wherein said target antigen is at least one of a-chain (CD25) of IL-2R, Amyloid beta, anti-EpCAM, anti-CD3, CD16, CD20, CD22, CD23, CD3, CD4, CD52, CD80, CTLA-4, EGFR, EpCAM, F protein of RSV, G250, glycoprotein IIB/IIIa R, HER2, HER2/neu R, HSP90, IgE antibody, IL-12, IL-23, IL-1b, IL-5, IL-6, RANKL, TNF alpha, TNFR, VEGF-A, glucagon receptor, GLP receptor, and LDL receptor.

Provided herein are heteromultimers, each heteromultimer comprising: at least a first polypeptide construct that comprises at least one cargo polypeptide and a first transporter polypeptide; and at least a second polypeptide construct that comprises at least one cargo polypeptide and a second transporter polypeptide. In certain embodiments, at least one cargo polypeptide is selected from the proteins listed in Table 2 or fragments, variants or derivatives thereof. In certain embodiments, at least one cargo polypeptide is selected from ligand, receptor, or antibody to one or more proteins listed in Table 2, or fragment, variant or derivative of said ligand, receptor or antibody. In certain embodiments, at least one cargo polypeptide targets a cell surface antigen from the group consisting of CD19, CD20, CD22, CD25, CD30, CD33, CD40, CD56, CD64, CD70, CD74, CD79, CD105, Cd138, CD174, CD205, CD227, CD326, CD340, MUC16, GPNMB, PSMA, Cripto, ED-B, TMEFF2, EphB2, EphA2, FAP, integrin, Mesothelin, EGFR, TAG-72, GD2, CAIX, 5T4. In certain embodiments, are heteromultimers, each heteromultimer comprising: at least a first polypeptide construct that comprises at least one cargo polypeptide and a first transporter polypeptide; and at least a second polypeptide construct that comprises at least one cargo polypeptide and a second transporter polypeptide, wherein at least one at least one cargo polypeptide is an antibody, or fragment or variant thereof. In certain embodiments, all cargo polypeptides are antibodies or fragments or variants thereof. In some embodiments, the cargo polypeptide is an antibody that binds to a protein listed in Table 2. In some embodiments, the antibody fragment comprises antibody Fc or Fab or Fv region. In some embodiment the cargo polypeptide is a non-antibody protein like nanobodies, affibody, maxibody, adnectins, domain antibody, evibody, ankyrin repeat proteins, anticalins, camlids or ligand protein or polypeptide binding to a therapeutically relavant target. In some embodiments, the antibody or its fragment is derived from an immunoglobulin selected from the group consisting of IgG, IgA, IgD, IgE, and IgM. In certain embodiments, the IgG is of subtype selected from IgG1, IgG2a, IgG2b, IgG3 and IgG4. In certain embodiments, the antibody is multispecific.

Provided herein are heteromultimers, each heteromultimer comprising: at least a first polypeptide construct that comprises at least one cargo polypeptide and a first transporter polypeptide of the invention; and at least a second polypeptide construct that comprises at least one cargo polypeptide and a second transporter polypeptide, wherein at least one cargo polypeptide is a therapeutic antibody. In some embodiments of the heteromultimers described herein, at least one cargo polypeptide is a therapeutic antibody or fragment or variant thereof, wherein the antibody is selected from antibody is selected from abagovomab, adalimumab, alemtuzumab, aurograb, bapineuzumab, basiliximab, belimumab, bevacizumab, briakinumab, canakinumab, catumaxomab, certolizumab pegol, certuximab, daclizumab, denosumab, efalizumab, galiximab, gemtuzumab ozagamicin, golimumab, ibritumomab tiuxetan, infliximab, ipilimumab, lumiliximab, mepolizumab, motavizumab, muromonab, mycograb, natalizumab, nimotuzumab, ocrelizumab, ofatumumab, omalizumab, palivizumab, panitumumab, pertuzumab, ranizumab, reslizumab, rituximab, teplizumab, toclizumab, tositumomab, trastuzumab, Proxinium, Rencarex, ustekinumab, and zalutumumab. In certain embodiments, the therapeutic antibody binds a disease related target antigen such as cancer antigen, inflammatory disease antigen or a metabolic disease antigen. In certain embodiments, the target antigen could be a protein on a cell surface and the cell could belong to the group of B-cell, T-cell, stromal cell, endothelial cell, vascular cell, myeloid cell, hematopoietic cell or carcinoma cell.

Described herein are heteromultimers, each heteromultimer comprising: at least a first polypeptide construct that comprises at least one cargo molecule, fragment; and at least a second polypeptide construct that comprises at least one cargo molecule and a second transporter polypeptide, wherein at least one cargo polypeptide is an enzyme, enzyme inhibitor, hormone, therapeutic polypeptide, antigen, radiotoxin and chemotoxin inclusive of but not restricted to neurotoxins, interferons, cytokine fusion toxins and chemokine fusion toxins, cytokine, antibody fusion protein or variant or fragment thereof. In some embodiments of the heteromultimers described herein, at least one cargo polypeptide comprises GLP-1 or fragment or variant thereof. In some embodiments, at least one cargo polypeptide comprises glucagon or fragment or variant thereof. In an embodiment, at least one cargo polypeptide comprises an EGF-A like domain. In certain embodiments, the toxin is an immunotoxin such as Denileukin diftitox and Anti-CD22 immunotoxin such as CAT-3888 and CAT-8015. In certain embodiments, the toxin is saporin. In some embodiments, the toxin is a mitotoxin. In some embodiments, the toxin is a diphtheria toxin. In some embodiments, the toxin is botulinux toxin type A. In some embodiments, the toxin is ricin or a fragment there of. In some embodiments, the toxin is a toxin from RTX family of toxins.

Described herein are heteromultimers, each heteromultimer comprising: at least a first polypeptide construct that comprises at least one cargo polypeptide and a first transporter polypeptide; and at least a second polypeptide construct that comprises at least one cargo polypeptide and a second transporter polypeptide, wherein the cargo polypeptide is attached to the transporter polypeptide by chemical conjugation, native ligation, chemical ligation, a disulfide bond or direct fusion or fusion via a linker. In certain embodiments, linkers for attaching cargo molecules such as cargo polypeptides to transporter polypeptides are selected from the linkers described in US5482858, US5258498 and US5856456, US2009060721, US6492123, US4946778, US5869620, US7385032, US5073627, US5108910, US7977457, US5856456, US7138497,US5837846, US5990275, EP1088888.

Provided herein are host cells comprising nucleic acid encoding a heteromultimer described herein. In certain embodiments, the nucleic acid encoding the first polypeptide construct and the nucleic acid encoding the second polypeptide construct are present in a single vector. In certain embodiments, the nucleic acid encoding the first polypeptide construct and the nucleic acid encoding the second polypeptide construct are present in separate vectors.

Provided herein is a method of making a heteromultimer, wherein said method comprises: culturing a host cell described herein such that the nucleic acid encoding a heteromultimer described herein is expressed; and recovering the heteromultimer from the cell culture. In some embodiments, the host cell is a prokaryotic cell or a eukaryotic cell. In some embodiments, the host cell is E. coli. In certain embodiments, the host cell is yeast cell. In some embodiments, the yeast is S. cerevisiae. In some embodiments, the yeast is Pichia. In a certain embodiment, the yeast is Pichia pastoris. In some embodiments, the yeast is glycosylation deficient, and/or protease deficient. In some embodiments, the host cell is a bacterial cell. In some embodiments, the host cell expressing a heteromultimer descried herein is a mammalian cell. In certain embodiments, the mammalian cell is a CHO cell, a BHK cell, NSO cell, COS cell or a human cell.

Provided is a pharmaceutical composition that comprises a heteromultimer described herein and a pharmaceutically acceptable adjuvant. Also provided are methods of treating an individual suffering from a disease or disorder, said method comprising administering to the individual an effective amount of a formulation or pharmaceutical composition described herein. In certain embodiments is a method of treating cancer in a patient, said method comprising administering to the patient a therapeutically effective amount of a heteromultimer described herein. In some embodiments is a method of treating an immune disorder in a patient, said method comprising administering to the patient a therapeutically effective amount of a heteromultimer described herein. Also provided is a method of treating an infectious disease in a patient, said method comprising administering to the patient a therapeutically effective amount of a heteromultimer described herein. In certain embodiments is a method of treating a cardiovascular disorder in a patient, said method comprising administering to the patient a therapeutically effective amount of a heteromultimer described herein. In certain embodiments is a method of treating a respiratory disorder in a patient, said method comprising administering to the patient a therapeutically effective amount of a heteromultimer described herein. In certain embodiments is a method of treating a metabolic disorder in a patient, said method comprising administering to the patient a therapeutically effective amount of a heteromultimer described herein. In certain embodiments is a method of treating one or more of Congenital adrenal hyperplasia, Gaucher's disease, Hunter syndrome, Krabbe disease, Metachromatic leukodystrophy, Niemann-Pick disease, Phenylketonuria (PKU), Porphyria, Tay-Sachs disease, and Wilson's disease in a patient, said method comprising administering to the patient a therapeutically effective amount of a heteromultimer described herein. Provided are methods of treating cancer comprising providing to a patient in need thereof an effective amount of the pharmaceutical composition odescribed herein. In certain embodiments is a method of inhibiting growth of a tumor, comprising contacting the tumor with an effective amount of the heteromultimer described herein. In some embodiments is a method of shirnking a tumor, comprising contacting the tumor with an effective amount of the heteromultimer provided herein.

Provided is a kit for detecting the presence of a biomarker of interest in an individual, said kit comprising (a) an amount of a heteromultimer described herein, wherein said heteromultimer comprises at least one cargo polypeptide such that said cargo polypeptide is capable of binding to the biomarker of interest; and (b) instructions for use.

Described herein are heteromultimer proteins that comprise at least two polypeptide constructs, wherein each polypeptide construct comprises at least one cargo polypeptide, and an albumin based polypeptide, such that said polypeptide constructs self-assemble to form the heteromultimer.

In certain aspects, the cargo polypeptide is fused to the albumin or alloalbumin based transporter polypeptide. In some aspects, the cargo polypeptide is fused to the transferrin based transporter polypeptide. In certain aspects, the cargo polypeptide is fused to the annexin based transporter polypeptide. In some aspects, the fusion is at the N terminus of the transporter polypeptide. In certain aspects, the fusion is at the C terminus of the transporter polypeptide. In some aspects, the fusion involves a bridging linker or spacer molecule. In some aspects, the cargo polypeptide is chemically conjugated to the transporter polypeptide. In certain aspects, the cargo polypeptide is attached to the transporter polypeptide by means of chemical ligation or a disulfide bond.

Provided herein are heteromultimer proteins that comprise at least two polypeptide constructs, wherein each polypeptide construct comprises at least one cargo polypeptide, and a transporter polypeptide, such that said transporter polypeptides self-assemble to form the heteromultimer. In some aspects, each transporter polypeptide is an alloalbumin based polypeptide, such that said alloalbumin based polypeptides self-assemble to form the heteromultimer. In some aspects, each transporter polypeptide is a transferrin based polypeptide. In some aspects, each transporter polypeptide is an annexin based polypeptide. In certain aspects, each monomeric transporter polypeptide is unstable and preferentially forms a heteromultimer with at least one other transporter polypeptide.

In some embodiments, a heteromultimer described herein is a heterodimer. In some embodiments cargo polypeptide is an antibody, enzyme, hormone, therapeutic polypeptide, antigen, chemotoxin, radiotoxin, cytokine or variant or fragment thereof. In some embodiments, the cargo polypeptide of one polypeptide construct functions in synergy with the cargo polypeptide of another polypeptide construct.

Described herein are heteromultimer proteins that comprise at least two polypeptide constructs, wherein each polypeptide construct comprises at least one cargo polypeptide, and an annexin based polypeptide, such that said annexin based polypeptides self-assemble to form the heteromultimer with a quasi-native structure of monomeric annexin or analog thereof. In some aspects, the annexin is Annexin A1. In some aspects, a heteromultimer described herein is a heterodimer. In some aspects cargo polypeptide is an antibody, enzyme, hormone, therapeutic polypeptide, antigen, chemotoxin, radiotoxin, cytokine, ligand to a receptor, receptor or variant or fragment thereof. In some aspects, the cargo polypeptide of one polypeptide construct functions in synergy with the cargo polypeptide of another polypeptide construct. In some aspects the cargo polypeptide can be an agonist or antagonist to the cargo polypeptide of another polypeptide construct.

Provided herein are heterodimer proteins that comprise at least two monomeric fusion proteins, wherein each monomeric fusion proteins comprises at least one cargo polypeptide fused to an albumin derived transporter polypeptide of the invention, such that said albumin derived polypeptides self-assemble to form the multifunctional heterodimer. In certain embodiments are heterodimeric proteins comprising a first monomer which comprises at least one cargo polypeptide fused to an albumin derived polypeptide; and a second monomer that comprises at least one cargo polypeptide fused to an albumin derived polypeptide. In certain embodiments, the at least one cargo polypeptide of the first monomer is different from the at least one cargo polypeptide of the second monomer. In certain embodiments, the at least one cargo polypeptide of the first monomer is the same as the at least one cargo polypeptide of the second monomer.

In certain aspects described herein are heteromultimer proteins that comprise at least two monomeric fusion proteins, wherein each monomeric fusion proteins comprises at least one cargo polypeptide fused to an alloalbumin derived polypeptide, such that said alloalbumin derived polypeptides self-assemble to form the multifunctional heteromultimer. In certain aspects are heteromultimer proteins that comprise at least two monomeric fusion proteins, wherein each monomeric fusion proteins comprises at least one cargo polypeptide fused to a transferrin derived polypeptide, such that said transferrin derived polypeptides self-assemble to form the heteromultimer. In certain aspects are heteromultimer proteins that comprise at least two monomeric fusion proteins, wherein each monomeric fusion proteins comprises at least one cargo polypeptide fused to an annexin derived polypeptide, such that said annexin derived polypeptides self-assemble to form the heteromultimer. In certain aspects, the annexin is Annexin A2.

In certain aspects are heteromultimer proteins comprising a first polypeptide construct which comprises at least one cargo polypeptide fused to an alloalbumin derived polypeptide; and a second polypeptide construct that comprises at least one cargo polypeptide fused to an alloalbumin derived polypeptide. In certain aspects, the at least one cargo polypeptide of the first polypeptide construct is different from the at least one cargo polypeptide of the second polypeptide construct. In certain aspects, the at least one cargo polypeptide of the first polypeptide construct is the same as the at least one cargo polypeptide of the second polypeptide construct.

Provided herein is a heteromultimer that comprises: at least two monomers, each comprising a transporter polypeptide and optionally at least one cargo molecule attached to said transporter polypeptide, wherein each transporter polypeptide is obtained by segmentation of a whole protein such that said transporter polypeptides self-assemble to form quasi-native whole protein. In certain embodiments, the heteromultimer is multispecific. In certain embodiments, the transporter polypeptides are not derived from an antibody. In some embodiments, each monomer preferentially forms the heteromultimer as compared to a monomer or a homomultimer. In an embodiment of the heteromultimer, at least one cargo molecule is a theraputic agent, or a biomolecule. In some embodiments, at least one cargo molecule is a biomolecule which is selected from a polypeptide, DNA, PNA, or RNA.In some embodiments, each transporter polypeptide is a derivate of albumin or alloalbumin. In an embodiment, each transporter polypeptide is a derivate of annexin. In certain embodiments, each transporter polypeptide is a derivate of transferrin.

In certain aspects are pharmaceutical formulations that comprise an albumin-based and/or alloalbumin-based heteromultimeric protein described herein and a pharmaceutically acceptable diluent or carrier. In certain aspects are pharmaceutical formulations that comprise a transferrin-based heteromultimeric protein described herein and a pharmaceutically acceptable diluent or carrier. In certain aspects are pharmaceutical formulations that comprise an annexin-based heteromultimeric protein described herein and a pharmaceutically acceptable diluent or carrier. In certain aspects are pharmaceutical formulations that comprise an Annexin-A2 based heteromultimeric protein described herein and a pharmaceutically acceptable diluent or carrier. In certain aspects, a formulation described herein is provided as part of a kit or container. In certain aspects, the kit or container is packaged with instructions pertaining to extended shelf life of the therapeutic protein. In some aspects, a heteromultimer described herein is used in a method of treating (e.g., ameliorating) preventing, or diagnosing a disease or disease symptom in an individual, comprising the step of administering said formulation to the individual.

Provided herein is a method of obtaining fusion protein scaffolds with a known number of conjugation sites based on any transport protein of interest.

Also provided are transgenic organisms modified to contain nucleic acid molecules described herein to encode and express monomeric fusion proteins described herein.

Other aspects and features of the present invention will become apparent to those ordinarily skilled in the art upon review of the following description of specific aspects of the invention in conjunction with the accompanying figures.

### BRIEF DESCRIPTION OF THE DRAWINGS

In drawings which illustrate embodiments of the invention,
**Figure 1** depicts the structure of the Human Serum Albumin (HSA) molecule. The alpha helical sections of the secondary structure are shown schematically along with the bonds represented as sticks.
**Figure 2** is a plot of buried solvent accessible surface area at the interface of two albumin-based polypeptides. ABH1 is represented by the structure to the left, while ABH2 is represented by the right structure.
**Figure 3** depicts two albumin-based polypeptides expressed separately. The two polypeptides are shown in light and dark grey respectively. Each polypeptide comprises two fusion sites for functional cargo proteins and these sites are represented as spheres. The disulphide residues in structure are shown as sticks.
**Figure 4** is a schematic representation of bispecific and other multifunctional therapeutics based on the multispecific heteromultimer described herein. The albumin-based, or alloalbumin-based polypeptides are denoted A1 and A2. Multifunctional heteromultimers are obtained by conjugating antigen binding motifs, cytokines and other forms of signaling molecules, chemotoxin, radiotoxins or other functionally relevant immunoconjugates to N and/or C terminal sites on A1 and A2 and this is represented by the □ symbol.
**Figure 5** is a schematic of a bispecific antibody derived from a heterodimeric Fc domain. Albumin or alloalbumin based polypeptides are connected to the C-terminal of the Fc to selectively drive the formation of heterodimers.

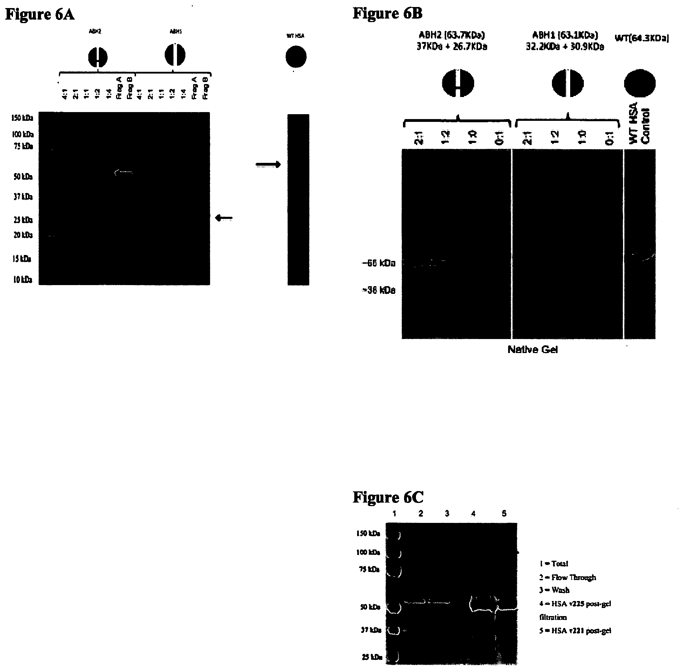

**Figure 6B** shows native gel electrophoresis profiles of full-length HSA and heterodimer scaffolds Albumin-based heteromultimer -1 (ABH1) and Albumin-based heteromultimer-2 (ABH2) formed by coexpression of HSA based transporter polypeptides. **Figure 6A** shows a non-reducing SDS-PAGE gel profile of ABH2, ABH1 and WT HSA. The two fragment polypeptides of ABH1 and ABH2 were co-expressed in different DNA ratios, as indicated by the ratios at the top of the figure. As controls and to observe homodimerization, fragments A and B were expressed independently. **Figure 6C** is a native gel of initial, flowthrough, wash and final elute after the Blue Sepharose purification of WT-HSA (v225) and ABH2 (v221).
**Figure 7** shows stability of wild type HSA and heterodimer scaffolds ABH1 and ABH2 studied using Differential Scanning Calorimetry
**Figures 8A-8B** show equilibrium binding isotherms 3000 nM FcRN 3x dilution series over 3000 RUs. Figure 8B shows Albumin and Figure 8A shows heteromultimer scaffold ABH1
**Figure 9** shows scheme for multivalent Albumin based heteromultimers comprising anti-Her2/neu and anti-CD 16 scFv bioactive fusions
**Figures 10A-10B** contain a non-reducing SDS PAGE analysis of the heteromultimer ABH2 fusions described in Table 8. The gel indicates all constructs form the correct complex with expected MW.
**Figure 11** shows structure of Annexin molecule based on the PDB structure 1MCX. The two monomers that will be derived by splitting the Annexin molecule are color coded as light and dark grey units. The sites of fusion for the cargo protein are represented as spheres.
**Figure 12** shows a plot of the buried solvent accessible surface area at the interface of Annexin based tranporter polypeptide-1, and Annexin based tranporter polypeptide-2.
**Figure 13** shows structure of transferrin molecule based on the PDB structure 1H76. The two monomers derived by splitting the transferrin molecule are color coded as light and dark grey units. The sites of fusion for the cargo protein are represented as spheres.
**Figure 14** shows a plot of the buried solvent accessible surface area at the interface of two transferrin based transporter polypeptides described herein. A split transferrin near residue position 330 as designed herein, forms a heterodimer with about 1800 Å² of buried surface area.
**Figure 15A-15P** depict heteromultimers comprising albumin based transporter polyptides that self-associate to form quasi-native monomeric albumin. Each pair of transporter polypeptides is obtained by segmentation of albumin at a unique site, different from the other
**Figures 16A** and **16B** provides the structures of ABH2 and ABH1 respectively. Both diagrams include a) the location of the cut site in WT-HSA to create the albumin-based protein, b) location of the new C' and N' cut sites and c) a simplified circular rendering of both ABH1 and ABH2, utilized in numerous later examples.
**Figure 17** depicts various mono, bi, tri and tetra-valent configurations of anti-Her2 scFv that can be attached to ABH2. Controls included the ABH2 scaffold, the anti-HER2 scFV, the one-armed Fc and the bivalent Fc. Additionally, the ability of the various ABH2 constructs to bind to SKOV cells expressing Her2 is indicated with the K_{D} values within the tables.
**Figure 18A** shows the binding curves of four anti-Her3 x Her2 loaded constructs (v1087, v1088, v1089 and v1090 to MALME-3M cells. Affinity for the cells was evaluated using FACS with FITC-labeled anti-HSA antibodies. The lower left graph displays the concentration on a linear scale while the lower right is on a logarithmic scale. **Figure 18B** shows the binding curves of the aforementioned four constructs to SKOV-3 cells. The lower left graph displays a linear scale while the lower right displays a logarithmic one.
**Figure 19A -D** demonstrate the stability of four anti-Her2 (v519, v520, v518 and v517) loaded ABH2 in human sera. Binding affinity to SKOV-3 cells was assessed using FACS with FITC-labeled anti-HSA antibody.
**Figure 20A** and **20B** shows the binding affinity of bispecific anti-Her2 x Her3 ABH2 (v1378) to FcRn as compared to the v1087 (MM-111) control. The graphs represent the surface plasmon resonance (SPR) curves of the two compounds represented as Resp. Diff. over Time.
**Figure 21** depicts the native gel profiles of both the ABH2 scaffold (v221) and a monovalent anti-Her2 ABH2 (v517) incubated for five days at three temperatures (37°C, room temperature and 4°C).
**Figure 22A** depicts the native gel profile of the total, flowthrough, wash and elution fractions of WT HSA after Blue Sepharose purification. **Figure 22B** is a chromatogram of WT-HSA after Blue Sepharose purification applied to a gel filtration column. Different peaks represent different protein products. **Figure 22C** is a chromatogram of WT-HSA after AlbuPure purification, fractionated via application to gel filtration column.
**Figure 23A** is a chromatogram of the liquid chromatography analysis of WT-HSA (v225) after AlbuPure purification and gel filtration. **Figures 23B-23E** are chromatograms of AlbuCORE scaffolds 1 (v225), 3 (v1636), 6 (v1638) and 9 (v1640) respectively after AlbuPure purification, separated into fractions and analyzed via gel filtration LC.
**Figure 24A** represents the gel filtration LC analysis of an albumin scaffold (v221) after AlbuPure purification. The express product was scaled-up via via transient co-expression in CHO cells. **Figure 24B** shows non-reducing (indicated by the -DTT) and reducing (indicated by the +DTT) SDS-PAGE gel profiles of the same variant.
**Figure 25A** represents the native gel profiles of the total, flowthrough, wash and final elute of WT-HSA and AlbuCORE-A proteins after the Albupure purification. **Figure 25B** represents an LC/MS analysis of the same two proteins.
**Figure 26A** is a diagram indicating the degree of spatial separation between the four termini in ABH2. **Figure 26B** is a comparative diagram demonstrating the degree of spatial separation between the variable regions of a normal antibody molecule. **Figure 26C** shows four Albucore scaffolds which have different and variable inter-termini distances for alternative antigen binding.
**Figure 27** shows the DSC melting curves of AlbuCORE scaffolds 1, 3, 6 and 9. In each graph, the curve and melting point of each albumin-based scaffold is compared to the curve and 75°C melting point of the WT HSA.
**Figure 28A** is the schematic rendering of anti-CD3xCD19 bispecific bivalent heteromultimers on either a WT-HSA or a spliced albumin scaffold. **Figure 28B** represents the schematic renderings of additional mononvalent anti-CD3 heteromultimer constructs as well as both monovalent and multivalent anti-CD 19 heteromultimer constructs. **Figure 28C** represents a non-reducing PAGE gel of the previous described constructs after each was expressed in a suspension growing human CHO system.
**Figure 29** provides the Ka and Hill Slope measurements of four anti-CD3xCD19 bispecific bivalent heteromultimers on either a WT-HSA or spliced albumin scaffold. The binding tests assessed the variant's ability to bind Jurkat or Raji cells as analyzed using FACS.
**Figure 30A** displays FACS cell population graphs of the BiTE-like control (v891), an anti-CD3xCD19 albumin-based heteromultimer (v1093) and an anti-CD3xCD19 Het-Fc-based heteromultimer (v873). Binding to Raji and Jurkat cells was assessed. **Figure 30B** shows additional FACS graphs of the three aforementioned constructs and as compared against an albumin control (v221).
**Figure 31A** shows a SDS-PAGE of the v218 expression product before, during and after purification using Co2+ affinity. **Figure 31B** is a chromatogram of the same construct after additional purification via gel filtration.
**Figure 32** Plot of residue contacts made by a subset of residues in the human serum albumin derived from its 3-dimensional structure.
**Figure 33** Plot of nature of contacts and interactions achieved by select hotspot residues in human serum albumin.
**Figure 34** A graphical representation of hot spot residues or clusters (patches) of hotspot residues in human serum albumin.
**Figure 35** shows a gel demonstrating the expression of several albumin-based heteromultimers comprising introduced cysteine residues.

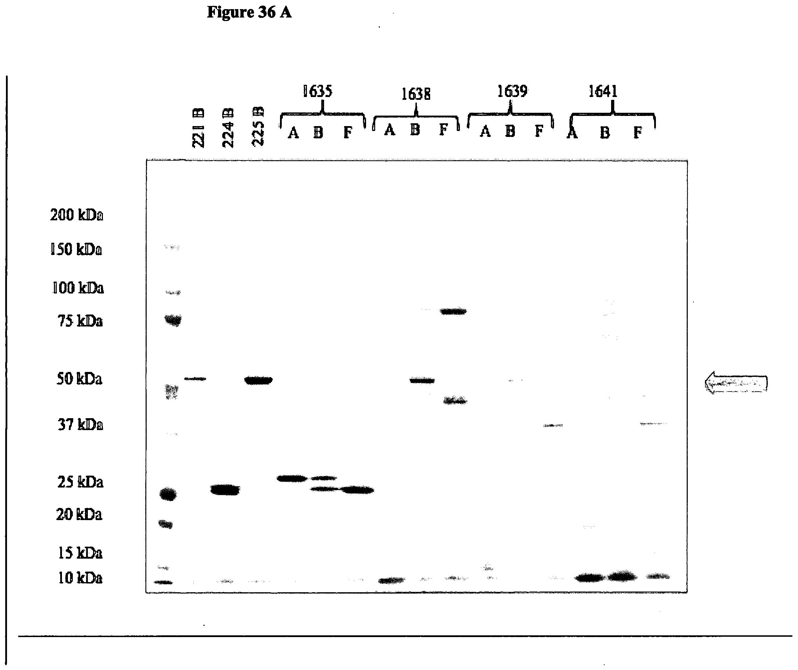

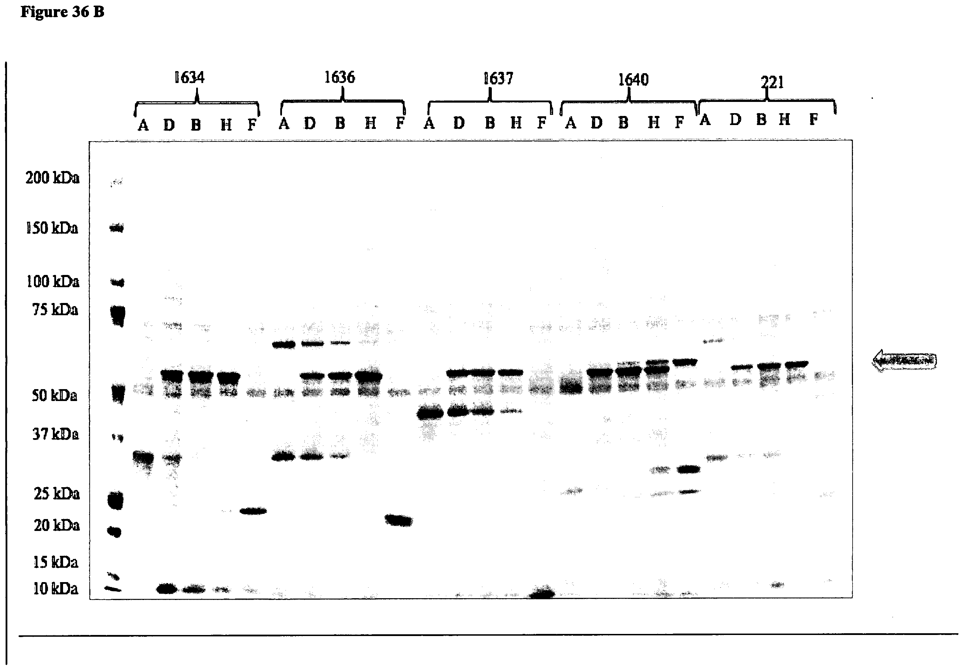

**Figure 36A** provides a non-reducing gel showing the expression of heteromultimer variants 1635, 1638, 1639, and 1641; **Figure 36B** provides a non-reducing gel showing the expression of heteromultimer variants 1634, 1636, 1637, 1640, and 221. CHO cells were transfected with the following DNA ratios: Ratio A = plasmid A:plasmid B 100%:0%; Ratio D = plasmid A: plasmid B 66%:34%; Ratio B = plasmid A: plasmid B 50%:50%; Ratio H = plasmid A: plasmid B 34%:66%; and Ratio F = plasmid A:plasmid B 0%/100%.

### DETAILED DESCRIPTION

In the realm of therapeutic proteins, bispecific molecules exhibit dual target specificities or are able to simultaneously perform multiple functional roles by providing the necessary spatiotemporal organization necessary for drug action. In one aspect, bispecific molecules are particularly interesting when the mode of therapeutic action involves retargeting of effector cells or molecules to a target such as a tumor cell [Muller D. and Kontermann R.E. (2010) Biodrugs 24, 89-98]. The development of bispecific therapeutic proteins with favorable pharmacokinetics and functional activity in stable and homogeneous condition has been a challenge. Attempts have been made to assemble bispecific units from multiple antigen binding domains using a number of approaches. These techniques have involved using heterodimeric antibody IgG molecule, using leucine zipper proteins such as the Fos/Jun pair or other scaffolds assembled from the alternate organizations of the light and heavy chains of the variable domains in an antibody. Kipriyanov and Le Gall have reviewed the design of a variety of bispecific constructs [Kipriyanov S.M. & Le Gall F. (2004) Curr Opin Drug Discov Dev 7, 233-242]. The use of a heterodimeric antibody IgG molecule wherein mutations are introduced in the CH3 domain of the antibody to achieve the heterodimer and hence introduce the two unique antigen binding sites into one molecule is very attractive because of the natural immunoglobulin like structure of this construct. Further, the Fc portion of the antibody is involved in interactions with the neonatal Fc receptor (FcRn) which mediates an endocytic salvage pathway and this is attributed to improved serum half-life of the antibody molecule [Roopenian D. & Akilesh S. (2007) Nature Rev Immunol 7, 715-725]. On the other hand, antibody based bispecific molecules have been problematic in clinical trials because of the strong cytokine responses as a result of the concurrent effector activity induced via the Fc portion of the bispecific antibody [Weiner L.M.; Alpaugh R.K. et al. (1996) Cancer Immunol Immunother 42, 141-150]. This highlights the needs for novel scaffolds that can aid in the design of bispecific and immunoconjugate molecules.

The human serum album (HSA) protein is the most abundant component of blood, accounting for close to 60% of the total protein in blood serum at a concentration of about 40 mg/ml. Albumin is also one of the longest-lived proteins in the circulatory system with a half-life of about 19 days. Interestingly, the same endocytic salvage pathway dependent on FcRn molecules that prevents antibody degradation is known to interact with the HSA molecule as well [Chaudhary C.; Mehnaz S. et al. (2003) J Exp Med 197, 315-322].

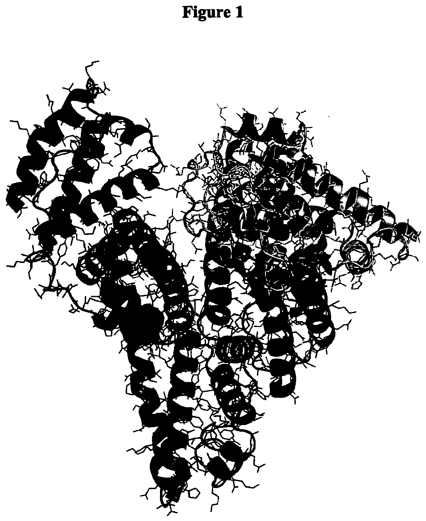

HSA (shown in Figure 1) is a non-glycosylated 585-residue single polypeptide protein and the 3-dimensional structure of the protein was first observed using X-ray crystallography by Carter and coworkers [reviewed in Carter, D.C. & Ho, J.X. (1994) Adv Prot Chem 45, 153-203]. The HSA protein consists of three homologous domains: DI, DII, DIII, attributed to gene duplication, a feature common to the serum albumin in other species as well [Gray J.E. & Doolittle R.F. (1992) Protein Sci 1, 289-302]. Each of the three domains have been expressed and characterized separately and shown to be independently stable [Dockal M., Carter D.C. & Ruker F. (1999) J Biol Chem 274, 29303-29310]. Each domain is made up of 10 helical segments and based on the inter-helical organization each domain can be further classified into 2 sub-domains comprised of helix 1-6 and 7-10 respectively. HSA has 17 disulphide bonds in total and all these cysteine pairs forming the linkages are within the individual domains. In general, HSA is a very stable due to the large number of disulphide bonds as well as the predominantly helical fold. The sequence identities of albumin molecules across a number of species is quite large, greater than 70% among albumin cDNA derived from humans, horse, bovine, rat, etc. [Carter, D.C. & Ho, J.X. (1994) Adv Prot Chem 45, 153-203].

Split protein pairs have been used as sensors to understand protein-protein interactions in the area of functional proteomics. The approach involves identifying suitable segments from a protein that can reconstitute to form an active native-like protein. Generating new split proteins is technically demanding. For a protein to be split in a functionally useful manner, the segmentation site has to yield two segments that efficiently reconstitute into the quasi-native protein when associated to each other. Further, the component protein segments should be soluble enough to stay in solution and selectively associate with the partner segments such that manufacture yields and purification will be economical. Deriving split protein segments that would recombine to form the quasi-native structure resembling the monomeric native protein is quite challenging [Tafelmeyer P., Johnsson N. & Johnsson K. Chem & Biol 11, 681-689]. Such split proteins have not been used in the design of protein therapeutics, or as cargo delivery vehicles in the past.

The present invention provides heteromultimers comprisng a first monomer that comprises (i) a first transporter polypeptide; and a second monomer that comprises (ii) a second transporter polypeptide; wherein each of said first and second transporter polypeptide comprises an amino acid sequence with at least 90% identity to a segment of an albumin polypeptide; and wherein said first and second transporter polypeptides are obtained by segmentation of said albumin polypeptide at a segmentation site, wherein said transporter polypeptides self-assemble to form a quasi-native structure of the monomeric form of said albumin polypeptide. In one embodiment, the segmentation site is selected such that it resides on a loop of the albumin polypeptide that has a high solvent accessible surface area (SASA) and limited contact with the rest of the albumin structure, b) results in a complementary interface between the transporter polypeptides, wherein the interface is apolar, extensive and interdigitate, and wherein the location of the segmentation site is: (a) between residues 339 and 340 of SEQ ID NO:1; or (b) between residues 300 and 301 of SEQ ID NO: 1; or (c) between residues 364 and 365 of SEQ ID NO: 1; or (d) between residues 441 and 442 of SEQ ID NO:1; or (e) between residues 171 and 172 of SEQ ID NO:1; or (f) between residues 281 and 282 of SEQ ID NO:1; or (g) after residue 83 and before residue 85 of SEQ ID NO:1 and residue 84 is deleted; wherein the numbering of residues begins with the first residue after the signal sequence. Such heteromultimers exhibit stability comparable to that of wild-type albumin. The first and second monomers may further comprise at least one cargo polypeptide. Heteromultimers comprising said at least one cargo polypeptide may be used as therapeutics for the treatment of disease.

### Definitions

It is to be understood that this invention is not limited to the particular protocols; cell lines, constructs, and reagents described herein and as such may vary. It is also to be understood that the terminology used herein is for the purpose of describing particular embodiments only, and is not intended to limit the scope of the present invention, which will be limited only by the appended claims.

As used herein and in the appended claims, the singular forms "a," "an," and "the" include plural reference unless the context clearly indicates otherwise. Thus, for example, reference to a "HSA", "HA", "albumin", "human serum albumin" and various capitalized, hyphenated and unhyphenated forms is a reference to one or more such proteins and includes variants, derivatives, fragments, equivalents thereof known to those of ordinary skill in the art, and so forth.

Unless defined otherwise, all technical and scientific terms used herein have the same meaning as commonly understood to one of ordinary skill in the art to which this invention belongs. Although any methods, devices, and materials similar or equivalent to those described herein can be used in the practice or testing of the invention, the preferred methods, devices and materials are now described.

A "heteromultimer" or "heteromultimeric polypeptide" is a molecule comprising at least a first monomer comprising a first transporter polypeptide and a second monomer comprising a second transporter polypeptide, wherein the second polypeptide differs in amino acid sequence from the first polypeptide by at least one amino acid residue. The heteromultimer can comprise a "heterodimer" formed by the first and second transporter polypeptides. In certain embodiments, the heteromultimer can form higher order tertiary structures such as, but not restricted to trimers and tetramers. In some embodiments, transporter polypeptides in addition to the first and second transporter polypeptides are present. In certain embodiments, the assembly of transporter polypeptides to form the heteromultimer is driven by surface area burial. In some embodiments, the transporter polypeptides interact with each other by means of electrostatic interactions and/or salt-bridge interactions that drive heteromultimer formation by favoring heteromultimer formation and/or disfavoring homomultimer formation. In some embodiments, the transporter polypeptides inteact with each other by means of hydrophobic interactions that drive heteromultimer formation by favoring heteromultimer formation and/or disfavoring homomultimer formation. In certain embodiments, the transporter polypeptides inteact with each other by means of covalent bond formation. In certain embodiments, the covalent bonds are formed between naturally present or introduced cysteines that drive heteromultimer formation. In certain embodiments of the heteromultimers described herein, no covalent bonds are formed between the monomers. In some embodiments, the transporter polypeptides inteact with each other by means of packing/size-complementarity/knobs-into-holes/protruberance-cavity type interactions that drive heteromultimer formation by favoring heteromultimer formation and/or disfavoring homomultimer formation. In some embodiments, the transporter polypeptides inteact with each other by means of cation-pi interactions that drive heteromultimer formation by favoring heteromultimer formation and/or disfavoring homomultimer formation. In certain embodiments the individual transporter polypeptides cannot exist as isolated monomers in solution. In certain embodiments, the heteromultimer is the preferred state of the individual transporter polypeptides as compared to the monomer.

The term "bispecific" is intended to include any agent, e.g., heteromultimer, monomer, protein, peptide, or protein or peptide complex, which has two different binding specificities. For example, in some embodiments, the molecule may bind to, or interact with, (a) a cell surface target molecule and (b) an Fc receptor on the surface of an effector cell. In certain embodiments of a heteromultimer described herein, at least one monomer is bispecific formed by attaching to the same transporter polypeptide, two cargo molecules with different binding specificities. In certain embodiments of a heteromultimer described herein, the heteromultimer is itself bispecific formed by attaching to the transporter polypeptides, at least two cargo molecules with different specificities. The term "multispecific molecule" or "heterospecific molecule" is intended to include any agent, e.g., a protein, peptide, or protein or peptide complex, which has more than two different binding specificities. For example, the molecule may bind to, or interact with, (a) a cell surface target molecule such as but not limited to cell surface antigens, (b) an Fc receptor on the surface of an effector cell, and (c) at least one other component. Accordingly, embodiments of the heteromultimers described herein, are inclusive of, but not limited to, bispecific, trispecific, tetraspecific, and other multispecific molecules. In certain embodiments, these molecules are directed to cell surface antigens, such as CD30, and to other targets, such as Fc receptors on effector cells.

Unless indicated otherwise, the expression "multivalent" is used throughout this specification to denote a heteromultimer comprising at least two sites of attachment for target molecules. The multivalent heteromultimer is designed to have multiple binding sites for desired targets. In certain embodiments, the binding sites are on at least one cargo molecules attached to a transporter polypeptide. In certain embodiments, at least one binding site is on a transporter polypeptide. The expression "bivalent" is used throughout this specification to denote a heteromultimer comprising two target binding sites. In certain embodiments of a bivalent heteromultimer, both binding sites are on the same monomer. The expression "trivalent" is used throughout this specification to denote a heteromultimer comprising three target binding sites. The expression "tetravalent" is used throughout this specification to denote a heteromultimer comprising four target binding sites.

"Fusion proteins" and polypeptides are created by joining two or more genes that originally code for separate polypeptides. Translation of this fusion gene results in a single polypeptide with functional properties derived from each of the original polypeptides. In embodiments of the heteromultimers described herein, at least one monomer may comprise a fusion protein formed by the fusion of at least one cargo polypeptide to the N- or C-terminus of a transporter polypeptide.

The term "substantially purified" refers to a heteromultimer described herein, or variant thereof that may be substantially or essentially free of components that normally accompany or interact with the protein as found in its naturally occurring environment, i.e. a native cell, or host cell in the case of recombinantly produced heteromultimer that in certain embodiments, is substantially free of cellular material includes preparations of protein having less than about 30%, less than about 25%, less than about 20%, less than about 15%, less than about 10%, less than about 5%, less than about 4%, less than about 3%, less than about 2%, or less than about 1% (by dry weight) of contaminating protein. When the heteromultimer or variant thereof is recombinantly produced by the host cells, the protein in certain embodiments is present at about 30%, about 25%, about 20%, about 15%, about 10%, about 5%, about 4%, about 3%, about 2%, or about 1% or less of the dry weight of the cells. When the heteromultimer or variant thereof is recombinantly produced by the host cells, the protein, in certain embodiments, is present in the culture medium at about 5 g/L, about 4 g/L, about 3 g/L, about 2 g/L, about 1 g/L, about 750 mg/L, about 500 mg/L, about 250 mg/L, about 100 mg/L, about 50 mg/L, about 10 mg/L, or about 1 mg/L or less of the dry weight of the cells. In certain embodiments, "substantially purified" heteromultimer produced by the methods described herein, has a purity level of at least about 30%, at least about 35%, at least about 40%, at least about 45%, at least about 50%, at least about 55%, at least about 60%, at least about 65%, at least about 70%, specifically, a purity level of at least about 75%, 80%, 85%, and more specifically, a purity level of at least about 90%, a purity level of at least about 95%, a purity level of at least about 99% or greater as determined by appropriate methods such as SDS/PAGE analysis, RP-HPLC, SEC, and capillary electrophoresis.

A "recombinant host cell" or "host cell" refers to a cell that includes an exogenous polynucleotide, regardless of the method used for insertion, for example, direct uptake, transduction, f-mating, or other methods known in the art to create recombinant host cells. The exogenous polynucleotide may be maintained as a nonintegrated vector, for example, a plasmid, or alternatively, may be integrated into the host genome.

As used herein, the term "medium" or "media" includes any culture medium, solution, solid, semi-solid, or rigid support that may support or contain any host cell, including bacterial host cells, yeast host cells, insect host cells, plant host cells, eukaryotic host cells, mammalian host cells, CHO cells, prokaryotic host cells, E. coli, or Pseudomonas host cells, and cell contents. Thus, the term may encompass medium in which the host cell has been grown, e.g., medium into which the protein has been secreted, including medium either before or after a proliferation step. The term also may encompass buffers or reagents that contain host cell lysates, such as in the case where a heteromultimer described herein is produced intracellularly and the host cells are lysed or disrupted to release the heteromultimer.

"Refolding," as used herein describes any process, reaction or method which transforms disulfide bond containing polypeptides from an improperly folded or unfolded state to a native or properly folded conformation with respect to disulfide bonds.

"Cofolding," as used herein, refers specifically to refolding processes, reactions, or methods which employ at least two monomeric polypeptides which interact with each other and result in the transformation of unfolded or improperly folded polypeptides to native, properly folded polypeptides.

As used herein, the term "modulated serum half-life" means the positive or negative change in circulating half-life of a cargo polypeptide that is comprised by a heteromultimer described herein relative to its native form. Serum half-life is measured by taking blood samples at various time points after administration of heteromultimer, and determining the concentration of that molecule in each sample. Correlation of the serum concentration with time allows calculation of the serum half-life. Increased serum half-life desirably has at least about two-fold, but a smaller increase may be useful, for example where it enables a satisfactory dosing regimen or avoids a toxic effect. In some embodiments, the increase is at least about three-fold, at least about five-fold, or at least about ten-fold.

The term "modulated therapeutic half-life" as used herein means the positive or negative change in the half-life of the therapeutically effective amount of a cargo polypeptide comprised by a heteromultimer described herein, relative to its non-modified form. Therapeutic half-life is measured by measuring pharmacokinetic and/or pharmacodynamic properties of the molecule at various time points after administration. Increased therapeutic half-life desirably enables a particular beneficial dosing regimen, a particular beneficial total dose, or avoids an undesired effect. In some embodiments, the increased therapeutic half-life results from increased potency, increased or decreased binding of the modified molecule to its target, increased or decreased breakdown of the molecule by enzymes such as proteases, or an increase or decrease in another parameter or mechanism of action of the non-modified molecule or an increase or decrease in receptor-mediated clearance of the molecule.

The term "isolated," when applied to a nucleic acid or protein, denotes that the nucleic acid or protein is free of at least some of the cellular components with which it is associated in the natural state, or that the nucleic acid or protein has been concentrated to a level greater than the concentration of its in vivo or in vitro production. It can be in a homogeneous state. Isolated substances can be in either a dry or semi-dry state, or in solution, including but not limited to, an aqueous solution. It can be a component of a pharmaceutical composition that comprises additional pharmaceutically acceptable carriers and/or excipients. Purity and homogeneity are typically determined using analytical chemistry techniques such as polyacrylamide gel electrophoresis or high performance liquid chromatography. A protein which is the predominant species present in a preparation is substantially purified. In particular, an isolated gene is separated from open reading frames which flank the gene and encode a protein other than the gene of interest. The term "purified" denotes that a nucleic acid or protein gives rise to substantially one band in an electrophoretic gel. Particularly, it may mean that the nucleic acid or protein is at least 85% pure, at least 90% pure, at least 95% pure, at least 99% or greater pure.

The term "nucleic acid" refers to deoxyribonucleotides, deoxyribonucleosides, ribonucleosides, or ribonucleotides and polymers thereof in either single- or doublestranded form. Unless specifically limited, the term encompasses nucleic acids containing known analogues of natural nucleotides which have similar binding properties as the reference nucleic acid and are metabolized in a manner similar to naturally occurring nucleotides. Unless specifically limited otherwise, the term also refers to oligonucleotide analogs including PNA (peptidonucleic acid), analogs of DNA used in antisense technology (phosphorothioates, phosphoroamidates, and the like). Unless otherwise indicated, a particular nucleic acid sequence also implicitly encompasses conservatively modified variants thereof (including but not limited to, degenerate codon substitutions) and complementary sequences as well as the sequence explicitly indicated. Specifically, degenerate codon substitutions may be achieved by generating sequences in which the third position of one or more selected (or all) codons is substituted with mixed-base and/or deoxyinosine residues (Batzer et al., Nucleic Acid Res. 19:5081 (1991); Ohtsuka et al., J. Biol. Chem. 260:2605-2608 (1985); Rossolini et al., Mol. Cell. Probes 8:91-98 (1994)).

The terms "polypeptide," "peptide" and "protein" are used interchangeably herein to refer to a polymer of amino acid residues. That is, a description directed to a polypeptide applies equally to a description of a peptide and a description of a protein, and vice versa. The terms apply to naturally occurring amino acid polymers as well as amino acid polymers in which one or more amino acid residues is a non-naturally encoded amino acid. As used herein, the terms encompass amino acid chains of any length, including full length proteins, wherein the amino acid residues are linked by covalent peptide bonds.

The term "amino acid" refers to naturally occurring and non-naturally occurring amino acids, as well as amino acid analogs and amino acid mimetics that function in a manner similar to the naturally occurring amino acids. Naturally encoded amino acids are the 20 common amino acids (alanine, arginine, asparagine, aspartic acid, cysteine, glutamine, glutamic acid, glycine, histidine, isoleucine, leucine, lysine, methionine, phenylalanine, praline, serine, threonine, tryptophan, tyrosine, and valine) and pyrrolysine and selenocysteine. Amino acid analogs refers to compounds that have the same basic chemical structure as a naturally occurring amino acid, i.e., an a carbon that is bound to a hydrogen, a carboxyl group, an amino group, and an R group, such as, homoserine, norleucine, methionine sulfoxide, methionine methyl sulfonium. Such analogs have modified R groups (such as, norleucine) or modified peptide backbones, but retain the same basic chemical structure as a naturally occurring amino acid. Reference to an amino acid includes, for example, naturally occurring proteogenic L-amino acids; D-amino acids, chemically modified amino acids such as amino acid variants and derivatives; naturally occurring non-proteogenic amino acids such as β-alanine, ornithine, etc.; and chemically synthesized compounds having properties known in the art to be characteristic of amino acids. Examples of non-naturally occurring amino acids include, but are not limited to, α-methyl amino acids (e.g. α-methyl alanine), D-amino acids, histidine-like amino acids (e.g., 2-amino-histidine, β-hydroxy-histidine, homohistidine), amino acids having an extra methylene in the side chain ("homo" amino acids), and amino acids in which a carboxylic acid functional group in the side chain is replaced with a sulfonic acid group (e.g., cysteic acid). The incorporation of non-natural amino acids, including synthetic non-native amino acids, substituted amino acids, or one or more D-amino acids into the proteins of the present invention may be advantageous in a number of different ways. D-amino acid-containing peptides, etc., exhibit increased stability in vitro or in vivo compared to L-amino acid-containing counterparts. Thus, the construction of peptides, etc., incorporating D-amino acids can be particularly useful when greater intracellular stability is desired or required. More specifically, D-peptides, etc., are resistant to endogenous peptidases and proteases, thereby providing improved bioavailability of the molecule, and prolonged lifetimes in vivo when such properties are desirable. Additionally, D-peptides, etc., cannot be processed efficiently for major histocompatibility complex class II-restricted presentation to T helper cells, and are therefore, less likely to induce humoral immune responses in the whole organism.

Amino acids may be referred to herein by either their commonly known three letter symbols or by the one-letter symbols recommended by the IUPAC-IUB Biochemical Nomenclature Commission. Nucleotides, likewise, may be referred to by their commonly accepted single-letter codes.

"Conservatively modified variants" applies to both amino acid and nucleic acid sequences. With respect to particular nucleic acid sequences, "conservatively modified variants" refers to those nucleic acids which encode identical or essentially identical amino acid sequences, or where the nucleic acid does not encode an amino acid sequence, to essentially identical sequences. Because of the degeneracy of the genetic code, a large number of functionally identical nucleic acids encode any given protein. For instance, the codons GCA, GCC, GCG and GCU all encode the amino acid alanine. Thus, at every position where an alanine is specified by a codon, the codon can be altered to any of the corresponding codons described without altering the encoded polypeptide. Such nucleic acid variations are "silent variations," which are one species of conservatively modified variations. Every nucleic acid sequence herein which encodes a polypeptide also describes every possible silent variation of the nucleic acid. One of ordinary skill in the art will recognize that each codon in a nucleic acid (except AUG, which is ordinarily the only codon for methionine, and TGG, which is ordinarily the only codon for tryptophan) can be modified to yield a functionally identical molecule. Accordingly, each silent variation of a nucleic acid which encodes a polypeptide is implicit in each described sequence.

As to amino acid sequences, one of ordinary skill in the art will recognize that individual substitutions, deletions or additions to a nucleic acid, peptide, polypeptide, or protein sequence which alters, adds or deletes a single amino acid or a small percentage of amino acids in the encoded sequence is a "conservatively modified variant" where the alteration results in the deletion of an amino acid, addition of an amino acid, or substitution of an amino acid with a chemically similar amino acid. Conservative substitution tables providing functionally similar amino acids are known to those of ordinary skill in the art. Such conservatively modified variants are in addition to and do not exclude polymorphic variants, interspecies homologs, and alleles of the invention.

Conservative substitution tables providing functionally similar amino acids are known to those of ordinary skill in the art. The following eight groups each contain amino acids that are conservative substitutions for one another:
1) Alanine (A), Glycine (G);
2) Aspartic acid (D), Glutamic acid (E);
3) Asparagine (N), Glutamine (Q);
4) Arginine (R), Lysine (K);
5) Isoleucine (I), Leucine (L), Methionine (M), Valine (V);
6) Phenylalanine (F), Tyrosine (Y), Tryptophan (W);
7) Serine (S), Threonine (T); and [0139] 8) Cysteine (C), Methionine (M) (see, e.g., Creighton, Proteins: Structures and Molecular Properties (W H Freeman & Co.; 2nd edition (December 1993)

The terms "identical" or percent "identity," in the context of two or more nucleic acids or polypeptide sequences, refer to two or more sequences or subsequences that are the same. Sequences are "substantially identical" if they have a percentage of amino acid residues or nucleotides that are the same (i.e., about 60% identity, about 65%, about 70%, about 75%, about 80%, about 85%, about 90%, or about 95% identity over a specified region), when compared and aligned for maximum correspondence over a comparison window, or designated region as measured using one of the following sequence comparison algorithms (or other algorithms available to persons of ordinary skill in the art) or by manual alignment and visual inspection. This definition also refers to the complement of a test sequence. The identity can exist over a region that is at least about 50 amino acids or nucleotides in length, or over a region that is 75-100 amino acids or nucleotides in length, or, where not specified, across the entire sequence of a polynucleotide or polypeptide. A polynucleotide encoding a polypeptide of the present invention, including homologs from species other than human, may be obtained by a process comprising the steps of screening a library under stringent hybridization conditions with a labeled probe having a polynucleotide sequence of the invention or a fragment thereof, and isolating full-length cDNA and genomic clones containing said polynucleotide sequence. Such hybridization techniques are well known to the skilled artisan.

For sequence comparison, typically one sequence acts as a reference sequence, to which test sequences are compared. When using a sequence comparison algorithm, test and reference sequences are entered into a computer, subsequence coordinates are designated, if necessary, and sequence algorithm program parameters are designated. Default program parameters can be used, or alternative parameters can be designated. The sequence comparison algorithm then calculates the percent sequence identities for the test sequences relative to the reference sequence, based on the program parameters.

A "comparison window", as used herein, includes reference to a segment of any one of the number of contiguous positions selected from the group consisting of from 20 to 600, usually about 50 to about 200, more usually about 100 to about 150 in which a sequence may be compared to a reference sequence of the same number of contiguous positions after the two sequences are optimally aligned. Methods of alignment of sequences for comparison are known to those of ordinary skill in the art. Optimal alignment of sequences for comparison can be conducted, including but not limited to, by the local homology algorithm of Smith and Waterman (1970) Adv. Appl. Math. 2:482c, by the homology alignment algorithm of Needleman and Wunsch (1970) J. Mol. Biol. 48:443, by the search for similarity method of Pearson and Lipman (1988) Proc. Nat'l. Acad. Sci. USA 85:2444, by computerized implementations of these algorithms (GAP, BESTFIT, FASTA, and TFASTA in the Wisconsin Genetics Software Package, Genetics Computer Group, 575 Science Dr., Madison, Wis.), or by manual alignment and visual inspection (see, e.g., Ausubel et al., Current Protocols in Molecular Biology (1995 supplement)).

One example of an algorithm that is suitable for determining percent sequence identity and sequence similarity are the BLAST and BLAST 2.0 algorithms, which are described in Altschul et al. (1997) Nuc. Acids Res. 25:3389-3402, and Altschul et al. (1990) J. Mol. Biol. 215:403-410, respectively. Software for performing BLAST analyses is publicly available through the National Center for Biotechnology Information available at the World Wide Web at ncbi.nlm.nih.gov. The BLAST algorithm parameters W, T, and X determine the sensitivity and speed of the alignment. The BLASTN program (for nucleotide sequences) uses as defaults a wordlength (W) of 11, an expectation (E) or 10, M=5, N=-4 and a comparison of both strands. For amino acid sequences, the BLASTP program uses as defaults a wordlength of 3, and expectation (E) of 10, and the BLOSUM62 scoring matrix (see Henikoff and Henikoff (1992) Proc. Natl. Acad. Sci. USA 89:10915) alignments (B) of 50, expectation (E) of 10, M=5, N=-4, and a comparison of both strands. The BLAST algorithm is typically performed with the "low complexity" filter turned off.

The BLAST algorithm also performs a statistical analysis of the similarity between two sequences (see, e.g., Karlin and Altschul (1993) Proc. Natl. Acad. Sci. USA 90:5873-5787). One measure of similarity provided by the BLAST algorithm is the smallest sum probability (P(N)), which provides an indication of the probability by which a match between two nucleotide or amino acid sequences would occur by chance. For example, a nucleic acid is considered similar to a reference sequence if the smallest sum probability in a comparison of the test nucleic acid to the reference nucleic acid is less than about 0.2, or less than about 0.01, or less than about 0.001.

The phrase "selectively (or specifically) hybridizes to" refers to the binding, duplexing, or hybridizing of a molecule only to a particular nucleotide sequence under stringent hybridization conditions when that sequence is present in a complex mixture (including but not limited to, total cellular or library DNA or RNA).

The phrase "stringent hybridization conditions" refers to hybridization of sequences of DNA, RNA, or other nucleic acids, or combinations thereof under conditions of low ionic strength and high temperature as is known in the art. Typically, under stringent conditions a probe will hybridize to its target subsequence in a complex mixture of nucleic acid (including but not limited to, total cellular or library DNA or RNA) but does not hybridize to other sequences in the complex mixture. Stringent conditions are sequence-dependent and will be different in different circumstances. Longer sequences hybridize specifically at higher temperatures. An extensive guide to the hybridization of nucleic acids is found in Tijssen, Laboratory Techniques in Biochemistry and Molecular Biology--Hybridization with Nucleic Probes, "Overview of principles of hybridization and the strategy of nucleic acid assays" (1993).

As used herein, the term "eukaryote" refers to organisms belonging to the phylogenetic domain Eucarya such as animals (including but not limited to, mammals, insects, reptiles, birds, etc.), ciliates, plants (including but not limited to, monocots, dicots, algae, etc.), fungi, yeasts, flagellates, microsporidia, protists, etc.

As used herein, the term "prokaryote" refers to prokaryotic organisms. For example, a non-eukaryotic organism can belong to the Eubacteria (including but not limited to, Escherichia coli, Thermus thermophilus, Bacillus stearothermophilus, Pseudomonas fluorescens, Pseudomonas aeruginosa, Pseudomonas putida, etc.) phylogenetic domain, or the Archaea (including but not limited to, Methanococcus jannaschii, Methanobacterium thermoautotrophicum, Halobacterium such as Haloferax volcanii and Halobacterium species NRC-1, Archaeoglobus fulgidus, Pyrococcus furiosus, Pyrococcus horikoshii, Aeuropyrum pernix, etc.) phylogenetic domain.

The term "subject" as used herein, refers to an animal, in some embodiments a mammal, and in other embodiments a human, who is the object of treatment, observation or experiment. An animal may be a companion animal (e.g., dogs, cats, and the like), farm animal (e.g., cows, sheep, pigs, horses, and the like) or a laboratory animal (e.g., rats, mice, guinea pigs, and the like).

The term "effective amount" as used herein refers to that amount of heteromultimer being administered, which will relieve to some extent one or more of the symptoms of the disease, condition or disorder being treated. Compositions containing the heteromultimer described herein can be administered for prophylactic, enhancing, and/or therapeutic treatments.

The terms "enhance" or "enhancing" means to increase or prolong either in potency or duration a desired effect. Thus, in regard to enhancing the effect of therapeutic agents, the term "enhancing" refers to the ability to increase or prolong, either in potency or duration, the effect of other therapeutic agents on a system. An "enhancing-effective amount," as used herein, refers to an amount adequate to enhance the effect of another therapeutic agent in a desired system. When used in a patient, amounts effective for this use will depend on the severity and course of the disease, disorder or condition, previous therapy, the patient's health status and response to the drugs, and the judgment of the treating physician.

The term "modified," as used herein refers to any changes made to a given polypeptide, such as changes to the length of the polypeptide, the amino acid sequence, chemical structure, co-translational modification, or post-translational modification of a polypeptide. The form "(modified)" term means that the polypeptides being discussed are optionally modified, that is, the polypeptides under discussion can be modified or unmodified.

The term "post-translationally modified" refers to any modification of a natural or non-natural amino acid that occurs to such an amino acid after it has been incorporated into a polypeptide chain. The term encompasses, by way of example only, co-translational in vivo modifications, co-translational in vitro modifications (such as in a cell-free translation system), post-translational in vivo modifications, and post-translational in vitro modifications.

The term "segmentation" refers to a precise internal splice of the original protein sequence which results in "segments" of the protein sequence that preferentially associate as heteromultimers to form a quasi-native protein structure. Alternatively, segmentation can include deletion of more than one amino acid residue. In one embodiment, the deletion is one amino acid residue. In another embodiment, the deletion is two amino acid residues. In another embodiment, the deletion is three amino acid residues.

### Quasi-native Monomer Structure:

With reference to a monomeric native protein or its structure, quasi-native proteins and/or 'quasi-native structures' or quasi-native monomeric structures are heteromultimer assemblies of polypeptides derived from segments of the monomeric native protein such that said heteromultimer assemblies present functional and structural characteristics comparable to the monomeric native protein. In some embodiments, the segments are components of a polypeptide construct that also comprise other molecular entities. Proteins are naturally dynamic molecules and display an ensemble of structural configurations although we ascribe a native structure to it, such as the one obtained by X-ray crystallography. The quasi-native structures can be considered to resemble one of the alternate structural configurations in the ensemble. On a different front, homologous proteins sequences or proteins belonging to common structural families tend to fold into similar structural geometries. The member proteins belonging to this family can be deemed to achieve a quasi-native structure relative to each other. Some of the unique sequences in the protein family could also exhibit similar functional attributes and hence can be referred to as quasi-native proteins relative to each other. In the case of heteromultimers described here comprising of two or more polypeptide constructs each of which have a transporter polypeptide component, the transporter polypeptides assemble to form a quasi-native structure similar to the native monomeric form of the protein that the transporter polypeptides are derived from. The reference native protein in this case is the monomer protein from which each transporter polypeptide is derived and the reference native structure is the structure of the protein from which the transporter polypeptide is derived. We describe a case where two or more different polypeptides self-assemble to form a heteromultimer with structural and functional characteristics comparable to the native protein which itself is a monomeric entity. In some embodiments, the assembled heteromultimer has at least 50% of the activity of the monomeric native protein. In some embodiments, the assembled heteromultimer has at least 60% of the activity of the monomeric native protein. In specific embodiments, the assembled heteromultimer has at least 75% of the activity of the monomeric native protein. In some embodiments, the assembled heteromultimer has at least 80% of the activity of the monomeric native protein. In particular embodiments, the assembled heteromultimer has at least 90% of the activity of the monomeric native protein. In some embodiments, the assembled heteromultimer has at least 95% of the activity of the monomeric native protein. In some embodiments, the assembled heteromultimer has at least 99% of the activity of the monomeric native protein. In certain embodiments provided are, heteromultimers comprising transporter polypeptides derived from from albumin or allo-albumin such that the transporter polypeptides self-assemble to form a heteromultimer that exhibits native albumin like structural and/or functional characteristics such as FcRn, SPARC and/or gp60 binding. In certain embodiments are heteromultimers comprising transporter polypeptides, each transporter polypeptide comprising an amino acid sequence that has at least 75% sequence identity with an amino acid segment obtained from the native monomeric protein. In some embodiments are heteromultimers comprising transporter polypeptides, each transporter polypeptide comprising an amino acid sequence that has at least 80% sequence identity with an amino acid segment obtained from the native monomeric protein. In exemplary embodiments are heteromultimers comprising transporter polypeptides, each transporter polypeptide comprising an amino acid sequence that has at least 85% sequence identity with an amino acid segment obtained from the native monomeric protein. In specific embodiments are heteromultimers comprising transporter polypeptides, each transporter polypeptide comprising an amino acid sequence that has at least 90% sequence identity with an amino acid segment obtained from the native monomeric protein. In certain embodiments are heteromultimers comprising transporter polypeptides, each transporter polypeptide comprising an amino acid sequence that has at least 95% sequence identity with an amino acid segment obtained from the native monomeric protein. In some embodiments are heteromultimers comprising transporter polypeptides, each transporter polypeptide comprising an amino acid sequence that has at least 99% sequence identity with an amino acid segment obtained from the native monomeric protein. In some embodiments, the native monomeric protein is a mammalian albumin or derivative or analog thereof. In certain embodiments, the native monomeric protein is human serum albumin. In certain embodiments, we present polypeptide segments derived from transferrin that self-assemble to form a heteromultimer that exhibits native transferrin like structural and functional characteristics. In certain embodiments, we present polypeptide segments derived from annexin that self-assemble to form a heteromultimer that exhibits native annexin like structural and functional characteristics. These heteromultimers are referred to as being quasi-native.

### Transporter polypeptide

As used herein, the term "transporter polypeptide" or "transporter polypeptide" or "transporter peptide" or "transporter" refers to a polypeptide, such that said transporter polypeptide is capable of forming heteromultimeric proteins with other such transporter polypeptides in solution, and wherein said heteromultimeric proteins have a quasi-native structure of a monomeric protein from which at least one transporter polypeptide is derived. In certain embodiments of the heteromultimers described herein, all transporter polypeptides are derived from the same albumin or alloalbumin protein. In certain other embodiments, the heteromultimers are formed by transporter polypeptides derived from various albumin and alloalbumin proteins. In certain embodiments of the heteromultimers described herein, the transporter polypeptides are derived from transferrin. In certain embodiments of the heteromultimers described herein, all transporter polypeptides are derived from annexin proteins. In certain embodiments, the heteromultimers are formed by transporter polypeptides derived from the same annexin protein. In some embodiments, the heteromultimers are formed by transporter polypeptides derived from different annexin proteins. In an embodiment, the heteromultimers are formed by transporter polypeptides derived from annexin A2.

In certain embodiments, transporter polypeptides are segments of a monomeric whole protein, wherein said segments are capable of assembling to form a heteromultimer such that said heteromultimer forms a quasi-native structure similar to the native monomeric whole protein. In an embodiment, the transporter polypeptides are segments from a beta-barrel protein. In certain embodiments provided are, heteromultimers comprising transporter polypeptides that assemble to form a quasi-native structure similar to a native monomeric protein, each transporter polypeptide comprising an amino acid sequence with at least 50% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 60% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 75% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 80% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 90% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 95% identity to a segment of the native monomeric protein. In certain embodiments provided are, heteromultimers comprising transporter polypeptides that assemble to form a quasi-native structure similar to a native monomeric protein, each transporter polypeptide comprising an amino acid sequence with at least 99% identity to a segment of the native monomeric protein. In an embodiment, the native monomeric protein is a beta-propeller protein. In some embodiments, the native monomeric protein is a helical bundle protein. In embodiments, the trasporter polypeptides are generated from for instance, but not restricted to proteins comprising a zinc finger motif, a helix-turn-helix motif or a beta-hairpin motif. In some embodiments, the transporter polypeptides comprise segments obtained from non-immunogenic proteins that are structurally stable, and have favorable biological properties.

Transporter polypeptides are derived from a protein by segmentation of the protein at a segmentation site. The segmentation site is designed on the basis of the following two decision criteria.

First, the segmentation site is selected such that it resides on a loop within the protein that has a high relative SASA and limited contact with the rest of the protein structure. In one embodiment, the segmentation site resides in a loop that is flexible and accessible. In another embodiment, the segmentation site resides in a loop that is less likely to contribute to the protein core stability and thus impact stability of the assembled quasi-native protein structure relative to the intact protein structure.

Second, the segmentation site is selected such that it results in a complementary interface between the transporter polypeptides, wherein the interface is apolar, extensive and interdigitated. The apolarity of the interface relates to the number of apolar amino acids in the interface. As is known in the art, amino acids can be broadly classified as polar and apolar residues. While polar residues interact favourably with water in the solvent environment, apolar residues do not make energetically favourable contact with water. For this reason, the apolar residues in a protein tend to cluster with other apolar residues and exclude water molecules from their local environment and this is energetically favourable. Such interactions involving apolar residues is also referred to as hydrophobic contact.

The buried surface area at the interface is derived from the loss in the molecular or solvent accessible surface between the unassociated and associated states of the two interacting polypeptides. In one embodiment, the interface buried area is between about 6000 Å² and about 2000 Å². In another embodiment, the interface buried area is between about 4000 Å² and 2000 Å². In another embodiment, the interface buried area is between about 3000 Å² and 2000 Å². In one embodiment, the apolar contribution to the interface buried area is greater than 50% of the net buried surface area at the interface.

With respect to the interface and its interdigitation, an important factor driving any protein-protein interaction and complex formation is the contact of surface residues at the interface formed between the two contacting proteins. Physico-chemical and structural complementarity of the two contacting protein surfaces in the associated form is a hallmark of favourable and stable complex formation. The physico-chemical complementarity is defined by the preferable interactions and could comprise hydrogen bonds, charge based salt-bridges, various types of electrostatic interactions, hydrophobic and van der Waals contacts across interacting protein surface. The protein surface at the 3-dimensional level is defined by protuberances and cavities formed due to the differences in size and orientation of various amino acids that constitute the surface region. In an favourable protein-protein interface, the protuberance and cavities from the two interacting protein surfaces interdigitate to result in structural complementarity. The larger and more extensive the interface between the interacting proteins, the protein-protein contact is potentially more stable.

In one embodiment, the interface between the two transported polypeptides is rich in hotspot residues, i.e. individual residues or group of residues involved in a well-connected network of interactions. The interactions could be based on typical interactions observed in proteins such as hydrogen bonds, charged residue interactions such as salt-bridges, van der Waals contacts which show strong structural complementarity and hydrophobic residue clusters. This maximizes the interface stability and prevents the complex from dissociating.

In one embodiment, the segmentation site is selected to retain a natural disulfide bond present in the protein from which the transporter polypeptides are derived, across the segmented sections so as to covalently connect the two segments. That was meant to further increase the stability of the heterodimerizing quasi-native protein-like structure that self assembles from the two derived segments following segmentation. In this embodiment, loop regions in the protein sequence are chosen that satisfy the disulfide criteria described here i.e. have a disulfide very close to the segmentation site in the loop region. Besides the function of covalently crosslinking the two chains, this disulfide could also serve as a surrogate for the loop being open.

In another aspect described herein, a disulphide link across the two segments is engineered by introducing individual amino acid to cysteine mutations at selected positions on each of the two derived segments.

In another embodiment, we engineer amino acids at the interface of the two segments to further increase or improve the hotspots at the interface or enhance the structural and physico-chemical complementarity between the two segments in the assembled quasi-native albumin structure.

### Albumin

As used herein, "albumin" refers collectively to albumin protein or amino acid sequence, or an albumin segment or variant, having one or more functional activities (e.g., biological activities) of albumin. In particular "albumin" refers to human albumin or segments thereof (see for example, EP 201 239, EP 322 094 WO 97/24445, WO95/23857) especially the mature form of human albumin as shown in FIG. 1, or albumin from other vertebrates, or segments thereof, or analogs or variants of these molecules or fragments thereof. Inc ertain embodiments, albumin refers to a truncated version of albumin.

The term "quasi-native albumin" refers to a heteromultimer molecule that has structure and/or function similar to the whole albumin, and wherein said heteromultimer molecule is formed by the assembly of two or more monomeric polypeptides designed based on the sequence of the whole albumin. In certain embodiments, the polypeptides of interest here comprise "segments" that preferentially associate as heteromultimeric pairs to form a quasi-native protein. In some embodiment this quas-native protein is quasi-native albumin. In some embodiments, the quasi-native albumin has 90% of the activity of the whole albumin. In some embodiments, the quasi-native albumin has 75% of the activity of whole-albumin. In an embodiment, the quasi-native albumin has 50% of the activity of whole albumin. In some embodiments, the quasi-native albumin has 50-75% of the activity of whole albumin. In an embodiment, quasi-native albumin has 80% of the activity of whole albumin. In some embodiments, the quasi-native albumin retains about 90% of the structural characteristics of the whole albumin as determined by structural modeling. In some embodiments, the quasi-native albumin retains about 80% of the structural characteristics of the whole albumin as determined by structural modeling. In some embodiments, the quasi-native albumin retains about 70% of the structural characteristics of the whole albumin as determined by structural modeling. In some embodiments, the quasi-native albumin retains about 50% of the structural characteristics of the whole albumin as determined by structural modeling. In some embodiments, the quasi-native albumin retains about 50-75% of the structural characteristics of the whole albumin as determined by structural modeling.

The terms, human serum albumin (HSA) and human albumin (HA) are used interchangeably herein. The terms, "albumin and serum albumin" are broader, and encompass human serum albumin (and fragments and variants thereof) as well as albumin from other species (and fragments and variants thereof).

In certain embodiments, each albumin-based monomer of the heteromultimeric proteins described herein is based on a variant of normal HA. Each cargo polypeptide portion of the heteromultimeric proteins of the invention may also be variants of the Therapeutic proteins as described herein. The term "variants" includes insertions, deletions and substitutions, either conservative or non conservative, where such changes do not substantially alter one or more of the oncotic, useful ligand-binding and non-immunogenic properties of albumin, or the active site, or active domain which confers the therapeutic activities of the Therapeutic proteins. In certain embodiments variants also could mean alternate heteromultimer species derived by segmenting the albumin at alternate locations in its primary sequence.

In certain embodiments, the heteromultimeric proteins described herein include naturally occurring polymorphic variants of human albumin and fragments of human albumin, for example those fragments disclosed in EP 322 094 (namely HA (Pn), where n is 369 to 419).

In certain embodiments, the albumin is derived from any vertebrate, especially any mammal that includes but is not limited to human, cow, sheep, rat, mouse, rabbit, horse, dog or pig. In certain embodiments, the albumin is derived from non-mammalian albumins including, but are not limited to hen and salmon.

The sequence of human albumin is as shown, in the preprotein form with the N-terminal signaling residues MKWVTFISLLFLFSSAYSRGVFRR:

SEQ ID NO: 1














### Alloalbumin

An alloalbumin is a genetic variant of albumin. In certain embodiments the alloalbumin is human alloalbumin (HAA). Alloalbumins that differ in electrophoretic mobility from albumin have been identified through population genetics surveys in the course of clinical electrophoresis, or in blood donor surveys. As markers of mutation and migration, alloalbumins are of interest to geneticists, biochemists, and anthropologists, but most of these alloalbumin are not associated with disease (Minchioti et al. Human Mutations 29(8), 1007-1016(2008)).

**Table 1: List of substitutions comprised by various alloalbumins as compared to HA of SEQ ID NO: 1. Thermostability, half-life information and other HAAs are provided in Krogh-hansen et al. Biochim Biophys Acta 1747, 81-88(2005); and WO2011051489.**

| Mutation | Thermostability (C) (positive=stabilizing, negative=destabilizing) | Effect on half-life (% change) |
|---|---|---|
| H3Y | N/A | N/A |
| H3Q | N/A | N/A |
| Q32Stop | N/A | N/A |
| E60K | N/A | N/A |
| D63N | 6.07 | N/A |
| L66P | N/A | N/A |
| E82K | 2.03 | N/A |
| R114G | N/A | N/A |
| R114Stop | N/A | N/A |
| E119K | N/A | N/A |
| V122E | 0.57 | N/A |
| H128R | N/A | N/A |
| Y140C | N/A | N/A |
| A175Stop | N/A | N/A |
| C177F | -1.59 | N/A |
| R218H | N/A | N/A |
| R218P | N/A | N/A |
| K225Q* | -4.86 | N/A |
| K240E | N/A | N/A |
| E244Stop | N/A | N/A |
| Q268R | N/A | N/A |
| D269G | 3.67 | N/A |
| K276N | 4.87 | N/A |
| K313N | -7.16 | N/A |
| D314G | -0.38 | N/A |
| D314V | N/A | N/A |
| N318K | N/A | N/A |
| A320T, & -1R | N/A | 6.16 |
| E321K | 1.42 | N/A |
| E333K | -2.56 | N/A |
| E354K | N/A | N/A |
| E358K | N/A | N/A |
| K359K | -6.56 | N/A |
| D365H | 0.89 | N/A |
| D365V | N/A | N/A |
| E368G | N/A | N/A |
| K372E | N/A | N/A |
| D375N | N/A | N/A |
| D375H | -0.09 | N/A |
| E376K | N/A | N/A |
| E376Q | N/A | N/A |
| E382K | N/A | N/A |
| Q385Stop | N/A | N/A |
| Y401Stop | N/A | N/A |
| R410C | N/A | N/A |
| E479K | N/A | N/A |
| D494N | N/A | 0.84 |
| E501K | 0.13 | N/A |
| E505K | 1.87 | N/A |
| I513N | N/A | N/A |
| V533M | N/A | N/A |
| K536E | N/A | N/A |
| K541E | 6.12 | N/A |
| D550G | N/A | N/A |
| D550A | N/A | N/A |
| K560E | 0.70 | N/A |
| D563N | 4.17 | N/A |
| E565K | N/A | N/A |
| E570K | -6.53 | N/A |
| K573E | 2.08 | 2.7 |
| K574N | N/A | N/A |
| L575insertion(TCCC KSSCLRLITSHLKASQ PTMRIRERK) | -5.30 | N/A |
| Frameshift after 567; Stop at 582 | N/A | -5.7% |
| Frameshift after 572; Stop at 578 | N/A | -8.9 % |

### Annexin:

As used herein, "annexin" refers to a group of cellular proteins found in eukaryotic organisms. Annexin is also known as lipocortin. As used herein "annexin" may refer to any annexin protein, or to specific annexin proteins such as "annexin A1," "annexin A2," and "annexin A5." Annexins are characterized by their calcium dependent ability to bind negatively charged phospholipids (i.e. membrane walls). Annexins are characterized by a repeat protein scaffold limited to 30-50 kDa in size with fairly ubiquitous tissue distribution. The basic structure of an annexin is composed of two domains: a structurally conserved C terminal "core" region and a divergent N terminal domain. The core region binds the phospholipid cellular membrane in a Ca²⁺ dependent manner. The N terminal region binds cytoplasmic proteins. Annexins are important in various cellular and physiological processes and provide a membrane scaffold. The C terminal core is composed of four annexin repeats. Annexin is characterized by its flexible repeat-like nature that influences its intrinsic membrane-sensing abilities. For instance, the affinity towards specific biomembranes can be controlled by the number of repeats. With the characteristic phospholipid sensing, annexin can be useful to sense/target intestinal junctions for drug delivery. Another potential application for an annexin is targeting intestinal tight junctions and the Zonula Occludens region (ZO-1), which is known to be particularly difficult to traverse for larger protein therapeutics, significantly impairing drug absorption.

The term "quasi-native annexin" refers to a heteromultimer molecule that has structure and/or function similar to the whole annexin, and wherein said heteromultimer molecule is formed by the assembly of two or more monomeric polypeptides designed based on the sequence of the whole annexin. In certain embodiments, the monomeric polypeptides are "segments" that preferentially associate as heteromultimeric pairs to form a quasi-native protein. In some embodiments, the quasi- annexin has 90% of the activity of the whole annexin. In some embodiments, the quasi- annexin has 75% of the activity of whole- annexin. In an embodiment, the quasi- annexin has 50% of the activity of whole annexin. In some embodiments, the quasi- annexin has 50-75% of the activity of whole annexin. In an embodiment, quasi- annexin has 80% of the activity of whole annexin. In some embodiments, the quasi- annexin has 90% of the structure of whole annexin as determined by molecular modeling. In some embodiments, the quasi- annexin has 80% of the structure of whole annexin as determined by molecular modeling. In some embodiments, the quasi- annexin has 70% of the structure of whole annexin as determined by molecular modeling. In some embodiments, the quasi- annexin has 50% of the structure of whole annexin as determined by molecular modeling. In some embodiments, the quasi-annexin has 50%-75% of the structure of whole annexin as determined by molecular modeling.

The sequence of Human wild-type Annexin A2 is as shown:

SEQ ID NO: 14







### Transferrin:

Transferrins are monomeric proteins of about 76 kDa molecular weight present in all vertebrates and function as a iron-binding and transporting protein. Recombinant human transferrin and its fusions is being considered for the management of various diseases including thalassemia, atransferrinemia, age related macular degeneration, type 2 diabetes, during stem cell transplantation and in the treatment of acute infectious disease caused by the anthrax bacteria. Transferrin is stable in the gastrointestinal environment and a number of studies have shown that intact protein-transferrin conjugates can be orally delivered and remain bioactive.

The term "quasi-transferrin" refers to a heteromultimer molecule that has structure and/or function similar to the whole transferrin, and wherein said heteromultimer molecule is formed by the assembly of two or more monomeric polypeptides designed based on the sequence of the whole transferrin. In certain embodiments, the monomeric polypeptides are "segments" that preferentially associate as heteromultimeric pairs to form a quasi-native protein. In some embodiments, the quasi- transferrin has 90% of the activity of the whole transferrin. In some embodiments, the quasi- transferrin has 75% of the activity of whole-transferrin. In an embodiment, the quasi- transferrin has 50% of the activity of whole transferrin. In some embodiments, the quasi- transferrin has 50-75% of the activity of whole transferrin. In an embodiment, quasi- transferrin has 80% of the activity of whole transferrin. In some embodiments, the quasi- transferrin has 90% of the structure of whole transferrin as determined by molecular modeling. In some embodiments, the quasi-transferrin has 80% of the structure of whole transferrin as determined by molecular modeling. In some embodiments, the quasi- transferrin has 70% of the structure of whole transferrin as determined by molecular modeling. In some embodiments, the quasi-transferrin has 50% of the structure of whole transferrin as determined by molecular modeling. In some embodiments, the quasi- transferrin has 50%-75% of the structure of whole transferrin as determined by molecular modeling.

The sequence of wildtype Human Transferrin is as shown:

SEQ ID NO: 19
MRLAVGALLV CAVLGLCLAV PDKTVRWCAV SEHEATKCQS FRDHMKSVIP
SDGPSVACVK KASYLDCIRA IAANEADAVT LDAGLVYDAY LAPNNLKPVV
AEFYGSKEDP QTFYYAVAVV KKDSGFQMNQ LRGKKSCHTG LGRSAGWNIP
IGLLYCDLPE PRKPLEKAVA NFFSGSCAPC ADGTDFPQLC QLCPGCGCST
LNQYFGYSGA FKCLKDGAGD VAFVKHSTIF ENLANKADRD QYELLCLDNT
RKPVDEYKDC HLAQVPSHTV VARSMGGKED LIWELLNQAQ EHFGKDKSKE
FQLFSSPHGK DLLFKDSAHG FLKVPPRMDA KMYLGYEYVT AIRNLREGTC
PEAPTDECKP VKWCALSHHE RLKCDEWSVN SVGKIECVSA ETTEDCIAKI
MNGEADAMSL DGGFVYIAGK CGLVPVLAEN YNKSDNCEDT PEAGYFAVAV
VKKSASDLTW DNLKGKKSCH TAVGRTAGWN IPMGLLYNKI NHCRFDEFFS
EGCAPGSKKD SSLCKLCMGS GLNLCEPNNK EGYYGYTGAF RCLVEKGDVA
FVKHQTVPQN TGGKNPDPWA KNLNEKDYEL LCLDGTRKPV EEYANCHLAR
APNHAVVTRK DKEACVHKIL RQQQHLFGSN VTDCSGNFCL FRSETKDLLF
RDDTVCLAKL HDRNTYEKYL GEEYVKAVGN LRKCSTSSLL EACTFRRP

### Cargo molecule:

A heteromultimer described herein comprises polypeptides that comprise at least one cargo molecule, and at least one transporter polypeptide, said cargo molecule and transporter polypeptide associated with one another, by means inclusive of, but not restricted to genetic fusion or chemical conjugation. In some embodiments, the cargo polypeptide is fused to either the N or C terminus of the transporter polypeptide. In some embodiments, the cargo polypeptides are fused to both the N and C terminus of the transporter polypeptide. In some embodiment the cargo polypeptide is conjugated to Cysteine 37 position of the transporter polypeptide derived by segmenting human albumin. In certain embodiments, at least one cargo molecule is a therapeutic agent. In certain agents, the cargo molecule is a toxin. In certain embodiments, the cargo molecule is an antigen, or analogs thereof. In an embodiment, the cargo molecule is a natural product, analog, or prodrug thereof. In certain embodiments, the cargo molecule is a therapeutic agent such as a cytotoxin, e.g., a cytostatic or cytocidal agent, a therapeutic agent or a radioactive metal ion, e.g., alpha-emitters such as, for example, 213Bi. A cytotoxin or cytotoxic agent includes any agent that is detrimental to cells. Examples include paclitaxol, cytochalasin B, gramicidin D, ethidium bromide, emetine, mitomycin, etoposide, tenoposide, vincristine, vinblastine, colchicin, doxorubicin, daunorubicin, dihydroxy anthracin dione, mitoxantrone, mithramycin, actinomycin D, 1-dehydrotestosterone, glucocorticoids, procaine, tetracaine, lidocaine, propranolol, and puromycin and analogs or homologs thereof. Therapeutic agents include, but are not limited to, antimetabolites (e.g., methotrexate, 6mercaptopurine, 6thioguanine, cytarabine, 5-fluorouracil decarbazine), alkylating agents (e.g., mechlorethamine, thioepa chlorambucil, melphalan, carmustine (BSNU) and lomustine (CCNU), cyclothosphamide, busulfan, dibromomannitol, streptozotocin, mitomycin C, and cis-dichlorodiamine platinum (II) (DDP) cisplatin), anthracyclines (e.g., daunorubicin (formerly daunomycin) and doxorubicin), antibiotics (e.g., dactinomycin (formerly actinomycin), bleomycin, mithramycin, and anthramycin (AMC)), and anti-mitotic agents (e.g., vincristine and vinblastine).

In certain embodiment, the cargo molecule is a biomolecule. In an embodiment, the cargo molecule is a natural or synthetic nucleic acid. In some embodiments, at least one cargo molecule is one or more of a DNA, PNA, and/or RNA oligomer. In certain embodiments, a heteromultimer described herein comprises monomeric proteins that comprise at least one cargo polypeptide, or fragments or variants thereof, and at least one transporter polypeptide, said cargo polypeptide and transporter polypeptide associated with one another, by means inclusive of, but not restricted to genetic fusion or chemical conjugation

As used herein, "Cargo polypeptide" refers to proteins, polypeptides, antibodies, peptides or fragments or variants thereof, having one or more therapeutic and/or biological activities. Cargo polypeptides encompassed by the invention include but are not limited to, proteins, polypeptides, peptides, antibodies, substrates or ligands to therapeutically relevant target proteins and biologics. (The terms peptides, proteins, and polypeptides are used interchangeably herein.) Specifically the term "Cargo polypeptide" encompasses antibodies and fragments and variants thereof. Thus a heteromultimer described herein may contain at least a fragment or variant of a cargo polypeptide, and/or at least a fragment or variant of an antibody. Additionally, in certain embodiments, the term "Cargo polypeptide" refers to the endogenous or naturally occurring correlate of a cargo polypeptide.

As a non-limiting example, a "Cargo biomolecule" is a biomolecule such as but not restricted to a protein, DNA, or RNA that is useful to treat, prevent or ameliorate a disease, condition or disorder. As a non-limiting example, a "Cargo polypeptide" may be one that binds specifically to a particular cell type (normal (e.g., lymphocytes) or abnormal e.g., (cancer cells)) and therefore may be used to target a compound (drug, or cytotoxic agent) to that cell type specifically.

In another non-limiting example, a "Cargo molecule" is a molecule that has a biological, activity, and in particular, a biological activity that is useful for treating preventing or ameliorating a disease. A non-inclusive list of biological activities that may be possessed by a Cargo molecule, for instance a Cargo polypeptide includes, enhancing the immune response, promoting angiogenesis, inhibiting angiogenesis, regulating hematopoietic functions, stimulating nerve growth, enhancing an immune response, inhibiting an immune response, or any one or more of the biological activities described herein.

Cargo polypeptides corresponding to a cargo polypeptide portion of a heteromultimer protein described herein, such as cell surface and secretory proteins, are often modified, by the attachment of one or more oligosaccharide groups. The modification, referred to as glycosylation, can dramatically affect the physical properties of proteins and can be important in protein stability, secretion, and localization. Glycosylation occurs at specific locations along the polypeptide backbone. There are usually two major types of glycosylation: glycosylation characterized by O-linked oligosaccharides, which are attached to serine or threonine residues; and glycosylation characterized by N-linked oligosaccharides, which are attached to asparagine residues in an Asn-X-Ser/Thr sequence, where X can be any amino acid except proline. N-acetylneuramic acid (also known as sialic acid) is usually the terminal residue of both N-linked and Blinked oligosaccharides. Variables such as protein structure and cell type influence the number and nature of the carbohydrate units within the chains at different glycosylation sites. Glycosylation isomers are also common at the same site within a given cell type.

Table 2 provides a non-exhaustive list of Cargo polypeptides that correspond to a Cargo polypeptide portion of a heteromultimer described herein. The "Cargo Polypeptide" column discloses Cargo polypeptide molecules followed by parentheses containing scientific and brand names that comprise, or alternatively consist of, that Cargo polypeptide molecule or a fragment or variant thereof. In an embodiment the cargo molecule is a molecule that binds to a protein disclosed in the "Cargo polypeptide" column, or in Zhu et al. (Nucleic Acids Res. 38(1), D787-D791 (2009)); Wishart et al. (Nucleic Acids Res 36, D901-D906 (2008)); Ahmed et al. (Nucleic Acids Res 39, D960-D967 (2011)), or a protein that belongs in the class of therapeutic target molecules.

"Cargo polypeptide" as used herein may refer either to an individual Cargo polypeptide molecule (as defined by the amino acid sequence obtainable from the CAS and Genbank accession numbers), or to the entire group of Cargo polypeptide associated with a given Cargo polypeptide molecule disclosed in this column, or a Cargo polypeptide that binds to a polypeptide molecule disclosed in this column.

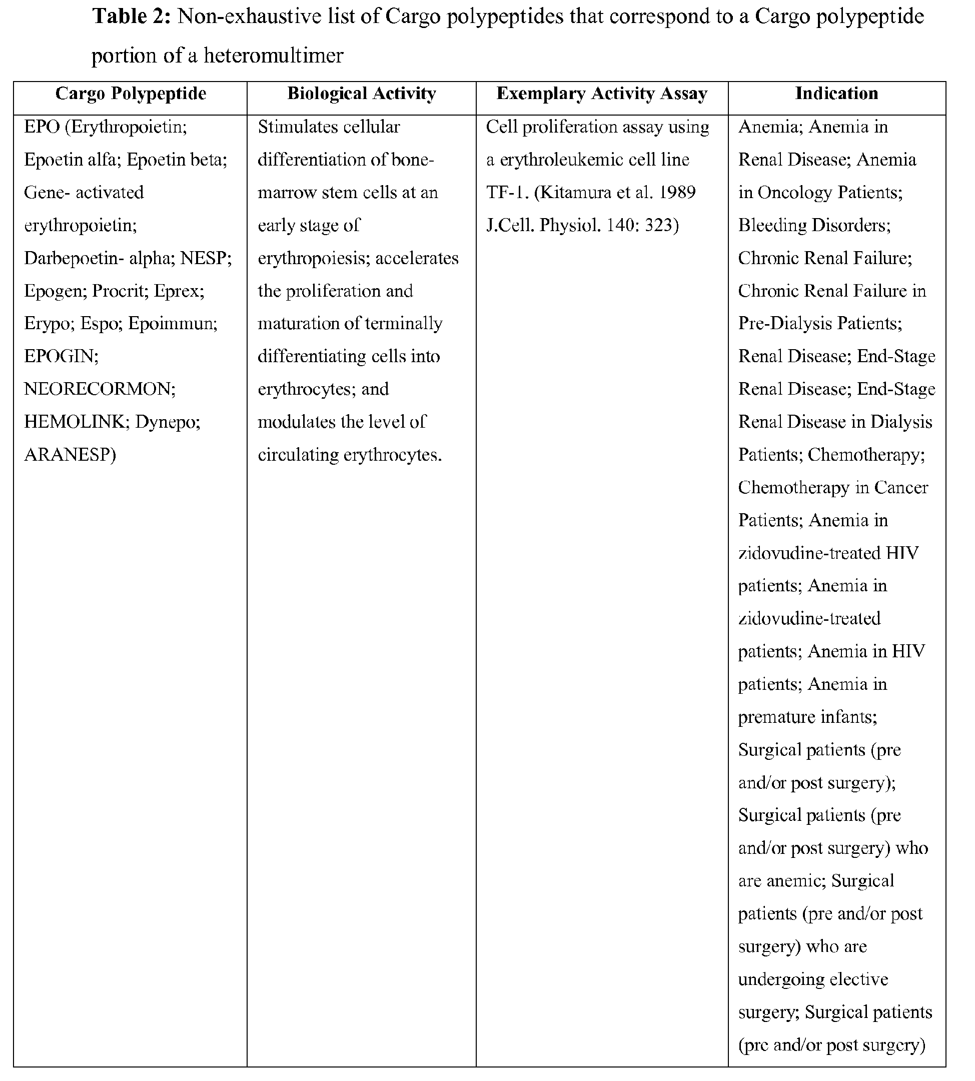

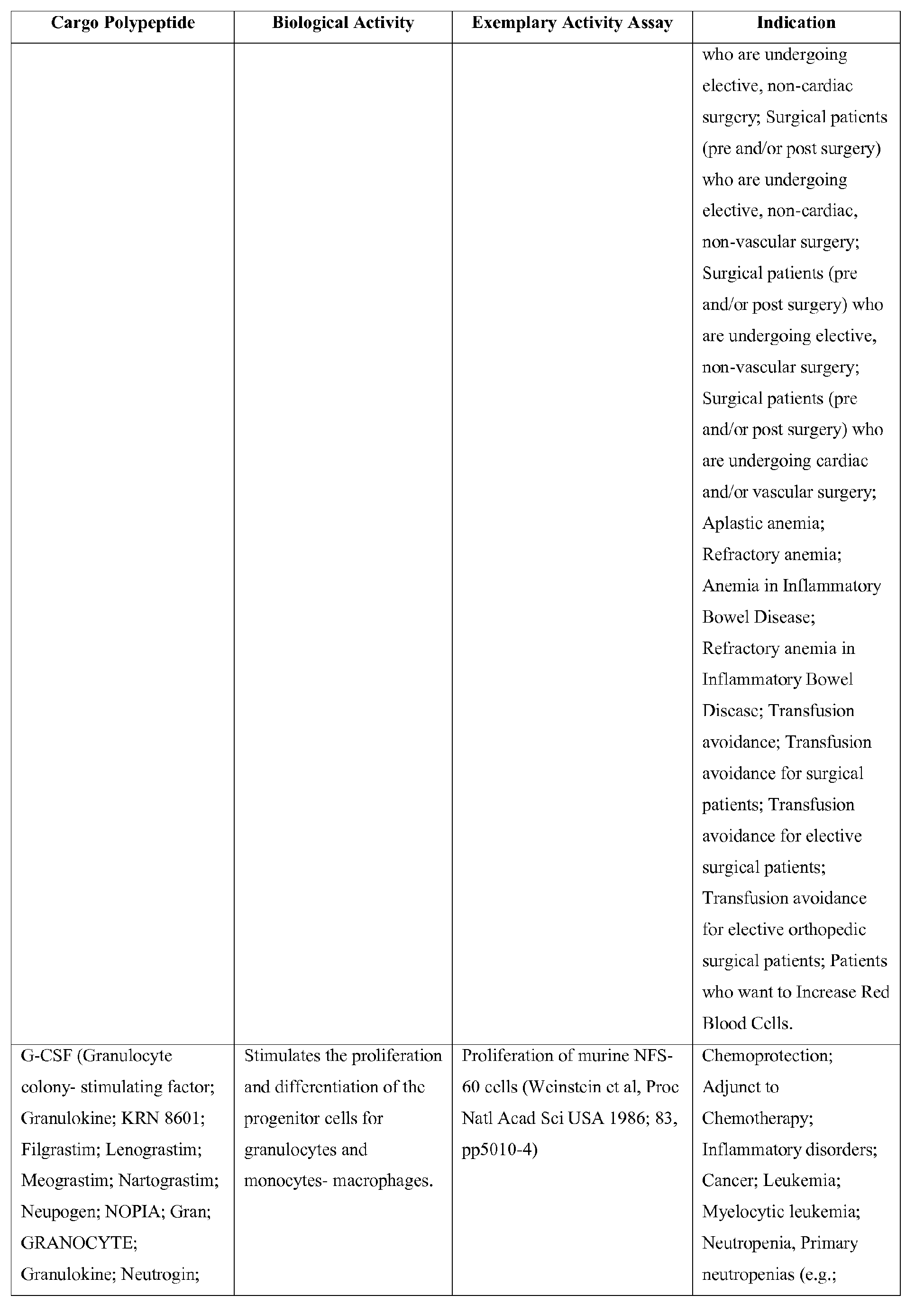

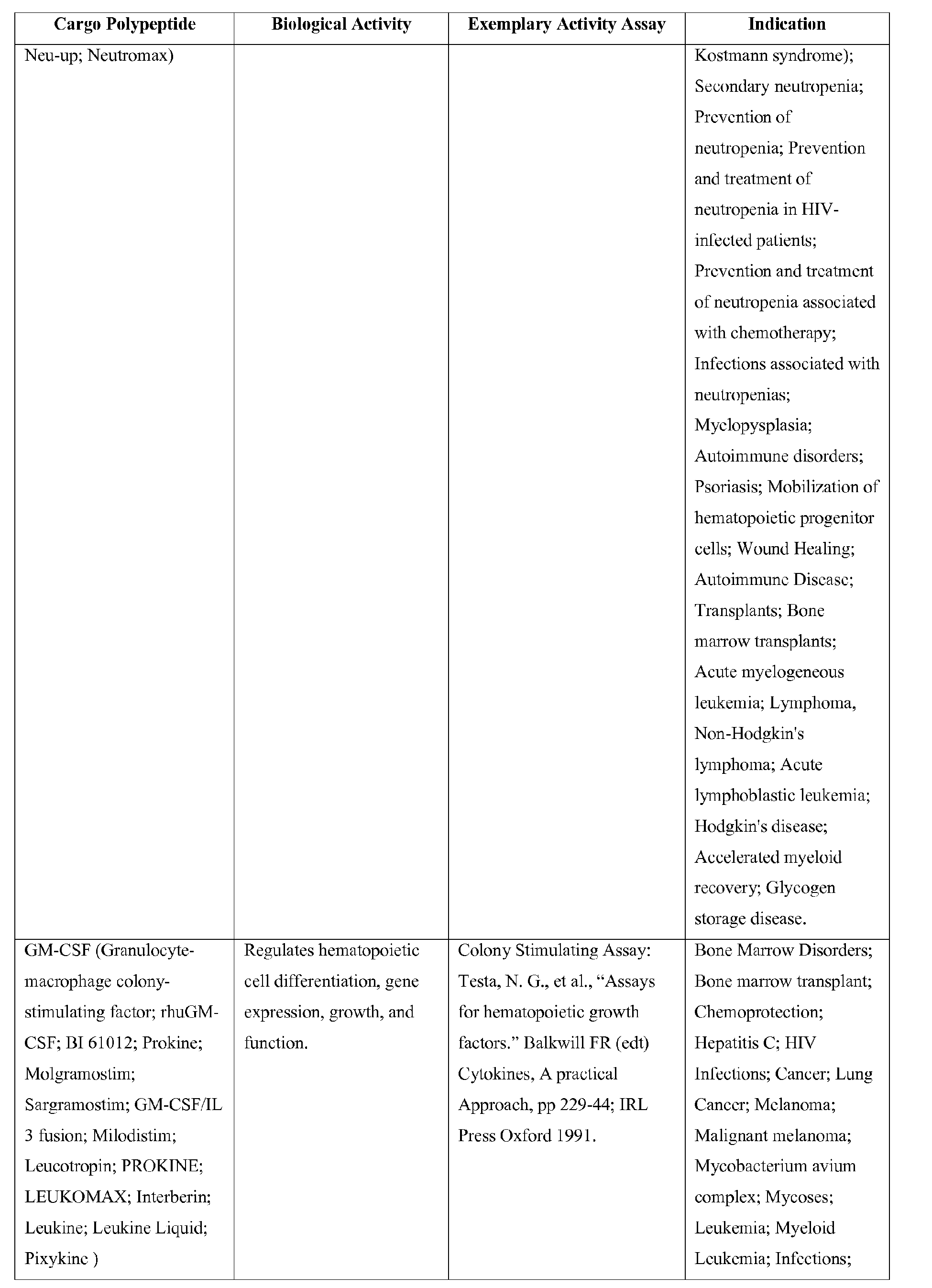

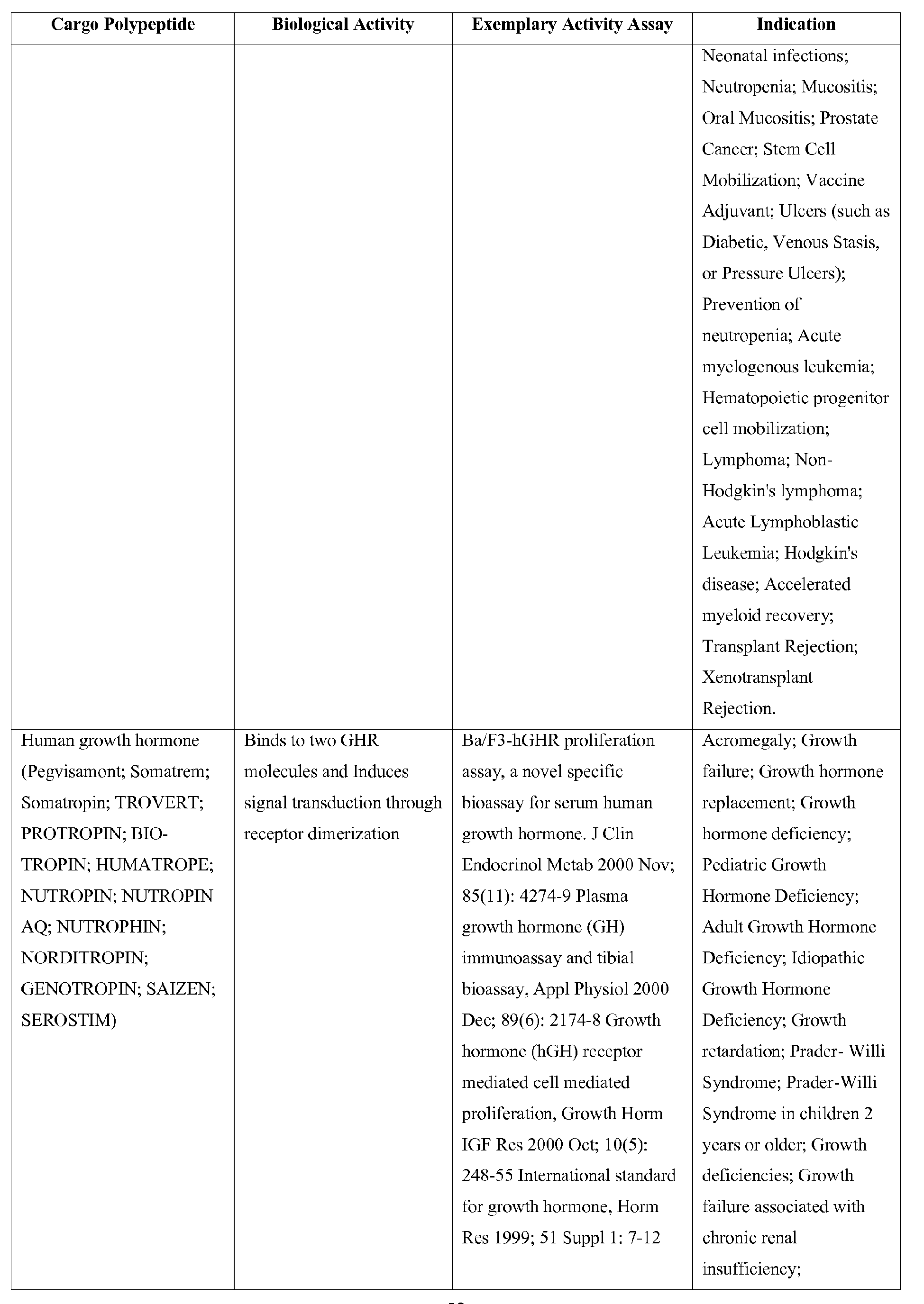

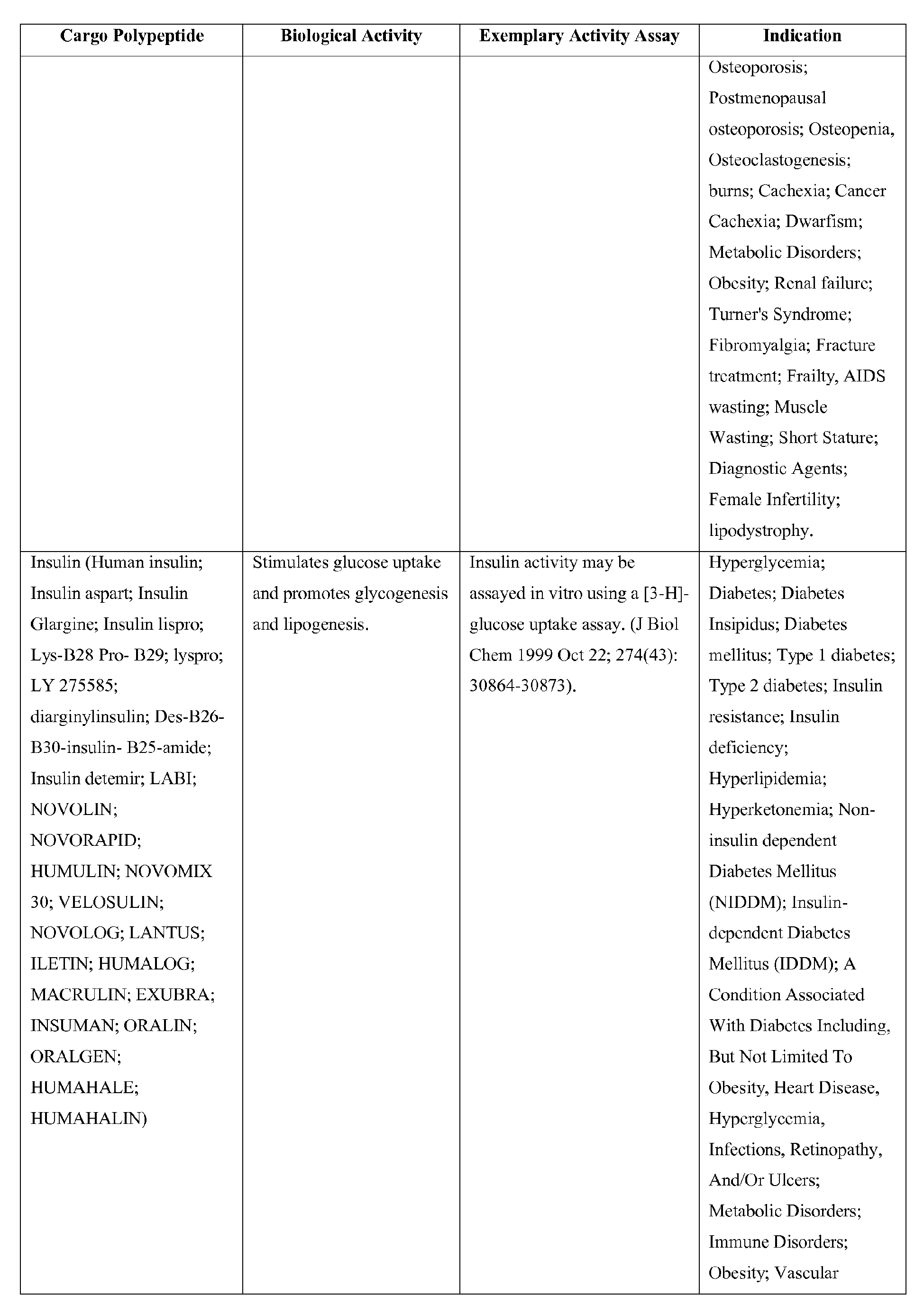

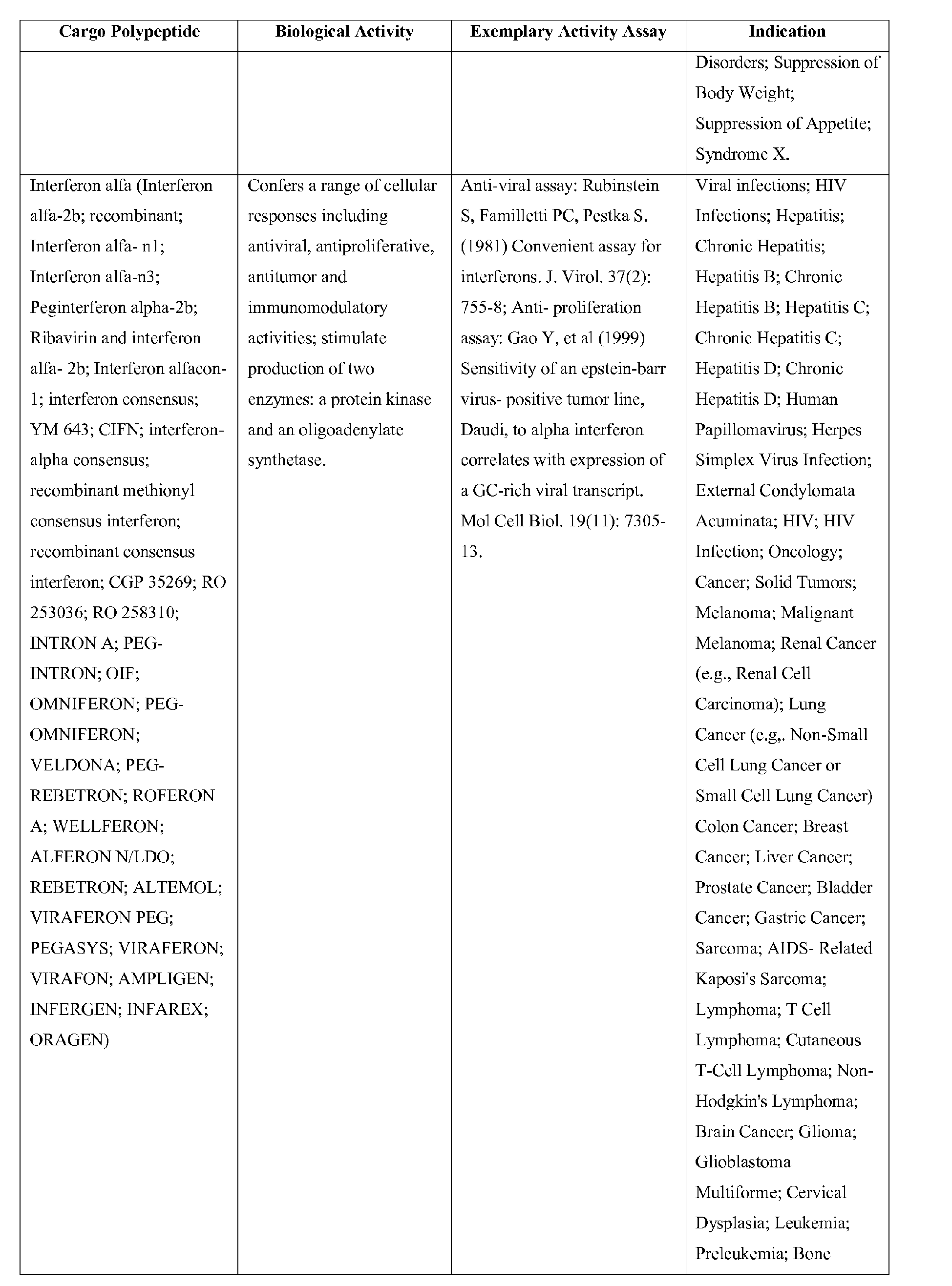

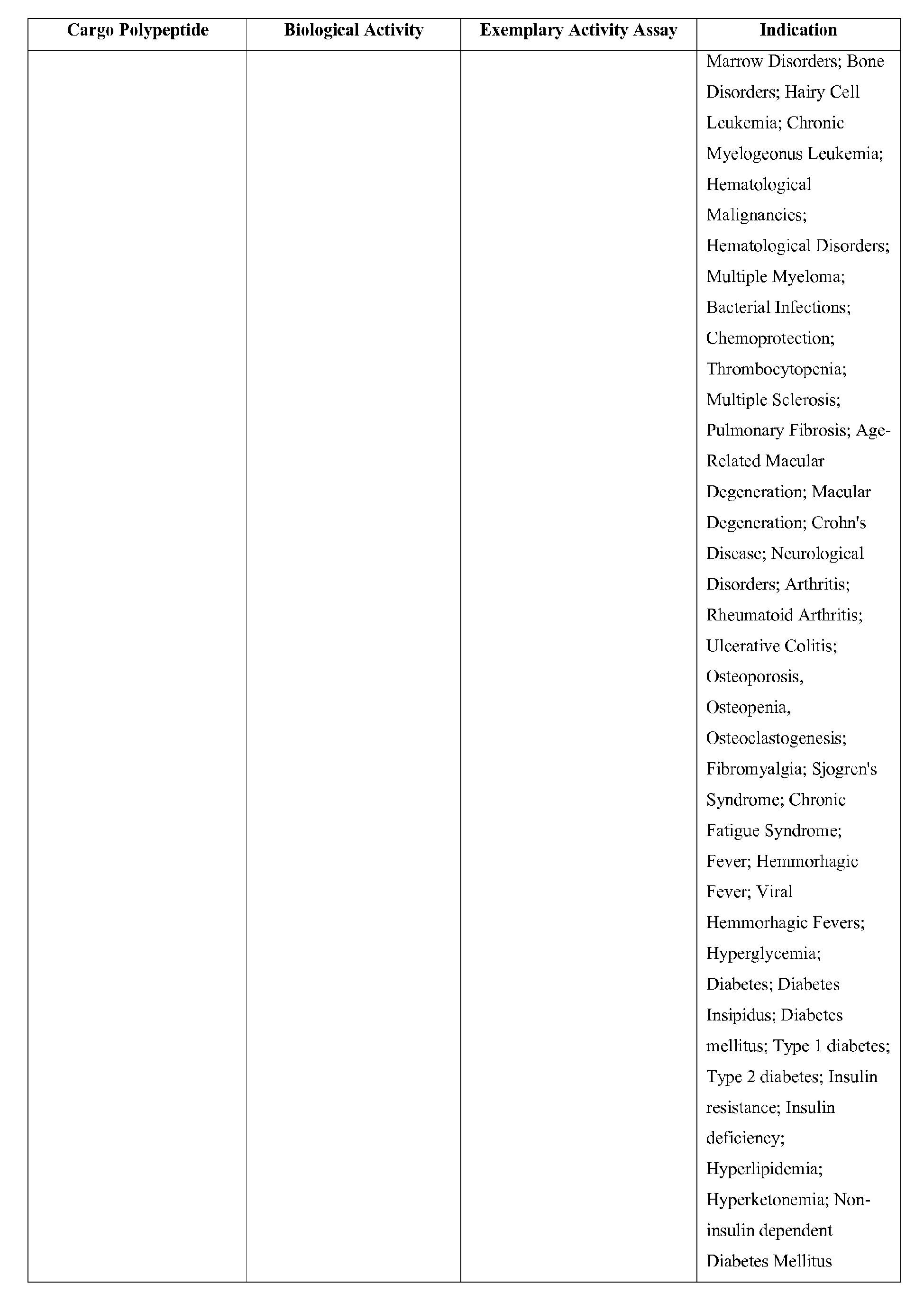

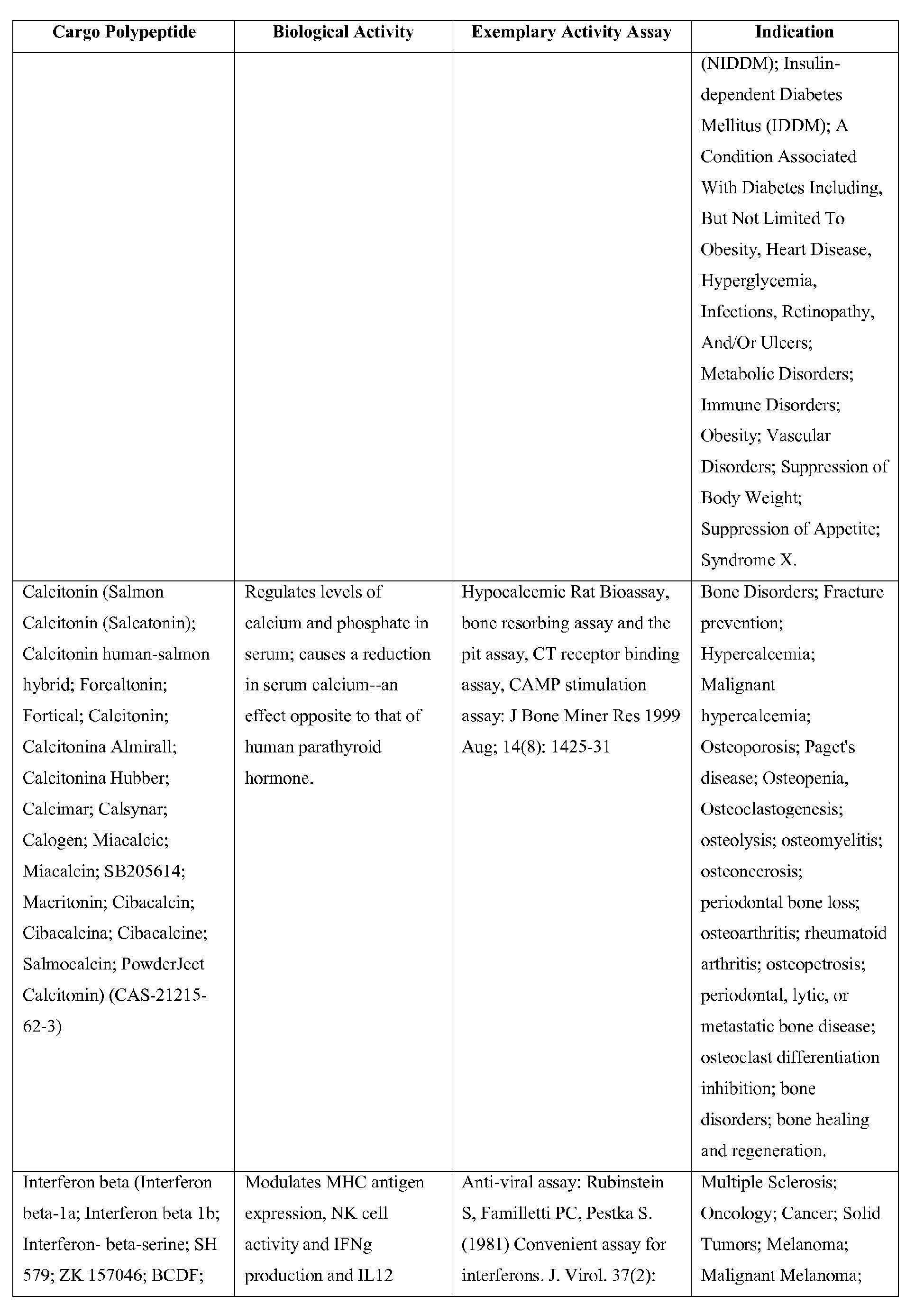

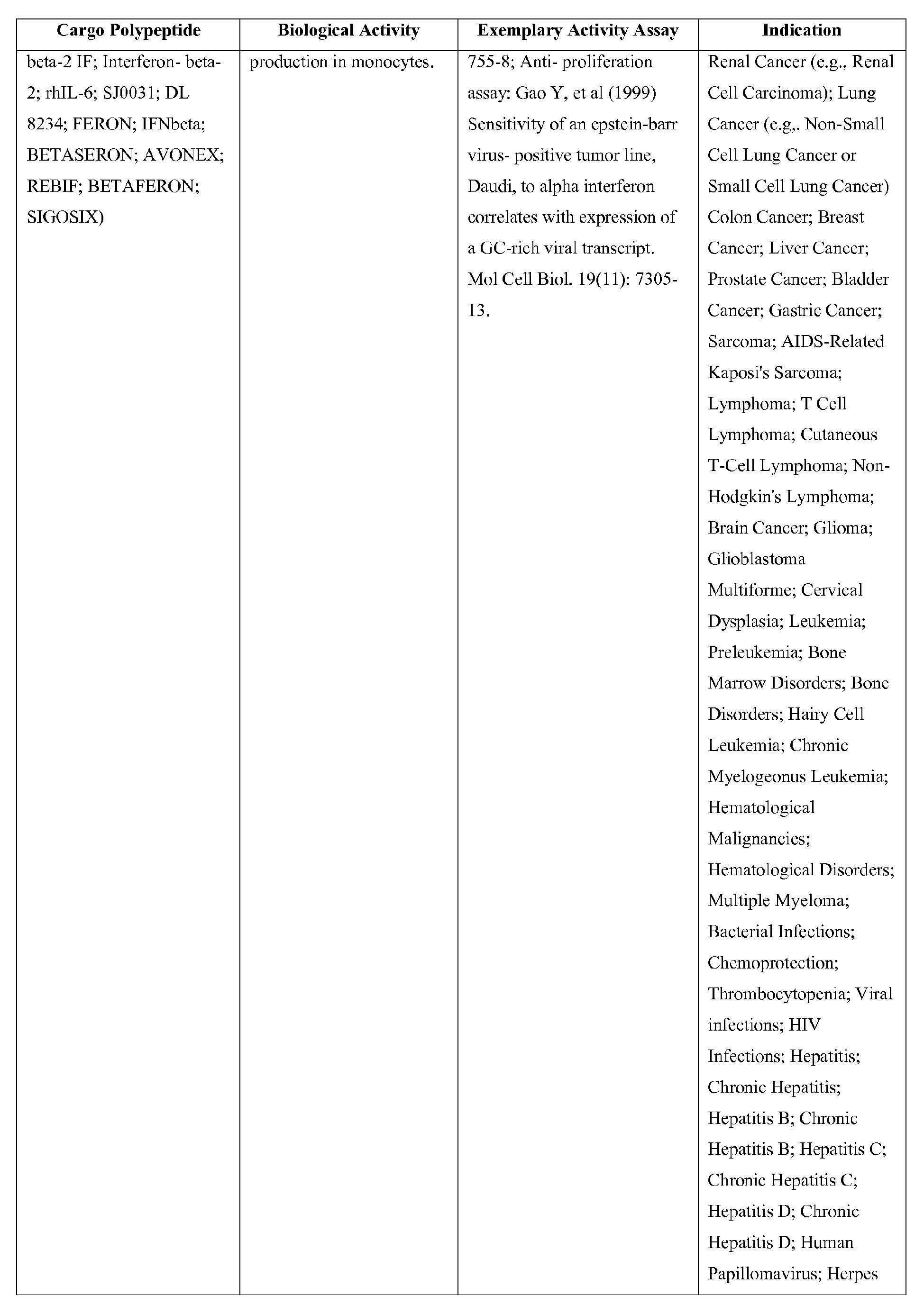

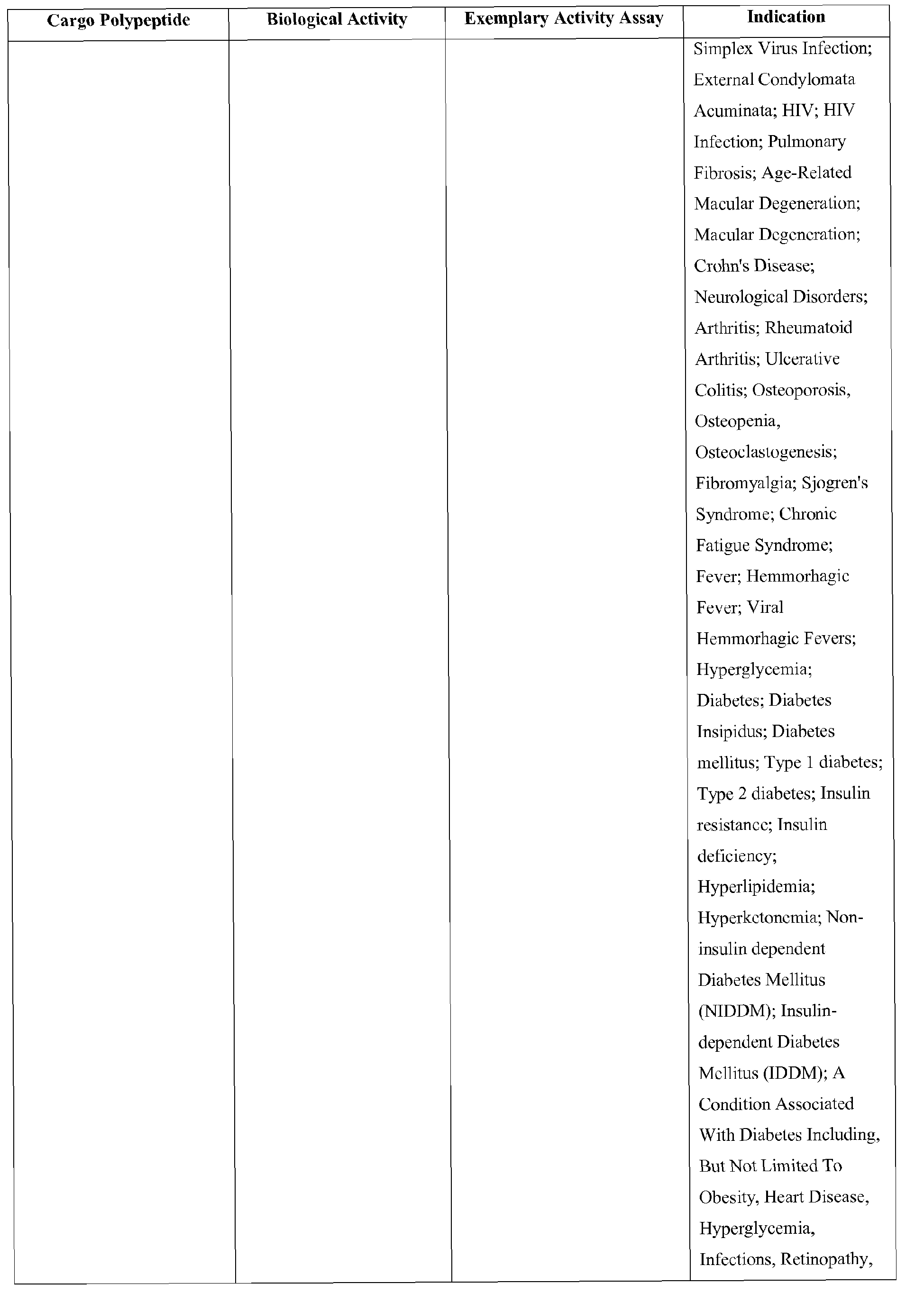

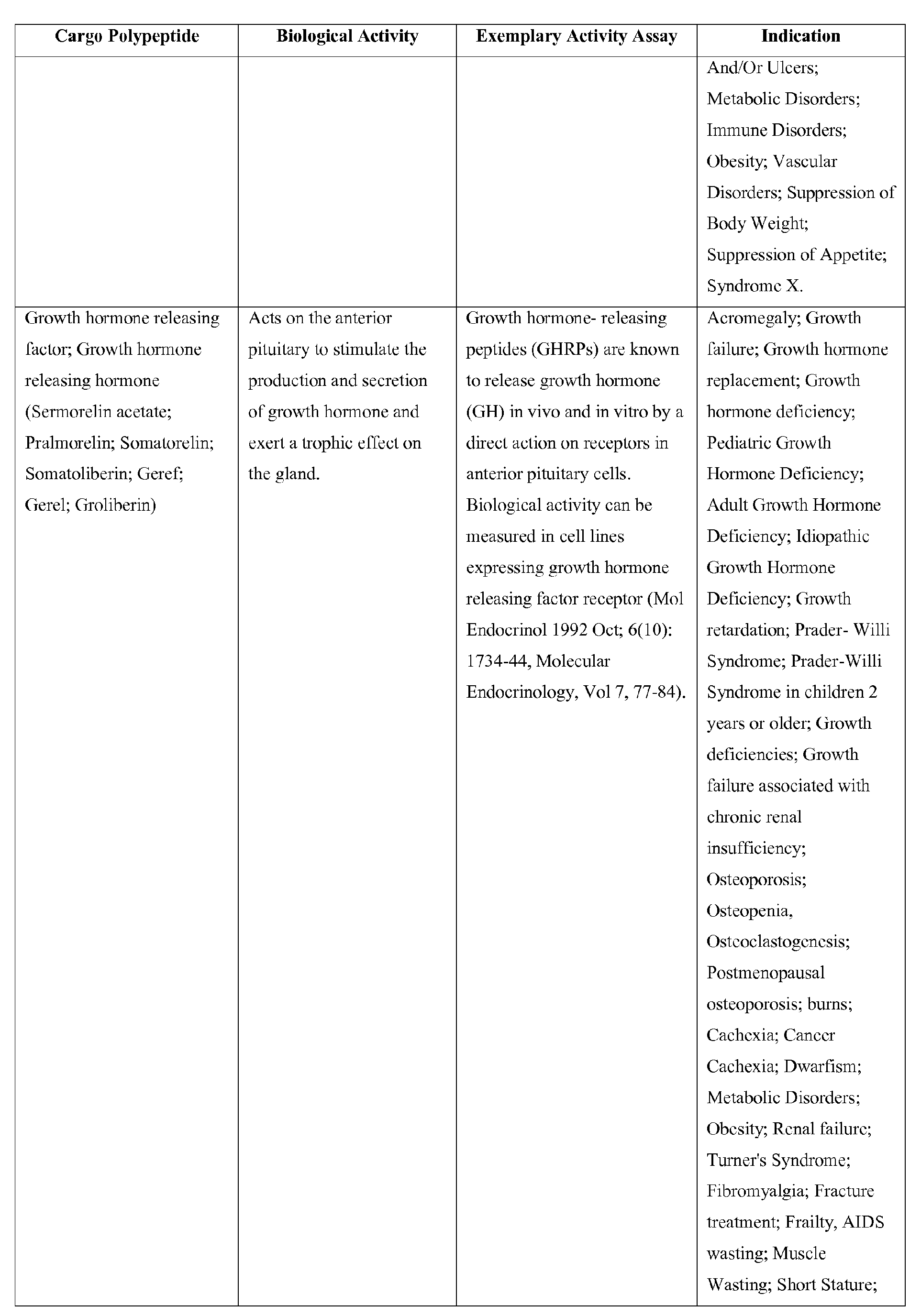

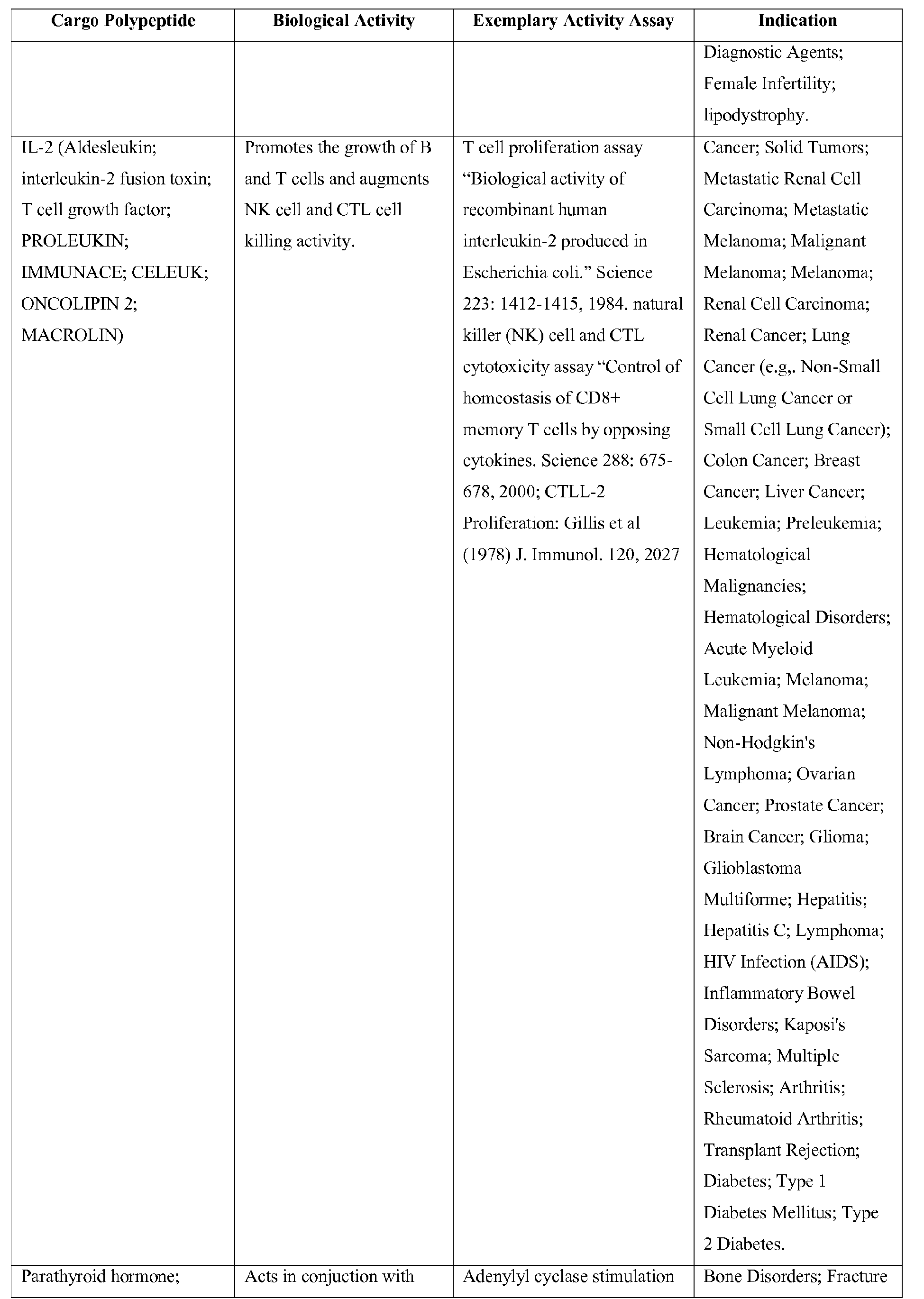

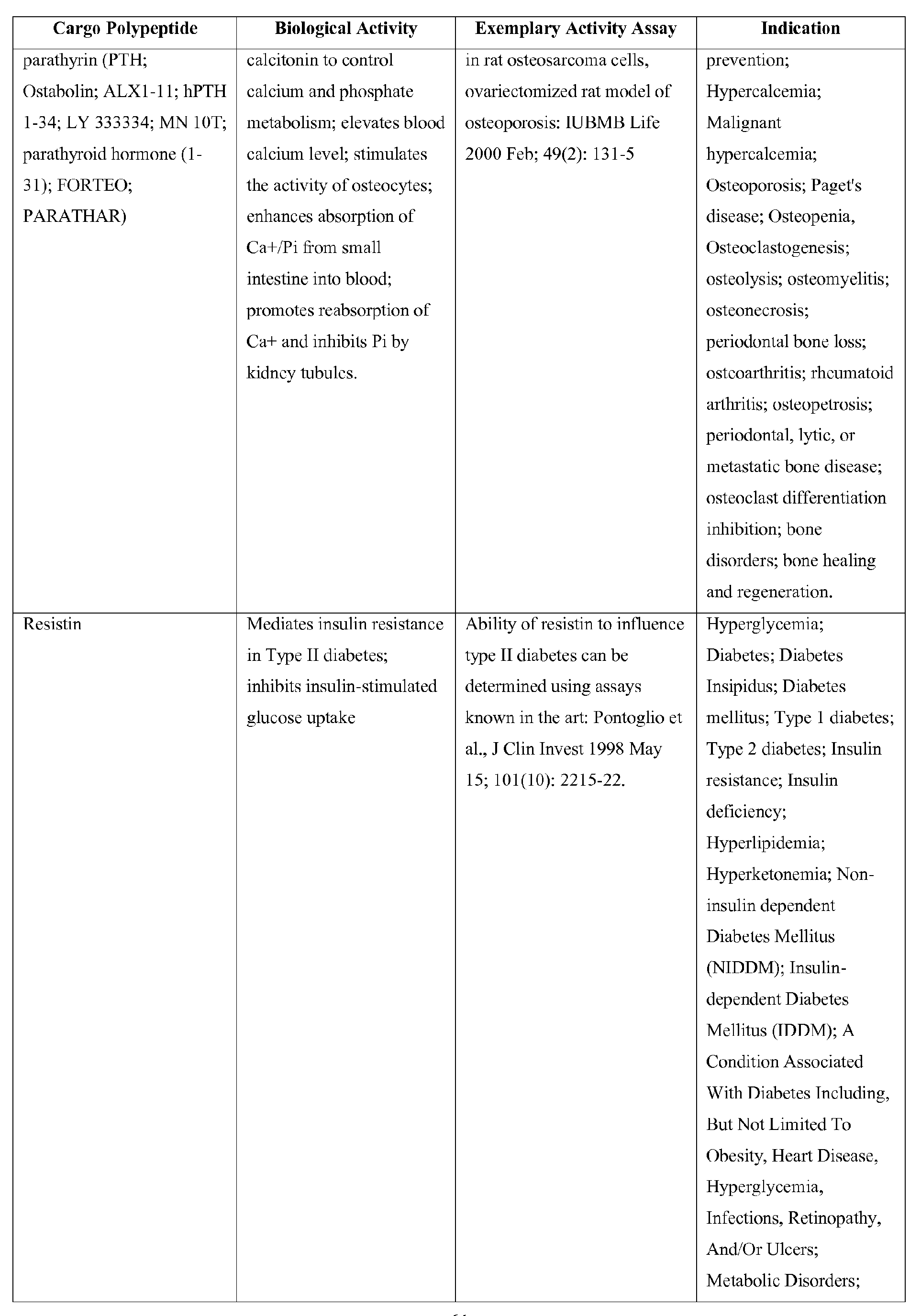

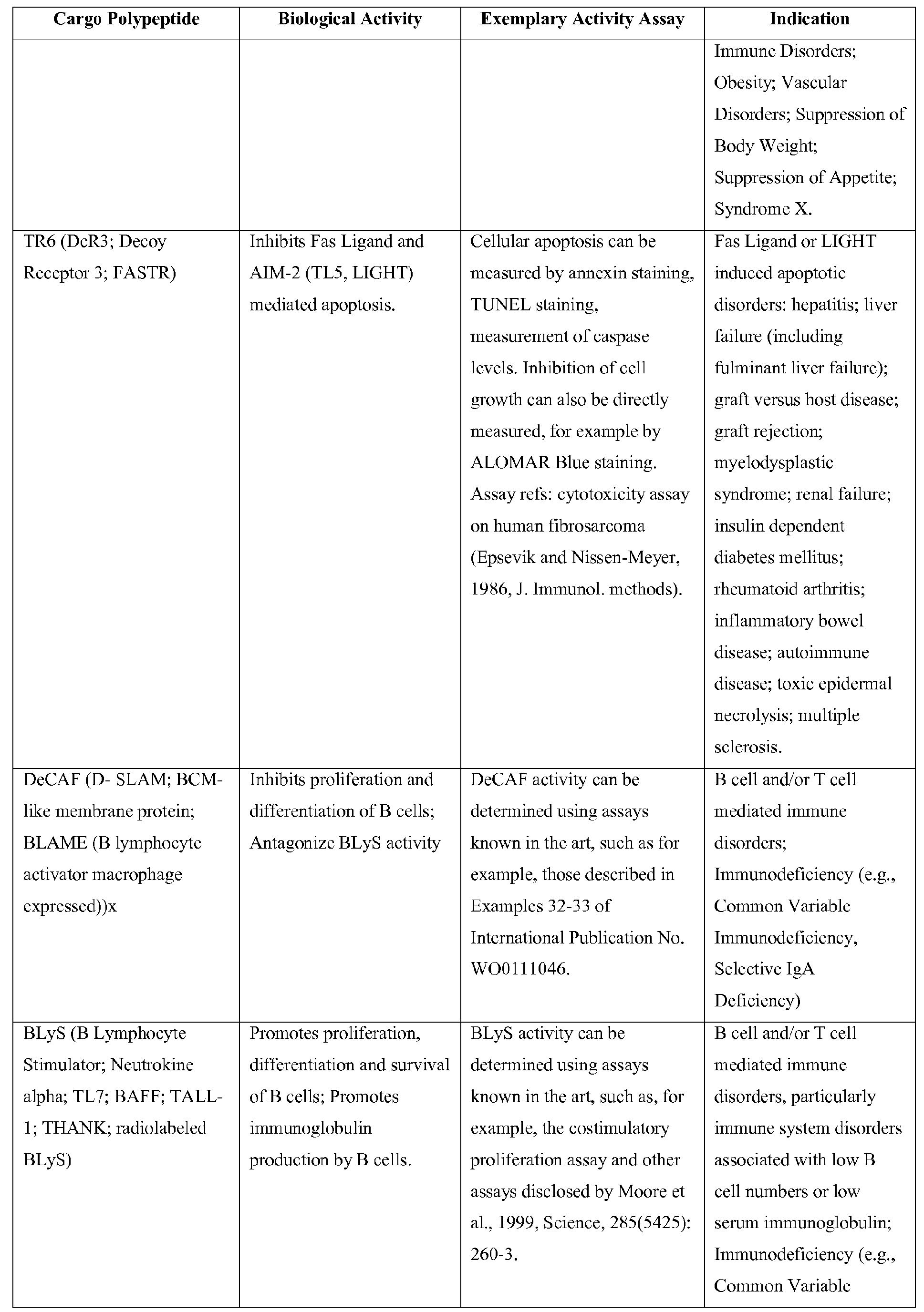

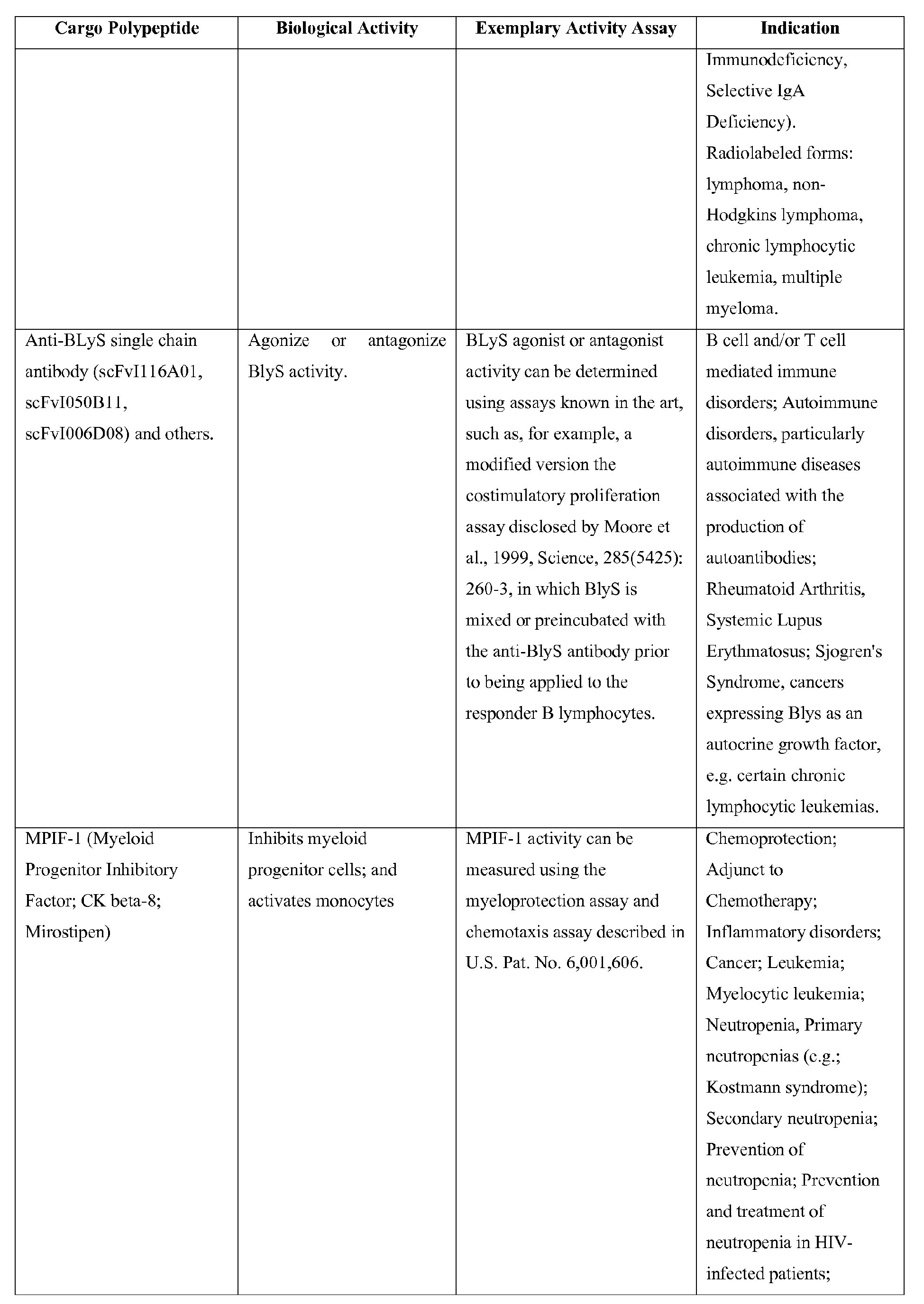

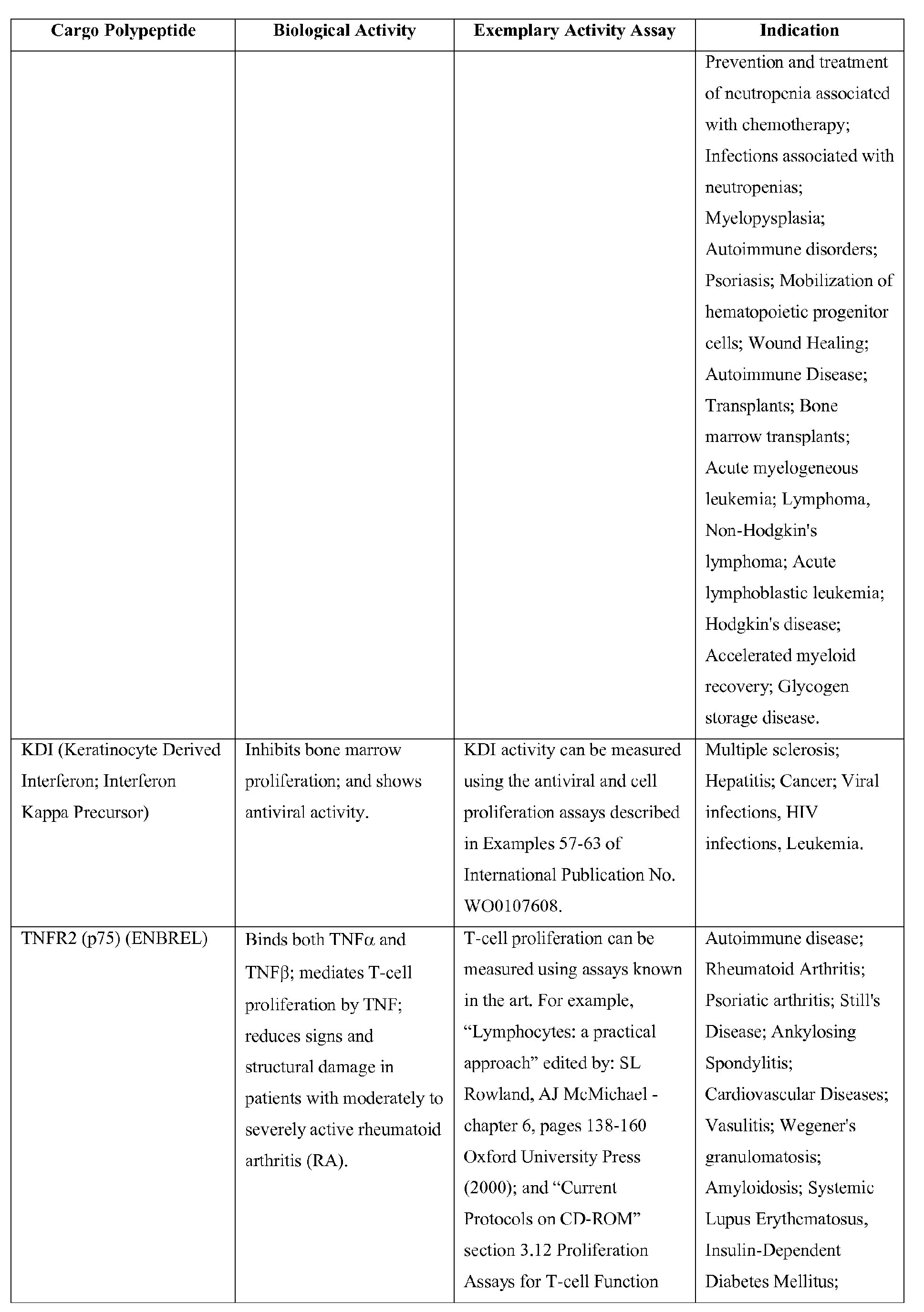

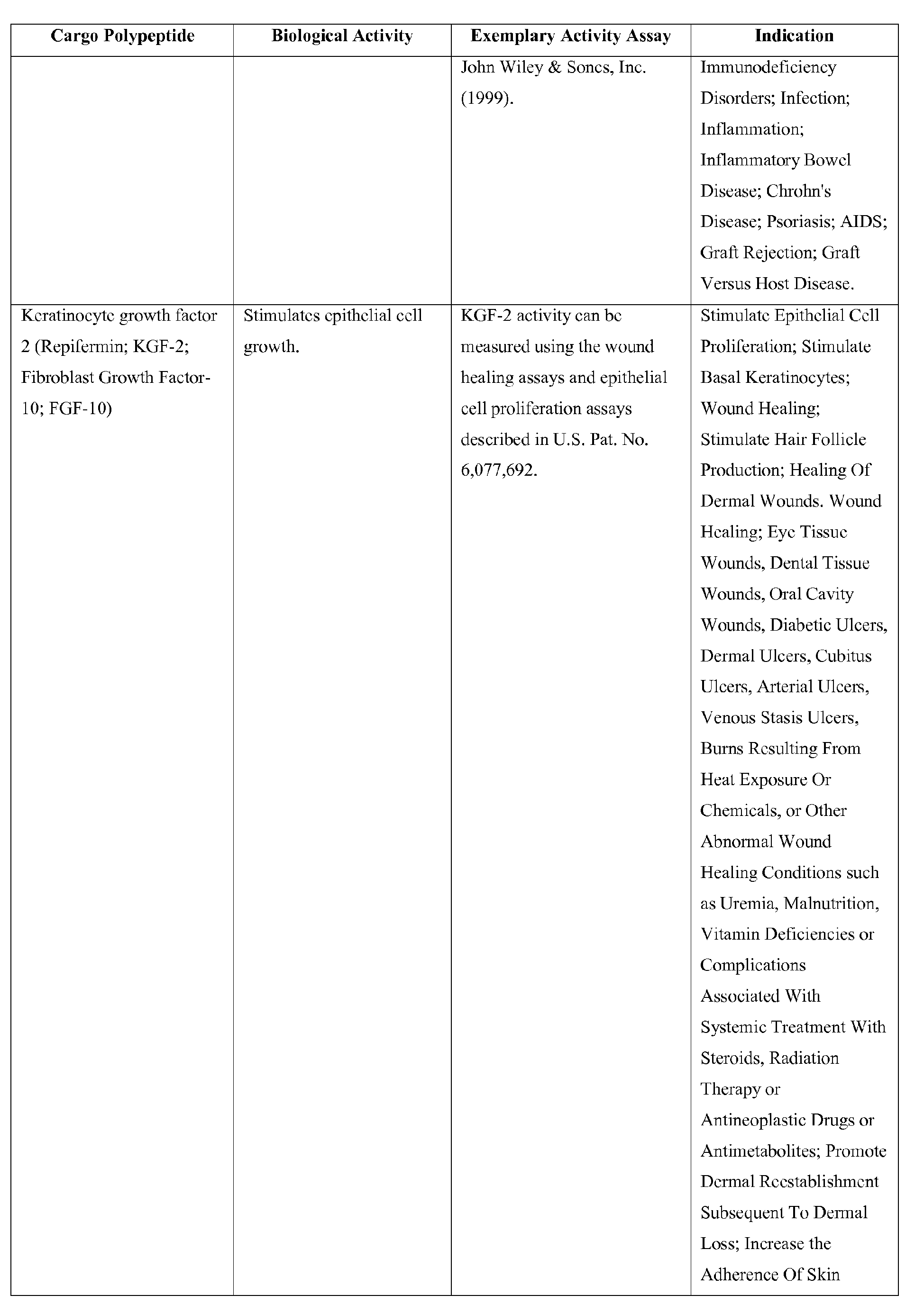

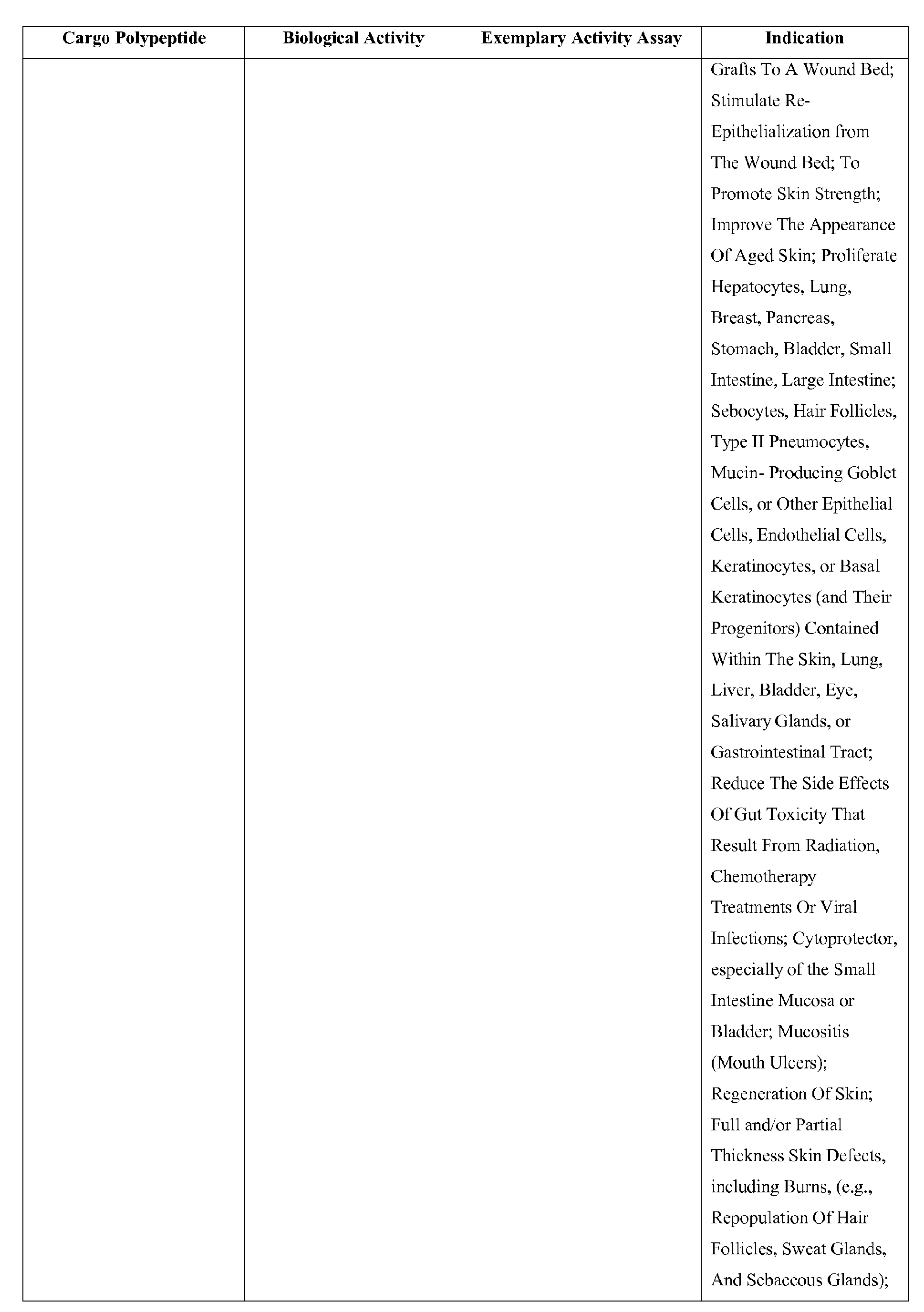

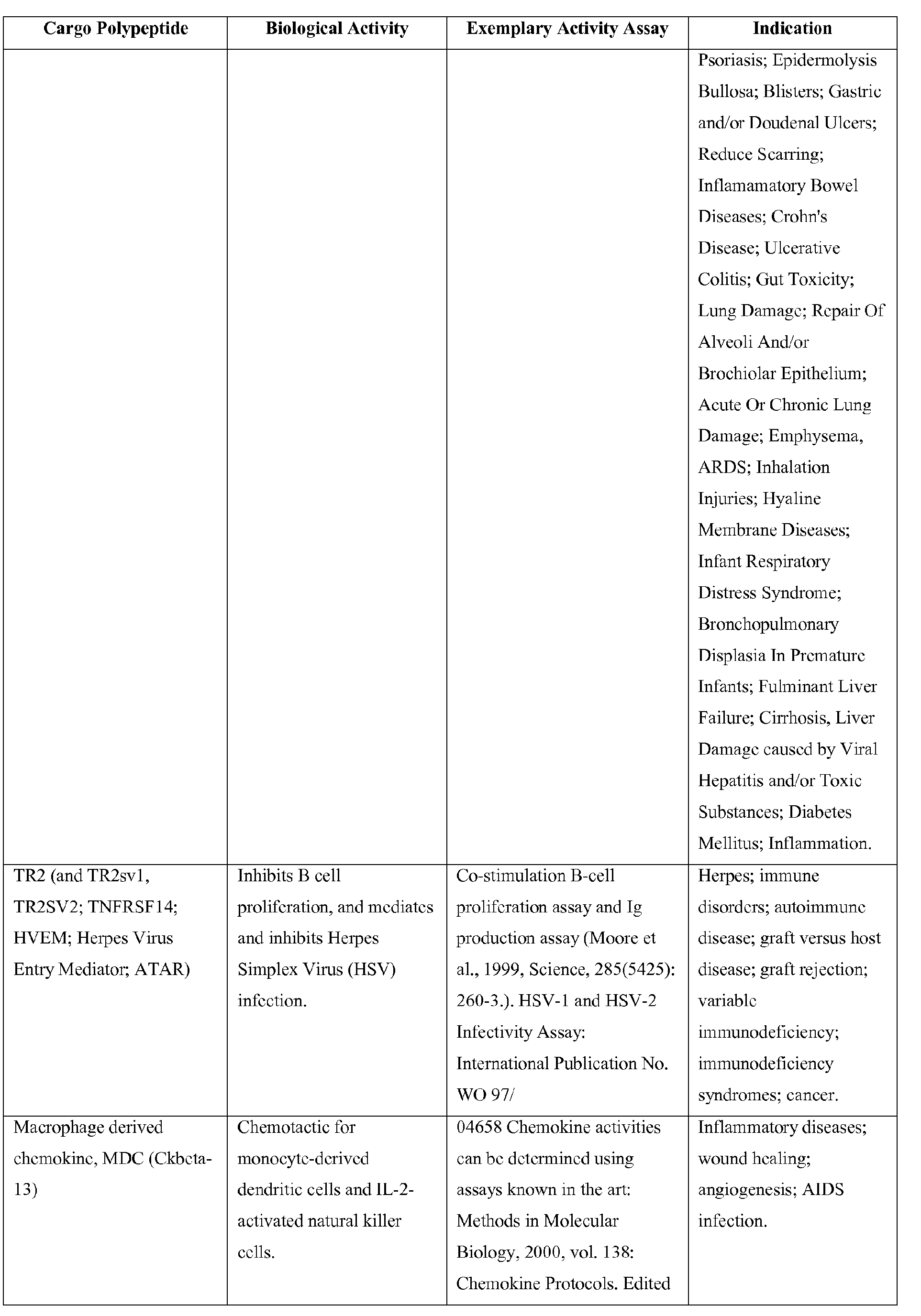

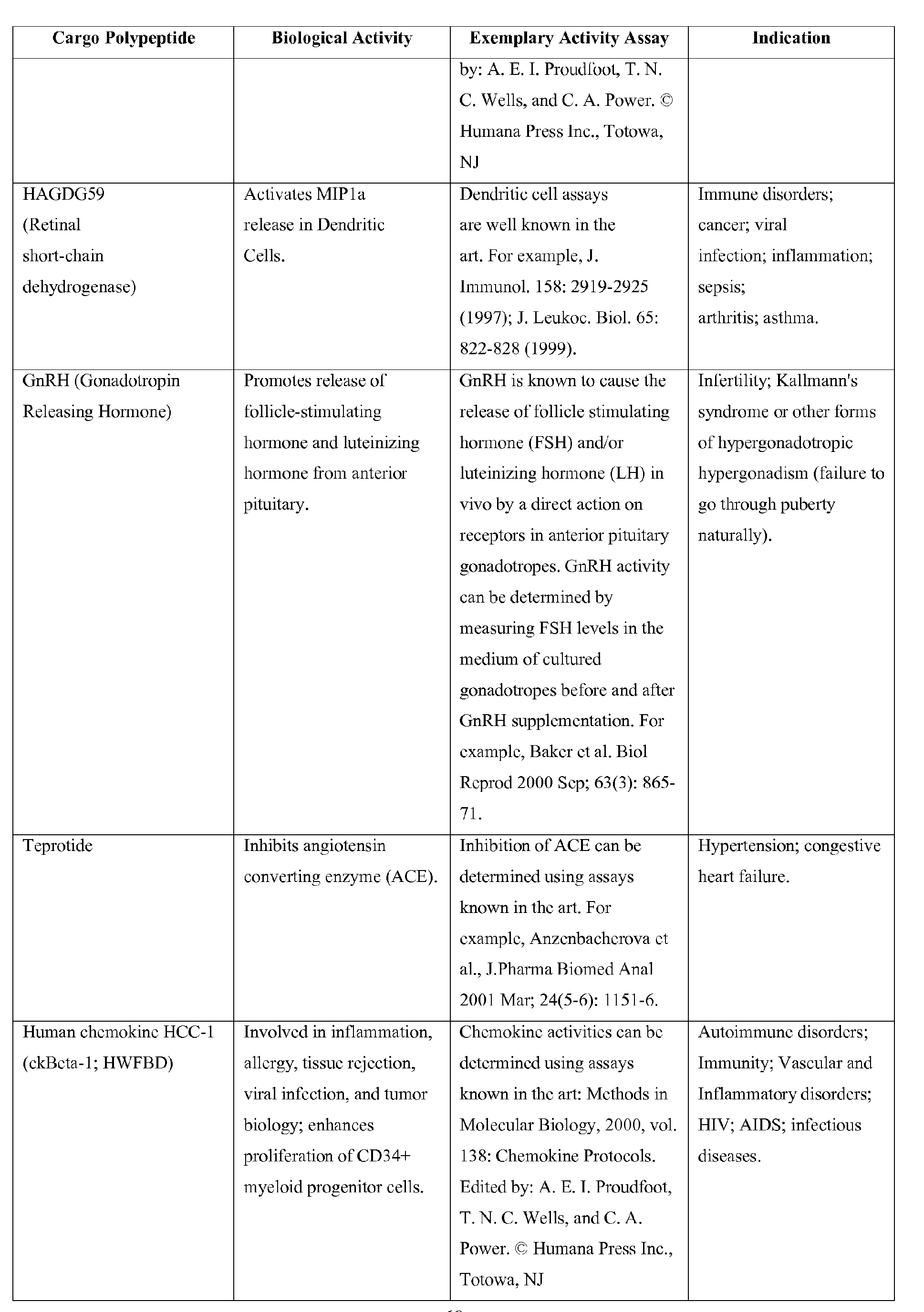

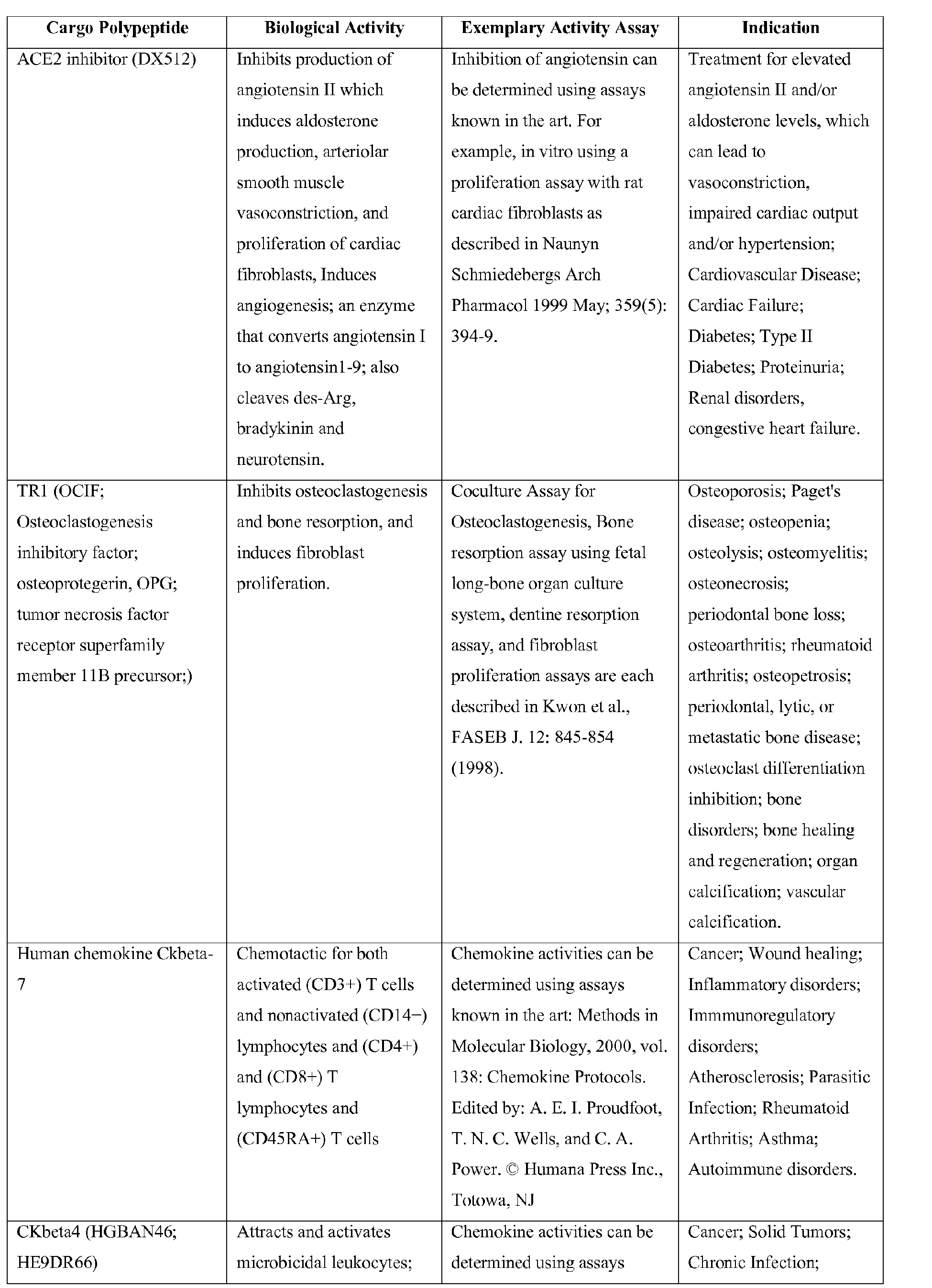

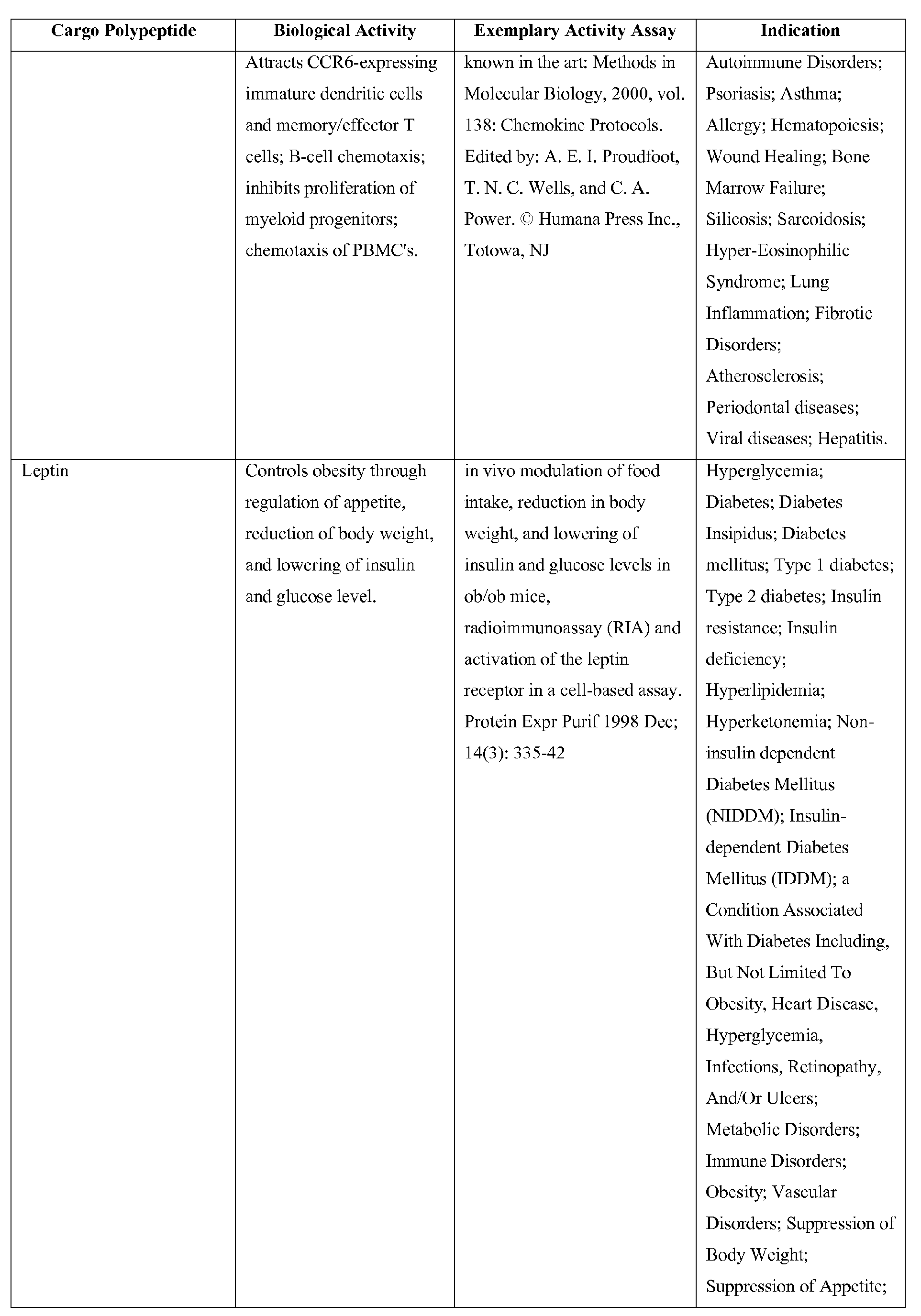

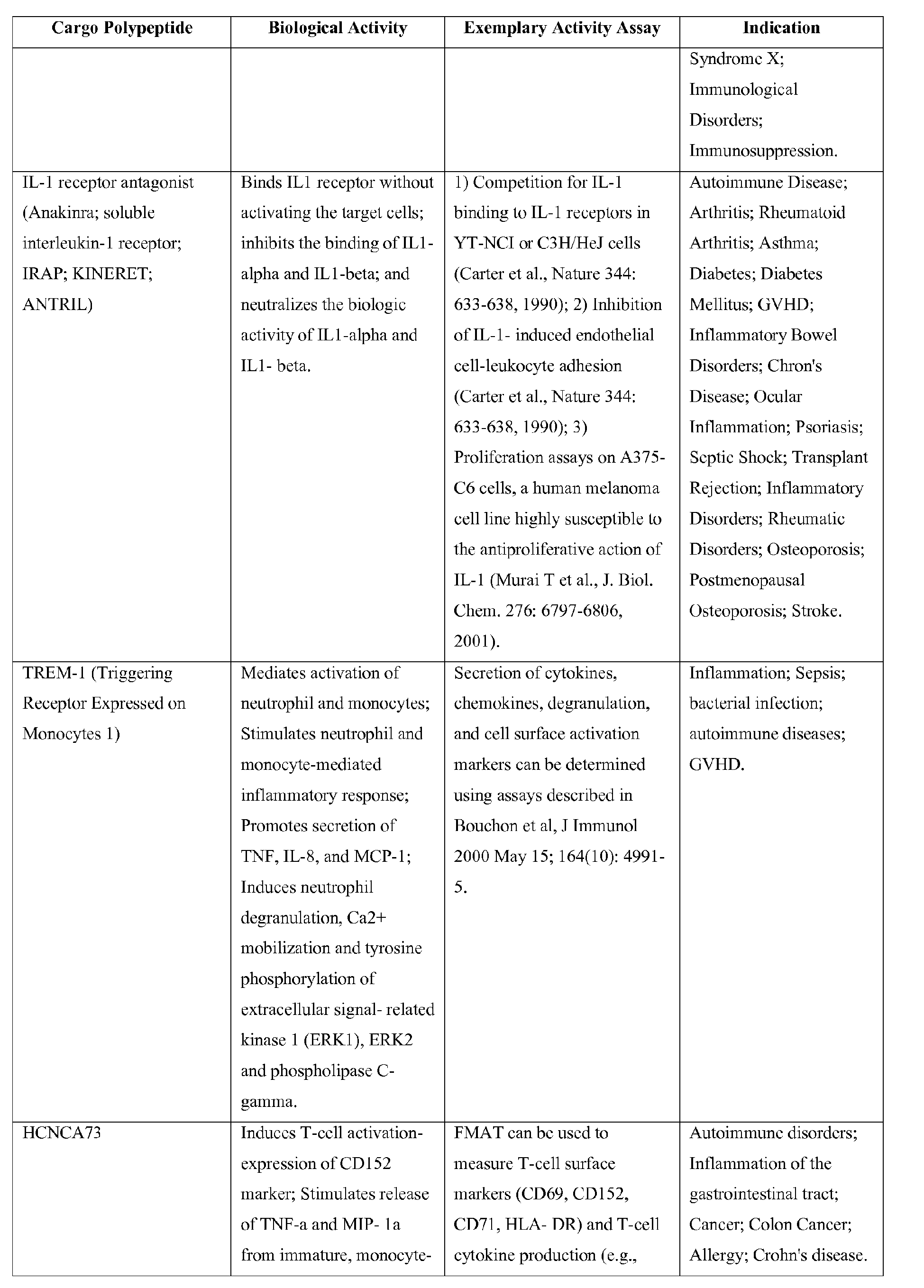

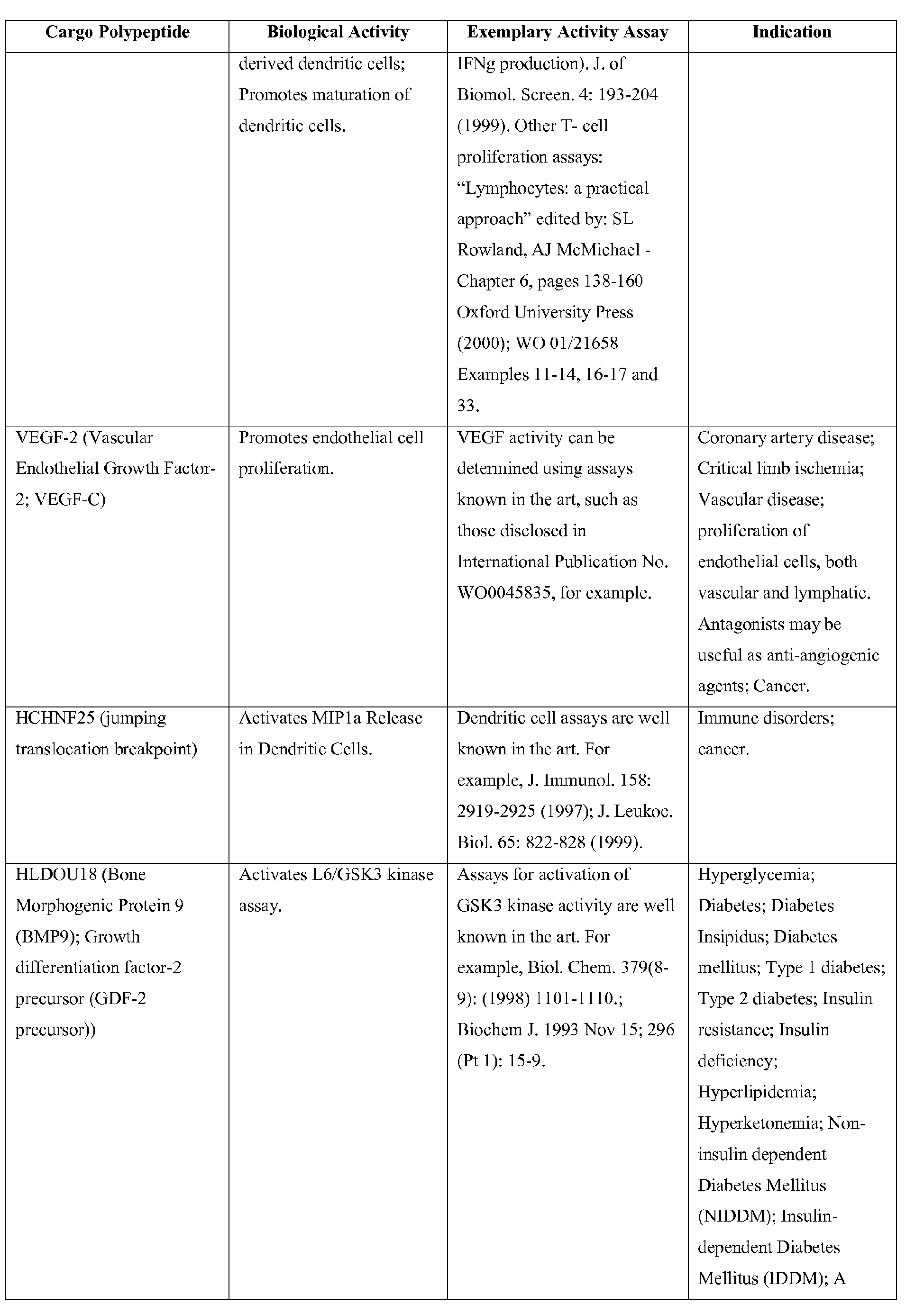

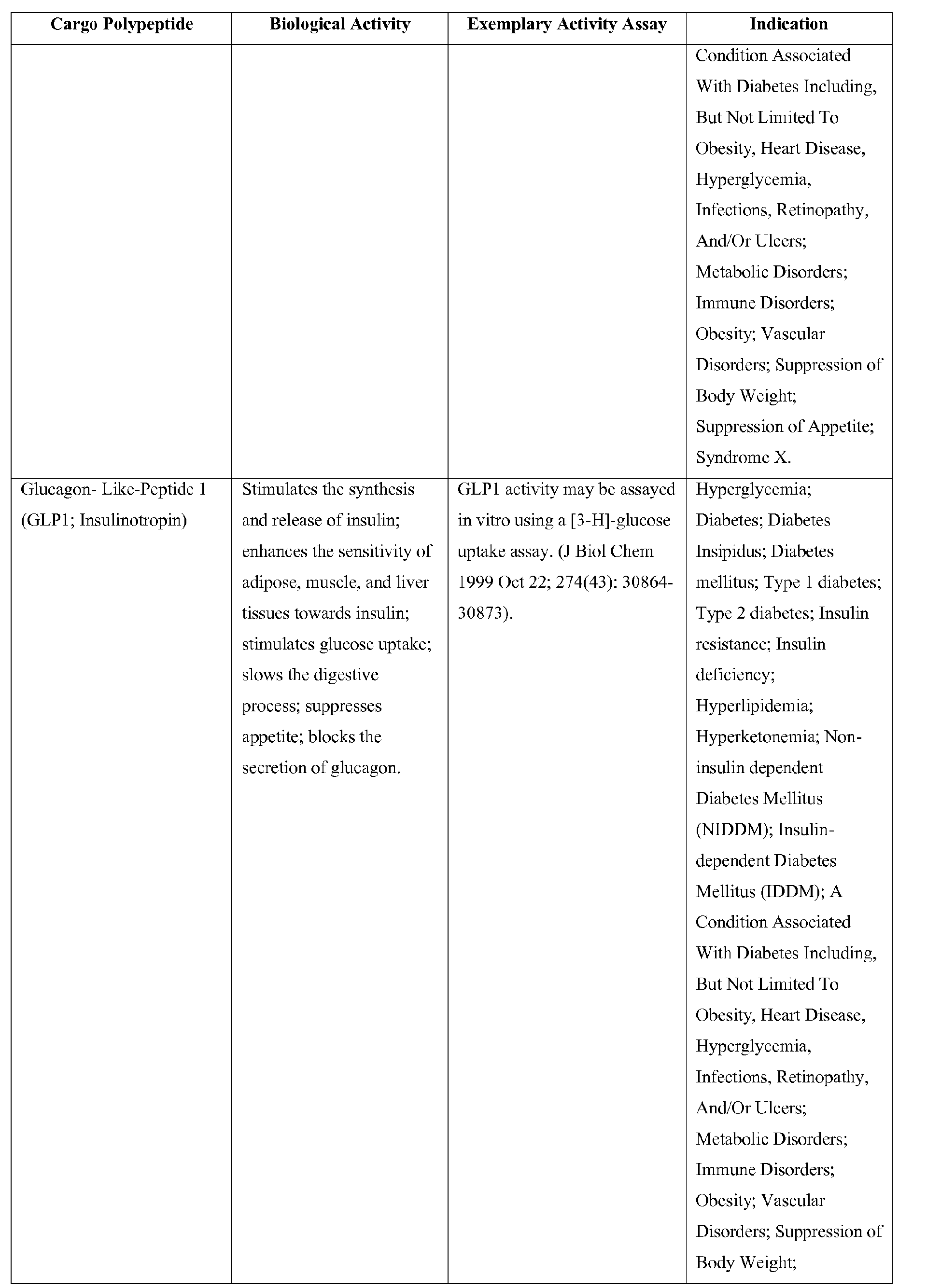

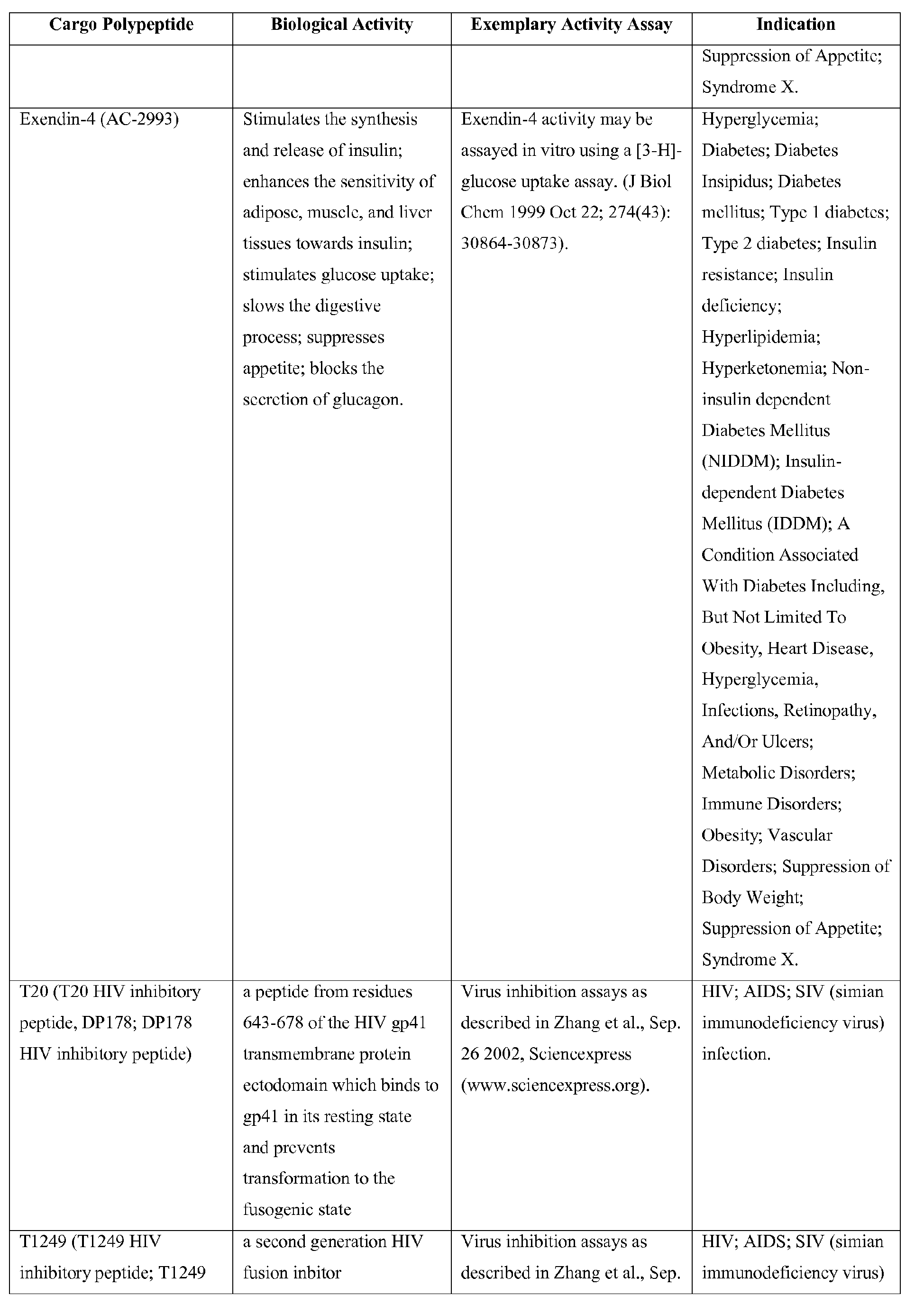

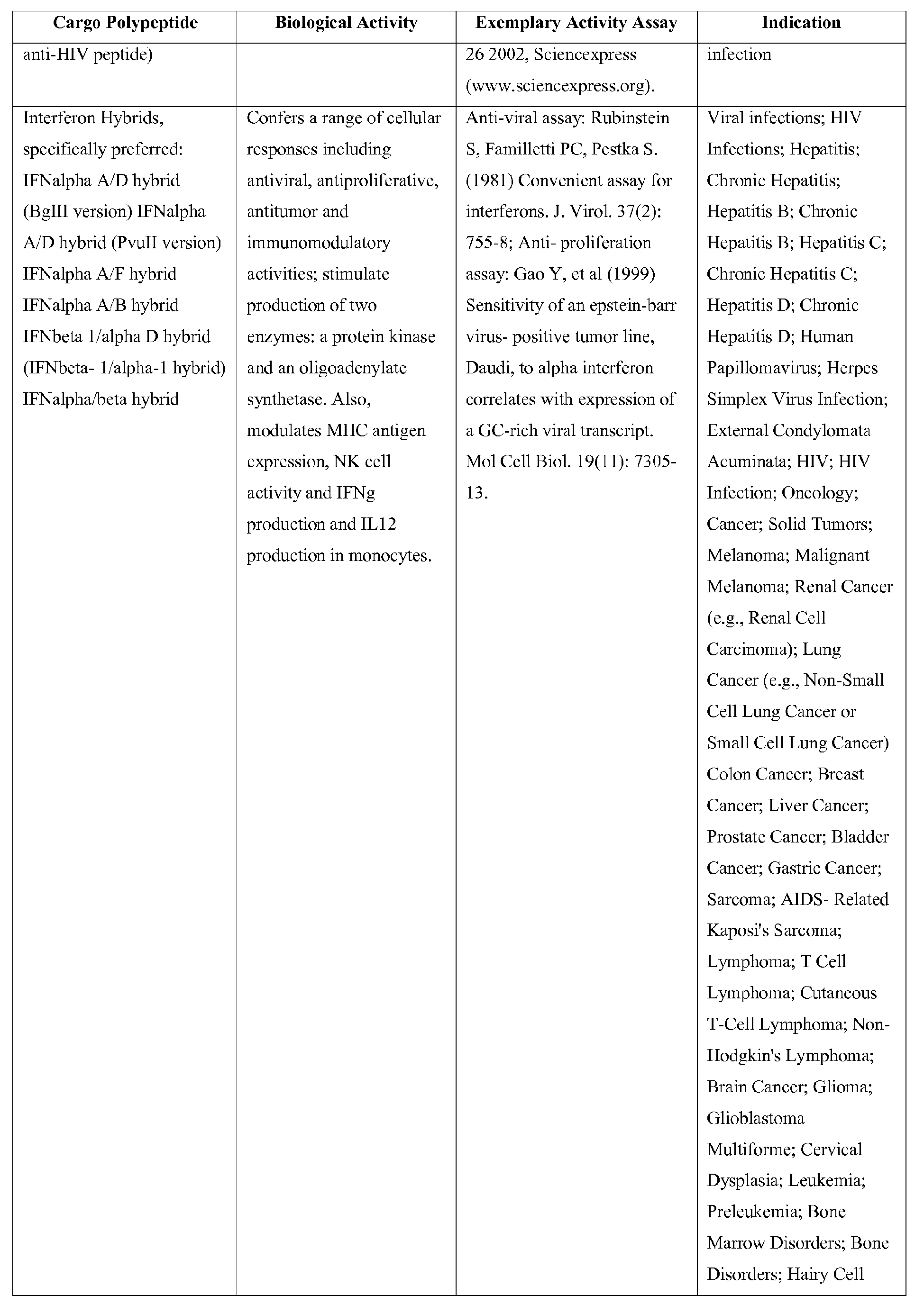

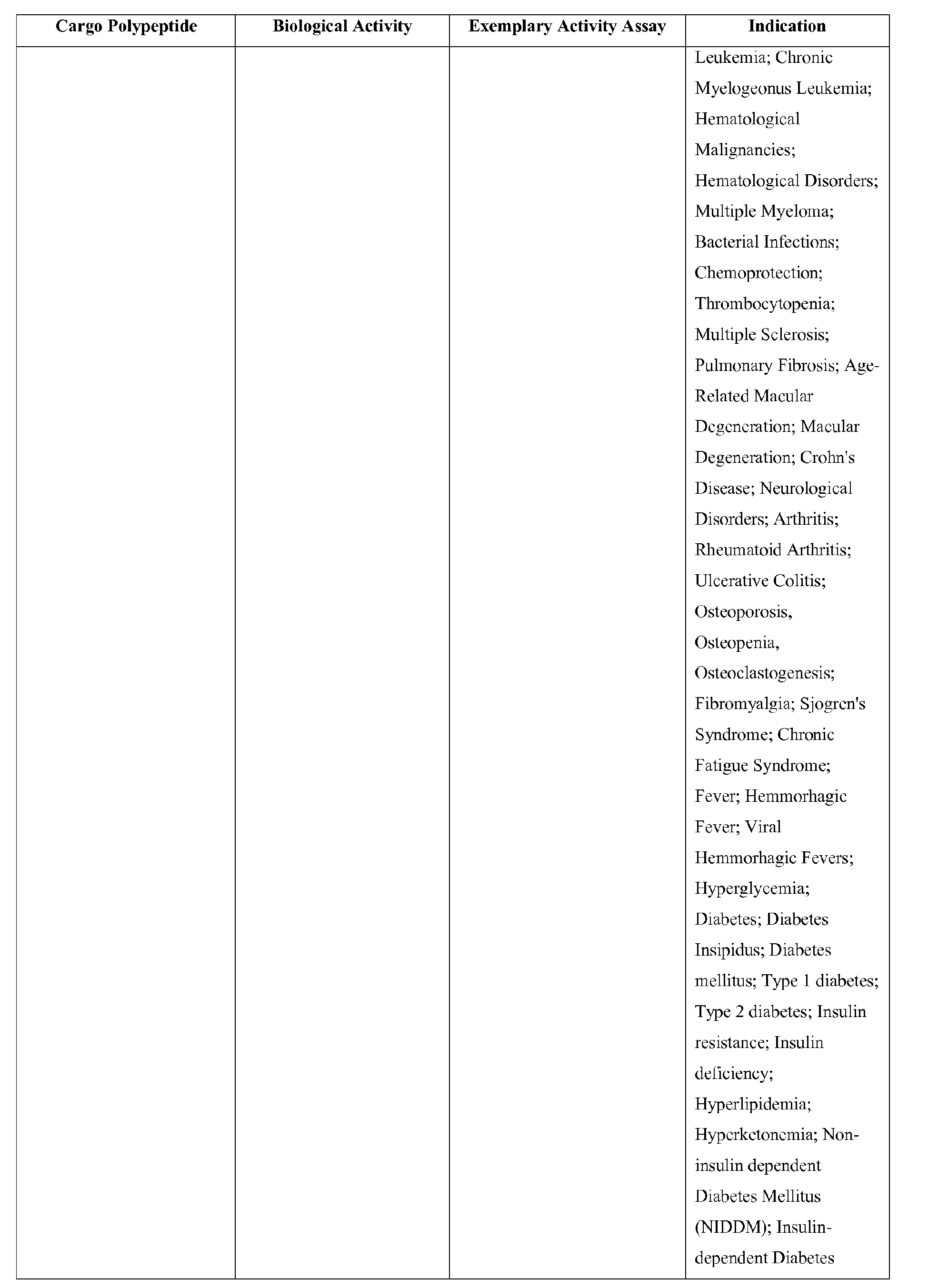

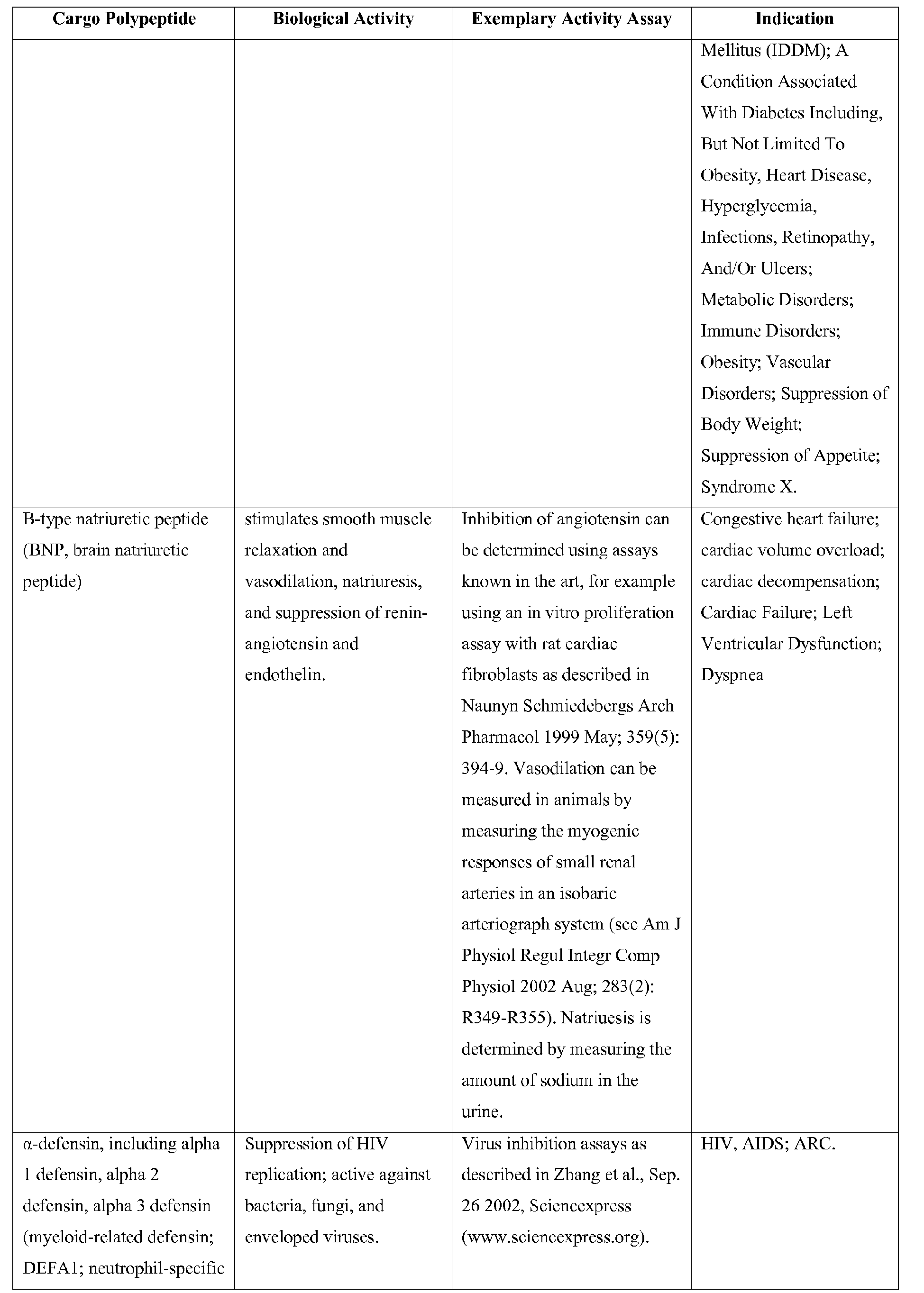

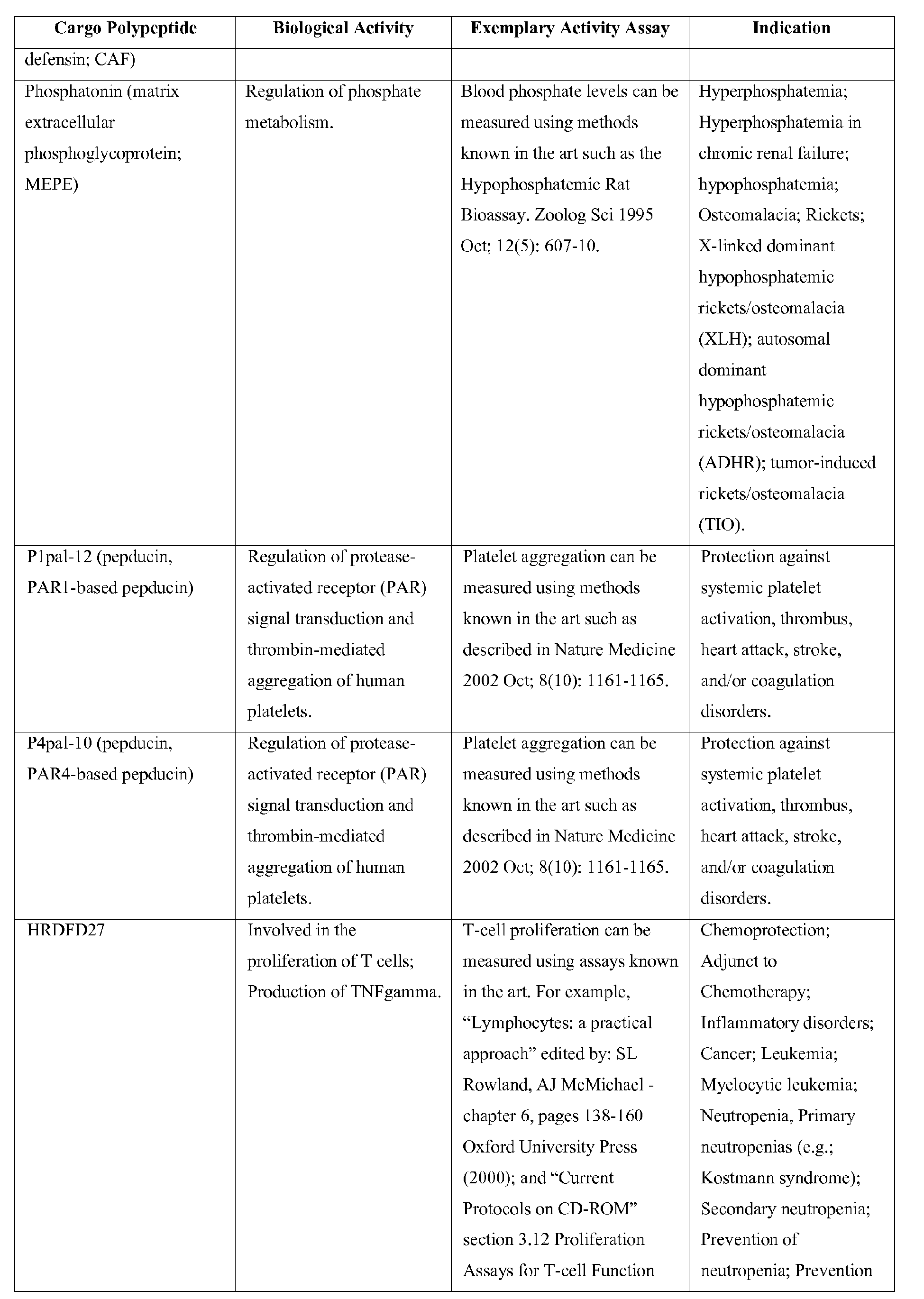

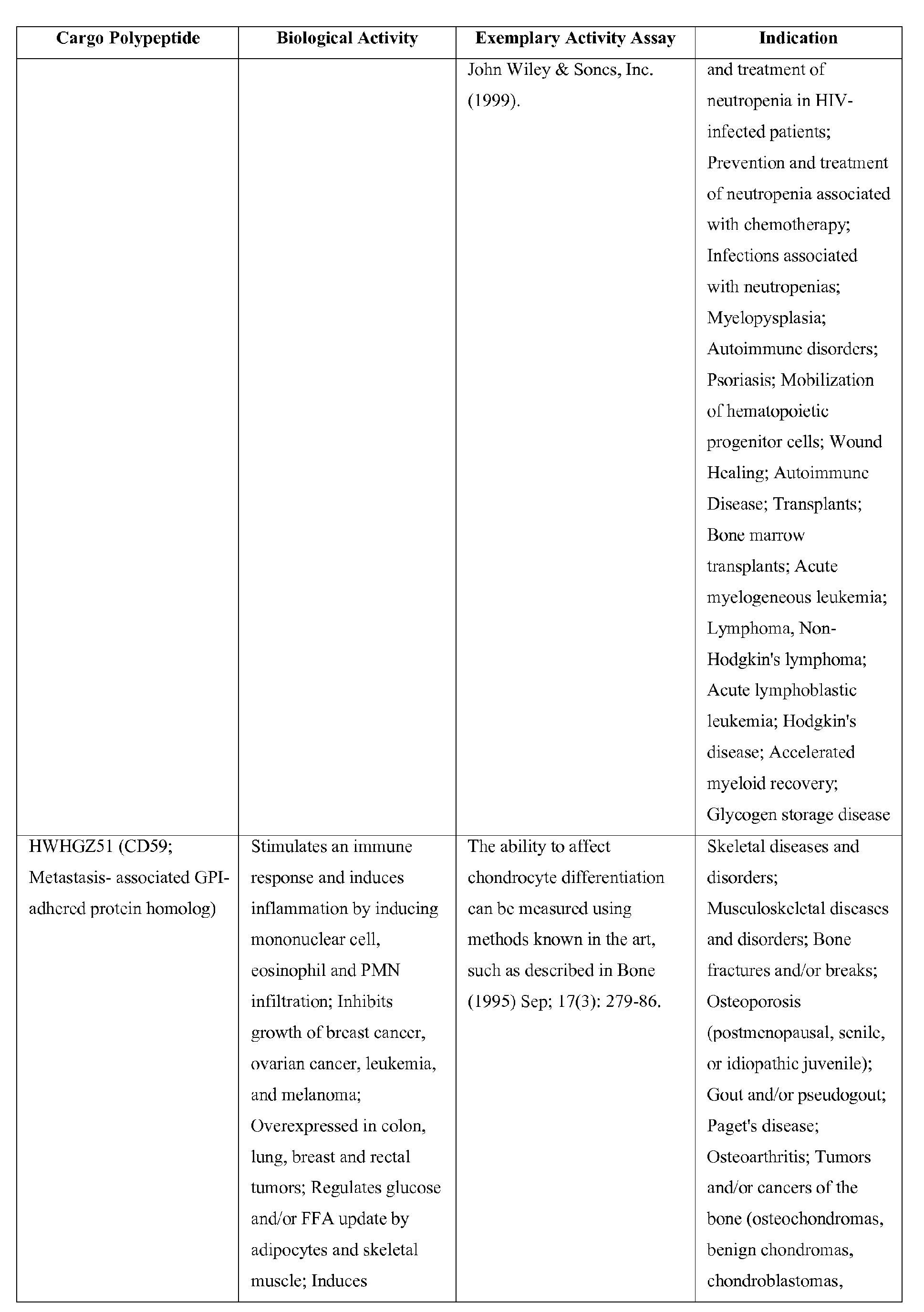

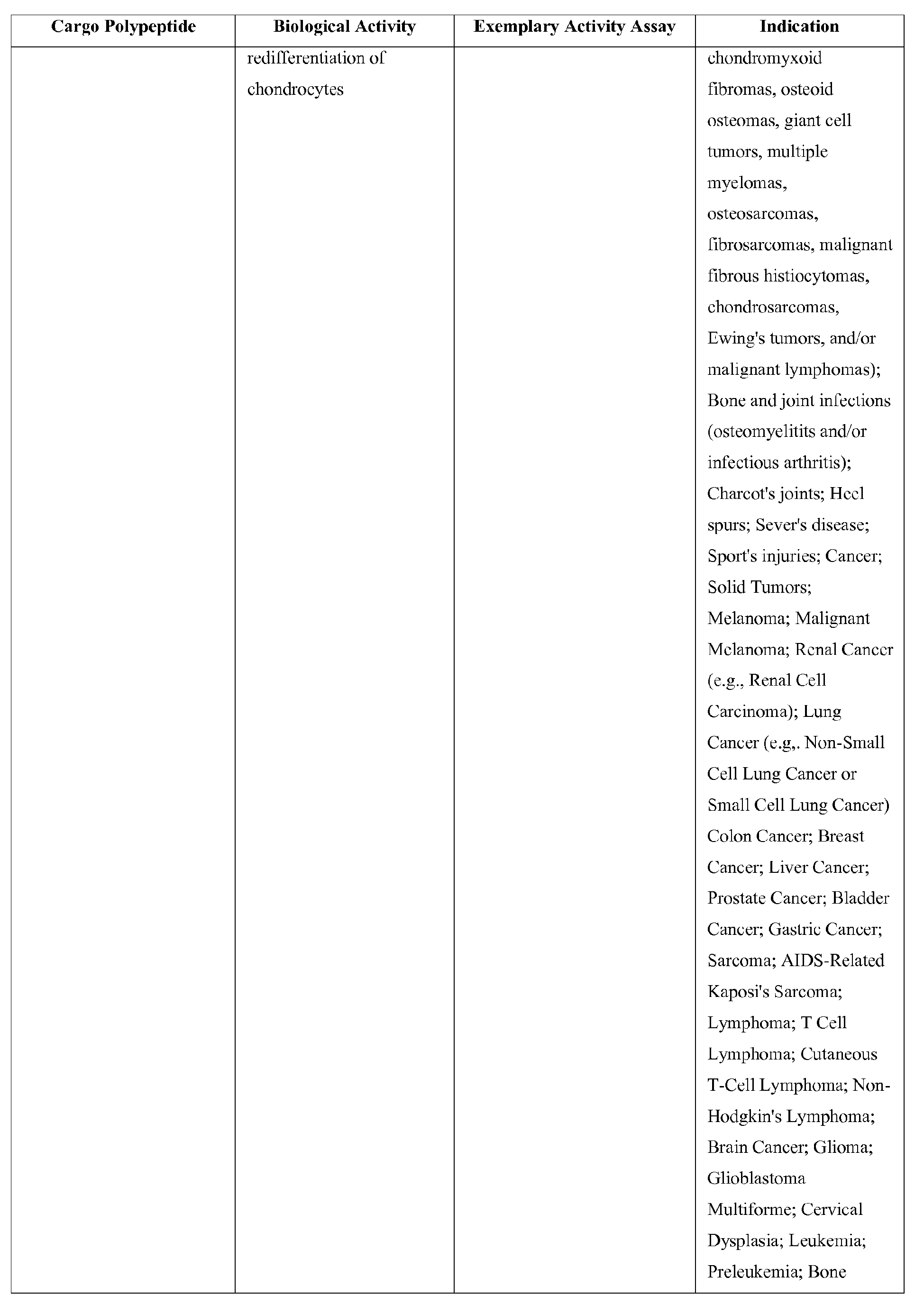

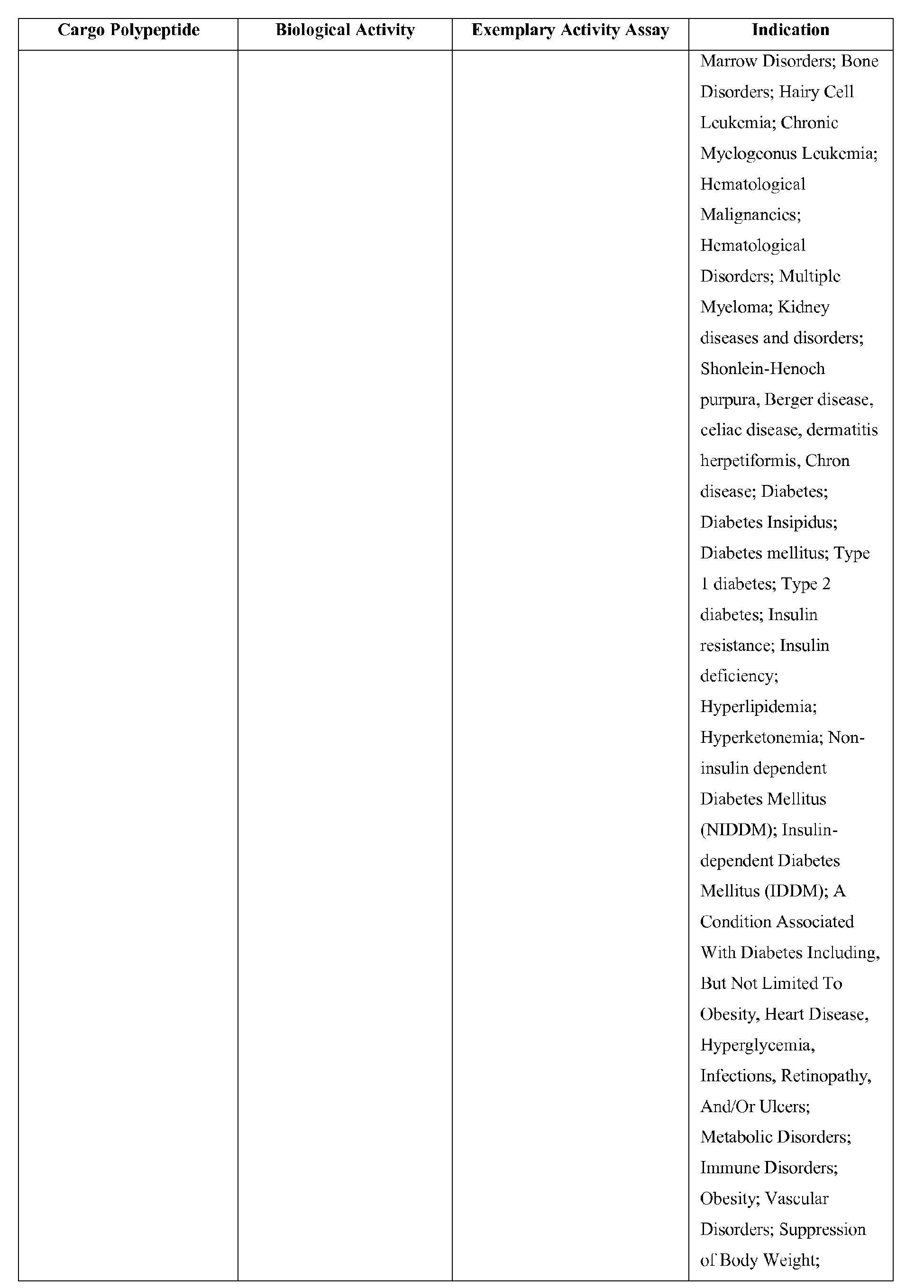

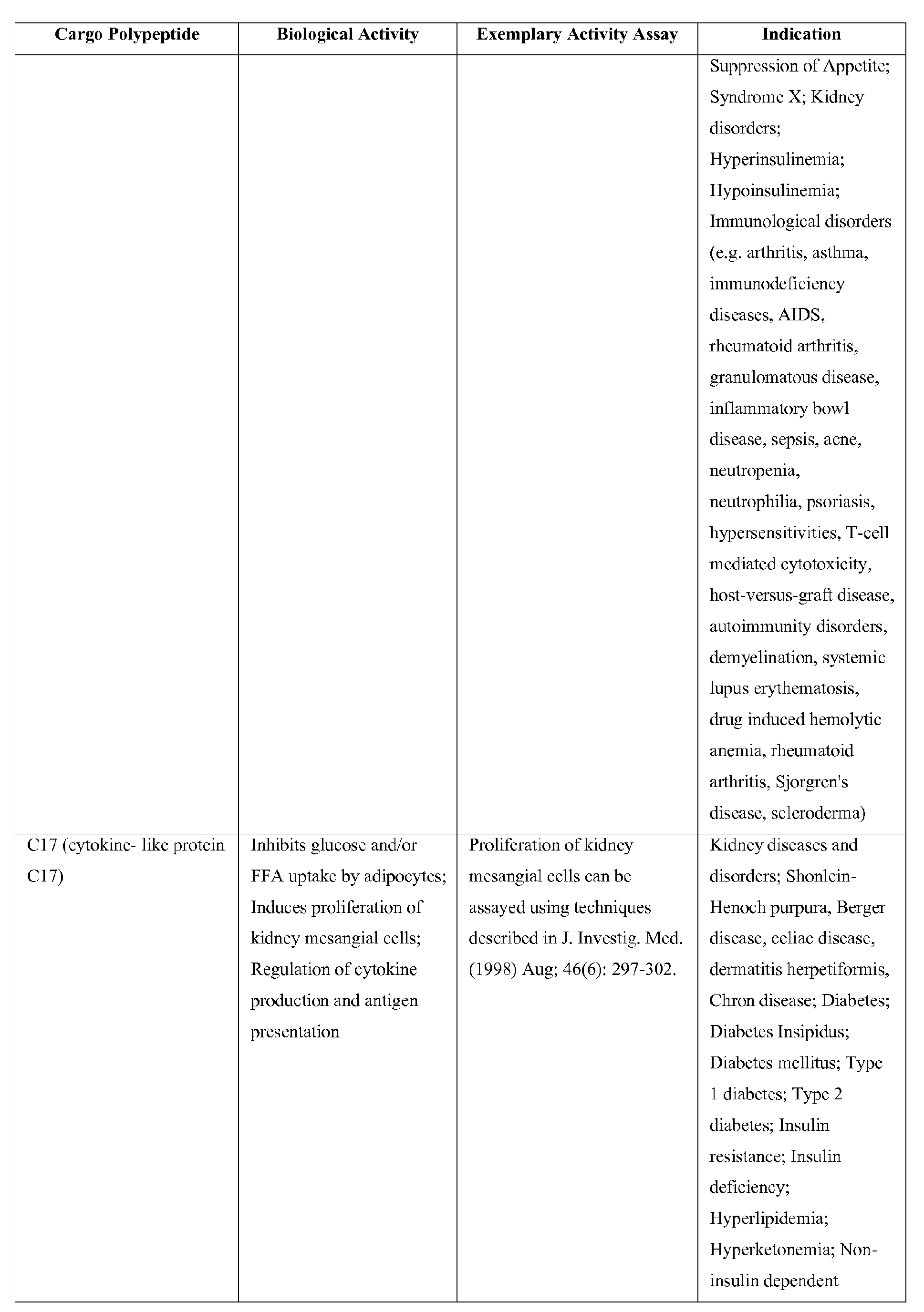

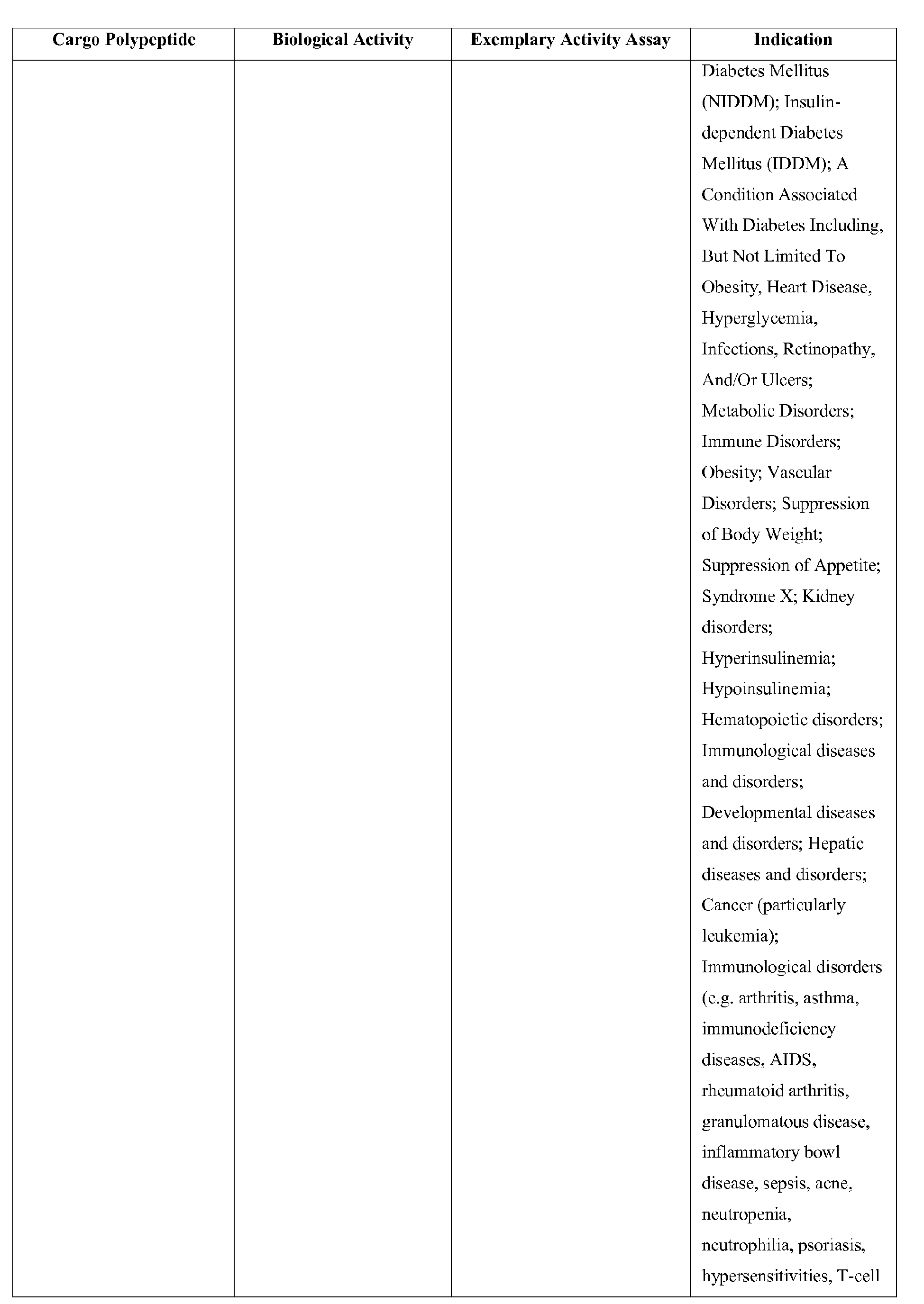

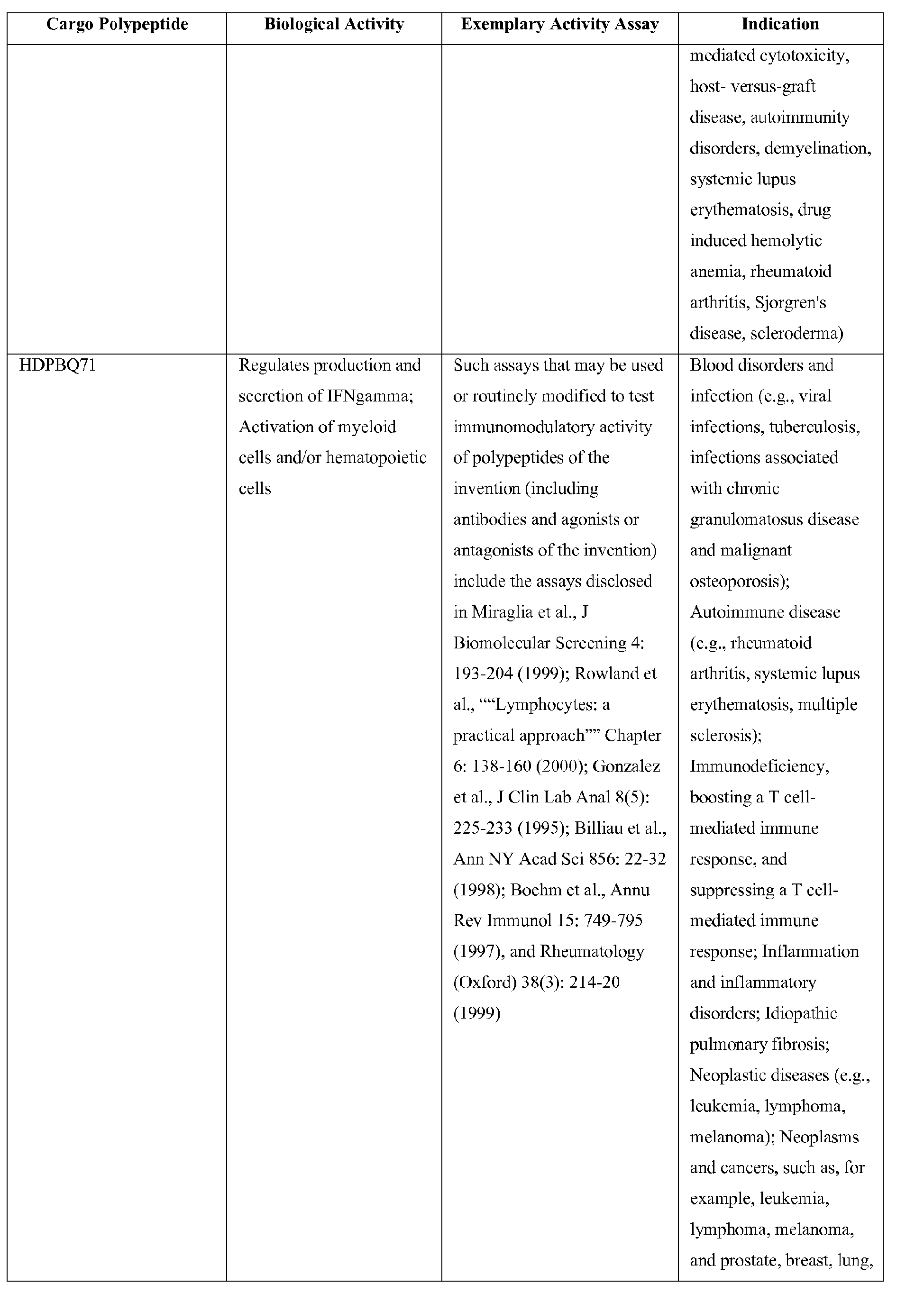

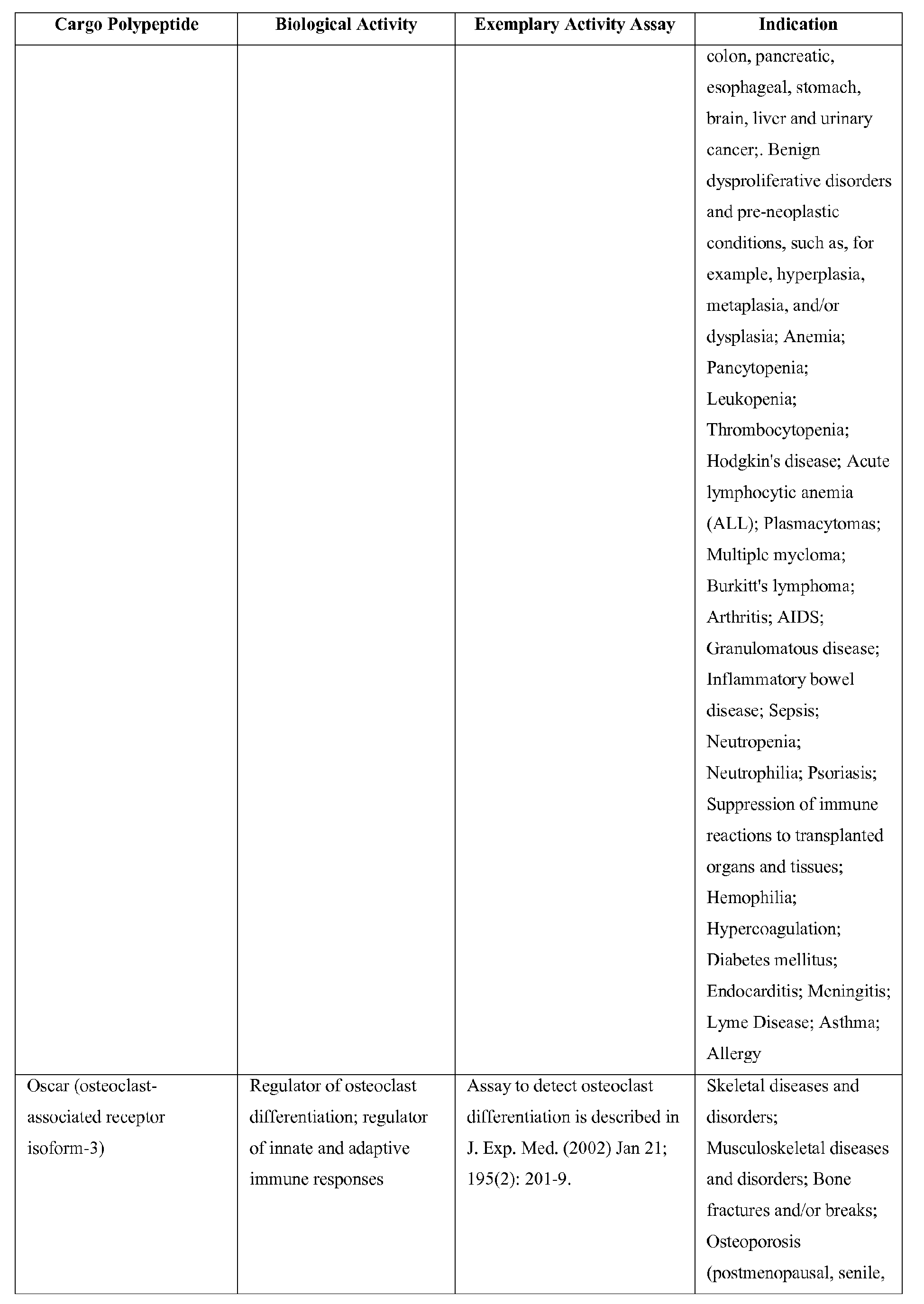

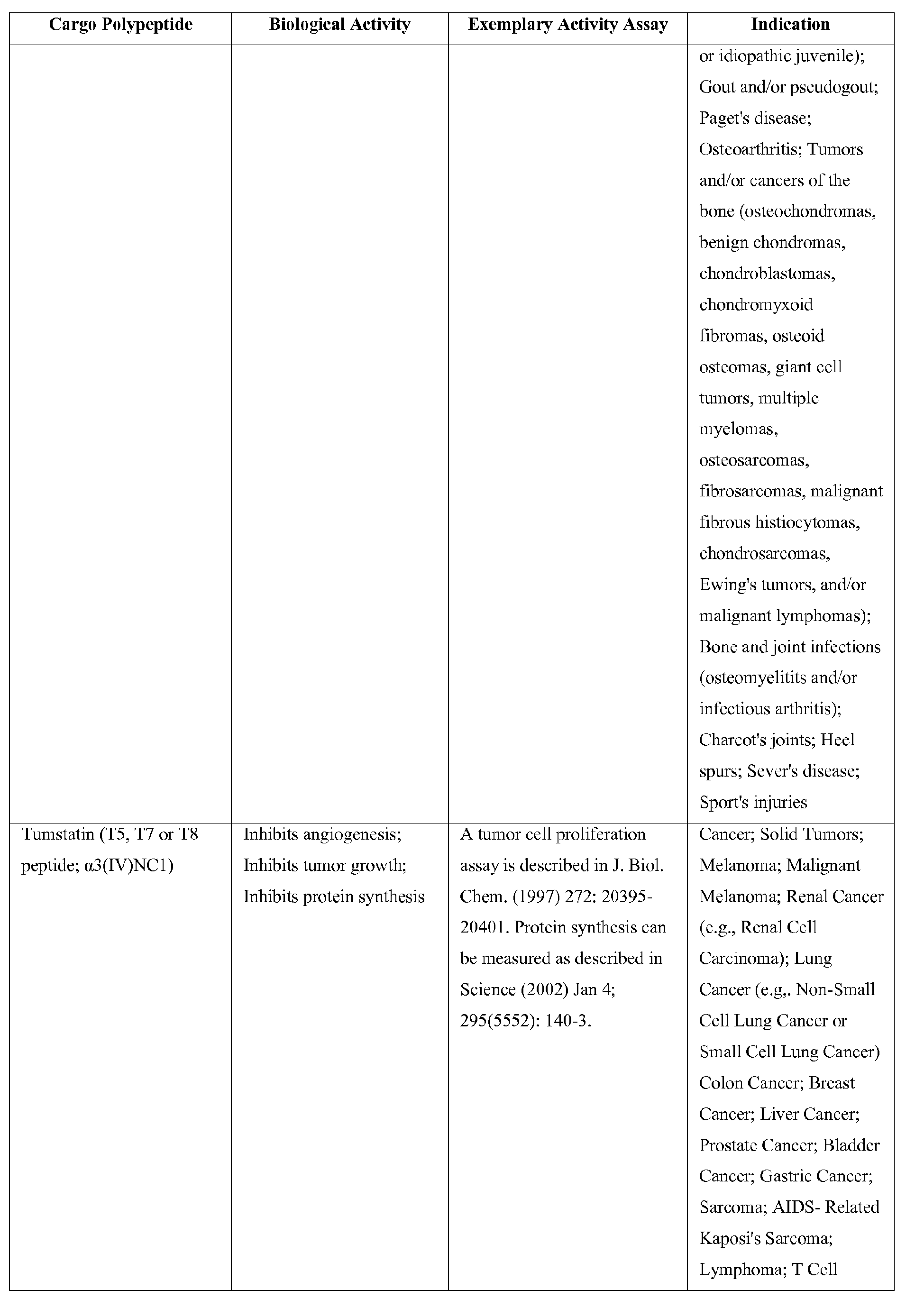

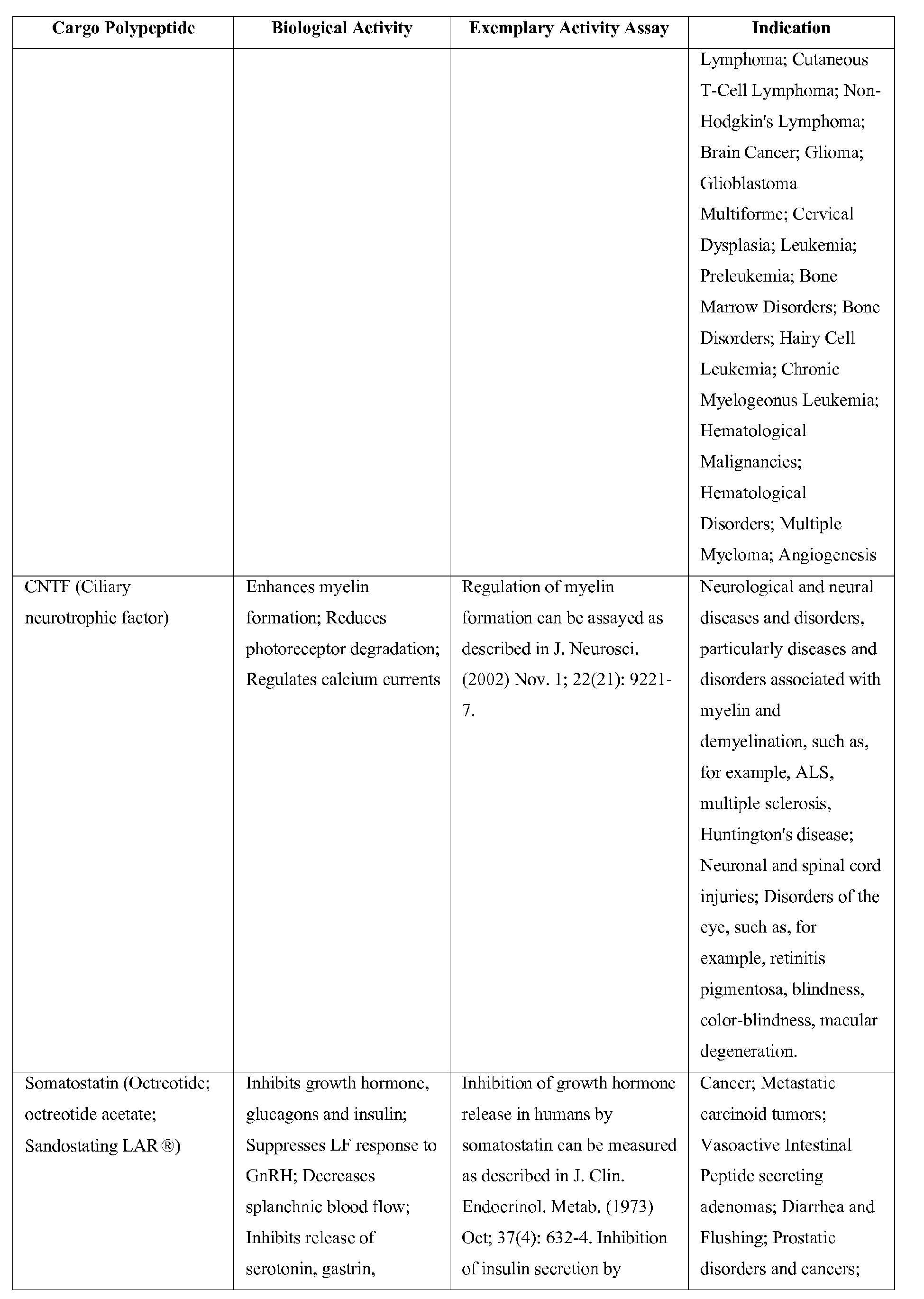

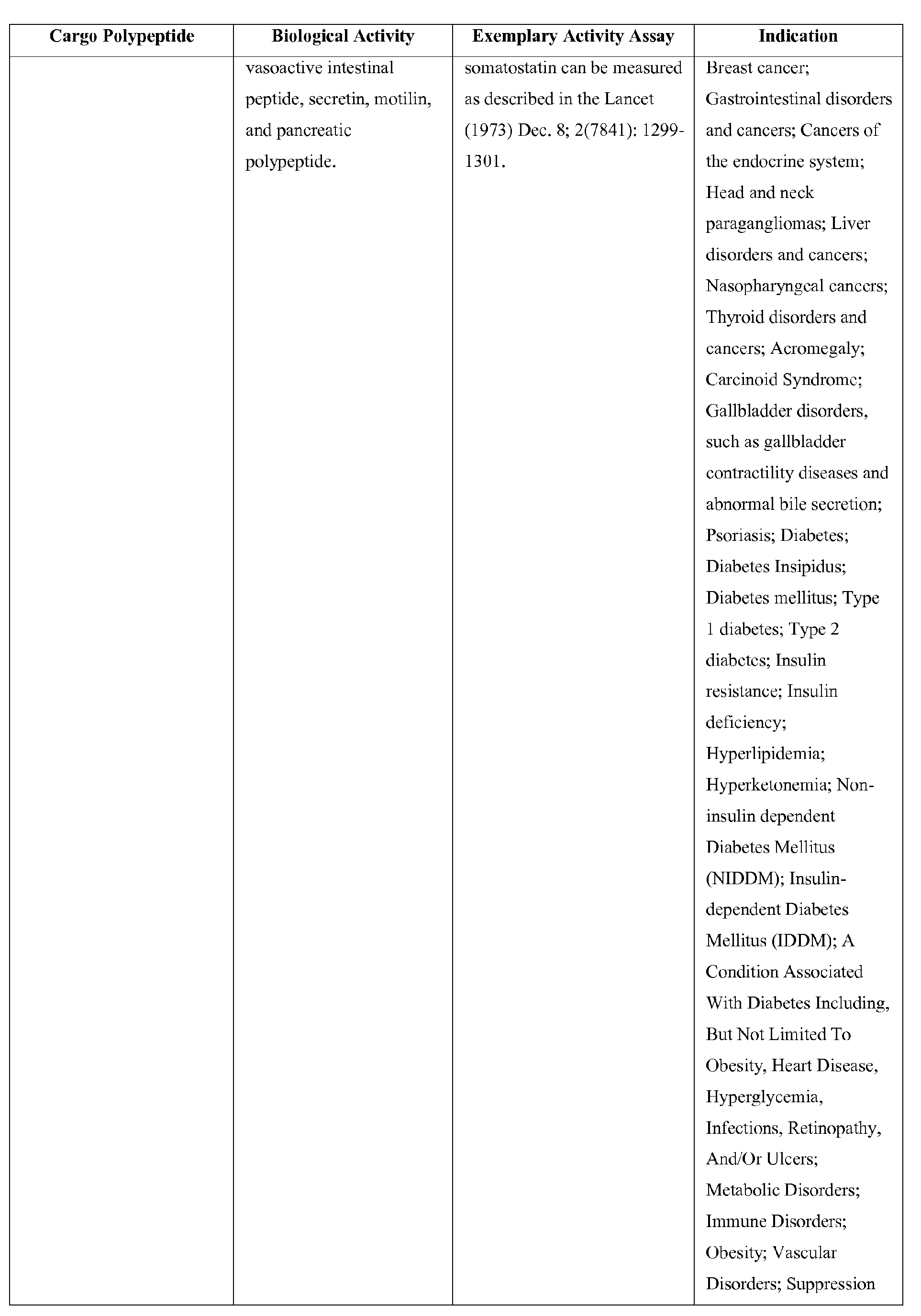

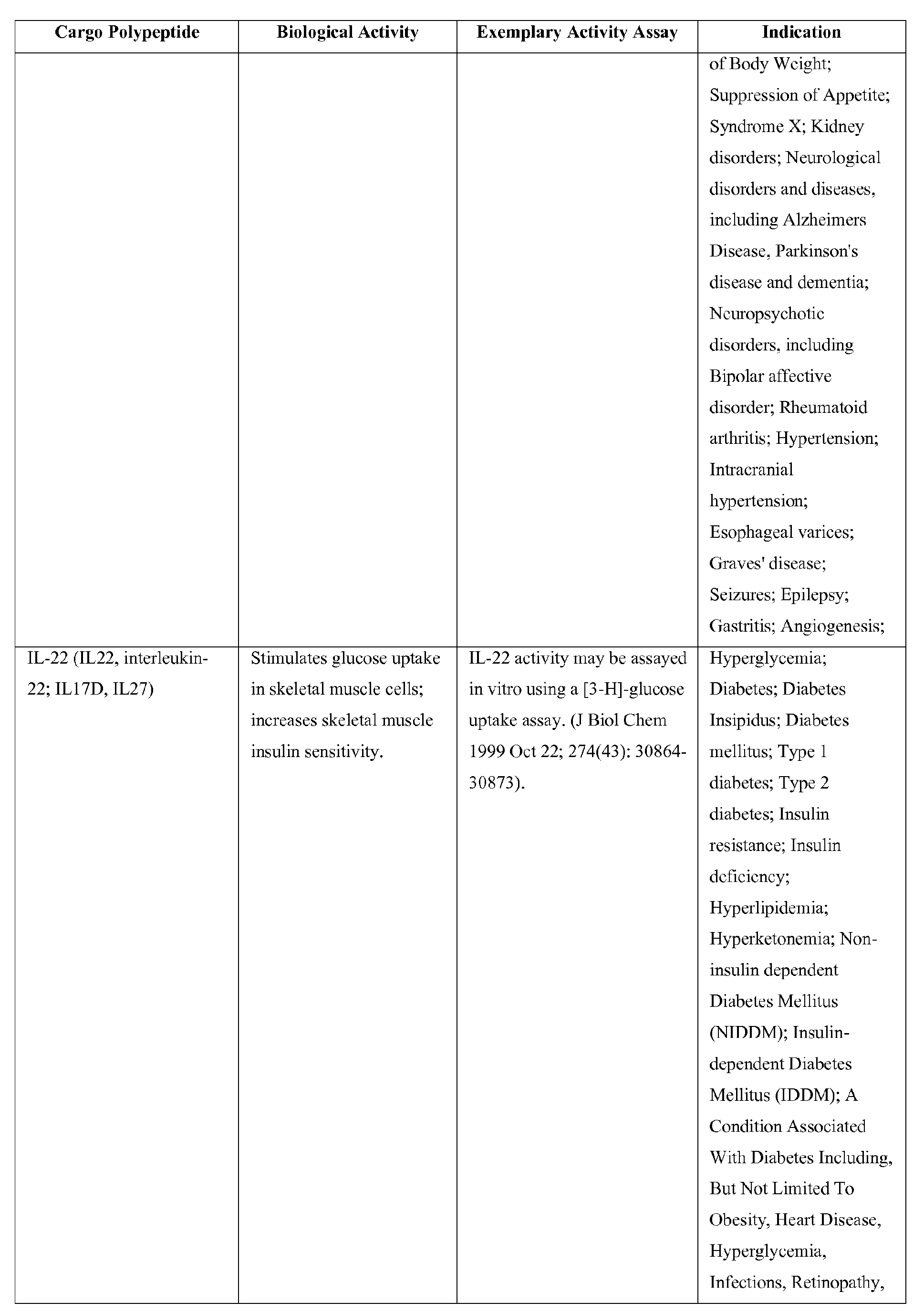

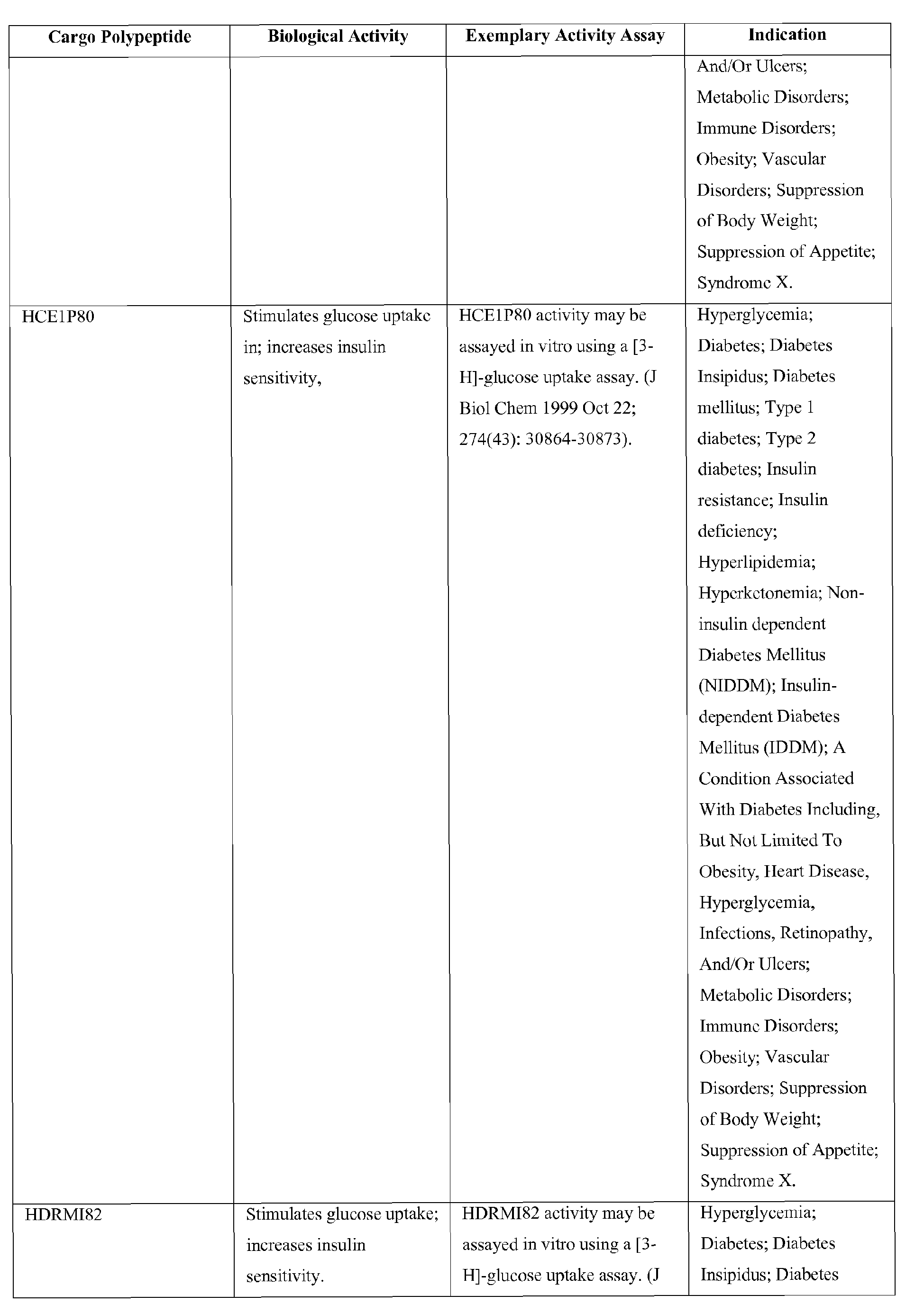

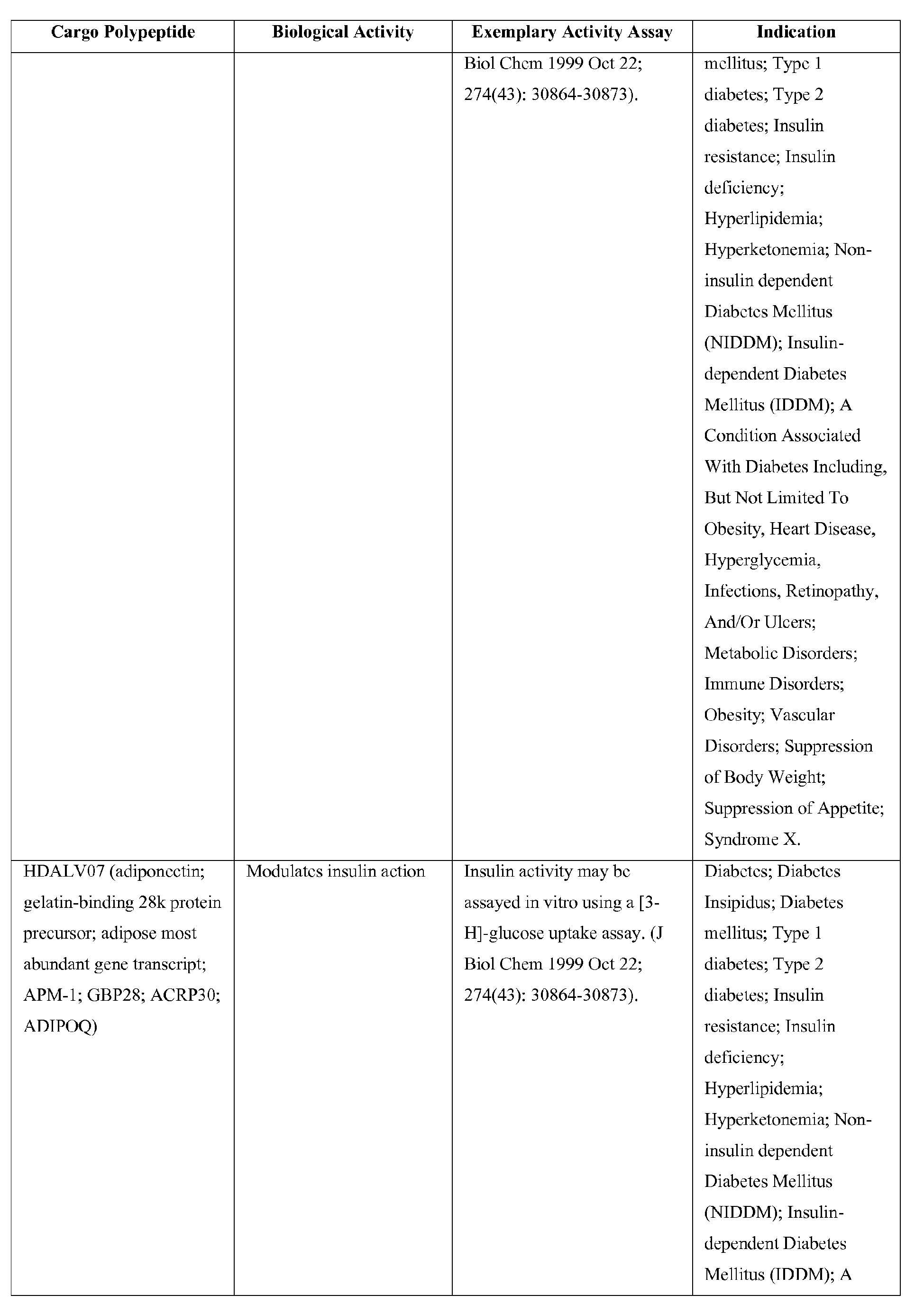

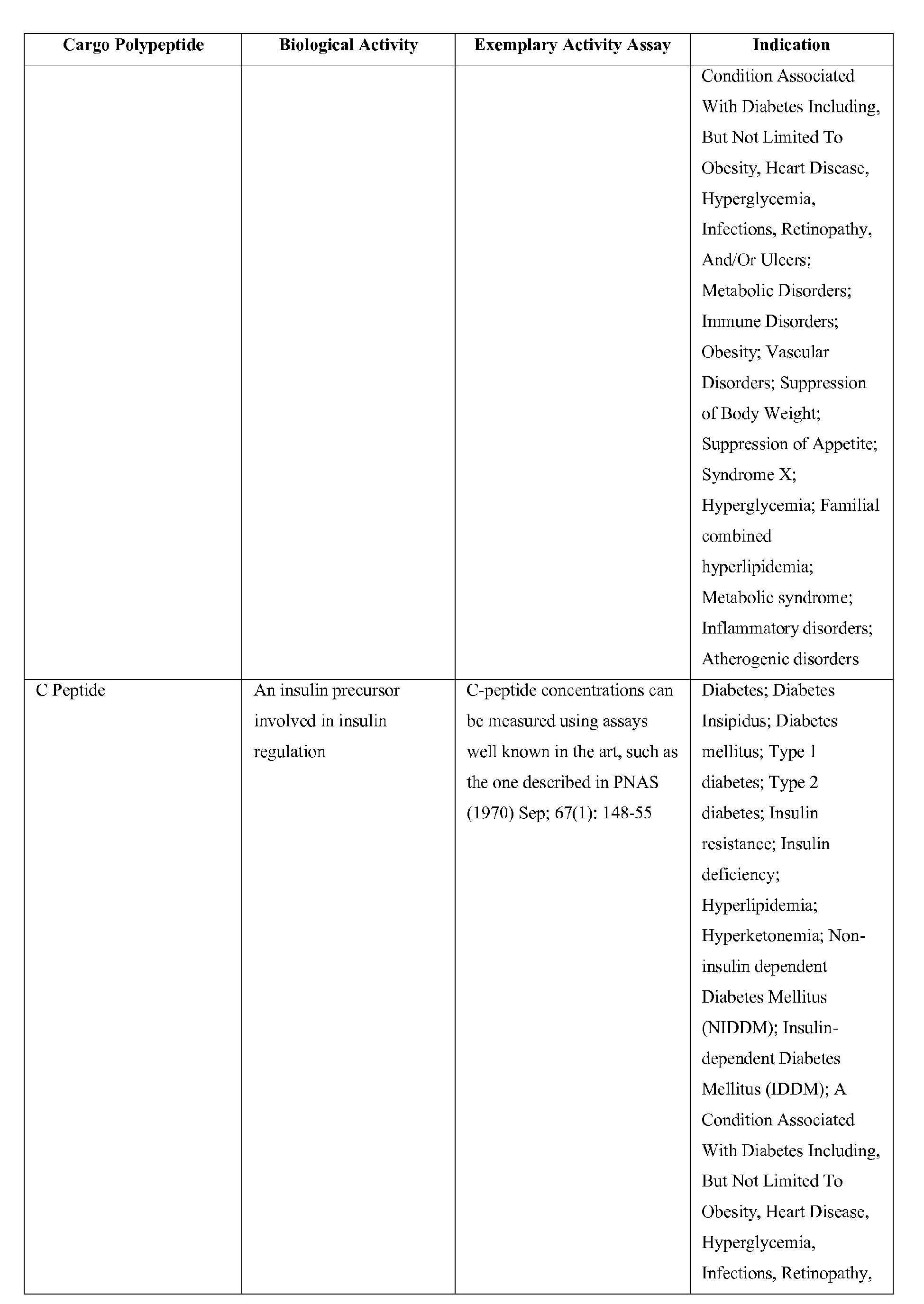

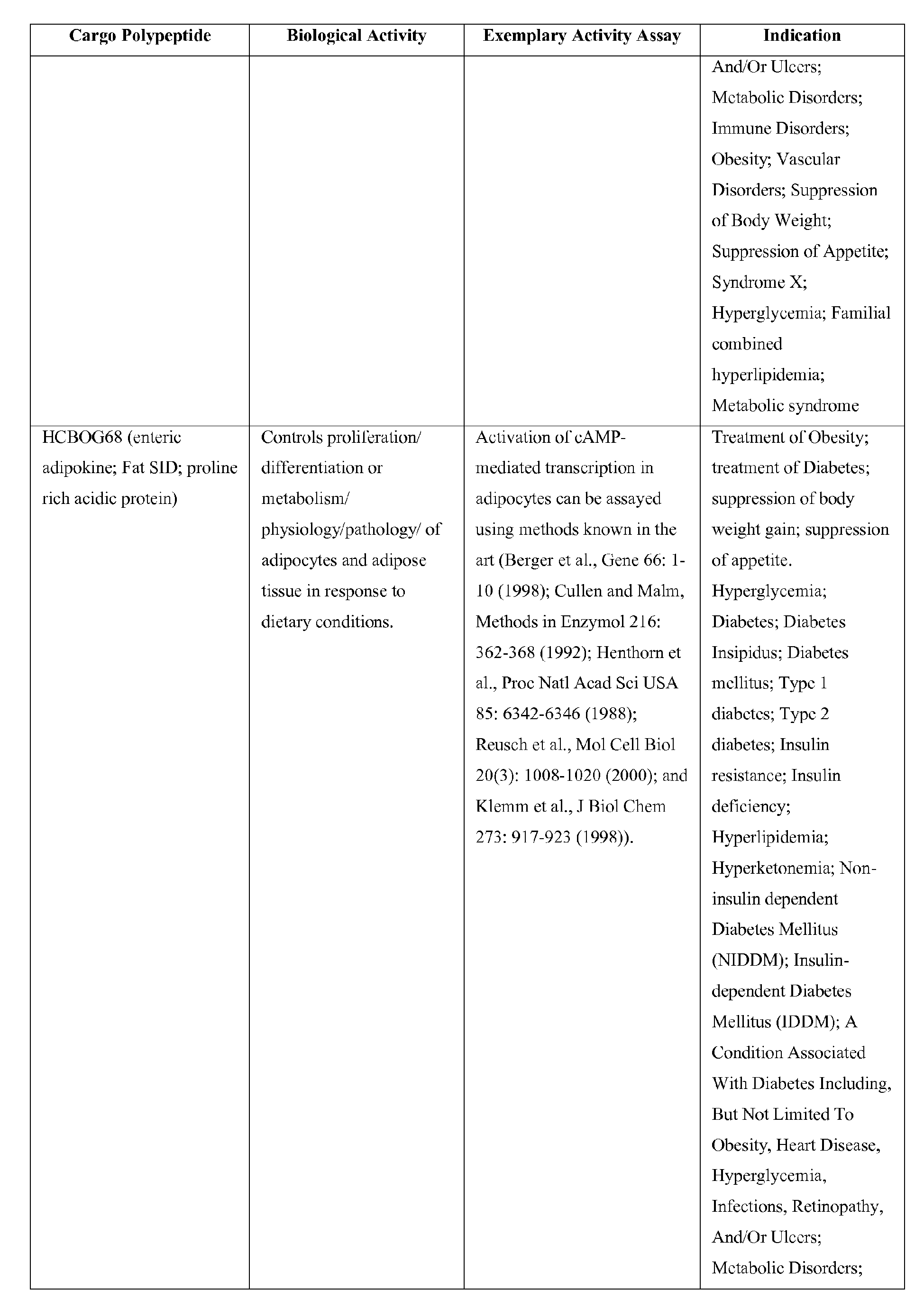

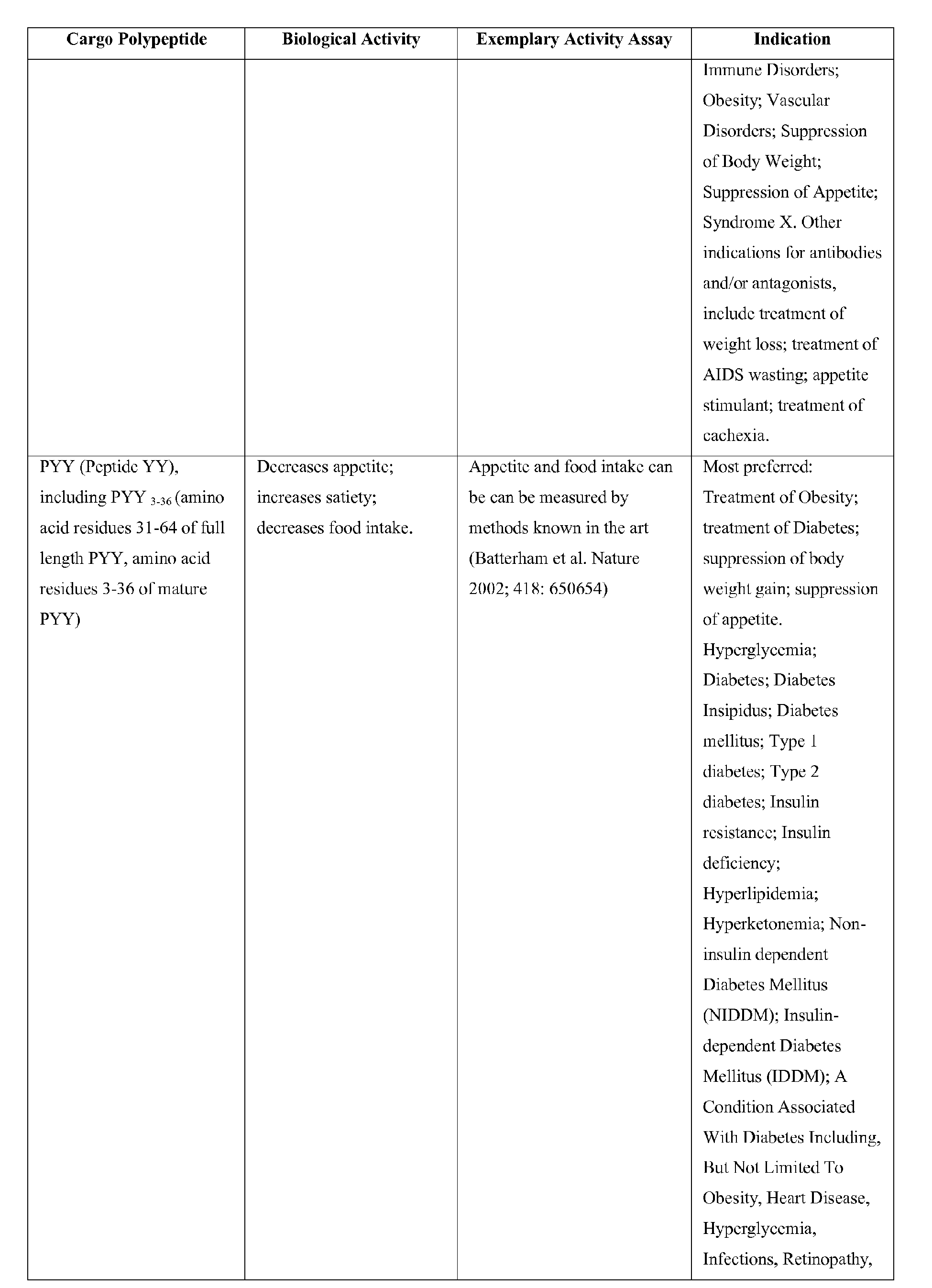

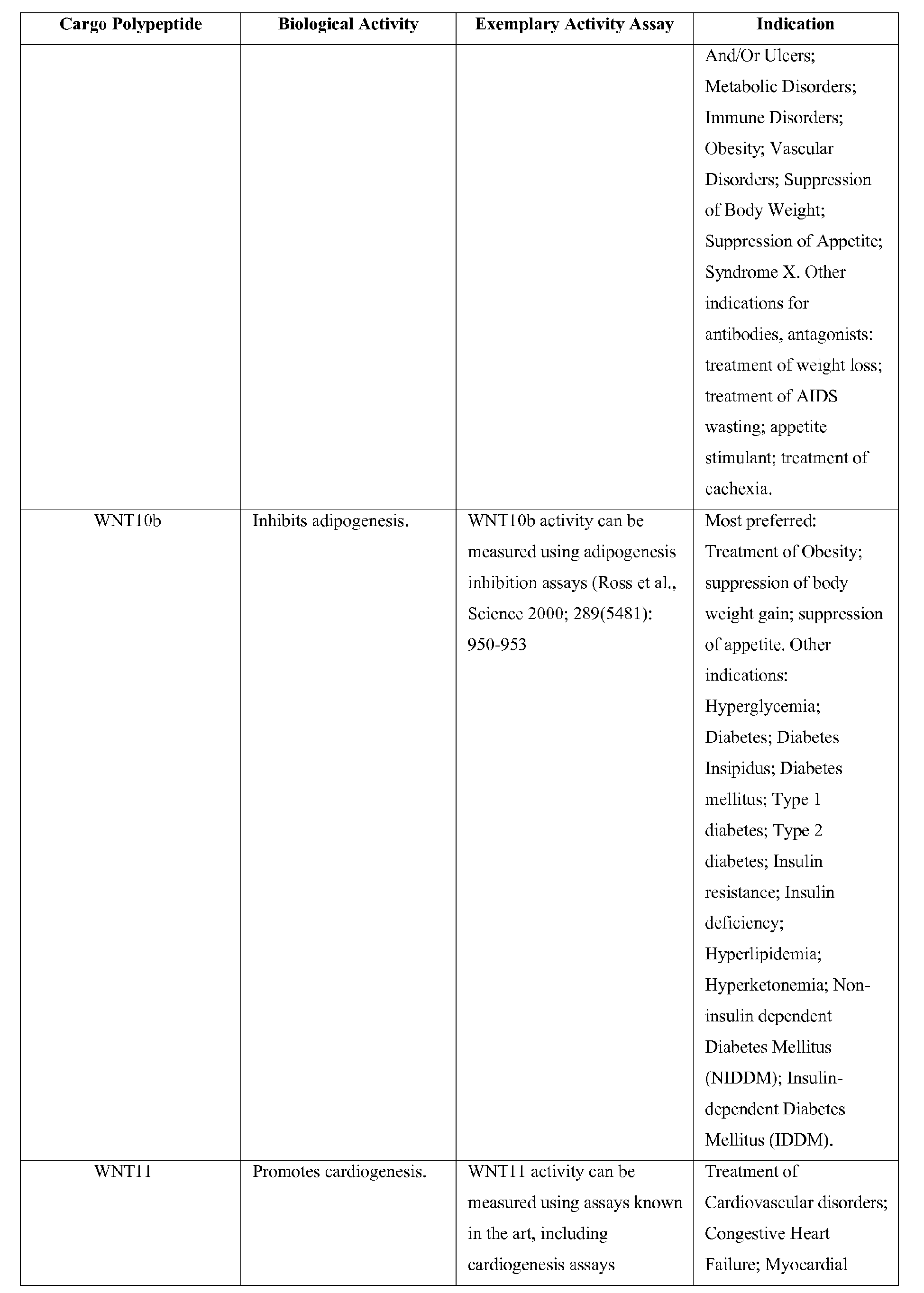

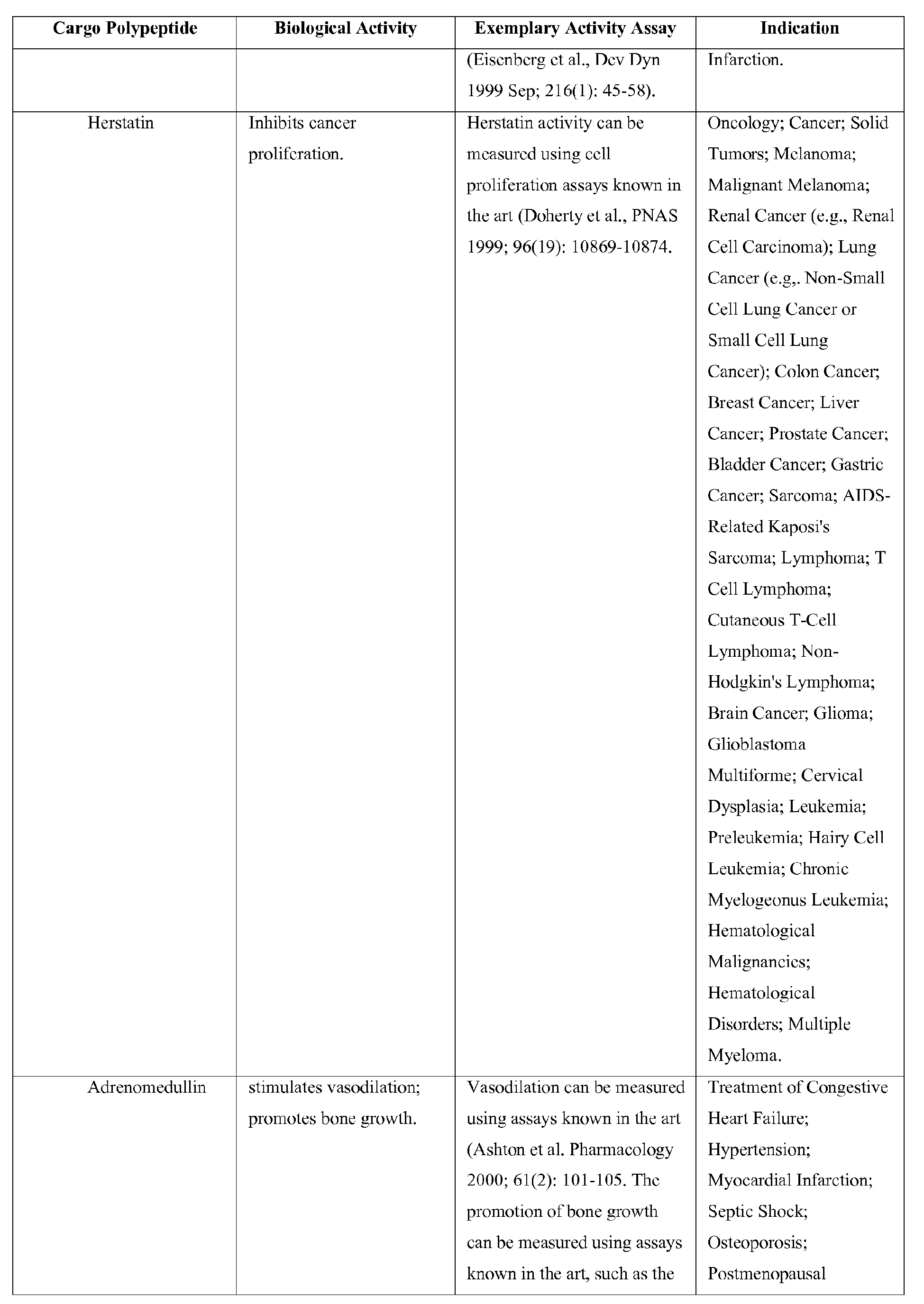

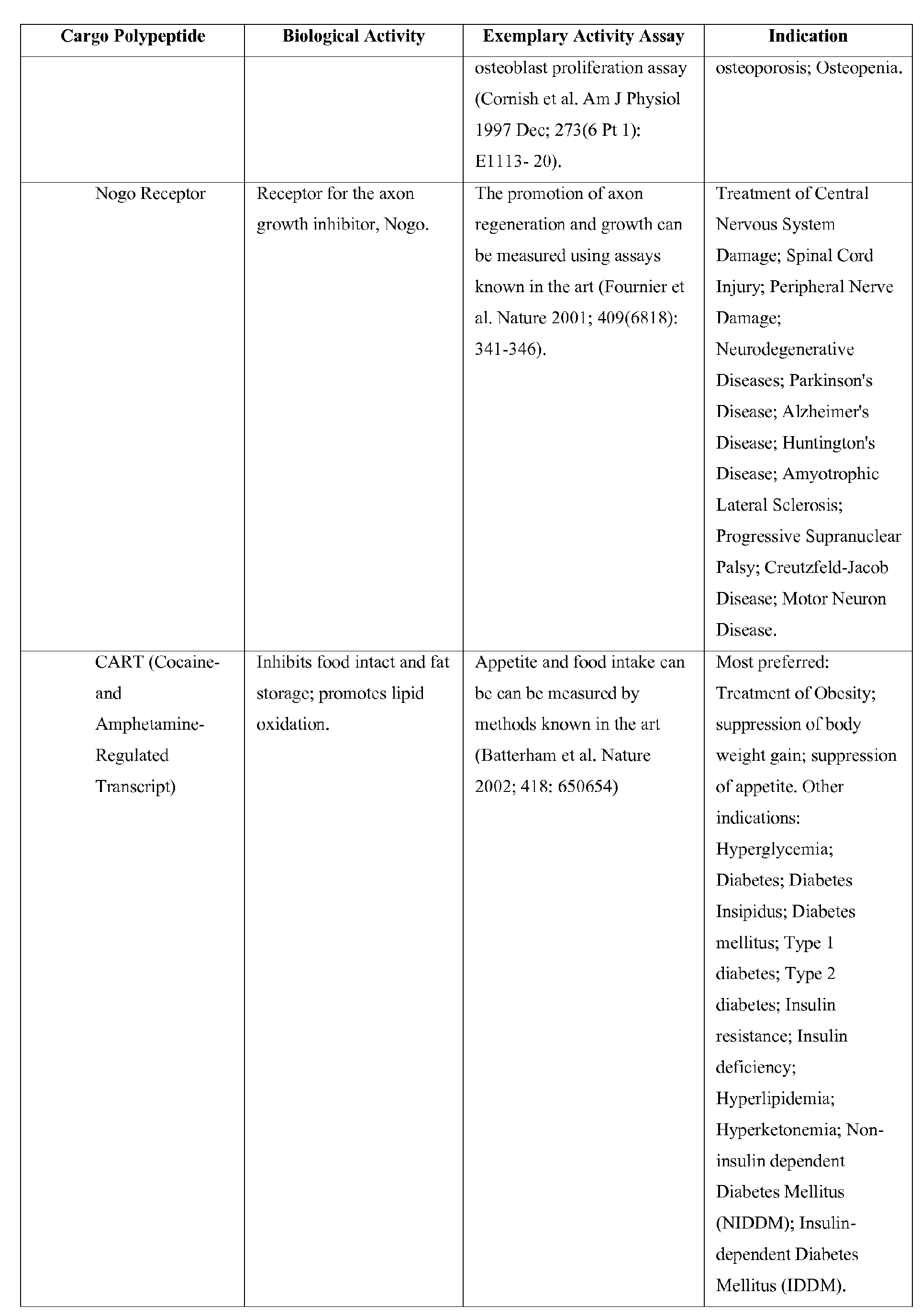

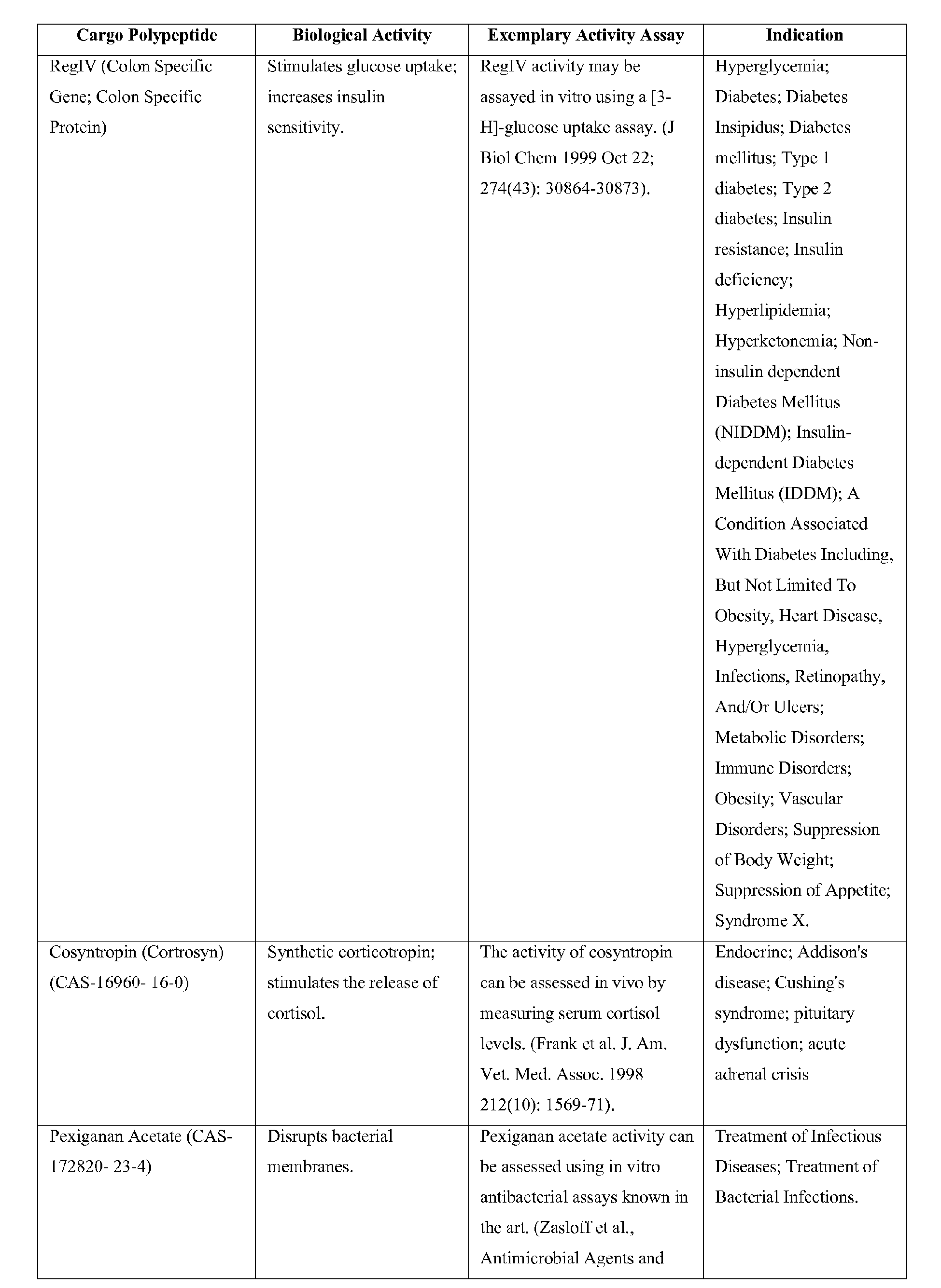

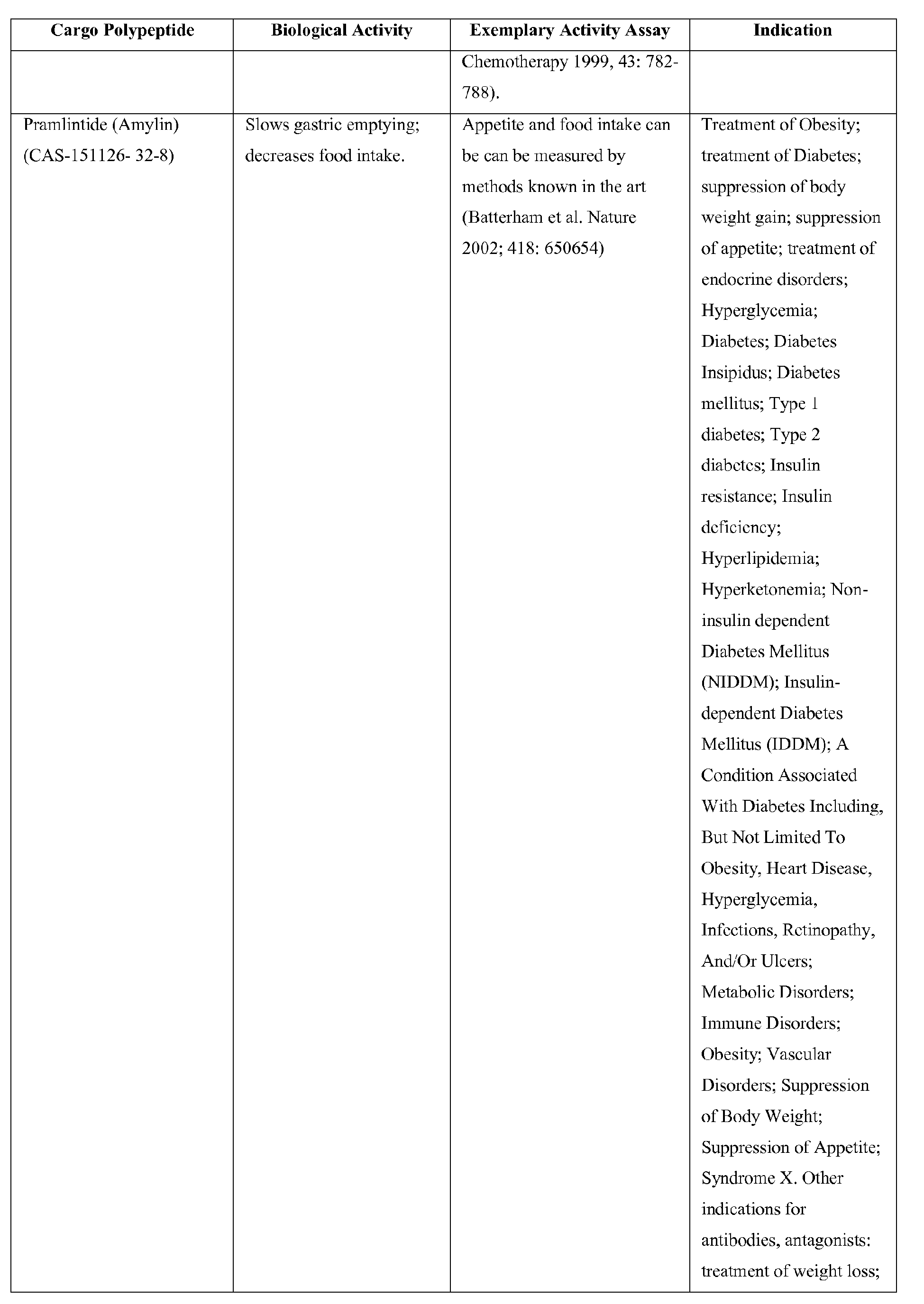

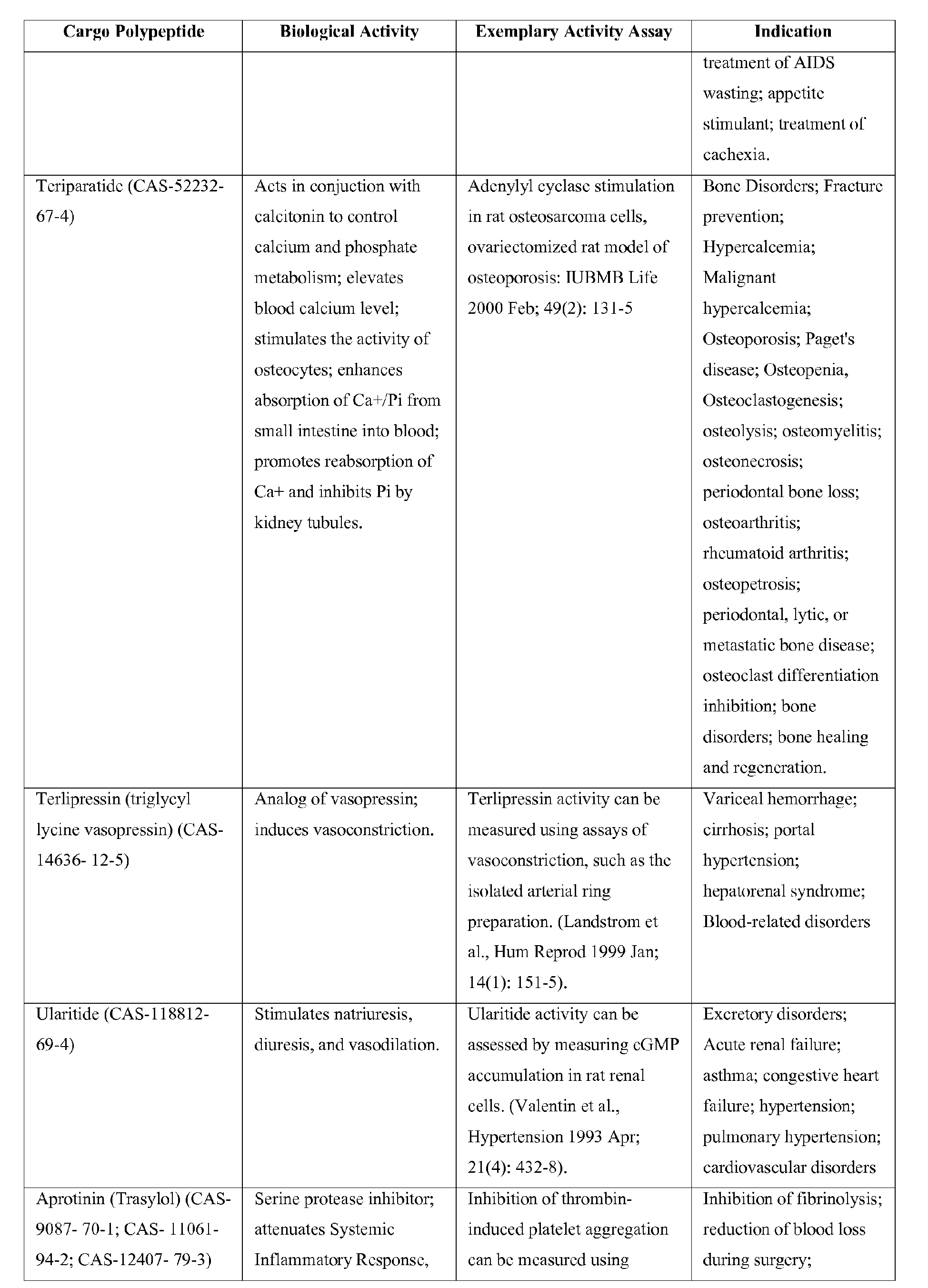

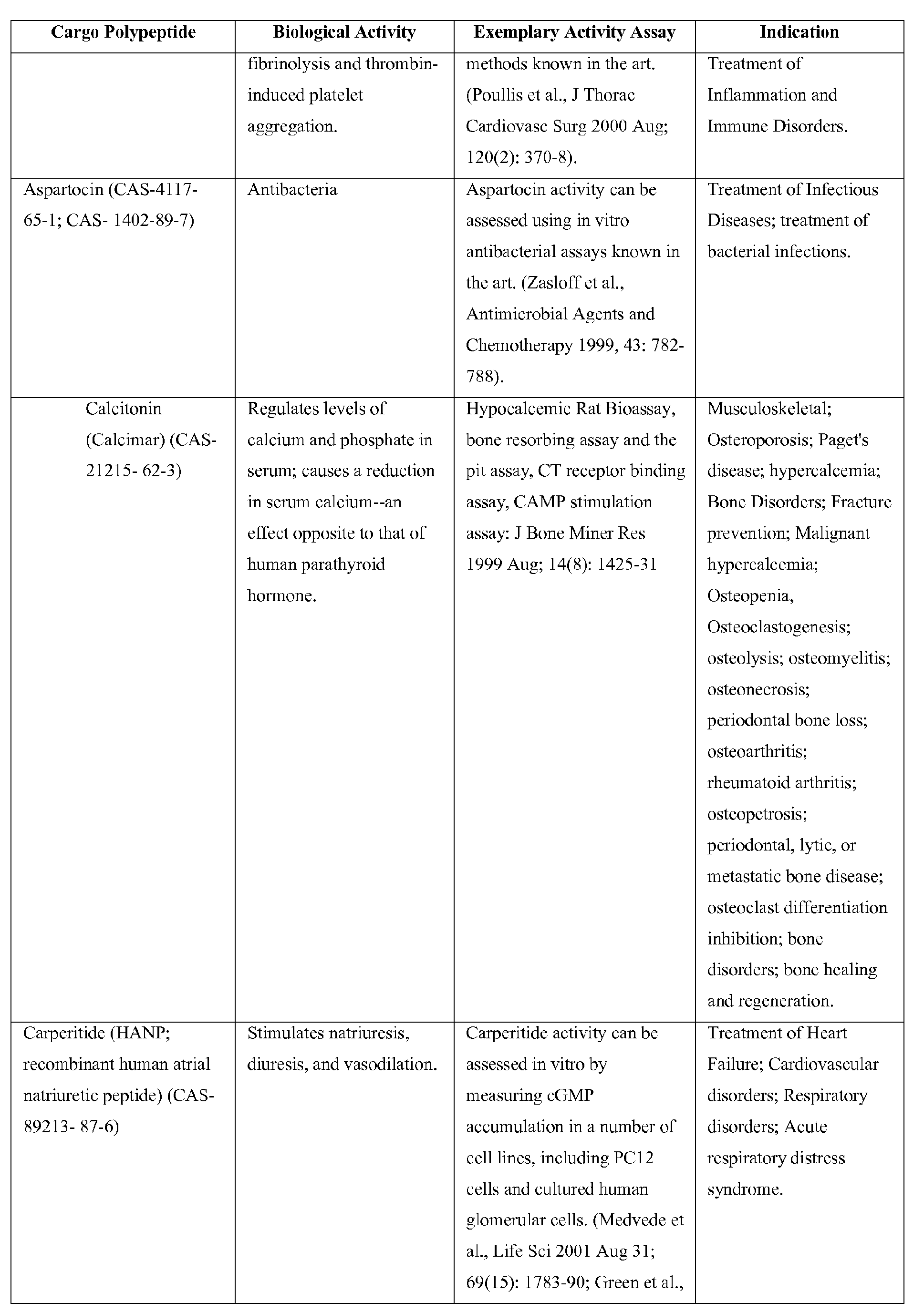

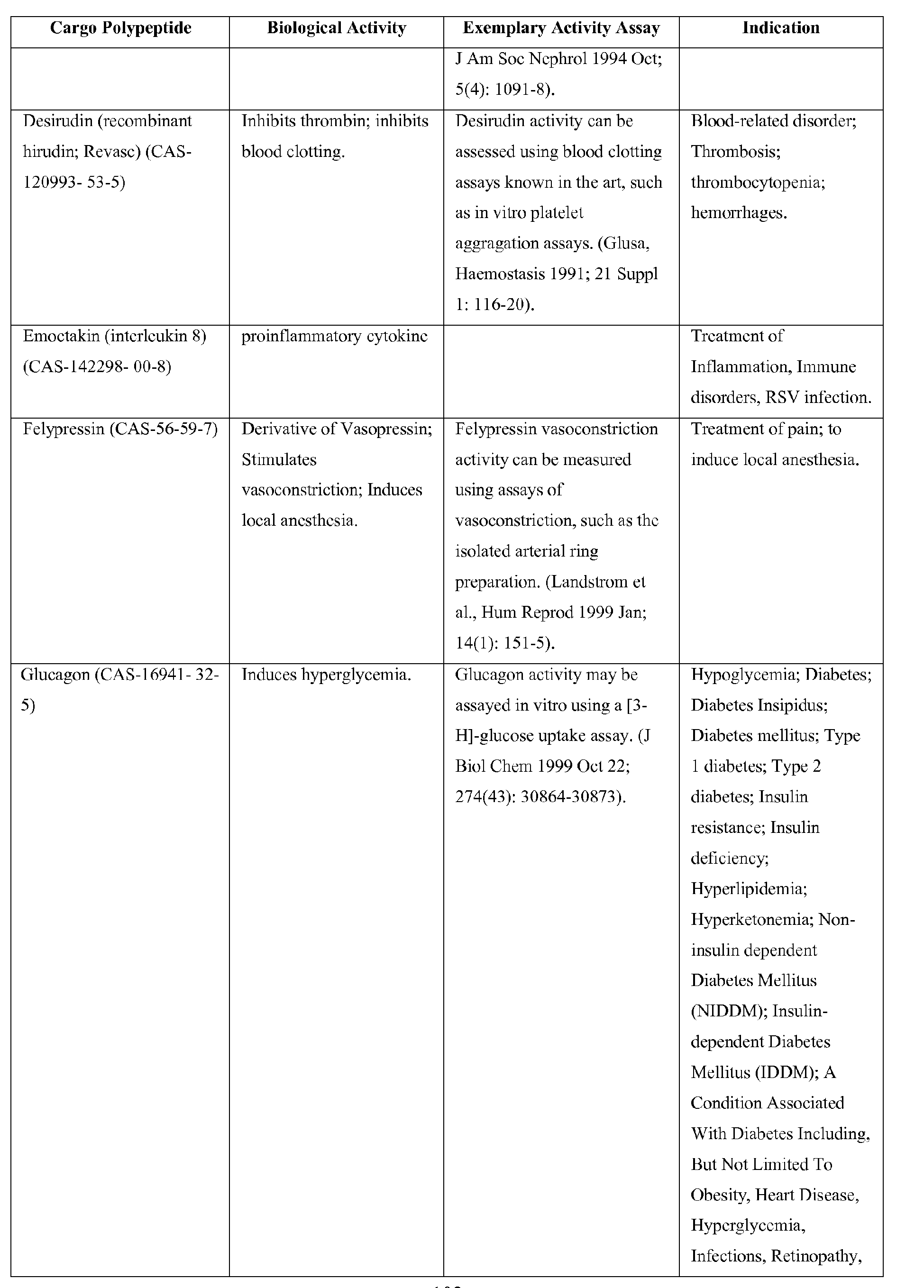

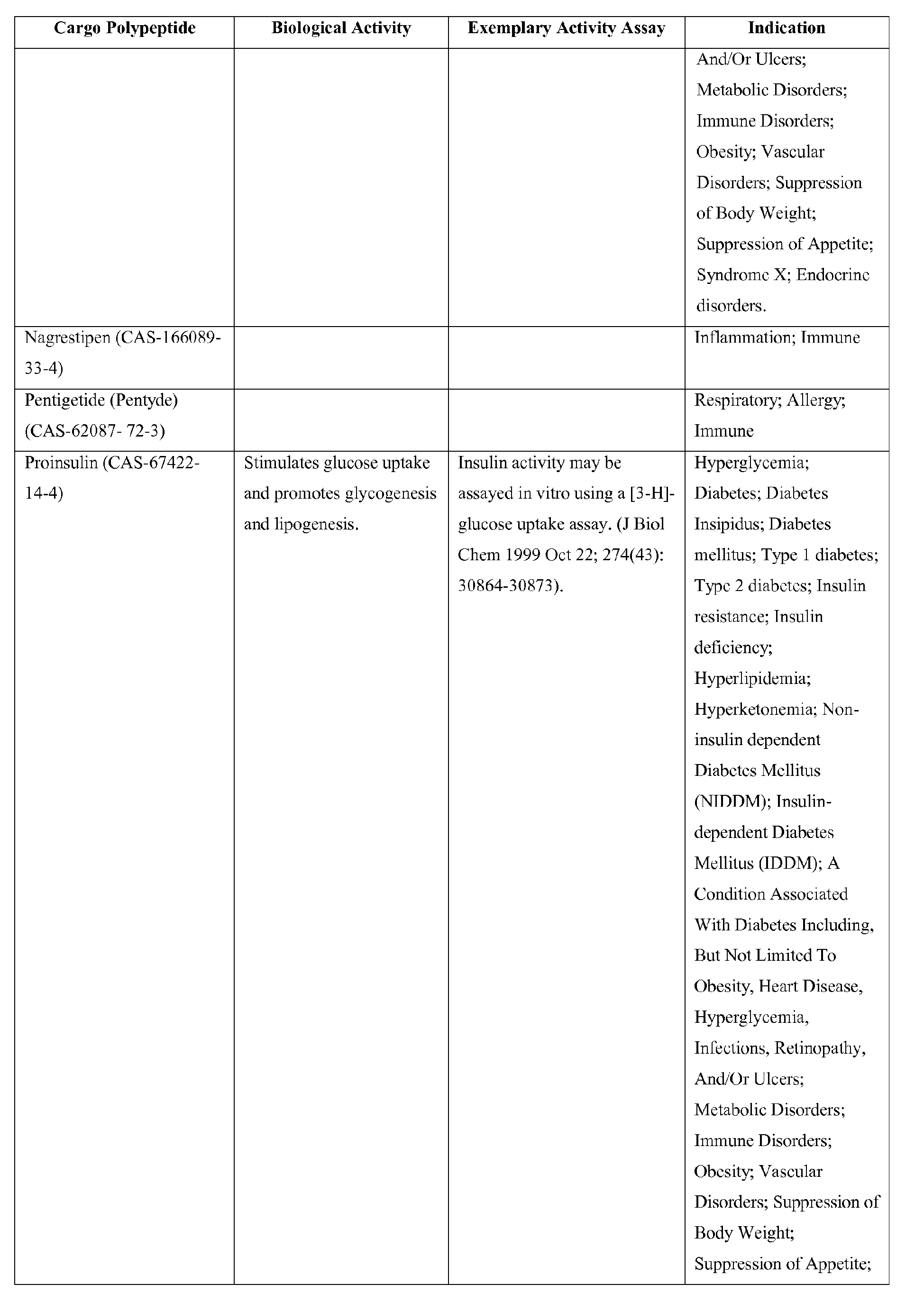

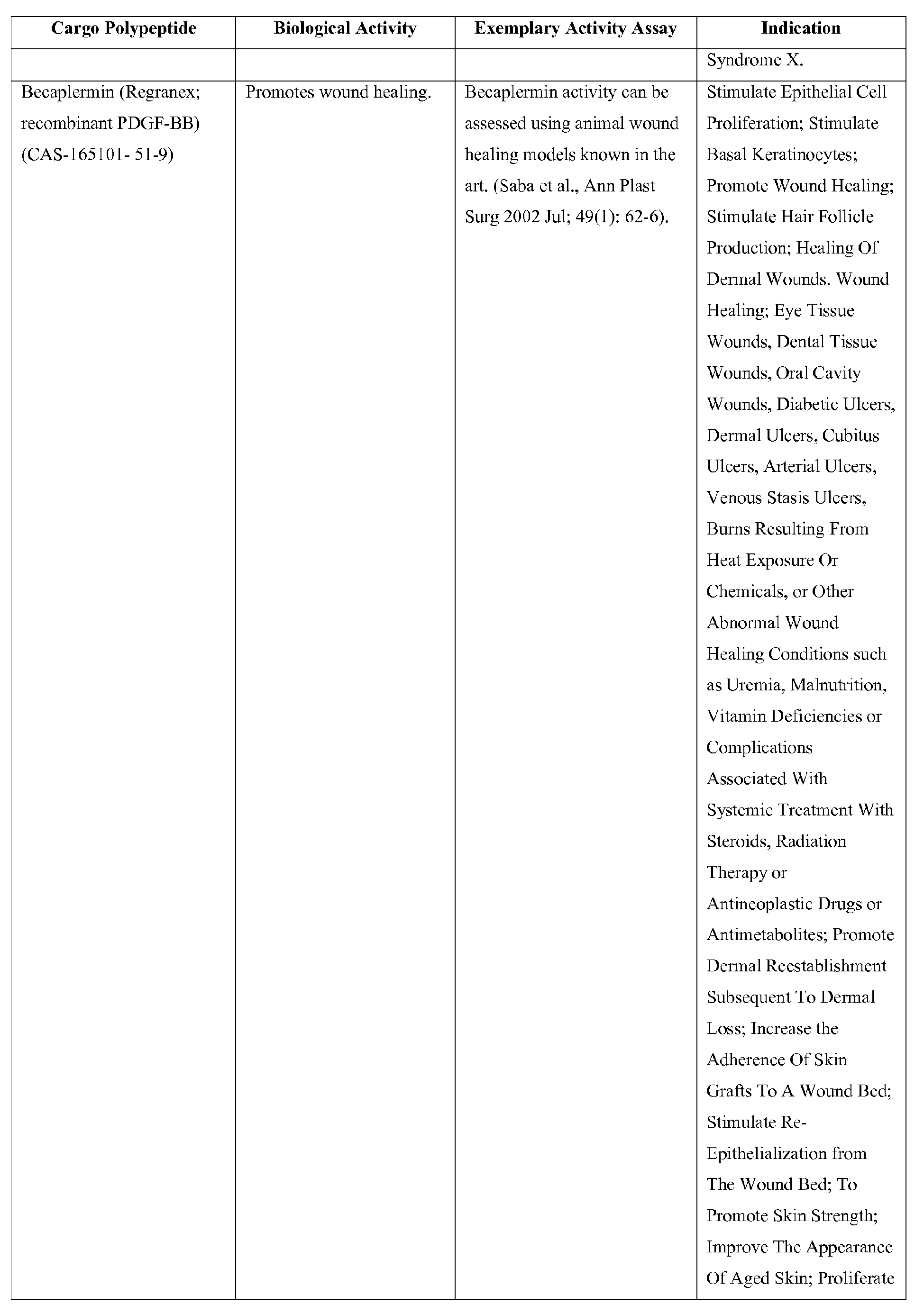

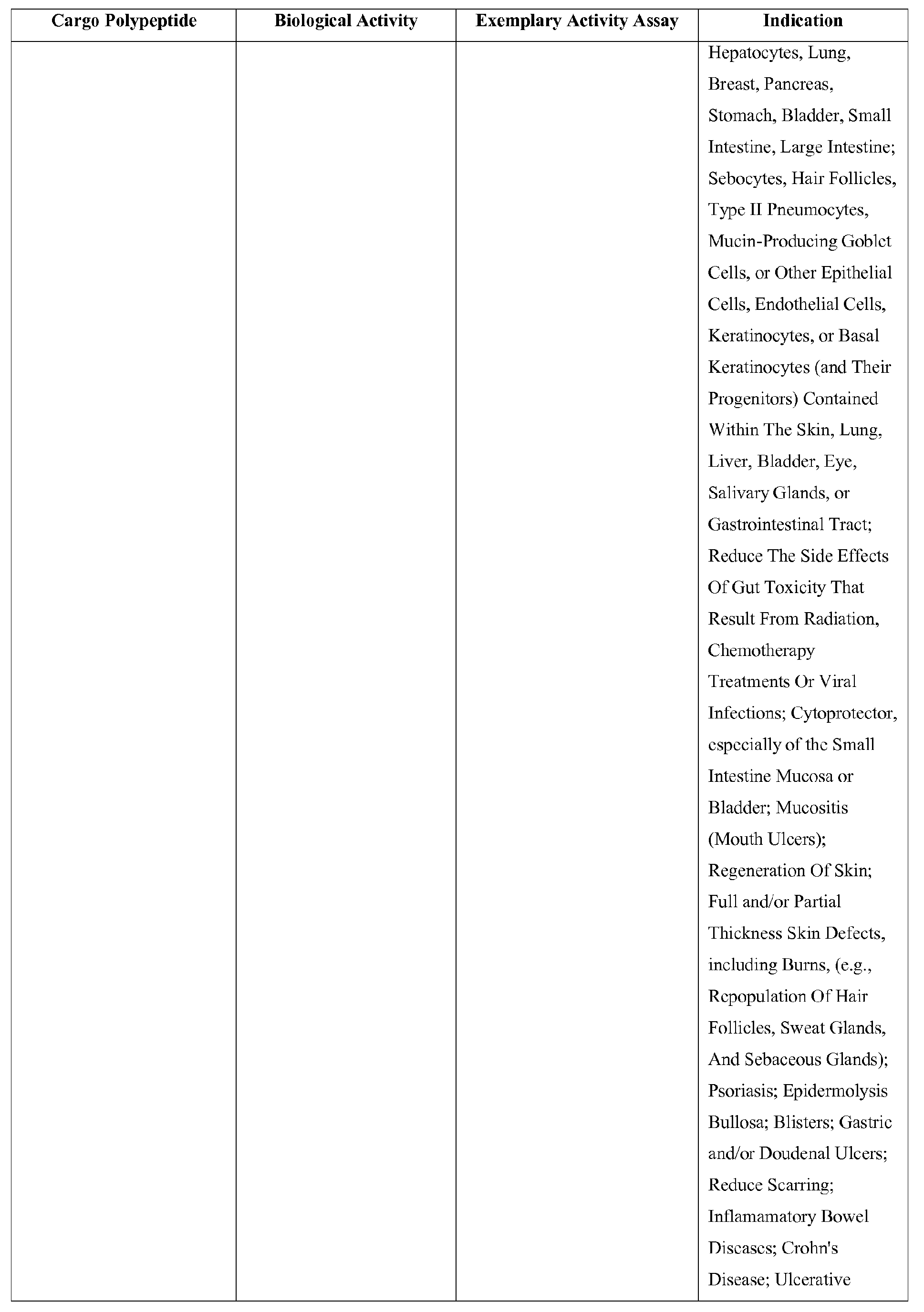

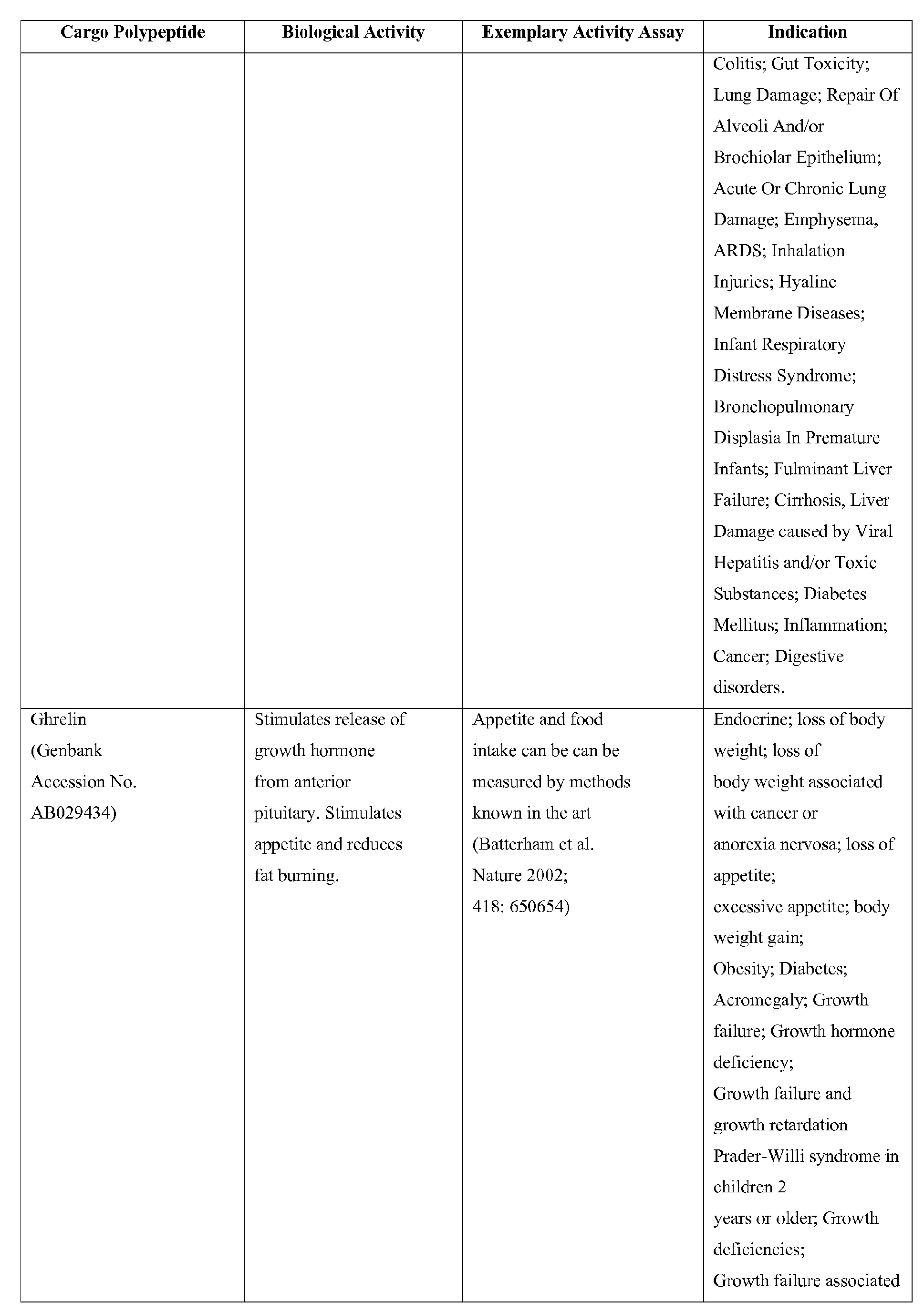

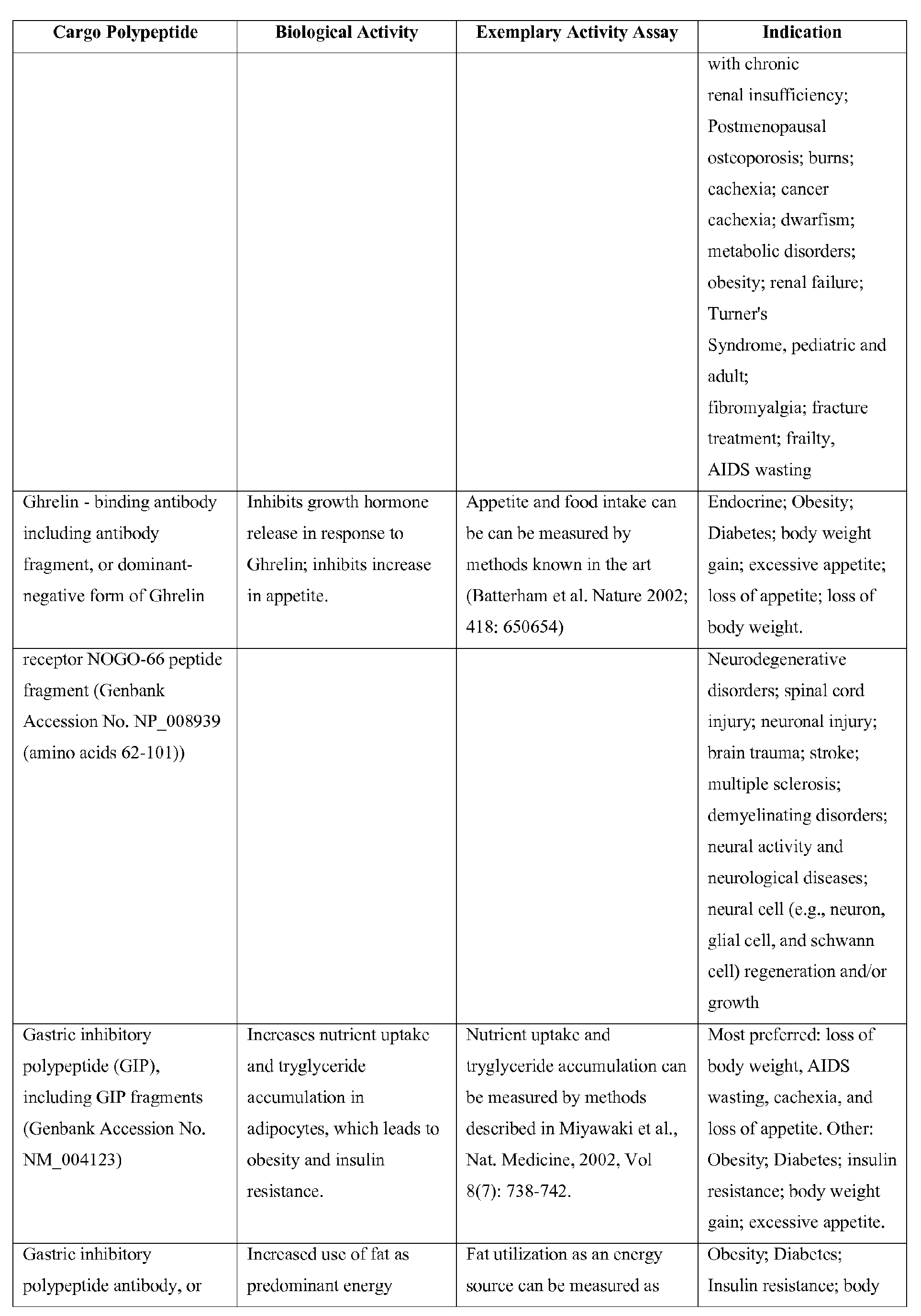

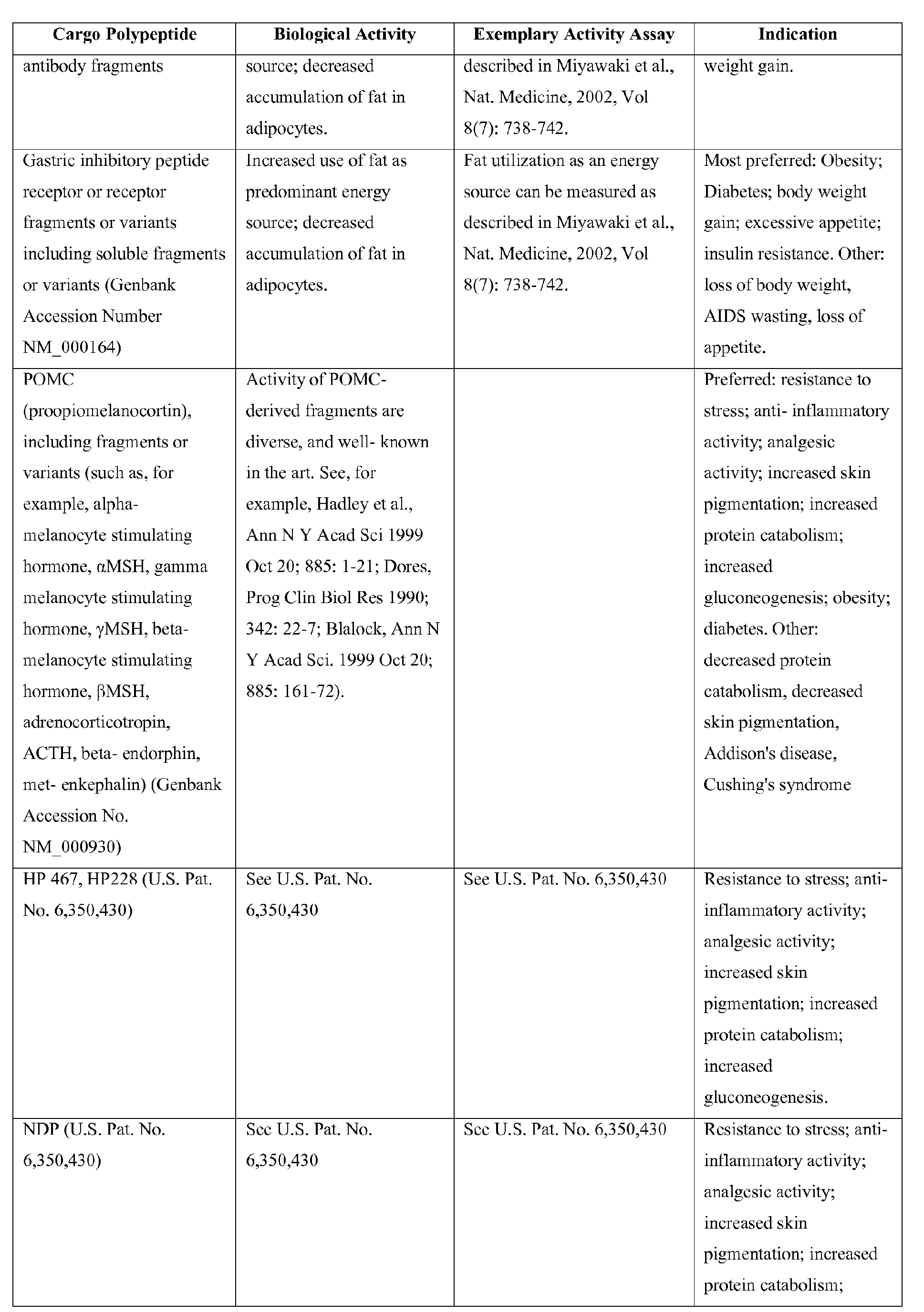

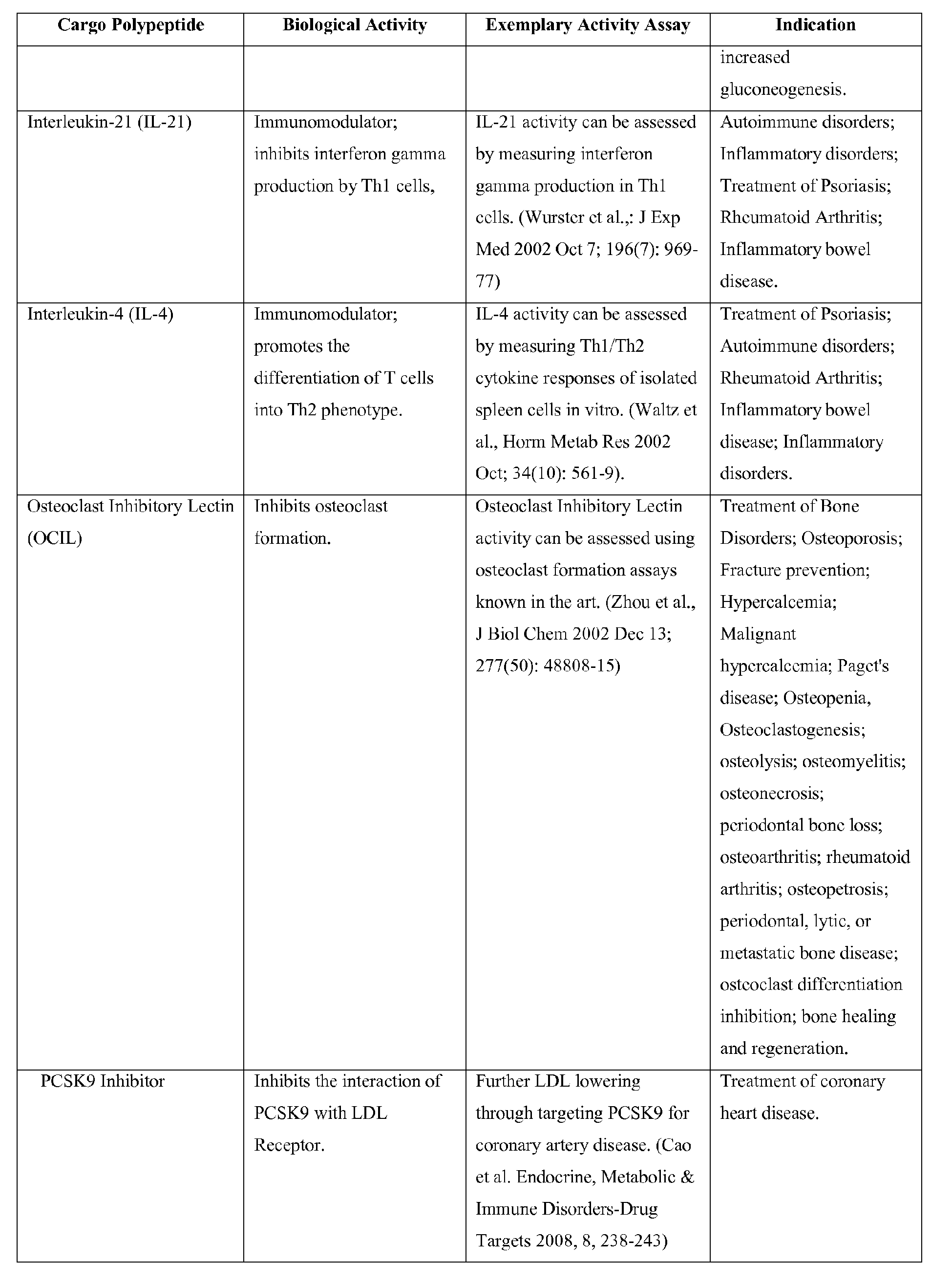

In one embodiment, the cargo polypeptide is an antigen binding polypeptide construct. In another embodiment, the cargo polypeptide is an antigen-binding polypeptide construct that is an antibody, or fragment or variant thereof. In one embodiment, the antigen-binding polypeptide construct is a Fab fragment of an antibody, or an scFv, or a single domain antibody or fragment thereof.

In one embodiment the cargo polypeptide is selected from an antigen-binding polypeptide construct that binds to CD3, CD19, CD20, HER2, or HER3. Suitable examples of antigen binding polypeptide constructs are known in the art and include those derived from anti-CD3, anti-CD 19, anti-HER2, and anti-HER3 antibodies.

### Functional Activity :

"A polypeptide having functional activity" refers to a polypeptide capable of displaying one or more known functional activities associated with the full-length, pro-protein, and/or mature form of a cargo polypeptide. Such functional activities include, but are not limited to, biological activity, antigenicity [ability to bind (or compete with a polypeptide for binding) to an anti-polypeptide antibody], immunogenicity (ability to generate antibody which binds to a specific polypeptide described herein), ability to form multimers with polypeptides described herein, and ability to bind to a receptor or ligand for a polypeptide. In certain embodiments, the functional activity includes the ability to improve the expression and stability of a partner protein.

"A polypeptide having biological activity" refers to a polypeptide exhibiting activity similar to, but not necessarily identical to, an activity of a therapeutic protein described herein, including mature forms, as measured in a particular biological assay, with or without dose dependency. In the case where dose dependency does exist, it need not be identical to that of the polypeptide, but rather substantially similar to the dose-dependence in a given activity as compared to the polypeptide described herein (i.e., the candidate polypeptide will exhibit greater activity or not more than about 25-fold less, or not more than about tenfold less activity, or not more than about three-fold less activity relative to a polypeptide described herein, or presented in Table 2).

In certain embodiments, a heteromultimer described herein has at least one biological and/or therapeutic activity associated with the cargo molecule when said cargo molecule is not linked to the transporter polypeptide. In certain embodiments, a heteromultimer described herein has at least one biological and/or therapeutic activity associated with the cargo polypeptide when said cargo polypeptide is not linked to the transporter polypeptide. In certain embodiments, a heteromultimeric protein described herein has at least one biological and/or therapeutic activity associated with the cargo polypeptide portion (or fragment or variant thereof) when said cargo polypeptide is not linked to the albumin or alloalbumin based polypeptide.

The heteromultimeric proteins described herein can be assayed for functional activity (e.g., biological activity) using or routinely modifying assays known in the art, as well as assays described herein. Additionally, one of skill in the art may routinely assay fragments of a protein corresponding to a cargo protein portion of an albumin or alloalbumin based monomeric polypeptide, for activity using assays referenced in its corresponding row of Table 2 (e.g., in column 3 of Table 2). In certain embodiments, are assay of fragments of an albumin protein corresponding to an albumin protein portion of a heteromultimer, for activity using assays known in the art and/or as described in the Examples section below.

For example, in one embodiment where one is assaying for the ability of a heteromultimeric protein described herein to bind or compete with a Cargo polypeptide for binding to an anti-Cargo polypeptide antibody and/or anti-albumin antibody, various immunoassays known in the art can be used, including but not limited to, competitive and non-competitive assay systems using techniques such as radioimmunoassays, ELISA (enzyme linked immunosorbent assay), "sandwich" immunoassays, immunoradiometric assays, gel diffusion precipitation reactions, immunodiffusion assays, in situ immunoassays (using colloidal gold, enzyme or radioisotope labels, for example), western blots, precipitation reactions, agglutination assays (e.g., gel agglutination assays, hemagglutination assays), complement fixation assays, immunofluorescence assays, protein A assays, and immunoelectrophoresis assays, etc. In one embodiment, antibody binding is detected by detecting a label on the primary antibody. In another embodiment, the primary antibody is detected by detecting binding of a secondary antibody or reagent to the primary antibody. In a further embodiment, the secondary antibody is labeled. Many means are known in the art for detecting binding in an immunoassay and are within the scope of the present invention.

In certain embodiments, where a binding partner (e.g., a receptor or a ligand) is identified for a cargo molecule comprised by a heteromultimer described herein, binding to that binding partner by a heteromultimer described herein is assayed, e.g., by means well-known in the art, such as, for example, reducing and non-reducing gel chromatography, protein affinity chromatography, and affinity blotting. See generally, Phizicky et al., Microbiol. Rev. 59:94-123 (1995). In another embodiment, the ability of physiological correlates of a heteromultimeric protein to bind to a substrate(s) of polypeptides corresponding to the cargo protein portion of the heteromultimer can be routinely assayed using techniques known in the art.

### Biological Activities

In certain embodiments, heteromultimers described herein, are used in assays to test for one or more biological activities. If a heteromultimer exhibits an activity in a particular assay, it is likely that at least one cargo protein comprised by one or more monomers of the heteromultimer is implicated in the diseases associated with the biological activity. Thus, the heteromultimer is of use in a treatment of the associated disease.

In certain embodiments, provided is a method of treating a disease or disorder comprising administering to a patient in which such treatment, prevention or amelioration is desired, a heteromultimer described herein, in an amount effective to treat, prevent or ameliorate the disease or disorder.

Provided herein are monomeric albumin or alloalbumin based fusion proteins produced by a cell, wherein said proteins are encoded by polynucleotides, wherein said monomeric proteins comprise at least one cargo protein, and an albumin or alloalbumin derived polypeptide, such that said monomers form heteromultimers in solution. In certain embodiments, when the polynucleotides are used to express the encoded protein from a cell, the cell's natural secretion and processing steps produces a protein that lacks at least one signal sequence. The specific amino acid sequence of the signal sequence is well known in the art.

In certain embodiments, heteromultimers described herein are used in the diagnosis, prognosis, prevention and/or treatment of diseases and/or disorders of the endocrine system. In some embodiments, heteromultimers described herein are used in the diagnosis, prognosis, prevention and/or treatment of diseases and/or disorders of the nervous system.

In certain embodiments, heteromultimers described herein are used in the diagnosis, prognosis, prevention and/or treatment of diseases and/or disorders of the immune system. In certain embodiments, heteromultimers described herein are used in the diagnosis, prognosis, prevention and/or treatment of diseases and/or disorders of the respiratory system.

In certain embodiments, heteromultimers described herein are used in the diagnosis, prognosis, prevention and/or treatment of diseases and/or disorders of the cardiovascular system. In some embodiments, heteromultimers described herein are used in the diagnosis, prognosis, prevention and/or treatment of diseases and/or disorders of the reproductive system.

In certain embodiments, heteromultimers described herein are used in the diagnosis, prognosis, prevention and/or treatment of diseases and/or disorders of the digestive system. In certain embodiments, heteromultimer proteins described herein are used in the diagnosis, prognosis, prevention and/or treatment of diseases or disorders relating to the blood.

In certain embodiments, heteromultimers described herein are used in the diagnosis and/or prognosis of diseases and/or disorders associated with at least one tissue(s) in which at least one gene of interest is expressed, wherein a heteromultimer described herein comprises a cargo molecule that binds said at least one gene of interest.

In some embodiments, heteromultimers described herein and/or polynucleotides encoding the albumin/alloalbumin based monomers that associate to form heteromultimers described herein, are used in the diagnosis, detection and/or treatment of diseases and/or disorders associated with activities that include, but are not limited to, prohormone activation, neurotransmitter activity, cellular signaling, cellular proliferation, cellular differentiation, and cell migration.

### Therapeutic Uses:

In an aspect, heteromultimers described herein are directed to antibody-based therapies which involve administering heteromultimers described comprising cargo polypeptide(s) which is an antibody, a fragment or variant of an antibody, to a patient for treating one or more of the disclosed diseases, disorders, or conditions. Therapeutic compounds described herein include, but are not limited to, heteromultimers described herein, nucleic acids encoding heteromultimers described herein.

In a specific embodiment, are antibody-based therapies which involve administering heteromultimers described herein comprising at least a fragment or variant of an antibody to a patient for treating one or more diseases, disorders, or conditions, including but not limited to: neural disorders, immune system disorders, muscular disorders, reproductive disorders, gastrointestinal disorders, pulmonary disorders, cardiovascular disorders, renal disorders, proliferative disorders, and/or cancerous diseases and conditions, and/or as described elsewhere herein.

A summary of the ways in which the heteromultimer proteins of the invention comprising at least a fragment or variant of an antibody are used therapeutically includes binding locally or systemically in the body or by direct cytotoxicity of the antibody, e.g. as mediated by complement (CDC) or by effector cells (ADCC). Some of these approaches are described in more detail below. Armed with the teachings provided herein, one of ordinary skill in the art will know how to use the heteromultimers described herein for diagnostic, monitoring or therapeutic purposes without undue experimentation.

The heteromultimers described herein, comprising at least a fragment or variant of an antibody may be administered alone or in combination with other types of treatments (e.g., radiation therapy, chemotherapy, hormonal therapy, immunotherapy and anti-tumor agents). Generally, administration of products of a species origin or species reactivity (in the case of antibodies) that is the same species as that of the patient is preferred. Thus, in an embodiment, human antibodies, fragments derivatives, analogs, or nucleic acids, are administered to a human patient for therapy or prophylaxis.

### Gene Therapy:

In a specific embodiment, nucleic acids comprising sequences encoding heteromultimer proteins described herein are administered to treat, inhibit or prevent a disease or disorder associated with aberrant expression and/or activity of a protein, by way of gene therapy. Gene therapy refers to therapy performed by the administration to a subject of an expressed or expressible nucleic acid. In this embodiment of the invention, the nucleic acids produce their encoded protein that mediates a therapeutic effect. Any of the methods for gene therapy available in the art can be used.

### Demonstration of Therapeutic or Prophylactic Activity:

The heteromultimers or pharmaceutical compositions described herein are tested in vitro, and then in vivo for the desired therapeutic or prophylactic activity, prior to use in humans. For example, in vitro assays to demonstrate the therapeutic or prophylactic utility of a compound or pharmaceutical composition include, the effect of a compound on a cell line or a patient tissue sample. The effect of the compound or composition on the cell line and/or tissue sample can be determined utilizing techniques known to those of skill in the art including, but not limited to, rosette formation assays and cell lysis assays. In accordance with the invention, in vitro assays which can be used to determine whether administration of a specific compound is indicated, include in vitro cell culture assays in which a patient tissue sample is grown in culture, and exposed to or otherwise administered a heteromultimer, and the effect of such heteromultimer upon the tissue sample is observed.

### Therapeutic/Prophylactic Administration and Composition

Provided are methods of treatment, inhibition and prophylaxis by administration to a subject of an effective amount of a heteromultimer or pharmaceutical composition described herein. In an embodiment, the heteromultimer is substantially purified (e.g., substantially free from substances that limit its effect or produce undesired side-effects). In certain embodiments, the subject is an animal, including but not limited to animals such as cows, pigs, horses, chickens, cats, dogs, etc., and in certain embodiments, a mammal, and most preferably human.

Various delivery systems are known and can be used to administer a heteromultimer formulation described herein, e.g., encapsulation in liposomes, microparticles, microcapsules, recombinant cells capable of expressing the compound, receptor-mediated endocytosis (see, e.g., Wu and Wu, J. Biol. Chem. 262:4429-4432 (1987)), construction of a nucleic acid as part of a retroviral or other vector, etc. Methods of introduction include but are not limited to intradermal, intramuscular, intraperitoneal, intravenous, subcutaneous, intranasal, epidural, and oral routes. The compounds or compositions may be administered by any convenient route, for example by infusion or bolus injection, by absorption through epithelial or mucocutaneous linings (e.g., oral mucosa, rectal and intestinal mucosa, etc.) and may be administered together with other biologically active agents. Administration can be systemic or local. In addition, in certain embodiments, it is desirable to introduce the heteromultimer compositions described herein into the central nervous system by any suitable route, including intraventricular and intrathecal injection; intraventricular injection may be facilitated by an intraventricular catheter, for example, attached to a reservoir, such as an Ommaya reservoir. Pulmonary administration can also be employed, e.g., by use of an inhaler or nebulizer, and formulation with an aerosolizing agent.

In a specific embodiment, it is desirable to administer the heteromultimers, or compositions described herein locally to the area in need of treatment; this may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, non-porous, or gelatinous material, including membranes, such as sialastic membranes, or fibers. Preferably, when administering a protein, including an antibody, of the invention, care must be taken to use materials to which the protein does not absorb.

In another embodiment, the heteromultimers or composition can be delivered in a vesicle, in particular a liposome (see Langer, Science 249:1527-1533 (1990); Treat et al., in Liposomes in the Therapy of Infectious Disease and Cancer, Lopez-Berestein and Fidler (eds.), Liss, New York, pp. 353-365 (1989); Lopez-Berestein, ibid., pp. 317-327; see generally ibid.)

In yet another embodiment, the heteromultimers or composition can be delivered in a controlled release system. In one embodiment, a pump may be used (see Langer, supra; Sefton, CRC Crit. Ref. Biomed. Eng. 14:201 (1987); Buchwald et al., Surgery 88:507 (1980); Saudek et al., N. Engl. J. Med. 321:574 (1989)). In another embodiment, polymeric materials can be used (see Medical Applications of Controlled Release, Langer and Wise (eds.), CRC Pres., Boca Raton, Fla. (1974); Controlled Drug Bioavailability, Drug Product Design and Performance, Smolen and Ball (eds.), Wiley, New York (1984); Ranger and Peppas, J., Macromol. Sci. Rev. Macromol. Chem. 23:61 (1983); see also Levy et al., Science 228:190 (1985); During et al., Ann. Neurol. 25:351 (1989); Howard et al., J. Neurosurg. 71:105 (1989)). In yet another embodiment, a controlled release system can be placed in proximity of the therapeutic target, e.g., the brain, thus requiring only a fraction of the systemic dose (see, e.g., Goodson, in Medical Applications of Controlled Release, supra, vol. 2, pp. 115-138 (1984)).

Other controlled release systems are discussed in the review by Langer (Science 249:1527-1533 (1990)).

In a specific embodiment comprising a nucleic acid encoding a heteromultimer decribed herein, the nucleic acid can be administered in vivo to promote expression of its encoded protein, by constructing it as part of an appropriate nucleic acid expression vector and administering it so that it becomes intracellular, e.g., by use of a retroviral vector (see U.S. Pat. No. 4,980,286), or by direct injection, or by use of microparticle bombardment (e.g., a gene gun; Biolistic, Dupont), or coating with lipids or cell-surface receptors or transfecting agents, or by administering it in linkage to a homeobox-like peptide which is known to enter the nucleus (see e.g., Joliot et al., Proc. Natl. Acad. Sci. USA 88:1864-1868 (1991)), etc. Alternatively, a nucleic acid can be introduced intracellularly and incorporated within host cell DNA for expression, by homologous recombination.

Also provided herein are pharmaceutical compositions. Such compositions comprise a therapeutically effective amount of a compound, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, such as peanut oil, soybean oil, mineral oil, sesame oil and the like. Water is a preferred carrier when the pharmaceutical composition is administered intravenously. Saline solutions and aqueous dextrose and glycerol solutions can also be employed as liquid carriers, particularly for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

In certain embodiments, the composition comprising the heteromultimer is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lignocaine to ease pain at the site of the injection. Generally, the ingredients are supplied either separately or mixed together in unit dosage form, for example, as a dry lyophilized powder or water free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water for injection or saline can be provided so that the ingredients may be mixed prior to administration.

In certain embodiments, the compositions described herein are formulated as neutral or salt forms. Pharmaceutically acceptable salts include those formed with anions such as those derived from hydrochloric, phosphoric, acetic, oxalic, tartaric acids, etc., and those formed with cations such as those derived from sodium, potassium, ammonium, calcium, ferric hydroxide isopropylamine, triethylamine, 2-ethylamino ethanol, histidine, procaine, etc.

The amount of the composition described herein which will be effective in the treatment, inhibition and prevention of a disease or disorder associated with aberrant expression and/or activity of a Therapeutic protein can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. Effective doses are extrapolated from dose-response curves derived from in vitro or animal model test systems.

### Methods of Recombinant and Synthetic Production of Heteromultimer Proteins:

In certain embodiments are heteromultimers produced as recombinant molecules by secretion from yeast, a microorganism such as a bacterium, or a human or animal cell line. In embodiments, the polypeptides are secreted from the host cells.

Embodiments include a cell, such as a yeast cell transformed to express a heteromultimer protein described herein. In addition to the transformed host cells themselves, are provided culture of those cells, preferably a monoclonal (clonally homogeneous) culture, or a culture derived from a monoclonal culture, in a nutrient medium. If the polypeptide is secreted, the medium will contain the polypeptide, with the cells, or without the cells if they have been filtered or centrifuged away. Many expression systems are known and may be used, including bacteria (for example E. coli and Bacillus subtilis), yeasts (for example Saccharomyces cerevisiae, Kluyveromyces lactis and Pichia pastoris, filamentous fungi (for example Aspergillus), plant cells, animal cells and insect cells.

A heteromultimer described herein is produced in conventional ways, for example from a coding sequence inserted in the host chromosome or on a free plasmid. The yeasts are transformed with a coding sequence for the desired protein in any of the usual ways, for example electroporation. Methods for transformation of yeast by electroporation are disclosed in Becker & Guarente (1990) Methods Enzymol. 194, 182.

Successfully transformed cells, i.e., cells that contain a DNA construct of the present invention, can be identified by well known techniques. For example, cells resulting from the introduction of an expression construct can be grown to produce the desired polypeptide. Cells can be harvested and lysed and their DNA content examined for the presence of the DNA using a method such as that described by Southern (1975) J. Mol. Biol. 98, 503 or Berent et al. (1985) Biotech. 3, 208. Alternatively, the presence of the protein in the supernatant can be detected using antibodies.

Useful yeast plasmid vectors include pRS403-406 and pRS413-416 and are generally available from Stratagene Cloning Systems, La Jolla, Calif. 92037, USA. Plasmids pRS403, pRS404, pRS405 and pRS406 are Yeast Integrating plasmids (YIps) and incorporate the yeast selectable markers HIS3, 7RP1, LEU2 and URA3. Plasmids pRS413-416 are Yeast Centromere plasmids (Ycps).

A variety of methods have been developed to operably link DNA to vectors via complementary cohesive termini. For instance, complementary homopolymer tracts can be added to the DNA segment to be inserted to the vector DNA. The vector and DNA segment are then joined by hydrogen bonding between the complementary honmopolymeric tails to form recombinant DNA molecules.

Synthetic linkers containing one or more restriction sites provide an alternative method of joining the DNA segment to vectors. The DNA segment, generated by endonuclease restriction digestion, is treated with bacteriophage T4 DNA polymerase or E. coli DNA polymerase 1, enzymes that remove protruding, -single-stranded termini with their 3' 5'-exonucleolytic activities, and fill in recessed 3'-ends with their polymerizing activities.

The combination of these activities therefore generates blunt-ended DNA segments. The blunt-ended segments are then incubated with a large molar excess of linker molecules in the presence of an enzyme that is able to catalyze the ligation of blunt-ended DNA molecules, such as bacteriophage T4 DNA ligase. Thus, the products of the reaction are DNA segments carrying polymeric linker sequences at their ends. These DNA segments are then cleaved with the appropriate restriction enzyme and ligated to an expression vector that has been cleaved with an enzyme that produces termini compatible with those of the DNA segment.

Synthetic linkers containing a variety of restriction endonuclease sites are commercially available from a number of sources including International Biotechnologies Inc, New Haven, Conn., USA.

Exemplary genera of yeast contemplated to be useful in the practice of the present invention as hosts for expressing the albumin, fusion proteins are Pichua (formerly classified as Hansenula), Saccharomyces, Kluyveromyces, Aspergillus, Candida, Torulopsis, Torulaspora, Schizosaccharomyces, Citeromyces, Pachysolen, Zygosaccharomyces, Debaromyces, Trichoderma, Cephalosporium, Humicola, Mucor, Neurospora, Yarrowia, Metschunikowia, Rhodosporidium, Leucosporidium, Botryoascus, Sporidiobolus, Endomycopsis, and the like. Preferred genera are those selected from the group consisting of Saccharomyces, Schizosaccharomyces, Kluyveromyces, Pichia and Torulaspora. Examples of Saccharomyces spp. are S. cerevisiae, S. italicus and S. rouxii.

Examples of Kluyveromyces spp. are K. fragilis, K. lactis and K. marxianus. A suitable Torulaspora species is T. delbrueckii. Examples of Pichia (Hansenula) spp. are P. angusta (formerly H. polymorpha), P. anomala (formerly H. anomala) and P. pastoris. Methods for the transformation of S. cerevisiae are taught generally in EP 251 744, EP 258 067 and WO 90/01063.

Preferred exemplary species of Saccharomyces include S. cerevisiae, S. italicus, S. diastaticus, and Zygosaccharomyces rouxii. Preferred exemplary species of Kluyveromyces include K. fragilis and K. lactis. Preferred exemplary species of Hansenula include H. polymorpha (now Pichia angusta), H. anomala (now Pichia anomala), and Pichia capsulata. Additional preferred exemplary species of Pichia include P. pastoris. Preferred exemplary species of Aspergillusinclude A. niger and A. nidulans. Preferred exemplary species of Yarrowia include Y. lipolytica. Many preferred yeast species are available from the ATCC. For example, the following preferred yeast species are available from the ATCC and are useful in the expression of albumin fusion proteins: Saccharomyces cerevisiae, Hansen, teleomorph strain BY4743 yap3 mutant (ATCC Accession No. 4022731); Saccharomyces cerevisiae Hansen, teleomorph strain BY4743 hsp150 mutant (ATCC Accession No. 4021266); Saccharomyces cerevisiae Hansen, teleomorph strain BY4743 pmt1 mutant (ATCC Accession No. 4023792); Saccharomyces cerevisiae Hansen, teleomorph (ATCC Accession Nos. 20626; 44773; 44774; and 62995); Saccharomyces diastaticus Andrews et Gilliland ex van der Walt, teleomorph (ATCC Accession No. 62987); Kluyveromyces lactis (Dombrowski) van der Walt, teleomorph (ATCC Accession No. 76492); Pichia angusta (Teunisson et al.) Kurtzman, teleomorph deposited as Hansenula polymorpha de Morais et Maia, teleomorph (ATCC Accession No. 26012); Aspergillus niger van Tieghem, anamorph (ATCC Accession No. 9029); Aspergillus niger van Tieghem, anamorph (ATCC Accession No. 16404); Aspergillus nidulans (Eidam) Winter, anamorph (ATCC Accession No. 48756); and Yarrowia lipolytica (Wickerham et al.) van der Walt et von Arx, teleomorph (ATCC Accession No. 201847).

Suitable promoters for S. cerevisiae include those associated with the PGKI gene, GAL1 or GAL10 genes, CYCI, PH05, TRP1, ADH1, ADH2, the genes for glyceraldehyde-3-phosphate dehydrogenase, hexokinase, pyruvate decarboxylase, phosphofructokinase, triose phosphate isomerase, phosphoglucose isomerase, glucokinase, alpha-mating factor pheromone, [a mating factor pheromone], the PRBI promoter, the GUT2 promoter, the GPDI promoter, and hybrid promoters involving hybrids of parts of 5' regulatory regions with parts of 5' regulatory regions of other promoters or with upstream activation sites (e.g. the promoter of EP-A-258 067).

Convenient regulatable promoters for use in Schizosaccharomyces pombe are the thiamine-repressible promoter from the nmt gene as described by Maundrell (1990) J. Biol. Chem. 265, 10857-10864 and the glucose repressible jbpl gene promoter as described by Hoffman & Winston (1990) Genetics 124, 807-816.

Methods of transforming Pichia for expression of foreign genes are taught in, for example, Cregg et al. (1993), and various Phillips patents (e.g. U.S. Pat. No. 4,857,467), and Pichia expression kits are commercially available from Invitrogen BV, Leek, Netherlands, and Invitrogen Corp., San Diego, Calif. Suitable promoters include AOX1 and AOX2. Gleeson et al. (1986) J. Gen. Microbiol. 132, 3459-3465 include information on Hansenula vectors and transformation, suitable promoters being MOX1 and FMD1; whilst EP 361 991, Fleer et al. (1991) and other publications from Rhone-Poulenc Rorer teach how to express foreign proteins in Kluyveromyces spp., a suitable promoter being PGKI.

The transcription termination signal is preferably the 3' flanking sequence of a eukaryotic gene which contains proper signals for transcription termination and polyadenylation. Suitable 3' flanking sequences may, for example, be those of the gene naturally linked to the expression control sequence used, i.e. may correspond to the promoter. Alternatively, they may be different in which case the termination signal of the S. cerevisiae ADHI gene is preferred.

In certain embodiments, the desired heteromultimer protein is initially expressed with a secretion leader sequence, which may be any leader effective in the yeast chosen. Leaders useful in S. cerevisiae include that from the mating factor alpha polypeptide (MFα-1) and the hybrid leaders of EP-A-387 319. Such leaders (or signals) are cleaved by the yeast before the mature albumin is released into the surrounding medium. Further such leaders include those of S. cerevisiae invertase (SUC2) disclosed in JP 62-096086 (granted as 911036516), acid phosphatase (PH05), the pre-sequence of MFα-1, 0 glucanase (BGL2) and killer toxin; S. diastaticus glucoamylase Il; S. carlsbergensis α-galactosidase (MEL1); K. lactis killer toxin; and Candida glucoarnylase.

Provided are vectors containing a polynucleotide encoding a heteromultimer protein described herein, host cells, and the production of the heteromultimer proteins by synthetic and recombinant techniques. The vector may be, for example, a phage, plasmid, viral, or retroviral vector. Retroviral vectors may be replication competent or replication defective. In the latter case, viral propagation generally will occur only in complementing host cells.

In certain embodiments, the polynucleotides encoding heteromultimer proteins described herein are joined to a vector containing a selectable marker for propagation in a host. Generally, a plasmid vector is introduced in a precipitate, such as a calcium phosphate precipitate, or in a complex with a charged lipid. If the vector is a virus, it may be packaged in vitro using an appropriate packaging cell line and then transduced into host cells.

In certain embodiments, the polynucleotide insert is operatively linked to an appropriate promoter, such as the phage lambda PL promoter, the E. coli lac, trp, phoA and rac promoters, the SV40 early and late promoters and promoters of retroviral LTRs, to name a few. Other suitable promoters will be known to the skilled artisan. The expression constructs will further contain sites for transcription initiation, termination, and, in the transcribed region, a ribosome binding site for translation. The coding portion of the transcripts expressed by the constructs will preferably include a translation initiating codon at the beginning and a termination codon (UAA, UGA or UAG) appropriately positioned at the end of the polypeptide to be translated.

As indicated, the expression vectors will preferably include at least one selectable marker. Such markers include dihydrofolate reductase, G418, glutamine synthase, or neomycin resistance for eukaryotic cell culture, and tetracycline, kanamycin or ampicillin resistance genes for culturing in E. coli and other bacteria. Representative examples of appropriate hosts include, but are not limited to, bacterial cells, such as E. coli, Streptomyces and Salmonella typhimurium cells; fungal cells, such as yeast cells (e.g., Saccharomyces cerevisiae or Pichia pastoris (ATCC Accession No. 201178)); insect cells such as Drosophila S2 and Spodoptera Sf9 cells; animal cells such as CHO, COS, NSO, 293, and Bowes melanoma cells; and plant cells. Appropriate culture mediums and conditions for the above-described host cells are known in the art.

Among vectors preferred for use in bacteria include pQE70, pQE60 and pQE-9, available from QIAGEN, Inc.; pBluescript vectors, Phagescript vectors, pNH8A, pNH16a, pNH18A; pNH46A, available from Stratagene Cloning Systems, Inc.; and ptrc99a, pKK223-3, pKK233-3, pDR540, pRIT5 available from Pharmacia Biotech, Inc. Among preferred eukaryotic vectors are pWLNEO, pSV2CAT, pOG44, pXT1 and pSG available from Stratagene; and pSVK3, pBPV, pMSG and pSVL available from Pharmacia. Preferred expression vectors for use in yeast systems include, but are not limited to pYES2, pYD1, pTEF1/Zeo, pYES2/GS, pPICZ, pGAPZ, pGAPZalph, pPIC9, pPIC3.5, pHIL-D2, pHIL-Sl, pPIC3.5K, pPIC9K, and PA0815 (all available from Invitrogen, Carlbad, CA). Other suitable vectors will be readily apparent to the skilled artisan.

In one embodiment, polynucleotides encoding a heteromultimer protein described herein are fused to signal sequences that will direct the localization of a protein of the invention to particular compartments of a prokaryotic or eukaryotic cell and/or direct the secretion of a protein of the invention from a prokaryotic or eukaryotic cell. For example, in E. coli, one may wish to direct the expression of the protein to the periplasmic space. Examples of signal sequences or proteins (or fragments thereof) to which the heteromultimeric proteins are fused in order to direct the expression of the polypeptide to the periplasmic space of bacteria include, but are not limited to, the pelB signal sequence, the maltose binding protein (MBP) signal sequence, MBP, the ompA signal sequence, the signal sequence of the periplasmic E. coli heat-labile enterotoxin B-subunit, and the signal sequence of alkaline phosphatase. Several vectors are commercially available for the construction of fusion proteins which will direct the localization of a protein, such as the pMAL series of vectors (particularly the pMAL-.rho. series) available from New England Biolabs. In a specific embodiment, polynucleotides albumin fusion proteins of the invention may be fused to the pelB pectate lyase signal sequence to increase the efficiency of expression and purification of such polypeptides in Gram-negative bacteria. See, U.S. Pat. Nos. 5,576,195 and 5,846,818.

Examples of signal peptides that are fused to a heteromultimeric protein in order to direct its secretion in mammalian cells include, but are not limited to, the MPIF-1 signal sequence (e.g., amino acids 1-21 of GenBank Accession number AAB51134), the stanniocalcin signal sequence (MLQNSAVLLLLVISASA), and a consensus signal sequence (MPTWAWWLFLVLLLALWAPARG). A suitable signal sequence that may be used in conjunction with baculoviral expression systems is the gp67 signal sequence (e.g., amino acids 1-19 of GenBank Accession Number AAA72759).

Vectors which use glutamine synthase (GS) or DHFR as the selectable markers can be amplified in the presence of the drugs methionine sulphoximine or methotrexate, respectively. An advantage of glutamine synthase based vectors are the availabilty of cell lines (e.g., the murine myeloma cell line, NSO) which are glutamine synthase negative. Glutamine synthase expression systems can also function in glutamine synthase expressing cells (e.g., Chinese Hamster Ovary (CHO) cells) by providing additional inhibitor to prevent the functioning of the endogenous gene. A glutamine synthase expression system and components thereof are detailed in PCT publications: WO87/04462; WO86/05807; WO89/10036; WO89/10404; and WO91/06657. Additionally, glutamine synthase expression vectors can be obtained from Lonza Biologics, Inc. (Portsmouth, N.H.). Expression and production of monoclonal antibodies using a GS expression system in murine myeloma cells is described in Bebbington et al., Bio/technology 10:169(1992) and in Biblia and Robinson Biotechnol. Prog. 11:1(1995).

Also provided are host cells containing vector constructs described herein, and additionally host cells containing nucleotide sequences that are operably associated with one or more heterologous control regions (e.g., promoter and/or enhancer) using techniques known of in the art. The host cell can be a higher eukaryotic cell, such as a mammalian cell (e.g., a human derived cell), or a lower eukaryotic cell, such as a yeast cell, or the host cell can be a prokaryotic cell, such as a bacterial cell. A host strain may be chosen which modulates the expression of the inserted gene sequences, or modifies and processes the gene product in the specific fashion desired. Expression from certain promoters can be elevated in the presence of certain inducers; thus expression of the genetically engineered polypeptide may be controlled. Furthermore, different host cells have characteristics and specific mechanisms for the translational and post-translational processing and modification (e.g., phosphorylation, cleavage) of proteins. Appropriate cell lines can be chosen to ensure the desired modifications and processing of the foreign protein expressed.

Introduction of the nucleic acids and nucleic acid constructs of the invention into the host cell can be effected by calcium phosphate transfection, DEAE-dextran mediated transfection, cationic lipid-mediated transfection, electroporation, transduction, infection, or other methods. Such methods are described in many standard laboratory manuals, such as Davis et al., Basic Methods In Molecular Biology (1986). It is specifically contemplated that the polypeptides of the present invention may in fact be expressed by a host cell lacking a recombinant vector.

In addition to encompassing host cells containing the vector constructs discussed herein, the invention also encompasses primary, secondary, and immortalized host cells of vertebrate origin, particularly mammalian origin, that have been engineered to delete or replace endogenous genetic material (e.g., the coding sequence corresponding to a Cargo polypeptide is replaced with a heteromultimer protein corresponding to the Cargo polypeptide), and/or to include genetic material. The genetic material operably associated with the endogenous polynucleotide may activate, alter, and/or amplify endogenous polynucleotides.

In addition, techniques known in the art may be used to operably associate heterologous polynucleotides (e.g., polynucleotides encoding an albumin protein, or a fragment or variant thereof) and/or heterologous control regions (e.g., promoter and/or enhancer) with endogenous polynucleotide sequences encoding a Therapeutic protein via homologous recombination (see, e.g., U.S. Pat. No. 5,641,670, issued Jun. 24, 1997; International Publication Number WO 96/29411; International Publication Number WO 94/12650; Koller et al., Proc. Natl. Acad. Sci. USA 86:8932-8935 (1989); and Zijlstra et al., Nature 342:435-438 (1989).

Heteromultimer proteins described herein can be recovered and purified from recombinant cell cultures by well-known methods including ammonium sulfate or ethanol precipitation, acid extraction, anion or cation exchange chromatography, phosphocellulose chromatography, hydrophobic interaction chromatography, affinity chromatography, hydroxylapatite chromatography, hydrophobic charge interaction chromatography and lectin chromatography. Most preferably, high performance liquid chromatography ("HPLC") is employed for purification.

In certain embodiments the heteromultimer proteins of the invention are purified using Anion Exchange Chromatography including, but not limited to, chromatography on Q-sepharose, DEAE sepharose, poros HQ, poros DEAF, Toyopearl Q, Toyopearl QAE, Toyopearl DEAE, Resource/Source Q and DEAE, Fractogel Q and DEAE columns.

In specific embodiments the proteins described herein are purified using Cation Exchange Chromatography including, but not limited to, SP-sepharose, CM sepharose, poros HS, poros CM, Toyopearl SP, Toyopearl CM, Resource/Source S and CM, Fractogel S and CM columns and their equivalents and comparables.

In addition, heteromultimer proteins described herein can be chemically synthesized using techniques known in the art (e.g., see Creighton, 1983, Proteins: Structures and Molecular Principles, W. H. Freeman & Co., N.Y and Hunkapiller et al., Nature, 310:105-111 (1984)). For example, a polypeptide corresponding to a fragment of a polypeptide can be synthesized by use of a peptide synthesizer. Furthermore, if desired, nonclassical amino acids or chemical amino acid analogs can be introduced as a substitution or addition into the polypeptide sequence. Non-classical amino acids include, but are not limited to, to the D-isomers of the common amino acids, 2,4diaminobutyric acid, alpha-amino isobutyric acid, 4aminobutyric acid, Abu, 2-amino butyric acid, g-Abu, e-Ahx, 6amino hexanoic acid, Aib, 2-amino isobutyric acid, 3-amino propionic acid, ornithine, norleucine, norvaline, hydroxyproline, sarcosine, citrulline, homocitrulline, cysteic acid, t-butylglycine, t-butylalanine, phenylglycine, cyclohexylalanine, β-alanine, fluoro-amino acids, designer amino acids such as β-methyl amino acids, Cα-methyl amino acids, Nα-methyl amino acids, and amino acid analogs in general. Furthermore, the amino acid can be D (dextrorotary) or L (levorotary).

Provided are heteromultimers which are differentially modified during or after translation, e.g., by glycosylation, acetylation, phosphorylation, amidation, derivatization by known protecting/blocking groups, proteolytic cleavage, linkage to an antibody molecule or other cellular ligand, etc. Any of numerous chemical modifications may be carried out by known techniques, including but not limited, to specific chemical cleavage by cyanogen bromide, trypsin, chymotrypsin, papain, V8 protease, NaBH₄ ; acetylation, formylation, oxidation, reduction; metabolic synthesis in the presence of tunicamycin; etc.

Additional post-translational modifications encompassed herein include, for example, e.g., N-linked or O-linked carbohydrate chains, processing of N-terminal or C-terminal ends), attachment of chemical moieties to the amino acid backbone, chemical modifications of N-linked or O-linked carbohydrate chains, and addition or deletion of an N-terminal methionine residue as a result of procaryotic host cell expression. The heteromultimer proteins are modified with a detectable label, such as an enzymatic, fluorescent, isotopic or affinity label to allow for detection and isolation of the protein.

Examples of suitable enzymes include horseradish peroxidase, alkaline phosphatase, beta-galactosidase, or acetylcholinesterase; examples of suitable prosthetic group complexes include streptavidin biotin and avidin/biotin; examples of suitable fluorescent materials include umbelliferone, fluorescein, fluorescein isothiocyanate, rhodamine, dichlorotriazinylamine fluorescein, dansyl chloride or phycoerythrin; an example of a luminescent material includes luminol; examples of bioluminescent materials include luciferase, luciferin, and aequorin; and examples of suitable radioactive material include iodine, carbon, sulfur, tritium, indium, technetium, thallium, gallium, palladium, molybdenum, xenon, fluorine.

In specific embodiments, heteromultimer proteins or fragments or variants thereof are attached to macrocyclic chelators that associate with radiometal ions.

As mentioned, the heteromultimer described herein is modified by either natural processes, such as post-translational processing, or by chemical modification techniques which are well known in the art. It will be appreciated that the same type of modification may be present in the same or varying degrees at several sites in a given polypeptide. Polypeptides of the invention may be branched, for example, as a result of ubiquitination, and they may be cyclic, with or without branching. Cyclic, branched, and branched cyclic polypeptides may result from posttranslation natural processes or may be made by synthetic methods. Modifications include acetylation, acylation, ADP-ribosylation, amidation, covalent attachment of flavin, covalent attachment of a heme moiety, covalent attachment of a nucleotide or nucleotide derivative, covalent attachment of a lipid or lipid derivative, covalent attachment of phosphotidylinositol, cross-linking, cyclization, disulfide bond formation, demethylation, formation of covalent cross-links, formation of cysteine, formation of pyroglutamate, formylation, gamma-carboxylation, glycosylation, GPI anchor formation, hydroxylation, iodination, methylation, myristylation, oxidation, pegylation, proteolytic processing, phosphorylation, prenylation, racemization, selenoylation, sulfation, transfer-RNA mediated addition of amino acids to proteins such as arginylation, and ubiquitination. (See, for instance, PROTEINS--STRUCTURE AND MOLECULAR PROPERTIES, 2nd Ed., T. E. Creighton, W. H. Freeman and Company, New York (1993); POST-TRANSLATIONAL COVALENT MODIFICATION OF PROTEINS, B. C. Johnson, Ed., Academic Press, New York, pgs. 1-12 (1983); Seifter et al., Meth. Enzymol. 182:626-646 (1990); Rattan et al., Ann. N.Y. Acad. Sci. 663:48-62 (1992)).

In certain embodiments, heteromultimeric proteins may also be attached to solid supports, which are particularly useful for immunoassays or purification of polypeptides that are bound by, that bind to, or associate with albumin fusion proteins of the invention. Such solid supports include, but are not limited to, glass, cellulose, polyacrylamide, nylon, polystyrene, polyvinyl chloride or polypropylene.

In embodiments where the heteromultimeric protein comprises only the VH domain of an antibody, it may be necessary and/or desirable to coexpress the protein with the VL domain of the same antibody, such that the VH-albumin fusion protein and VL protein will associate (either covalently or non-covalently) post-translationally.

In embodiments where the heteromultimeric protein comprises only the VL domain of an antibody, it may be necessary and/or desirable to coexpress the fusion protein with the VH domain of the same antibody, such that the VL-albumin fusion protein and VH protein will associate (either covalently or non-covalently) post-translationally.

Also provided herein are chemically modified derivatives of the heteromultimeric proteins which may provide additional advantages such as increased solubility, stability and circulating time of the polypeptide, or decreased immunogenicity (see U.S. Pat. No. 4,179,337). The chemical moieties for derivitization may be selected from water soluble polymers such as polyethylene glycol, ethylene glycol/propylene glycol copolymers, carboxymethylcellulose, dextran, polyvinyl alcohol and the like. The proteins may be modified at random positions within the molecule, or at predetermined positions within the molecule and may include one, two, three or more attached chemical moieties.

The polymer may be of any molecular weight, and may be branched or unbranched. For polyethylene glycol, the preferred molecular weight is between about 1 kDa and about 100 kDa (the term "about" indicating that in preparations of polyethylene glycol, some molecules will weigh more, some less, than the stated molecular weight) for ease in handling and manufacturing. Other sizes may be used, depending on the desired therapeutic profile (e.g., the duration of sustained release desired, the effects, if any on biological activity, the ease in handling, the degree or lack of antigenicity and other known effects of the polyethylene glycol to a Therapeutic protein or analog). For example, the polyethylene glycol may have an average molecular weight of about 200, 500, 1000, 1500, 2000, 2500, 3000, 3500, 4000, 4500, 5000, 5500, 6000, 6500, 7000, 7500, 8000, 8500, 9000, 9500, 10,000, 10,500, 11,000, 11,500, 12,000, 12,500, 13,000, 13,500, 14,000, 14,500, 15,000, 105,500, 16,000, 16,500, 17,000, 17,500, 18,000, 18,500, 19,000, 19,500, 20,000, 25,000, 30,000, 35,000, 40,000, 45,000, 50,000, 55,000, 60,000, 65,000, 70,000, 75,000, 80,000, 85,000, 90,000, 95,000, or 100,000 kDa.

The presence and quantity of heteromultimer proteins described herein may be determined using ELISA, a well known immunoassay known in the art. In one ELISA protocol that would be useful for detecting/quantifying heteromultimers described herein, comprises the steps of coating an ELISA plate with an anti-human serum albumin antibody, blocking the plate to prevent non-specific binding, washing the ELISA plate, adding a solution containing the protein described herein (at one or more different concentrations), adding a secondary anti-cargo polypeptide specific antibody coupled to a detectable label (as described herein or otherwise known in the art), and detecting the presence of the secondary antibody. In an alternate version of this protocol, the ELISA plate might be coated with the anti-cargo polypeptide specific antibody and the labeled secondary reagent might be the anti-human albumin specific antibody.

### EXAMPLES

### Example 1: The Protein Splitting Method

Specific protein-protein association is driven by strong surface complementarity between interacting partners and the accompanying structural and thermodynamic changes. The surface complementarity provides an opportunity to form contacts that support the creation of favorable electrostatic and hydrophobic interactions. Electrostatic interactions involve the formation of salt bridges, hydrogen bonds and the pervasive dispersion interactions. Solvent exclusion and reorganization around non-polar atomic groups at the interface and its associated entropic effects play a role in the hydrophobic component of the binding thermodynamics. Residues with geometries that are optimized for hydrophobic interaction with one another will form contacts (i.e. stacking, pi-pi, cation-pi contacts favorable for stabilizing a protein-protein interface). Similar thermodynamic effects control multi-step protein folding processes that involve the pre-organization of secondary structural units and tertiary domains, which is followed by their association to form the folded quaternary state of the protein. An alternate mechanism to protein folding and binding involves a coupled protein folding and binding process that ultimately results in the quaternary state of the protein. It is reasonable to assume that there would be aspects of both the processes in action during the quaternary structure formation of a complex protein. In the context of some protein-protein association, the individual protein components need to be co-expressed or be present in the same medium and each of the components or monomers will stably fold into its final structural state only on association with its obligate partner. (Fig. 6)

As described here, generation of a split protein involves recognizing a segmentation site in the native protein, using information from sequence, secondary structure and fold that will yield at least two transporter polypeptides that efficiently form the quasi-native protein structure by self-assembling to form a heteromultimer together. For example, these split protein transporter polypeptides selectively self-assemble and form the quasi-native state when co-expressed. While generating a split protein complementary pair of transporter polypeptides, in a way, the attempt is to emulate a number of naturally occurring obligate protein-protein complexes that exhibit their functionality as a complex while being non-functional in their uncomplexed state. A successful implementation of the strategy results in polypeptides that selectively self-assemble to form heteromultimers with each other, are soluble as individual entities and for functional relevance, do not impair the folding, binding and activity of other components in the environment. The intrinsic nature of the polypeptides to reconstitute with each other has applications in area of creating heteromultimeric fusion entities out of cargo molecules that are not efficient at forming multimers by themselves. In some embodiment, the functional role of the split protein segments is to act as transporter polypeptides that drive heteromultimerization and spatial localization of the multiple cargo molecules fused to the transporter polypeptides.

The segmentation sites within the human serum albumin invented here were designed on the basis of following decision criteria. (1) The segmentation site should reside on a loop with high relative SASA and limited residue contact count to rest of the protein. This was aimed to maximize the chances of finding flexible and accessible loops that are less likely to contribute to the protein core stability and thus impact stability of the assembled quasi-native albumin structure relative to the human serum albumin. (2) The interface should be rich in hotspot residues. By hotspot we mean individual or group of residues involved in a well-connected networks of interactions. The interactions could be based on typical interactions observed in proteins such as hydrogen bonds, charged residue interactions such as salt-bridges, van der Waals contacts which show strong structural complementarity and hydrophobic residue clusters. That is to maximize the interface stability and prevent the complex from dissociating. (3) The interface should be as apolar, extensive and interdigitate as possible. Again, that was to maximize the chances that the two chains exist as a permanent complex and do not dissociate into its individual components. (4) In one embodiment we aimed to retain a natural disulfide bond present in the human serum albumin across the segmented sections so as to covalently connect the two chains. That was meant to further increase the stability of the heterodimerizing quasi-native albumin like structure that self assembles from the two derived segments following segmentation. We locate loop regions in the human serum albumin structure / sequence that satisfy the disulfide criteria described here i.e. have a disulfide very close to the segmentation site in the loop region. Besides the obvious advantage of covalently crosslinking the two chains, this disulfide could also serve as a surrogate for the loop being open on segmentation. (5) In one embodiment, we engineer a disulphide link across the two segments by introducing individual amino acid to cysteine mutations at selected positions on each of the two derived segments. (6) In one embodiment, we engineer amino acids at the interface of the two segments to further increase or improve the hotspots at the interface or enhance the structural and physico-chemical complementarity between the two segments in the assembled quasi-native albumin structure.

Table 3 below shows the interface parameters and association free energies for AlbuCORE scaffolds. The buried interface area between the quasi-native structure derived from assembly of the first and second segment derived from HSA can be computed using a structure based computation using algorithms known in the art (e.g. H. Edelsbrunner and P. Koehl. The weighted volume derivative of a space filling diagram. Proc. Natl. Acad. Sci. (USA) 100, 2203-2208 (2003)). The computed area can be further classified into polar and apolar surface area. The apolar area is an indicator of buried hydrophobic amino acid side chains at the interface. The ΔΔG_{assoc} refers to the computed free energy difference between the associated state and the unassociated state of the two segment structures. The theoretically computed free energy accounts for hydrogen bonds and other electrostatic interactions, van der waals contact, desolvation effects and surface complementarity between the two contacting segment surfaces. Methods to compute free energy of association are known in the art (e.g. Simonson T, Archontis G, Karplus M. Free energy simulations come of age: protein-ligand recognition. Acc Chem Res. (2002) 35, 430-7)

**Table 3: Interface parameters and association free energies for AlbuCORE scaffolds**

| Scaffold | Interface Buried Area (Å²) | Carbon (apolar) Area (Å²) | Carbon (apolar) contribution (%) | ΔΔG_{assoc} (kcal/mol) |
|---|---|---|---|---|
| AlbuCORE_1A | 2892.0 | 1963.2 | 67.88 | -39.13 |
| AlbuCORE_2A | 2282.4 | 1508.2 | 66.08 | -41.11 |
| AlbuCORE_3 | 3395.6 | 2298.3 | 67.68 | -52.69 |
| AlbuCORE_ 4 | 5550.6 | 3690.8 | 66.49 | -180.99 |
| AlbuCORE_6 | 3278.3 | 2149.2 | 65.56 | -55.57 |
| AlbuCORE_7 | 3925.9 | 2541.2 | 64.73 | -75.20 |
| AlbuCORE_9 | 4227.0 | 2709.1 | 64.09 | -122.49 |
| AlbuCORE_13 | 2982.1 | 1978.7 | 66.35 | -71.80 |

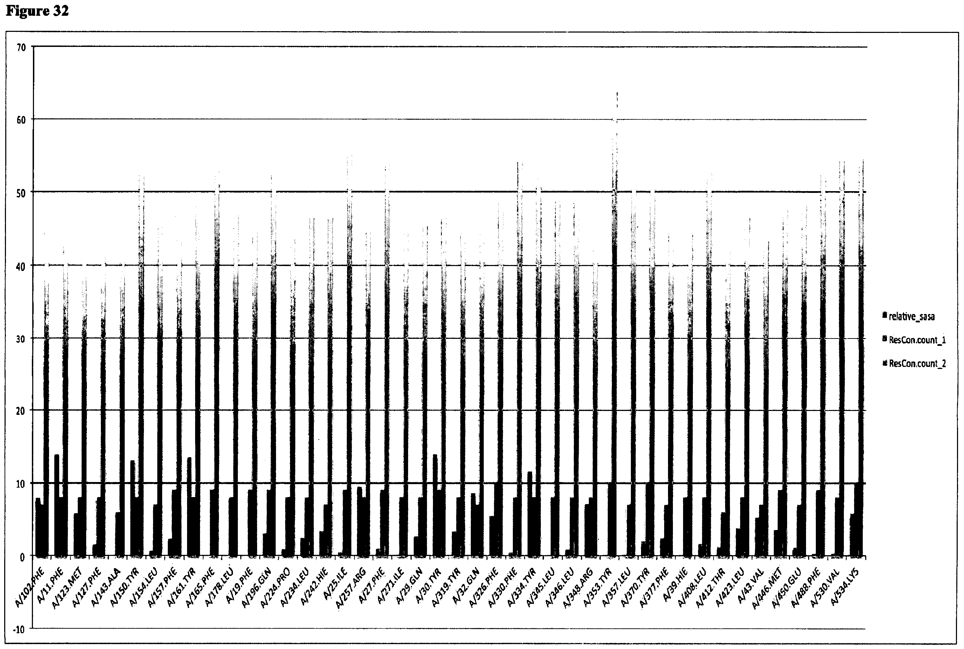

Figure 32 shows a plot of residue contacts made by a subset of residues in the human serum albumin derived from its 3-dimensional structure. Residue contacts can be defined by the inter-residue or certain intra-residue atom-atom interaction that fall within a specified distance range and are of certain physico-chemical nature. Residues or groups of residues with strong residue contacts can be recognized as hotspots. Such residue contact analysis can also be employed to determine loop (or unstructured secondary structural regions in the 3D protein structure) residues, such as those making limited residue contact. The plot also shows residue-wise solvent accessible surface area (SASA) which can be computed using algorithms known in the art (e.g. H. Edelsbrunner and P. Koehl. The weighted volume derivative of a space filling diagram. Proc. Natl. Acad. Sci. (USA) 100, 2203-2208 (2003)).

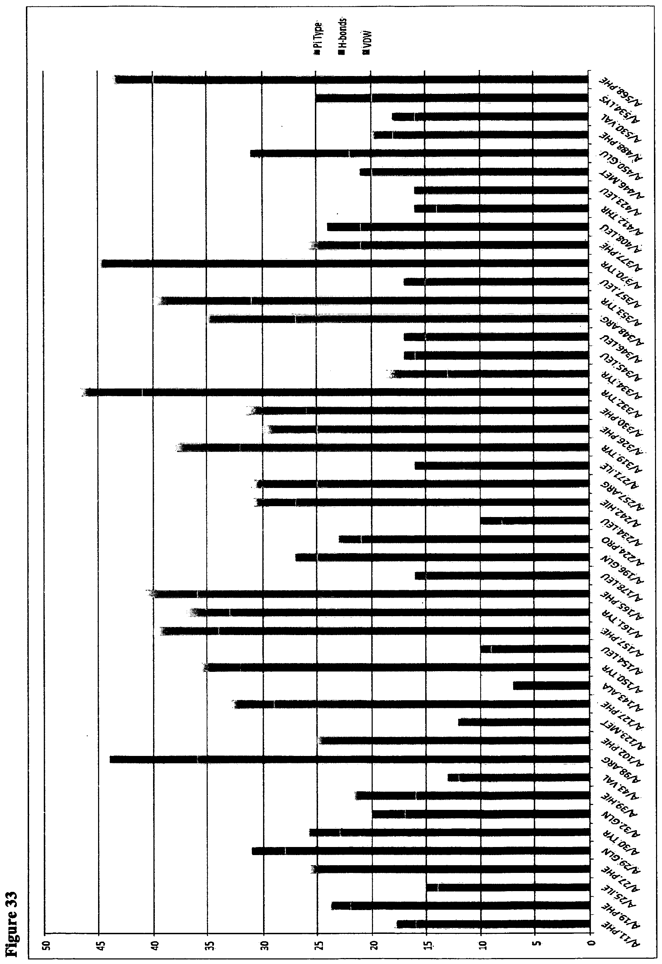

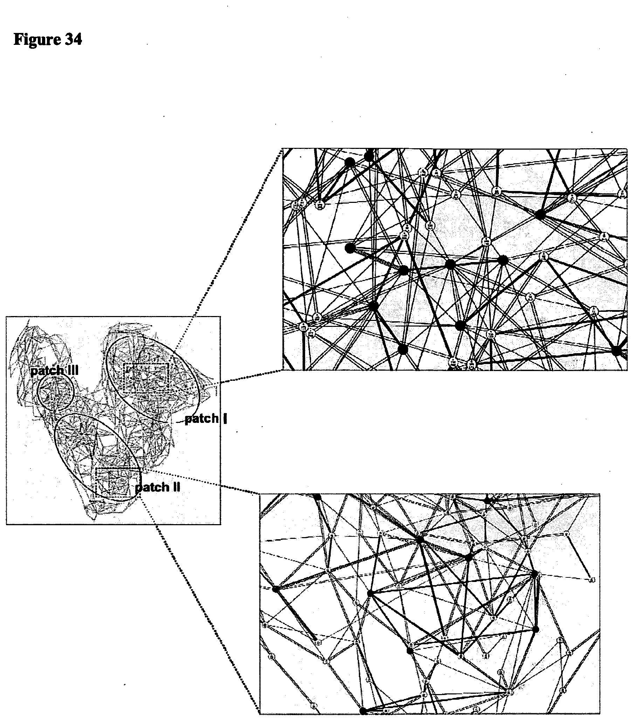

Figure 33 shows a plot of nature of contacts and interactions achieved by select hotspot residues in human serum albumin. Figure 34 is a graphical representation of hot spot residues or clusters (patches) of hotspot residues in human serum albumin.

**Table 4. New segmentation sites in HSA (AlbuCORE) and nature of loop where the segmentation was introduced. The column "loop length" refer to the length (in amino acid residues) of the loop connecting the two nearest secondary structure elements, and the column "sec str distance" shows the distance between the Cα carbons of the N- and C- termini of those same secondary structure elements (all α-helices in this case). Interface S-S indicates the disulfide bond at the interface of the derived segments (transporter polypeptides).**

| **Scaffold** | **Segmentation/Deletion** | **Interface S-S** | **loop length** | **sec str distance** |
|---|---|---|---|---|
| **AlbuCORE_1A** | segment@(339-340) | 316.CYS - 361.CYS | 5 res | 12.3 Å |
| **AlbuCORE_ 2A** | segment@(300-301) | none | 13 res | 28.4 Å |
| AlbuCORE_3 | segment@(364-365) | 360.CYS - 369.CYS | 3 res | 7.2 Å |
| AlbuCORE_4 | segment@(441-442) | 437.CYS - 448.CYS | 7 res | 6.0 Å |
| AlbuCORE_6 | delete(84) | 75.CYS - 91.CYS | 12 res | 12.2 Å |
| AlbuCORE_7 | segment@(171-172) | 168.CYS - 177.CYS | 4 res | 5.7 Å |
| AlbuCORE_9 | segment@(281-282) | 278.CYS - 289.CYS | 2 res | 9.1 Å |
| AlbuCORE_13 | segment@(114-115) | none | 14 res | 36.4 Å |

### Example 2:Preparation of HA/Alloalbumin based heteromultimer proteins

Shown is a method to determine the segmentation site along the HSA sequence and structure that will yield monomeric polypeptide chains that stably fold and self-assemble to form a quasi-native quaternary structure of the original protein. One of the critical requirements for such stable association is the formation of a large buried area of surface complementarity at the interface between the polypeptide chains. The native fold of the original protein provides indication of the natural complementarity of regions within the protein.

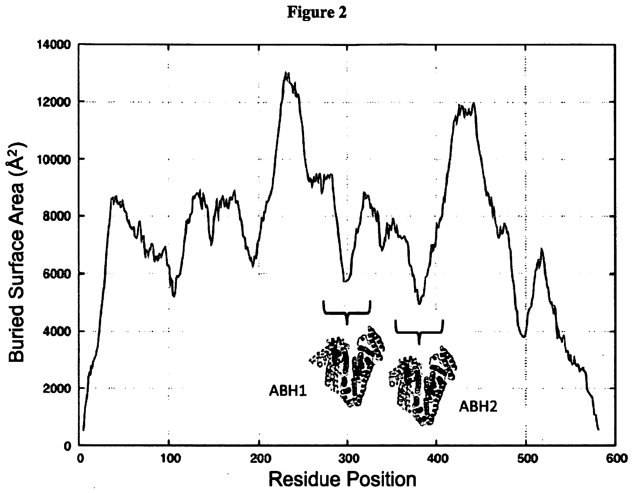

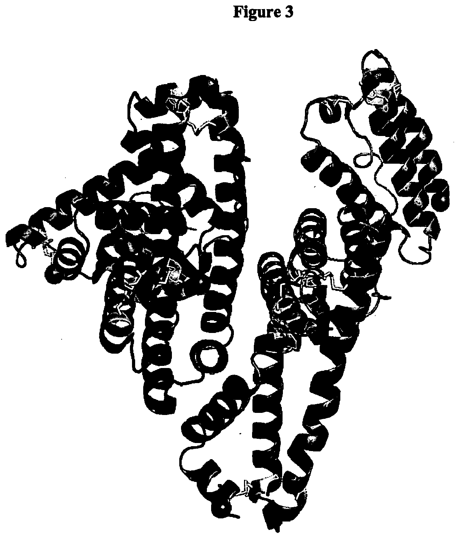

Figure 2 shows the solvent accessible surface area buried at the interface of two albumin-based polypeptides that would ideally fold into the quasi-native structure of HSA, when the segmentation point is moved along the protein sequence. The dashed marking at the bottom indicate the helical secondary structural regions in the protein structure. The analysis indicates that a large surface area, of the order of about 2000 Å² or greater is buried when the split segmentation is introduced anywhere between residues 30 and 520 with a few exceptions, retaining hydrophobic interfaces. In some embodiment the computed molecular surface area is employed instead of the solvent accessible surface area. In some embodiment the buried surface area is in the range of 2000 Å² to 6000 Å². A larger buried interface was observed for the non-covalent ABH1. Additionally, the analysis also revealed that some loop regions are relatively surface exposed. Albumin has an exceptionally large number of disulfide bridges that contribute to the stability of the native protein structure. Section of the protein near residues 110, 190, 300, 390 and 500 provide sites for segmentation that do not split the residues involved in a disulphide link across the two transporter polypeptides. Segmentation in other regions would result in heterodimers with a cross linking disulphide bond between the two transporter polypeptide pairs. Figure 3 presents a model representation of one such quasi-native albumin structure derived by removal of loop from residues 294 to 303 in the HSA sequence. The total buried surface area for the two albumin based polypeptides of SEQ ID No. 2, and SEQ ID No: 3 shown herein is approximately 5500 Å². This is considerably larger than the average of 1910 - 3880 Å² observed in a number of protein-protein heterodimeric and homodimeric co-complex structures [Bahadhur R.P. & Zacharias M. (2008) Cell Mol Life Sci 65, 1059-1072]. This suggests that there is a strong likelihood for the two polypeptides to selectively associate with each other if the folding pathway of the two polypeptides is fairly independent of each other.

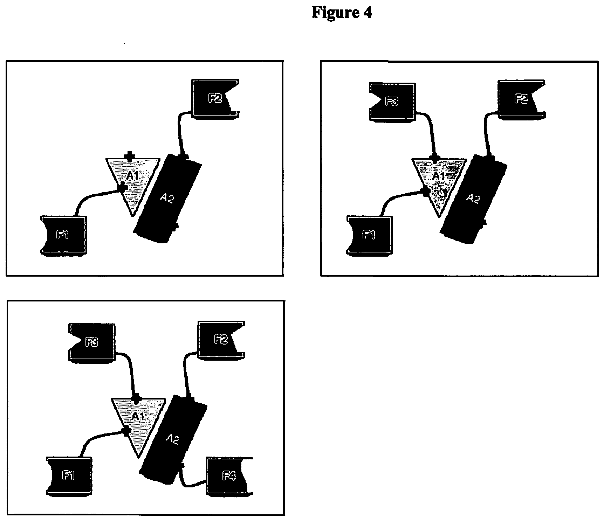

In an aspect of this invention, selective formation of a stable quasi-native structure with the two polypeptides (the pair formed by SEQ ID No. 2 and SEQ ID No. 3 or the transporter pair formed by SEQ ID No. 8 and SEQ ID No. 10) gives us the opportunity to employ these polypeptides to drive the formation of bispecific or other multifunctional molecules after fusing the appropriate cargo proteins of interest to the N or C terminus of the albumin based polypeptides employed as transporter polypeptides. A number of other alternate segmentation patterns resulting in transporter polypeptide pair heterodimer can be designed. The fused cargo proteins can be antigen binding domains or other payloads such as chemotoxins, radiotoxins or cytokines (as represented in Figure 4). The resulting heterodimers have many of the favorable properties intrinsic to HSA including properties like half-life and stability in serum, tissue penetrability transport characteristics and low immunogenicity. Traditional linkers such as (Gly₄Ser)ₓ can be used for the fusion of the cargo protein with the transporter polypeptide.

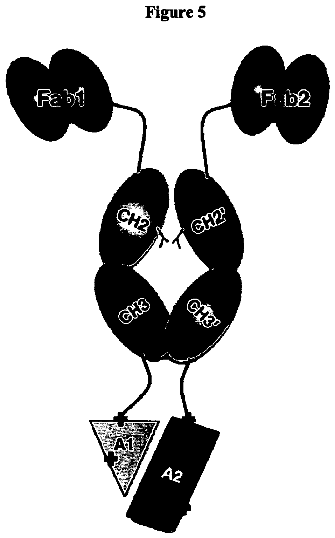

In another aspect of this invention, each of the HSA based transporter polypeptides is fused independently to the C-terminus of two heavy chains in a bispecific Fc molecule (as represented in Figure 5). The strong and selective pairing of the two transporter polypeptides (such as SEQ ID No. 2, and SEQ ID No. 3) drives the selectively heterodimerization of the Fc and also contribute to its stability and other valuable pharmacokinetic properties.

Serum albumin preprotein NP_000468.1 GI 4502027 mRNA sequence from NM_000477.5, Consensus CDS (CCDS) ID 3555.1
SEQ ID No. 4: Residue 1-24 (EFATMAVMAPRTLVLLLSGALALTQTWAG) is the N-terminal export signal sequence region that gets cleaved. This sequence fulfills the same role as the natural signal sequence but it's optimized for mammalian and CHO cell lines.

SEQ ID No. 1: gi|4502027|ref|NP_000468.1| serum albumin preproprotein [Homo sapiens] In parentheses is the N-terminal export sequence that is cleaved resulting in serum albumin.

(EFATMAVMAPRTLVLLLSGALALTQTWAG)DAHKSEVAHRFKDLGEENFKA
LVLIAFAQYLQQCPFEDHVKLVNEVTEFAKTCVADESAENCDKSLHTLFGDK
LCTVATLRETYGEMADCCAKQEPERNECFLQHKDDNPNLPRLVRPEVDVMCT
AFHDNEETFLKKYLYEIARRHPYFYAPELLFFAKRYKAAFTECCQAADKAAC
LLPKLDELRDEGKASSAKQRLKCASLQKFGERAFKAWAVARLSQRFPKAEFA
EVSKLVTDLTKVHTECCHGDLLECADDRADLAKYICENQDSISSKLKECCEK
PLLEKSHCIAEVENDEMPADLPSLAADFVESKDVCKNYAEAKDVFLGMFLYE
YARRHPDYSVVLLLRLAKTYETTLEKCCAAADPHECYAKVFDEFKPLVEEPQ
NLIKQNCELFEQLGEYKFQNALLVRYTKKVPQVSTPTLVEVSRNLGKVGSKC
CKHPEAKRMPCAEDYLSVVLNQLCVLHEKTPVSDRVTKCCTESLVNRRPCFS
ALEVDETYVPKEFNAETFTFHADICTLSEKERQIKKQTALVELVKHKPKATK
EQLKAVMDDFAAFVEKCCKADDKETCFAEEGKKLVAASQAALGL

SEQ ID No. 5: Human serum albumin nucleotide CCDS Sequence (1852 nt)

GAATTCGCCACTATGGCTGTGATGGCCCCTAGGACCCTGGTGCTGCTGCTGTCCGGA
GCTCTGGCTCTGACTCAGACCTGGGCTGGAGATGCACACAAGAGTGAGGTTGCTCAT
CGGTTTAAAGATTTGGGAGAAGAAAATTTCAAAGCCTTGGTGTTGATTGCCTTTGCT
CAGTATCTTCAGCAGTGTCCATTTGAAGATCATGTAAAATTAGTGAATGAAGTAACT
GAATTTGCAAAAACATGTGTTGCTGATGAGTCAGCTGAAAATTGTGACAAATCACTT
CATACCCTTTTTGGAGACAAATTATGCACAGTTGCAACTCTTCGTGAAACCTATGGT
GAAATGGCTGACTGCTGTGCAAAACAAGAACCTGAGAGAAATGAATGCTTCTTGCAA
CACAAAGATGACAACCCAAACCTCCCCCGATTGGTGAGACCAGAGGTTGATGTGATG
TGCACTGCTTTTCATGACAATGAAGAGACATTTTTGAAAAAATACTTATATGAAATT
GCCAGAAGACATCCTTACTTTTATGCCCCGGAACTCCTTTTCTTTGCTAAAAGGTAT
AAAGCTGCTTTTACAGAATGTTGCCAAGCTGCTGATAAAGCTGCCTGCCTGTTGCCA
AAGCTCGATGAACTTCGGGATGAAGGAAGGCTTCGTCTGCCAAACAGAGACTCAAG
TGTGCCAGTCTCCAAAAATTTGGAGAAAGAGCTTTCAAAGCATGGGCAGTAGCTCGC
CTGAGCCAGAGATTTCCCAAAGCTGAGTTTGCAGAAGTTTCCAAGTTAGTGACAGAT
CTTACCAAAGTCCACACGGAATGCTGCCATGGAGATCTGCTTGAATGTGCTGATGAC

AGGGCGGACCTTGCCAAGTATATCTGTGAAAATCAAGATTCGATCTCCAGTAAACTG
AAGGAATGCTGTGAAAAACCTCTGTTGGAAAAATCCCACTGCATTGCCGAAGTGGAA
AATGATGAGATGCCTGCTGACTTGCCTTCATTAGCTGCTGATTTTGTTGAAAGTAAG
GATGTTTGCAAAAACTATGCTGAGGCAAAGGATGTCTTCCTGGGCATGTTTTTGTAT
GAATATGCAAGAAGGCATCCTGATTACTCTGTCGTGCTGCTGCTGAGACTTGCCAAG
ACATATGAAACCACTCTAGAGAAGTGCTGTGCCGCTGCAGATCCTCATGAATGCTAT
GCCAAAGTGTTCGATGAATTTAAACCTCTTGTGGAAGAGCCTCAGAATTTAATCAAA
CAAAATTGTGAGCTTTTTGAGCAGCTTGGAGAGTACAAATTCCAGAATGCGCTATTA
GTTCGTTACACCAAGAAAGTACCCCAAGTGTCAACTCCAACTCTTGTAGAGGTCTCA
AGAAACCTAGGAAAAGTGGGCAGCAAATGTTGTAAACATCCTGAAGCAAAAAGAATG
CCCTGTGCAGAAGACTATCTATCCGTGGTCCTGAACCAGTTATGTGTGTTGCATGAG
AAAACGCCAGTAAGTGACAGAGTCACCAAATGCTGCACAGAATCCTTGGTGAACAGG
CGACCATGCTTTTCAGCTCTGGAAGTCGATGAAACATACGTTCCCAAAGAGTTTAAT
GCTGAAACATTCACCTTCCATGCAGATATATGCACACTTTCTGAGAAGGAGAGACAA
ATCAAGAAACAAACTGCACTTGTTGAGCTCGTGAAACACAAGCCCAAGGCAACAAAA
GAGCAACTGAAAGCTGTTATGGATGATTTCGCAGCTTTTGTAGAGAAGTGCTGCAAG
GCTGACGATAAGGAGACCTGCTTTGCCGAGGAGGGTAAAAAACTTGTTGCTGCAAGT
CAAGCTGCCTTAGGCTTATGA

The protein and nucleotide sequence of albumin based polypeptides useful as transporter polypeptides are as follows:

### Albumin based heteromultimer 1:

Albumin based Transporter polypeptide 1-Ver 1: SEQ ID No. 2: 
DAHKSEVAHRFKDLGEENFKALVLIAFAQYLQQCPFEDHVKLVNEVTEFAKTCVADE
SAENCDKSLHTLFGDKLCTVATLRETYGEMADCCAKQEPERNECFLQHKDDNPNLPR
LVRPEVDVMCTAFHDNEETFLKKYLYEIARRHPYFYAPELLFFAKRYKAAFTECCQA
ADKAACLLPKLDELRDEGKASSAKQRLKCASLQKFGERAFKAWAVARLSQRFPKAEF
AEVSKLVTDLTKVHTECCHGDLLECADDRADLAKYICENQDSISSKLKECCEKPLLE
KSHCIAEV
Nucleotide sequence encoding Albumin based Transporter polypeptide 1-Ver 1: SEQ ID No. 6: 
GATGCACACAAGAGTGAGGTTGCTCATCGGTTTAAAGATTTGGGAGAAGAAAATTTC
AAAGCCTTGGTGTTGATTGCCTTTGCTCAGTATCTTCAGCAGTGTCCATTTGAAGAT
CATGTAAAATTAGTGAATGAAGTAACTGAATTTGCAAAAACATGTGTTGCTGATGAG
TCAGCTGAAAATTGTGACAAATCACTTCATACCCTTTTTGGAGACAAATTATGCACA
GTTGCAACTCTTCGTGAAACCTATGGTGAAATGGCTGACTGCTGTGCAAAACAAGAA
CCTGAGAGAAATGAATGCTTCTTGCAACACAAAGATGACAACCCAAACCTCCCCCGA
TTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCG
GAACTCCTTTTCTTTGCTAAAAGGTATAAAGCTGCTTTTACAGAATGTTGCCAAGCT
GCTGATAAAGCTGCCTGCCTGTTGCCAAAGCTCGATGAACTTCGGGATGAAGGGAAG
GCTTCGTCTGCCAAACAGAGACTCAAGTGTGCCAGTCTCCAAAAATTTGGAGAAAGA
GCTTTCAAAGCATGGGCAGTAGCTCGCCTGAGCCAGAGATTTCCCAAAGCTGAGTTT
GCAGAAGTTTCCAAGTTAGTGACAGATCTTACCAAAGTCCACACGGAATGCTGCCAT
GGAGATCTGCTTGAATGTGCTGATGACAGGGCGGACCTTGCCAAGTATATCTGTGAA
AATCAAGATTCGATCTCCAGTAAACTGAAGGAATGCTGTGAAAAACCTCTGTTGGAA
AAATCCCACTGCATTGCCGAAGTGTGA
Albumin based Transporter polypeptide 2-Ver1 : SEQ ID No. 3: 
SLAADFVESKDVCKNYAEAKDVFLGMFLYEYARRHPDYSVVLLLRLAKTYETTLEKC
CAAADPHECYAKVFDEFKPLVEEPQNLIKQNCELFEQLGEYKFQNALLVRYTKKVPQ
VSTPTLVEVSRNLGKVGSKCCKHPEAKRMPCAEDYLSVVLNQLCVLHEKTPVSDRVT
KCCTESLVNRRPCFSALEVDETYVPKEFNAETFTFHADICTLSEKERQIKKQTALVE
LVKHKPKATKEQLKAVMDDFAAFVEKCCKADDKETCFAEEGKKLVAASQAALGL
Nucleotide sequence encoding Albumin based Transporter polypeptide 2-Ver1 : SEQ ID No. 7:

TCATTAGCTGCTGATTTTGTTGAAAGTAAGGATGTTTGCAAAAACTATGCTGAGGCA
AAGGATGTCTTCCTGGGCATGTTTTTGTATGAATATGCAAGAAGGCATCCTGATTAC
TCTGTCGTGCTGCTGCTGAGACTTGCCAAGACATATGAAACCACTCTAGAGAAGTGC
TGTGCCGCTGCAGATCCTCATGAATGCTATGCCAAAGTGTTCGATGAATTTAAACCT
CTTGTGGAAGAGCCTCAGAATTTAATCAAACAAAATTGTGAGCTTTTTGAGCAGCTT
GGAGAGTACAAATTCCAGAATGCGCTATTAGTTCGTTACACCAAGAAAGTACCCCAA
GTGTCAACTCCAACTCTTGTAGAGGTCTCAAGAAACCTAGGAAAAGTGGGCAGCAAA

TGTTGTAAACATCCTGAAGCAAAAAGAATGCCCTGTGCAGAAGACTATCTATCCGTG
GTCCTGAACCAGTTATGTGTGTTGCATGAGAAAACGCCAGTAAGTGACAGAGTCACC
AAATGCTGCACAGAATCCTTGGTGAACAGGCGACCATGCTTTTCAGCTCTGGAAGTC
GATGAAACATACGTTCCCAAAGAGTTTAATGCTGAAACATTCACCTTCCATGCAGAT
ATATGCACACTTTCTGAGAAGGAGAGACAAATCAAGAAACAAACTGCACTTGTTGAG
CTCGTGAAACACAAGCCCAAGGCAACAAAAGAGCAACTGAAAGCTGTTATGGATGAT
TTCGCAGCTTTTGTAGAGAAGTGCTGCAAGGCTGACGATAAGGAGACCTGCTTTGCC
GAGGAGGGTAAAAAACTTGTTGCTGCAAGTCAAGCTGCCTTAGGCTTATGA

### Albumin based heteromultimer 2:

Albumin based Transporter polypeptide 1-Ver 2: SEQ ID No. 8: 
DAHKSEVAHRFKDLGEENFKALVLIAFAQYLQQCPFEDHVKLVNEVTEFAKTCVADE
SAENCDKSLHTLFGDKLCTVATLRETYGEMADCCAKQEPERNECFLQHKDDNPNLPR
LVRPEVDVMCTAFHDNEETFLKKYLYEIARRHPYFYAPELLFFAKRYKAAFTECCQA
ADKAACLLPKLDELRDEGKASSAKQRLKCASLQKFGERAFKAWAVARLSQRFPKAEF
AEVSKLVTDLTKVHTECCHGDLLECADDRADLAKYICENQDSISSKLKECCEKPLLE
KSHCIAEVENDEMPADLPSLAADFVESKDVCKNYAEAKDVFLGMFLYEYARA
Nucleotide sequence encoding Albumin based Transporter polypeptide 1-Ver 2: SEQ ID No. 9: 
GATGCACACAAGAGTGAGGTTGCTCATCGGTTTAAAGATTTGGGAGAAGAAAATTTC
AAAGCCTTGGTGTTGATTGCCTTTGCTCAGTATCTTCAGCAGTGTCCATTTGAAGAT
CATGTAAAATTAGTGAATGAAGTAACTGAATTTGCAAAAACATGTGTTGCTGATGAG
TCAGCTGAAAATTGTGACAAATCACTTCATACCCTTTTTGGAGACAAATTATGCACA
GTTGCAACTCTTCGTGAAACCTATGGTGAAATGGCTGACTGCTGTGCAAAACAAGAA
CCTGAGAGAAATGAATGCTTCTTGCAACACAAAGATGACAACCCAAACCTCCCCCGA
TTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCG
GAACTCCTTTTCTTTGCTAAAAGGTATAAAGCTGCTTTTACAGAATGTTGCCAAGCT
GCTGATAAAGCTGCCTGCCTGTTGCCAAAGCTCGATGAACTTCGGGATGAAGGGAAG
GCTTCGTCTGCCAAACAGAGACTCAAGTGTGCCAGTCTCCAAAAATTTGGAGAAAGA
GCTTTCAAAGCATGGGCAGTAGCTCGCCTGAGCCAGAGATTTCCCAAAGCTGAGTTT
GCAGAAGTTTCCAAGTTAGTGACAGATCTTACCAAAGTCCACACGGAATGCTGCCAT
 
GGAGATCTGCTTGAATGTGCTGATGACAGGGCGGACCTTGCCAAGTATATCTGTGAA
AATCAAGATTCGATCTCCAGTAAACTGAAGGAATGCTGTGAAAAACCTCTGTTGGAA
AAATCCCACTGCATTGCCGAAGTGGAAAATGATGAGATGCCTGCTGACTTGCCTTCA
TTAGCTGCTGATTTTGTTGAAAGTAAGGATGTTTGCAAAAACTATGCTGAGGCAAAG
GATGTCTTCCTGGGCATGTTTTTGTATGAATATGCAAGAGCATGA
Albumin based Transporter polypeptide 2-Ver 2: SEQ ID No. 10: 
SVVLLLRLAKTYETTLEKCCAAADPHECYAKVFDEFKPLVEEPQNLIKQNCELFEQL
GEYKFQNALLVRYTKKVPQVSTPTLVEVSRNLGKVGSKCCKHPEAKRMPCAEDYLSV
VLNQLCVLHEKTPVSDRVTKCCTESLVNRRPCFSALEVDETYVPKEFNAETFTFHAD
ICTLSEKERQIKKQTALVELVKHKPKATKEQLKAVMDDFAAFVEKCCKADDKETCFA
EEGKKLVAASQAALGL
Nucleotide sequence encoding Albumin based Transporter polypeptide 2-Ver 2: SEQ ID No. 11:

TCTGTCGTGCTGCTGCTGAGACTTGCCAAGACATATGAAACCACTCTAGAGAAGTGC
TGTGCCGCTGCAGATCCTCATGAATGCTATGCCAAAGTGTTCGATGAATTTAAACCT
CTTGTGGAAGAGCCTCAGAATTTAATCAAACAAAATTGTGAGCTTTTTGAGCAGCTT
GGAGAGTACAAATTCCAGAATGCGCTATTAGTTCGTTACACCAAGAAAGTACCCCAA
GTGTCAACTCCAACTCTTGTAGAGGTCTCAAGAAACCTAGGAAAAGTGGGCAGCAAA
TGTTGTAAACATCCTGAAGCAAAAAGAATGCCCTGTGCAGAAGACTATCTATCCGTG
GTCCTGAACCAGTTATGTGTGTTGCATGAGAAAACGCCAGTAAGTGACAGAGTCACC
AAATGCTGCACAGAATCCTTGGTGAACAGGCGACCATGCTTTTCAGCTCTGGAAGTC
GATGAAACATACGTTCCCAAAGAGTTTAATGCTGAAACATTCACCTTCCATGCAGAT
ATATGCACACTTTCTGAGAAGGAGAGACAAATCAAGAAACAAACTGCACTTGTTGAG
CTCGTGAAACACAAGCCCAAGGCAACAAAAGAGCAACTGAAAGCTGTTATGGATGAT
TTCGCAGCTTTTGTAGAGAAGTGCTGCAAGGCTGACGATAAGGAGACCTGCTTTGCC
GAGGAGGGTAAAAAACTTGTTGCTGCAAGTCAAGCTGCCTTAGGCTTATGA

### Generation and Expression of HA or HAA based heteromultimers

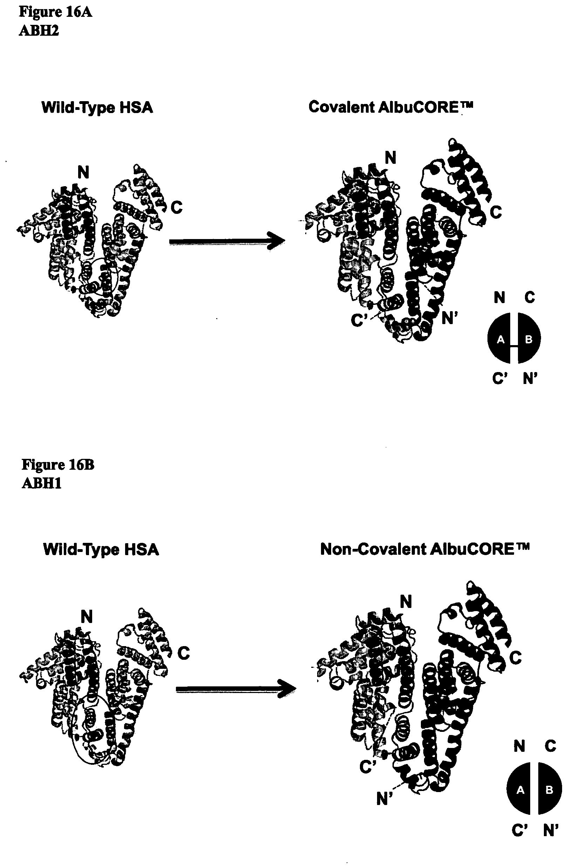

The genes encoding the full length WT HA and the HA based transporter polypeptide monomers were constructed via gene synthesis using codons optimized for human/mammalian expression. The constructs were designed from known full-length Human Serum Albumin Preprotein (GENEBANK: NP_000468.1), after exclusion of the signal sequence EFATMAVMAPRTLVLLLSGALALTQTWAG. The final gene products were subcloned into the mammalian expression vector pTT5 (NRC-BRI, Canada) (Durocher et al). High level and high-throughput recombinant protein production by transient transfection of suspension-growing human CHO-3E7 was performed. See *Table 3* for construct boundaries of the two scaffolds described here: Albumin based heteromultimer 1 (ABH1) and Albumin based heteromultimer 2 (ABH2). Albumin based heteromultimer 2 comprises one disulfide bond between the two transporter polypeptides, while Albumin based heteromultimer 1 is formed entirely by non-covalent interactions. Figures 16A and 16B depict the protein structure and symbolic rendering of ABH1 and ABH2 respectively. As the two figures demonstrate, both ABH constructs are composed of two transporter polypeptides A and B, sequences described in *Table 3.* Figure 6A provides SDS-PAGE (non-reducing) gel analysis of the two heteromultimer constructs (ABH1 and ABH2), after co-expression (different DNA transfection ratios are shown). WT full-length HSA is shown as control. As expected, ABH2 retains the disulfide linkage in non-reducing SDS-PAGE, with a MW roughly double the non-disulfide linked ABH1. Figure 6A also demonstrates that formation of heterodimers in ABH2 is dependent on DNA transfection ratio, with the 1:1 producing the most heterodimeric product. Finally, 6A demonstrates that fragment A of ABH2, when expressed as stand-alone molecule, is more susceptible to forming homodimers and staying monomeric, a result also observed in Figure 6B. Figure 6B provides Native gel analysis of the two Albumin based heteromultimer constructs (ABH1 and ABH2), after co-expression (1:1 DNA level). WT full-length HSA is shown as control. ABH1 and ABH2 both form a complex of expected mass, comparable to the full-length WT HSA. Furthermore, upon expression, neither the transporter polypeptides forming ABH1 nor the ones forming ABH2 homodimerize; rather they preferably form a stable hetercomplex. See *Table 5* below for details.

**Table 5: Albumin based heteromultimer constructs**

| **Construct** | **Segment Boundaries*** | **MW (KDa)** |
|---|---|---|
| Wild Type HA | 1:585 (SEQ ID NO: 1) | 64.3 |
| ABH1 (AlbuCORE 2) | 1:293 (SEQ ID NO: 2) | 32.2 |
| | 304:585 (SEQ ID NO: 3) | 30.9 |
| ABH2 (AlbuCORE 1) | 1:337 (SEQ ID NO: 8) | 37 |
| | 342:585 (SEQ ID NO: 10) | 26.7 |

WT-HSA and the two Albumin based heteromultimers (ABH1 and ABH2) were expressed in CHO-3E7 cell line grown in suspension in FreeStyle F17 medium (Invitrogen) supplemented with 0.1% w/v pluronic and 4 mM glutamine. The day of transfection cell density should be around 1.5-2 million cells/ml and viability must be greater than 97%. Transfection was carried out as described in patent application WO 2009/137911 using a mixture of plasmid DNA made of 5% pTTo-GFP plasmid (green fluorescent protein to determine transfection efficiency, *Table 4*)*,* 15% pTT22-AKT plasmid, 21 % HSA plasmids (10.63% of each), 68.37% of Salmon Sperm DNA. Following transfection, the shake flask containing cells was then placed on an orbital shaker set to 120 rpm in a humidified incubator with 5% CO2 at 37°C. Twenty-four hours post-transfection, 1 % w/v TN1 and 0.5 mM VPA (Valproic acid) were added to the cultures. The cultures were then transferred on an orbital shaker (120 rpm) placed in a humidified incubator with 5% CO2 set at 32°C. At 24-48 hours, GFP positive cells should be between 30-60% as determined by flow cytometry. Cells were harvested 7 days post-transfection and spun at 4,000 rpm for 20 minutes. The supernatant was filter-sterlized (clarified) using a 0.45 µm filter (Millipore). The supernatant was stored at 4 °C for short-term storage and at -80 °C for long-term storage. Prior to purification, the frozen supernatant was thawed at 37 °C, re-filtered and degassed through a 0. 45 µm membrane filter under vacuum for 5 - 10 minutes.

**Table 6: Cell viability at different stages of expression for WT and ABH1 construct.**

| **HSA scaffold** | **% GFP 48 hrs post-transfection** | **% viability 48 hrs post-transfection** | **% viability 48 hrs post-transfection** |
|---|---|---|---|
| Wild Type HSA | 67 | 94.6 | 72.3 |
| ABH2 | 66.3 | 93.6 | 77.1 |

### Purification of ABH2, HSA and heteromultimers

As observed from Figure 6A, the 1:1 DNA transfection ratio of the two transporter polypeptides produced the highest level of heterodimeric ABH2 product. CHO cells transiently co-expressing this ratio of the two polypeptides were cultured as described above. Subsequent heterodimer purification was carried out as follows. The wild-type human Albumin was transfected into CHO cells and purified similarly to the ABH2.

Purification was performed by gravity flow using a bench-top QIAGEN-tip 500 column packed with a Blue Sepharose matrix (GE Healthcare). The Blue Sepharose matrix was equilibrated with 20 ml of PBS pH 7.2. The sample was loaded at a flow rate of 5 ml/min and subsequently washed with with 20 ml of PBS. The protein was eluted with 0.1 M Na2HPO4 pH 7.2 supplemented with 1 M NaCl and collected in 1 ml fractions (20 ml total). Fractions containing HSA (as per Bradford protein assay) were pooled, and applied on a HiLoad 16/60 Superdex 200 prep grade gel filtration column coupled to an AKTA Express system (GE Healthcare) using a flow rate of 1 ml/ml. Protein with a purity of >85% was collected; fractions containing pure sample were pooled and concentrated by centrifugation using an Amicon Ultra membrane with a cutoff weight of 10 000 MWCO. Figure 6C shows SDS-PAGE (non-reducing) analysis of the ABH2 heteromultimer and WT HSA, both after the final stage of purification. Both constructs show the expected MW. Purification yields were comparable to wild-type full length HSA, at about 10mg/L. This result was also comparable to the 9mg/L generated by V1087 control performed in HEK cells. Yields were determined by nanodrop A280 measured after Blue Sepharose and size exclusion chromatography.

### Stability determination of Albumin based Heteromultimers using Differential Scanning Calorimetry (DSC)

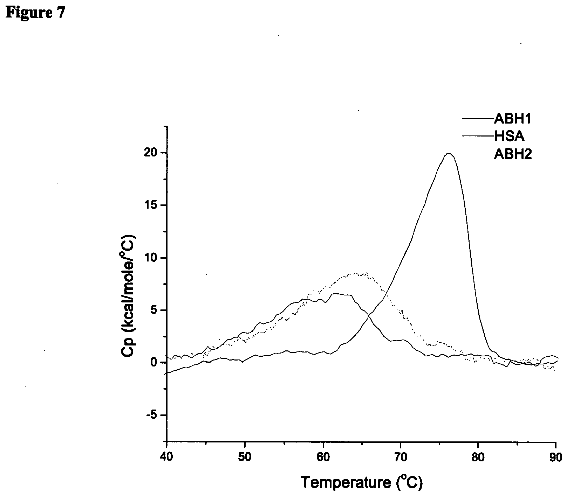

All DSC experiments were carried out using a GE or MicroCal VP-Capillary instrument. The proteins were buffer-exchanged into PBS (pH 7.4) and diluted to 0.3 to 0.7mg/mL with 0.137mL loaded into the sample cell and measured with a scan rate of 1°C/min from 20 to 100°C. Data was analyzed using the Origin software (GE Healthcare) with the PBS buffer background subtracted. See Table 7 and Figure 7 for resulting melting temperature determined.

**Table 7: Melting temperature for Albumin based heteromultimers**

| **Molecule** | **Measured Mass (Da)** | **Theoretical MW (Da)** | **Tm° C** |
|---|---|---|---|
| HSA Wild Type | 66620 | 66470 | 75 |
| ABH2 | 66100 | 65880 | 63 |
| ABH1 | Not determined | Not determined | 60 |

### Evaluation of FcRn Binding of HSA and ABH2 using Surface Plasmon Resonance

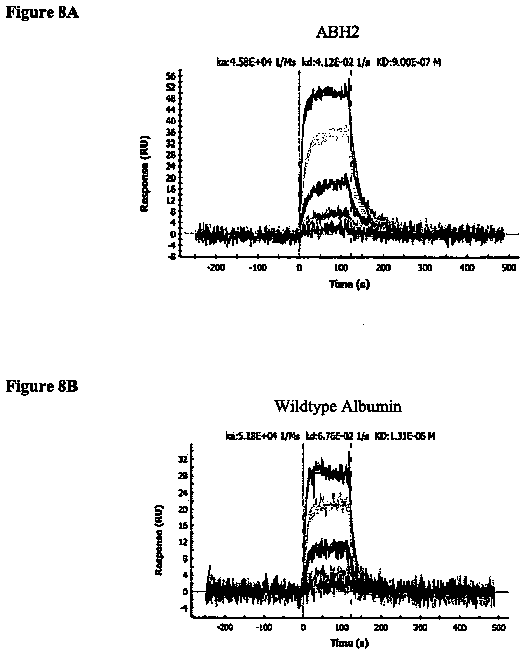

As seen in Figures 8A-B, when HSA and a HSA-based heteromultimer are immobilized on the SPR surface, affinity towards FcRn appears to be comparable between the full length WT HSA and ABH2, indicating FcRn binding functionality of albumin is retained by the heteromultimer formed by the self-assembly of albumin based transporter polypeptides. The following Table 8 illustrates FcRn binding data. Values in parenthesis refer to standard deviation.

**Table 8: Kinetic and Equilibrium fit of FcRn Binding of HSA and ABH2 using Surface Plasmon Resonance**

| | Ka (1/Ms) Grouped Fitted | Kd (1/s) Grouped Fitted | KD (M) Grouped Fitted | |
|---|---|---|---|---|
| HSA | 5.3E+04 (7E+03) | 7.0E-02 (2.0E-02) | 1.4E-06 (6.0E-07) | Kinetic fit |
| ABH2 | 5.0E+04 (4E+03) | 4.2E-02 (8.0E-03) | 8.0E-07 (2.0E-07) | Kinetic fit |
| HSA | | | 9.0E-07 (1.0E-07) | Equilibrium Fit |
| ABH2 | | | 9.0E-07 (1.0E-07) | Equilibrium Fit |

### Example 3: Generation and Expression of Albumin based heteromultimers with mono- and tetravalency comprising anti-Her2/neu and anti-CD16 scFv bioactive fusions.

Multivalent heteromultimer ABH2 was generated by expressing its single monomeric transporter polypeptides, SEQ ID NO: 8 and SEQ ID NO: 10, fused at one or both termini to cargo polypeptides that are either antiHer2scFv (4D5) and/or anti-CD16 scFv (NM3E). The term 4D5 as used herein throughout refers to the humanized sequences of the 4D5 antibody and corresponds to the amino acid sequence of the variable domains in the trastuzumab antibody along with the linker region between the Vl and Vh domains in the scFv molecular format. These form a set of 8 base construct monomers based on transporter polypeptide 1 and 8 base construct monomers based on transporter polypeptide 2. Different combinations of these base constructs were combined upon co-expression to form heteromultimers displaying all combination of the two cargo polypeptides at any of the four terminal positions of the two transporter polypeptides, ranging from monovalent to tetravalent.

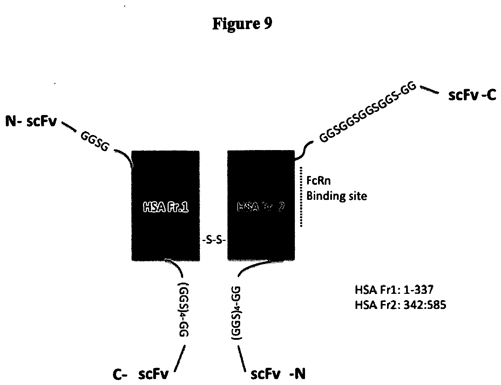

As shown in Figure 9, the bioactive cargo polypeptides were fused to the heteromultimer transporter polypeptides via a GGSG linker, for the N terminus of one monomer and a longer (GGS)4GG linker for all other termini in the other monomer.

**Table 9** illustrates the 16 base constructs (Base construct #1-Base construct #16) that were generated by fusing the 4D5 and NM3E cargo polypeptides to either N or C terminus of transporter polypeptide 1 (F1) or transporter polypeptide 2 (F2). F1: corresponds to SEQ ID 8 and F2 corresponds to SEQ ID 10.

**Table 9:**

| | Single Fusions | | | |
|---|---|---|---|---|
| # | Fusion 1 | Fusion 2 | Fusion 3 | SEQ ID NOs (nucleotide/amino acid): |
| 1 | NM3E2 | F1 | | 95/96 |
| 2 | F1 | NM3E2 | | 97/98 |
| 3 | NM3E2 | F2 | | 99/100 |
| 4 | F2 | NM3E2 | | 101/102 |
| 5 | 4D5 | F1 | | 103/104 |
| 6 | F1 | 4D5 | | 105/106 |
| 7 | 4D5 | F2 | | 107/108 |
| 8 | F2 | 4D5 | | 109/110 |

| | Double Fusions | | | |
|---|---|---|---|---|
| 9 | NM3E2 | F1 | NM3E2 | 111/112 |
| 10 | NM3E2 | F2 | NM3E2 | 113/114 |
| 11 | 4D5 | F1 | 4D5 | 115/116 |
| 12 | 4D5 | F2 | 4D5 | 117/118 |
| 13 | NM3E2 | F1 | 4D5 | 119/120 |
| 14 | 4D5 | F1 | NM3E2 | 121/122 |
| 15 | NM3E2 | F2 | 4D5 | 123/124 |
| 16 | 4D5 | F2 | NM3E2 | 125/126 |

Multivalent constructs were generated as outlined in Example 2 using heteromultimer ABH2. The final gene products were subcloned into the mammalian expression vector pTT5 (NRC-BRI, Canada) (Durocher et al). High level and high-throughput recombinant protein production by transient transfection of suspension-growing human CHO-3E7 was performed. Additional detail regarding the preparation and expression of these constructs are found in Example 11.

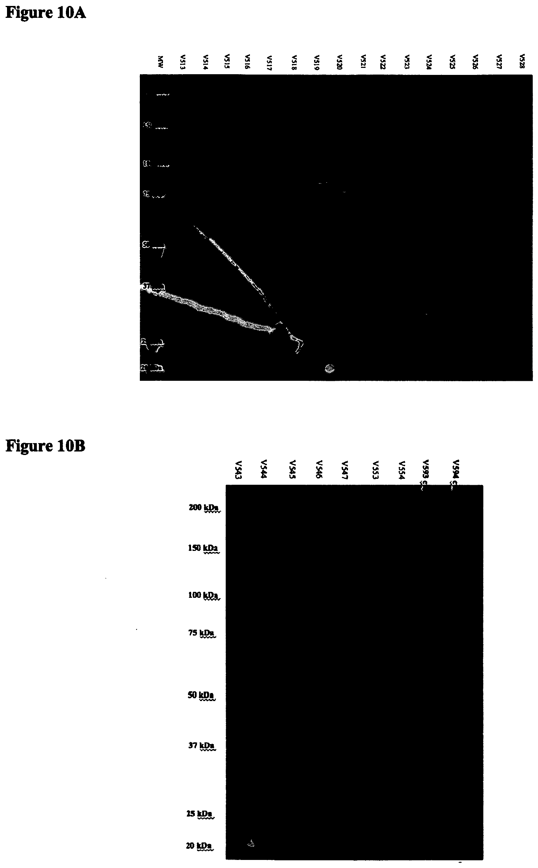

Purification was performed by application of the cellular supernatant with expressed protein to a QIAGEN-tip 500 column packed with Blue Sepharose matrix (GE Healthcare) coupled to an AKTA Express system (GE Healthcare) using a flow rate of 1 ml/ml. The column was equilibrated with equilibrated with sample buffer composed of 20 ml of PBS pH 7.2, 300 mM NaCl. The sample was loaded at a flow rate of 5 ml/min and subsequently washed with sample buffer. The protein was eluted by applicatuion of NaCl gradient ranging from 300 mM to 2000 mM. Fractions eluting in higher salt concentration were the purest and were pooled, concentrated and subsequently applied to a HiLoad 16/60 Superdex 200 prep grade gel filtration column coupled to an AKTA Express system (GE Healthcare) using a flow rate of 1 ml/ml. Protein with a purity of >85% was collected; fractions containing pure sample were pooled and concentrated by centrifugation using an Amicon Ultra membrane with a cutoff weight of 10 000 MWCO. Figures 10A-10B shows SDS-PAGE (non-reducing) analysis of the ABH2 heteromultimer fused to different cargo polypeptides. The position of those polypeptides in the heteromultimer relative to the transporter polypeptides is outlined in table 10 below. All constructs showed the expected molecular weight.

**Table 10: Monovalent, multivalent, and multispecific constructs that were generated by fusing the 4D5 and NM3 cargo polypeptides to either N or C terminus of transporter polypeptide 1 or transporter polypeptide 2 of ABH2.**

| **Variant** | **N terminus-transporter polypeptide 1 (SEQ ID No: 8)** | **C terminus-transporter polypeptide 1 (SEQ ID No: 8)** | **N terminus-transporter polypeptide 2 (SEQ ID No: 10)** | **C terminus-transporter polypeptide 2 (SEQ ID No: 10)** | **Valency** | **Sequences (polypeptide)** |
|---|---|---|---|---|---|---|
| 513 | NM3E | | | | monovalent | SEQ ID NO: 96 |
| | | | | | | SEQ ID NO: 10 |
| 514 | | NM3E | | | monovalent | SEQ ID No: 98 |
| | | | | | | SEQ ID NO: 10 |
| **515** | | | **NM3E** | | **monovalent** | SEQ ID NO: 8 |
| | | | | | | SEQ ID No: 100 |
| 516 | | | | NM3E | monovalent | SEQ ID NO: 8 |
| | | | | | | SEQ ID No: 102 |
| 517 | 4D5 | | | | monovalent | SEQ ID No: 104 |
| | | | | | | SEQ ID NO: 10 |
| 518 | | 4D5 | | | monovalent | SEQ ID No: 106 |
| | | | | | | SEQ ID NO: 10 |
| 519 | | | 4D5 | | monovalent | SEQ ID NO: 8 |
| | | | | | | SEQ ID No: 108 |
| 520 | | | | 4D5 | monovalent | SEQ ID NO: 8 |
| | | | | | | SEQ ID No: 110 |
| 521 | NM3E | | NM3E | | bivalent | SEQ ID No: 96 |
| | | | | | | SEQ ID No: 100 |
| 522 | NM3E | | | NM3E | bivalent | SEQ ID No: 96 |
| | | | | | | SEQ ID No: 102 |
| 523 | | NM3E | NM3E | | bivalent | SEQ ID No: 98 |
| | | | | | | SEQ ID No: 100 |
| 524 | | NM3E | | NM3E | bivalent | SEQ ID No: 98 |
| | | | | | | SEQ ID No: 102 |
| 525 | 4D5 | | 4D5 | | bivalent | SEQ ID No: 104 |
| | | | | | | SEQ ID No: 108 |
| 526 | 4D5 | | | 4D5 | bivalent | SEQ ID No: 104 |
| | | | | | | SEQ ID No: 110 |
| 527 | | 4D5 | 4D5 | | bivalent | SEQ ID No: 106 |
| | | | | | | SEQ ID No: 108 |
| 528 | | 4D5 | | 4D5 | bivalent | SEQ ID No: |
| | | | | | | 106+108/110 |
| 529 | NM3E | NM3E | | | bivalent | SEQ ID No:112 |
| | | | | | | SEQ ID NO: 10 |
| 530 | | | NM3E | NM3E | bivalent | SEQ ID NO: 8 |
| | | | | | | SEQ ID No: 114 |
| 531 | 4D5 | 4D5 | | | bivalent | SEQ ID No: 116 |
| | | | | | | SEQ ID NO: 10 |
| 532 | | | 4D5 | 4D5 | bivalent | SEQ ID NO: 8 |
| | | | | | | SEQ ID No: 118 |
| 540 | 4D5 | 4D5 | | 4D5 | trivalent | SEQ ID No: 116 |
| | | | | | | SEQ ID No: 110 |
| 542 | 4D5 | 4D5 | 4D5 | 4D5 | tetravalent | SEQ ID No: 116 |
| | | | | | | SEQ ID No: 118 |
| 543 | NM3E | | 4D5 | | bispecific | SEQ ID No: 96 |
| | | | | | | SEQ ID No: 108 |
| 544 | NM3E | | | 4D5 | bispecific | SEQ ID No: 96 |
| | | | | | | SEQ ID No: 110 |
| 545 | | NM3E | 4D5 | | bispecific | SEQ ID No: 98 |
| | | | | | | SEQ ID No: 108 |
| 546 | | NM3E | | 4D5 | bispecific | SEQ ID No: 98 |
| | | | | | | SEQ ID No: 110 |
| 547 | 4D5 | | NM3E | | bispecific | SEQ ID No: 104 |
| | | | | | | SEQ ID No: 100 |
| 548 | | 4D5 | NM3E | | bispecific | SEQ ID No: 106 |
| | | | | | | SEQ ID No: 100 |
| 549 | 4D5 | | | NM3E | bispecific | SEQ ID No: 104 |
| | | | | | | SEQ ID No: 102 |
| 550 | | 4D5 | | NM3E | bispecific | SEQ ID No: 106 |
| | | | | | | SEQ ID No: 102 |
| 551 | NM3E | 4D5 | | | bispecific | SEQ ID No: 120 |
| | | | | | | SEQ ID NO: 10 |
| 552 | 4D5 | NM3E | | | bispecific | SEQ ID No: 122 |
| | | | | | | SEQ ID NO: 10 |
| 553 | | | NM3E | 4D5 | bispecific | SEQ ID NO: 8 |
| | | | | | | SEQ ID No: 124 |
| 554 | | | 4D5 | NM3E | bispecific | SEQ ID NO: 8 |
| | | | | | | SEQ ID No: 126 |
| 593 | 4D5 | | | NM3E | bispecific | SEQ ID No: 128 |
| 594 | NM3E | | | 4D5 | bispecific | SEQ ID No: 130 |

### SPR binding of monovalent ABH2 fused to a single antiCD16scFv

Purified heteromultimer ABH2 fused to a single antiCD16scFv to the N terminus of transporter polypeptide SEQ ID 10 (construct v515) was used in a binding experiment using Surface Plasmon Resonance (SPR). Soluble CD16 was covalently immobilized onto a CM5 surface and ABH2 fused to antiCD16scFv was captured and binding kinetics were determined.

*SPR supplies.* GLM sensorchips, the Biorad ProteOn amine coupling kit (EDC, sNHS and ethanolamine), and 10mM sodium acetate buffers were purchased from Bio-Rad Laboratories (Canada) Ltd. (Mississauga, ON). Recombinant Her-2 protein was purchased from eBioscience (San Diego, CA). HEPES buffer, EDTA, and NaCl were purchased from from Sigma-Aldrich (Oakville, ON). 10% Tween 20 solution was purchased from Teknova (Hollister, CA).

*SPR biosensor assays.* All surface plasmon resonance assays were carried out using a BioRad ProteOn XPR36 instrument (Bio-Rad Laboratories (Canada) Ltd. (Mississauga, ON)) with HBST running buffer (10mM HEPES, 150 mM NaCl, 3.4 mM EDTA, and 0.05% Tween 20 pH 7.4) at a temperature of 25°C. The CD 16 capture surface was generated using a GLM sensorchip activated by a 1:5 dilution of the standard BioRad sNHS/EDC solutions injected for 300 s at 30 µL/min in the analyte (horizontal) direction. Immediately after the activation, a 4.0 µg/mL solution of CD16 in 10 mM NaOAc pH 4.5 was injected in the ligand (vertical) direction at a flow rate of 25 µL/min until approximately 3000 resonance units (RUs) were immobilized. Remaining active groups were quenched by a 300 s injection of 1M ethanolamine at 30 µL/min in the analyte direction, and this also ensures mock-activated interspots are created for blank referencing.

A 500nM 3-fold dilution series of V515 was injected over 3000 RUs CD16aWT (L6) compared to blank (L5). Flow rate 50 uL / min for 120s, with a 240s disassociation phase. Injections were repeated in standard running buffer (DPBS/3.4mM EDTA/0.05% Tween20) and running buffer with an additional 350mM NaCl. Sensorgrams were aligned and double-referenced using the buffer blank injection and interspots, and the resulting sensorgrams were analyzed using ProteOn Manager software v3.0. Typically, K_{D} values were determined from binding isotherms using the Equilibrium Fit model. For high affinity interactions with slow off-rates, kinetic and affinity values were additionally determined by fitting the referenced sensorgrams to the 1:1 Langmuir binding model using local Rₘₐₓ, and affinity constants (K_{D} M) were derived from the resulting rate constants (k_{d} s⁻¹/ kₐM⁻¹s⁻¹). All K_{D} values are reported as the mean and standard deviation from three independent runs.

As shown in Table 11, ABH2 heteromultimer fused to a single antiCD16scFv has full activity and binds its target with good reproducibility and KD similar to the free anti CD 16 scFv (NM3E).

**Table 11: SPR data for monovalent ABH2 fused to a single antiCD16scFv.**

| | **Injection #1** | | | | **Infection #2** | | | | | |
|---|---|---|---|---|---|---|---|---|---|---|
| | **ka** | **kd** | **KD** | | **ka** | **kd** | **KD** | | **KD (M) Ave** | **KD SD** |
| | **1/Ms** | **1/s** | **M** | | **1/Ms** | **1/s** | **M** | | | |
| **NM3E** | 5.37E+04 | 5.76E-03 | 1.07E-07 | | 5.89E+04 | 6.03E-03 | 1.02E-07 | | 1.05E-07 | 4.E-09 |
| **V515 Dec** | 6.11E+04 | 6.71E-03 | 1.10E-07 | | | | | | | |
| **V515 Jan** | 5.56E+04 | 7.30E-03 | 1.31E-07 | | | | | | | |

### Example 4 Preparation of HA or HAA based heteromultimer proteins wherein cargo protein(s) comprise one or more EGF-A like domain.

The peptide sequence of the EGF-A domain in PCSK9 protein or another polypeptide sequence homologous to the EGF-A domain, capable of specifically binding the low density lipoprotein receptor (LDL-R) is derived by sequencing or from a database such as GenBank. The cDNA for the cargo polypeptide comprising EGF-A like domain is isolated by a variety of means including but not exclusively, from cDNA libraries, by RT-PCR and by PCR using a series of overlapping synthetic oligonucleotide primers, all using standard methods. In certain examples, the cargo protein is engineered to improve stability, target binding features or other biophysical or therapeutically relevant properties. The polypeptide is employed as the cargo protein in the creation of a heteromultimer with application in the treatment of hypercholesterolemia. The first and second monomeric fusion polypeptide sequence is derived by fusing the cargo protein sequence directly or with an intermediate linker peptide to the N-terminus and/or C-terminus of HA or HAA based transporter polypeptide such as SEQ ID No: 2, SEQ ID NO: 3, SEQ ID NO: 8 or SEQ ID NO: 10. This monomeric fusion protein sequence is reverse translated to its corresponding DNA sequence to be introduced in an expression vector, sequence optimized for expression in a particular cell line of interest. The first and second monomeric fusion proteins are transfected and coexpressed in the cell line of interest. In certain cases, the transfection is in 1:1 ratio for the two vectors. In some examples, the ratio is selected from 1.5:1, 2:1, 1:1.5, 1:2 etc.

### Example 5 Preparation of HA or HAA based heteromultimeric proteins wherein cargo protein(s) are the GLP-1 and/or Glucagon.

The peptide sequence of GLP-1 or another polypeptide sequence homologous to this peptide, capable of specifically binding the GLP-1 receptor or acting as a GLP-1 agonist is derived by sequencing or from a database such as GenBank. Alternately, the peptide sequence of Glucagon or another polypeptide sequence homologous to this peptide, capable of specifically binding the Glucagon receptor or acting as a Glucagon receptor agonist is derived by sequencing or from a database such as GenBank. The cDNA for each cargo polypeptide comprising GLP-1 or Glucagon is isolated by a variety of means including but not exclusively, from cDNA libraries, by RT-PCR and by PCR using a series of overlapping synthetic oligonucleotide primers, all using standard methods. In certain examples, these GLP-1 or Glucagon based cargo polypeptides are engineered to improve stability, target binding features or other biophysical or therapeutically relevant properties. These GLP-1 and Glucagon based polypeptides are employed as one or more cargo molecules in the creation of a heteromultimer with application in the treatment of type-2 diabetes or another disease related to glucose metabolism. The first and second monomeric fusion polypeptide sequence is derived by fusing the cargo protein sequence directly or with an intermediate linker peptide to the N-terminus and/or C-terminus of HA or HAA based transporter polypeptide such as SEQ ID No: 2, SEQ ID NO: 3, SEQ ID NO: 8 or SEQ ID NO: 10. The fusion proteins can be monospecific with either GLP-1 or Glucagon like polypeptides or be bispecific (coagonist) with both the GLP-1 and Glucagon like polypeptides. Each monomeric fusion protein sequence is reverse translated to its corresponding DNA sequence to be introduced in an expression vector, sequence optimized for expression in a particular cell line of interest. The first and second monomeric fusion proteins are transfected and coexpressed in the cell line of interest. In certain cases, the transfection is in 1:1 ratio for the two vectors. In some examples, the ratio is selected from 1.5:1, 2:1, 1:1.5, 1:2 etc.
Sequence of Cargo molecule GLP-1
   SEQ ID No: 12: HAEGTFTSDVSSYLEGQAAKEFIAWLVKGRG
Sequence of Cargo molecule Glucagon
   SEQ ID NO: 13: HSQGTFTSDYSKYLDSRRAQDFVQWLMNT

### Example 6: Annexin protein repeat as membrane-sensing multivalent scaffold

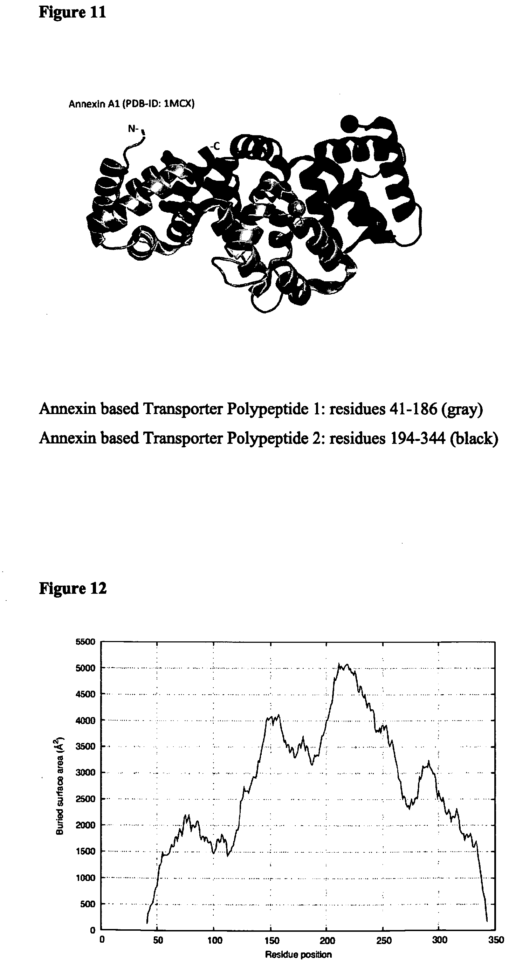

Annexin is split with an extensive interface to generate a multivalent heteromultimer scaffold comprising two transporter polypeptides. Annexin is a 346 residue protein (PDB ID 1MCX). Heteromultimer comprising two transporter polypeptides based on annexin split in the region between residue 186 and 194 is shown in Figure 11. When co-expressed in solution, the large interfacial area between the two transporter polypeptides leads to self-assembly of the heterodimer. The self-assembly of the two units allows for the design of multivalent construct with transporter polypeptides based on the annexin core. Two structures are available, Pig and Human. The two structures are superimposable with an rmsd of 0.6 A. The following stretch of sequence can be removed from the human Annexin sequence DRSEDF (residues 160 through 165). The truncation does not break any secondary structure element and does not involve introducing or removing any Proline residue.
Human annexin WT Sequence 
SEQ ID NO: 14:



 



Sequence of Annexin based transporter polypeptide-1: 
SEQ ID NO: 15:
SAVSPYPTFNPSSDVAALHKAIMVKGVDEATIIDILTKRNNAQRQQIKAAYLQETGKPLDE
TLKKALTGHLEEVVLALLKTPAQFDADELRAAMKGLGTDEDTLIEILASRTNKEIRDINRV
YREELKRDLAKDITSDTSGDFRNALLSLAKG
Sequence of Annexin based transporter polypeptide-2:

SEQ ID NO: 16:
GVNEDLADSDARALYEAGERRKGTDVNVFNTILTTRSYPQLRRVFQKYTKYSKHDMNKV
LDLELKGDIEKCLTAIVKCATSKPAFFAEKLHQAMKGVGTRHKALIRIMVSRSEIDMNDIK
AFYQKMYGISLCQAILDETKGDYEKILVALCGGN

Figure 12 shows a plot of the buried solvent accessible surface area at the interface of Annexin based tranporter polypeptide-1 (ABT-1), and Annexin based tranporter polypeptide-2 (ABT-2). A split annexin near residue position 186 forms a heterodimer with about 3200 Å2 of buried surface area. The transporter polypeptides such as ABT-1 and ABT-2 based on Annexin can be used to attach cargo biomolecules using the same methods as described above for albumin based transporter polypeptides.

### Example 7: Transferrin as a multivalent scaffold

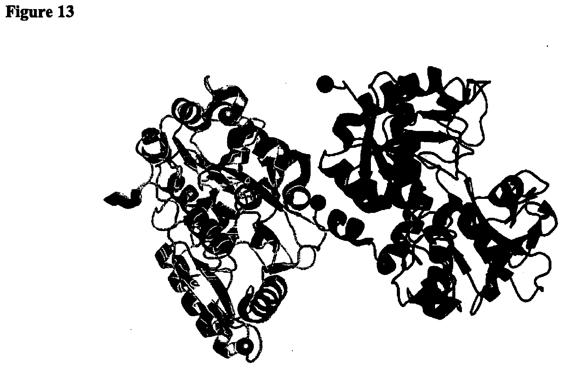

Based on the large number of therapeutically relevant properties of transferrin, this protein presents itself as an interesting scaffold molecule for the design of multivalent protein fusion drugs following the creation of a self-assembling protein and its split component parts. The structure of transferrin is shown in Figure 13 based on the crystal structure (1H76) available in the protein data bank [Hall DR et al. Acta Crystallogr D 2002, 58, 70-80]. The transferrin molecule is composed of two structurally similar lobes, the N and C terminal lobes, connected by a short peptide linker between residues 333 and 342.

A heterodimer is designed based on transferrin protein, said heterodimer comprising a first transporter polypeptide involving residues 1-333 of transferrin and a second transporter polypeptide composed of residues from 342 to the C-terminus of the original transferrin sequence. When coexpressed, the two transporter polypeptides fold independently and pair to form a quasi-transferrin scaffold capable of maintaining its therapeutically relevant properties. Furthermore, such a Transferrin scaffold allows for the production of multivalent fusion molecules, e.g. a multivalent GLP-1 fusion with trasnsporter polypeptides based on transferring. These fusions can be similar to the GLP-1-fusion polypeptides with Albumin based transporter polypeptides.

Figure 13 provides structure of transferrin molecule based on the PDB structure 1H76. The two monomering transporter polypeptides derived by splitting the transferrin molecule are color coded as light and dark grey units. The sites of fusion for the cargo molecules are represented as spheres.

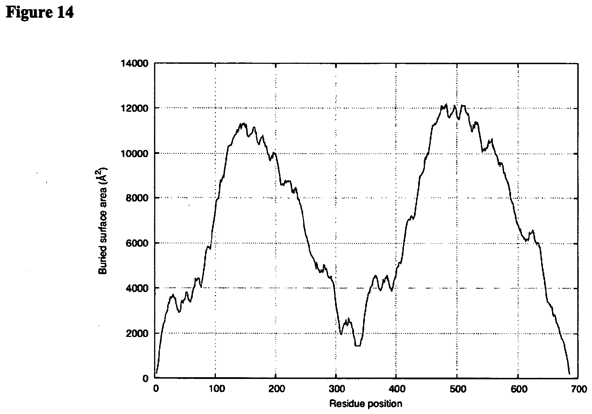

Figure 14 shows a plot of the buried solvent accessible surface area at the interface of two transferrin based polypeptides. A split transferrin near residue position 330 such as the two transporter polypeptides shown below, forms a heterodimer with about 1800 Å2 of buried surface area.
Sequence of Transferrin based transporter polypeptide-1: 
SEQ ID NO: 17:
MRLAVGALLV CAVLGLCLAV PDKTVRWCAV SEHEATKCQS FRDHMKSVIP
SDGPSVACVK KASYLDCIRA IAANEADAVT LDAGLVYDAY LAPNNLKPVV
AEFYGSKEDP QTFYYAVAVV KKDSGFQMNQ LRGKKSCHTG LGRSAGWNIP
IGLLYCDLPE PRKPLEKAVA NFFSGSCAPC ADGTDFPQLC QLCPGCGCST
LNQYFGYSGA FKCLKDGAGD VAFVKHSTIF ENLANKADRD QYELLCLDNT
RKPVDEYKDC HLAQVPSHTV VARSMGGKED LIWELLNQAQ EHFGKDKSKE
FQLFSSPHGK DLLFKDSAHG FLKVPPRMDA KMYLGYEYVT AIRNLREG.
Sequence of Transferrin based transporter polypeptide-2:

SEQ ID NO: 18:ECKPVKWCALSHHE RLKCDEWSVN SVGKIECVSA ETTEDCIAKI
MNGEADAMSL DGGFVYIAGK CGLVPVLAEN YNKSDNCEDT PEAGYFAVAV
VKKSASDLTW DNLKGKKSCH TAVGRTAGWN IPMGLLYNKI NHCRFDEFFS
EGCAPGSKKD SSLCKLCMGS GLNLCEPNNK EGYYGYTGAF RCLVEKGDVA
FVKHQTVPQN TGGKNPDPWA KNLNEKDYEL LCLDGTRKPV EEYANCHLAR
APNHAVVTRK DKEACVHKIL RQQQHLFGSN VTDCSGNFCL FRSETKDLLF
RDDTVCLAKL HDRNTYEKYL GEEYVKAVGN LRKCSTSSLL EACTFRRP

### Example 8: Design of disulfide stabilized HA or HAA based heteromultimer proteins

Albumin based transporter polypeptides were designed wherein as compared to albumin, specific positions were mutated to cysteine in each of the transporter polypeptides to enable the selective formation of a disulfide link between two transporter polypeptide, resulting in a stable heterodimer with a quasi-native albumin monomer structure.

The residues for the introduction of cysteines were selected taking into consideration the following factors:
a. Form a covalent bond between the two transporter polypeptides, thereby increasing the stability of the heteromultimer and the resultant quasi-native monomeric form.
b. must be within specific CA-CA and CB-CB limits to form a disulphide bond
c. compatible local environment (generally preferring shorter hydrophobic side chains in partially solvent exposed regions) is not likely to interfere with existing polar contacts.

Mutagenesis was performed by QuickChange, and expression and purification were performed as presented in Examples 1 and 2 above.

Table 12 below identifies a number of albumin-based heteromultimers comprising introduced cysteines that were designed. ABH3-8 were designed based on the ABH2 design, while ABH9-12 were based on the ABH1 design.

**Table 12: Albumin based heteromultimers comprising introduced cysteines**

| **Hetero-multimer** | **Variant ID** | **Transporter polypeptide 1 (first-last amino acid of the N-terminal albumin segment are denoted)** | **Transporter polypeptide 2 (first-last amino acid of the C-terminal albumin segment are denoted)** | **New Cys in transporter polypeptide 1** | **New CYS in transporter polypeptide 2** |
|---|---|---|---|---|---|
| ABH3 | 881 | 1-337 (SEQ ID NO 19) | 342-585 (SEQ ID NO 20) | A217C | V343C |
| ABH4 | 882 | 1-337 (SEQ ID NO 21) | 342-585 (SEQ ID NO 22) | L331C | A350C |
| ABH5 | 883 | 1-337 (SEQ ID NO 23) | 342-585 (SEQ ID NO 24) | A217C | L346C |
| ABH6 | 884 | 1-337 (SEQ ID NO 25) | 342-585 (SEQ ID NO 26) | W214C | V343C |
| ABH7 | 885 | 1-337 (SEQ ID NO 27) | 342-585 (SEQ ID NO 28) | A335C | L346C |
| ABH8 | 886 | 1-337 (SEQ ID NO 29) | 342-585 (SEQ ID NO 30) | L198C | V455C |
| ABH9 | 887 | 1-293 (SEQ ID NO 31) | 304-585 (SEQ ID NO 32) | A217C | A335C |
| ABH10 | 888 | 1-293 (SEQ ID NO 33) | 304-585 (SEQ ID NO 34) | A217C | L331C |
| ABH11 | 889 | 1-293 (SEQ ID NO 35) | 304-585 (SEQ ID NO 36) | L198C | N458C |
| ABH12 | 890 | 1-293 (SEQ ID NO 37) | 304-585 (SEQ ID NO 38) | A194C | V455C |

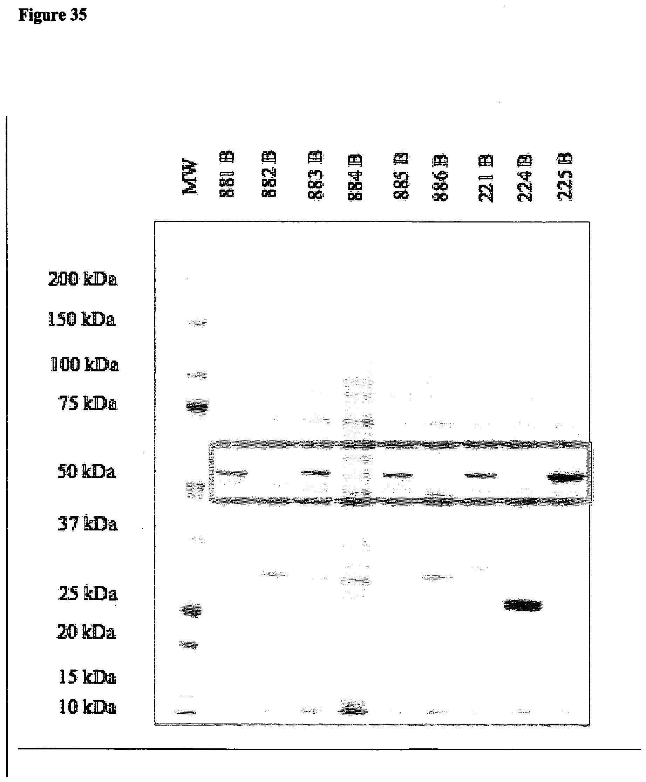

Variants ABH3, ABH4, ABH5, ABH6, ABH7 and ABH8 were expressed with DNA ratio 1:1 (Fragment 1 :Fragment 2) in small scale 2 mL CHO cultures. A gel of expression is shown in Figure 35. WT-Albumin (v225) along with previous variants AlbuCORE 1 and AlbuCORE 2 were also expressed as controls. Variant ABH3, ABH5 and ABH7 expressed similarly to ABH2, while the remaining variants displayed lower expression and possible disulfide scrambling, noticeable by multiple bands in non-reducing SDS PAGE, potentially because of the presence of the natural disulfide positions as well as the introduced cysteine mutations. Recovery of expression may be achieved by changing the cut site (see Example 9) or transfecting with different DNA ratios. No Linkers were present at the termini of these constructs. Introduction of the disulfide bonding cysteine mutations in variants such as ABH9, ABH10, ABH11 and ABH12 are expected to address this issue since they lack a natural disulfide bonding positions in the original interface..

### Example 9: Design of HA or HAA based heteromultimer proteins based on different segmentation sites

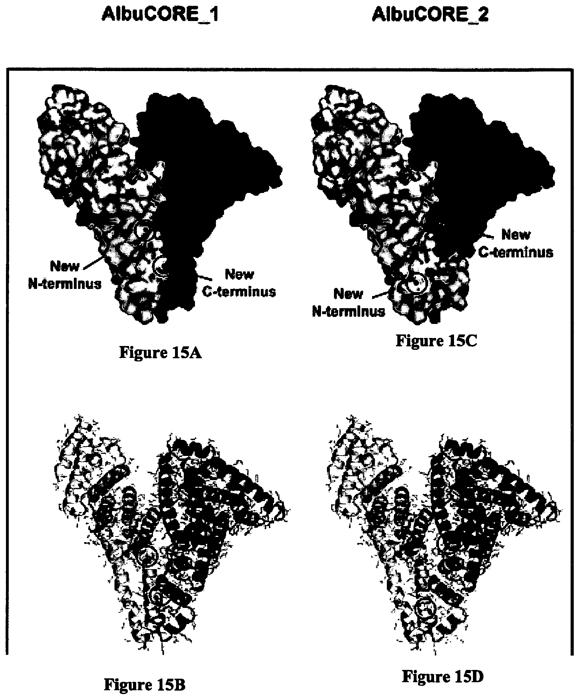

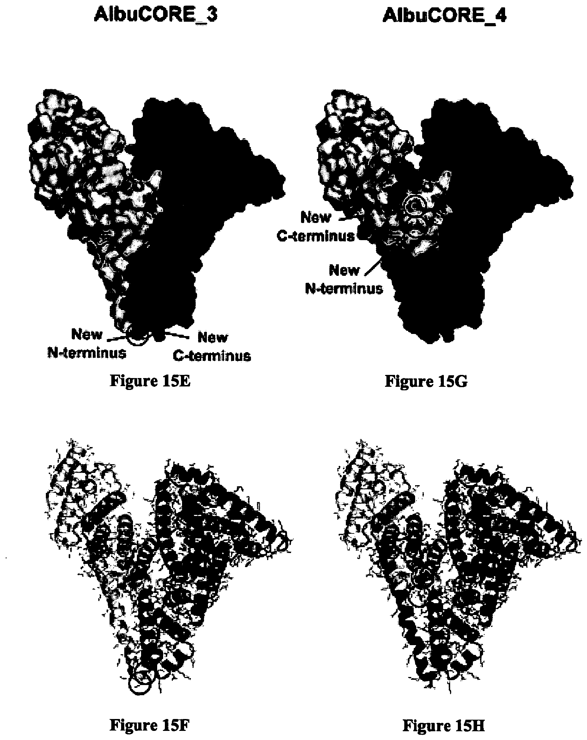

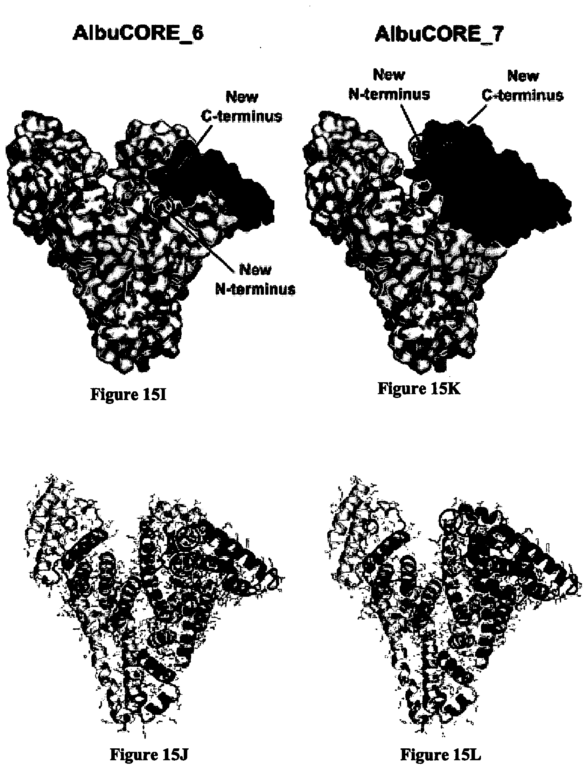

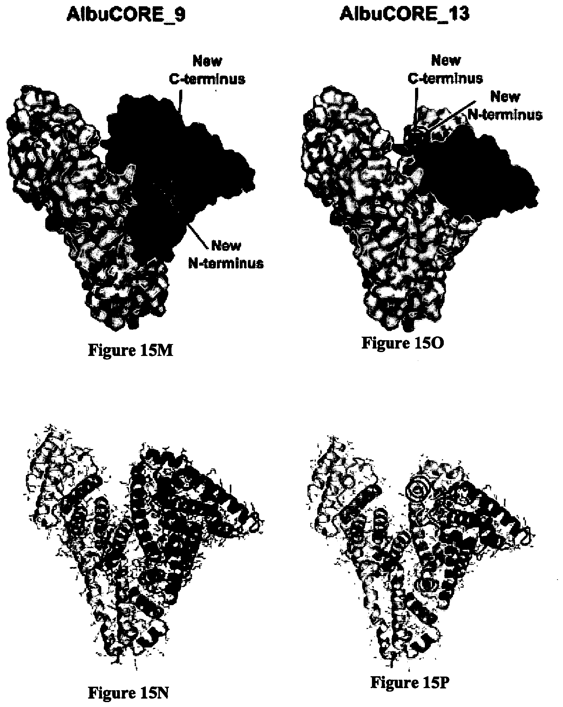

Table 13 below provides heteromultimer constructs as described herein comprising transporter polypeptides designed by segmenting albumin at various sites as shown in Figures 15A-15P and listed in Table 1. Differently from previous constructs described in Example 8 and proceeding ones, all AlbuCORE constructs in Example 9 were created with a GGGGS linker fused to the C terminus of Fragment 1 and the N terminus of fragment 2. This was done for multiple reasons: 1) The presence of a short linker re-creates a typical fusion molecule and allows us to understand the compatibility of the cut site(s) with the presence of a linker. 2) The presence of a linker may also help stabilize the local environment surrounding the cut site, including shielding some exposed hydrophobic patches. 3) Presence of a linker reduces the number of variables in subsequent phases, when a warhead is fused to the molecule. See Table 13 for expression results. Expression was initially tested in small scale CHO cultures with the two fragment in each AlbuCORE variant performed in different combinations of ratios. The gel in the Figures 35A and B illustrates the expression results.

**Table 13: Albumin based heteromultimers constructs based on different albumin segments.**

| **Heteromultimer** | **ID** | **Transporter polypeptide 1 (first-last amino acid of the N-terminal albumin segment are denoted)** | **Transporter polypeptide 2 (first-last amino acid of the C-terminal albumin segment are denoted)** |
|---|---|---|---|
| ABH13 | AlbuCORE_1A | 1-339 (SEQ ID NO 39) | 340-585 (SEQ ID NO 40) |
| ABH14 | AlbuCORE_2A | 1-300 (SEQ ID NO 41) | 301-585 (SEQ ID NO 42) |
| ABH15 | AlbuCORE_3 | 1-364 (SEQ ID NO 43) | 365-585 (SEQ ID NO 44) |
| ABH16 | AlbuCORE_4 | 1-441 (SEQ ID NO 45) | 442-585 (SEQ ID NO 46) |
| ABH17 | AlbuCORE_6 | 1-83 (SEQ ID NO 47) | 85-585 (SEQ ID NO 48) |
| ABH18 | AlbuCORE_7 | 1-171 (SEQ ID NO 49) | 172-585 (SEQ ID NO 50) |
| ABH19 | AlbuCORE_9 | 1-281 (SEQ ID NO 51) | 282-585 (SEQ ID NO 52) |
| ABH20 | AlbuCORE_13 | 1-114 (SEQ ID NO 53) | 115-585 (SEQ ID NO 54) |

Expression of selected variants was performed as described in Example 2 , while purification was carried out as described in Example 17. The characteristics of these molecules with respect to the linkers used, disulphide cross-links, and a summary of the expression results are shown in Table 14.

**Table 14: Results of expression of variants listed in Table 13:**

| SEQ ID (polypeptide) | **Variant** | **Scaffold** | **Split /Deletion** | **frag1:frag2** | **frag1 MW** | **frag2 MW** | **Interface S-S *[disulfide link between the two fragments forming AlbuCORE]*** | **Optimal DNA ratio (Fragment 1:Fragment 2)** | **Expression (compared to AlbuCORE_1 and cloned WTHSA). Rough estimate based on band intensity (assessed by software).** | **Linkers** |
|---|---|---|---|---|---|---|---|---|---|---|
| 39+40 | 1634 | AlbuCORE_1A | nick(339-340) | 1-339:340-585 | 39.03 kDa | 28.10 kDa | 316.CYS - 361.CYS | 1:1 | ∼ 30% better than closely related AlbuCORE _1. Single fragments still visible in 1:0 and 0:1 DNA ratios. When in excess, no clear signs of multimeriza tion. | Fragment 1: GGGGS (new C terminus) Fragment 2: GGGGS (new N terminus) |
| 41+42 | 1635 | AlbuCORE_2A | nick(300-301) | 1-300:301-585 | 34.53 kDa | 32.59 kDa | N/A | Only 1:1,1:0 and 0:1 was tested | Expression is ∼30 % better than AlbuCORE _1 but needs native gel for assessment of complex. | Fragment 1: GGGGS (new C terminus) Fragment 2: GGGGS (new N terminus) |
| 43+44 | 1636 | AlbuCORE_3 | nick(364-365) | 1-364:365-585 | 41.78 kDa | 25.35 kDa | 360.CYS-369.CY | **1:2** | **∼ 30-50 % better.** Fragment 1 tends to multimerize when in access (DNA ratio not optimized) | Fragment 1: GGGGS (new C terminus) Fragment 2: GGGGS (new N tenninus) |
| 45+46 | 1637 | AlbuCORE_4 | nick(441-442) | 1-441:442-585 | 50.65 kDa | 16.48 kDa | 437.CYS - 448.CYS | **1:2** | **∼ 30-50 % better.** Fragment 1 in excess when DNA ratio not optimized. No signs of multimerization. | Fragment 1: GGGGS (new C terminus) Fragment 2: GGGGS (new N terminus) |
| 47+48 | 1638 | AlbuCORE_6 | Δ(84) | 1-83:85-585 | 9.68 kDa | 57.29 kDa | 75.CYS - 91.CYS | Only 1:1, 1:0 and 0:1 was tested | Faint dimer band present, 1:1 appears fine. Fragment 2 tends to multimerize when expressed in 0:1 DNA ratio. **In 1:1 no clear signs of scrambling nor multimerization.** | Fragment 1: GGGGS (new C terminus) Fragment 2: GGGGS (new N terminus) |
| 49+50 | 1639 | AlbuCORE_7 | nick(171-172) | 1-171:172-585 | 20.18 kDa | 46.95 kDa | 168.CYS-177.CY S | Weak expression | 50 % or more lower. | Fragment 1: GGGGS (new C terminus) Fragment 2: GGGGS (new N terminus) |
| 51+52 | 1640 | AlbuCORE_9 | nick(281-282) | 1-281:282-585 | 32.43 kDa | 34.70 kDa | 278.CYS - 289.CYS | **2:1** | **~ 30-50 % better.** Fragment 1 tends to multimerize when in access (DNA ratio not optimized). Results unclear for 0:1 lane. | Fragment 1: GGG GS (new C terminus) Fragment 2: GGGGS (new N terminus) |
| 53+54 | 1641 | AlbuCORE_13 | nick(114-115) | 1-114:115 -585 | 13.31 kDa | 53.82 kDa | N/A | Weak expression | 50 % or more lower. | Fragment 1: GGGGS (new C terminus) Fragment 2: GGGGS (new N terminus) |
| This is ABH2 | 221 | AlbuCORE_1 (CONTROL) | nick(338-341) | 1-337:342 -585 | 38.48 kDa | 27.51 kDa | 316.CYS - 361.CYS | | Weak expression of Fragment 1 and Fragment 2 still visible in 1:0 and 0:1. The single fragments show sign of aggregation, likely due to disulfide scrambling. Awaiting reducing SDS-PAGE to confirm. | Fragm ent 1: GGGGS (new C terminus) Fragment 2: GGGGS (new N terminus) |

### Example 10: Multiple Cargo Proteins

The heteromultimer proteins described herein (e.g, containing a cargo polypeptide (or fragment or variant thereof) fused to transporter albumin segment or variant thereof) may additionally be fused to other proteins to generate "multifusion proteins". These multifusion proteins can be used for a variety of applications. For example, fusion of the proteins described herein to His-tag IgG domains, and maltose binding protein facilitates purification. (See e.g EP A 394,827; Traunecker et al., Nature 331:84-86 (1988)). Nuclear localization signals fused to the polypeptides can target the protein to a specific subcellular localization. Furthermore, the fusion of additional protein sequences to proteins described herein may further increase the solubility and/or stability of the heteromultimer. The heteromultimer proteins described above can be made using or routinely modifying techniques known in the art and/or by modifying the following protocol, which outlines the fusion of a polypeptide to an IgG molecule.

Briefly, the human Fc portion of the IgG molecule can be PCR amplified, using primers that span the 5' and 3' ends of the sequence described below. These primers also should have convenient restriction enzyme sites that will facilitate cloning into an expression vector, preferably a mammalian or yeast expression vector.

For example, if pC4 (ATCC Accession No. 209646) is used, the human Fc portion can be ligated into the BamHI cloning site. Note that the 3' BamHI site should be destroyed. Next, the vector containing the human Fc portion is re-restricted with BamHI, linearizing the vector, and a polynucleotide encoding a heteromultimeric protein described herein (generated and isolated using techniques known in the art), is ligated into this BamHI site. Note that the polynucleotide encoding the fusion protein of the invention is cloned without a stop codon; otherwise an Fc containing fusion protein will not be produced.

If the naturally occurring signal sequence is used to produce the heteromultimeric protein described herein, pC4 does not need a second signal peptide. Alternatively, if the naturally occurring signal sequence is not used, the vector can be modified to include a heterologous signal sequence. (See, e.g., International Publication No. WO 96/34891.)

### Example 11. Preparation of anti-Her2, monospecific, mono- to tetra-valent cargo-loaded albumin-based polypeptides; anti- Her2 x Her3 bi-specific, bivalent cargo loaded albumin-based polypeptides; and various controls.

A number of different cargo-loaded albumin based transporter polypeptides were prepared as described in this example. The anti-HER2 scFvs described in Table 8 of Example 3 were prepared as follows. Briefly, ABH2 was loaded with anti-Her2 scFvs in various cis and trans configurations according to the methods described here. The final mono-specific polypeptide ranged from mono to tetra-valent. Details of the amino acid and nucleic acid sequences of the component polypeptides are also shown in Table 8. In a similar fashion, ABH2 was also loaded with anti-Her2 and anti-Her3 scFvs in both cis and trans configurations, producing bi-specific, bivalent molecules. As controls utilized in various Examples, V1087, single scFvs, one-arm Fcs and bivalent Fcs were also produced.

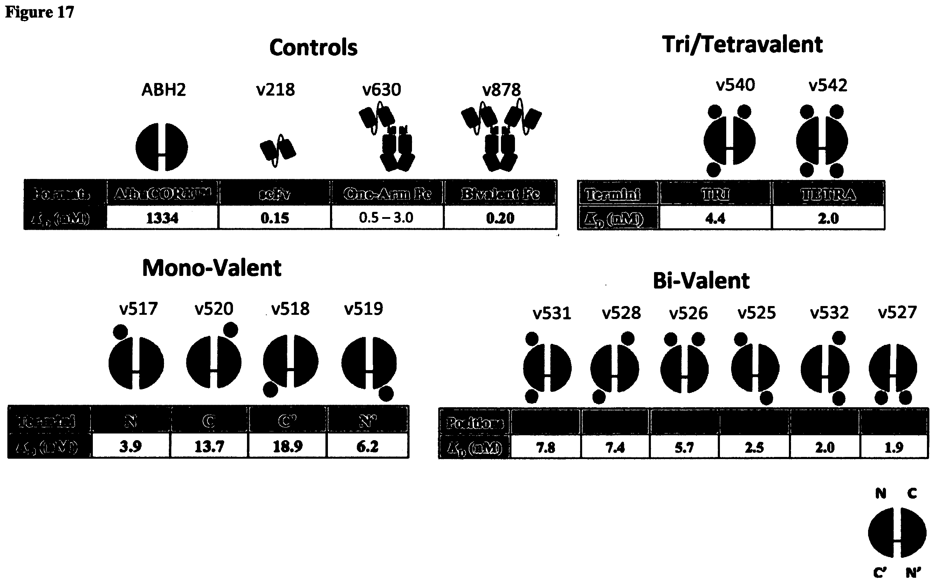

*ABH2 loaded with anti-Her2 4D5 scFv.* The different possible configurations of ABH2 loaded anti-Her2 are schematically rendered in Figure 17. As this figure shows, numerous cis and trans variations were prepared depending on whether the scFv is attached to the N or C termini of either fragment A or B of ABH2. A short GGSG linker was designed at the natural N terminus of ABH2 Fragment 1 and a longer (GGS)₄GG linker was designed at the C terminus of ABH2 Fragment 1 and N and C termini of ABH2 Fragment 2. The final gene products were subcloned into the mammalian expression vector pTT5 (NRC-BRI, Canada) (Durocher et al). High level and high-throughput recombinant protein production by transient transfection of suspension-growing human CHO-3E7 was performed. Purification was performed by application of the cellular supernatant with expressed protein to a QIAGEN-tip 500 column packed with Blue Sepharose matrix (GE Healthcare) coupled to an AKTA Express system (GE Healthcare) using a flow rate of 1 ml/ml. The column was equilibrated with equilibrated with sample buffer composed of 20 ml of PBS pH 7.2, 300 mM NaCl. The sample was loaded at a flow rate of 5 ml/min and subsequently washed with sample buffer. The protein was eluted by application of NaCl gradient ranging from 300 mM to 2000 mM. Fractions eluting in higher salt concentration were the purest and were pooled, concentrated and subsequently applied to a HiLoad 16/60 Superdex 200 prep grade gel filtration column coupled to an AKTA Express system (GE Healthcare) using a flow rate of 1 ml/ml. Protein with a purity of >85% was collected; fractions containing pure sample were pooled and concentrated by centrifugation using an Amicon Ultra membrane with a cutoff weight of 10 000 MWCO. Figures 10A-10B shows SDS-PAGE (non-reducing) analysis of the ABH2 heteromultimer fused to different cargo polypeptides. The position of those polypeptides in the heteromultimer relative to the transporter polypeptides is outlined in Table 8, and in Figure 17. All constructs showed the expected molecular weight. Monovalent ABH2 fused to one anti-Her2 4D5 scFv fusion are exemplified by variants 517, 518, 519, 520 as described in Table 8. Bivalent and trivalent ABH2 fused to two or three anti-Her2 4D5 scFv fusions are exemplified by variants 525, 526, 527, 528, 531, 532, 540 as described in Table 8. All of these variants were produced by co-expression of the two Albumin fragments as described in the following paragraph. Finally, attaching anti-Her2 scFvs to all four possible termini formed the tetravalent fusion protein, referred to as variant 542.

### ABH2 loaded with anti-Her2 x anti-Her3 scFvs (variants 1377, 1378, and 1090).

Different versions of albumin-based heteromultimers fused to anti-HER2 and anti-HER3 were prepared. The anti-HER2 and anti-HER3 warheads that were used target the same receptors as MM-111, with the main difference being the anti-HER2 warhead, which was either the non-neutralizing B1D2 warhead used in MM-111 or the neutralizing 4D5 warhead.

AlbuCORE_1 (ABH2) fragment 1 was fused to anti-HER3 at the N terminus through a GGGS linker and complexed with fragment 2 fused to antiHER2 (4D5) through a (GGSG)4GG linker positioned at the N terminus of fragment 2 (yielding variant 1378, with warheads in trans) or the C terminus of fragment 2 (yielding v1377, with warheads in cis). A third type of AlbuCORE_1 (ABH2) molecule was formed by combining fragment 1 fused to antiHER3 at its N terminus through a GGGS linker and fragment 2 fused to antiHER2 (B1D2) at its C terminus through a GGGS linker (variant number 1090). This molecule has warheads in cis and is almost identical to MM-111. The main differences between the MM-111 polypeptide and the ABH2-based polypeptide are that 1) the linkers used in the MM-111 polypeptide were more hydrophobic, while the linkers used on the ABH2-based polypeptides were polyGLY)(S) and 2) The ABH2-based polypeptides lack the C34S/N504Q mutation originally introduced by Merrimack.

### MM-111 and derivatives (variant 1087, 1088, and 1089)

The MM-111 molecule is a single polypeptide fusion protein of two scFvs, anti-Her2 (B1D2) and anti-Her3 (H3), linked to the C and N termini, respectively, of a modified human serum albumin protein, and is produced by Merrimack. The resulting molecule is bispecific and bivalent. As a control, a variant of the MM-111 molecule was constructed in which the anti-Her3 (H3) warhead was fused to the N terminus of albumin by a short AAS linker, while anti-Her2 (B1D2) was fused to the C terminus through an AAAL linker to create the benchmark control variant 1087. This control variant lacks the C34S/N504Q mutation originally introduced by Merrimack in their MM-111 molecule. Additional MM-111-related molecules were constructed: 1) albumin fused to only the anti-Her3 (H3) warhead at the N terminus (v1088) and 2) the anti-Her2 (B1D2) fused to the C terminus (vl089). In both these cases the linkers used were identical to MM-111. All molecules were expressed in a 1:1 ratio in CHO cells and purified as described for the multimeric 4D5 fusions.

### Single anti-Her2 scFvs

Free Anti-Her2 human scFv (v218) was expressed in 1L CHO3E7 cells (v218). A (His)6 tag was fused to the C terminus through a cleavable linker containing a TEV protease site. The protein was purified by metal (Co2+) affinity (column volume=1 mL) coupled to a fractogel system followed by Superdex 200 size exclusion chromatography. Post purification yields were 3.6 mg of 4D5 scFv after Co2+ affinity and 1.38 mg after size exclusion.

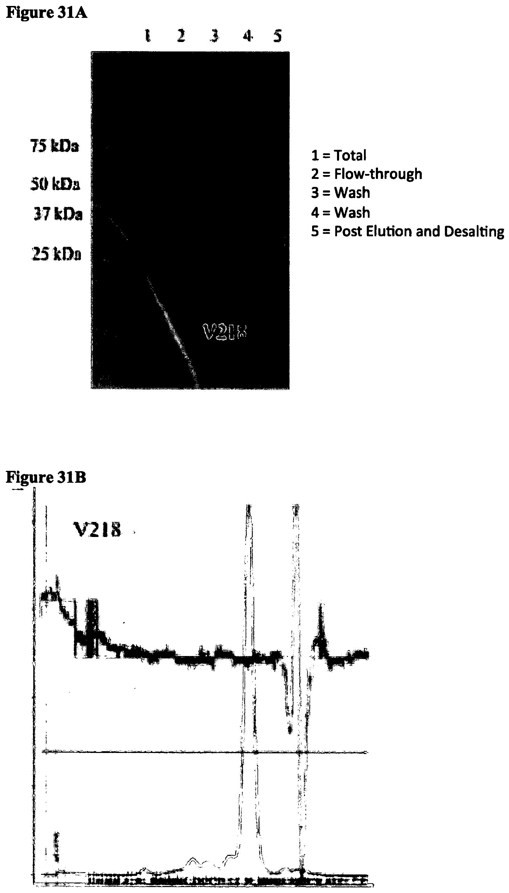

Figure 31A is a SDS-PAGE depicting the purification of v218 after Co2+ affinity. The expression product post-washes, column elution and desalting are provided. Figure 31B is the chromatography profile of the variant following the second purification through the size exclusion column.

### Control Fc molecules (878, 628 and 630):

A single anti-Her2 (4D5) human scFv was fused to an Fc fragment to produce a monovalent, mono-specific antibody product. In order to ensure formation of the heterodimeric Fc, heterodriver mutations were included in the Fc chain.

V878: OA4-scFv-B1D2, a monovalent anti-Her2 antibody, where the Her2 binding domain is a scFv on chain A, and the Fc region is a heterodimer having the mutations L351Y_F405A_Y407V in Chain A, and T366L_K392M_T394W in Chain B.

V628: a bivalent anti-Her2 antibody, where both Her2 binding domains are in the scFv format, and the Fc region is a heterodimer having the mutations L351Y_S400E_F405A_Y407V in Chain A, and T366I_N39OR_K392M_T394W in Chain B.

V630: a monovalent anti-Her2 antibody, where the Her2 binding domain is an scFv, and the Fc region is a heterodimer having the mutations L351Y_S400E_F405A_Y407Vin Chain A, and T366I_N390R_K392M_T394W in Chain B.

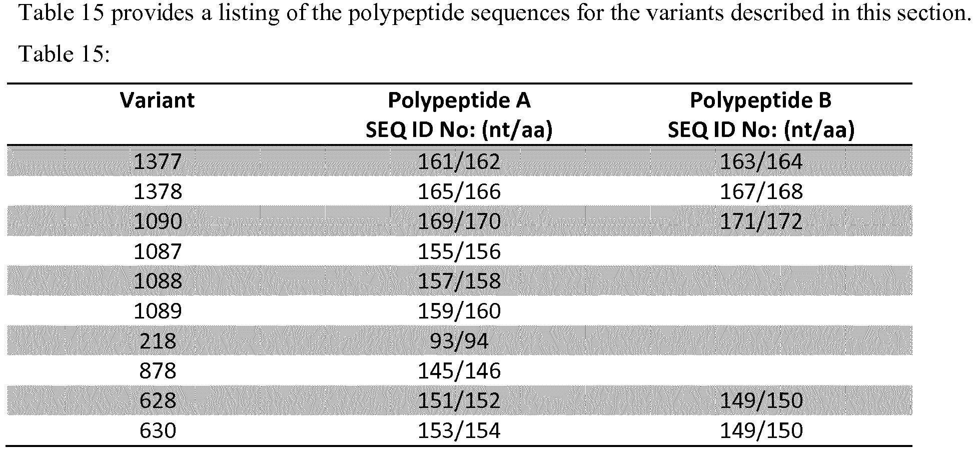

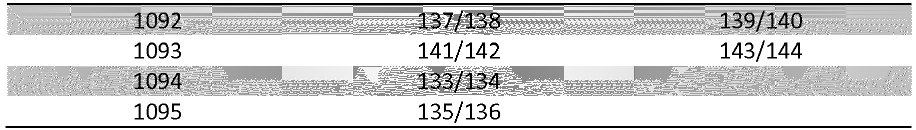

### Example 12. Target binding to SKOV cells by ABH2 fused to anti-Her2 4D5 scFvs in multivalent configurations.

The affinity of ABH2 anti-Her2 fusion proteins to Her2⁺ cells was examined in this Example. The various scFv attachment configurations are provided in Figure 17. The ABH2 (AlbuCORE™) alone, the anti-Her2 scFv alone, a monovalent one-armed Fc molecule and a bivalent two-armed Fc molecule were utilized as controls. These are summarized in the "Controls" panel of Figure 17. The ABH2 protein was constructed as per Example 2 and the fusion proteins were created as per Example 11.

### FACS Binding Assessment

The binding affinity of the various anti-Her2 ABH2 constructs and controls to SKOV cells expressing Her2 was assessed via FACS. Detection was performed using an anti-HSA polyclonal (FITC labeled). All K_{D} determined are apparent.

### Whole Cell Binding by FACS Protocol:

Once cells have reached sufficient viability, (ie 2x10⁶ cells/ml cells (> 80% viability)) the media was aspirated from the T75 culture flask and washed with cold PBS, which was subsequently aspirated. 3mL dissociation buffer per flask was added and cells counted. The suspension was then centrifuged at 1050 rpm for 5 minutes and cells resuspended in RPMI media containing 5% FBS @ 1X10e5 cells/ 100µl. 100 ul of cell suspension was added to eppendorf tubes with the subsequent addition of 10µl/ tube of the primary antibody (goat anti human HSA). The cells were then incubated for 2 hours at 4°C on a rotating support. Tubes were then centrifuged for two minutes at 2000 rpm at room temperature. The media was aspirated, and the pellet washed with 500 ul RPMI media containing 5% FBS and re-centrifuged. The secondary antibody (Alexa Fluor 488-conjugated anti goat IgG) was added to final concentration of 2ug/sample. The pellet was resuspended in 100 ul solution, incubated for 1hour at 4C on rotating support and centrifuged 2 minutes at 2000 rpm. The cells were then washed with 500 ul RPMI media containing 5% FBS re-centrifuged and re-suspended in 500 ul per pellet for flow cytometric analysis.

The K_{D} values for each construct are provided below the representative rendering in Figure 17. All K_{D} determined are apparent and values represent an average of six concentrations tested per molecule, up to 3 µM. As the figure demonstrates, increasing the valency tends to increase binding affinity, indicated by the decrease in apparent K_{D} value. Generally, anti-Her2 scFv attached to the N termini of either fragment polypeptide exhibits greater affinity for Her2 than one attached to the C terminus. In some embodiments, this property of the multispecific multivalent species to reduce effective affinity on fusion at specific positions can be employed to attenuate the affinity and activity of some fusion partner species.

### Example 13. Bi-specific binding of ABH2 fused to anti-Her2 and anti-Her 3 scFvs.

The binding affinity of an ABH2 loaded in a cis configuration with anti-Her2 and anti-Her3 scFvs to MALME-3M cells were assessed and compared to the v1087 control. Both the fusion protein and control were constructed as per Example 11.

### FACS analysis of bi-specific binding

The binding affinity of an exemplary albumin-based heteromultimer variant 1090 (anti-Her2 x anti-Her3 ABH2) to MALME-3M and SKOV cells was evaluated using FACS with FITC-labeled anti-HSA antibodies, as described in Example 12. As described in Example 11, v1090 comprises B1D2/H3 fusions, Merrimack's linker length, and the less hydrophobic linker polyGLY)(S).

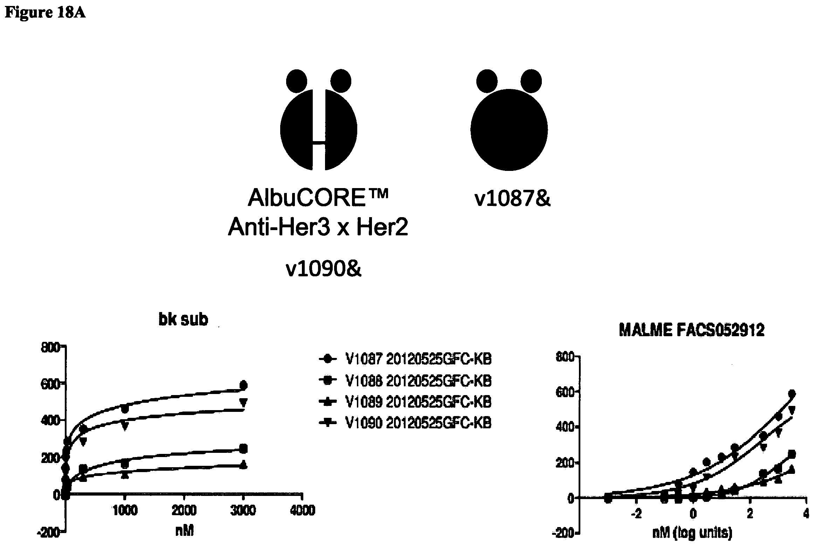

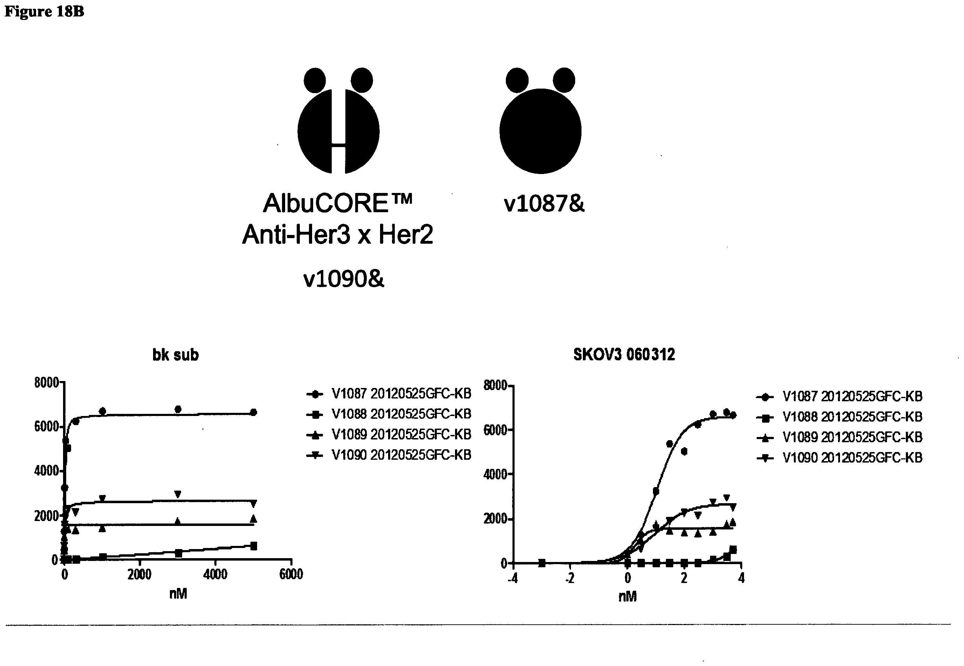

Figure 18A depicts the binding of variants 1088, 1089 and 1090 compared to the control 1087 in SKOV cells. Figure 18B depicts the binding of variants 1088, 1089 and 1090 compared to the control 1087 in MALME-3M cells. Table 16 below provides data regarding characterization of the binding of these variants in both cell types. All units in Table 16 are nM unless otherwise indicated. Binding to MALME-3M cells for 1087 and 1090 was 5-55 nM and 200-10,000 nM, respectively. Binding to SKOV cells for 1087 and 1090 was 10 nM and 9.4 nM, respectively.

**Table 16: Binding data for variants in SKOV and MALME-3M cells.**

| **Variant** | **warhead at natural N terminus (pos. 1)** | **warhead at natural C terminus (pos. 2)** | Kd (MALME 20120529) | Bmax (MALME 20120529) | **Kd (SKOV 20120603)** | **HillSlope (MALME 20120529)** | **Bmax (MALME 20120603)** |
|---|---|---|---|---|---|---|---|
| 1087 (MM-111) | HER3 | HER2 | 12 uM | 1469 | 10.69 | 0.97 | 6583 |
| 1088 (Unsplit HSA) | HER3 | -- | 0.8 uM | 74 | 5 uM | 1.0 | |
| 1089 (Unsplit HSA) | -- | HER2 | 60 uM | 1149 | 1.8 | 1.8 | 1572 |
| 1090 (AlbuCORE) | HER3 | HER2 | 0.2 uM | 323 | 9.4 | 0.7 | 2658 |

### Example 14. Serum stability of ABH2 fused to anti-Her2.

The stability of ABH2 loaded with an anti-Her2 in human sera was performed to assess potential proteolytic cleavage of the linker. Five variants were tested: v517, v518, v519, and v520

### Human sera incubation and subsequent FACS analysis.

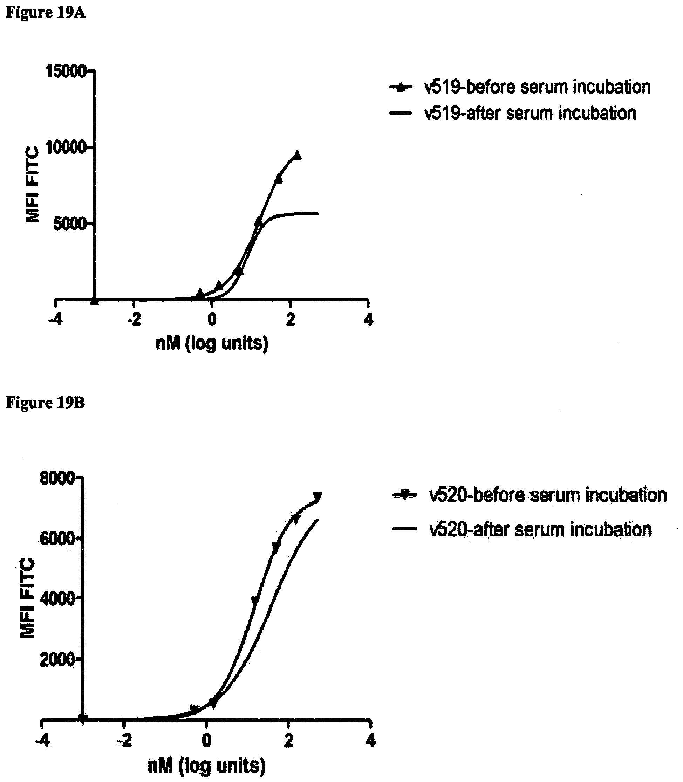

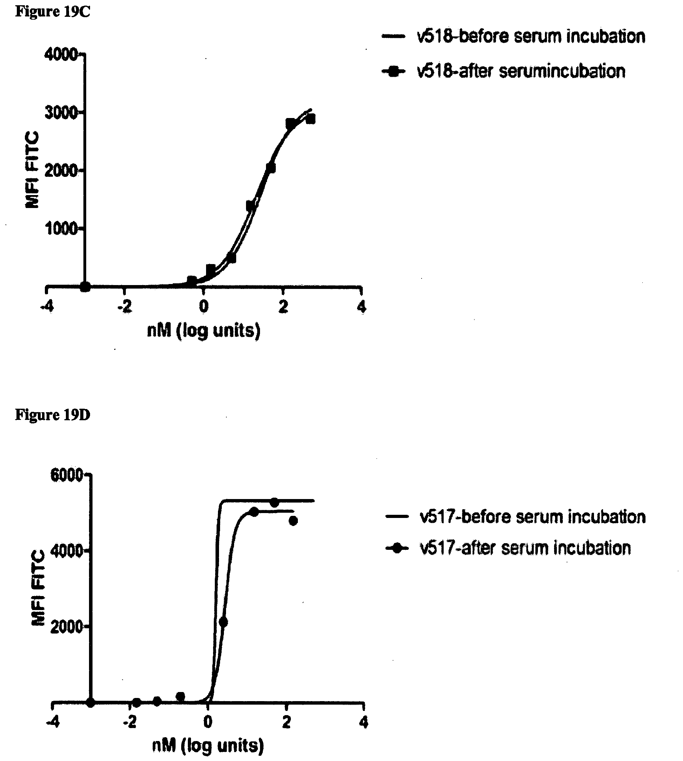

The anti-Her2 ABH2 fusion proteins described above was tested for binding to SKOV cells and then incubated in previously frozen human sera at 100 µg/ml (Sigma #H4522) for 24 hours at room temperature, before being re-tested for binding to the same stock of SKOV cells. Detection was performed using goat FITC labeled polyclonal anti-HSA antibody. The fusion protein's binding affinity to SKOV-3 cells was assessed using FACS, with pre-incubation proteins used as a control. Figures 19A to D depicts the binding curve of both the pre-incubation and post-incubation samples of the variants tested.

As shown in Figures 19A to D, all fusions appeared stable and capable of binding to the target antigen after serum incubation.

### Example 15. FcRn binding of ABH2 fused to anti-Her2 and anti-Her3 scFvs.

The affinity of ABH2 loaded in a trans configuration with anti-Her2 and anti-Her3 scFvs was assessed for binding to FcRn and compared to both wild-type HSA and the 1087 control.

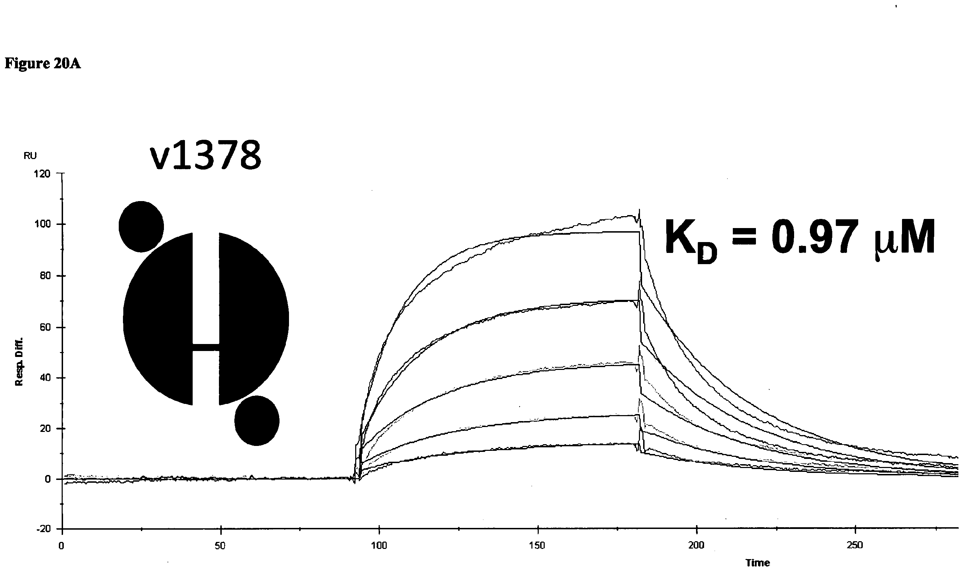

For this experiment the benchmark control v1087 and one of the AlbuCORE HER2/HER3 fusions (v1378) was used. Figure 20A illustrates the trans configuration of the v1378 fusion.

### FcRn Binding.

Human FcRn (hFcRn) was immobilized on the SPR surface and purified anti-Her3 x Her2 ABH2 (v1378) and v1087 were flowed over at pH 6.0. Methods are identical to Example 2, except the directionality of the assay was reversed.

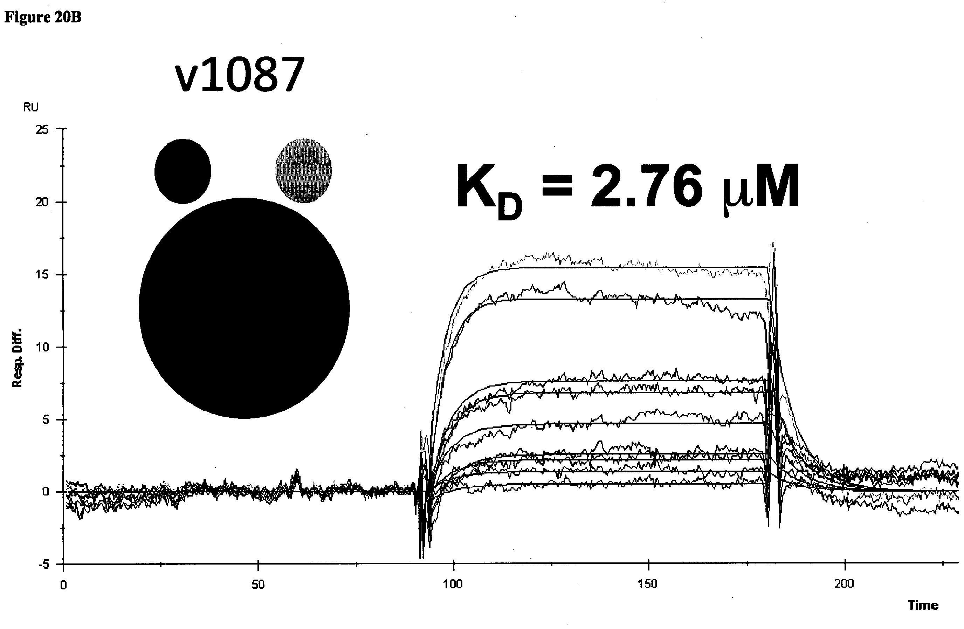

Figure 20A and 20B show the different binding curves of Anti-Her2 x Her3 ABH2 and V1087 respectively. The Anti-Her2 x Her3 ABH2 bound hFcRn with a KD of 0.97 µM, while the V1087 molecule bound hFcRn with a KD of 2.76 µM. From the figures, it can be seen that both the fusion ABH2 and v1087 are comparable in FcRn binding affinity to wild-type-HSA (Kd = 1.4µM at pH 5.5, as described in Chaudhury, C et al. Biochemistry 2006, 45, 4983-4990*).*

### Example 16. Bench-top stability of ABH2 and monovalent anti-Her2 ABH2.

The bench-top stability of both the ABH2 scaffold and a monovalent ABH2 anti-Her2 fusion protein (v517) was assessed over a five-day period at three different temperatures.

### Benchtop Stability

Samples from each molecule were divided into nine tubes, with each tube containing ∼10 µg of protein. An initial amount of material corresponding to 10 µg of protein was placed in PBS at 1mg/mL and incubated at 37°C (bath), room temperature (RT) or 4°C (fridge); protein stability was assessed over the course of five days. At each time point an aliquot was extracted and mixed 1:1 with Commassie Blue loading buffer with subsequent freezing. After the last time step all aliquots were thawed and loaded on a native gel, along with reducing and non-reducing SDS-PAGE.

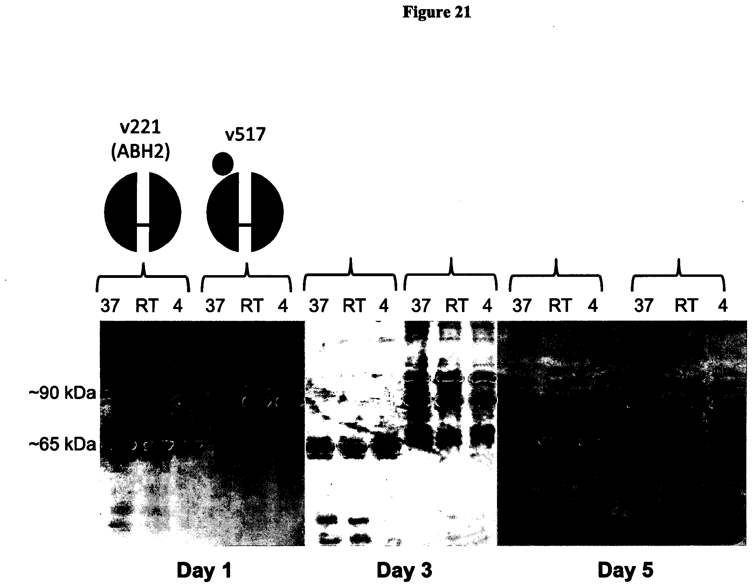

Figure 21 is a native gel of the ABH2 scaffold and anti-Her2 ABH2 fusion on days 1, 3 and 5. As the figure demonstrates, both the ABH2 scaffold and the anti-Her2 ABH2 remained stable over the 5 day period. The doublet band visible for the fusion protein likely represents two different reduction states of the disulfide. In presence of reducing agent the two bands in fact collapse into one.

### Example 17. Purification optimization using the AlbuPure® Process

As an alternate to the Blue Sepharose purification described in Example 2, which leads to some amount of residual contaminants in the elution and the need for high salt elution, a AlbuPure® based affinity purification method was employed. Optimization procedures consisted in testing binding to the Blue Sepharose matrix in both batch and direct mode, different elution protocols were tried, along with different pH and temperatures. Finally a specific resin (i.e. AlbuPure®) tailored towards the purification of albumin and albumin fusions was tested. Different binding and elution conditions were tested using AlbuPure®, involving binding in presence of different salts and elution using a pH gradient or competing detergents.

### Purification of Wild-type HSA: Comparison of Blue Sepharose to AlbuPure®

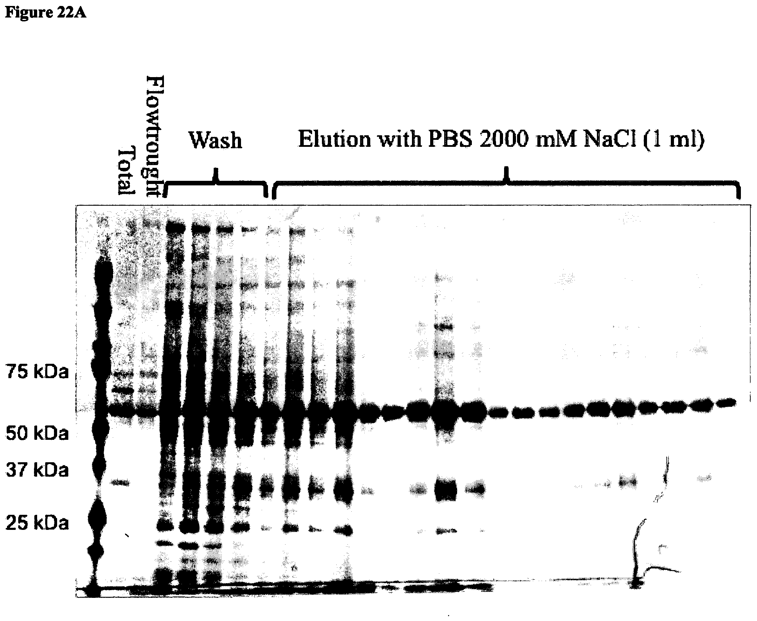

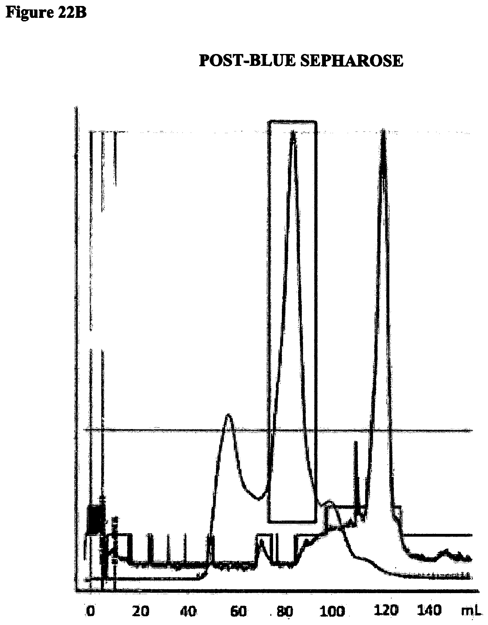

Blue Sepharose purification was performed on wild-type HSA as per methods described in Example 2. Figure 22A is a non-reducing SDS-PAGE gel profile of the total, flowthrough, wash and elution fractions obtained during the Blue Sepharose purification. Figure 22B represents the LC analysis of the Blue Sepharose elution fraction after gel filtration (SEC). As can be seen from the numerous peaks in the chromatogram, there was significant protein and ion contamination even after the purification.

AlbuPure® purification was performed on wild-type HSA as follows. The cellular supernatant containing wild-type HSA was solubilized in PBS. Prepacked AlbuPure® columns (column volume: 1 mL) were equilibrated in 50 mM sodium acetate at pH 5.3. After loading the supernatant the column was washed three times with solutions containing 50 mM sodium acetate but increasing pH (i.e. 1^{st} was pH 6; 2^{nd} wash pH 7). The final wash was performed in ammonium acetate, pH 8.0. The protein of interest was then eluted using a solution containing 50 mM ammonium acetate, pH 7.0 supplemented with 10 mM sodium octanoate. The AlbuPure® process only required one step to obtain a comparable amount of protein to that of Blue Sepharose. Protein yields from 500 mL initial CHO culture are shown in Table 17 below.

**Table 17: Protein yields after purification**

| Variant ID | Blue Sepharose purification (pre SEC) | Blue Sepharose purification (post SEC) | AlbuCORE (octanoate elution) | Initial cell culture | Comments |
|---|---|---|---|---|---|
| V221 (naked HSA) | 8.6 mg | 2.79 mg | 10.34 mg | 500 mL | |
| V519 | 3.3 mg | 1.8 mg | 8.16 mg | 500 mL | HER2 fusion. Warhead appears to be proteolytically cleaved |
| V520 | 4 mg | 0.93 mg | 4.3 mg | 500 mL | HER2 fusion. Warhead appears to be proteolytically cleaved |

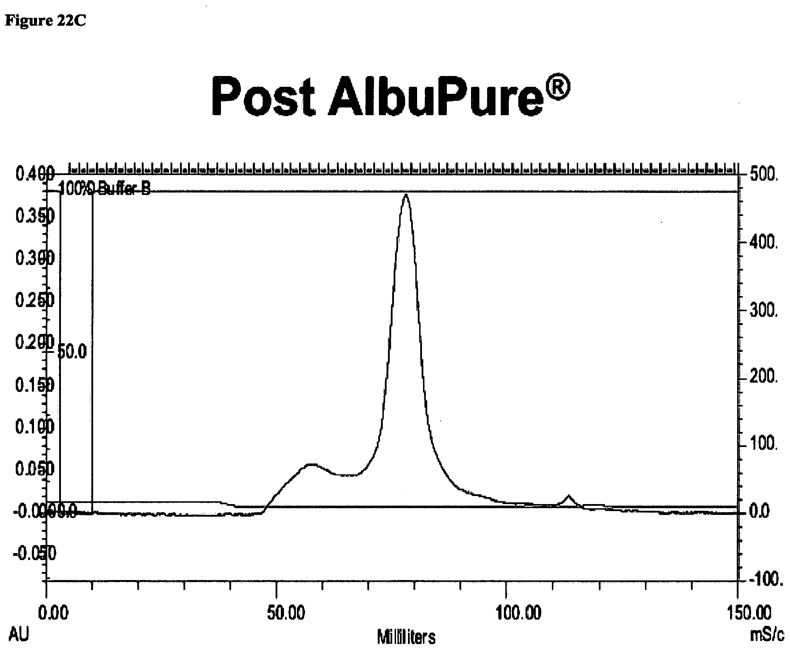

Figure 22C represents a LC analysis of the AlbuPure affinity column elution fraction after gel filtration (SEC) A comparison of Figures 22B and 22C demonstrate that the AlbuPure process results in a final solution with a much higher level of purity than that from Blue Sepharose along with lower amount of higher MW aggregating protein.

### Purification of engineered AlbuCOREs: Comparison of Wild-type HSA and Engineered AlbuCOREs using AlbuPure®

The two fragment peptides of AlbuCORE-1A, 3, 6 and 9 described in Table 11 were transiently co-expressed in CHO cells using separate vectors. The optimal DNA ratio for expression of the two fragments constituting the different AlbuCORE molecules were (fragment 1:fragment 2) AlbuCORE-1= 1:1; AlbuCORE-3=1:2; AlbuCORE-6=1:1; AlbuCORE-9=2:1. All expressions were transient and performed in identical fashion to the previous AlbuCORE molecules. Wild-type HSA was also transiently expressed in CHO cells. Expression yields of the fusion proteins were within 75% of the WT-HSA (approx. 50-70mg/L). Amount of material was determined by A280 after albupure purification.

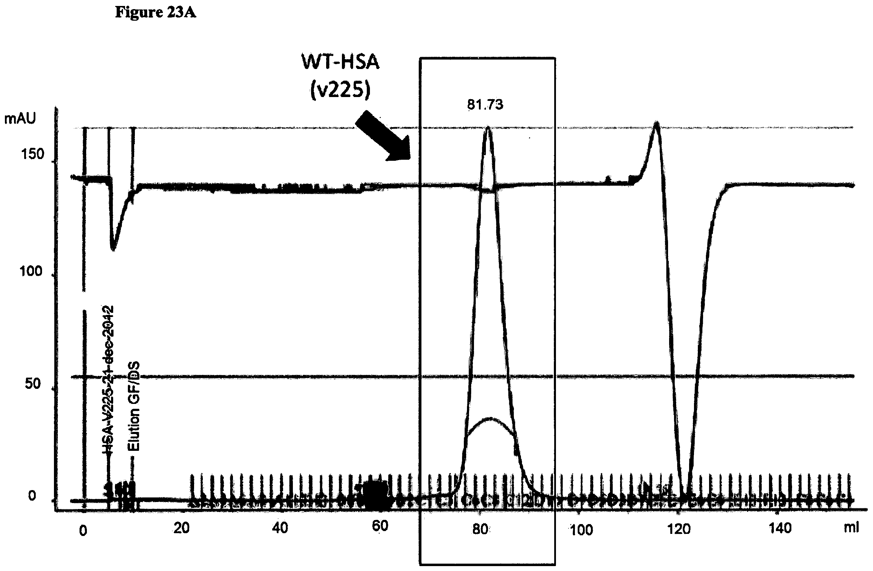

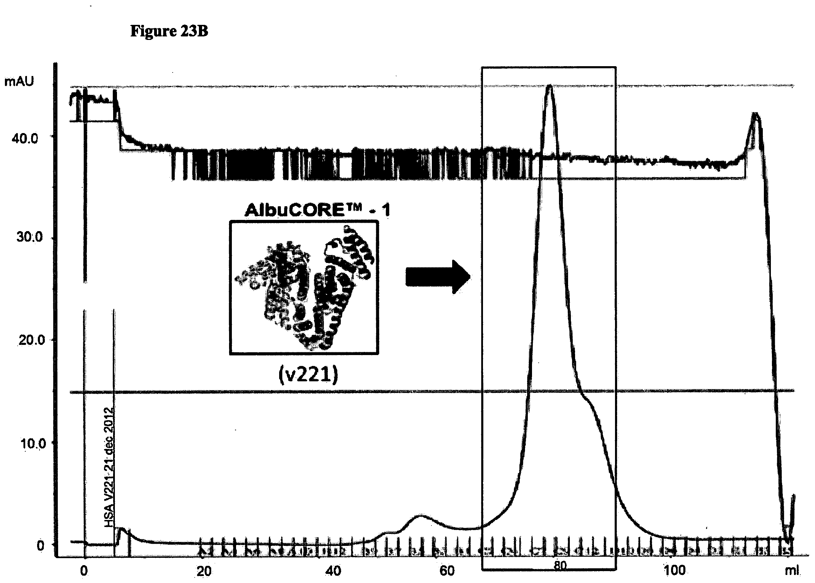

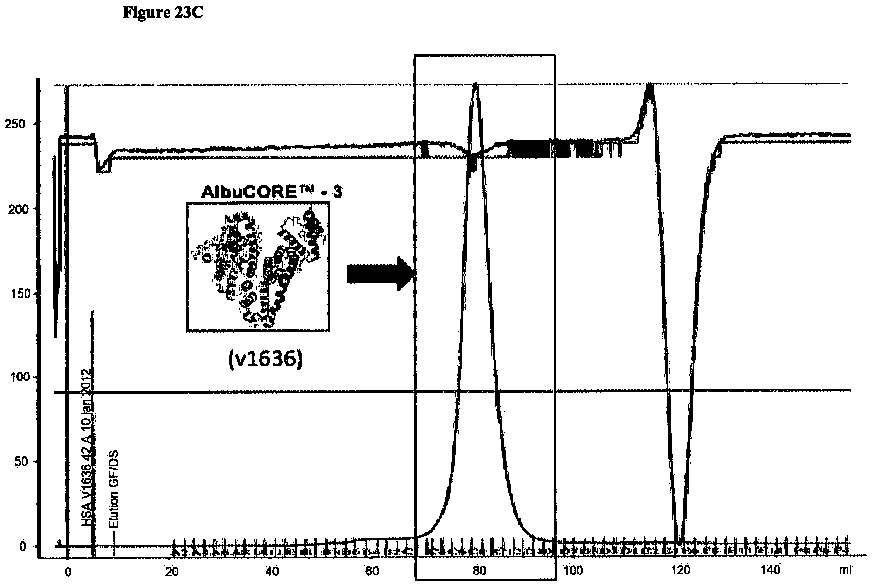

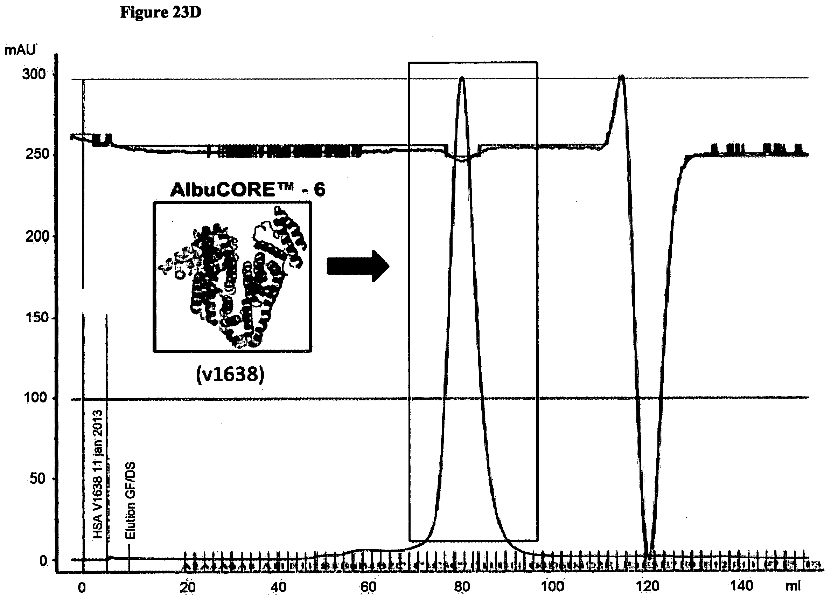

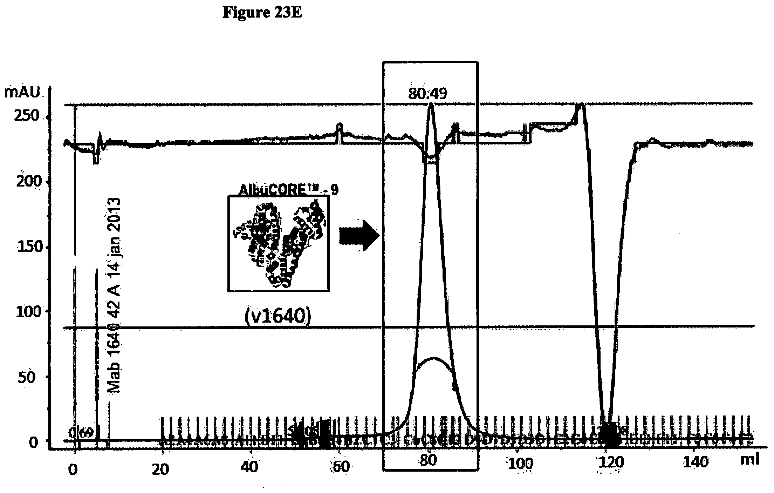

After co-expression, the AlbuCORE scaffolds were purified using the AlbuPure® protocol described above. To assess purity, the elute from the AlbuPure® affinity column was run through a gel filtration system (SEC) and the flowthrough analyzed using LC. The same was performed for wild-type HSA. Figure 23A represents the chromatogram of the wild-type HSA, while Figures 23B, 23C, 23D, and 23E are chromatograms of AlbuCORE 1, 3, 6 and 9 respectively. As these figures demonstrate, AlbuPure® purification yields are comparable results between wild-type and engineered AlbuCORE. The yields in terms of mg of purified protein from 500 mL initial CHO culture after AlbuPure® purification were determined to be: AlbuCORE-1=16 mg ; AlbuCORE-3=25 mg; AlbuCORE-6=27; AlbuCORE-9=25 mg.

### Scale-up Expression and Purification of AlbuCORE-1

AlbuCORE-1 was transiently co-expressed in CHO using the pTT5 vector at a ratio of 1:1 fragment A:B, see example 2. Purified yields of AlbuCORE-1 in CHO cells were approx. 10mg/L, which is comparable to the 9mg/L yield of vl087 in CHO cells, calculated from the same amount of starting material.

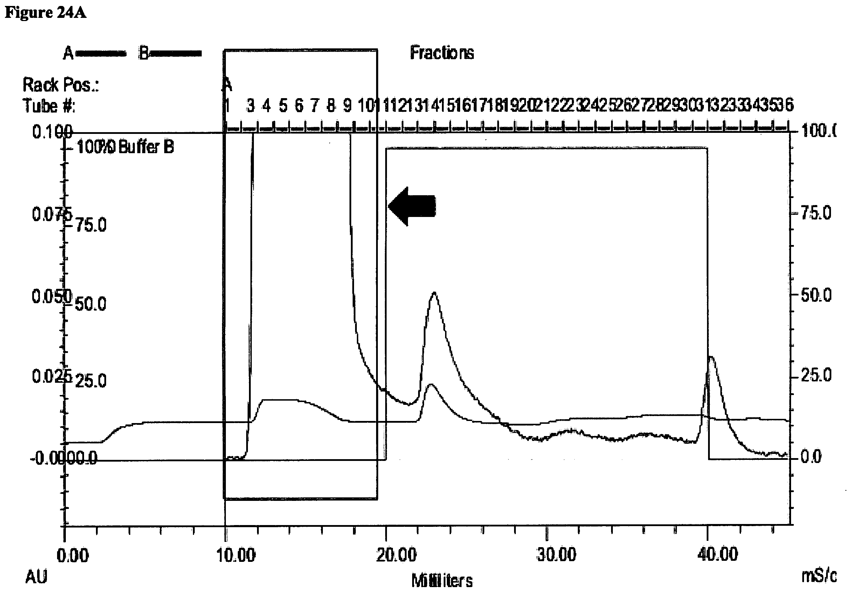

AlbuCORE-1 was purified using the AlbuPURE protocol described above. The affinity column elute was applied to a gel filtration-LC analysis system. Figure 24A represents the chromatogram of the AlbuPure purification. The main peak represents the protein of interest.

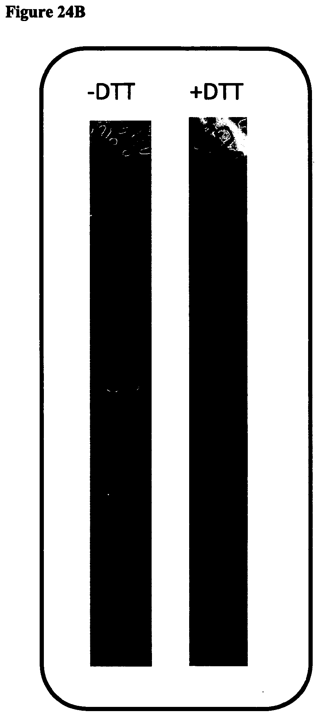

The purified samples were also analyzed via reducing and non-reducing SDS-PAGE gels, depicted in Figure 24B. As the figure demonstrates, in non-reducing conditions, the key elution product is the whole AlbuCORE-1 protein; in reducing conditions, the AlbuCORE-1 protein separates into the two fragments previous described. Significantly, there is little contamination, especially when compared with Figure 22A.

### SDS-PAGE and LC/MS Analysis of AlbuCORE-1 and WT HSA

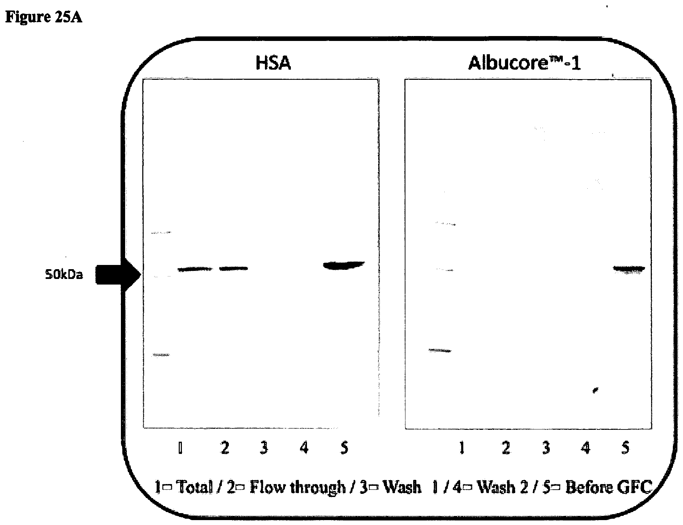

The total, flowthrough, wash and elute of the wild-type HSA and AlbuCORE-1 purifications were collected and analyzed via non-reducing SDS-PAGE. As shown in Figure 25A, representing the aforementioned gel profiles, demonstrate, there is little contamination after the AlbuPure process (compare Figure 25A and Figure 22A) and there is also no homodimer formation.

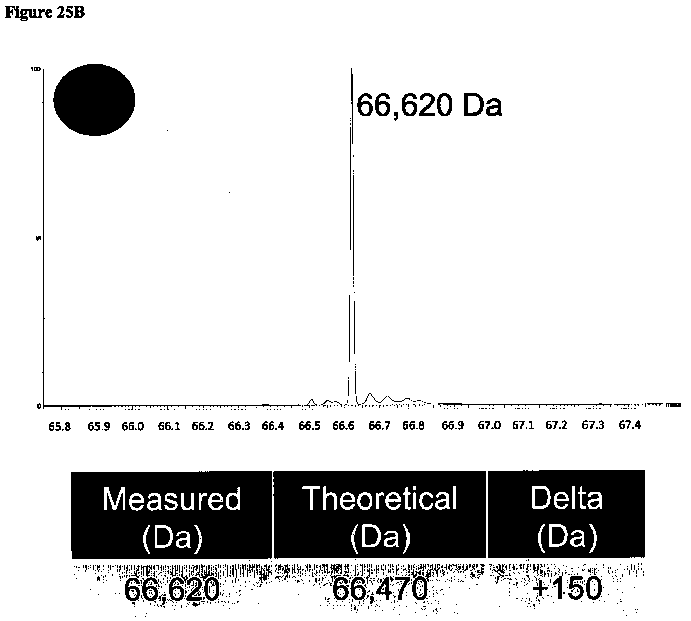

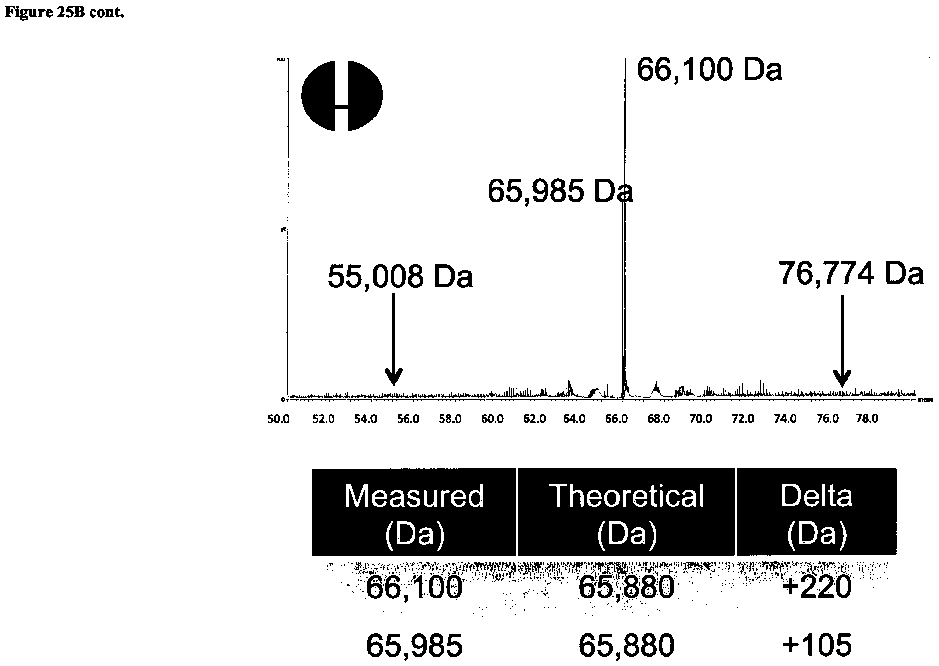

The purified protein was then analyzed using LC/MS. The protein samples were analyzed by LC-MS using an Agilent 1100 HPLC system coupled to an LTQ-Orbitrap XL hybrid mass spectrometer (ThermoFisher Scientific) via a high-flow electrospray interface. The samples (2.5 µg) were injected onto a 2.1 x 10 mm Poros R2 column (Applied Biosystems) and eluted using a 2 mL/min gradient of 20-90% ACN, 0.1% FA over 3 minutes. The flow was split post-column to direct 100 µL/min into the electrospray interface. The column and solvent were heated to 80°C to improve protein peak shape. The LTQ-Orbitrap XL was calibrated using ThermoFisher Scientific's LTQ Positive Ion ESI calibration solution (caffeine, MRFA and Ultramark 1621), and tuned using a 25ug/uL solution of BSA. The cone voltage (source fragmentation setting) was 40 V, the FT resolution was 7,500 and the scan range was m/z 400-4,000. The protein spectra were deconvoluted using ThermoFisher's Promass software (input range: m/z 2500-3050; output mass range: 100,000-160,000 Da; Peak width: 1.5; Merge width: 0.5; Baseline removal: 0.5 (low)). The abundances of the hetero- and homodimer antibody species were determined directly from the resulting molecular weight profiles. The linearity of response was confirmed using defined mixtures of antibodies. Limits of detection were approximately 2%. Figure 25B provides the spectrograms of purified AlbuCORE-1 and WT-HSA. As the spectrogram for AlbuCORE-1 demonstrates, there is no homodimer formation (such as A:A or B:B). Variation from the theoretical molecular weight may be due to ion binding, miscellaneous ligands, or glycation.

### Example 18. Spatial organization of termini of albumin-based polypeptides

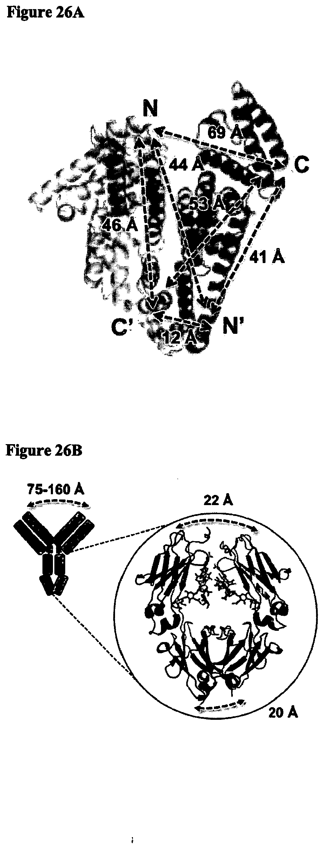

Albumin-based polypeptides provide an alternative scaffold to antibodies for delivery of cargo molecules. As the difference between Figures 26A and 26B demonstrate, engineered albumin-based polypeptides have a greater degree of positional freedom compared to regular antibodies, as the spatial separation between the four termini varies significantly more. Distances were determined after modeling the AlbuCORE molecule onto one of the available crystal structures: PDBID= 4EMX. Furthermore, the four termini are also oriented at different planar angles.

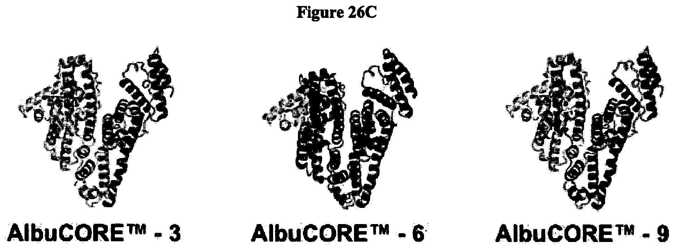

Finally, the distances and angles between the termini can be subject to further modulation by choosing different segmentations sites, binding domains and/or linkers. Figure 26C demonstrates alternative AlbuCORE scaffolds available to vary the inter-termini distances for antigen binding optimization in multivalent binding modes.

### Example 19. Stability of additional albumin-based polypeptides as measured via DSC

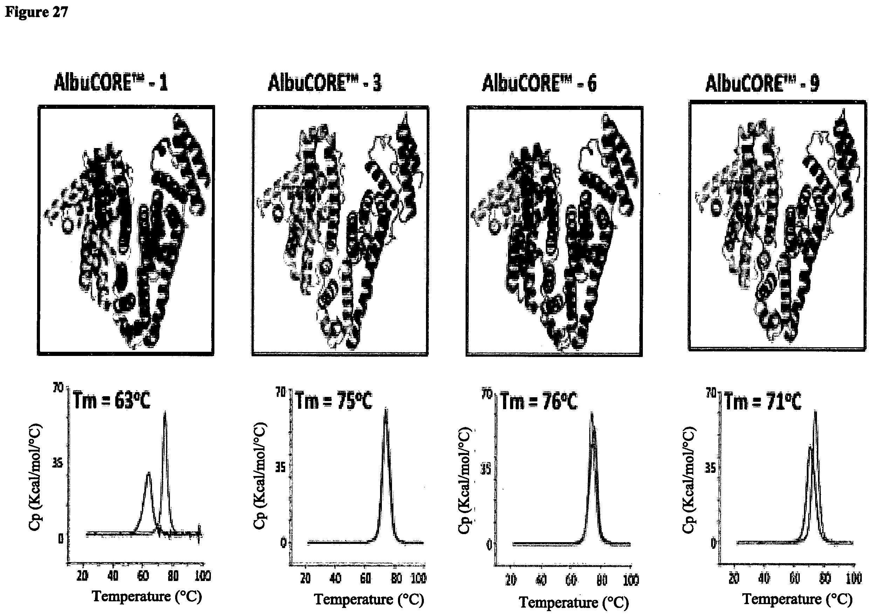

To determine the stability and melting points of additional AlbuCORE polypeptides, DSC experiments were performed on AlbuCORE-3, 6 and 9 as well as controls WT-Albumin and AlbuCORE-1. DSC experiments were performed as set out in Example 2. Figure 27 shows the melting curve of four scaffolds, as compared to that of the WT-HSA. As the Figure shows, scaffolds 3, 6 and 9 possess melting points very similar to the 75°C demonstrated by the wild-type. Furthermore, AlbuCORE-6 was found to be more thermally stable than wild-type HSA, at 76°C. Additional DSC experiments were performed on AlbuCORE 1A and 4. The Tm of AlbuCORE 1A was 66°C and the Tm of AlbuCORE-4 was 70°C. AlbuCORE-1A narrows the gap between the two fragments in AlbuCORE 1 (ABH1) to one residue only and adds a GGGGS linker to both the N and C non natural termini. The same applies to AlbuCORE 2 (ABH2) and AlbuCORE-2A.

### Example 20: Preparation of Anti-CD3 x CD19 heteromultimer constructs.

Anti-CD3 and anti-CD 19 scFvs were attached to several different antibody platforms as follows. The albumin-based polypeptide platform was AlbuCORE-1; controls included WT-HSA, the Azymetric scaffold (v873) and the anti-CD3 x CD19 BiTE control v891.

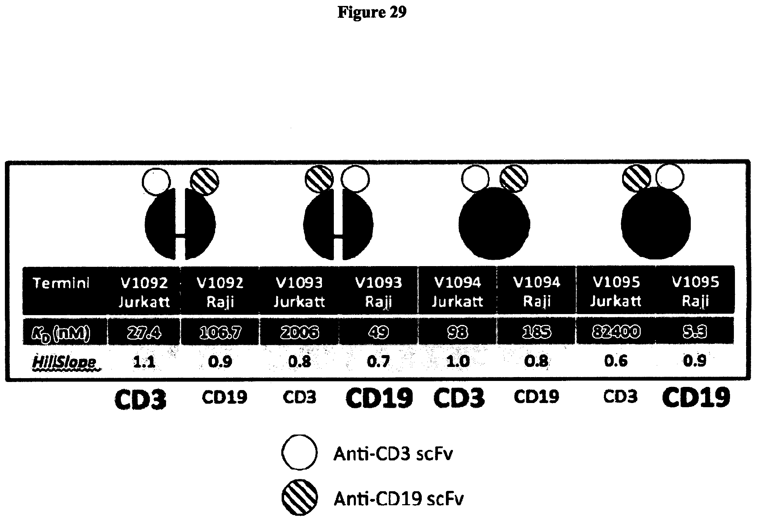

ABH2 loaded with anti-CD19 x anti-CD3 scFvs (1092, 1093, 1094, and 1095): The sequences for the anti-CD19 and anti-CD3 scFvs were chosen from two molecules that are currently in clinical trials and are well documented and tested for stability and production. The anti-CD 19 and anti-CD3 scFv were directly adopted from the BiTE molecule blinatumomab. The antiCD3 scFv was chosen in the VH-VL orientation, consistent with what used in BiTE. The benchmark molecule was BiTE. AlbuCORE_1 (ABH2) CD3/CD19 fusions were created by attaching the antiCD3 warhead to the natural N terminus of fragment 1 and the antiCD19 to the C terminus of fragment 2 (v1092). A second molecule was created where the warheads were reversed (i.e. anti-CD 19 warhead at the natural N terminus of fragment 1 and the anti-CD3 at the C terminus of fragment 2, vl093). The linkers used were identical to the ones used for the multivalent HER2 AlbuCORE experiments: GGGS at the N terminus of fragment 1 and (GGSG)₄GG at the C terminus of fragment 2. Expression and purification were performed as previously described for the multivalent HER2. Molecules 1093 and 1094 were designed to accommodate two different fusions at their natural termini. The scFv fusions were linked to the albumin molecule through a GGS linker at the N terminus and a GGSG linker at the C terminus. The length of the linkers reflect the ones used in the MM-111 molecule, despite having a different sequence type. Schematic representations of these molecules are shown in Figure 29.

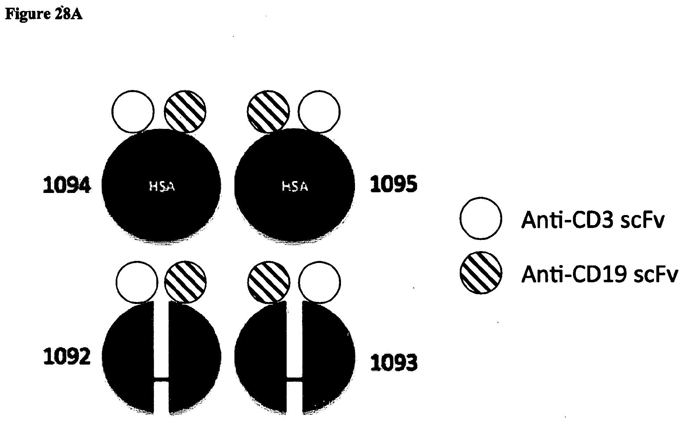

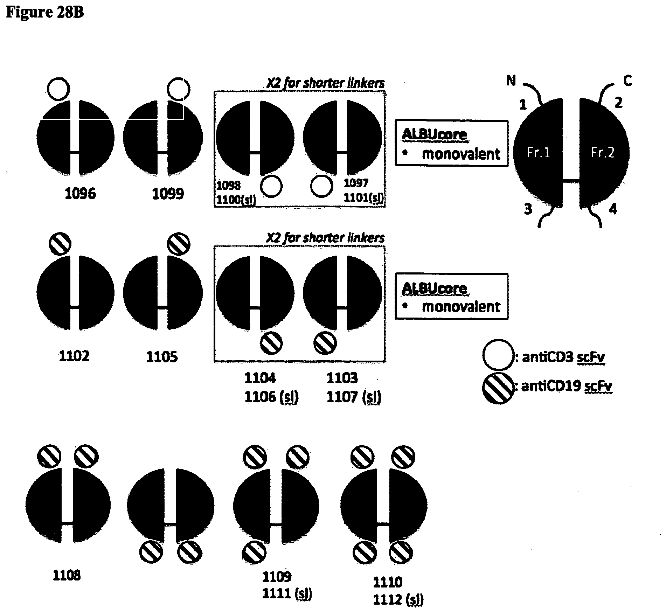

Additional monovalent and multivalent CD19 heteromultimers and monovalent CD3 heteromultimers based on ABH2 in which the relative positions of the cargo polypeptides were changed were designed with schematic representations of some of these heteromultimers shown in Figure 28B. These heteromultimers were generated by expressing its single monomeric transporter polypeptides, SEQ ID NO: 8 and SEQ ID NO: 10, fused at one or both termini to cargo polypeptides that are either antiCD19scFv (CD19) and/or anti-CD3 scFv (CD3). Different combinations of these base constructs were combined upon co-expression to form heteromultimers displaying all combination of the two cargo polypeptides at any of the four terminal positions of the two transporter polypeptides, ranging from monovalent to tetravalent. Table 18 illustrates the base constructs (that were generated by fusing the CD3 and/or CD19 cargo polypeptides to either N or C terminus of transporter polypeptide 1 (F1) or transporter polypeptide 2 (F2). F1: corresponds to SEQ ID 8 and F2 corresponds to SEQ ID 10.

**Table 18: Base constructs for CD3 and CD19 monovalent and multivalent constructs**

| | **Single Fusions** | | | |
|---|---|---|---|---|
| # | Fusion 1 | Fusion 2 | Fusion 3 | Polypeptide SEQ ID NOs: |
| 1 | CD3 | F1 | | 137/138 |
| 2 | F1 | CD3 | | 119/120 |
| 3 | F1 | CD3 (short linker) | | 181/182 |
| 4 | CD3 | F2 | | 179/180 |
| 5 | CD3 (short linker) | F2 | | 177/178 |
| 6 | F2 | CD3 | | 141/142 |
| 7 | CD19 | F1 | | 143/144 |
| 8 | F1 | CD19 | | 183/184 |
| 9 | F1 | CD19 (short linker) | | 189/190 |
| 10 | CD19 | F2 | | 187/188 |
| 11 | CD19 (short linker) | F2 | | 185/186 |
| 12 | F2 | CD19 | | 139/140 |

| | **Double Fusions** | | | |
|---|---|---|---|---|
| 13 | CD19 | F1 | CD19 | 191/192 |
| 14 | CD19 | F1 | CD19 (shorter linker) | 193/194 |
| 15 | CD19 | F2 | CD19 | 195/196 |
| 16 | CD19 (shorter linker) | F2 | CD19 | 197/198 |
| 17 | CD3 | HSA | CD19 | 133/134 |
| 18 | CD19 | HSA | CD3 | 135/136 |

As shown in Figure 28B, the bioactive cargo polypeptides were fused to the heteromultimer transporter polypeptides via a GGSG linker, for the N terminus of one monomer and a longer (GGS)₄GG linker for all other termini in the other monomer. In addition a version of each fusion protein was created with a shorter linker at their non-natural terminus (ie C' of Fragment 1 and N' of Fragment 2) formed by the sequence GGSGGSG, replacing (GGS)₄GG.

A summary of the heteromultimers that were prepared are shown in Table 19.

**Table 19: Monovalent, multivalent, and multispecific constructs that were generated by fusing the CD3 and CD 19 cargo polypeptides to either N or C terminus of transporter polypeptide 1 or transporter polypeptide 2 of ABH2.**

| **Variant** | **N terminus-transporter polypeptide 1 (SEQ ID No: 8)** | **C terminus-transporter polypeptide 1 (SEQ ID No: 8)** | **N terminus-transporter polypeptide 2 (SEQ ID No: 10)** | **C terminus-transporter polypeptide** 2 **(SEQ ID No: 10)** | **Valency** | **Sequences (Polypeptide)** |
|---|---|---|---|---|---|---|
| 1096 | CD3 | | | | monovalent | SEQ ID No: 138 |
| | | | | | | SEQ ID NO: 10 |
| 1097 | | CD3 | | | monovalent | SEQ ID No: 120 |
| | | | | | | SEQ ID NO: 10 |
| **1101 (shorter linker)** | | **CD3** | | | **monovalent** | SEQ ID No: 182 |
| | | | | | | SEQ ID NO: 10 |
| 1098 | | | CD3 | | monovalent | SEQ ID NO: 8 |
| | | | | | | SEQ ID No: 180 |
| 1100 (shorter linker) | | | CD3 | | monovalent | SEQ ID NO: 8 |
| | | | | | | SEQ ID No: 178 |
| 1099 | | | | CD3 | monovalent | SEQ ID NO: 8 |
| | | | | | | SEQ ID No: 142 |
| 1102 | CD19 | | | | monovalent | SEQ ID No: 144 |
| | | | | | | SEQ ID NO: 10 |
| 1103 | | CD19 | | | monovalent | SEQ ID No: 184 |
| | | | | | | SEQ ID NO: 10 |
| 1107 | | CD19 | | | monovalent | SEQ ID No: 190 |
| | | | | | | SEQ ID NO: 10 |
| 1104 | | | CD19 | | monovalent | SEQ ID NO: 8 |
| | | | | | | SEQ ID No: 188 |
| 1106 | | | CD19 | | monovalent | SEQ ID NO: 8 |
| | | | | | | SEQ ID No: 186 |
| 1105 | | | | CD19 | monovalent | SEQ ID NO: 8 |
| | | | | | | SEQ ID No: 140 |
| 1108 | CD19 | | | CD19 | bivalent | SEQ ID No: 144 |
| | | | | | | SEQ ID No: 140 |
| 1109 | CD19 | CD19 | | CD19 | trivalent | SEQ ID No: 192 |
| | | | | | | SEQ ID No: 140 |
| 1110 | CD19 | CD19 | CD19 | CD19 | tetravalent | SEQ ID No: 192 |
| | | | | | | SEQ ID No: 196 |
| 1111 (shorter linker at non-natural terminus) | CD19 | CD19 | | CD19 | trivalent | SEQ ID No: 194 |
| | | | | | | SEQ ID No: 140 |
| **1112** (shorter linker at non-natural termini) | CD19 | CD19 | CD19 | CD19 | tetravalent | SEQ ID No: 194 |
| | | | | | | SEQ ID No: 198 |
| **1092** | CD3 | | | CD19 | bispecific | SEQ ID No: 138 |
| | | | | | | SEQ ID No: 140 |
| **1093** | CD19 | | | CD3 | bispecific | SEQ ID No: 144 |
| | | | | | | SEQ ID No: 142 |

Multivalent constructs were generated as outlined in Example 2 using heteromultimer ABH2. The final gene products were subcloned into the mammalian expression vector pTT5 (NRC-BRI, Canada) (Durocher et al). High level and high-throughput recombinant protein production by transient transfection of suspension-growing human CHO-3E7 was performed as described in Example 2.

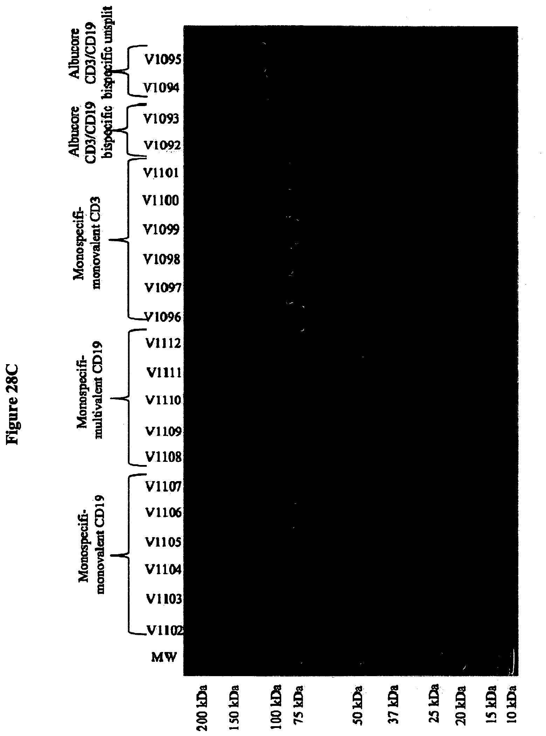

The expression results are shown in Figure 28C. The monovalent CD3 and CD19 fusions express at similar levels. The expression of monovalent CD3 and CD19 have the same pattern (in terms of MW) as the one observed with HER2 fusions, indicating that variations in migration on nonreducing gel are independent of the warhead type.

### Example 21: Ability of anti-CD3 x CD19 loaded albumin-based polypeptide to bind to B-cells and T-cells.

The ability of Anti-CD3 x CD19 loaded AlbuCORE-1 (v1092 and 1093) to CD3⁺ and CD19⁺ cells was assessed using FACS and compared to WT-HSA loaded with the same anti-CD scFvs (1094 and 1095). The AlbuCORE-1 constructs were additionally tested against the Azymetric™ and the BiTE MT-103 for B-cell and T-cell binding as follows. The method was carried out as described in Example 13, but in MALME-3M cells and SKOV cells.

The results are shown in Figure 29 which shows the K_{D} and Hill Slope data for the tested variants in Jurkat and Raji cells.

### Example 22: Ability of anti-CD13 x CD19 loaded albumin-based polypeptide to bind to B-cells and T-cells.

The ability of variants 1092, and 1093 to bridge B- and T-cells was determined as follows.

### Whole Cell Bridging by FACS

1 x 10⁶ cells/ml suspended in RPMI were labeled with 0.3 µM of the appropriate □ CellTrace label and mixed and incubated at 37°C in a water bath for 25 minutes. Pellets were resuspended in 2 ml of L10 + GS1 + NaN₃ to a final concentration 5x x10⁶ cells/ml. Cell suspensions were analyzed (1/5 dilution) by flow cytometry to verify the appropriate cell labeling and laser settings. Flow-check and flow-set Fluorospheres were used to verify instrument standardization, optical alignment and fluidics.

After flow cytometry verification, and prior to bridging, each cell line was mixed together at the desired ratio, at a final concentration of 1x10⁶ cells/ml.

T:T bridging was assessed with Jurkat-violet + Jurkat-FarRed, □B:B was assessed with RAJI-violet + RAJI-FarRed □ and T:B bridging was assessed with Jurkat-violet + RAJI-FarRed.

Antibody variants were diluted to 2x in L10+GS1+NaN3 at room temperature then added to cells followed by gentle mixing and a 30 min incubation.

Following the 30 min incubation 2 µl of propidium iodide was added and slowly mixed and immediately analyzed by flow cytometry.

Bridging % was calculated as the percentage of events that are simultaneously labeled violet and Far-red.□

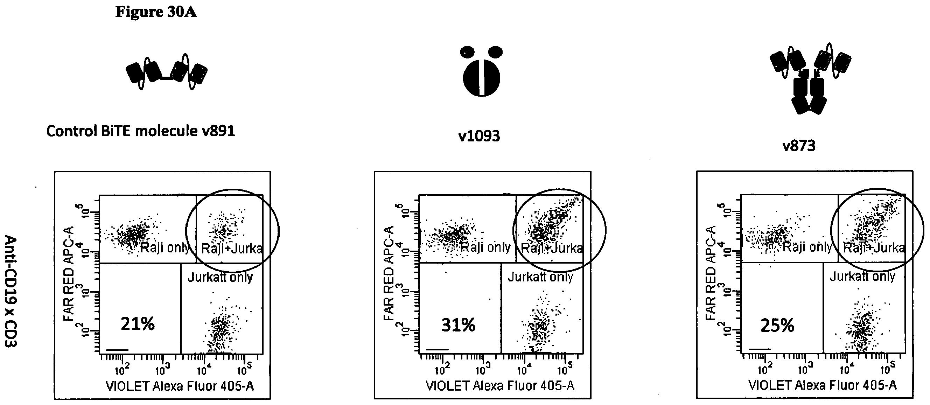

Figure 30A compares the ability of v1093, a bi-specific CD3/CD19 antibody (v873) and the BiTE-like control v891 to bridge Jurkat CD3 T cells (bottom right quadrant) with Raji CD19 B cells (top left quadrant) by FACS. Bridged T-B cells appear in top right quadrant. This result demonstrates that at concentrations of as low as 0.3 nM v1093 is able to specifically bridge Jurkat T cells and Raji B cells to a similar extent (31% of total cells) as BiTE (21% of total cells) and a bi-specific CD3/CD19 antibody with an heterodimeric Fc (25%).

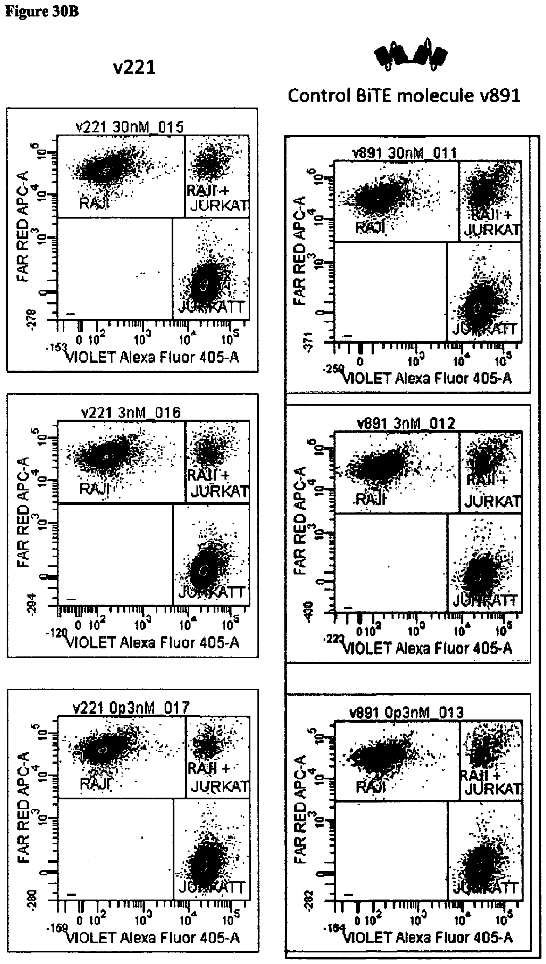

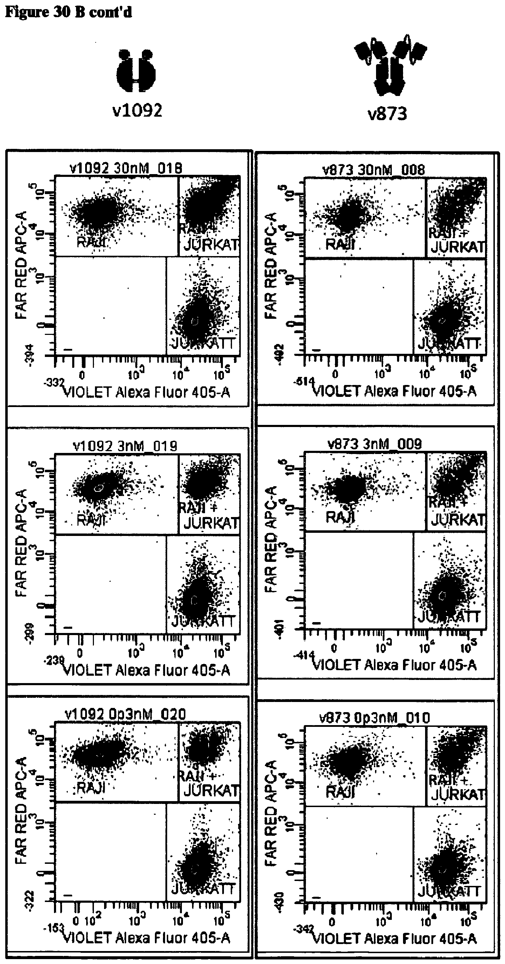

Figure 30B compares the ability of v1092, to the controls v891, v873, and v221. This figure shows that v1092 is able to bridge Jurkat T cells and Raji B cells to a similar extent to v891 (BITE).

### SEQUENCES

1. SEQ ID NO: 91 (v438: NM32 scFv NT) 
TCAAGCGAGCTGACCCAGGACCCCGCCGTGAGCGTCGCACTGGGGCAGACCGTGCGCA
TCACATGCCAGGGAGATAGCCTGCGATCCTACTATGCATCTTGGTACCAGCAGAAGCCA
GGACAGGCACCTGTGCTGGTCATCTATGGAAAAACAATAGACCATCAGGCATCCCCG
ACAGGTTCAGCGGAAGCTCCTCTGGCAACACAGCTTCTCTGACCATTACAGGCGCACAG
GCCGAGGACGAAGCAGATTACTATTGCAACAGTCGGGATAGTTCAGGGAATCACGTGG
TCTTTGGAGGAGGAACTAAGCTGACCGTGGGAGGAGGATCAGGAGGAGGAAGCGGAG
GAGGCAGCGGAGGAGGATCTGGAGGAGGAAGTGGAGGTGCAGCTGGTCGAAAGCG
GAGGAGGAGTGGTCCGACCTGGAGGGTCACTGCGACTGAGCTGTGCAGCTTCCGGCTT
CACATTTGACGATTACGGGATGTCATGGGTGAGACAGGCCCCAGGGAAAGGACTGGAA
TGGGTCTCCGGCATCAACTGGAATGGAGGCTCTACTGGATACGCCGACAGTGTGAAGG
GCAGGTTCACCATTTCCCGCGATAACGCTAAAAATTCTCTGTATCTGCAGATGAACAGT
CTGAGGGCCGAGGACACTGCCGTGTACTATTGTGCCCGGGGCAGATCCCTGCTGTTTGA
TTACTGGGGCCAGGGGACACTGGTGACTGTCTCTCGCGGCAGTGAAAATCTGTATTTTC
AG
2. SEQ ID NO: 92 (v438: NM32 scFv AA) 
SSELTQDPAVSVALGQTVRITCQGDSLRSYYASWYQQKPGQAPVLVIYGKNNRPSGIPDRFS
GSSSGNTASLTITGAQAEDEADYYCNSRDSSGNHVVFGGGTKLTVGGGSGGGSGGGSGGG
SGGGSGEVQLVESGGGVVRPGGSLRLSCAASGFTFDDYGMSWVRQAPGKGLEWVSGINW
NGGSTGYADSVKGRFTISRDNAKNSLYLQMNSLRAEDTAVYYCARGRSLLFDYWGQGTL
VTVSRGSENLYFQ
3. SEQ ID NO: 93 (V218: 4D5 scFv NT) 
GACATCCAGATGACCCAGTCTCCATCCTCCCTGTCTGCATCTGTAGGAGACAGAGTCAC
CATCACTTGCCGGGCAAGTCAGGACGTTAACACCGCTGTAGCTTGGTATCAGCAGAAA
CCAGGGAAAGCCCCTAAGCTCCTGATCTATTCTGCATCCTTTTTGTACAGTGGGGTCCC
ATCAAGGTTCAGTGGCAGTCGATCTGGGACAGATTTCACTCTCACCATCAGCAGTCTGC
AACCTGAAGATTTTGCAACTTACTACTGTCAACAGCATTACACTACCCCACCCACTTTC
GGCCAAGGGACCAAAGTGGAGATCAAAGGTGGTTCTGGTGGTGGTTCTGGTGGTGGTT
CTGGTGGTGGTTCTGGTGGTGGTTCTGGTGAAGTGCAGCTGGTGGAGTCTGGGGGAGGC
TTGGTACAGCCTGGCGGGTCCCTGAGACTCTCCTGTGCAGCCTCTGGATTCAACATTAA
AGATACTTATATCCACTGGGTCCGGCAAGCTCCAGGGAAGGGCCTGGAGTGGGTCGCA
CGTATTTATCCCACAAATGGTTACACACGGTATGCGGACTCTGTGAAGGGCCGATTCAC
CATCTCCGCAGACACTTCCAAGAACACCGCGTATCTGCAAATGAACAGTCTGAGAGCT
GAGGACACGGCCGTTTATTACTGTTCAAGATGGGGCGGAGACGGTTTCTACGCTATGGA
CTACTGGGGCCAAGGGACCCTGGTCACCGTCTCCTCAGGCAGCGAGAACCTGTATTTTC
AG
4. SEQ ID NO: 94 (V218: 4D5 scFv AA) 
DIQMTQSPSSLSASVGDRVTITCRASQDVNTAVAWYQQKPGKAPKLLIYSASFLYSGVPSRF
SGSRSGTDFTLTISSLQPEDFATYYCQQHYTTPPTFGQGTKVEIKGGSGGGSGGGSGGGSGG
GSGEVQLVESGGGLVQPGGSLRLSCAASGFNIKDTYIHWVRQAPGKGLEWVARIYPTNGY
TRYADSVKGRFTISADTSKNTAYLQMNSLRAEDTAVYYCSRWGGDGFYAMDYWGQGTL
VTVSSGSENLYFQ
5. SEQ ID NO: 95 (Base construct # 1) 
AGTAGCGAACTGACACAGGACCCCGCAGTGAGCGTCGCACTGGGACAGACAGTGCGAATC
ACTTGCCAGGGGGACTCACTGCGGAGCTACTATGCCTCCTGGTACCAGCAGAAACCAGGC
CAGGCTCCCGTGCTGGTCATCTATGGCAAGAACAATAGGCCTAGTGGGATTCCAGATCGCT
TTTCAGGGAGCTCCTCTGGAAACACTGCAAGTCTGACCATTACAGGCGCTCAGGCAGAGG
ACGAAGCCGATTACTATTGCAACAGCAGGGACAGTTCAGGGAATCACGTGGTCTTCGGAG
GAGGAACTAAGCTGACCGTGGGAGGAGGCAGCGGAGGAGGATCTGGAGGAGGAAGTGGA
GGAGGATCAGGAGGAGGAAGCGGAGAGGTGCAGCTGGTCGAAAGCGGAGGAGGAGTGGT
CCGGCCAGGAGGGTCCCTGAGACTGTCTTGTGCCGCTAGTGGATTCACTTTTGACGATTAC
GGAATGTCATGGGTCCGGCAGGCACCTGGCAAGGGACTGGAGTGGGTGAGCGGCATCAAC
TGGAATGGAGGCTCCACAGGGTACGCTGATTCTGTGAAAGGACGCTTTACTATTAGCCGAG
ACAACGCCAAGAACAGCCTGTATCTGCAGATGAACTCTCTGAGAGCTGAGGATACCGCAG
TGTACTATTGCGCCAGGGGCCGCTCTCTGCTGTTCGACTACTGGGGACAGGGCACACTGGT
GACTGTCTCACGCGGGGAAGCGGGGATGCTCACAAGTCCGAGGTCGCACATCGATTCAA
AGACCTGGGAGAGGAAAATTTTAAGGCCCTGGTGCTGATCGCCTTCGCTCAGTATCTGCAG
CAGTGCCCTTTTGAAGACCACGTGAAACTGGTCAACGAGGTGACCGAGTTCGCCAAGACA
TGCGTGGCCGACGAGAGTGCTGAAAATTGTGATAAATCACTGCATACCCTGTTTGGAGATA
AGCTGTGTACCGTGGCCACACTGCGGGAGACATACGGCGAAATGGCAGACTGCTGTGCCA
AACAGGAGCCTGAAAGAAACGAGTGCTTCCTGCAGCACAAGGACGATAACCCCAATCTGC
CTCGACTGGTGCGGCCAGAAGTGGACGTCATGTGTACTGCTTTCCACGATAATGAGGAAAC
CTTTCTGAAGAAATACCTGTATGAGATTGCCCGGAGACATCCATACTTTTATGCCCCCGAA
CTGCTGTTCTTTGCTAAGCGCTATAAAGCAGCCTTCACCGAGTGCTGTCAGGCTGCAGATA
AGGCCGCTTGCCTGCTGCCAAAACTGGACGAGCTGAGAGATGAAGGCAAAGCAAGCTCCG
CCAAGCAGAGGCTGAAATGTGCAAGCCTGCAGAAGTTCGGCGAGAGGGCCTTTAAAGCAT
GGGCCGTGGCTAGACTGTCTCAGAGGTTCCCCAAGGCTGAGTTTGCAGAAGTCAGTAAGCT
GGTGACTGACCTGACCAAAGTGCACACAGAGTGCTGTCATGGCGACCTGCTGGAATGCGC
CGACGATCGCGCCGATCTGGCTAAGTACATCTGTGAGAACCAGGACTCCATTTCTAGTAAG
CTGAAAGAGTGCTGTGAAAAGCCACTGCTGGAGAAATCTCATTGCATCGCTGAGGTGGAA
AATGACGAAATGCCCGCAGATCTGCCTAGCCTGGCAGCCGACTTCGTCGAGTCCAAGGAT
GTGTGTAAAAACTATGCCGAGGCTAAAGATGTGTTTCTGGGAATGTTTCTGTATGAGTATG
CAAGAGCATGAGGATCC
6. SEQ ID NO: 96 (Base construct # 1 Protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARA
7. SEQ ID NO: 97 (Base construct # 2 NT) 
GATGCTCATAAATCTGAGGTCGCTCACCGGTTCAAGGATCTGGGCGAGGAAAACTTTAAAG
CACTGGTGCTGATCGCTTTCGCACAGTACCTGCAGCAGTGCCCCTTTGAGGACCACGTGAA
GCTGGTCAACGAGGTGACAGAGTTCGCCAAAACTTGCGTCGCCGACGAGTCTGCTGAAAA
TTGTGATAAGAGTCTGCATACACTGTTTGGAGATAAACTGTGTACTGTGGCCACCCTGAGA
GAGACTTATGGCGAAATGGCAGACTGCTGTGCCAAGCAGGAGCCTGAAAGGAACGAGTGC
TTCCTGCAGCATAAAGACGATAACCCCAATCTGCCTAGGCTGGTGCGCCCAGAAGTGGAC
GTCATGTGTACCGCCTTCCACGATAATGAGGAAACATTTCTGAAGAAATACCTGTATGAGA
TTGCCCGGAGACATCCATACTTTTATGCACCCGAACTGCTGTTCTTTGCCAAGAGATACAA
AGCCGCTTTCACCGAGTGCTGTCAGGCAGCCGATAAGGCTGCATGCCTGCTGCCAAAACTG
GACGAGCTGCGAGATGAAGGGAAGGCCAGCTCCGCTAAGCAGCGGCTGAAATGTGCTAGC
CTGCAGAAGTTCGGAGAGCGAGCCTTCAAGGCATGGGCTGTGGCACGACTGTCCCAGCGG
TTCCCCAAAGCAGAGTTTGCCGAAGTCTCTAAGCTGGTGACAGACCTGACTAAAGTGCACA
CCGAGTGCTGTCATGGCGACCTGCTGGAATGCGCCGACGATCGAGCTGATCTGGCAAAGT
ACATCTGTGAGAATCAGGACAGCATTTCTAGTAAGCTGAAAGAGTGCTGTGAAAAGCCTCT
GCTGGAGAAATCCCACTGCATCGCCGAGGTGGAAAACGACGAAATGCCAGCTGATCTGCC
CTCACTGGCCGCTGACTTTGTCGAGAGCAAGGATGTGTGTAAAAATTATGCCGAAGCTAAG
 
GATGTGTTCCTGGGCATGTTTCTGTACGAGTATGCAAGGGCAGGAGGTCCGGAGGCTCTG
GAGGAAGTGGAGGGTCAGGAGGCTCAAGCGAACTGACTCAGGACCCCGCTGTGAGCGTCG
CACTGGGACAGACTGTGAGGATCACCTGCCAGGGGGACAGCCTGCGCTCCTACTATGCATC
CTGGTACCAGCAGAAGCCTGGCCAGGCCCCAGTGCTGGTCATCTATGGCAAAAACAATCG
GCCCTCAGGGATTCCTGATCGGTTCAGCGGGTCCTCTAGTGGAAACACAGCTTCTCTGACC
ATTACAGGCGCTCAGGCAGAGGACGAAGCCGATTACTATTGCAACAGCCGCGACTCAAGC
GGGAATCATGTGGTCTTCGGAGGAGGAACCAAGCTGACAGTGGGAGGAGGCTCTGGAGGA
GGCAGTGGGGGAGGCTCAGGAGGAGGCAGCGGAGGAGGCTCCGGAGAGGTCCAGCTGGT
GGAAAGCGGAGGAGGAGTGGTCCGCCCAGGAGGATCTCTGCGACTGAGTTGTGCAGCCTC
AGGATTCACCTTTGACGATTACGGAATGAGTTGGGTCCGGCAGGCACCTGGAAAGGGACT
GGAGTGGGTGAGCGGCATCAACTGGAATGGCGGGAGCACTGGGTACGCTGATTCCGTGAA
GGGAAGATTCACCATTTCCAGGGACAACGCCAAAAATTCTCTGTATCTGCAGATGAATAGT
CTGAGAGCCGAGGACACAGCTGTGTACTATTGCGCCAGGGGGAGGTCTCTGCTGTTCGACT
ACTGGGGGCAGGGCACTCTGGTCACTGTGTCAAGATGAGGATCC
8. SEQ ID NO: 98 (Base construct # 2 Protein) 
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGSSELTQDPAVSVALGQTVRITCQG
DSLRSYYASWYQQKPGQAPVLVIYGKNNRPSGIPDRFSGSSSGNTASLTITGAQAEDEADYYC
NSRDSSGNHVVFGGGTKLTVGGGSGGGSGGGSGGGSGGGSGEVQLVESGGGVVRPGGSLRLS
CAASGFTFDDYGMSWVRQAPGKGLEWVSGINWNGGSTGYADSVKGRFTISRDNAKNSLYLQ
MNSLRAEDTAVYYCARGRSLLFDYWGQGTLVTVSR
9. SEQ ID NO: 99 (Base construct #3 NT) 
TCCTCCGAGCTGACCCAGGACCCTGCCGTGTCCGTCGCTCTGGGACAGACCGTGCGGATCA
CATGCCAGGGAGATAGCCTGAGATCCTACTATGCTAGCTGGTACCAGCAGAAACCCGGCC
 
AGGCACCTGTGCTGGTCATCTATGGAAGAACAATCGCCCATCTGGCATCCCCGACCGATT
CAGTGGAAGCTCCTCTGGCAACACAGCCTCTCTGACTATTACCGGCGCTCAGGCAGAGGAC
GAAGCTGATTACTATTGCAACAGCAGGGATAGTTCAGGGAATCACGTGGTCTTTGGAGGA
GGAACTAAGCTGACCGTGGGAGGAGGATCTGGAGGAGGAAGTGGCGGGGGATCAGGAGG
AGGAAGCGGAGGAGGCAGCGGAGAGGTGCAGCTGGTCGAAAGCGGAGGAGGAGTGGTCA
GACCAGGAGGGTCTCTGAGGCTGAGTTGTGCCGCTTCAGGCTTCACCTTTGACGATTACGG
AATGTCTTGGGTGCGGCAGGCACCTGGAAAGGGACTGGAGTGGGTGAGTGGCATCAACTG
GAATGGAGGCAGCACAGGATACGCAGACTCCGTGAAAGGCCGATTCACTATTTCACGGGA
TAACGCCAAGAATAGCCTGTATCTGCAGATGAACAGCCTGAGAGCAGAGGACACAGCCGT
GTACTATTGTGCCAGGGGCCGCTCTCTGCTGTTTGATTACTGGGGGCAGGGAACACTGGTG
ACTGTCAGCCGAGGAGGATCTGGAGGGAGTGGAGGCTCAGGAGGAAGCGGAGGGTCCGT
GGTCCTGCTGCTGCGACTGGCTAAAACTTACGAGACCACACTGGAAAAGTGCTGTGCAGCC
GCTGACCCCCATGAGTGCTATGCAAAAGTGTTCGATGAGTTCAAGCCTCTGGTCGAGGAAC
CACAGAACCTGATCAAACAGAATTGTGAGCTGTTCGAACAGCTGGGCGAGTACAAGTTTC
AGAACGCCCTGCTGGTGAGATATACCAAGAAAGTGCCCCAGGTCTCTACACCTACTCTGGT
GGAGGTCAGTAGGAATCTGGGCAAAGTGGGGTCAAAATGCTGTAAGCACCCAGAGGCTAA
GCGCATGCCCTGCGCAGAAGACTACCTGAGCGTGGTCCTGAACCAGCTGTGTGTGCTGCAT
GAGAAAACTCCAGTGTCCGATAGGGTCACTAAGTGCTGTACCGAAAGCCTGGTGAACCGG
AGACCTTGCTTCTCCGCCCTGGAGGTGGACGAAACCTATGTCCCAAAAGAGTTTAATGCCG
AAACCTTCACATTTCACGCTGATATCTGTACCCTGTCCGAGAAGGAACGCCAGATTAAGAA
ACAGACAGCTCTGGTGGAGCTGGTCAAGCATAAACCCAAGGCAACAAAAGAACAGCTGAA
GGCCGTGATGGACGATTTCGCAGCCTTTGTGGAGAAATGCTGTAAGGCCGACGATAAGGA
AACTTGCTTTGCTGAAGAAGGGAAGAAACTGGTCGCCGCATCACAGGCTGCTCTGGGACT
GTGAGGATCC
10. SEQ ID NO: 100 (Base Construct #3 Protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
GSGGSGGSGGSVVLLLRLAKTYETTLEKCCAAADPHECYAKVFDEFKPLVEEPQNLIKQNCEL
 
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGL
11. SEQ ID NO: 101 (Base construct # 4) 
AGCGTCGTCCTGCTGCTGAGACTGGCTAAAACATACGAGACCACACTGGAAAAGTGCTGT
GCCGCTGCAGACCCTCACGAGTGCTATGCCAAAGTGTTCGATGAGTTCAAGCCTCTGGTCG
AGGAACCACAGAACCTGATCAAACAGAATTGTGAGCTGTTCGAACAGCTGGGCGAGTACA
AGTTTCAGAACGCCCTGCTGGTGAGGTATACTAAGAAAGTGCCCCAGGTCAGTACTCCTAC
CCTGGTGGAGGTCTCACGGAATCTGGGAAAGTGGGAAGCAAATGCTGTAAGCACCCAGA
GGCAAAGAGAATGCCCTGCGCCGAAGACTACCTGAGCGTGGTCCTGAACCAGCTGTGTGT
GCTGCATGAGAAAACTCCAGTGAGCGATAGGGTCACAAAGTGCTGTACTGAATCCCTGGT
GAACCGGAGACCTTGCTTCTCTGCCCTGGAGGTGGACGAAACCTATGTCCCAAAGGAGTTT
AATGCTGAAACATTCACTTTTCACGCAGATATCTGTACACTGAGCGAGAAGGAACGCCAGA
TTAAGAAACAGACTGCCCTGGTGGAGCTGGTCAAGCATAAACCCAAGGCCACCAAAGAAC
AGCTGAAGGCTGTGATGGACGATTTCGCCGCTTTTGTCGAGAAATGCTGTAAGGCAGACGA
TAAGGAAACATGCTTCGCCGAGGAAGGGAAGAAACTGGTGGCAGCAAGCCAGGCTGCACT
GGGACTGGGAGGGTCTGGAGGCAGTGGAGGATCAGGAGGGAGCGGAGGCAGCTCCGAGC
TGACCCAGGACCCCGCTGTGAGCGTCGCACTGGGACAGACCGTGCGCATCACATGTCAGG
GCGATTCCCTGCGATCTTACTATGCTTCCTGGTACCAGCAGAAACCCGGCCAGGCACCTGT
GCTGGTCATCTATGGAAAGAACAATAGACCAAGTGGCATTCCCGACAGGTTCTCAGGCTCT
AGTTCAGGGAACACCGCCTCCCTGACCATTACAGGCGCACAGGCCGAGGACGAAGCTGAT
TACTATTGCAACTCTCGGGATAGCTCCGGCAATCATGTGGTCTTTGGGGGAGGCACTAAGC
TGACCGTGGGGGGAGGCAGTGGGGGAGGCTCAGGAGGAGGCAGCGGAGGAGGCTCCGGA
GGAGGCTCTGGCGAGGTGCAGCTGGTCGAATCCGGAGGAGGAGTGGTCCGACCAGGAGGA
AGTCTGCGACTGTCATGTGCCGCTAGCGGGTTCACCTTTGACGATTACGGAATGAGTTGGG
TGCGACAGGCACCTGGAAAGGGACTGGAGTGGGTGTCTGGCATCAACTGGAATGGCGGGT
CCACTGGCTACGCAGACTCTGTGAAAGGGAGGTTTACCATTAGCCGCGATAACGCCAAGA
ACAGCCTGTATCTGCAGATGAACAGCCTGCGCGCCGAGGACACAGCTGTGTACTATTGCGC
 
CAGGGGGAGGTCACTGCTGTTTGATTACTGGGGGCAGGGGACTCTGGTCACTGTGTCACGG
TGAGGATCC
12. SEQ ID NO: 102 (Protein Base Construct #4) 
SVVLLLRLAKTYETTLEKCCAAADPHECYAKVFDEFKPLVEEPQNLIKQNCELFEQLGEYKFQ
NALLVRYTKKVPQVSTPTLVEVSRNLGKVGSKCCKHPEAKRMPCAEDYLSVVLNQLCVLHEK
TPVSDRVTKCCTESLVNRRPCFSALEVDETYVPKEFNAETFTFHADICTLSEKERQIKKQTALVE
LVKHKPKATKEQLKAVMDDFAAFVEKCCKADDKETCFAEEGKKLVAASQAALGLGGSGGSG
GSGGSGGSSELTQDPAVSVALGQTVRITCQGDSLRSYYASWYQQKPGQAPVLVIYGKNNRPSG
IPDRFSGSSSGNTASLTITGAQAEDEADYYCNSRDSSGNHVVFGGGTKLTVGGGSGGGSGGGS
GGGSGGGSGEVQLVESGGGVVRPGGSLRLSCAASGFTFDDYGMSWVRQAPGKGLEWVSGIN
WNGGSTGYADSVKGRFTISRDNAKNSLYLQMNSLRAEDTAVYYCARGRSLLFDYWGQGTLV
TVSR
13. SEQ ID NO: 103 (Base construct 5) 
GACATTCAGATGACACAGTCCCCAAGCTCCCTGAGCGCTTCCGTCGGCGATCGAGTGACTA
TCACCTGCCGAGCCTCTCAGGACGTCAACACTGCTGTGGCATGGTACCAGCAGAAGCCTGG
GAAAGCACCAAAGCTGCTGATCTACTCTGCCAGTTTTCTGTATTCTGGAGTGCCCAGTAGA
TTCTCAGGAAGCAGGTCCGGCACCGATTTTACACTGACTATCTCTAGTCTGCAGCCTGAGG
ACTTCGCCACATACTATTGCCAGCAGCACTATACCACACCCCCTACATTTGGACAGGGCAC
TAAAGTGGAAATTAAGGGCGGGTCAGGCGGAGGGAGCGGAGGAGGGTCCGGAGGAGGGT
CTGGAGGAGGGAGTGGAGAGGTGCAGCTGGTCGAATCCGGAGGAGGACTGGTGCAGCCTG
GAGGCTCACTGAGGCTGAGCTGTGCCGCTTCCGGCTTCAACATCAAGGATACCTACATTCA
TTGGGTCAGACAGGCTCCTGGGAAAGGACTGGAGTGGGTGGCAAGGATCTATCCAACCAA
TGGGTACACACGGTATGCCGATAGCGTGAAGGGAAGATTCACTATTTCTGCTGACACTAGT
AAAAACACCGCATACCTGCAGATGAATAGCCTGAGGGCAGAGGACACCGCCGTGTACTAT
TGCTCCCGCTGGGGGGGAGACGGCTTTTACGCCATGGATTATTGGGGCCAGGGGACCCTGG
TGACAGTCTCAAGCGGCGGGTCAGGAGATGCACACAAAAGCGAGGTCGCCCATCGCTTCA
AGGACCTGGGCGAGGAAAATTTTAAAGCCCTGGTGCTGATTGCCTTCGCTCAGTACCTGCA
GCAGTGCCCATTCGAAGACCACGTGAAGCTGGTCAACGAGGTGACCGAATTTGCCAAAAC
 
ATGCGTCGCTGACGAGTCCGCAGAAAATTGTGATAAGTCTCTGCATACACTGTTCGGCGAT
AAACTGTGTACTGTGGCCACCCTGCGCGAGACTTATGGGGAAATGGCCGACTGCTGTGCTA
AGCAGGAGCCAGAACGAAACGAGTGCTTTCTGCAGCACAAGGACGATAACCCAAATCTGC
CAAGGCTGGTGCGCCCAGAAGTGGACGTCATGTGTACTGCTTTCCACGATAATGAGGAAAC
CTTTCTGAAGAAATACCTGTATGAGATCGCCCGGAGACATCCATACTTCTATGCCCCCGAA
CTGCTGTTCTTTGCTAAACGGTACAAGGCAGCCTTTACCGAGTGCTGTCAGGCTGCAGATA
AAGCCGCTTGCCTGCTGCCTAAGCTGGACGAGCTGCGAGATGAAGGCAAGGCATCCTCTG
CCAAACAGCGGCTGAAGTGTGCCAGCCTGCAGAAATTCGGGGAGCGGGCTTTTAAGGCAT
GGGCCGTGGCTCGACTGTCTCAGCGGTTCCCAAAGGCTGAGTTTGCAGAAGTCAGTAAACT
GGTGACAGACCTGACTAAGGTGCACACAGAGTGCTGTCATGGCGACCTGCTGGAATGCGC
CGACGATAGAGCCGATCTGGCTAAGTACATCTGTGAGAACCAGGACAGCATTAGTTCAAA
GCTGAAAGAGTGCTGTGAAAAACCTCTGCTGGAGAAGAGCCACTGCATCGCAGAGGTGGA
AAATGACGAAATGCCCGCCGATCTGCCTAGTCTGGCAGCCGACTTCGTCGAGTCAAAAGAT
GTGTGTAAGAACTACGCCGAAGCAAAAGATGTGTTTCTGGGAATGTTTCTGTATGAGTATG
CCCGAGCCTGAGGATCC
14. SEQ ID NO: 104 (Base construct 5 protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARA
15. SEQ ID NO: 105 (Base construct # 6) 
GACGCACATAAGTCCGAGGTCGCTCACAGGTTCAAAGATCTGGGCGAGGAAAACTTTAAG
GCCCTGGTGCTGATCGCTTTCGCACAGTACCTGCAGCAGTGCCCATTCGAAGACCACGTGA
AACTGGTCAACGAAGTGACTGAATTTGCCAAGACCTGCGTCGCCGACGAGTCCGCTGAAA
ATTGTGATAAATCTCTGCATACTCTGTTCGGGGATAAGCTGTGTACCGTGGCCACACTGCG
CGAGACCTATGGAGAAATGGCAGACTGCTGTGCCAAACAGGAGCCAGAACGAAACGAGT
GCTTTCTGCAGCATAAGGACGATAACCCAAATCTGCCAAGGCTGGTGCGCCCAGAAGTGG
ACGTCATGTGTACCGCCTTCCACGATAATGAGGAAACATTTCTGAAGAAATACCTGTATGA
GATTGCCCGGAGACATCCATACTTCTATGCCCCCGAACTGCTGTTCTTTGCTAAGCGCTACA
AAGCCGCTTTTACCGAGTGCTGTCAGGCAGCCGATAAAGCTGCATGCCTGCTGCCTAAGCT
GGACGAGCTGAGGGATGAAGGAAAGGCCAGCTCCGCTAAACAGCGCCTGAAGTGTGCCTC
TCTGCAGAAATTCGGCGAGCGGGCTTTTAAGGCATGGGCTGTCGCACGACTGAGCCAGCG
GTTCCCAAAGGCAGAGTTTGCCGAAGTCTCCAAACTGGTGACTGACCTGACCAAGGTGCAC
ACCGAGTGCTGTCATGGCGACCTGCTGGAATGCGCCGACGATAGAGCTGATCTGGCAAAG
TACATCTGTGAGAACCAGGACAGCATTTCTAGTAAGCTGAAAGAGTGCTGTGAAAAACCC
CTGCTGGAGAAGAGCCACTGCATCGCAGAGGTGGAAAACGACGAAATGCCTGCCGATCTG
CCAAGTCTGGCCGCTGACTTCGTCGAGTCAAAAGATGTGTGTAAGAATTATGCCGAAGCTA
AGGATGTGTTCCTGGGCATGTTTCTGTACGAGTATGCACGAGCAGGAGGGAGCGGAGGCT
CCGGAGGATCTGGCGGGAGTGGAGGCGACATCCAGATGACTCAGTCCCCTTCAAGCCTGA
GTGCTTCAGTCGGCGATCGCGTGACTATTACCTGCCGAGCCTCTCAGGACGTCAATACAGC
TGTGGCATGGTACCAGCAGAAGCCCGGCAAAGCTCCTAAGCTGCTGATCTACAGCGCATCC
TTTCTGTATTCAGGGGTGCCCAGCAGATTCTCTGGCAGTAGATCAGGGACAGATTTTACAC
TGACTATTTCCTCTCTGCAGCCTGAGGACTTCGCCACTTACTATTGCCAGCAGCACTATACC
ACACCCCCTACATTTGGACAGGGCACTAAAGTGGAAATCAAGGGAGGCAGCGGAGGAGG
ATCTGGAGGAGGAAGTGGAGGAGGATCAGGAGGAGGAAGCGGAGAGGTCCAGCTGGTGG
AAAGCGGAGGAGGACTGGTGCAGCCTGGAGGGTCCCTGAGACTGTCTTGTGCAGCCAGTG
GCTTCAACATCAAAGATACCTACATTCATTGGGTCAGACAGGCTCCTGGGAAGGGACTGGA
GTGGGTGGCAAGGATCTATCCAACAAATGGATACACTCGGTATGCCGATAGCGTGAAAGG
CCGGTTCACCATTTCAGCAGACACCAGCAAGAACACAGCCTACCTGCAGATGAACAGCCT
GCGAGCTGAGGACACAGCAGTGTACTATTGCAGTCGGTGGGGCGGCGATGGCTTTTACGCT
ATGGACTATTGGGGGCAGGGGACACTGGTGACTGTGAGTTCTTGAGGATCC
16. SEQ ID NO: 106 (Protein Base construct # 6) 
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGDIQMTQSPSSLSASVGDRVTITCR
ASQDVNTAVAWYQQKPGKAPKLLIYSASFLYSGVPSRFSGSRSGTDFTLTISSLQPEDFATYYC
QQHYTTPPTFGQGTKVEIKGGSGGGSGGGSGGGSGGGSGEVQLVESGGGLVQPGGSLRLSCAA
SGFNIKDTYIHWVRQAPGKGLEWVARIYPTNGYTRYADSVKGRFTISADTSKNTAYLQMNSLR
AEDTAVYYCSRWGGDGFYAMDYWGQGTLVTVSS
17. SEQ ID NO: 107 (Base construct # 7) 
GACATTCAGATGACACAGAGCCCAAGCTCCCTGTCCGCATCTGTGGGCGACCGAGTCACA
ATCACTTGCCGGGCCTCCCAGGATGTGAACACTGCTGTCGCATGGTACCAGCAGAAACCAG
GGAAGGCTCCCAAACTGCTGATCTACAGTGCATCATTCCTGTATAGTGGCGTGCCATCAAG
GTTTAGCGGCTCCCGATCTGGAACCGACTTCACCCTGACAATCTCTAGTCTGCAGCCCGAG
GATTTTGCCACATACTATTGCCAGCAGCACTATACCACCCCCTACTTTCGGGCAGGGAA
CCAAGGTGGAGATCAAGGGAGGGAGCGGAGGAGGGTCCGGAGGAGGGTCTGGAGGCGGG
AGTGGAGGAGGGTCAGGAGAGGTGCAGCTGGTCGAAAGCGGAGGAGGACTGGTGCAGCC
TGGAGGCAGCCTGCGACTGTCCTGTGCCGCTTCTGGCTTTAACATCAAGGACACCTACATT
CATTGGGTGCGGCAGGCACCTGGCAAAGGACTGGAGTGGGTGGCTAGAATCTATCCAACT
AATGGATACACCAGATATGCTGACAGCGTGAAGGGCAGGTTTACTATCAGTGCTGATACAT
CAAAGAACACTGCATACCTGCAGATGAATAGCCTGCGCGCCGAGGATACCGCTGTGTACT
ATTGTAGCCGATGGGGGGGAGACGGCTTCTACGCCATGGATTATTGGGGACAGGGCACCC
TGGTGACAGTCTCAAGCGGAGGGAGTGGAGGCTCAGGAGGAAGCGGAGGGTCCGGAGGC
TCTGTGGTCCTGCTGCTGAGACTGGCTAAGACCTACGAGACTACCCTGGAAAAATGCTGTG
CAGCCGCTGACCCCCACGAGTGCTATGCAAAGGTGTTCGATGAGTTCAAGCCTCTGGTCGA
GGAACCACAGAACCTGATCAAGCAGAATTGTGAGCTGTTCGAACAGCTGGGCGAGTACAA
 
GTTTCAGAACGCCCTGCTGGTGAGGTATACAAAGAAAGTGCCCCAGGTCAGCACTCCTACC
CTGGTGGAGGTCTCCAGGAATCTGGGAAGGTCGGATCTAAGTGCTGTAAACACCCAGAG
GCAAAACGCATGCCCTGCGCCGAAGACTACCTGTCCGTGGTCCTGAATCAGCTGTGTGTGC
TGCATGAGAAGACCCCTGTGTCTGATCGAGTCACCAAATGCTGTACAGAAAGTCTGGTGAA
CCGGAGACCCTGCTTTTCTGCCCTGGAGGTGGACGAAACATATGTCCCTAAGGAGTTCAAT
GCCGAAACATTCACTTTTCACGCTGATATCTGTACACTGTCCGAGAAGGAACGCCAGATTA
AGAAACAGACTGCTCTGGTGGAGCTGGTCAAGCATAAACCAAAGGCAACCAAGGAACAGC
TGAAAGCCGTGATGGACGATTTCGCAGCCTTTGTCGAGAAGTGCTGTAAAGCCGACGATAA
GGAAACTTGTTTCGCCGAGGAAGGCAAAAAACTGGTCGCAGCATCACAGGCAGCACTGGG
ACTGTGAGGATCC
18. SEQ ID NO: 108 (Base construct # 7 Protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
GSGGSGGSGGSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGL
19. SEQ ID NO: 109 (Base construct # 8) 
TCCGTCGTCCTGCTGCTGAGACTGGCTAAGACCTACGAGACCACACTGGAAAAATGCTGTG
CCGCTGCAGACCCCCACGAGTGCTATGCCAAGGTGTTCGATGAGTTCAAGCCTCTGGTCGA
GGAACCACAGAACCTGATCAAGCAGAATTGTGAGCTGTTCGAACAGCTGGGCGAGTACAA
ATTTCAGAACGCCCTGCTGGTGAGGTATACAAAGAAAGTGCCCCAGGTCTCTACACCTACT
CTGGTGGAGGTCAGTAGGAATCTGGGCAAGGTCGGGTCAAAATGCTGTAAGCACCCAGAG
GCCAAACGCATGCCCTGCGCTGAAGACTACCTGTCTGTGGTCCTGAACCAGCTGTGTGTGC
TGCATGAGAAGACCCCTGTGAGCGATCGAGTCACCAAATGCTGTACAGAAAGCCTGGTGA
 
ATCGGAGACCCTGCTTTTCCGCTCTGGAGGTGGACGAAACATATGTCCCTAAGGAGTTCAA
TGCAGAAACCTTCACATTTCACGCCGATATCTGTACTCTGTCCGAGAAGGAACGCCAGATT
AAGAAACAGACCGCCCTGGTGGAGCTGGTCAAGCATAAACCAAAGGCTACTAAGGAACAG
CTGAAAGCAGTGATGGACGATTTCGCCGCTTTTGTCGAGAAATGCTGTAAGGCAGACGATA
AGGAAACCTGCTTTGCCGAGGAAGGCAAGAAACTGGTGGCAGCCAGCCAGGCTGCACTGG
GACTGGGAGGGTCCGGAGGCTCTGGAGGAAGTGGAGGGTCAGGAGGCGACATCCAGATG
ACACAGAGCCCAAGCTCCCTGTCAGCAAGCGTGGGCGACCGAGTCACTATTACCTGTCGG
GCCTCCCAGGATGTGAATACTGCAGTCGCCTGGTACCAGCAGAAACCAGGAAAGGCTCCC
AAACTGCTGATCTACTCCGCATCTTTCCTGTATAGCGGCGTGCCATCCAGGTTTAGTGGATC
ACGCAGCGGCACAGACTTCACACTGACTATTTCTAGTCTGCAGCCCGAGGATTTTGCCACT
TACTATTGCCAGCAGCACTATACTACCCCCCCTACCTTCGGACAGGGCACAAAGGTGGAGA
TCAAGGGAGGATCTGGAGGAGGAAGTGGAGGAGGATCAGGAGGAGGAAGCGGAGGAGGC
AGCGGAGAGGTGCAGCTGGTCGAATCTGGAGGAGGACTGGTGCAGCCTGGAGGGTCTCTG
CGACTGAGTTGTGCCGCTTCAGGCTTTAACATCAAGGACACCTACATTCATTGGGTGCGGC
AGGCACCTGGGAAGGGACTGGAGTGGGTCGCTAGAATCTATCCAACTAATGGGTACACCA
GATATGCCGACAGCGTGAAGGGAAGGTTCACCATTAGCGCCGATACATCCAAAAACACTG
CTTACCTGCAGATGAACAGCCTGCGCGCTGAGGATACAGCAGTGTACTATTGCAGTCGATG
GGGCGGCGATGGGTTCTACGCAATGGACTACTGGGGACAGGGGACTCTGGTCACCGTCAG
CAGCTGAGGATCC
20. SEQ ID NO: 110 (Base construct # 8 Protein) 
SVVLLLRLAKTYETTLEKCCAAADPHECYAKVFDEFKPLVEEPQNLIKQNCELFEQLGEYKFQ
NALLVRYTKKVPQVSTPTLVEVSRNLGKVGSKCCKHPEAKRMPCAEDYLSVVLNQLCVLHEK
TPVSDRVTKCCTESLVNRRPCFSALEVDETYVPKEFNAETFTFHADICTLSEKERQIKKQTALVE
LVKHKPKATKEQLKAVMDDFAAFVEKCCKADDKETCFAEEGKKLVAASQAALGLGGSGGSG
GSGGSGGDIQMTQSPSSLSASVGDRVTITCRASQDVNTAVAWYQQKPGKAPKLLIYSASFLYS
GVPSRFSGSRSGTDFTLTISSLQPEDFATYYCQQHYTTPPTFGQGTKVEIKGGSGGGSGGGSGG
GSGGGSGEVQLVESGGGLVQPGGSLRLSCAASGFNIKDTYIHWVRQAPGKGLEWVARIYPTNG
YTRYADSVKGRFTISADTSKNTAYLQMNSLRAEDTAVYYCSRWGGDGFYAMDYWGQGTLVT
VSS
21. SEQ ID NO: 111 (Base construct # 9) 
TCAAGCGAACTGACTCAGGACCCCGCTGTGAGCGTCGCACTGGGACAGACTGTGCGGATC
ACCTGCCAGGGGGACTCCCTGAGATCTTACTATGCCTCCTGGTACCAGCAGAAACCAGGCC
AGGCTCCCGTGCTGGTCATCTATGGCAAGAACAATAGACCTTCCGGGATTCCAGATAGGTT
TTCTGGAAGCTCCTCTGGCAACACAGCTAGCCTGACCATTACAGGAGCCCAGGCTGAGGAC
GAAGCAGATTACTATTGCAACTCCAGGGACAGTTCAGGCAATCACGTGGTCTTCGGCGGGG
GAACAAAGCTGACTGTGGGAGGAGGATCAGGAGGAGGAAGCGGAGGAGGCAGCGGAGGA
GGATCTGGAGGAGGAAGTGGAGAGGTGCAGCTGGTCGAAAGCGGAGGAGGAGTGGTCAG
GCCTGGAGGGTCACTGCGACTGAGCTGTGCCGCTTCCGGATTCACATTTGACGATTACGGA
ATGTCTTGGGTCCGGCAGGCACCAGGAAAGGGACTGGAGTGGGTGAGTGGCATCAACTGG
AATGGAGGCTCTACAGGGTACGCTGATAGTGTGAAAGGACGCTTTACTATTAGTCGAGACA
ACGCCAAGAACAGCCTGTATCTGCAGATGAACAGCCTGAGAGCCGAGGATACTGCTGTGT
ACTATTGTGCCAGGGGCCGCTCCCTGCTGTTCGACTACTGGGGGCAGGGAACCCTGGTGAC
AGTCTCTAGGGGGGAAGTGGCGATGCTCACAAGAGCGAGGTCGCACATCGCTTCAAAGA
CCTGGGGGAGGAAAATTTTAAGGCCCTGGTGCTGATCGCATTCGCCCAGTATCTGCAGCAG
TGCCCTTTTGAAGACCACGTGAAACTGGTCAACGAGGTGACCGAGTTCGCCAAGACATGC
GTGGCAGACGAGTCCGCCGAAAATTGTGATAAATCTCTGCATACTCTGTTTGGGGATAAGC
TGTGTACTGTGGCCACCCTGCGGGAGACCTACGGAGAAATGGCTGACTGCTGTGCAAAAC
AGGAGCCAGAAAGAAACGAGTGCTTCCTGCAGCACAAGGACGATAACCCCAATCTGCCTC
GACTGGTGCGGCCCGAAGTGGACGTCATGTGTACTGCCTTCCACGATAATGAGGAAACCTT
TCTGAAGAAATACCTGTATGAGATTGCCCGGAGACATCCCTACTTTTATGCCCCTGAACTG
CTGTTCTTTGCTAAGCGGTACAAAGCAGCCTTCACCGAGTGCTGTCAGGCTGCAGATAAGG
CCGCTTGCCTGCTGCCAAAACTGGACGAGCTGCGAGATGAAGGAAAGCTAGCTCCGCAA
AGCAGAGACTGAAATGTGCAAGCCTGCAGAAGTTCGGCGAGAGGGCCTTTAAAGCTTGGG
CAGTGGCCAGACTGAGCCAGAGGTTCCCCAAGGCCGAGTTTGCTGAAGTCTCCAAGCTGGT
GACAGACCTGACTAAAGTGCACACCGAGTGCTGTCATGGCGACCTGCTGGAATGCGCCGA
CGATCGCGCAGATCTGGCCAAATACATCTGTGAGAACCAGGACTCTATTTCTAGTAAGCTG
AAAGAGTGCTGTGAAAAGCCTCTGCTGGAGAAAAGCCACTGCATCGCTGAGGTGGAAAAC
GACGAAATGCCCGCAGATCTGCCTAGTCTGGCAGCCGACTTTGTCGAGTCAAAGGATGTGT
 
GTAAAAATTATGCTGAAGCAAAGGATGTGTTCCTGGGCATGTTTCTGTACGAGTATGCACG
AGCTGGAGGGAGTGGAGGCTCAGGAGGAAGCGGCGGTCCGGAGGCTCAAGCGAACTGA
CCCAGGACCCCGCCGTGTCTGTCGCTCTGGGACAGACAGTGAGGATCACTTGCCAGGGCG
ACTCTCTGCGCAGTTACTATGCAAGTTGGTATCAGCAGAAGCCTGGCCAGGCCCCTGTCCT
GGTCATCTATGGCAAGAATAATCGCCCTAGTGGGATTCCAGATCGATTTTCAGGGTCCTCT
AGTGGAAACACAGCTTCTCTGACTATTACCGGCGCACAGGCCGAGGACGAAGCCGATTAC
TATTGCAACAGCAGAGACTCAAGCGGCAATCATGTGGTCTTCGGAGGAGGAACCAAGCTG
ACAGTGGGAGGAGGCTCAGGCGGCGGCAGCGGAGGAGGCTCCGGGGGAGGCTCTGGAGG
AGGCAGTGGAGAGGTCCAGCTGGTGGAATCCGGAGGAGGAGTGGTCCGACCAGGAGGATC
ACTGAGACTGTCCTGTGCTGCATCCGGATTCACCTTCGATGATTACGGAATGAGCTGGGTC
AGGCAGGCACCTGGCAAGGGCCTGGAATGGGTGTCCGGCATCAACTGGAATGGCGGGTCA
ACCGGGTACGCTGATAGCGTGAAAGGACGGTTCACAATTAGCAGGGATAATGCTAAGAAC
AGCTTATATCTGCAAATGAACAGCCTGCGCGCAGAGGACACAGCCGTGTACTATTGCGCC
CGGGGGCGGAGCCTGCTGTTTGATTACTGGGGGCAGGGCACACTGGTGACCGTCTCTCGGT
GAGGATCC
22. SEQ ID NO: 112 (Base construct # 9 protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGSSELTQDPAVSVALGQTVRITCQG
DSLRSYYASWYQQKPGQAPVLVIYGKNNRPSGIPDRFSGSSSGNTASLTITGAQAEDEADYYC
NSRDSSGNHVVFGGGTKLTVGGGSGGGSGGGSGGGSGGGSGEVQLVESGGGVVRPGGSLRLS
CAASGFTFDDYGMSWVRQAPGKGLEWVSGINWNGGSTGYADSVKGRFTISRDNAKNSLYLQ
MNSLRAEDTAVYYCARGRSLLFDYWGQGTLVTVSR
23. SEQ ID NO: 113 (Base construct # 10) 
AGTAGCGAACTGACCCAGGACCCCGCAGTGAGCGTCGCACTGGGGCAGACAGTGAGAATC
ACTTGCCAGGGAGATTCTCTGAGGAGTTACTATGCCTCCTGGTACCAGCAGAAACCCGGCC
AGGCTCCTGTGCTGGTCATCTATGGAAGAACAATAGGCCAAGCGGCATCCCCGACCGCTT
CTCCGGCAGCTCCTCTGGAACACAGCTAGCCTGACTATTACCGGCGCTCAGGCAGAGGAC
GAAGCAGATTACTATTGCAACTCCAGGGATAGTTCAGGCAATCACGTGGTCTTTGGCGGGG
GAACAAAGCTGACTGTGGGAGGAGGAAGCGGAGGAGGCAGCGGAGGGGGATCTGGAGGA
GGAAGTGGAGGAGGATCAGGAGAGGTGCAGCTGGTCGAAAGCGGAGGAGGAGTGGTCCG
CCCTGGAGGGAGCCTGCGACTGTCCTGTGCCGCTTCTGGCTTCACCTTTGACGATTACGGA
ATGAGCTGGGTGCGGCAGGCACCAGGGAAGGGACTGGAGTGGGTGTCCGGCATCAACTGG
AATGGAGGCTCCACAGGATACGCAGACTCTGTGAAAGGCCGATTCACTATTTCTCGGGATA
ACGCCAAGAATAGTCTGTATCTGCAGATGAACAGCCTGAGAGCTGAGGACACTGCAGTGT
ACTATTGTGCCAGGGGCCGCAGCCTGCTGTTTGATTACTGGGGCCAGGGAACCCTGGTGAC
AGTCTCCAGGGGAGGATCAGGAGGGAGCGGAGGCTCCGGAGGATCTGGAGGGAGTGTGGT
CCTGCTGCTGCGACTGGCTAAAACCTACGAGACCACACTGGAAAAGTGCTGTGCAGCCGCT
GACCCTCATGAGTGCTATGCCAAAGTGTTCGATGAGTTCAAGCCACTGGTCGAGGAACCCC
AGAACCTGATCAAACAGAATTGTGAGCTGTTCGAACAGCTGGGCGAGTACAAGTTTCAGA
ACGCCCTGCTGGTGCGCTATACCAAGAAAGTGCCTCAGGTCAGCACACCAACTCTGGTGGA
AGTCTCCCGGAATCTGGGGAAAGTGGGATCTAAATGCTGTAAGCACCCCGAGGCTAAGAG
AATGCCTTGCGCAGAAGACTACCTGTCTGTGGTCCTGAACCAGCTGTGTGTGCTGCATGAG
AAAACCCCAGTGAGCGATAGGGTCACCAAGTGCTGTACAGAAAGTCTGGTGAACCGGAGA
CCATGCTTCTCAGCCCTGGAGGTGGACGAAACATATGTCCCCAAAGAGTTTAATGCCGAAA
CCTTCACATTTCACGCTGATATCTGTACTCTGTCCGAGAAGGAACGCCAGATTAAGAAACA
GACCGCCCTGGTGGAGCTGGTCAAGCATAAACCCAAGGCAACAAAAGAACAGCTGAAGGC
CGTGATGGACGATTTCGCAGCCTTTGTCGAGAAATGCTGTAAGGCTGACGATAAGGAAACT
TGCTTCGCAGAGGAAGGAAAGAAACTGGTGGCTGCAAGCCAGGCAGCTCTGGGACTGGGA
GGCTCAGGAGGAAGCGGCGGGTCCGGAGGCTCTGGGGGAAGCTCCGAGCTGACCCAGGAC
CCAGCCGTGTCTGTCGCTCTGGGCCAGACTGTGCGCATCACCTGTCAGGGGGATAGTCTGC
GATCATACTATGCAAGTTGGTATCAGCAGAAACCTGGCCAGGCCCCAGTCCTGGTCATCTA
TGGGAAGAATAATCGACCTTCCGGCATCCCCGACCGGTTCTCCGGATCTAGTTCAGGCAAC
ACAGCCTCTCTGACTATTACCGGCGCCCAGGCTGAGGACGAAGCTGATTACTATTGCAACA
 
GCAGGGATAGCTCCGGAAACCACGTGGTCTTTGGAGGAGGAACTAAGCTGACCGTGGGAG
GAGGAAGTGGCGGGGGATCAGGCGGCGGAAGCGGCGGCAGCGGAGGAGGATCTGGC
GAAGTGCAGCTGGTCGAATCTGGCGGAGGAGTGGTCCGGCCAGGAGGGAGTCTGAGACTG
TCATGTGCAGCCAGCGGCTTCACATTCGATGATTACGGAATGTCTTGGGTGCGGCAGGCAC
CTGGAAAGGGCCTGGAATGGGTGAGTGGCATCAACTGGAACGGCGGCAGTACCGGATACG
CTGACTCAGTGAAAGGCAGATTCACAATTTCTAGAGACAATGCTAAGAATAGTTTATATCT
GCAAATGAACAGCCTGAGAGCAGAGGACACTGCCGTGTACTATTGCGCCCGGGGGAGGTC
ACTGCTGTTCGATTACTGGGGGCAGGGCACTCTGGTCACTGTGTCAAGGTGAGGATCC
24. SEQ ID NO: 114 (Base construct # 10 protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
GSGGSGGSGGSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGLGGSGGSGGSGGSGGSSELTQDPAVSVALGQTVRITCQGDSLRSYYASWYQQKPGQAPV
LVIYGKNNRPSGIPDRFSGSSSGNTASLTITGAQAEDEADYYCNSRDSSGNHVVFGGGTKLTVG
GGSGGGSGGGSGGGSGGGSGEVQLVESGGGVVRPGGSLRLSCAASGFTFDDYGMSWVRQAP
GKGLEWVSGINWNGGSTGYADSVKGRFTISRDNAKNSLYLQMNSLRAEDTAVYYCARGRSLL
FDYWGQGTLVTVSR
25. SEQ ID NO: 115 (Base construct # 11) 
GATATTCAGATGACTCAGTCTCCTAGCTCCCTGTCAGCTAGCGTCGGCGATCGGGTGACAA
TCACTTGCAGAGCCAGCCAGGACGTCAACACAGCCGTGGCTTGGTACCAGCAGAAGCCCG
GAAAAGCACCTAAGCTGCTGATCTACTCCGCCTCTTTTCTGTATTCTGGCGTGCCCAGTAGA
TTCAGTGGATCAAGGAGCGGCACCGATTTTACCCTGACAATCTCTAGTCTGCAGCCTGAGG
ACTTTGCCACATACTATTGCCAGCAGCACTATACCACCCCCTACTTTCGGGCAGGGAAC
CAAGGTGGAAATCAAAGGCGGGTCAGGCGGAGGGAGCGGAGGAGGGTCCGGAGGAGGGT
CTGGAGGAGGGAGTGGAGAGGTGCAGCTGGTCGAATCTGGAGGAGGACTGGTGCAGCCAG
GAGGCTCACTGCGGCTGAGCTGTGCCGCTTCCGGCTTCAACATCAAAGATACCTACATTCA
TTGGGTCCGACAGGCACCAGGCAAGGGACTGGAGTGGGTGGCTAGAATCTATCCCACCAA
TGGCTACACACGATATGCCGATAGCGTGAAAGGCGGTTTACAATTTCTGCAGACACTAGT
AAGAACACCGCCTACCTGCAGATGAACAGCCTGCGCGCTGAGGACACTGCAGTGTACTAT
TGTAGTCGATGGGGGGGAGACGGCTTCTACGCCATGGATTATTGGGGACAGGGCACCCTG
GTGACAGTCTCAAGCGGAGGGTCCGGCGATGCACACAAGTCTGAGGTCGCTCATAGATTC
AAAGACCTGGGGGAGGAAAATTTTAAGGCCCTGGTGCTGATTGCATTCGCCCAGTACCTGC
AGCAGTGCCCCTTTGAAGACCACGTGAAACTGGTCAACGAGGTGACAGAGTTCGCCAAGA
CTTGCGTCGCCGACGAGAGTGCTGAAAATTGTGATAAATCACTGCATACACTGTTTGGGGA
TAAGCTGTGTACTGTGGCCACCCTGCGGGAGACTTATGGAGAAATGGCAGACTGCTGTGCC
AAACAGGAGCCTGAAAGAAACGAGTGCTTCCTGCAGCACAAGGACGATAACCCTAATCTG
CCAAGGCTGGTGCGCCCAGAAGTGGACGTCATGTGTACTGCCTTCCACGATAATGAGGAA
ACCTTTCTGAAGAAATACCTGTATGAGATCGCCCGGAGACATCCCTACTTTTATGCTCCTGA
ACTGCTGTTCTTTGCAAAACGGTACAAGGCAGCCTTCACCGAGTGCTGTCAGGCTGCAGAT
AAGGCCGCTTGCCTGCTGCCCAAACTGGACGAGCTGCGGGATGAAGGCAAGGCTTCCTCT
GCAAAGCAGAGACTGAAATGTGCAAGCCTGCAGAAGTTCGGGGAGAGGGCCTTTAAAGCT
TGGGCAGTCGCACGACTGAGCCAGCGATTCCCTAAGGCCGAGTTTGCTGAAGTCTCCAAGC
TGGTGACAGACCTGACTAAAGTGCACACCGAGTGCTGTCATGGCGACCTGCTGGAATGCG
CCGACGATCGCGCAGATCTGGCCAAGTACATCTGTGAGAACCAGGACAGCATTAGTTCAA
AGCTGAAAGAGTGCTGTGAAAAGCCACTGCTGGAGAAATCCCACTGCATTGCTGAGGTGG
AAAACGACGAAATGCCAGCAGATCTGCCCAGCCTGGCAGCCGACTTCGTCGAGTCCAAGG
ATGTGTGTAAAAATTATGCTGAAGCAAAGGATGTGTTCCTGGGCATGTTTCTGTACGAGTA
TGCCAGGGCTGGAGGCAGTGGAGGATCAGGAGGGAGCGGAGGCTCCGGAGGAGACATCC
 
AGATGACCCAGAGCCCAAGCTCCCTGTCCGCTTCTGTCGGCGATAGGGTGACTATTACCTG
CCGCGCCTCCCAGGACGTCAATACAGCAGTGGCCTGGTACCAGCAGAAACCTGGAAGGC
TCCAAAACTGCTGATCTACAGTGCATCATTCCTGTATTCAGGAGTGCCAAGCCGCTTTAGC
GGGTCCCGATCTGGAACTGATTTCACACTGACTATCTCTAGTCTGCAGCCCGAGGACTTTG
CCACCTATTACTGCCAGCAGCACTACACTACCCCACCCACCTTCGGGCAGGGAACAAAGGT
GGAAATCAAAGGGGGGTCCGGCGGCGGGTCTGGCGGAGGGAGTGGAGGAGGGTCAGGCG
GCGGGAGCGGCGAGGTCCAGCTGGTGGAATCCGGCGGCGGCCTGGTGCAGCCTGGAGGCT
CCCTGCGACTGTCTTGTGCTGCAAGTGGCTTTAACATCAAGGACACTTACATTCATTGGGTC
AGGCAGGCTCCTGGCAAGGGCCTGGAATGGGTGGCACGAATCTATCCAACAAATGGATAC
ACTAGGTACGCCGATAGCGTGAAAGGCAGGTTCACCATTTCAGCCGACACCAGCAAGAAC
ACAGCTTACCTGCAAATGAACAGCCTGAGGGCTGAGGACACAGCAGTGTACTATTGCAGC
CGCTGGGGCGGGGACGGGTTCTATGCTATGGACTATTGGGGGCAGGGCACTCTGGTCACTG
TGTCAAGCTGAGGATCC
26. SEQ ID NO: 116 (Base construct # 11 protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGDIQMTQSPSSLSASVGDRVTITCR
ASQDVNTAVAWYQQKPGKAPKLLIYSASFLYSGVPSRFSGSRSGTDFTLTISSLQPEDFATYYC
QQHYTTPPTFGQGTKVEIKGGSGGGSGGGSGGGSGGGSGEVQLVESGGGLVQPGGSLRLSCAA
SGFNIKDTYIHWVRQAPGKGLEWVARIYPTNGYTRYADSVKGRFTISADTSKNTAYLQMNSLR
AEDTAVYYCSRWGGDGFYAMDYWGQGTLVTVSS
27. SEQ ID NO: 117 (Base construct # 12) 
GACATTCAGATGACTCAGAGCCCAAGCTCCCTGAGCGCATCCGTGGGCGACAGAGTCACC
ATCACATGCAGGGCCTCCCAGGATGTGAACACCGCTGTCGCATGGTACCAGCAGAAACCT
GGGAAGGCTCCAAAACTGCTGATCTACTCTGCAAGTTTCCTGTATAGTGGAGTGCCATCAA
GGTTTTCAGGCAGCCGCTCCGGGACCGACTTCACTCTGACCATCTCTAGTCTGCAGCCCGA
GGATTTCGCCACATACTATTGCCAGCAGCACTATACCACACCCCCTACCTTTGGCCAGGGG
ACAAAAGTGGAAATTAAGGGAGGGAGCGGAGGAGGGTCCGGAGGAGGGTCTGGAGGCGG
GAGTGGAGGAGGGTCAGGAGAGGTGCAGCTGGTCGAATCCGGAGGAGGACTGGTGCAGC
CAGGAGGCAGCCTGCGGCTGTCCTGTGCCGCTTCTGGCTTCAACATCAAAGACACCTACAT
TCATTGGGTGCGCCAGGCTCCAGGAAAGGGACTGGAGTGGGTCGCACGAATCTATCCCAC
TAATGGGTACACCCGGTATGCCGATTCCGTGAAAGGAAGATTCACAATTAGTGCAGATACA
TCAAAGAACACTGCCTACCTGCAGATGAACAGCCTGCGAGCAGAGGATACTGCCGTGTAC
TATTGTAGTCGGTGGGGGGGAGACGGCTTTTACGCCATGGATTATTGGGGGCAGGGAACCC
TGGTGACAGTCTCAAGCGGAGGGTCAGGAGGCAGCGGAGGCAGCGGAGGGTCTGGAGGC
AGTGTGGTCCTGCTGCTGAGGCTGGCTAAAACCTACGAGACTACCCTGGAAAAGTGCTGTG
CAGCCGCTGACCCCCACGAGTGCTATGCCAAAGTGTTCGATGAGTTCAAGCCACTGGTCGA
GGAACCCCAGAACCTGATCAAACAGAATTGTGAGCTGTTCGAACAGCTGGGCGAGTACAA
GTTTCAGAACGCCCTGCTGGTGCGCTATACCAAGAAAGTGCCTCAGGTCTCTACACCAACT
CTGGTGGAGGTCAGTAGGAATCTGGGGAAAGTGGGATCAAAGTGCTGTAAACACCCCGAG
GCCAAGCGCATGCCTTGCGCTGAAGACTACCTGTCTGTGGTCCTGAACCAGCTGTGTGTGC
TGCATGAGAAAACCCCCGTGAGCGATCGGGTCACCAAGTGCTGTACAGAAAGCCTGGTGA
ACCGGAGACCCTGCTTCTCCGCTCTGGAGGTGGACGAAACATATGTCCCTAAGGAGTTTAA
TGCTGAAACCTTCACATTTCACGCAGATATCTGTACACTGTCCGAGAAGGAAAGACAGATT
AAGAAACAGACTGCCCTGGTGGAGCTGGTCAAGCATAAACCTAAGGCCACAAAAGAACAG
CTGAAGGCTGTGATGGACGATTTCGCAGCCTTTGTCGAGAAGTGCTGTAAAGCCGACGATA
AGGAAACTTGCTTCGCTGAGGAAGGAAAGAAACTGGTGGCTGCAAGCCAGGCAGCTCTGG
GCCTGGGAGGATCAGGAGGGAGCGGAGGCTCCGGAGGATCTGGAGGGGACATCCAGATG
ACCCAGTCTCCTTCCTCTCTGTCTGCTAGTGTGGGCGACCGCGTCACTATTACCTGTCGAGC
CAGCCAGGATGTGAATACAGCCGTCGCTTGGTACCAGCAGAAGCCCGGCAAAGCACCTAA
GCTGCTGATCTACTCAGCCAGCTTTCTGTATAGCGGGGTGCCTTCCCGATTCTCCGGATCTC
GGAGTGGCACTGACTTTACACTGACTATCAGTTCACTGCAGCCAGAGGATTTCGCCACCTA
 
TTACTGCCAGCAGCACTACACAACTCCACCCACTTTTGGCCAGGGGACCAAAGTGGAAATC
AAGGGAGGCTCTGGAGGAGGCAGTGGAGGAGGCTCAGGAGGAGGCAGCGGAGGAGGCTC
CGGCGAAGTGCAGCTGGTCGAATCTGGCGGCCTGGTCCAGCCAGGAGGATCTCTGAG
GCTGAGTTGTGCAGCCTCAGGCTTCAACATCAAGGATACTTACATTCATTGGGTGCGGCAG
GCACCTGGAAAGGGCCTGGAATGGGTCGCTAGAATCTATCCAACTAATGGCTACACCAGA
TATGCCGACAGCGTGAAAGGGCGCTTTACCATTAGCGCAGATACATCCAAAAATACCGCTT
ACCTGCAGATGAATAGCCTGAGAGCTGAGGATACAGCAGTGTACTATTGCTCCAGATGGG
GCGGCGATGGGTTTTACGCAATGGACTACTGGGGACAGGGAACACTGGTCACCGTCTCTTC
TTGAGGATCC
28. SEQ ID NO: 118 (Base construct # 12 protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
GSGGSGGSGGSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGLGGSGGSGGSGGSGGDIQMTQSPSSLSASVGDRVTITCRASQDVNTAVAWYQQKPGKAP
KLLIYSASFLYSGVPSRFSGSRSGTDFTLTISSLQPEDFATYYCQQHYTTPPTFGQGTKVEIKGGS
GGGSGGGSGGGSGGGSGEVQLVESGGGLVQPGGSLRLSCAASGFNIKDTYIHWVRQAPGKGL
EWVARIYPTNGYTRYADSVKGRFTISADTSKNTAYLQMNSLRAEDTAVYYCSRWGGDGFYA
MDYWGQGTLVTVSS
29. SEQ ID NO: 119 (Base construct # 13) 
AGTTCTGAGCTGACCCAGGACCCCGCTGTGAGCGTCGCACTGGGACAGACAGTGCGGATC
ACTTGCCAGGGCGACAGCCTGAGATCCTACTATGCTAGCTGGTACCAGCAGAAGCCTGGCC
AGGCACCAGTGCTGGTCATCTATGGAAAAAACAATAGACCCAGCGGCATTCCTGATAGGTT
CTCCGGGAGCTCCTCTGGAAACACAGCTAGCCTGACTATTACCGGCGCCCAGGCTGAGGAC
GAAGCCGATTACTATTGCAACAGCAGGGACAGTTCAGGGAATCACGTGGTCTTTGGAGGA
GGAACTAAGCTGACCGTGGGAGGAGGCAGCGGAGGAGGATCTGGAGGAGGAAGTGGAGG
AGGATCAGGAGGAGGAAGCGGAGAGGTGCAGCTGGTCGAAAGCGGAGGAGGAGTGGTCA
GGCCAGGAGGGTCCCTGCGACTGTCTTGTGCCGCTAGTGGGTTCACTTTTGACGATTACGG
AATGAGTTGGGTCAGGCAGGCACCAGGAAAGGGACTGGAGTGGGTGAGCGGCATCAACTG
GAATGGAGGCAGTACAGGCTACGCTGATTCAGTGAAGGGGCGCTTCACTATTTCTCGAGAC
AACGCCAAAAATAGTCTGTATCTGCAGATGAACTCACTGCGCGCCGAGGATACAGCTGTGT
ACTATTGCGCCAGGGGCCGCTCCCTGCTGTTTGACTACTGGGGGCAGGGAACACTGGTGAC
TGTCTCACGGGGGGGAAGCGGAGATGCACACAAATCTGAGGTCGCCCATAGATTCAAGGA
CCTGGGCGAGGAAAATTTTAAAGCCCTGGTGCTGATCGCATTCGCCCAGTATCTGCAGCAG
TGCCCTTTCGAAGACCACGTGAAGCTGGTCAACGAGGTGACAGAATTTGCCAAAACTTGCG
TCGCAGACGAGAGCGCCGAAAATTGTGATAAGTCCCTGCATACCCTGTTCGGCGATAAACT
GTGTACCGTGGCCACACTGAGGGAGACATACGGGGAAATGGCTGACTGCTGTGCAAAGCA
GGAGCCCGAACGCAACGAGTGCTTTCTGCAGCACAAAGACGATAACCCAAATCTGCCCCG
ACTGGTGCGGCCTGAAGTGGACGTCATGTGTACTGCCTTCCACGATAATGAGGAAACCTTT
CTGAAGAAATACCTGTATGAGATTGCCCGGAGACATCCCTACTTCTATGCTCCTGAACTGC
TGTTCTTTGCAAAGCGGTACAAAGCAGCCTTTACCGAGTGCTGTCAGGCTGCAGATAAAGC
CGCTTGCCTGCTGCCTAAGCTGGACGAGCTGAGGGATGAAGGCAAGGCTAGCTCCGCAAA
ACAGCGCCTGAAGTGTGCTAGCCTGCAGAAATTCGGCGAGCGGGCCTTCAAGGCTTGGGC
AGTGGCCAGACTGTCACAGAGGTTCCCAAAGGCCGAGTTTGCTGAAGTCAGCAAACTGGT
GACTGACCTGACCAAGGTGCACACCGAGTGCTGTCATGGCGACCTGCTGGAATGCGCCGA
CGATAGAGCAGATCTGGCCAAGTACATCTGTGAGAACCAGGACTCCATTTCTAGTAAGCTG
AAAGAGTGCTGTGAAAAACCCCTGCTGGAGAAGTCTCATTGCATCGCCGAGGTGGAAAAC
GACGAAATGCCAGCTGATCTGCCCTCTCTGGCAGCCGACTTCGTCGAGAGTAAAGATGTGT
GTAAGAATTATGCTGAAGCAAAGGATGTGTTCCTGGGCATGTTTCTGTACGAGTATGCACG
AGCTGGAGGGTCTGGAGGCAGTGGAGGATCAGGAGGGAGCGGAGGCGACATCCAGATGA
 
CCCAGTCCCCTTCAAGCCTGAGTGCTTCAGTCGGCGATCGAGTGACAATTACTTGCCGGGC
CTCTCAGGACGTCAATACAGCAGTGGCTTGGTATCAGCAGAAGCCTGGAAAGCACCAAA
GCTGCTGATCTACAGCGCCTCCTTTCTGTATTCCGGAGTGCCTTCTCGGTTCTCTGGCAGTA
GATCAGGGACTGATTTTACCCTGACAATTTCCTCTCTGCAGCCAGAGGACTTCGCCACCTA
CTATTGCCAGCAGCACTATACCACACCCCCTACCTTTGGCCAGGGGACAAAAGTGGAAATC
AAGGGGGGAAGTGGCGGGGGATCAGGCGGCGGAAGCGGCGGCGGCAGCGGCGGCGGATC
TGGAGAGGTCCAGCTGGTGGAAAGCGGAGGAGGACTGGTGCAGCCAGGAGGGAGTCTGA
GACTGTCATGTGCTGCAAGCGGCTTCAACATCAAGGATACCTACATTCACTGGGTCAGGCA
GGCCCCAGGAAAAGGCCTGGAGTGGGTGGCCCGCATCTATCCCACCAATGGGTACACACG
CTATGCCGATTCCGTGAAGGGACGATTCACAATTTCCGCCGACACTTCTAAAAACACCGCT
TACCTGCAGATGAACAGCCTGCGAGCCGAGGACACTGCTGTGTACTATTGTTCTAGATGGG
GCGGGGACGGGTTTTACGCAATGGACTACTGGGGGCAGGGGACTCTGGTCACTGTCAGCA
GCTGAGGATCC
30. SEQ ID NO: 120 (Base construct # 13 protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGDIQMTQSPSSLSASVGDRVTITCR
ASQDVNTAVAWYQQKPGKAPKLLIYSASFLYSGVPSRFSGSRSGTDFTLTISSLQPEDFATYYC
QQHYTTPPTFGQGTKVEIKGGSGGGSGGGSGGGSGGGSGEVQLVESGGGLVQPGGSLRLSCAA
SGFNIKDTYIHWVRQAPGKGLEWVARIYPTNGYTRYADSVKGRFTISADTSKNTAYLQMNSLR
AEDTAVYYCSRWGGDGFYAMDYWGQGTLVTVSS
31. SEQ ID NO: 121 (Base construct # 14) 
GACATTCAGATGACCCAGTCCCCAAGCTCCCTGTCTGCTAGTGTCGGCGATCGGGTGACTA
TCACCTGCAGAGCCTCTCAGGACGTCAACACAGCCGTGGCTTGGTACCAGCAGAAGCCTG
GCAAAGCACCAAAGCTGCTGATCTACTCAGCCAGCTTTCTGTATAGCGGGGTGCCTTCCAG
ATTCTCCGGCTCTAGGAGTGGGACTGATTTTACACTGACTATCTCTAGTCTGCAGCCAGAG
GACTTCGCCACCTACTATTGCCAGCAGCACTATACCACACCCCCTACATTTGGGCAGGGAA
CTAAAGTGGAAATTAAGGGAGGGTCTGGAGGAGGGAGTGGAGGAGGGTCAGGCGGAGGG
AGCGGAGGAGGGTCCGGCGAGGTGCAGCTGGTCGAAAGCGGAGGAGGACTGGTGCAGCC
TGGAGGCTCTCTGAGGCTGAGTTGTGCCGCTTCAGGCTTCAACATCAAGGATACCTACATT
CATTGGGTCCGACAGGCTCCAGGCAAAGGGCTGGAGTGGGTGGCAAGAATCTATCCCACA
AATGGCTACACTAGATATGCCGATAGCGTGAAGGGGAGGTTCACAATTAGCGCTGACACC
TCCAAAAACACAGCATACCTGCAGATGAATAGTCTGCGGGCTGAGGACACTGCAGTGTAC
TATTGTAGCAGATGGGGGGGAGACGGCTTTTACGCCATGGATTATTGGGGACAGGGCACTC
TGGTGACCGTCTCAAGCGGAGGAGCGGGGATGCACACAAATCCGAGGTCGCCCATCGCT
TCAAGGACCTGGGAGAGGAAAATTTTAAAGCCCTGGTGCTGATTGCATTCGCCCAGTACCT
GCAGCAGTGCCCCTTCGAAGACCACGTGAAGCTGGTCAACGAGGTGACCGAATTTGCCAA
AACATGCGTCGCCGACGAGTCAGCTGAAAATTGTGATAAGAGCCTGCATACCCTGTTCGGA
GATAAACTGTGTACAGTGGCCACTCTGAGGGAGACATATGGCGAAATGGCAGACTGCTGT
GCCAAGCAGGAGCCCGAACGCAACGAGTGCTTTCTGCAGCACAAAGACGATAACCCAAAT
CTGCCCAGGCTGGTGCGCCCTGAAGTGGACGTCATGTGTACTGCCTTCCACGATAATGAGG
AAACCTTTCTGAAGAAATACCTGTATGAGATCGCCCGGAGACATCCCTACTTCTATGCCCC
TGAACTGCTGTTCTTTGCTAAACGGTACAAGGCAGCCTTTACCGAGTGCTGTCAGGCTGCA
GATAAAGCCGCTTGCCTGCTGCCTAAGCTGGACGAGCTGAGGGATGAAGGAAAGGCTTCC
TCTGCAAAACAGCGCCTGAAGTGTGCCTCCCTGCAGAAATTCGGCGAGCGGGCTTTTAAGG
CTTGGGCAGTGGCACGACTGTCCCAGCGATTCCCAAAGGCCGAGTTTGCTGAAGTCTCTAA
ACTGGTGACCGACCTGACAAAGGTGCACACCGAGTGCTGTCATGGCGACCTGCTGGAATG
CGCCGACGATAGAGCAGATCTGGCCAAGTACATCTGTGAGAACCAGGACTCCATTAGTTC
AAAGCTGAAAGAGTGCTGTGAAAAACCCCTGCTGGAGAAGTCTCACTGCATCGCAGAGGT
GGAAAACGACGAAATGCCAGCAGATCTGCCTTCCCTGGCAGCAGACTTCGTCGAGTCTAA
AGATGTGTGTAAGAATTATGCTGAAGCAAAGGATGTGTTCCTGGGCATGTTTCTGTACGAG
TATGCACGAGCTGGAGGCTCAGGAGGAAGCGGAGGGTCCGGAGGCTCTGGGGGAAGCTCC
 
GAACTGACCCAGGACCCCGCTGTGAGCGTCGCACTGGGACAGACTGTGCGCATTACCTGCC
AGGGAGACAGTCTGCGATCATACTATGCTTCCTGGTACCAGCAGAAGCCAGGCCAGGCAC
CCGTGCTGGTCATCTATGGAAAAAACAATCGACCTTCCGGCATCCCCGATCGGTTCTCTGG
ATCTAGTTCAGGCAACACAGCTAGCCTGACCATCACAGGGGCACAGGCCGAGGACGAAGC
CGATTACTATTGCAACAGCAGAGACAGCTCCGGCAATCATGTGGTCTTTGGAGGAGGAACT
AAGCTGACCGTGGGAGGAGGATCTGGAGGAGGAAGTGGCGGGGGATCAGGAGGAGGAAG
CGGAGGAGGCAGCGGAGAGGTCCAGCTGGTGGAAAGCGGAGGAGGAGTGGTCAGGCCAG
GAGGGTCTCTGCGACTGAGTTGTGCTGCATCAGGCTTCACTTTTGACGATTACGGAATGAG
CTGGGTCAGGCAGGCACCAGGGAAGGGACTGGAGTGGGTGAGCGGCATCAACTGGAATG
GAGGCTCTACAGGATACGCTGATAGTGTGAAGGGCCGCTTCACTATTAGTCGAGACAACGC
CAAAAATTCACTGTATCTGCAGATGAATAGCCTGCGCGCCGAGGACACAGCTGTGTACTAT
TGCGCCAGAGGAAGGTCACTGCTGTTTGATTATTGGGGGCAGGGCACACTGGTCACCGTCT
CCCGCTGAGGATCC
32. SEQ ID NO: 122 (Base construct # 14 protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARAGGSGGSGGSGGSGGSSELTQDPAVSVALGQTVRITCQG
DSLRSYYASWYQQKPGQAPVLVIYGKNNRPSGIPDRFSGSSSGNTASLTITGAQAEDEADYYC
NSRDSSGNHVVFGGGTKLTVGGGSGGGSGGGSGGGSGGGSGEVQLVESGGGVVRPGGSLRLS
CAASGFTFDDYGMSWVRQAPGKGLEWVSGINWNGGSTGYADSVKGRFTISRDNAKNSLYLQ
MNSLRAEDTAVYYCARGRSLLFDYWGQGTLVTVSR
33. SEQ ID NO: 123 (Base construct # 15) 
TCTTCAGAACTGACCCAGGACCCCGCAGTGAGCGTCGCACTGGGCCAGACCGTGAGAATC
ACATGCCAGGGGGATTCCCTGAGGTCTTACTATGCTAGCTGGTACCAGCAGAAGCCAGGCC
AGGCACCCGTGCTGGTCATCTATGGCAAAAACAATAGGCCTTCAGGGATTCCAGACCGCTT
TAGCGGAAGCTCCTCTGGCAACACAGCAAGCCTGACAATTACTGGCGCTCAGGCAGAGGA
CGAAGCCGATTACTATTGCAACAGCAGGGATAGTTCAGGCAATCACGTGGTCTTCGGAGG
AGGAACTAAGCTGACCGTGGGAGGAGGATCTGGAGGAGGAAGTGGCGGGGGATCAGGAG
GAGGAAGCGGAGGAGGCAGCGGAGAGGTGCAGCTGGTCGAAAGCGGAGGAGGAGTGGTC
CGCCCAGGAGGGTCTCTGCGACTGAGTTGTGCCGCTTCAGGATTCACCTTTGACGATTACG
GAATGTCCTGGGTGAGGCAGGCACCAGGGAAGGGACTGGAGTGGGTCTCTGGCATCAACT
GGAATGGAGGCTCTACAGGGTACGCTGACAGTGTGAAGGGACGGTTCACCATTTCCCGGG
ATAACGCCAAAAATTCTCTGTATCTGCAGATGAATAGTCTGCGCGCTGAGGACACCGCAGT
GTACTATTGTGCCAGGGGCCGCAGTCTGCTGTTCGATTACTGGGGCCAGGGAACACTGGTG
ACTGTCAGCCGAGGAGGAAGTGGAGGGTCAGGAGGCAGCGGAGGCAGCGGAGGGTCTGT
GGTCCTGCTGCTGAGACTGGCTAAGACATACGAGACCACACTGGAAAAATGCTGTGCAGC
CGCTGACCCCCATGAGTGCTATGCCAAGGTGTTCGATGAGTTCAAGCCACTGGTCGAGGAA
CCCCAGAACCTGATCAAGCAGAATTGTGAGCTGTTCGAACAGCTGGGCGAGTACAAATTTC
AGAACGCCCTGCTGGTGCGCTATACCAAGAAAGTGCCTCAGGTCTCAACCCCAACACTGGT
GGAGGTCAGCAGGAATCTGGGCAAGGTCGGGTCCAAATGCTGTAAGCACCCCGAGGCAAA
ACGCATGCCTTGCGCCGAAGACTACCTGTCCGTGGTCCTGAACCAGCTGTGTGTGCTGCAT
GAGAAGACACCTGTGTCTGATCGGGTCACTAAATGCTGTACCGAATCTCTGGTGAACCGGA
GACCTTGCTTTAGTGCCCTGGAGGTGGACGAAACTTATGTCCCAAAGGAGTTCAATGCTGA
AACTTTCACCTTTCACGCAGATATCTGTACCCTGAGCGAGAAGGAAAGACAGATTAAGAA
ACAGACAGCCCTGGTGGAGCTGGTCAAGCATAAACCAAAGGCCACCAAGGAACAGCTGAA
AGCTGTGATGGACGATTTCGCAGCCTTTGTCGAGAAATGCTGTAAGGCTGACGATAAGGAA
ACATGCTTCGCAGAGGAAGGGAAGAAACTGGTGGCTGCATCCCAGGCAGCTCTGGGACTG
GGAGGCAGTGGAGGATCAGGAGGGAGCGGAGGCTCCGGAGGAGACATCCAGATGACTCA
GTCCCCAAGCTCCCTGTCAGCAAGCGTGGGCGACCGGGTCACAATTACTTGTAGAGCTTCT
CAGGATGTGAATACCGCCGTCGCTTGGTACCAGCAGAAACCCGGCAAGGCCCCTAAACTG
CTGATCTACTCCGCTTCTTTCCTGTATAGCGGAGTGCCATCCCGGTTCAGCGGGTCAAGGA
GCGGAACTGACTTCACCCTGACAATTTCTAGTCTGCAGCCTGAGGATTTTGCCACCTACTAT
 
TGCCAGCAGCACTATACTACCCCCCTACTTTCGGACAGGGCACCAAGGTGGAAATCAAA
GGAGGGTCTGGAGGAGGGAGTGGAGGAGGGTCAGGCGGAGGGAGCGGAGGAGGGTCCGG
CGAAGTCCAGCTGGTCGAATCCGGAGGAGGACTGGTGCAGCCTGGAGGCTCTCTGAGGCT
GAGTTGTGCAGCCTCAGGCTTTAACATCAAGGACACCTACATTCATTGGGTGCGGCAGGCA
CCAGGGAAAGGACTGGAGTGGGTGGCCAGAATCTATCCCACAAATGGATACACTCGATAT
GCCGACTCTGTGAAGGGCCGGTTCACAATTAGCGCAGATACCTCCAAAAACACAGCCTAC
CTGCAGATGAACAGCCTGCGCGCCGAGGATACTGCTGTGTACTATTGCAGCCGATGGGGC
GGGGACGGCTTCTACGCTATGGACTATTGGGGGCAGGGGACTCTGGTGACAGTGAGCAGC
TGAGGATCC
34. SEQ ID NO: 124 (Base construct # 15 protein) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
GSGGSGGSGGSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGLGGSGGSGGSGGSGGDIQMTQSPSSLSASVGDRVTITCRASQDVNTAVAWYQQKPGKAP
KLLIYSASFLYSGVPSRFSGSRSGTDFTLTISSLQPEDFATYYCQQHYTTPPTFGQGTKVEIKGGS
GGGSGGGSGGGSGGGSGEVQLVESGGGLVQPGGSLRLSCAASGFNIKDTYIHWVRQAPGKGL
EWVARIYPTNGYTRYADSVKGRFTISADTSKNTAYLQMNSLRAEDTAVYYCSRWGGDGFYA
MDYWGQGTLVTVSS
35. SEQ ID NO: 125 (Base construct # 16) 
GATATTCAGATGACCCAGAGCCCAAGCTCCCTGAGTGCATCAGTGGGCGACAGAGTCACA
ATCACTTGCAGGGCTAGCCAGGATGTGAACACAGCTGTCGCATGGTACCAGCAGAAACCA
GGCAAGGCTCCCAAACTGCTGATCTACAGCGCATCCTTCCTGTATTCCGGCGTGCCCTCTA
GGTTTTCTGGGAGTCGCTCAGGAACTGACTTCACCCTGACAATCTCTAGTCTGCAGCCTGA
GGATTTTGCCACCTACTATTGCCAGCAGCACTACACCACACCCCCTACTTTCGGCCAGGGG
ACCAAGGTGGAGATCAAGGGCGGAGTGGAGGCGGTCAGGCGGAGGGAGCGGAGGAGG
GTCCGGAGGAGGGTCTGGCGAGGTGCAGCTGGTCGAAAGCGGAGGAGGACTGGTGCAGCC
TGGAGGCAGTCTGCGGCTGTCATGTGCCGCTAGCGGCTTCAACATCAAGGACACCTACATT
CATTGGGTGCGCCAGGCACCAGGAAAAGGCCTGGAGTGGGTCGCCCGAATCTATCCCACC
AATGGGTACACAAGATATGCCGACTCCGTGAAGGGACGCTTTACAATTTCCGCTGATACTT
CTAAAAACACCGCATACCTGCAGATGAATAGTCTGAGAGCAGAGGATACTGCCGTGTACT
ATTGTAGCAGATGGGGGGGAGACGGCTTCTACGCCATGGACTACTGGGGCCAGGGCACTC
TGGTGACCGTCTCAAGCGGAGGGAGCGGAGGCTCCGGAGGATCTGGAGGGAGTGGAGGCT
CAGTGGTCCTGCTGCTGAGGCTGGCTAAGACCTACGAGACTACCCTGGAAAAATGCTGTGC
AGCCGCTGACCCCCACGAGTGCTATGCCAAGGTGTTCGATGAGTTCAAGCCACTGGTCGAG
GAACCCCAGAACCTGATCAAGCAGAATTGTGAGCTGTTCGAACAGCTGGGCGAGTACAAA
TTTCAGAACGCCCTGCTGGTGCGCTATACAAAGAAAGTGCCTCAGGTCAGTACTCCAACCC
TGGTGGAAGTCTCACGGAATCTGGGAAAGGTCGGCAGCAAGTGCTGTAAACACCCCGAGG
CAAAAAGAATGCCTTGCGCCGAAGACTACCTGAGCGTGGTCCTGAATCAGCTGTGTGTGCT
GCATGAGAAGACACCTGTGAGCGATAGGGTCACAAAATGCTGTACTGAATCCCTGGTGAA
CCGGAGACCTTGCTTTTCTGCTCTGGAGGTGGACGAAACTTATGTCCCAAAGGAGTTCAAT
GCCGAAACATTCACTTTTCACGCTGATATCTGTACCCTGAGCGAGAAGGAACGCCAGATTA
AGAAACAGACAGCCCTGGTGGAGCTGGTCAAGCATAAACCAAAGGCAACTAAGGAACAG
CTGAAAGCCGTGATGGACGATTTCGCAGCCTTTGTCGAGAAGTGCTGTAAAGCCGACGATA
AGGAAACCTGCTTTGCTGAGGAAGGCAAGAAACTGGTGGCTGCAAGCCAGGCAGCTCTGG
GACTGGGAGGAAGCGGAGGGTCCGGAGGCTCTGGGGGAAGTGGAGGGTCCTCTGAGCTGA
CCCAGGACCCCGCTGTGTCCGTCGCACTGGGACAGACCGTGCGAATTACATGTCAGGGCG
ATTCACTGCGGAGCTACTATGCTTCTTGGTACCAGCAGAAGCCTGGCCAGGCACCAGTGCT
GGTCATCTATGGAAAAAACAATCGGCCCAGTGGCATTCCTGACAGATTTTCAGGCAGTTCA
AGCGGGAACACCGCATCCCTGACCATCACAGGCGCCCAGGCTGAGGACGAAGCCGATTAC
 
TATTGCAACTCTAGGGATTCCTCTGGCAATCATGTGGTCTTCGGAGGCGGACAAAGCTGA
CTGTGGGAGGAGGGAGTGGCGGAGGGTCAGGCGGCGGAGCGGCGGGTCCGGCGGC
GGGTCTGGAGAAGTGCAGCTGGTCGAATCCGGAGGAGGAGTGGTCCGCCCAGGAGGCAGT
CTGCGACTGTCATGTGCAGCCAGCGGGTTCACCTTTGACGATTACGGAATGTCCTGGGTGC
GGCAGGCACCAGGCAAGGGACTGGAGTGGGTGTCTGGCATCAACTGGAATGGGGGCAGCA
CAGGCTACGCTGACTCTGTGAAGGGGCGATTCACTATTAGCCGGGATAACGCCAAAAATTC
CCTGTATCTGCAGATGAACAGCCTGAGAGCCGAGGACACAGCTGTGTACTATTGCGCCAG
GGGGCGGTCACTGCTGTTTGATTATTGGGGGCAGGGAACTCTGGTCACTGTCTCTAGGTGA
GGATCC
36. SEQ ID NO: 126 (Base construct # 16 protein) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
GSGGSGGSGGSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLSVV
LNQLCVLHEKTPVSDRVTKCCTESLVNRRPCFSALEVDETYVPKEFNAETFTFHADICTLSEKE
RQIKKQTALVELVKHKPKATKEQLKAVMDDFAAFVEKCCKADDKETCFAEEGKKLVAASQA
ALGLGGSGGSGGSGGSGGSSELTQDPAVSVALGQTVRITCQGDSLRSYYASWYQQKPGQAPV
LVIYGKNNRPSGIPDRFSGSSSGNTASLTITGAQAEDEADYYCNSRDSSGNHVVFGGGTKLTVG
GGSGGGSGGGSGGGSGGGSGEVQLVESGGGVVRPGGSLRLSCAASGFTFDDYGMSWVRQAP
GKGLEWVSGINWNGGSTGYADSVKGRFTISRDNAKNSLYLQMNSLRAEDTAVYYCARGRSLL
FDYWGQGTLVTVSR
37. SEQ ID NO: 127 (Sequence for v593) 
GACATTCAGATGACACAGAGCCCAAGCTCCCTGTCTGCAAGTGTCGGCGATCGAGTGACA
ATCACTTGCCGGGCTTCCCAGGACGTCAACACTGCCGTGGCTTGGTACCAGCAGAAACCTG
GGAAGGCCCCAAAACTGCTGATCTACTCAGCTAGCTTTCTGTATAGCGGAGTGCCCTCCCG
GTTCTCCGGATCTAGAAGTGGCACCGATTTTACCCTGACAATCTCTAGTCTGCAGCCTGAG
GACTTCGCCACATACTATTGCCAGCAGCACTATACCACACCCCCTACCTTTGGGCAGGGAA
CAAAGGTGGAAATCAAAGGAGGGTCTGGAGGAGGGAGTGGAGGAGGGTCAGGCGGAGGG
AGCGGAGGAGGGTCCGGCGAGGTGCAGCTGGTCGAAAGCGGAGGAGGACTGGTGCAGCC
TGGAGGCTCTCTGAGGCTGAGTTGTGCCGCTTCAGGCTTCAACATCAAAGATACCTACATT
CATTGGGTCCGCCAGGCTCCAGGCAAGGGACTGGAGTGGGTGGCACGAATCTATCCCACA
AATGGATACACTCGGTATGCCGATTCCGTGAAAGGCAGATTCACTATTAGCGCTGACACCT
CCAAGAACACAGCATACCTGCAGATGAATAGTCTGCGAGCAGAGGACACCGCCGTGTACT
ATTGCTCACGGTGGGGGGGAGACGGCTTTTACGCCATGGATTATTGGGGACAGGGCACTCT
GGTGACCGTCTCAAGCGGAGGGAGCGGAGATGCACACAAGTCCGAGGTCGCTCATCGCTT
CAAAGACCTGGGCGAGGAAAACTTTAAGGCCCTGGTGCTGATTGCATTCGCCCAGTACCTG
CAGCAGTGCCCATTCGAGGACCACGTGAAACTGGTCAACGAAGTGACTGAATTTGCCAAG
ACCTGCGTGGCTGACGAGTCAGCAGAAAATTGTGATAAAAGCCTGCATACACTGTTCGGCG
ATAAGCTGTGTACAGTGGCCACTCTGAGGGAGACTTATGGGGAAATGGCCGACTGCTGTGC
TAAACAGGAGCCAGAACGCAACGAGTGCTTTCTGCAGCACAAGGACGATAACCCAAATCT
GCCCAGACTGGTGAGGCCCGAAGTGGACGTCATGTGTACAGCCTTCCACGATAATGAGGA
AACTTTTCTGAAGAAATACCTGTATGAGATCGCTCGGAGACATCCCTACTTCTATGCCCCTG
AACTGCTGTTCTTTGCTAAGAGGTACAAAGCAGCCTTTACCGAGTGCTGTCAGGCTGCAGA
TAAGGCCGCTTGCCTGCTGCCAAAACTGGACGAGCTGAGAGATGAAGGCAAGGCATCCTC
TGCCAAGCAGAGGCTGAAATGTGCCTCCCTGCAGAAGTTCGGGGAGAGGGCTTTTAAAGC
TTGGGCAGTGGCACGACTGAGCCAGCGATTCCCAAAGGCTGAGTTTGCAGAAGTCTCCAA
GCTGGTGACCGACCTGACAAAAGTGCACACCGAGTGCTGTCATGGCGACCTGCTGGAATG
CGCCGACGATCGCGCCGATCTGGCTAAGTACATCTGTGAGAACCAGGACAGCATTAGTTCA
AAGCTGAAAGAGTGCTGTGAAAAGCCTCTGCTGGAGAAATCCCACTGCATTGCAGAGGTG
GAAAACGACGAAATGCCAGCAGATCTGCCTTCCCTGGCAGCAGACTTCGTCGAGTCTAAG
GATGTGTGTAAAAATTACGCTGAAGCAAAGGATGTGTTCCTGGGCATGTTTCTGTACGAGT
ATGCCAGGCGCCACCCTGACTACAGCGTGGTCCTGCTGCTGCGGCTGGCTAAAACCTATGA
 
GACTACCCTGGAAAAGTGCTGTGCTGCAGCCGATCCACATGAGTGCTATGCCAAGGTCTTC
GACGAGTTCAAGCCACTGGTGGAGGAACCCCAGAACCTGATCAAACAGAATTGTGAGCTG
TTTGAACAGCTGGGCGAGTACAAGTTCCAGAACGCCCTGCTGGTGAGATATACAAAGAAA
GTCCCTCAGGTGAGTACTCCAACCCTGGTGGAAGTCTCACGGAATCTGGGCAAAGTGGGG
AGCAAGTGCTGTAAACACCCCGAGGCAAAGAGAATGCCTTGCGCCGAAGATTACCTGTCT
GTGGTCCTGAATCAGCTGTGTGTGCTGCATGAGAAAACTCCTGTCAGCGACCGGGTGACTA
AGTGCTGTACCGAATCCCTGGTGAACCGACGGCCTTGCTTCTCTGCCCTGGAGGTCGATGA
AACATATGTGCCAAAGGAGTTTAATGCAGAAACATTCACTTTTCACGCCGACATCTGTACT
CTGAGCGAGAAGGAAAGACAGATTAAGAAACAGACCGCCCTGGTCGAGCTGGTGAAGCAT
AAACCAAAGGCTACCAAGGAACAGCTGAAAGCAGTCATGGACGATTTCGCTGCATTTGTG
GAGAAGTGCTGTAAAGCAGACGATAAGGAAACATGCTTCGCCGAGGAAGGGAAGAAACT
GGTGGCAGCTAGCCAGGCAGCACTGGGACTGGGAGGCTCAGGAGGAAGCGGAGGGTCCG
GAGGCTCTGGAGGAAGCTCCGAGCTGACCCAGGACCCCGCAGTGTCTGTCGCACTGGGAC
AGACAGTGAGGATTACTTGTCAGGGGGACAGTCTGCGCTCATACTATGCTAGCTGGTACCA
GCAGAAACCAGGCCAGGCACCCGTGCTGGTCATCTATGGCAAGAACAATCGCCCTTCCGG
GATTCCAGATCGATTCTCTGGGTCTAGTTCAGGAAACACCGCATCTCTGACCATCACAGGC
GCCCAGGCTGAGGACGAAGCTGATTACTATTGCAACAGCAGAGACAGCTCCGGCAATCAC
GTGGTCTTTGGAGGAGGAACTAAGCTGACCGTGGGAGGAGGATCTGGAGGAGGAAGTGGC
GGGGGATCAGGAGGAGGAAGCGGAGGAGGCAGCGGAGAGGTCCAGCTGGTGGAAAGCGG
AGGAGGCGTGGTCAGACCAGGAGGGTCTCTGAGACTGTCCTGTGCTGCATCAGGATTCACC
TTTGACGATTACGGCATGTCTTGGGTCAGGCAGGCACCTGGGAAGGGCCTGGAATGGGTG
AGTGGCATCAACTGGAATGGAGGCTCTACCGGGTACGCCGATAGTGTGAAAGGAAGGTTC
ACAATTAGTCGCGACAACGCTAAGAACAGCCTGTATCTGCAGATGAATAGCCTGCGCGCT
GAGGACACAGCAGTGTACTATTGCGCCAGGGGGAGGTCACTGCTGTTTGATTATTGGGGGC
AGGGAACTCTGGTCACTGTGTCACGGTGAGGATCC
38. SEQ ID NO: 128 (Protein Sequence for v593) 
DIQMTQSPSSLSASVGDRVTITCRASQDVNTAVAWYQQKPGKAPKLLIYSASFLYSGVPSRFSG
SRSGTDFTLTISSLQPEDFATYYCQQHYTTPPTFGQGTKVEIKGGSGGGSGGGSGGGSGGGSGE
VQLVESGGGLVQPGGSLRLSCAASGFNIKDTYIHWVRQAPGKGLEWVARIYPTNGYTRYADS
VKGRFTISADTSKNTAYLQMNSLRAEDTAVYYCSRWGGDGFYAMDYWGQGTLVTVSSGGSG
 
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARRHPDYSVVLLLRLAKTYETTLEKCCAAADPHECYAKVF
DEFKPLVEEPQNLIKQNCELFEQLGEYKFQNALLVRYTKKVPQVSTPTLVEVSRNLGKVGSKC
CKHPEAKRMPCAEDYLSVVLNQLCVLHEKTPVSDRVTKCCTESLVNRRPCFSALEVDETYVPK
EFNAETFTFHADICTLSEKERQIKKQTALVELVKHKPKATKEQLKAVMDDFAAFVEKCCKADD
KETCFAEEGKKLVAASQAALGLGGSGGSGGSGGSGGSSELTQDPAVSVALGQTVRITCQGDSL
RSYYASWYQQKPGQAPVLVIYGKNNRPSGIPDRFSGSSSGNTASLTITGAQAEDEADYYCNSR
DSSGNHVVFGGGTKLTVGGGSGGGSGGGSGGGSGGGSGEVQLVESGGGVVRPGGSLRLSCAA
SGFTFDDYGMSWVRQAPGKGLEWVSGINWNGGSTGYADSVKGRFTISRDNAKNSLYLQMNS
LRAEDTAVYYCARGRSLLFDYWGQGTLVTVSR
39. SEQ ID NO: 129 (Sequence for v594) 
AGTAGCGAACTGACCCAGGACCCCGCAGTGAGCGTCGCACTGGGGCAGACAGTGCGAATC
ACTTGCCAGGGAGACAGCCTGCGGTCCTACTATGCTTCCTGGTACCAGCAGAAACCTGGCC
AGGCACCAGTGCTGGTCATCTATGGAAGAACAATCGGCCCAGCGGCATCCCCGATAGAT
TCTCCGGCAGCTCCTCTGGGAACACCGCCTCTCTGACAATTACTGGGGCCCAGGCTGAGGA
CGAAGCTGATTACTATTGCAACAGCAGGGACAGTTCAGGAAATCACGTGGTCTTTGGAGG
AGGAACTAAGCTGACCGTGGGAGGAGGCAGCGGAGGAGGATCTGGAGGAGGAAGTGGAG
GAGGATCAGGAGGAGGAAGCGGAGAGGTGCAGCTGGTCGAAAGCGGAGGAGGAGTGGTC
AGACCTGGAGGGTCCCTGAGGCTGTCTTGTGCCGCTAGTGGCTTCACCTTTGACGATTACG
GAATGAGTTGGGTCCGGCAGGCACCAGGAAAGGGACTGGAGTGGGTGTCAGGCATCAACT
GGAATGGAGGCAGTACCGGATACGCCGATTCAGTGAAAGGCAGGTTCACAATTTCTCGCG
ACAACGCTAAGAATAGTCTGTATCTGCAGATGAACTCACTGAGAGCTGAGGATACAGCAG
TGTACTATTGCGCCAGAGGCAGGTCTCTGCTGTTTGACTACTGGGGGCAGGGAACACTGGT
GACTGTCTCACGAGGAGGAAGCGGCGATGCACACAAGTCCGAGGTCGCTCATAGATTCAA
AGACCTGGGGGAGGAAAATTTTAAGGCCCTGGTGCTGATCGCATTCGCCCAGTATCTGCAG
CAGTGCCCATTCGAGGACCACGTGAAACTGGTCAACGAGGTGACCGAATTTGCCAAGACA
 
TGCGTGGCCGACGAGAGCGCTGAAAATTGTGATAAATCCCTGCATACACTGTTCGGGATA
AGCTGTGTACCGTGGCCACACTGAGGGAGACTTACGGAGAAATGGCAGACTGCTGTGCCA
AACAGGAGCCAGAACGCAACGAGTGCTTTCTGCAGCACAAGGACGATAACCCAAATCTGC
CACGACTGGTGCGACCAGAAGTGGACGTCATGTGTACAGCCTTCCACGATAATGAGGAAA
CTTTTCTGAAGAAATACCTGTATGAGATCGCCCGGAGACATCCCTACTTCTATGCTCCTGAA
CTGCTGTTCTTTGCAAAACGGTACAAGGCAGCCTTTACCGAGTGCTGTCAGGCTGCAGATA
AGGCCGCTTGCCTGCTGCCAAAACTGGACGAGCTGAGAGATGAAGGCAAGGCAAGCTCCG
CCAAGCAGAGGCTGAAATGTGCTAGCCTGCAGAAGTTCGGGGAGAGGGCCTTCAAGGCTT
GGGCAGTGGCACGACTGTCACAGAGATTCCCCAAGGCTGAGTTTGCAGAAGTCAGCAAGC
TGGTGACTGACCTGACCAAAGTGCACACCGAGTGCTGTCATGGCGACCTGCTGGAATGCGC
CGACGATCGCGCCGATCTGGCTAAGTACATCTGTGAGAACCAGGACAGCATTTCTAGTAAG
CTGAAAGAGTGCTGTGAAAAGCCTCTGCTGGAGAAATCCCACTGCATCGCCGAGGTGGAA
AACGACGAAATGCCAGCTGATCTGCCCTCTCTGGCAGCCGACTTCGTCGAGAGTAAGGATG
TGTGTAAAAATTACGCTGAAGCAAAGGATGTGTTCCTGGGCATGTTTCTGTACGAGTATGC
AAGGCGACACCCAGACTACTCCGTGGTCCTGCTGCTGCGGCTGGCTAAAACCTATGAGACC
ACACTGGAAAAGTGCTGTGCTGCAGCCGATCCTCATGAGTGCTATGCCAAGGTCTTCGACG
AGTTCAAGCCACTGGTGGAGGAACCCCAGAACCTGATCAAGCAGAATTGTGAGCTGTTTG
AACAGCTGGGCGAGTACAAGTTCCAGAACGCCCTGCTGGTGAGATATACAAAGAAAGTCC
CTCAGGTGTCAACCCCAACACTGGTGGAGGTCAGCCGGAATCTGGGAAAGTGGGCAGCA
AATGCTGTAAGCACCCCGAGGCAAAGAGAATGCCTTGCGCCGAAGATTACCTGTCTGTGGT
CCTGAACCAGCTGTGTGTGCTGCATGAGAAAACTCCTGTCAGTGACAGGGTGACCAAGTGC
TGTACAGAATCTCTGGTGAACCGACGGCCTTGCTTCAGTGCCCTGGAGGTCGATGAAACAT
ATGTGCCAAAGGAGTTTAATGCCGAAACTTTCACCTTTCACGCTGACATCTGTACTCTGAG
CGAGAAGGAACGCCAGATTAAGAAACAGACCGCCCTGGTCGAGCTGGTGAAGCATAAACC
AAAGGCAACAAAGGAACAGCTGAAAGCCGTCATGGACGATTTCGCTGCATTTGTGGAGAA
ATGCTGTAAGGCCGACGATAAGGAAACTTGCTTCGCTGAGGAAGGAAAGAAACTGGTGGC
AGCTTCCCAGGCAGCACTGGGACTGGGAGGGTCTGGAGGCAGTGGAGGATCAGGAGGGAG
CGGAGGCGACATCCAGATGACCCAGTCCCCCTCAAGCCTGAGTGCCTCAGTCGGCGATCGC
GTGACAATTACTTGTCGAGCTTCTCAGGACGTCAATACAGCCGTGGCTTGGTATCAGCAGA
AGCCTGGAAAGGCACCAAAACTGCTGATCTACAGCGCCTCCTTTCTGTATTCCGGCGTGCC
CTCTCGATTCTCTGGAAGTCGGTCAGGCACCGATTTTACCCTGACAATTTCCTCTCTGCAGC
 
CTGAGGACTTCGCCACATACTATTGCCAGCAGCACTATACTACCCCCCCTACTTTTGGCCA
GGGGACCAAGGTGGAAATCAAAGGGGGAAGTGGCGGGGGATCAGGCGGCGGAAGCGGCG
GCGGCAGCGGCGGCGGATCTGGAGAGGTCCAGCTGGTGGAAAGCGGAGGAGGACTGGTG
CAGCCTGGAGGGAGTCTGCGACTGTCATGTGCTGCAAGCGGCTTCAACATCAAAGATACCT
ACATTCATTGGGTCAGGCAGGCCCCTGGAAAGGGCCTGGAATGGGTGGCACGAATCTATC
CCACTAATGGCTACACCAGATATGCCGATTCCGTGAAAGGGCGCTTCACTATTTCCGCTGA
CACATCTAAGAACACTGCATACCTGCAGATGAACAGCCTGCGCGCTGAGGACACCGCAGT
GTACTATTGCTCTCGATGGGGCGGCGACGGCTTCTACGCAATGGACTACTGGGGGCAGGGG
ACACTGGTGACTGTGAGCAGCTGAGGATCC
40. SEQ ID NO: 130 (Protein Sequence for v594) 
SSELTQDPAVSVALGQTVRITCQGDSLRSYYASWYQQKPGQAPVLVIYGKNNRPSGIPDRFSGS
SSGNTASLTITGAQAEDEADYYCNSRDSSGNHVVFGGGTKLTVGGGSGGGSGGGSGGGSGGG
SGEVQLVESGGGVVRPGGSLRLSCAASGFTFDDYGMSWVRQAPGKGLEWVSGINWNGGSTG
YADSVKGRFTISRDNAKNSLYLQMNSLRAEDTAVYYCARGRSLLFDYWGQGTLVTVSRGGSG
DAHKSEVAHRFKDLGEENFKALVLIAFAQYLQQCPFEDHVKLVNEVTEFAKTCVADESAENC
DKSLHTLFGDKLCTVATLRETYGEMADCCAKQEPERNECFLQHKDDNPNLPRLVRPEVDVMC
TAFHDNEETFLKKYLYEIARRHPYFYAPELLFFAKRYKAAFTECCQAADKAACLLPKLDELRD
EGKASSAKQRLKCASLQKFGERAFKAWAVARLSQRFPKAEFAEVSKLVTDLTKVHTECCHGD
LLECADDRADLAKYICENQDSISSKLKECCEKPLLEKSHCIAEVENDEMPADLPSLAADFVESK
DVCKNYAEAKDVFLGMFLYEYARRHPDYSVVLLLRLAKTYETTLEKCCAAADPHECYAKVF
DEFKPLVEEPQNLIKQNCELFEQLGEYKFQNALLVRYTKKVPQVSTPTLVEVSRNLGKVGSKC
CKHPEAKRMPCAEDYLSVVLNQLCVLHEKTPVSDRVTKCCTESLVNRRPCFSALEVDETYVPK
EFNAETFTFHADICTLSEKERQIKKQTALVELVKHKPKATKEQLKAVMDDFAAFVEKCCKADD
KETCFAEEGKKLVAASQAALGLGGSGGSGGSGGSGGDIQMTQSPSSLSASVGDRVTITCRASQ
DVNTAVAWYQQKPGKAPKLLIYSASFLYSGVPSRFSGSRSGTDFTLTISSLQPEDFATYYCQQH
YTTPPTFGQGTKVEIKGGSGGGSGGGSGGGSGGGSGEVQLVESGGGLVQPGGSLRLSCAASGF
NIKDTYIHWVRQAPGKGLEWVARIYPTNGYTRYADSVKGRFTISADTSKNTAYLQMNSLRAE
DTAVYYCSRWGGDGFYAMDYWGQGTLVTVSS
41. SEQ ID NO: 19 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVCRLSQR
FPKAEFAEVSKLVTDLTKVHTECCHGDLLECADDRADLAKYICENQDSISSKLKECCEKPLLEK
SHCIAEVENDEMPADLPSLAADFVESKDVCKNYAEAKDVFLGMFLYEYARA
42. SEQ ID NO: 55 (Nucleic Acid sequence encoding protein of SEQ ID NO: 19)
   CL_#1042_HSA-1_337_DSS_CH-A_A217C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGACTCAAGTGTGCCAGTCTCCAAAAATTTGGAGAAAGAGCTTTCAAAGCATGGGC
AGTATGCCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTTGTATGAATATGCAAGAGC
ATGA
43. SEQ ID NO: 20 (Signal Sequence underlined) 
ATMAVMAPRTLVLLLSGALALTQTWAGSCVLLLRLAKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL
44. SEQ ID NO: 56: (Nucleic Acid sequence encoding protein of SEQ ID NO: 20)
   CL_#1045_HSA-342_585_DSS_CH-B_V343C 
CGCCACTATGGCTGTGATGGCCCCTAGGACCCTGGTGCTGCTGCTGTCCGGAGCT
CTGGCTCTGACTCAGACCTGGGCTGGATCTTGCGTGCTGCTGCTGAGACTTGCCAAGACAT
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCGTGGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAACT
TGTTGCTGCAAGTCAAGCTGCCTTAGGCTTATGA
45. SEQ ID NO: 21 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVARLSQ
RFPKAEFAEVSKLVTDLTKVHTECCHGDLLECADDRADLAKYICENQDSISSKLKECCEKPLLE
KSHCIAEVENDEMPADLPSLAADFVESKDVCKNYAEAKDVFLGMFCYEYARA
46. SEQ ID NO: 57: (Nucleic Acid sequence encoding protein of SEQ ID NO: 21)
   CL_#1043_HSA-1_337_DSS_CH-A_L331C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGAAGGCTTCGTCTGCCAA
ACAGAGACTCAAGTGTGCCAGTCTCCAAAAATTTGGAGAAAGAGCTTTCAAAGCATGGGC
AGTAGCTCGCCTGAGCCAGAGATTTCCCAAAGCTGAGTTTGCAGAAGTTTCCAAGTTAGTG
 
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTGCTATGAATATGCAAGAGC
ATGA
47. SEQ ID NO: 22 (Signal Sequence underlined) 
ATMAVMAPRTLVLLLSGALALTQTWAGSVVLLLRLCKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL
48. SEQ ID NO: 58 (Nucleic Acid sequence encoding protein of SEQ ID NO: 22)
   CL_#1047_HSA-342_585_DSS_CH-B_A350C 
GCCACTATGGCTGTGATGGCCCCTAGGACCCTGGTGCTGCTGCTGTCCGGAGCT
CTGGCTCTGACTCAGACCTGGGCTGGATCTGTCGTGCTGCTGCTGAGACTTTGCAAGACAT
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCGTGGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAACT
TGTTGCTGCAAGTCAAGCTGCCTTAGGCTTATGA
49. SEQ ID NO: 23 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVCRLSQR
FPKAEFAEVSKLVTDLTKVHTECCHGDLLECADDRADLAKYICENQDSISSKLKECCEKPLLEK
SHCIAEVENDEMPADLPSLAADFVESKDVCKNYAEAKDVFLGMFLYEYARA
50. SEQ ID NO: 59 (Nucleic Acid sequence encoding protein of SEQ ID NO: 23)
   CL_#1042_HSA-1_337_DSS_CH-A_A217C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGACTCAAGTGTGCCAGTCTCCAAAAATTTGGAGAAAGAGCTTTCAAAGCATGGGC
AGTATGCCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTTGTATGAATATGCAAGAGC
ATGA
51. SEQ ID NO: 24 (Signal Sequence underlined) 
ATMAVMAPRTLVLLLSGALALTQTWAGSVVLCLRLAKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL*
52. SEQ ID NO: 60 (Nucleic Acid sequence encoding protein of SEQ ID NO: 24)
   CL_#1046_HSA-342_585_DSS_CH-B_L346C 
GCCACTATGGCTGTGATGGCCCCTAGGACCCTGGTGCTGCTGCTGTCCGGAGCT
CTGGCTCTGACTCAGACCTGGGCTGGATCTGTCGTGCTGTGCCTGAGACTTGCCAAGACAT
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCGTGGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAC
 TTGTTGCTGCAAGTCAAGCTGCCTTAGGCTTATGA
53. SEQ ID NO: 25 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKACAVARLSQR
FPKAEFAEVSKLVTDLTKVHTECCHGDLLECADDRADLAKYICENQDSISSKLKECCEKPLLEK
SHCIAEVENDEMPADLPSLAADFVESKDVCKNYAEAKDVFLGMFLYEYARA
54. SEQ ID NO: 61 (Nucleic Acid sequence encoding protein of SEQ ID NO: 25)
   CL_#1041_HSA-1_337_DSS_CH-A_W214C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGACTCAAGTGTGCCAGTCTCCAAAAATTTGGAGAAAGAGCTTTCAAAGCATGCGC
AGTAGCTCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTTGTATGAATATGCAAGAGC
ATGA
55. SEQ ID NO: 26 (Signal Sequence underlined) 
ATMAVMAPRTLVLLLSGALALTQTWAGSCVLLLRLAKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL
56. SEQ ID NO 62: (Nucleic Acid sequence encoding protein of SEQ ID NO: 26)
   CL_#1045_HSA-342_DSS_CH-B_V343C 
GCCACTATGGCTGTGATGGCCCCTAGGACCCTGGTGCTGCTGCTGTCCGGAGCT
CTGGCTCTGACTCAGACCTGGGCTGGATCTTGCGTGCTGCTGCTGAGACTTGCCAAGACAT
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCGTGGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAC
TTGTTGCTGCAAGTCAAGCTGCCTTAGGCTTATGA
57. SEQ ID NO: 27 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVARLSQ
RFPKAEFAEVSKLVTDLTKVHTECCHGDLLECADDRADLAKYICENQDSISSKLKECCEKPLLE
KSHCIAEVENDEMPADLPSLAADFVESKDVCKNYAEAKDVFLGMFLYEYCRA
58. SEQ ID NO: 63 (Nucleic Acid sequence encoding protein of SEQ ID NO: 27)
   CL_#1044_HSA-1_337_DSS_CH-A_A335C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
 
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGACTCAAGTGTGCCAGTCTCCAAAAATTTGGAGAAAGAGCTTTCAAAGCATGGGC
AGTAGCTCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTTGTATGAATATTGCAGAGC
ATGA
59. SEQ ID NO: 28 (Signal Sequence underlined) 
ATMAVMAPRTLVLLLSGALALTQTWAGSVVLCLRLAKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSVVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL
60. SEQ ID NO: 64 (Nucleic Acid sequence encoding protein of SEQ ID NO: 28)
   CL_#1046_HSA-342_585_DSS_CH-B_L346C 
GCCACTATGGCTGTGATGGCCCCTAGGACCCTGGTGCTGCTGCTGTCCGGAGCT
CTGGCTCTGACTCAGACCTGGGCTGGATCTGTCGTGCTGTGCCTGAGACTTGCCAAGACAT
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCGTGGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAACT
TGTTGCTGCAAGTCAAGCTGCCTTAGGCTTATGA
61. SEQ ID NO: 29 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRCKCASLQKFGERAFKAWAVARLSQ
RFPKAEFAEVSKLVTDLTKVHTECCHGDLLECADDRADLAKYICENQDSISSKLKECCEKPLLE
KSHCIAEVENDEMPADLPSLAADFVESKDVCKNYAEAKDVFLGMFLYEYARA
62. SEQ ID NO: 65 (Nucleic Acid sequence encoding protein of SEQ ID NO: 29)
   CL_#1040_HSA-1_337_DSS_CH-A_L198C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGATGCAAGTGTGCCAGTCTCCAAAAATTTGGAGAAAGAGCTTTCAAAGCATGGGC
AGTAGCTCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGGAAAATGA
TGAGATGCCTGCTGACTTGCCTTCATTAGCTGCTGATTTTGTTGAAAGTAAGGATGTTTGCA
AAAACTATGCTGAGGCAAAGGATGTCTTCCTGGGCATGTTTTTGTATGAATATGCAAGAGC
ATGA
63. SEQ ID NO: 30 (Signal Sequence underlined) 
ATMAVMAPRTLVLLLSGALALTQTWAGSVVLLLRLAKTYETTLEKCCAAADPHECYAKVFDE
FKPLVEEPQNLIKQNCELFEQLGEYKFQNALLVRYTKKVPQVSTPTLVEVSRNLGKVGSKCCK
HPEAKRMPCAEDYLSCVLNQLCVLHEKTPVSDRVTKCCTESLVNRRPCFSALEVDETYVPKEF
NAETFTFHADICTLSEKERQIKKQTALVELVKHKPKATKEQLKAVMDDFAAFVEKCCKADDK
ETCFAEEGKKLVAASQAALGL
64. SEQ ID NO: 66 (Nucleic Acid sequence encoding protein of SEQ ID NO: 30)
   CL_#1048_HSA-342_DSS_CH-B_V455C 
GCCACTATGGCTGTGATGGCCCCTAGGACCCTGGTGCTGCTGCTGTCCGGAGCT
CTGGCTCTGACTCAGACCTGGGCTGGATCTGTCGTGCTGCTGCTGAGACTTGCCAAGACAT
 
ATGAAACCACTCTAGAGAAGTGCTGTGCCGCTGCAGATCCTCATGAATGCTATGCCAAAGT
GTTCGATGAATTTAAACCTCTTGTGGAAGAGCCTCAGAATTTAATCAAACAAAATTGTGAG
CTTTTTGAGCAGCTTGGAGAGTACAAATTCCAGAATGCGCTATTAGTTCGTTACACCAAGA
AAGTACCCCAAGTGTCAACTCCAACTCTTGTAGAGGTCTCAAGAAACCTAGGAAAAGTGG
GCAGCAAATGTTGTAAACATCCTGAAGCAAAAAGAATGCCCTGTGCAGAAGACTATCTAT
CCTGCGTCCTGAACCAGTTATGTGTGTTGCATGAGAAAACGCCAGTAAGTGACAGAGTCAC
CAAATGCTGCACAGAATCCTTGGTGAACAGGCGACCATGCTTTTCAGCTCTGGAAGTCGAT
GAAACATACGTTCCCAAAGAGTTTAATGCTGAAACATTCACCTTCCATGCAGATATATGCA
CACTTTCTGAGAAGGAGAGACAAATCAAGAAACAAACTGCACTTGTTGAGCTCGTGAAAC
ACAAGCCCAAGGCAACAAAAGAGCAACTGAAAGCTGTTATGGATGATTTCGCAGCTTTTGT
AGAGAAGTGCTGCAAGGCTGACGATAAGGAGACCTGCTTTGCCGAGGAGGGTAAAAAACT
TGTTGCTGCAAGTCAAGCTGCCTTAGGCTTATGA
65. SEQ ID NO: 31 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVCRLSQR
FPKAEFAEVSKLVTDLTKVHTECCHGDLLECADDRADLAKYICENQDSISSKLKECCEKPLLEK
SHCIAEV
66. SEQ ID NO: 67 (Nucleic Acid sequence encoding protein of SEQ ID NO: 31)
   CL_#1051_HSA-1_293_CH-A_A217C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGAAGGCTTCGTCTGCCAA
ACAGAGACTCAAGTGTGCCAGTCTCCAAAAATTTGGAGAAAGAGCTTTCAAAGCATGGGC
AGTATGCCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGTGA
67. SEQ ID NO: 32 (Signal Sequence underlined) 
ATMAVMAPRTLVLLLSGALALTQTWAGSLAADFVESKDVCKNYAEAKDVFLGMFLYEYCRR
HPDYSVVLLLRLAKTYETTLEKCCAAADPHECYAKVFDEFKPLVEEPQNLIKQNCELFEQLGE
YKFQNALLVRYTKKVPQVSTPTLVEVSRNLGKVGSKCCKHPEAKRMPCAEDYLSVVLNQLCV
LHEKTPVSDRVTKCCTESLVNRRPCFSALEVDETYVPKEFNAETFTFHADICTLSEKERQIKKQT
ALVELVKHKPKATKEQLKAVMDDFAAFVEKCCKADDKETCFAEEGKKLVAASQAALGL
68. SEQ ID NO: 68 (Nucleic Acid sequence encoding protein of SEQ ID NO: 32)
   CL_#1053_HSA-304_585_CH-B_A335C 
GCCACTATGGCTGTGATGGCCCCTAGGACCCTGGTGCTGCTGCTGTCCGGAGCT
CTGGCTCTGACTCAGACCTGGGCTGGATCATTAGCTGCTGATTTTGTTGAAAGTAAGGATG
TTTGCAAAAACTATGCTGAGGCAAAGGATGTCTTCCTGGGCATGTTTTTGTATGAATATTGC
AGAAGGCATCCTGATTACTCTGTCGTGCTGCTGCTGAGACTTGCCAAGACATATGAAACCA
CTCTAGAGAAGTGCTGTGCCGCTGCAGATCCTCATGAATGCTATGCCAAAGTGTTCGATGA
ATTTAAACCTCTTGTGGAAGAGCCTCAGAATTTAATCAAACAAAATTGTGAGCTTTTTGAG
CAGCTTGGAGAGTACAAATTCCAGAATGCGCTATTAGTTCGTTACACCAAGAAAGTACCCC
AAGTGTCAACTCCAACTCTTGTAGAGGTCTCAAGAAACCTAGGAAAAGTGGGCAGCAAAT
GTTGTAAACATCCTGAAGCAAAAAGAATGCCCTGTGCAGAAGACTATCTATCCGTGGTCCT
GAACCAGTTATGTGTGTTGCATGAGAAAACGCCAGTAAGTGACAGAGTCACCAAATGCTG
CACAGAATCCTTGGTGAACAGGCGACCATGCTTTTCAGCTCTGGAAGTCGATGAAACATAC
GTTCCCAAAGAGTTTAATGCTGAAACATTCACCTTCCATGCAGATATATGCACACTTTCTGA
GAAGGAGAGACAAATCAAGAAACAAACTGCACTTGTTGAGCTCGTGAAACACAAGCCCAA
GGCAACAAAAGAGCAACTGAAAGCTGTTATGGATGATTTCGCAGCTTTTGTAGAGAAGTG
CTGCAAGGCTGACGATAAGGAGACCTGCTTTGCCGAGGAGGGTAAAAAACTTGTTGCTGC
AAGTCAAGCTGCCTTAGGCTTATGA
69. SEQ ID NO: 33 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVCRLSQR
FPKAEFAEVSKLVTDLTKVHTECCHGDLLECADDRADLAKYICENQDSISSKLKECCEKPLLEK
SHCIAEV
70. SEQ ID NO: 69 (Nucleic Acid sequence encoding protein of SEQ ID NO: 33)
   CL_#1051_HSA-1293_CH-A_A217C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
 
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGGAAGGCTTCGTCTGCCAA
ACAGAGACTCAAGTGTGCCAGTCTCCAAAAATTTGGAGAAAGAGCTTTCAAAGCATGGGC
AGTATGCCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGTGA
71. SEQ ID NO: 34 (Signal Sequence underlined) 
ATMAVMAPRTLVLLLSGALALTQTWAGSLAADFVESKDVCKNYAEAKDVFLGMFCYEYARR
HPDYSVVLLLRLAKTYETTLEKCCAAADPHECYAKVFDEFKPLVEEPQNLIKQNCELFEQLGE
YKFQNALLVRYTKKVPQVSTPTLVEVSRNLGKVGSKCCKHPEAKRMPCAEDYLSVVLNQLCV
LHEKTPVSDRVTKCCTESLVNRRPCFSALEVDETYVPKEFNAETFTFHADICTLSEKERQIKKQT
ALVELVKHKPKATKEQLKAVMDDFAAFVEKCCKADDKETCFAEEGKKLVAASQAALGL
72. SEQ ID NO: 70 (Nucleic Acid sequence encoding protein of SEQ ID NO: 34)
   CL_#1052_HSA-304_585_CH-B_L331C 
GCCACTATGGCTGTGATGGCCCCTAGGACCCTGGTGCTGCTGCTGTCCGGAGCT
CTGGCTCTGACTCAGACCTGGGCTGGATCATTAGCTGCTGATTTTGTTGAAAGTAAGGATG
TTTGCAAAAACTATGCTGAGGCAAAGGATGTCTTCCTGGGCATGTTTTGCTATGAATATGC
AAGAAGGCATCCTGATTACTCTGTCGTGCTGCTGCTGAGACTTGCCAAGACATATGAAACC
ACTCTAGAGAAGTGCTGTGCCGCTGCAGATCCTCATGAATGCTATGCCAAAGTGTTCGATG
AATTTAAACCTCTTGTGGAAGAGCCTCAGAATTTAATCAAACAAAATTGTGAGCTTTTTGA
GCAGCTTGGAGAGTACAAATTCCAGAATGCGCTATTAGTTCGTTACACCAAGAAAGTACCC
CAAGTGTCAACTCCAACTCTTGTAGAGGTCTCAAGAAACCTAGGAAAAGTGGGCAGCAAA
TGTTGTAAACATCCTGAAGCAAAAAGAATGCCCTGTGCAGAAGACTATCTATCCGTGGTCC
TGAACCAGTTATGTGTGTTGCATGAGAAAACGCCAGTAAGTGACAGAGTCACCAAATGCT
GCACAGAATCCTTGGTGAACAGGCGACCATGCTTTTCAGCTCTGGAAGTCGATGAAACATA
CGTTCCCAAAGAGTTTAATGCTGAAACATTCACCTTCCATGCAGATATATGCACACTTTCTG
AGAAGGAGAGACAAATCAAGAAACAAACTGCACTTGTTGAGCTCGTGAAACACAAGCCCA
AGGCAACAAAAGAGCAACTGAAAGCTGTTATGGATGATTTCGCAGCTTTTGTAGAGAAGT
GCTGCAAGGCTGACGATAAGGAGACCTGCTTTGCCGAGGAGGGTAAAAAACTTGTTGCTG
CAAGTCAAGCTGCCTTAGGCTTATGA
73. SEQ ID NO: 35 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRCKCASLQKFGERAFKAWAVARLSQ
RFPKAEFAEVSKLVTDLTKVHTECCHGDLLECADDRADLAKYICENQDSISSKLKECCEKPLLE
KSHCIAEV
74. SEQ ID NO: 71 (Nucleic Acid sequence encoding protein of SEQ ID NO: 35)
   CL_#1050_HSA-1293_CH-A_L198C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGAAGGCTTCGTCTGCCAA
ACAGAGATGCAAGTGTGCCAGTCTCCAAAAATTTGGAGAAAGAGCTTTCAAAGCATGGGC
AGTAGCTCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGTGA
75. SEQ ID NO: 36 (Signal Sequence underlined) 
ATMAVMAPRTLVLLLSGALALTQTWAGSLAADFVESKDVCKNYAEAKDVFLGMFLYEYARR
HPDYSVVLLLRLAKTYETTLEKCCAAADPHECYAKVFDEFKPLVEEPQNLIKQNCELFEQLGE
YKFQNALLVRYTKKVPQVSTPTLVEVSRNLGKVGSKCCKHPEAKRMPCAEDYLSVVLCQLCV
LHEKTPVSDRVTKCCTESLVNRRPCFSALEVDETYVPKEFNAETFTFHADICTLSEKERQIKKQT
ALVELVKHKPKATKEQLKAVMDDFAAFVEKCCKADDKETCFAEEGKKLVAASQAALGL
76. SEQ ID NO: 72 (Nucleic Acid sequence encoding protein of SEQ ID NO: 36)
   CL_#1055_HSA-304_585_CH-B_N458C 
GCCACTATGGCTGTGATGGCCCCTAGGACCCTGGTGCTGCTGCTGTCCGGAGCT
CTGGCTCTGACTCAGACCTGGGCTGGATCATTAGCTGCTGATTTTGTTGAAAGTAAGGATG
TTTGCAAAAACTATGCTGAGGCAAAGGATGTCTTCCTGGGCATGTTTTTGTATGAATATGC
AAGAAGGCATCCTGATTACTCTGTCGTGCTGCTGCTGAGACTTGCCAAGACATATGAAACC
ACTCTAGAGAAGTGCTGTGCCGCTGCAGATCCTCATGAATGCTATGCCAAAGTGTTCGATG
AATTTAAACCTCTTGTGGAAGAGCCTCAGAATTTAATCAAACAAAATTGTGAGCTTTTTGA
GCAGCTTGGAGAGTACAAATTCCAGAATGCGCTATTAGTTCGTTACACCAAGAAAGTACCC
CAAGTGTCAACTCCAACTCTTGTAGAGGTCTCAAGAAACCTAGGAAAAGTGGGCAGCAAA
TGTTGTAAACATCCTGAAGCAAAAAGAATGCCCTGTGCAGAAGACTATCTATCCGTGGTCC
TGTGCCAGTTATGTGTGTTGCATGAGAAAACGCCAGTAAGTGACAGAGTCACCAAATGCTG
CACAGAATCCTTGGTGAACAGGCGACCATGCTTTTCAGCTCTGGAAGTCGATGAAACATAC
GTTCCCAAAGAGTTTAATGCTGAAACATTCACCTTCCATGCAGATATATGCACACTTTCTGA
GAAGGAGAGACAAATCAAGAAACAAACTGCACTTGTTGAGCTCGTGAAACACAAGCCCAA
GGCAACAAAAGAGCAACTGAAAGCTGTTATGGATGATTTCGCAGCTTTTGTAGAGAAGTG
CTGCAAGGCTGACGATAAGGAGACCTGCTTTGCCGAGGAGGGTAAAAAACTTGTTGCTGC
AAGTCAAGCTGCCTTAGGCTTATGA
77. SEQ ID NO: 37 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSCKQRLKCASLQKFGERAFKAWAVARLSQR
FPKAEFAEVSKLVTDLTKVHTECCHGDLLECADDRADLAKYICENQDSISSKLKECCEKPLLEK
SHCIAEV
78. SEQ ID NO: 73 (Nucleic Acid sequence encoding protein of SEQ ID NO: 37)
   CL_#1049_HSA-1_293_CH-A_A194C 
GCCACTATGGCTGTGATGGCCCCTAGGACCCTGGTGCTGCTGCTGTCCGGAGCT
CTGGCTCTGACTCAGACCTGGGCTGGAGATGCACACAAGAGTGAGGTTGCTCATCGGTTTA
AAGATTTGGGAGAAGAAAATTTCAAAGCCTTGGTGTTGATTGCCTTTGCTCAGTATCTTCA
GCAGTGTCCATTTGAAGATCATGTAAAATTAGTGAATGAAGTAACTGAATTTGCAAAAACA
TGTGTTGCTGATGAGTCAGCTGAAAATTGTGACAAATCACTTCATACCCTTTTTGGAGACA
AATTATGCACAGTTGCAACTCTTCGTGAAACCTATGGTGAAATGGCTGACTGCTGTGCAAA
ACAAGAACCTGAGAGAAATGAATGCTTCTTGCAACACAAAGATGACAACCCAAACCTCCC
CCGATTGGTGAGACCAGAGGTTGATGTGATGTGCACTGCTTTTCATGACAATGAAGAGACA
TTTTTGAAAAAATACTTATATGAAATTGCCAGAAGACATCCTTACTTTTATGCCCCGGAACT
CCTTTTCTTTGCTAAAAGGTATAAAGCTGCTTTTACAGAATGTTGCCAAGCTGCTGATAAAG
CTGCCTGCCTGTTGCCAAAGCTCGATGAACTTCGGGATGAAGGAAGGCTTCGTCTTGCAA
ACAGAGACTCAAGTGTGCCAGTCTCCAAAAATTTGGAGAAAGAGCTTTCAAAGCATGGGC
AGTAGCTCGCCTGAGCCAGAGATTTCCCAAAGCTGAGTTTGCAGAAGTTTCCAAGTTAGTG
ACAGATCTTACCAAAGTCCACACGGAATGCTGCCATGGAGATCTGCTTGAATGTGCTGATG
ACAGGGCGGACCTTGCCAAGTATATCTGTGAAAATCAAGATTCGATCTCCAGTAAACTGAA
GGAATGCTGTGAAAAACCTCTGTTGGAAAAATCCCACTGCATTGCCGAAGTGTGA
79. SEQ ID NO: 38 (Signal Sequence underlined) 
ATMAVMAPRTLVLLLSGALALTQTWAGSLAADFVESKDVCKNYAEAKDVFLGMFLYEYARR
HPDYSVVLLLRLAKTYETTLEKCCAAADPHECYAKVFDEFKPLVEEPQNLIKQNCELFEQLGE
YKFQNALLVRYTKKVPQVSTPTLVEVSRNLGKVGSKCCKHPEAKRMPCAEDYLSCVLNQLCV
LHEKTPVSDRVTKCCTESLVNRRPCFSALEVDETYVPKEFNAETFTFHADICTLSEKERQIKKQT
ALVELVKHKPKATKEQLKAVMDDFAAFVEKCCKADDKETCFAEEGKKLVAASQAALGL
80. SEQ ID NO: 74 (Nucleic Acid sequence encoding protein of SEQ ID NO: 38)
   CL_#1054_HSA-304_585_CH-B_V455C 
GCCACTATGGCTGTGATGGCCCCTAGGACCCTGGTGCTGCTGCTGTCCGGAGCT
CTGGCTCTGACTCAGACCTGGGCTGGATCATTAGCTGCTGATTTTGTTGAAAGTAAGGATG
TTTGCAAAAACTATGCTGAGGCAAAGGATGTCTTCCTGGGCATGTTTTTGTATGAATATGC
AAGAAGGCATCCTGATTACTCTGTCGTGCTGCTGCTGAGACTTGCCAAGACATATGAAACC
ACTCTAGAGAAGTGCTGTGCCGCTGCAGATCCTCATGAATGCTATGCCAAAGTGTTCGATG
AATTTAAACCTCTTGTGGAAGAGCCTCAGAATTTAATCAAACAAAATTGTGAGCTTTTTGA
GCAGCTTGGAGAGTACAAATTCCAGAATGCGCTATTAGTTCGTTACACCAAGAAAGTACCC
CAAGTGTCAACTCCAACTCTTGTAGAGGTCTCAAGAAACCTAGGAAAAGTGGGCAGCAAA
TGTTGTAAACATCCTGAAGCAAAAAGAATGCCCTGTGCAGAAGACTATCTATCCTGCGTCC
TGAACCAGTTATGTGTGTTGCATGAGAAAACGCCAGTAAGTGACAGAGTCACCAAATGCT
GCACAGAATCCTTGGTGAACAGGCGACCATGCTTTTCAGCTCTGGAAGTCGATGAAACATA
CGTTCCCAAAGAGTTTAATGCTGAAACATTCACCTTCCATGCAGATATATGCACACTTTCTG
AGAAGGAGAGACAAATCAAGAAACAAACTGCACTTGTTGAGCTCGTGAAACACAAGCCCA
AGGCAACAAAAGAGCAACTGAAAGCTGTTATGGATGATTTCGCAGCTTTTGTAGAGAAGT
GCTGCAAGGCTGACGATAAGGAGACCTGCTTTGCCGAGGAGGGTAAAAAACTTGTTGCTG
CAAGTCAAGCTGCCTTAGGCTTATGA
81. SEQ ID NO: 39 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVARLSQ
RFPKAEFAEVSKLVTDLTKVHTECCHGDLLECADDRADLAKYICENQDSISSKLKECCEKPLLE
KSHCIAEVENDEMPADLPSLAADFVESKDVCKNYAEAKDVFLGMFLYEYARRHPGGGGS
82. SEQ ID NO: 75 (Nucleic Acid sequence encoding protein of SEQ ID NO: 39) 
GCCACTATGGCTGTGATGGCACCTAGAACACTGGTCCTGCTGCTGTCAGGGGCA
CTGGCACTGACTCAGACTTGGGCTGGAGATGCTCATAAGAGCGAGGTCGCTCACAGGTTC
AAGGATCTGGGGGAGGAAAACTTTAAAGCCCTGGTGCTGATCGCATTCGCCCAGTACCTG
CAGCAGTGCCCCTTTGAGGACCACGTGAAGCTGGTCAACGAGGTGACAGAGTTCGCCAAA
ACTTGCGTCGCCGACGAGTCAGCTGAAAATTGTGATAAGAGCCTGCATACTCTGTTTGGG
GATAAACTGTGCACCGTGGCCACACTGAGGGAGACCTATGGAGAAATGGCAGACTGCTGT
GCCAAGCAGGAGCCCGAACGCAACGAGTGCTTCCTGCAGCATAAAGACGATAACCCCAAT
CTGCCTCGACTGGTGCGGCCTGAAGTGGACGTCATGTGTACCGCTTTCCACGATAATGAG
GAAACATTTCTGAAGAAATACCTGTATGAGATTGCCCGGAGACATCCTTACTTTTATGCT
 
CCAGAACTGCTGTTCTTTGCAAAGCGGTACAAAGCCGCTTTCACAGAGTGCTGTCAGGCA
GCCGATAAGGCTGCATGCCTGCTGCCAAAACTGGACGAGCTGCGCGATGAAGGCAAGGCC
AGCTCCGCTAAGCAGCGACTGAAATGTGCCTCTCTGCAGAAGTTCGGGGAGCGGGCTTTT
AAAGCTTGGGCAGTCGCCAGACTGAGTCAGAGGTTCCCCAAGGCAGAGTTTGCCGAAGTC
TCAAAGCTGGTGACTGACCTGACCAAAGTGCACACCGAGTGCTGTCATGGAGACCTGCTG
GAATGCGCCGACGATAGAGCTGATCTGGCAAAGTACATCTGTGAGAATCAGGACAGCATT
TCTAGTAAGCTGAAAGAGTGCTGTGAAAAGCCTCTGCTGGAGAAATCCCACTGCATCGCA
GAGGTGGAAAACGACGAAATGCCAGCAGATCTGCCATCCCTGGCAGCTGACTTTGTCGAG
TCTAAGGATGTGTGTAAAAATTATGCTGAAGCAAAGGATGTGTTCCTGGGCATGTTTCTG
TACGAGTATGCAAGGCGACATCCAGGAGGAGGCTCCTGA
83. SEQ ID NO: 40 (Signal Sequence underlined) 
ATMAVMAPRTLVLLLSGALALTQTWAGGGGGSDYSVVLLLRLAKTYETTLEKCCAAADPHE
CYAKVFDEFKPLVEEPQNLIKQNCELFEQLGEYKFQNALLVRYTKKVPQVSTPTLVEVSRNLG
KVGSKCCKHPEAKRMPCAEDYLSVVLNQLCVLHEKTPVSDRVTKCCTESLVNRRPCFSALEV
DETYVPKEFNAETFTFHADICTLSEKERQIKKQTALVELVKHKPKATKEQLKAVMDDFAAFVE
KCCKADDKETCFAEEGKKLVAASQAALGL
84. SEQ ID NO: 76 (Nucleic Acid sequence encoding protein of SEQ ID NO: 40) 
GCCACTATGGCTGTGATGGCACCTAGAACCCTGGTCCTGCTGCTGAGCGGCGCA
CTGGCACTGACACAGACTTGGGCTGGGGGGGGGGGGGAGCGACTACTCCGTGGTCCTG
CTGCTGCGGCTGGCAAAAACTTATGAGACCACACTGGAAAAGTGCTGTGCCGCTGCAGAC
CCTCACGAGTGCTACGCCAAAGTGTTCGATGAGTTCAAGCCCCTGGTCGAGGAACCTCAG
AACCTGATCAAACAGAATTGTGAGCTGTTCGAACAGCTGGGGGAGTACAAGTTTCAGAAC
GCCCTGCTGGTGCGGTATACAAAGAAAGTGCCACAGGTCTCTACTCCCACCCTGGTGGAG
GTCAGTAGGAATCTGGGCAAAGTGGGGTCAAAATGCTGTAAGCACCCTGAGGCCAAGCGC
ATGCCATGCGCTGAAGACTACCTGTCTGTGGTCCTGAACCAGCTGTGTGTGCTGCATGAG
AAAACCCCAGTGAGTGATCGAGTCACCAAGTGCTGTACAGAGAGCCTGGTGAACCGGAGA
CCCTGCTTCTCCGCTCTGGAGGTGGACGAAACATATGTCCCTAAGGAGTTTAATGCAGAA
ACATTCACTTTTCACGCCGATATCTGTACTCTGTCCGAGAAGGAAAGACAGATTAAGAAA
CAGACCGCCCTGGTGGAGCTGGTCAAGCATAAACCCAAGGCTACAAAAGAACAGCTGAAG
 
GCAGTGATGGACGATTTCGCCGCTTTTGTGGAGAAATGCTGTAAGGCCGACGATAAGGAA
ACTTGCTTCGCTGAGGAAGGAAAGAAACTGGTGGCAGCCAGCCAGGCTGCACTGGGCCTG
TGA
85. SEQ ID NO: 41 (Signal Sequence underlined) 
ATMAVMAPRTLVLLLSGALALTQTWAGDAHKSEVAHRFKDLGEENFKALVLIAFAQYLQQCP
FEDHVKLVNEVTEFAKTCVADESAENCDKSLHTLFGDKLCTVATLRETYGEMADCCAKQEPE
RNECFLQHKDDNPNLPRLVRPEVDVMCTAFHDNEETFLKKYLYEIARRHPYFYAPELLFFAKR
YKAAFTECCQAADKAACLLPKLDELRDEGKASSAKQRLKCASLQKFGERAFKAWAVARLSQ
RFPKAEFAEVSKLVTDLTKVHTECCHGDLLECADDRADLAKYICENQDSISSKLKECCEKPLLE
KSHCIAEVENDEMPAGGGGS
86. SEQ ID NO: 77 (Nucleic Acid sequence encoding protein of SEQ ID NO: 41) 
GCCACTATGGCCGTGATGGCACCTAGAACACTGGTCCTGCTGCTGAGCGGGGCA
CTGGCACTGACACAGACTTGGGCTGGAGACGCACACAAATCTGAGGTCGCTCACCGGTTC
AAGGATCTGGGCGAGGAAAACTTTAAAGCCCTGGTGCTGATCGCCTTCGCTCAGTACCTG
CAGCAGTGCCCTTTTGAGGACCACGTGAAGCTGGTCAACGAGGTGACAGAGTTCGCCAAA
ACTTGCGTCGCAGACGAGTCAGCCGAAAATTGTGATAAGAGCCTGCATACTCTGTTTGGG
GATAAACTGTGTACCGTGGCCACACTGCGCGAGACCTATGGAGAAATGGCTGACTGCTGT
GCAAAGCAGGAGCCCGAACGAAACGAGTGCTTCCTGCAGCATAAAGACGATAACCCCAAT
CTGCCTAGGCTGGTGCGCCCTGAAGTGGACGTCATGTGTACCGCTTTCCACGATAATGAG
GAAACATTTCTGAAGAAATACCTGTATGAGATTGCCCGGAGACATCCATACTTTTATGCC
CCCGAACTGCTGTTCTTTGCTAAGAGATACAAAGCCGCTTTCACAGAGTGCTGTCAGGCA
GCCGATAAGGCTGCATGCCTGCTGCCAAAACTGGACGAGCTGAGAGATGAAGGCAAGGCA
AGCTCCGCCAAGCAGAGGCTGAAATGTGCAAGCCTGCAGAAGTTCGGGGAGAGGGCCTTT
AAAGCATGGGCAGTCGCTCGACTGTCCCAGCGATTCCCCAAGGCTGAGTTTGCAGAAGTC
TCTAAGCTGGTGACTGATCTGACCAAAGTGCACACCGAGTGCTGTCATGGCGACCTGCTG
GAATGCGCCGACGATCGCGCCGATCTGGCTAAGTATATCTGTGAGAACCAGGACAGTATT
TCTAGTAAGCTGAAAGAGTGCTGTGAAAAGCCTCTGCTGGAGAAATCACACTGCATCGCT
GAGGTGGAAAATGACGAAATGCCAGCAGGCGGGGGAGGCTCCTGA
87. SEQ ID NO: 42 (Signal Sequence underlined) 
ATMAVMAPRTLVLLLSGALALTQTWAGGGGGSDLPSLAADFVESKDVCKNYAEAKDVF
LGMFLYEYARRHPDYSVVLLLRLAKTYETTLEKCCAAADPHECYAKVFDEFKPLVEEPQN
LIKQNCELFEQLGEYKFQNALLVRYTKKVPQVSTPTLVEVSRNLGKVGSKCCKHPEAKRM
PCAEDYLSVVLNQLCVLHEKTPVSDRVTKCCTESLVNRRPCFSALEVDETYVPKEFNAET
FTFHADICTLSEKERQIKKQTALVELVKHKPKATKEQLKAVMDDFAAFVEKCCKADDKET
CFAEEGKKLVAASQAALGL
88. SEQ ID NO: 78 (Nucleic Acid sequence encoding protein of SEQ ID NO: 42) 
GCCACTATGGCCGTGATGGCACCTAGAACCCTGGTCCTGCTGCTGAGCGGGGCA
CTGGCACTGACACAGACTTGGGCTGGGGGGGGGGGCAGCGACCTGCCATCCCTGGCA
GCTGACTTCGTGGAGTCAAAAGATGTCTGCAAGAACTACGCAGAAGCCAAGGACGTGTTC
CTGGGGATGTTTCTGTACGAGTATGCTCGGAGACACCCAGATTACAGCGTGGTCCTGCTG
CTGCGCCTGGCCAAAACTTATGAGACCACACTGGAAAAGTGCTGTGCAGCCGCTGACCCC
CATGAGTGCTATGCCAAAGTGTTCGATGAGTTCAAGCCCCTGGTCGAGGAACCTCAGAAC
CTGATCAAACAGAATTGTGAGCTGTTCGAACAGCTGGGCGAGTACAAGTTTCAGAACGCC
CTGCTGGTGCGATATACAAAGAAAGTGCCACAGGTCTCTACTCCCACCCTGGTGGAGGTC
AGTCGAAATCTGGGCAAAGTGGGGTCAAAATGCTGTAAGCACCCTGAGGCTAAGCGGATG
CCATGCGCAGAAGACTACCTGTCTGTGGTCCTGAATCAGCTGTGTGTGCTGCATGAGAAA
ACCCCTGTGAGTGATAGAGTCACCAAGTGCTGTACAGAAAGCCTGGTGAACAGGCGACCA
TGCTTCTCCGCACTGGAGGTGGACGAAACATATGTCCCTAAAGAGTTTAATGCCGAAACA
TTCACTTTTCACGCTGATATCTGTACTCTGTCCGAGAAGGAAAGGCAGATTAAGAAACAG
ACCGCCCTGGTGGAGCTGGTCAAGCATAAACCAAAGGCAACAAAAGAACAGCTGAAGGCC
GTGATGGACGATTTCGCAGCCTTTGTGGAGAAATGCTGTAAGGCTGACGATAAGGAAACT
TGTTTTGCAGAGGAAGGAAAGAAACTGGTGGCTGCATCTCAGGCCGCTCTGGGCCTGTGA
89. SEQ ID NO: 43 (Signal Sequence underlined) 
ATMAVMAPRTLVLLLSGALALTQTWAGDAHKSEVAHRFKDLGEENFKALVLIAFAQYL
QQCPFEDHVKLVNEVTEFAKTCVADESAENCDKSLHTLFGDKLCTVATLRETYGEMADCC
AKQEPERNECFLQHKDDNPNLPRLVRPEVDVMCTAFHDNEETFLKKYLYEIARRHPYFYA
PELLFFAKRYKAAFTECCQAADKAACLLPKLDELRDEGKASSAKQRLKCASLQKFGERAF
KAWAVARLSQRFPKAEFAEVSKLVTDLTKVHTECCHGDLLECADDRADLAKYICENQDSI
SSKLKECCEKPLLEKSHCIAEVENDEMPADLPSLAADFVESKDVCKNYAEAKDVFLGMFLYEY
ARRHPDYSVVLLLRLAKTYETTLEKCCAAAGGGGS
90. SEQ ID NO: 79 Nucleic Acid sequence encoding protein of SEQ ID NO: 43 
GCCACTATGGCTGTGATGGCTCCAAGAACCCTGGTCCTGCTGCTGTCCGGGGCACTGGCTC
TGACTCAGACATGGGCTGGGATGCTCATAAGTCTGAGGTCGCCCACCGATTCAAGGATCT
GGGGGAGGAAAACTTTAAAGCTCTGGTGCTGATCGCATTCGCCCAGTACCTGCAGCAGTGC
CCCTTTGAGGACCACGTGAAGCTGGTCAACGAGGTGACCGAGTTCGCCAAAACATGCGTC
GCCGACGAGTCAGCTGAAAATTGTGATAAGAGCCTGCATACACTGTTTGGGGATAAACTGT
GCACTGTGGCCACCCTGCGGGAGACTTATGGAGAAATGGCAGACTGCTGTGCCAAGCAGG
AGCCCGAAAGAAACGAGTGCTTCCTGCAGCATAAAGACGATAACCCCAATCTGCCTCGAC
TGGTGCGGCCTGAAGTGGACGTCATGTGTACTGCCTTCCACGATAATGAGGAAACCTTTCT
GAAGAAATACCTGTATGAGATTGCCCGGAGACATCCTTACTTTTATGCT
CCAGAACTGCTGTTCTTTGCAAAGCGCTACAAAGCCGCTTTCACCGAGTGCTGTCAGGCAG
CCGATAAGGCTGCATGCCTGCTGCCAAAACTGGACGAGCTGCGCGATGAAGGCAAGGCCA
GCTCCGCTAAGCAGCGACTGAAATGTGCCAGCCTGCAGAAGTTCGGGGAGAGGGCTTTTA
AAGCTTGGGCAGTGGCCAGACTGAGTCAGAGGTTCCCCAAGGCAGAGTTTGCCGAAGTCT
CAAAGCTGGTGACAGACCTGACTAAAGTGCACACAGAGTGCTGTCATGGAGACCTGCTGG
AATGCGCCGACGATCGCGCTGATCTGGCAAAGTACATCTGTGAGAATCAGGACAGCATTTC
TAGTAAGCTGAAAGAGTGCTGTGAAAAGCCTCTGCTGGAGAAATCCCACTGCATCGCAGA
GGTGGAAAACGACGAAATGCCAGCTGATCTGCCCTCCCTGGCCGCTGACTTTGTCGAGTCT
AAGGATGTGTGTAAAAATTATGCTGAAGCAAAGGATGTGTTCCTGGGCATGTTTCTGTACG
AGTATGCCAGGCGCCATCCAGACTACTCTGTGGTCCTGCTGCTGAGACTGGCCAAG
ACCTATGAGACCACACTGGAAAAATGCTGTGCAGCCGCTGGCGGGGAGGCAGTTGA
91. SEQ ID NO: 44 (Signal Sequence underlined) 
ATMAVMAPRTLVLLLSGALALTQTWAGGGGGSDPHECYAKVFDEFKPLVEEPQNLIKQ
NCELFEQLGEYKFQNALLVRYTKKVPQVSTPTLVEVSRNLGKVGSKCCKHPEAKRMPCAE
DYLSVVLNQLCVLHEKTPVSDRVTKCCTESLVNRRPCFSALEVDETYVPKEFNAETFTFH
ADICTLSEKERQIKKQTALVELVKHKPKATKEQLKAVMDDFAAFVEKCCKADDKETCFAE
EGKKLVAASQAALGL
92. SEQ ID NO: 80 (Nucleic Acid sequence encoding protein of SEQ ID NO: 44) 
GCCACTATGGCTGTGATGGCACCTAGAACCCTGGTCCTGCTGCTGTCCGGGGCA
CTGGCACTGACTCAGACTTGGGCTGGGGAGGCGGGGCAGCGACCCTCACGAGTGCTAC
GCAAAAGTGTTCGATGAGTTCAAGCCCCTGGTCGAGGAACCTCAGAACCTGATCAAACAG
AATTGTGAGCTGTTCGAACAGCTGGGGGAGTACAAGTTTCAGAACGCTCTGCTGGTGCGG
TATACCAAGAAAGTGCCACAGGTCAGCACCCCCACACTGGTGGAGGTCTCCAGGAATCTG
GGCAAAGTGGGGTCTAAATGCTGTAAGCACCCTGAGGCCAAGCGCATGCCATGCGCTGAA
GACTACCTGAGCGTGGTCCTGAACCAGCTGTGTGTGCTGCATGAGAAAACACCAGTGTCC
GATCGAGTCACAAAGTGCTGTACTGAGAGTCTGGTGAACCGGAGACCCTGCTTCTCAGCC
CTGGAGGTGGACGAAACTTATGTCCCTAAGGAGTTTAATGCAGAAACTTTCACCTTTCAC
GCCGATATCTGTACCCTGTCTGAGAAGGAAAGACAGATTAAGAAACAGACAGCCCTGGTG
GAGCTGGTCAAGCATAAACCCAAGGCTACTAAAGAACAGCTGAAGGCAGTGATGGACGAT
TTCGCCGCTTTTGTCGAGAAATGCTGTAAGGCCGACGATAAGGAAACCTGCTTCGCTGAG
GAAGGAAAGAAACTGGTGGCAGCCAGCCAGGCTGCACTGGGCCTGTGA
93. SEQ ID NO: 45 (Signal Sequence underlined) 
ATMAVMAPRTLVLLLSGALALTQTWAGDAHKSEVAHRFKDLGEENFKALVLIAFAQYLQ
QCPFEDHVKLVNEVTEFAKTCVADESAENCDKSLHTLFGDKLCTVATLRETYGEMADCCA
KQEPERNECFLQHKDDNPNLPRLVRPEVDVMCTAFHDNEETFLKKYLYEIARRHPYFYAPE
LLFFAKRYKAAFTECCQAADKAACLLPKLDELRDEGKASSAKQRLKCASLQKFGERAFKA
WAVARLSQRFPKAEFAEVSKLVTDLTKVHTECCHGDLLECADDRADLAKYICENQDSISSK
LKECCEKPLLEKSHCIAEVENDEMPADLPSLAADFVE
SKDVCKNYAEAKDVFLGMFLYEYARRHPDYSVVLLLRLAKTYETTLEKCCAAADPHECY
AKVFDEFKPLVEEPQNLIKQNCELFEQLGEYKFQNALLVRYTKKVPQVSTPTLVEVSRNLG
KVGSKCCKHPGGGGS
94. SEQ ID NO: 81 (Nucleic Acid sequence encoding protein of SEQ ID NO: 45) 
GCCACTATGGCTGTGATGGCACCTCGCACCCTGGTCCTGCTGCTGTCCGGGGCACTGGC
ACTGACTCAGACTTGGGCTGGAGATGCTCATAAGTCTGAGGTCGCTCACAGATTCAAGG
ATCTGGGCGAGGAAAACTTTAAAGCACTGGTGCTGATCGCATTCGCCCAGTACCTGCAG
CAGTGCCCCTTTGAGGACCACGTGAAGCTGGTCAACGAAGTGACTGAGTTCGCCAAA
ACCTGCGTCGCCGACGAGTCAGCTGAAAATTGTGATAAGAGCCTGCATACCCTGTTTGG
CGATAAACTGTGCACAGTGGCCACTCTGCGGGAGACATATGGGGAAATGGCAGACTGC
TGTGCCAAGCAGGAGCCTGAAAGAAACGAGTGCTTCCTGCAGCATAAAGACGATAACC
CCAATCTGCCTCGACTGGTGCGGCCAGAAGTGGACGTCATGTGTACAGCCTTCCACGAT
AATGAG
GAAACTTTTCTGAAGAAATACCTGTATGAGATTGCTCGGAGACATCCTTACTTTTATGC
TCCAGAACTGCTGTTCTTTGCAAAGAGGTACAAAGCCGCTTTCACCGAGTGCTGTCAGG
CAGCCGATAAGGCTGCATGCCTGCTGCCAAAACTGGACGAGCTGCGCGATGAAGGAAA
GGCCAGCTCCGCTAAGCAGCGACTGAAATGTGCCAGCCTGCAGAAGTTCGGCGAGCGA
GCTTTT
AAAGCTTGGGCAGTGGCCAGACTGTCCCAGAGGTTCCCCAAGGCAGAGTTTGCCGAAG
TCTCTAAGCTGGTGACCGACCTGACAAAAGTGCACACCGAGTGCTGTCATGGGGACCT
GCTGGAATGCGCCGACGATCGCGCTGATCTGGCAAAGTACATCTGTGAGAATCAGGAC
AGTATTTCTAGTAAGCTGAAAGAGTGCTGTGAAAAGCCTCTGCTGGAGAAATCACACT
 
GCATCGCA
GAGGTGGAAAACGACGAAATGCCAGCAGATCTGCCATCCCTGGCAGCTGACTTCGTCG
AGTCTAAGGATGTGTGTAAAAATTACGCTGAAGCAAAGGATGTGTTCCTGGGGATGTTT
CTGTACGAGTATGCCAGGCGCCACCCCGACTACAGTGTGGTCCTGCTGCTGCGGCTGGC
TAAGACTTATGAGACCACACTGGAAAAATGCTGTGCAGCCGCTGATCCTCATGAGTGCT
ATGCC
AAGGTCTTCGACGAGTTCAAGCCCCTGGTGGAGGAACCTCAGAACCTGATTAAGCAGA
ATTGTGAGCTGTTTGAACAGCTGGGAGAGTACAAATTCCAGAACGCCCTGCTGGTGAG
GTATACAAAGAAAGTGCCACAGGTGAGTACTCCCACCCTGGTGGAAGTCTCACGCAAT
CTGGGGAAGGTCGGAAGCAAGTGCTGTAAACACCCAGGCGGGGGAGGCTCCTGA
95. SEQ ID NO: 46 (Signal Sequence underlined) 
ATMAVMAPRTLVLLLSGALALTQTWAGGGGGSEAKRMPCAEDYLSVVLNQLCVLHEKT
PVSDRVTKCCTESLVNRRPCFSALEVDETYVPKEFNAETFTFHADICTLSEKERQIKKQT
ALVELVKHKPKATKEQLKAVMDDFAAFVEKCCKADDKETCFAEEGKKLVAASQAALGL
96. SEQ ID NO: 82 (Nucleic Acid sequence encoding protein of SEQ ID NO: 46) 
GCCACTATGGCTGTGATGGCACCTAGAACACTGGTCCTGCTGCTGAGCGGGGCA
CTGGCACTGACACAGACTTGGGCTGGAGGGGAGGGGGTCTGAGGCCAAAAGGATG
CCA
TGCGCTGAAGACTACCTGAGTGTGGTCCTGAACCAGCTGTGTGCTGCACGAGAAAA
CT
CCCGTGTCAGATCGCGTCACTAAGTGCTGTACCGAGAGCCTGGTGAACCGGAGACCCT
GC
TTCTCCGCCCTGGAGGTGGACGAAACATATGTCCCTAAAGAGTTTAATGCAGAAACCTT
C
ACATTTCACGCCGATATCTGTACACTGAGCGAGAAGGAACGACAGATTAAGAAACAGA
CT
GCCCTGGTGGAGCTGGTCAAGCATAAACCCAAGGCTACCAAAGAACAGCTGAAGGCAG
TG
 
ATGGACGATTTCGCCGCTTTTGTCGAGAAATGCTGTAAGGCCGACGATAAGGAAACAT
GC
TTCGCTGAGGAAGGCAAGAAACTGGTGGCAGCCTCTCAGGCTGCACTGGGGCTGTGA
97. SEQ ID NO: 47 (Signal Sequence underlined) 
ATMAVMAPRTLVLLLSGALALTQTWAGDAHKSEVAHRFKDLGEENFKALVLIAFAQYLQ
QCPFEDHVKLVNEVTEFAKTCVADESAENCDKSLHTLFGDKLCTVATLRETGGGGS
98. SEQ ID NO: 83 (Nucleic Acid sequence encoding protein of SEQ ID NO: 47) 
GCCACTATGGCTGTGATGGCTCCAAGAACACTGGTCCTGCTGCTGTCCGGGGCACTGGC
ACTGACTCAGACCTGGGCTGGGGATGCTCATAAATCTGAGGTCGCTCACCGGTTCAAGG
ACCTGGGGGAGGAAAACTTTAAAGCACTGGTGCTGATCGCCTTCGCTCAGTACCTGCAG
CAGTGCCCCTTTGAAGATCACGTGAAGCTGGTCAACGAAGTGACTGAGTTCGCCAAAA
CCTGCGTCGCAGACGAGAGCGCCGAAAATTGTGATAAGTCCCTGCATACACTGTTTGGC
GACAAACTGTGTACCGTGGCTACACTGAGAGAGACCGGCGGGGGAGGCAGCTGA
99. SEQ ID NO: 48 (Signal Sequence underlined) 
ATMAVMAPRTLVLLLSGALALTQTWAGGGGGSGEMADCCAKQEPERNECFLQHKDDNP
NLPRLVRPEVDVMCTAFHDNEETFLKKYLYEIARRHPYFYAPELLFFAKRYKAAFTECCQA
ADKAACLLPKLDELRDEGKASSAKQRLKCASLQKFGERAFKAWAVARLSQRFPKAEFAEV
SKLVTDLTKVHTECCHGDLLECADDRADLAKYICENQDSISSKLKECCEKPLLEKSHCIAEV
ENDEMPADLPSLAADFVESKDVCKNYAEAKDVFLGMFLYEYARRHPDYSVVLLLRLAKT
YETTLEKCCAAADPHECYAKVFDEFKPLVEEPQNLIKQNCELFEQLGEYKFQNALLVRYTK
KVPQVSTPTLVEVSRNLGKVGSKCCKHPEAKRMPCAEDYLSVVLNQLCVLHEKTPVSDRV
TKCCTESLVNRRPCFSALEVDETYVPKEFNAETFTFHADICTLSEKERQIKKQTALVELVKH
KPKATKEQLKAVMDDFAAFVEKCCKADDKETCFAEEGKKLVAASQAALGL
100. SEQ ID NO: 84 (Nucleic Acid sequence encoding protein of SEQ ID NO: 48) 
GCCACTATGGCTGTGATGGCACCTAGAACCCTGGTCCTGCTGCTGAGCGGGGCA
CTGGCACTGACACAGACTTGGGCTGGGGGGGGGGGGGCAGCGGCGAGATGGCTGACT
GC
TGTGCAAAACAGGAGCCAGAAAGGAACGAATGCTTCCTGCAGCACAAGGACGATAACC
CC
AATCTGCCTAGACTGGTGAGGCCCGAGGTGGACGTCATGTGTACAGCCTTCCACGATAA
T
GAGGAAACTTTTCTGAAGAAATACCTGTATGAGATCGCCCGGAGACATCCTTACTTCTA
 
T
GCACCAGAACTGCTGTTCTTTGCCAAACGCTACAAGGCCGCTTTTACCGAGTGCTGTCA
G
GCAGCCGATAAAGCTGCATGCCTGCTGCCAAAGCTGGACGAGCTGCGAGATGAAGGGA
AG
GCTAGCTCCGCAAAACAGAGACTGAAGTGTGCTAGCCTGCAGAAATTCGGAGAGCGAG
CC
TTCAAGGCATGGGCAGTGGCTCGACTGTCCCAGCGATTCCCTAAGGCCGAGTTTGCTGA
A
GTGTCTAAACTGGTCACCGACCTGACAAAGGTGCACACCGAGTGCTGTCATGGCGACCT
G
CTGGAATGCGCCGACGATCGCGCAGATCTGGCCAAGTACATCTGTGAGAACCAGGACA
GC
ATTTCTAGTAAGCTGAAAGAGTGCTGTGAAAAACCTCTGCTGGAGAAGTCCCACTGCAT
C
GCCGAGGTGGAAAACGACGAAATGCCAGCTGATCTGCCCTCACTGGCCGCTGACTTTGT
G
GAGAGCAAAGATGTCTGTAAGAATTACGCAGAAGCCAAGGATGTGTTCCTGGGCATGT
TT
CTGTACGAGTATGCCAGGCGCCACCCTGACTACTCCGTGGTCCTGCTGCTGAGGCTGGC
T
AAAACCTATGAGACCACACTGGAAAAGTGCTGTGCAGCCGCTGATCCACATGAGTGCT
AT
GCCAAAGTGTTCGACGAGTTCAAGCCCCTGGTCGAGGAACCTCAGAACCTGATCAAGC
AG
AATTGTGAGCTGTTCGAACAGCTGGGGGAGTACAAGTTTCAGAACGCCCTGCTGGTGA
GA
TATACAAAGAAAGTGCCACAGGTCTCCACTCCCACCCTGGTGGAGGTCTCTAGGAATCT
G
GGCAAAGTGGGGAGTAAATGCTGTAAGCACCCTGAGGCCAAGCGCATGCCATGCGCTG
AA
GATTACCTGAGTGTGGTCCTGAATCAGCTGTGTGTGCTGCATGAGAAAACACCAGTGTC
A
GACCGGGTCACTAAGTGCTGTACCGAATCACTGGTGAACCGACGACCATGCTTCAGCG
CA
CTGGAGGTGGATGAAACTTATGTCCCTAAGGAGTTTAATGCTGAAACATTCACTTTTCA
C
GCAGACATCTGCACCCTGTCTGAGAAGGAAAGACAGATTAAGAAACAGACAGCCCTGG
TG
GAGCTGGTCAAGCATAAACCCAAGGCCACTAAAGAACAGCTGAAGGCTGTGATGGACG
AT
TTCGCAGCCTTTGTCGAGAAATGCTGTAAGGCAGACGATAAGGAAACCTGCTTCGCCG
AG
GAAGGAAAGAAACTGGTGGCTGCAAGTCAGGCCGCTCTGGGCCTGTGA
101. SEQ ID NO: 49 (Signal Sequence underlined) 
ATMAVMAPRTLVLLLSGALALTQTWAGDAHKSEVAHRFKDLGEENFKALVLIAFAQYLQ
QCPFEDHVKLVNEVTEFAKTCVADESAENCDKSLHTLFGDKLCTVATLRETYGEMADCCA
KQEPERNECFLQHKDDNPNLPRLVRPEVDVMCTAFHDNEETFLKKYLYEIARRHPYFYAPE
LLFFAKRYKAAFTECCQAGGGGS
102. SEQ ID NO: 85 (Nucleic Acid sequence encoding protein of SEQ ID NO: 49) 
GCCACTATGGCCGTGATGGCACCTCGAACACTGGTCCTGCTGCTGTCCGGGGCA
CTGGCACTGACTCAGACCTGGGCTGGAGATGCTCATAAATCTGAGGTCGCTCACCGGTT
C
AAGGACCTGGGCGAGGAAAACTTTAAAGCACTGGTGCTGATCGCATTCGCCCAGTACC
TG
CAGCAGTGCCCCTTTGAGGATCACGTGAAGCTGGTCAACGAAGTGACTGAGTTCGCAA
AA
ACCTGCGTCGCTGACGAGAGCGCAGAAAATTGTGATAAGTCCCTGCATACCCTGTTTGG
G
GACAAACTGTGTACCGTGGCTACACTGCGAGAGACATATGGAGAAATGGCCGATTGCT
GT
GCTAAGCAGGAGCCTGAAAGAAACGAGTGCTTCCTGCAGCATAAAGACGATAACCCCA
AT
CTGCCTAGGCTGGTGCGCCCAGAAGTGGACGTCATGTGTACAGCCTTCCACGATAATGA
G
GAAACTTTTCTGAAGAAATACCTGTATGAGATTGCTCGGAGACATCCATACTTTTATGC
A
CCCGAACTGCTGTTCTTTGCCAAGCGGTACAAAGCCGCTTTCACCGAGTGCTGTCAGGC
C
GGCGGGGGAGGCAGCTGA
103. SEQ ID NO: 50 (Signal Sequence underlined) 
ATMAVMAPRTLVLLLSGALALTQTWAGGGGGSADKAACLLPKLDELRDEGKASSAKQR
LKCASLQKFGERAFKAWAVARLSQRFPKAEFAEVSKLVTDLTKVHTECCHGDLLECADDR
 
ADLAKYICENQDSISSKLKECCEKPLLEKSHCIAEVENDEMPADLPSLAADFVESKDVCK
NYAEAKDVFLGMFLYEYARRHPDYSVVLLLRLAKTYETTLEKCCAAADPHECYAKVFDEF
KPLVEEPQNLIKQNCELFEQLGEYKFQNALLVRYTKKVPQVSTPTLVEVSRNLGKVGSKC
CKHPEAKRMPCAEDYLSVVLNQLCVLHEKTPVSDRVTKCCTESLVNRRPCFSALEVDETY
VPKEFNAETFTFHADICTLSEKERQIKKQTALVELVKHKPKATKEQLKAVMDDFAAFVEK
CCKADDKETCFAEEGKKLVAASQAALGL
104. SEQ ID NO: 86 (Nucleic Acid sequence encoding protein of SEQ ID NO: 50) 
GCCACTATGGCTGTGATGGCTCCTAGAACCCTGGTCCTGCTGCTGTCCGGGGCACTGGC
ACTGACTCAGACTTGGGCTGGGGGGGGGGGGGTCTGCCGATAAAGCCGCTTGCCTG
CTGCCCAAGCTGGACGAGCTGAGAGATGAAGGAAGGCCAGCTCCGCTAAACAGAGG
CTGAAGTGTGCAAGTCTGCAGAAATTCGGAGAGAGGGCCTTTAAGGCTTGGGCAGTGG
CACGACTGTCCCAGCGATTCCCTAAGGCAGAGTTTGCCGAAGTGTCTAAACTGGTCACC
GACCTGACAAAGGTGCACACCGAGTGCTGTCATGGCGACCTGCTGGAATGCGCCGACG
ATCGCGCTGATCTGGCAAAGTACATCTGTGAGAACCAGGACAGCATTTCTAGTAAGCTG
AAAGAGTGCTGTGAAAAACCTCTGCTGGAGAAGTCCCACTGCATCGCTGAGGTGGAAA
ACGACGAAATGCCCGCAGATCTGCCTTCACTGGCAGCCGACTTCGTGGAGAGCAAAGA
TGTCTGTAAG
AATTACGCTGAAGCAAAGGATGTGTTCCTGGGCATGTTTCTGTACGAGTATGCCCGGAG
ACACCCTGACTACTCCGTGGTCCTGCTGCTGAGGCTGGCTAAAACATATGAGACCACAC
TGGAAAAGTGCTGTGCTGCAGCCGATCCACATGAGTGCTATGCCAAAGTGTTCGACGA
GTTCAAGCCACTGGTCGAGGAACCCCAGAACCTGATCAAGCAGAATTGTGAGCTGTTC
GAACAGCTGGGGGAGTACAAGTTTCAGAACGCCCTGCTGGTGCGGTATACTAAGAAAG
TGCCTCAGGTCTCCACTCCAACCCTGGTGGAGGTCTCTCGCAATCTGGGCAAAGTGGGG
AGTAAATGCTGTAAGCACCCCGAGGCCAAGCGAATGCCTTGCGCTGAAGATTACCTGA
GTGTGGTCCTGAATCAGCTGTGTGTGCTGCATGAGAAAACTCCAGTGTCAGACCGGGTC
ACTAAGTGCTGTACCGAGTCACTGGTGAACAGGCGACCATGCTTCAGCGCACTGGAGG
TGGATGAAACCTATGTCCCCAAGGAGTTTAATGCAGAAACATTCACTTTCCACGCCGAC
ATCTGTACACTGAGC
GAGAAGGAAAGACAGATTAAGAAACAGACTGCCCTGGTGGAGCTGGTCAAGCATAAA
CCCAAGGCTACCAAAGAACAGCTGAAGGCAGTGATGGACGATTTCGCTGCATTTGTCG
AGAAATGCTGTAAGGCCGACGATAAGGAAACATGCTTTGCTGAGGAAGGAAAGAAAC
TGGTGGCCGCTAGCCAGGCAGCCCTGGGCCTGTGA
105. SEQ ID NO: 51 (Signal Sequence underlined) 
ATMAVMAPRTLVLLLSGALALTQTWAGDAHKSEVAHRFKDLGEENFKALVLIAFAQYL
QQCPFEDHVKLVNEVTEFAKTCVADESAENCDKSLHTLFGDKLCTVATLRETYGEMADCC
 
AKQEPERNECFLQHKDDNPNLPRLVRPEVDVMCTAFHDNEETFLKKYLYEIARRHPYFYA
PELLFFAKRYKAAFTECCQAADKAACLLPKLDELRDEGKASSAKQRLKCASLQKFGERAF
KAWAVARLSQRFPKAEFAEVSKLVTDLTKVHTECCHGDLLECADDRADLAKYICENQDSI
SSKLKECCEKGGGGS
106. SEQ ID NO: 87 (Nucleic Acid sequence encoding protein of SEQ ID NO: 51) 
GCCACTATGGCCGTGATGGCACCTAGAACCCTGGTCCTGCTGCTGAGCGGGGCA
CTGGCACTGACACAGACTTGGGCTGGGATGCACATAAAAGCGAGGTCGCTCACCGGT
TC
AAGGATCTGGGCGAGGAAAACTTTAAAGCACTGGTGCTGATCGCCTTCGCTCAGTACCT
G
CAGCAGTGCCCCTTTGAAGACCACGTGAAGCTGGTCAACGAGGTGACAGAGTTCGCCA
AA
ACTTGCGTCGCAGACGAGTCAGCCGAAAATTGTGATAAGAGCCTGCATACTCTGTTTGG
G
GATAAACTGTGTACCGTGGCCACACTGCGCGAGACCTATGGAGAAATGGCTGACTGCT
GT
GCAAAGCAGGAGCCTGAACGAAACGAGTGCTTCCTGCAGCATAAAGACGATAACCCCA
AT
CTGCCTAGGCTGGTGCGCCCAGAAGTGGACGTCATGTGTACCGCTTTCCACGATAATGA
G
GAAACATTTCTGAAGAAATACCTGTATGAGATTGCCCGGAGACATCCATACTTTTATGC
C
CCCGAACTGCTGTTCTTTGCTAAGAGATACAAAGCCGCTTTCACAGAGTGCTGTCAGGC
A
GCCGATAAGGCTGCATGCCTGCTGCCCAAACTGGACGAGCTGAGAGATGAAGGCAAGG
CA
AGCTCCGCCAAGCAGAGGCTGAAATGTGCATCTCTGCAGAAGTTCGGGGAGAGGGCCT
TT
AAAGCATGGGCAGTGGCTCGACTGTCCCAGCGATTCCCTAAGGCTGAGTTTGCAGAAG
TC
 
TCTAAGCTGGTGACTGACCTGACCAAAGTGCACACCGAGTGCTGTCATGGCGACCTGCT
G
GAATGCGCCGACGATCGCGCCGATCTGGCTAAGTATATCTGTGAGAATCAGGACAGTA
TT
TCTAGTAAGCTGAAAGAGTGCTGTGAAAAGGGCGGGGAGGCTCCTGA
107. SEQ ID NO: 52 (Signal Sequence underlined) 
ATMAVMAPRTLVLLLSGALALTQTWAGGGGGSPLLEKSHCIAEVENDEMPADLPSLAA
DFVESKDVCKNYAEAKDVFLGMFLYEYARRHPDYSVVLLLRLAKTYETTLEKCCAAADPH
ECYAKVFDEFKPLVEEPQNLIKQNCELFEQLGEYKFQNALLVRYTKKVPQVSTPTLVEVS
RNLGKVGSKCCKHPEAKRMPCAEDYLSVVLNQLCVLHEKTPVSDRVTKCCTESLVNRRPC
FSALEVDETYVPKEFNAETFTFHADICTLSEKERQIKKQTALVELVKHKPKATKEQLKAV
MDDFAAFVEKCCKADDKETCFAEEGKKLVAASQAALGL
108. SEQ ID NO: 88 (Nucleic Acid sequence encoding protein of SEQ ID NO: 52) 
GCCACTATGGCTGTGATGGCTCCAAGAACCCTGGTGCTGCTGCTGTCCGGGGCT
CTGGCTCTGACTCAGACCTGGGCCGGGGGGGGGGGGAAGCCCCCTGCTGGAGAAGT
CC
CACTGCATCGCCGAGGTGGAAAACGACGAAATGCCCGCTGATCTGCCTTCTCTGGCCGC
T
GACTTCGTGGAGAGTAAAGATGTCTGTAAGAATTACGCAGAAGCCAAGGACGTGTTCC
TG
GGGATGTTTCTGTACGAGTATGCACGGAGACACCCTGATTACTCCGTGGTCCTGCTGCT
G
CGCCTGGCCAAAACTTATGAGACCACACTGGAAAAGTGCTGTGCAGCCGCTGACCCAC
AT
GAGTGCTATGCTAAAGTGTTCGATGAGTTCAAGCCACTGGTCGAGGAACCCCAGAACC
TG
ATCAAGCAGAATTGTGAGCTGTTCGAACAGCTGGGCGAGTACAAGTTTCAGAACGCCC
TG
 
CTGGTGCGATATACAAAGAAAGTGCCTCAGGTCTCAACTCCAACCCTGGTGGAGGTCA
GC
CGAAATCTGGGCAAAGTGGGGTCCAAATGCTGTAAGCACCCCGAGGCTAAGCGGATGC
CT
TGCGCAGAAGACTACCTGTCAGTGGTCCTGAACCAGCTGTGTGTGCTGCATGAGAAAA
CC
CCCGTGAGCGATAGAGTCACCAAGTGCTGTACAGAGTCTCTGGTGAACAGGCGCCCAT
GC
TTCAGTGCCCTGGAGGTGGACGAAACATATGTCCCCAAAGAGTTTAATGCCGAAACATT
C
ACTTTTCACGCTGATATCTGTACTCTGTCCGAGAAGGAAAGGCAGATTAAGAAACAGA
CC
GCCCTGGTGGAGCTGGTCAAGCATAAACCTAAGGCAACAAAAGAACAGCTGAAGGCCG
TG
ATGGACGATTTCGCAGCCTTTGTCGAGAAATGCTGTAAGGCTGACGATAAGGAAACTT
GC
TTTGCAGAGGAAGGAAAGAAACTGGTGGCTGCATCTCAGGCCGCTCTGGGCCTGTGA
109. SEQ ID NO: 53 (Signal Sequence underlined) 
ATMAVMAPRTLVLLLSGALALTQTWAGDAHKSEVAHRFKDLGEENFKALVLIAFAQYL
QQCPFEDHVKLVNEVTEFAKTCVADESAENCDKSLHTLFGDKLCTVATLRETYGEMADCC
AKQEPERNECFLQHKDDNPNLPRGGGGS
110. SEQ ID NO: 89 (Nucleic Acid sequence encoding protein of SEQ ID NO: 53) 
GCCACTATGGCTGTCATGGCTCCAAGAACACTGGTCCTGCTGCTGTCCGGGGCA
CTGGCACTGACTCAGACCTGGGCTGGGGATGCTCACAAGTCTGAGGTCGCCCACAGGTT
C
AAGGACCTGGGCGAGGAAAACTTTAAAGCTCTGGTGCTGATCGCCTTCGCTCAGTACCT
G
CAGCAGTGCCCATTTGAAGATCACGTGAAGCTGGTCAACGAAGTGACTGAGTTCGCCA
 
AA
ACCTGCGTCGCAGACGAGAGCGCCGAAAATTGTGATAAGTCCCTGCATACACTGTTTGG
G
GACAAACTGTGCACCGTGGCCACACTGCGGGAGACCTATGGAGAAATGGCTGATTGCT
GT
GCAAAGCAGGAGCCCGAACGGAATGAGTGTTTCCTGCAGCATAAAGACGATAACCCCA
AT
CTGCCTAGAGGCGGGGGAGGCAGCTGA
111. SEQ ID NO: 54 (Signal Sequence underlined) 
ATMAVMAPRTLVLLLSGALALTQTWAGGGGGSLVRPEVDVMCTAFHDNEETFLKKYLYE
IARRHPYFYAPELLFFAKRYKAAFTECCQAADKAACLLPKLDELRDEGKASSAKQRLKCAS
LQKFGERAFKAWAVARLSQRFPKAEFAEVSKLVTDLTKVHTECCHGDLLECADDRADLAK
YICENQDSISSKLKECCEKPLLEKSHCIAEVENDEMPADLPSLAADFVESKDVCKNYAEAKD
VFLGMFLYEYARRHPDYSVVLLLRLAKTYETTLEKCCAAADPHECYAKVFDEFKPLVEEP
QNLIKQNCELFEQLGEYKFQNALLVRYTKKVPQVSTPTLVEVSRNLGKVGSKCCKHPEAKR
MPCAEDYLSVVLNQLCVLHEKTPVSDRVTKCCTESLVNRRPCFSALEVDETYVPKEFNAET
FTFHADICTLSEKERQIKKQTALVELVKHKPKATKEQLKAVMDDFAAFVEKCCKADDKET
CFAEEGKKLVAASQAALGL
112. SEQ ID NO: 90 (Nucleic Acid sequence encoding protein of SEQ ID NO: 54) 
GCCACTATGGCTGTGATGGCACCTAGAACACTGGTCCTGCTGCTGAGCGGGGCA
CTGGCACTGACACAGACTTGGGCCGGGGGAGGGGGAGGGAGCCTGGTGAGGCCCGAG
GTG
GACGTCATGTGCACAGCCTTCCACGATAACGAGGAAACTTTTCTGAAGAAATACCTGTA
T
GAGATCGCCCGGAGACATCCATACTTCTATGCCCCCGAACTGCTGTTCTTTGCTAAACG
C
TACAAGGCCGCTTTTACCGAGTGCTGTCAGGCAGCCGATAAAGCTGCATGCCTGCTGCC
A
AAGCTGGACGAGCTGCGCGATGAAGGGAAGGCTAGCTCCGCAAAACAGCGACTGAAG
 
TGT
GCAAGCCTGCAGAAATTCGGAGAGCGAGCTTTTAAGGCATGGGCCGTGGCTAGACTGT
CC
CAGAGGTTCCCCAAGGCCGAGTTTGCTGAAGTGTCTAAACTGGTCACCGACCTGACAA
AG
GTGCACACCGAGTGCTGTCATGGCGACCTGCTGGAATGCGCCGACGATCGCGCAGATC
TG
GCCAAGTACATCTGTGAGAATCAGGACAGCATTTCTAGTAAGCTGAAAGAGTGCTGTG
AA
AAACCTCTGCTGGAGAAGTCCCACTGCATCGCCGAGGTGGAAAACGACGAAATGCCCG
CT
GATCTGCCTTCACTGGCCGCTGACTTCGTGGAGAGCAAAGATGTCTGTAAGAATTACGC
A
GAAGCCAAGGATGTGTTCCTGGGCATGTTTCTGTACGAGTATGCAAGGCGACACCCAG
AC
TACTCCGTGGTCCTGCTGCTGAGGCTGGCTAAAACCTATGAGACCACACTGGAAAAGTG
C
TGTGCAGCCGCTGATCCTCATGAGTGCTATGCCAAAGTGTTCGACGAGTTCAAGCCTCT
G
GTCGAGGAACCACAGAACCTGATCAAGCAGAATTGTGAGCTGTTCGAACAGCTGGGGG
AG
TACAAGTTTCAGAACGCCCTGCTGGTGCGCTATACAAAGAAAGTGCCCCAGGTCTCCAC
T
CCTACCCTGGTGGAGGTCTCTCGGAATCTGGGCAAAGTGGGGAGTAAATGCTGTAAGC
AC
CCAGAGGCTAAGAGAATGCCCTGCGCAGAAGATTACCTGAGTGTGGTCCTGAACCAGC
TG
TGTGTGCTGCATGAGAAAACACCTGTGTCAGACCGGGTCACTAAGTGCTGTACCGAATC
A
CTGGTGAACCGACGGCCTTGCTTCAGCGCCCTGGAGGTGGATGAAACTTATGTCCCAA
AG
 
GAGTTTAATGCAGAAACATTCACTTTTCACGCCGACATCTGTACCCTGTCTGAGAAGGA
A
AGACAGATTAAGAAACAGACAGCCCTGGTGGAGCTGGTCAAGCATAAACCAAAGGCTA
CT
AAAGAACAGCTGAAGGCAGTGATGGACGATTTCGCAGCCTTTGTCGAGAAATGCTGTA
AG
GCCGACGATAAGGAAACCTGCTTCGCTGAGGAAGGAAAGAAACTGGTGGCTGCAAGTC
AG
GCCGCTCTGGGCCTGTGA
113. SEQ ID NO: 131 (CD19_scFv_BiTE AA) 
DIQLTQSPASLAVSLGQRATISCKASQSVDYDGDSYLNWYQQIPGQPPKLLIYDASNLVSGIP
PRFSGSGSGTDFTLNIHPVEKVDAATYHCQQSTEDPWTFGGGTKLEIKGGGGSGGGGSGGG
GSQVQLQQSGAELVRPGSSVKISCKASGYAFSSYWMNWVKQRPGQGLEWIGQIWPGDGD
TNYNGKFKGKATLTADESSSTAYMQLSSLASEDSAVYFCARRETTTVGRYYYAMDYWGQ
GTTVTVSS
114. SEQ ID NO: 132 (CD3_scFv_VH-VL_BiTE AA) 
DIKLQQSGAELARPGASVKMSCKTSGYTFTRYTMHWVKQRPGQGLEWIGYINPSRGYTNY
NQKFKDKATLTTDKSSSTAYMQLSSLTSEDSAVYYCARYYDDHYCLDYWGQGTTLTVSSV
EGGSGGSGGSGGSGGVDDIQLTQSPAIMSASPGEKVTMTCRASSSVSYMNWYQQKSGTSP
KRWIYDTSKVASGVPYRFSGSGSGTSYSLTISSMEAEDAATYYCQQWSSNPLTFGAGTKLE
LK
115. SEQ ID NO: 133 (V1094 NT) 
GAATTCGCGACCATGGCCGTGATGGCACCTAGAACACTGGTCCTGCTGCTGAGCGGGG
CA
CTGGCACTGACACAGACTTGGGCTGGAGACATTAAGCTGCAGCAGAGCGGAGCTGAGC
TG
GCACGACCAGGAGCAAGTGTGAAAATGTCATGCAAGACAAGCGGCTATACTTTTACCA
GG
TACACTATGCACTGGGTGAAGCAGCGACCAGGACAGGGACTGGAGTGGATCGGGTACA
TT
AACCCTTCCAGAGGATATACCAACTACAATCAGAAGTTCAAAGATAAGGCCACCCTGA
CC
ACAGACAAGAGCTCCTCTACAGCCTATATGCAGCTGAGTTCACTGACTTCCGAGGATTC
T
GCCGTGTACTATTGCGCTAGGTACTATGACGATCATTATTGTCTGGACTACTGGGGGCA
 
G
GGAACTACCCTGACAGTGAGCTCCGTCGAGGGAGGGAGTGGAGGCTCAGGAGGAAGC
GGA
GGGTCCGGAGGAGTGGACGATATCCAGCTGACTCAGTCTCCTGCAATTATGTCAGCCAG
C
CCAGGCGAGAAGGTCACAATGACTTGCCGCGCCTCTAGTTCAGTGTCCTATATGAATTG
G
TACCAGCAGAAATCTGGCACCAGTCCCAAGAGATGGATCTATGATACATCCAAGGTCG
CC
TCTGGGGTGCCTTACAGGTTTTCCGGCTCTGGGAGTGGAACTTCATATAGCCTGACCAT
T
AGCTCCATGGAGGCTGAAGACGCCGCTACCTACTATTGCCAGCAGTGGTCTAGTAACCC
C
CTGACATTCGGCGCCGGGACTAAACTGGAGCTGAAGGGGGGATCTGACGCACACAAAA
GT
GAAGTCGCCCATCGGTTCAAGGATCTGGGCGAGGAAAATTTTAAAGCCCTGGTGCTGA
TC
GCCTTCGCTCAGTACCTGCAGCAGTGCCCTTTTGAGGACCACGTGAAGCTGGTCAACGA
G
GTGACCGAGTTCGCTAAAACATGCGTGGCTGATGAGTCTGCAGAAAATTGTGACAAGA
GT
CTGCATACACTGTTTGGGGATAAACTGTGTACCGTGGCCACACTGCGCGAGACTTACGG
A
GAAATGGCCGACTGCTGTGCTAAGCAGGAGCCTGAACGAAACGAGTGCTTCCTGCAGC
AC
AAAGACGATAACCCTAATCTGCCACGCCTGGTGCGACCAGAAGTGGATGTCATGTGTA
CT
GCTTTCCACGACAATGAGGAAACCTTTCTGAAGAAATATCTGTACGAGATCGCCCGGA
GA
CATCCATATTTTTACGCACCCGAACTGCTGTTCTTTGCCAAAAGATACAAGGCAGCCTT
C
ACCGAGTGCTGTCAGGCTGCAGATAAAGCCGCTTGCCTGCTGCCTAAGCTGGATGAGCT
G
CGAGACGAAGGAAAGGCCTCAAGCGCTAAACAGCGGCTGAAGTGTGCTAGCCTGCAGA
AA
TTCGGAGAGCGAGCCTTCAAGGCATGGGCAGTGGCTCGGCTGTCTCAGAGATTCCCAA
AG
GCAGAGTTTGCCGAAGTCAGCAAACTGGTGACTGACCTGACCAAGGTGCACACCGAGT
GC
TGTCATGGCGATCTGCTGGAATGCGCCGACGATAGAGCTGACCTGGCAAAGTACATCT
GT
GAGAACCAGGATAGCATTTCCTCTAAACTGAAGGAGTGCTGTGAAAAACCTCTGCTGG
AG
AAGTCCCACTGCATCGCCGAGGTGGAAAACGATGAAATGCCCGCTGACCTGCCTTCACT
G
GCAGCCGATTTCGTCGAGAGCAAAGACGTGTGTAAGAATTACGCAGAAGCCAAGGATG
 
TG
TTCCTGGGCATGTTTCTGTATGAGTACGCTAGGCGACACCCAGACTACAGCGTGGTCCT
G
CTGCTGCGGCTGGCCAAGACATATGAGACAACTCTGGAAAAATGCTGTGCTGCAGCCG
AC
CCTCATGAGTGCTATGCCAAAGTCTTCGATGAGTTCAAGCCTCTGGTGGAGGAACCACA
G
AACCTGATTAAGCAGAATTGTGAGCTGTTTGAACAGCTGGGCGAGTATAAATTCCAGA
AC
GCCCTGCTGGTGAGATACACCAAGAAAGTCCCCCAGGTGTCCACCCCTACACTGGTGG
AA
GTCTCTCGGAATCTGGGAAAGGTCGGCAGTAAATGCTGTAAGCACCCAGAGGCTAAAA
GA
ATGCCCTGCGCAGAAGATTACCTGTCCGTGGTCCTGAACCAGCTGTGTGTGCTGCATGA
G
AAGACACCAGTCTCTGACAGGGTGACCAAATGCTGTACAGAATCCCTGGTGAACCGAC
GG
CCTTGCTTTTCTGCCCTGGAGGTCGATGAAACCTATGTGCCAAAGGAGTTCAATGCTGA
A
ACTTTCACCTTTCACGCAGACATCTGTACTCTGAGCGAGAAAGAACGCCAGATTAAGAA
A
CAGACCGCCCTGGTCGAGCTGGTGAAACATAAGCCCAAAGCCACAAAAGAACAGCTGA
AG
GCTGTCATGGACGATTTCGCTGCATTTGTGGAGAAATGCTGTAAGGCAGACGATAAGG
AA
ACCTGCTTCGCCGAGGAAGGCAAGAAACTGGTGGCCGCTAGCCAGGCAGCACTGGGAC
TG
GGAGGGTCCGGGGATATCCAGCTGACCCAGTCACCAGCCAGCCTGGCTGTCTCACTGG
GG
CAGAGGGCCACAATTAGTTGTAAGGCTTCCCAGTCTGTGGACTACGATGGAGACTCCTA
T
CTGAACTGGTACCAGCAGATCCCAGGACAGCCACCTAAACTGCTGATCTACGACGCCA
GT
AATCTGGTGTCAGGCATCCCACCCCGCTTTAGTGGATCAGGCAGCGGGACAGACTTCAC
T
CTGAACATTCACCCAGTCGAGAAGGTGGATGCTGCAACCTACCATTGCCAGCAGAGCA
CT
GAGGACCCCTGGACCTTTGGAGGCGGGACAAAACTGGAAATCAAGGGAGGCGGGGGA
TCA
GGCGGAGGAGGCAGCGGAGGAGGAGGGTCCCAGGTGCAGCTGCAGCAGTCCGGAGCA
GAG
CTGGTCAGGCCAGGGAGTTCAGTGAAAATTAGCTGTAAGGCATCCGGGTATGCCTTCA
GC
TCCTACTGGATGAATTGGGTCAAACAGAGACCTGGCCAGGGCCTGGAGTGGATCGGAC
AG
ATTTGGCCCGGGGATGGAGACACTAACTACAATGGGAAGTTTAAAGGAAAGGCTACAC
 
TG
ACTGCAGATGAATCTAGTTCAACCGCCTACATGCAGCTGAGCTCCCTGGCATCCGAGGA
C
TCTGCCGTCTATTTCTGCGCTAGAAGGGAAACCACAACTGTGGGCAGGTACTATTACGC
C
ATGGACTATTGGGGCCAGGGGACCACAGTGACCGTCTCTAGTTGAGGATCC
116. SEQ ID NO: 134 (V1094 AA) 
EFATMAVMAPRTLVLLLSGALALTQTWAGDIKLQQSGAELARPGASVKMSCKTSGYTFTR
YTMHWVKQRPGQGLEWIGYINPSRGYTNYNQKFKDKATLTTDKSSSTAYMQLSSLTSEDS
AVYYCARYYDDHYCLDYWGQGTTLTVSSVEGGSGGSGGSGGSGGVDDIQLTQSPAIMSAS
PGEKVTMTCRASSSVSYMNWYQQKSGTSPKRWIYDTSKVASGVPYRFSGSGSGTSYSLTIS
SMEAEDAATYYCQQWSSNPLTFGAGTKLELKGGSDAHKSEVAHRFKDLGEENFKALVLIA
FAQYLQQCPFEDHVKLVNEVTEFAKTCVADESAENCDKSLHTLFGDKLCTVATLRETYGE
MADCCAKQEPERNECFLQHKDDNPNLPRLVRPEVDVMCTAFHDNEETFLKKYLYEIARRH
PYFYAPELLFFAKRYKAAFTECCQAADKAACLLPKLDELRDEGKASSAKQRLKCASLQKF
GERAFKAWAVARLSQRFPKAEFAEVSKLVTDLTKVHTECCHGDLLECADDRADLAKYICE
NQDSISSKLKECCEKPLLEKSHCIAEVENDEMPADLPSLAADFVESKDVCKNYAEAKDVFL
GMFLYEYARRHPDYSVVLLLRLAKTYETTLEKCCAAADPHECYAKVFDEFKPLVEEPQNLI
KQNCELFEQLGEYKFQNALLVRYTKKVPQVSTPTLVEVSRNLGKVGSKCCKHPEAKRMPC
AEDYLSVVLNQLCVLHEKTPVSDRVTKCCTESLVNRRPCFSALEVDETYVPKEFNAETFTF
HADICTLSEKERQIKKQTALVELVKHKPKATKEQLKAVMDDFAAFVEKCCKADDKETCFA
EEGKKLVAASQAALGLGGSGDIQLTQSPASLAVSLGQRATISCKASQSVDYDGDSYLNWY
QQIPGQPPKLLIYDASNLVSGIPPRFSGSGSGTDFTLNIHPVEKVDAATYHCQQSTEDPWTFG
GGTKLEIKGGGGSGGGGSGGGGSQVQLQQSGAELVRPGSSVKISCKASGYAFSSYWMNW
VKQRPGQGLEWIGQIWPGDGDTNYNGKFKGKATLTADESSSTAYMQLSSLASEDSAVYFC
ARRETTTVGRYYYAMDYWGQGTTVTVSS*GS
117. SEQ ID NO: 135 (V1095) 
GAATTCGCGACCATGGCCGTGATGGCACCTAGAACACTGGTCCTGCTGCTGAGCGGGG
CA
CTGGCACTGACACAGACTTGGGCTGGAGACATTCAGCTGACACAGTCCCCAGCTTCTCT
G
GCAGTCTCACTGGGCCAGCGGGCAACTATCAGCTGCAAGGCCTCACAGAGCGTGGACT
AC
GATGGAGACAGCTATCTGAACTGGTACCAGCAGATCCCAGGACAGCCACCTAAACTGC
TG
ATCTACGACGCCAGTAATCTGGTGTCAGGCATCCCACCCAGATTTTCCGGATCTGGCAG
T
GGGACCGACTTCACACTGAACATTCACCCAGTGGAGAAGGTCGATGCCGCTACCTACC
AT
TGCCAGCAGAGCACTGAGGACCCCTGGACCTTTGGCGGGGGAACAAAACTGGAAATCA
AG
GGCGGAGGAGGCTCTGGAGGAGGAGGGAGTGGAGGAGGAGGATCACAGGTGCAGCTG
 
CAG
CAGTCCGGAGCAGAGCTGGTCAGGCCAGGCAGCTCCGTGAAAATTAGCTGTAAGGCAT
CC
GGGTATGCCTTCTCTAGTTACTGGATGAATTGGGTGAAGCAGCGACCAGGACAGGGAC
TG
GAGTGGATCGGACAGATTTGGCCTGGGGATGGAGACACTAACTACAATGGGAAGTTTA
AA
GGAAAGGCTACTCTGACCGCAGATGAATCAAGCTCCACCGCCTACATGCAGCTGTCTA
GT
CTGGCCAGTGAGGACTCAGCTGTCTATTTCTGCGCACGGAGAGAAACCACAACTGTGG
GC
CGATACTATTACGCCATGGATTACTGGGGCCAGGGGACCACAGTGACCGTCTCAAGCG
GC
GGGAGCGATGCTCACAAATCCGAGGTCGCACATAGATTCAAGGACCTGGGGGAGGAAA
AC
TTTAAAGCCCTGGTGCTGATCGCCTTCGCTCAGTATCTGCAGCAGTGCCCCTTTGAAGA
C
CACGTGAAGCTGGTCAACGAAGTGACTGAGTTCGCTAAAACCTGCGTGGCCGATGAGT
CT
GCTGAAAATTGTGACAAGAGTCTGCATACCCTGTTTGGGGATAAACTGTGTACTGTGGC
C
ACCCTGCGCGAGACATACGGAGAAATGGCAGACTGCTGTGCCAAGCAGGAGCCTGAAC
GA
AACGAGTGCTTCCTGCAGCACAAAGACGATAACCCTAATCTGCCACGCCTGGTGCGAC
CA
GAAGTGGATGTCATGTGTACTGCTTTCCACGACAATGAGGAAACCTTTCTGAAGAAATA
T
CTGTACGAGATTGCCAGGCGCCATCCATATTTTTACGCACCCGAACTGCTGTTCTTTGCC
AAAAGGTACAAGGCAGCCTTCACCGAGTGCTGTCAGGCTGCAGATAAAGCCGCTTGCC
TG
CTGCCTAAGCTGGATGAGCTGCGAGACGAAGGAAAGGCCTCCTCTGCTAAACAGCGGC
TG
AAGTGTGCTAGCCTGCAGAAATTCGGCGAGAGGGCCTTCAAGGCATGGGCAGTGGCTC
GA
CTGTCTCAGAGATTCCCAAAGGCAGAGTTTGCCGAAGTCAGCAAACTGGTGACAGACC
TG
ACTAAGGTGCACACCGAGTGCTGTCATGGCGATCTGCTGGAATGCGCCGACGATCGCG
CT
GACCTGGCAAAGTACATCTGTGAGAACCAGGATAGCATTAGTTCAAAACTGAAGGAGT
GC
TGTGAAAAACCTCTGCTGGAGAAGTCCCACTGCATCGCCGAGGTGGAAAACGATGAAA
TG
CCAGCTGACCTGCCTTCCCTGGCAGCAGATTTCGTCGAGTCTAAAGACGTGTGTAAGAA
T
TACGCAGAAGCCAAGGATGTGTTCCTGGGCATGTTTCTGTATGAGTACGCTCGACGGCA
C
 
CCCGACTACTCCGTGGTCCTGCTGCTGAGGCTGGCCAAGACATATGAGACTACCCTGGA
A
AAATGCTGTGCTGCAGCCGACCCTCATGAGTGCTATGCCAAAGTCTTCGATGAGTTCAA
G
CCTCTGGTGGAGGAACCACAGAACCTGATTAAGCAGAATTGTGAGCTGTTTGAACAGC
TG
GGCGAGTATAAATTCCAGAACGCCCTGCTGGTGCGCTACACCAAGAAAGTCCCCCAGG
TG
AGTACACCTACTCTGGTGGAAGTCTCACGGAATCTGGGAAAGGTCGGCAGTAAATGCT
GT
AAGCACCCAGAGGCCAAAAGAATGCCCTGCGCTGAAGATTACCTGTCCGTGGTCCTGA
AC
CAGCTGTGTGTGCTGCATGAGAAGACACCCGTCTCTGACCGGGTGACCAAATGCTGTAC
A
GAAAGCCTGGTGAACAGAAGGCCTTGCTTTTCCGCCCTGGAGGTCGATGAAACCTATGT
G
CCAAAGGAGTTCAATGCTGAAACCTTCACATTTCACGCAGACATCTGTACTCTGAGCGA
G
AAAGAAAGACAGATTAAGAAACAGACCGCCCTGGTCGAGCTGGTGAAACATAAGCCC
AAA
GCCACAAAAGAACAGCTGAAGGCTGTCATGGACGATTTCGCTGCATTTGTGGAGAAAT
GC
TGTAAGGCAGACGATAAGGAAACTTGCTTCGCCGAGGAAGGCAAGAAACTGGTGGCAG
CT
TCCCAGGCAGCACTGGGACTGGGAGGCTCCGGGGACATCAAGCTGCAGCAGTCTGGAG
CA
GAGCTGGCTAGGCCTGGAGCCTCTGTGAAAATGAGTTGTAAGACATCAGGCTATACTTT
T
ACCAGGTACACTATGCACTGGGTCAAACAGAGACCTGGCCAGGGCCTGGAGTGGATCG
GC
TACATTAATCCCAGCCGCGGGTATACCAACTACAATCAGAAGTTCAAAGATAAGGCAA
CC
CTGACAACTGACAAGAGCTCCTCTACAGCCTATATGCAGCTGAGTTCACTGACTAGCGA
G
GATTCCGCCGTGTATTACTGCGCTCGGTATTACGACGATCATTATTGTCTGGACTACTGG
GGGCAGGGAACCACACTGACAGTCAGCTCCGTGGAGGGAGGATCTGGAGGGAGTGGA
GGC
TCAGGAGGAAGCGGAGGGGTGGACGATATCCAGCTGACCCAGTCTCCTGCTATTATGT
CA
GCAAGCCCAGGCGAGAAGGTCACAATGACTTGCAGAGCCTCTAGTTCAGTGTCCTATAT
G
AATTGGTATCAGCAGAAATCCGGCACCTCTCCCAAGAGATGGATCTATGATACAAGCA
AG
GTCGCCTCCGGGGTGCCTTACAGGTTTTCCGGCTCTGGGAGTGGAACATCATATAGCCT
G
ACTATTAGCTCCATGGAGGCTGAAGACGCTGCAACCTATTACTGCCAGCAGTGGTCTAG
 
T
AATCCCCTGACCTTCGGCGCCGGGACAAAACTGGAGCTGAAGTGAGGATCC
118. SEQ ID NO: 136 (V1095 AA) 
EFATMAVMAPRTLVLLLSGALALTQTWAGDIQLTQSPASLAVSLGQRATISCKASQSVDYD
GDSYLNWYQQIPGQPPKLLIYDASNLVSGIPPRFSGSGSGTDFTLNIHPVEKVDAATYHCQQ
STEDPWTFGGGTKLEIKGGGGSGGGGSGGGGSQVQLQQSGAELVRPGSSVKISCKASGYAF
SSYWMNWVKQRPGQGLEWIGQIWPGDGDTNYNGKFKGKATLTADESSSTAYMQLSSLAS
EDSAVYFCARRETTTVGRYYYAMDYWGQGTTVTVSSGGSDAHKSEVAHRFKDLGEENFK
ALVLIAFAQYLQQCPFEDHVKLVNEVTEFAKTCVADESAENCDKSLHTLFGDKLCTVATLR
ETYGEMADCCAKQEPERNECFLQHKDDNPNLPRLVRPEVDVMCTAFHDNEETFLKKYLYE
IARRHPYFYAPELLFFAKRYKAAFTECCQAADKAACLLPKLDELRDEGKASSAKQRLKCAS
LQKFGERAFKAWAVARLSQRFPKAEFAEVSKLVTDLTKVHTECCHGDLLECADDRADLAK
YICENQDSISSKLKECCEKPLLEKSHCIAEVENDEMPADLPSLAADFVESKDVCKNYAEAKD
VFLGMFLYEYARRHPDYSVVLLLRLAKTYETTLEKCCAAADPHECYAKVFDEFKPLVEEP
QNLIKQNCELFEQLGEYKFQNALLVRYTKKVPQVSTPTLVEVSRNLGKVGSKCCKHPEAKR
MPCAEDYLSVVLNQLCVLHEKTPVSDRVTKCCTESLVNRRPCFSALEVDETYVPKEFNAET
FTFHADICTLSEKERQIKKQTALVELVKHKPKATKEQLKAVMDDFAAFVEKCCKADDKET
CFAEEGKKLVAASQAALGLGGSGDIKLQQSGAELARPGASVKMSCKTSGYTFTRYTMHW
VKQRPGQGLEWIGYINPSRGYTNYNQKFKDKATLTTDKSSSTAYMQLSSLTSEDSAVYYCA
RYYDDHYCLDYWGQGTTLTVSSVEGGSGGSGGSGGSGGVDDIQLTQSPAIMSASPGEKVT
MTCRASSSVSYMNWYQQKSGTSPKRWIYDTSKVASGVPYRFSGSGSGTSYSLTISSMEAED
AATYYCQQWSSNPLTFGAGTKLELK*GS
119. SEQ ID NO: 137 (V1092, CD3, NT)
   CL_#1072_antiCD3_1_HSA-1_337_DSS> 
GAATTCGCGACCATGGCTGTGATGGCTCCTAGAACCCTGGTGCTGCTGCTGTCCGGCGC
T
CTGGCTCTGACTCAGACCTGGGCTGGGATATCAAACTGCAGCAGTCTGGAGCTGAGCT
G
GCACGACCAGGAGCCAGTGTCAAAATGTCATGCAAGACTAGCGGCTACACCTTCACCA
GA
TATACCATGCACTGGGTGAAGCAGAGGCCAGGACAGGGACTGGAATGGATCGGATATA
TT
AACCCCAGCCGCGGCTACACAAACTATAATCAGAAGTTCAAAGACAAGGCTACACTGA
CC
ACAGATAAGAGCTCCTCTACTGCATACATGCAGCTGAGTTCACTGACCAGCGAGGATTC
C
GCCGTGTACTATTGCGCTAGGTACTATGACGATCATTACTGTCTGGACTATTGGGGACA
G
GGCACTACCCTGACTGTGAGCTCCGTCGAAGGAGGGTCTGGAGGCAGTGGAGGATCAG
GA
 
GGGAGCGGAGGAGTGGACGATATCCAGCTGACCCAGAGCCCCGCCATTATGAGTGCTT
CA
CCTGGCGAGAAGGTCACCATGACATGCCGCGCCTCTAGTTCAGTGTCCTACATGAATTG
G
TATCAGCAGAAATCCGGCACATCTCCTAAGCGGTGGATCTACGATACTTCCAAAGTCGC
C
TCTGGGGTGCCATATCGGTTCAGCGGGTCCGGATCTGGCACCAGTTACTCACTGACAAT
T
AGCTCCATGGAGGCTGAAGACGCCGCTACCTACTATTGTCAGCAGTGGTCTAGTAACCC
T
CTGACTTTCGGGGCCGGAACCAAACTGGAGCTGAAGGGGGAAGTGATGCACACAAAT
CA
GAAGTCGCCCATAGGTTCAAGGACCTGGGCGAGGAAAATTTTAAAGCCCTGGTGCTGA
TC
GCTTTCGCACAGTATCTGCAGCAGTGCCCATTTGAGGATCACGTGAAGCTGGTCAACGA
G
GTGACAGAGTTCGCCAAAACTTGCGTCGCAGACGAGAGCGCCGAAAATTGTGATAAGT
CC
CTGCATACCCTGTTTGGGGATAAACTGTGTACTGTGGCCACCCTGAGGGAGACATACGG
A
GAAATGGCTGACTGCTGTGCAAAGCAGGAGCCCGAACGCAACGAGTGCTTCCTGCAGC
AC
AAAGACGATAACCCCAATCTGCCTAGGCTGGTGCGCCCTGAAGTGGACGTCATGTGTA
CC
GCTTTCCACGATAATGAGGAAACATTTCTGAAGAAATACCTGTATGAGATTGCCCGGAG
A
CATCCTTACTTTTATGCTCCAGAACTGCTGTTCTTTGCAAAACGGTACAAGGCAGCCTTC
ACAGAGTGCTGTCAGGCTGCAGATAAAGCCGCTTGCCTGCTGCCAAAGCTGGACGAGC
TG
CGCGATGAAGGAAAGGCCTCAAGCGCTAAACAGCGACTGAAGTGTGCCTCCCTGCAGA
AA
TTCGGCGAGCGGGCTTTTAAGGCATGGGCTGTGGCACGACTGAGCCAGCGGTTCCCCA
AG
GCAGAGTTTGCCGAAGTCTCCAAACTGGTGACAGACCTGACTAAGGTGCACACCGAGT
GC
TGTCATGGGGACCTGCTGGAATGCGCCGACGATAGAGCTGATCTGGCAAAGTATATCT
GT
GAGAACCAGGACTCTATTTCCTCTAAACTGAAGGAGTGCTGTGAAAAACCTCTGCTGGA
G
AAGAGCCACTGCATCGCAGAGGTGGAAAACGACGAAATGCCAGCCGATCTGCCCTCTC
TG
GCAGCCGACTTCGTCGAGAGTAAAGATGTGTGTAAGAATTACGCCGAAGCTAAGGACG
TG
TTCCTGGGCATGTTTCTGTACGAGTATGCAAGAGCCTGAGGATCC
120. SEQ ID NO: 138 (V1092 AA) 
EFATMAVMAPRTLVLLLSGALALTQTWAGDIKLQQSGAELARPGASVKMSCKTSGYTFTR
YTMHWVKQRPGQGLEWIGYINPSRGYTNYNQKFKDKATLTTDKSSSTAYMQLSSLTSEDS
AVYYCARYYDDHYCLDYWGQGTTLTVSSVEGGSGGSGGSGGSGGVDDIQLTQSPAIMSAS
PGEKVTMTCRASSSVSYMNWYQQKSGTSPKRWIYDTSKVASGVPYRFSGSGSGTSYSLTIS
SMEAEDAATYYCQQWSSNPLTFGAGTKLELKGGSDAHKSEVAHRFKDLGEENFKALVLIA
FAQYLQQCPFEDHVKLVNEVTEFAKTCVADESAENCDKSLHTLFGDKLCTVATLRETYGE
MADCCAKQEPERNECFLQHKDDNPNLPRLVRPEVDVMCTAFHDNEETFLKKYLYEIARRH
PYFYAPELLFFAKRYKAAFTECCQAADKAACLLPKLDELRDEGKASSAKQRLKCASLQKF
GERAFKAWAVARLSQRFPKAEFAEVSKLVTDLTKVHTECCHGDLLECADDRADLAKYICE
NQDSISSKLKECCEKPLLEKSHCIAEVENDEMPADLPSLAADFVESKDVCKNYAEAKDVFL
GMFLYEYARA*GS
121. SEQ ID NO: 139 (CL_#1079_HSA-342_585_DSS_antiCD19_2 nt) 
GAATTCGCGACCATGGCCGTGATGGCTCCCCGAACACTGGTGCTGCTGCTGTCCGGCGC
T
CTGGCTCTGACTCAGACTTGGGCTGGCTCCGTGGTGCTGCTGCTGCGGCTGGCCAAGAC
C
TATGAGACCACACTGGAAAAATGCTGTGCCGCTGCAGATCCACACGAGTGCTACGCTA
AG
GTGTTCGACGAGTTCAAGCCTCTGGTCGAGGAACCACAGAACCTGATCAAGCAGAATT
GT
GAGCTGTTCGAACAGCTGGGCGAGTATAAATTTCAGAACGCCCTGCTGGTGAGATACA
CA
AAGAAAGTGCCCCAGGTCTCTACACCTACTCTGGTGGAAGTCAGTCGAAATCTGGGAA
AG
GTCGGCTCAAAGTGCTGTAAACACCCAGAGGCAAAACGGATGCCCTGCGCCGAAGACT
AT
CTGAGCGTGGTCCTGAACCAGCTGTGTGTGCTGCATGAGAAGACACCCGTGTCCGATAG
G
GTCACCAAATGCTGTACAGAATCTCTGGTGAACCGGAGACCCTGCTTCAGTGCCCTGGA
G
GTGGACGAAACTTACGTCCCTAAGGAGTTTAATGCCGAAACCTTCACATTTCACGCTGA
T
ATCTGTACTCTGTCCGAGAAAGAACGCCAGATTAAGAAACAGACCGCCCTGGTGGAGC
TG
GTCAAGCATAAACCTAAGGCAACTAAGGAACAGCTGAAAGCCGTGATGGACGATTTCG
CC
GCTTTTGTCGAGAAGTGCTGTAAAGCTGACGATAAGGAAACCTGCTTCGCAGAGGAAG
GC
AAGAAACTGGTGGCAGCCTCTCAGGCTGCACTGGGACTGGGAGGGAGCGGGGACATCC
AG
CTGACACAGTCCCCTGCATCTCTGGCCGTGAGCCTGGGACAGCGAGCTACTATTTCCTG
T
AAGGCATCCCAGTCTGTGGACTATGATGGGGACAGCTATCTGAACTGGTACCAGCAGA
 
TC
CCAGGACAGCCCCCTAAGCTGCTGATCTACGATGCCAGCAATCTGGTGTCCGGCATCCC
A
CCCCGATTCAGTGGATCAGGCAGCGGACAGATTTACTCTGAACATTCACCCAGTGGA
G
AAGGTCGACGCCGCTACCTACCATTGCCAGCAGTCTACTGAGGACCCCTGGACCTTCGG
A
GGCGGGACAAAGCTGGAAATCAAAGGAGGCGGGGATCAGGCGGAGGAGGCAGCGGA
GGA
GGAGGGTCCCAGGTGCAGCTGCAGCAGAGCGGAGCAGAGCTGGTGAGACCTGGCAGCT
CC
GTCAAGATTTCTTGTAAAGCTAGTGGCTATGCATTTTCTAGTTACTGGATGAATTGGGT
G
AAGCAGAGGCCTGGACAGGGACTGGAGTGGATCGGACAGATTTGGCCAGGGGATGGA
GAC
ACCAACTATAATGGGAAATTCAAGGGAAAAGCCACTCTGACCGCTGACGAGTCAAGCT
CC
ACAGCCTATATGCAGCTGTCTAGTCTGGCAAGTGAGGATTCAGCCGTGTACTTTTGCGC
T
AGGCGCGAAACTACCACAGTCGGCAGGTACTATTACGCTATGGACTACTGGGGCCAGG
GG
ACTACCGTGACCGTCTCAAGCTGAGGATCC
122. SEQ ID NO: 140 (CL_#1079_HSA-342_585_DSS_antiCD19_2 AA) 
EFATMAVMAPRTLVLLLSGALALTQTWAGSVVLLLRLAKTYETTLEKCCAAADPHECYAK
VFDEFKPLVEEPQNLIKQNCELFEQLGEYKFQNALLVRYTKKVPQVSTPTLVEVSRNLGKV
GSKCCKHPEAKRMPCAEDYLSVVLNQLCVLHEKTPVSDRVTKCCTESLVNRRPCFSALEV
DETYVPKEFNAETFTFHADICTLSEKERQIKKQTALVELVKHKPKATKEQLKAVMDDFAAF
VEKCCKADDKETCFAEEGKKLVAASQAALGLGGSGDIQLTQSPASLAVSLGQRATISCKAS
QSVDYDGDSYLNWYQQIPGQPPKLLIYDASNLVSGIPPRFSGSGSGTDFTLNIHPVEKVDAA
TYHCQQSTEDPWTFGGGTKLEIKGGGGSGGGGSGGGGSQVQLQQSGAELVRPGSSVKISC
KASGYAFSSYWMNWVKQRPGQGLEWIGQIWPGDGDTNYNGKFKGKATLTADESSSTAY
MQLSSLASEDSAVYFCARRETTTVGRYYYAMDYWGQGTTVTVSS*GS
123. SEQ ID NO: 141 (CL_#1075_HSA-342_585_DSS_antiCD3_2) 
GAATTCGCGACCATGGCTGTGATGGCTCCTAGAACACTGGTGCTGCTGCTGTCCGGGGC
T
CTGGCTCTGACTCAGACCTGGGCTGGAAGCGTGGTGCTGCTGCTGCGGCTGGCAAAGA
CA
TATGAGACCACACTGGAAAAATGCTGTGCCGCTGCAGACCCTCACGAGTGCTACGCCA
AG
GTGTTCGATGAGTTCAAGCCCCTGGTCGAGGAACCTCAGAACCTGATCAAGCAGAATT
GT
 
GAGCTGTTCGAACAGCTGGGCGAGTACAAATTTCAGAACGCCCTGCTGGTGAGATATA
CC
AAGAAAGTGCCACAGGTCTCAACCCCCACACTGGTGGAAGTCAGCAGAAATCTGGGCA
AG
GTCGGGTCCAAGTGCTGTAAACACCCTGAGGCAAAAGGATGCCATGCGCCGAAGACT
AC
CTGTCCGTGGTCCTGAACCAGCTGTGTGTGCTGCATGAGAAGACACCAGTGTCTGATAG
G
GTCACTAAATGCTGTACCGAAAGCCTGGTGAACCGGAGACCTTGCTTCAGCGCCCTGGA
G
GTGGACGAAACCTACGTCCCAAAGGAGTTTAATGCTGAAACTTTCACCTTTCACGCAGA
T
ATCTGTACCCTGTCCGAGAAAGAACGCCAGATTAAGAAACAGACAGCCCTGGTGGAGC
TG
GTCAAGCATAAACCAAAGGCCACTAAGGAACAGCTGAAAGCTGTGATGGACGATTTCG
CC
GCTTTTGTCGAGAAGTGCTGTAAAGCAGACGATAAGGAAACCTGCTTCGCCGAGGAAG
GG
AAGAAACTGGTGGCAGCATCTCAGGCTGCACTGGGACTGGGAGGGTCTGGCGACATTA
AG
CTGCAGCAGAGTGGAGCAGAGCTGGCACGACCAGGAGCATCAGTGAAGATGAGCTGTA
AA
ACTTCCGGCTACACATTCACTAGGTATACCATGCACTGGGTGAAGCAGCGACCAGGAC
AG
GGACTGGAGTGGATCGGCTATATTAATCCCAGCCGAGGGTACACCAACTATAATCAGA
AG
TTCAAGGACAAAGCTACACTGACTACCGATAAGAGCTCCTCTACTGCATACATGCAGCT
G
AGTTCACTGACCTCCGAGGACTCTGCCGTGTACTATTGCGCTAGGTACTATGACGATCA
T
TACTGTCTGGATTATTGGGGACAGGGCACAACTCTGACAGTGAGCTCCGTCGAAGGAG
GC
AGTGGAGGATCAGGAGGGAGCGGAGGCTCCGGAGGAGTGGACGATATCCAGCTGACT
CAG
AGCCCCGCTATTATGTCAGCAAGCCCTGGCGAGAAGGTGACCATGACATGCCGGGCTT
CT
AGTTCAGTCAGCTACATGAACTGGTATCAGCAGAAGTCTGGAACAAGTCCCAAACGAT
GG
ATCTACGACACTTCTAAGGTGGCCAGTGGCGTCCCTTATCGGTTCTCCGGGTCTGGAAG
T
GGCACATCATACAGCCTGACTATTAGCTCCATGGAGGCCGAAGATGCCGCTACCTACTA
T
TGCCAGCAGTGGTCTAGTAATCCCCTGACCTTTGGGGCTGGAACAAAGCTGGAGCTGA
AA
TGAGGATCC
124. SEQ ID NO: 142 (CL_#1075_HSA-342_585_DSS_antiCD3_2 AA) 
EFATMAVMAPRTLVLLLSGALALTQTWAGSVVLLLRLAKTYETTLEKCCAAADPHECYAK
VFDEFKPLVEEPQNLIKQNCELFEQLGEYKFQNALLVRYTKKVPQVSTPTLVEVSRNLGKV
GSKCCKHPEAKRMPCAEDYLSVVLNQLCVLHEKTPVSDRVTKCCTESLVNRRPCFSALEV
DETYVPKEFNAETFTFHADICTLSEKERQIKKQTALVELVKHKPKATKEQLKAVMDDFAAF
VEKCCKADDKETCFAEEGKKLVAASQAALGLGGSGDIKLQQSGAELARPGASVKMSCKTS
GYTFTRYTMHWVKQRPGQGLEWIGYINPSRGYTNYNQKFKDKATLTTDKSSSTAYMQLSS
LTSEDSAVYYCARYYDDHYCLDYWGQGTTLTVSSVEGGSGGSGGSGGSGGVDDIQLTQSP
AIMSASPGEKVTMTCRASSSVSYMNWYQQKSGTSPKRWIYDTSKVASGVPYRFSGSGSGT
SYSLTISSMEAEDAATYYCQQWSSNPLTFGAGTKLELK*GS
125. SEQ ID NO: 143 CL_#1076_antiCD19_1_HSA-1_337_DSS NT 
GAATTCGCGACCATGGCCGTGATGGCACCTCGCACCCTGGTCCTGCTGCTGAGCGGGGC
A
CTGGCACTGACACAGACTTGGGCTGGAGATATTCAGCTGACTCAGAGCCCTGCTTCCCT
G
GCAGTCAGCCTGGGACAGCGAGCAACCATCTCCTGCAAGGCCAGCCAGTCCGTGGACT
AT
GATGGCGACTCCTATCTGAACTGGTACCAGCAGATCCCAGGGCAGCCCCCTAAACTGCT
G
ATCTACGATGCCTCAAATCTGGTGAGCGGCATCCCACCCAGATTTCTGGAAGTGGCTC
A
GGGACAGACTTCACTCTGAACATTCACCCCGTGGAGAAGGTCGATGCCGCTACCTACCA
T
TGCCAGCAGAGCACAGAGGACCCTTGGACTTTTGGCGGGGGAACCAAACTGGAAATCA
AG
GGAGGAGGAGGCAGTGGCGGAGGAGGGTCAGGAGGAGGAGGAAGCCAGGTGCAGCTG
CAG
CAGAGCGGAGCAGAGCTGGTCAGGCCAGGAAGCTCCGTGAAAATTTCCTGTAAGGCCT
CT
GGCTATGCTTTCTCTAGTTACTGGATGAATTGGGTGAAGCAGCGACCTGGACAGGGACT
G
GAGTGGATCGGGCAGATTTGGCCAGGGGATGGAGACACCAACTACAATGGAAAGTTTA
AA
GGCAAGGCAACTCTGACCGCCGATGAATCAAGCTCCACAGCCTATATGCAGCTGTCTA
GT
CTGGCATCTGAGGACAGTGCCGTCTACTTCTGCGCTCGGAGAGAAACCACAACTGTGG
GA
CGATACTATTACGCCATGGATTATTGGGGCCAGGGGACCACAGTGACCGTCTCAAGCG
GC
GGGTCCGATGCTCACAAATCTGAGGTCGCACATCGCTTCAAGGACCTGGGGGAGGAAA
AC
TTTAAAGCCCTGGTGCTGATCGCTTTCGCACAGTACCTGCAGCAGTGCCCCTTTGAAGA
C
 
CACGTGAAGCTGGTCAACGAGGTGACAGAGTTCGCCAAAACTTGCGTCGCTGATGAGA
GT
GCAGAAAATTGTGACAAGTCACTGCATACACTGTTTGGAGATAAACTGTGTACCGTGGC
C
ACACTGCGGGAGACTTATGGCGAAATGGCCGACTGCTGTGCTAAGCAGGAGCCAGAAA
GA
AACGAGTGCTTCCTGCAGCACAAAGACGATAACCCTAATCTGCCACGACTGGTGCGGC
CC
GAAGTGGATGTCATGTGTACCGCCTTCCACGACAATGAGGAAACATTTCTGAAGAAAT
AT
CTGTACGAGATTGCTAGGCGCCATCCATATTTTTACGCCCCCGAACTGCTGTTCTTTGCT
AAAAGGTACAAGGCAGCCTTCACTGAGTGCTGTCAGGCTGCAGATAAAGCCGCTTGCC
TG
CTGCCCAAGCTGGATGAGCTGCGCGACGAAGGGAAGGCCTCCTCTGCTAAACAGCGAC
TG
AAGTGTGCATCTCTGCAGAAATTCGGAGAGAGGGCTTTTAAGGCCTGGGCTGTGGCAA
GA
CTGAGCCAGAGGTTCCCTAAGGCAGAGTTTGCCGAAGTCAGCAAACTGGTGACTGACC
TG
ACCAAGGTGCACACAGAGTGCTGTCATGGCGATCTGCTGGAATGCGCCGACGATCGCG
CT
GACCTGGCAAAGTATATCTGTGAGAATCAGGATTCCATTAGTTCAAAACTGAAGGAGT
GC
TGTGAAAAACCTCTGCTGGAGAAGTCTCACTGCATCGCCGAGGTGGAAAACGATGAAA
TG
CCCGCTGACCTGCCTTCTCTGGCAGCCGATTTCGTCGAGAGTAAAGACGTGTGTAAGAA
T
TACGCCGAAGCTAAGGACGTGTTCCTGGGCATGTTTCTGTATGAGTACGCAAGAGCCTG
A
GGATCC
126. SEQ ID NO: 144 (CL_#1076_antiCD19_1_HSA-1_337_DSS AA) 
EFATMAVMAPRTLVLLLSGALALTQTWAGDIQLTQSPASLAVSLGQRATISCKASQSVDYD
GDSYLNWYQQIPGQPPKLLIYDASNLVSGIPPRFSGSGSGTDFTLNIHPVEKVDAATYHCQQ
STEDPWTFGGGTKLEIKGGGGSGGGGSGGGGSQVQLQQSGAELVRPGSSVKISCKASGYAF
SSYWMNWVKQRPGQGLEWIGQIWPGDGDTNYNGKFKGKATLTADESSSTAYMQLSSLAS
EDSAVYFCARRETTTVGRYYYAMDYWGQGTTVTVSSGGSDAHKSEVAHRFKDLGEENFK
ALVLIAFAQYLQQCPFEDHVKLVNEVTEFAKTCVADESAENCDKSLHTLFGDKLCTVATLR
ETYGEMADCCAKQEPERNECFLQHKDDNPNLPRLVRPEVDVMCTAFHDNEETFLKKYLYE
IARRHPYFYAPELLFFAKRYKAAFTECCQAADKAACLLPKLDELRDEGKASSAKQRLKCAS
LQKFGERAFKAWAVARLSQRFPKAEFAEVSKLVTDLTKVHTECCHGDLLECADDRADLAK
YICENQDSISSKLKECCEKPLLEKSHCIAEVENDEMPADLPSLAADFVESKDVCKNYAEAKD
VFLGMFLYEYARA*GS
127. SEQ ID NO: 145 (CL #1070_HER2_scFv_Fc_L351Y_F405A_Y407V NT) 
GAATTCGCCACCATGGCCGTGATGGCACCTAGAACACTGGTCCTGCTGCTGAGCGGAGC
C
CTGGCACTGACACAGACTTGGGCCGGACAGGTCCAGCTGGTGCAGAGCGGGGCAGAGG
TC
AAGAAACCCGGAGAAAGTCTGAAGATCTCATGCAAAGGGAGTGGATACTCATTCACCA
GC
TATTGGATTGCCTGGGTGAGGCAGATGCCTGGCAAGGGGCTGGAATACATGGGCCTGAT
C
TATCCAGGGGACAGCGATACAAAATACTCCCCCTCTTTCCAGGGCCAGGTCACAATTTC
C
GTGGACAAGAGTGTCTCAACTGCCTATCTGCAGTGGAGCTCCCTGAAACCTAGCGATTC
C
GCAGTGTACTTTTGTGCCAGGCACGACGTCGGGTATTGCACAGATCGCACTTGTGCTAA
G
TGGCCAGAGTGGCTGGGAGTGTGGGGACAGGGAACCCTGGTCACAGTGTCTAGTGGAG
GA
GGAGGCTCAAGCGGAGGAGGCTCTGGAGGAGGAGGGTCTCAGAGTGTGCTGACTCAGC
CA
CCTTCAGTCAGCGCAGCTCCTGGACAGAAGGTGACCATCTCCTGCTCTGGCAGCTCTAG
T
AACATTGGCAACAATTACGTGAGCTGGTATCAGCAGCTGCCTGGCACCGCCCCAAAGCT
G
CTGATCTACGACCACACAAATCGGCCCGCTGGGGTGCCTGATAGATTCAGTGGGTCAAA
A
AGCGGAACCTCCGCTTCTCTGGCAATTAGCGGCTTTCGCTCCGAGGACGAAGCTGATTA
C
TATTGTGCATCTTGGGACTACACACTGAGTGGCTGGGTGTTCGGAGGCGGACTAAGCT
G
ACCGTGCTGGGGGCAGCCGAACCAAAGTCAAGCGATAAAACTCATACCTGCCCACCAT
GT
CCTGCACCAGAGCTGCTGGGAGGACCTTCCGTGTTCCTGTTTCCTCCAAAGCCAAAAGA
C
ACCCTGATGATCAGCCGAACACCAGAAGTGACTTGCGTGGTCGTGGACGTCTCCCACGA
G
GACCCCGAAGTGAAGTTTAACTGGTACGTGGATGGCGTCGAGGTGCATAATGCCAAGA
CC
AAACCCCGAGAGGAACAGTACAACTCAACTTATCGGGTCGTGAGCGTCCTGACCGTGCT
G
CACCAGGACTGGCTGAACGGAAAGAGTATAAGTGCAAAGTGTCTAATAAGGCCCTGC
CC
GCTCCTATCGAGAAAACAATTAGCAAGGCAAAAGGCCAGCCAAGAGAACCCCAGGTGT
AC
ACTTATCCCCCTTCTAGGGACGAGCTGACCAAGAACCAGGTGAGCCTGACATGTCTGGT
C
AAAGGATTTTACCCCAGTGATATTGCTGTGGAGTGGGAATCCAATGGCCAGCCTGAAAA
 
C
AATTATAAGACCACACCACCCGTGCTGGACTCCGATGGATCTTTCGCTCTGGTGTCCAA
G
CTGACTGTCGATAAATCTCGGTGGCAGCAGGGCAACGTGTTTAGTTGTTCAGTCATGCA
T
GAGGCACTGCACAATCATTACACACAGAAGAGCCTGTCCCTGTCTCCCGGCAAATGAG
GA
TCC
128. SEQ ID NO: 146 (CL #1070_HER2_scFv_Fc_L351Y_F405A_Y407V AA) 








*GS
129. SEQ ID NO: 147 (CL_#1039) 
GAATTCGCTACAATGGCCGTGATGGCACCCCGAACACTGGTCCTGCTGCTGAGCGGCGC
A
CTGGCACTGACACAGACTTGGGCTGGAGAACCTAAGAGCTCCGACAAAACCCACACAT
GC
CCCCCTTGTCCAGCTCCAGAACTGCTGGGAGGACCATCCGTGTTCCTGTTTCCACCCAA
G
CCCAAAGATACACTGATGATCTCTCGAACTCCCGAGGTCACCTGCGTGGTCGTGGACGT
C
AGTCACGAGGACCCCGAAGTCAAGTTCAACTGGTACGTGGACGGCGTCGAAGTGCATA
AT
GCAAAGACTAAACCACGGGAGGAACAGTACAACTCAACATATAGAGTCGTGAGCGTCC
TG
ACTGTGCTGCATCAGGATTGGCTGAACGGCAAGGAGTATAAGTGCAAAGTGAGCAATA
AG
GCCCTGCCTGCTCCAATCGAGAAAACCATTAGCAAGGCAAAAGGGCAGCCCAGGGAAC
CT
CAGGTGTACACCCTGCCTCCAAGCCGCGACGAGCTGACAAAGAACCAGGTCTCCCTGCT
G
TGTCTGGTGAAAGGATTCTATCCTAGTGATATTGCCGTGGAGTGGGAATCAAATGGCCA
G
CCAGAGAACAATTACATGACTTGGCCCCCTGTGCTGGACTCTGATGGGAGTTTCTTTCTG
TATTCCAAGCTGACCGTGGACAAATCTAGATGGCAGCAGGGAAACGTCTTTTCTTGTAG
T
GTGATGCACGAAGCCCTGCACAATCATTACACACAGAAGTCACTGAGCCTGTCCCCTGG
 
C
AAATGAGGATCC
130. SEQ ID NO: 148 (CL_#1039, AA) 
EFATMAVMAPRTLVLLLSGALALTQTWAGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLLCLV
KGFYPSDIAVEWESNGQPENNYMTWPPVLDSDGSFFLYSKLTVDKSRWQQGNVFSCSVMH
EALHNHYTQKSLSLSPGK*GS
131. SEQ ID NO: 149 (CL_#719_scFv_FD5_Fc_IGG1_CH-A_L351Y_S400E_F405A_Y407V, NT> 
GAATTCGCCACCATGGCCGTGATGGCTCCTAGAACCCTGGTGCTGCTGCTGTCTGGAGC
T
CTGGCTCTGACTCAGACCTGGGCTGGAGACATCCAGATGACCCAGTCTCCATCCTCCCT
G
TCTGCATCTGTAGGAGACAGAGTCACCATCACTTGCCGGGCAAGTCAGGACGTTAACAC
C
GCTGTAGCTTGGTATCAGCAGAAACCAGGGAAAGCCCCTAAGCTCCTGATCTATTCTGC
A
TCCTTTTTGTACAGTGGGGTCCCATCAAGGTTCAGTGGCAGTCGATCTGGGACAGATTTC
ACTCTCACCATCAGCAGTCTGCAACCTGAAGATTTTGCAACTTACTACTGTCAACAGCA
T
TACACTACCCCACCCACTTTCGGCCAAGGGACCAAAGTGGAGATCAAAGGTGGTTCTGG
T
GGTGGTTCTGGTGGTGGTTCTGGTGGTGGTTCTGGTGGTGGTTCTGGTGAAGTGCAGCT
G
GTGGAGTCTGGGGGAGGCTTGGTACAGCCTGGCGGGTCCCTGAGACTCTCCTGTGCAGC
C
TCTGGATTCAACATTAAAGATACTTATATCCACTGGGTCCGGCAAGCTCCAGGGAAGGG
C
CTGGAGTGGGTCGCACGTATTTATCCCACAAATGGTTACACACGGTATGCGGACTCTGT
G
AAGGGCCGATTCACCATCTCCGCAGACACTTCCAAGAACACCGCGTATCTGCAAATGAA
C
AGTCTGAGAGCTGAGGACACGGCCGTTTATTACTGTTCAAGATGGGGCGGAGACGGTTT
C
TACGCTATGGACTACTGGGGCCAAGGGACCCTGGTCACCGTCTCCTCAGCCGCCGAGCC
C
AAGAGCAGCGATAAGACCCACACCTGCCCTCCCTGTCCAGCTCCAGAACTGCTGGGAG
GA
CCTAGCGTGTTCCTGTTTCCCCCTAAGCCAAAAGACACTCTGATGATTTCCAGGACTCCC
GAGGTGACCTGCGTGGTGGTGGACGTGTCTCACGAGGACCCCGAAGTGAAGTTCAACT
GG
 
TACGTGGATGGCGTGGAAGTGCATAATGCTAAGACAAAACCAAGAGAGGAACAGTACA
AC
TCCACTTATCGCGTCGTGAGCGTGCTGACCGTGCTGCACCAGGACTGGCTGAACGGAA
G
GAGTATAAGTGCAAAGTCAGTAATAAGGCCCTGCCTGCTCCAATCGAAAAAACCATCTC
T
AAGGCCAAAGGCCAGCCAAGGGAGCCCCAGGTGTACACATACCCACCCAGCAGAGACG
AA
CTGACCAAGAACCAGGTGTCCCTGACATGTCTGGTGAAAGGCTTCTATCCTAGTGATAT
T
GCTGTGGAGTGGGAATCAAATGGACAGCCAGAGAACAATTACAAGACCACACCTCCAG
TG
CTGGACGAGGATGGCAGCTTCGCCCTGGTGTCCAAGCTGACAGTGGATAAATCTCGATG
G
CAGCAGGGGAACGTGTTTAGTTGTTCAGTGATGCATGAAGCCCTGCACAATCATTACAC
T
CAGAAGAGCCTGTCCCTGTCTCCCGGCAAATGAGGATCC
132. SEQ ID NO: 150 (CL_#719_scFv_FD5_Fc_IGG1_CH-A_L351Y_S400E_F405A_Y407V, AA) 
EFATMAVMAPRTLVLLLSGALALTQTWAGDIQMTQSPSSLSASVGDRVTITCRASQDVNTA
VAWYQQKPGKAPKLLIYSASFLYSGVPSRFSGSRSGTDFTLTISSLQPEDFATYYCQQHYTTP
PTFGQGTKVEIKGGSGGGSGGGSGGGSGGGSGEVQLVESGGGLVQPGGSLRLSCAASGFNI
KDTYIHWVRQAPGKGLEWVARIYPTNGYTRYADSVKGRFTISADTSKNTAYLQMNSLRAE
DTAVYYCSRWGGDGFYAMDYWGQGTLVTVSSAAEPKSSDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTYPPSRDELTKNQV
SLTCLVKGFYPSDIAVEWESNGQPENNYKTTPPVLDEDGSFALVSKLTVDKSRWQQGNVFS
CSVMHEALHNHYTQKSLSLSPGK*GS
133. SEQ ID NO: 151 (CL_#720_scFv_FD5_Fc_IGG1_CH-B_T366I N390R K392M T394W) 
GAATTCGCCACCATGGCCGTGATGGCTCCTAGAACCCTGGTGCTGCTGCTGTCTGGAGC
T
CTGGCTCTGACTCAGACCTGGGCTGGAGACATCCAGATGACCCAGTCTCCATCCTCCCT
G
TCTGCATCTGTAGGAGACAGAGTCACCATCACTTGCCGGGCAAGTCAGGACGTTAACAC
C
GCTGTAGCTTGGTATCAGCAGAAACCAGGGAAAGCCCCTAAGCTCCTGATCTATTCTGC
A
TCCTTTTTGTACAGTGGGGTCCCATCAAGGTTCAGTGGCAGTCGATCTGGGACAGATTTC
ACTCTCACCATCAGCAGTCTGCAACCTGAAGATTTTGCAACTTACTACTGTCAACAGCA
T
TACACTACCCCACCCACTTTCGGCCAAGGGACCAAAGTGGAGATCAAAGGTGGTTCTGG
T
GGTGGTTCTGGTGGTGGTTCTGGTGGTGGTTCTGGTGGTGGTTCTGGTGAAGTGCAGCT
 
G
GTGGAGTCTGGGGGAGGCTTGGTACAGCCTGGCGGGTCCCTGAGACTCTCCTGTGCAGC
C
TCTGGATTCAACATTAAAGATACTTATATCCACTGGGTCCGGCAAGCTCCAGGGAAGGG
C
CTGGAGTGGGTCGCACGTATTTATCCCACAAATGGTTACACACGGTATGCGGACTCTGT
G
AAGGGCCGATTCACCATCTCCGCAGACACTTCCAAGAACACCGCGTATCTGCAAATGAA
C
AGTCTGAGAGCTGAGGACACGGCCGTTTATTACTGTTCAAGATGGGGCGGAGACGGTTT
C
TACGCTATGGACTACTGGGGCCAAGGGACCCTGGTCACCGTCTCCTCAGCCGCCGAGCC
C
AAGAGCAGCGATAAGACCCACACCTGCCCTCCCTGTCCAGCTCCAGAACTGCTGGGAG
GA
CCTAGCGTGTTCCTGTTTCCCCCTAAGCCAAAAGACACTCTGATGATTTCCAGGACTCCC
GAGGTGACCTGCGTGGTGGTGGACGTGTCTCACGAGGACCCCGAAGTGAAGTTCAACT
GG
TACGTGGATGGCGTGGAAGTGCATAATGCTAAGACAAAACCAAGAGAGGAACAGTACA
AC
TCCACTTATCGCGTCGTGAGCGTGCTGACCGTGCTGCACCAGGACTGGCTGAACGGAA
G
GAGTATAAGTGCAAAGTCAGTAATAAGGCCCTGCCTGCTCCAATCGAAAAAACCATCTC
T
AAGGCCAAAGGCCAGCCAAGGGAGCCCCAGGTGTACACACTGCCACCCAGCAGAGACG
AA
CTGACCAAGAACCAGGTGTCCCTGATCTGTCTGGTGAAAGGCTTCTATCCTAGTGATAT
T
GCTGTGGAGTGGGAATCAAATGGACAGCCAGAGAACAGATACATGACCTGGCCTCCAG
TG
CTGGACAGCGATGGCAGCTTCTTCCTGTATTCCAAGCTGACAGTGGATAAATCTCGATG
G
CAGCAGGGGAACGTGTTTAGTTGTTCAGTGATGCATGAAGCCCTGCACAATCATTACAC
T
CAGAAGAGCCTGTCCCTGTCTCCCGGCAAATGAGGATCC
134. SEQ ID NO: 152 (CL_#720_scFv_FD5_Fc_IGG1_CH-B_T366I N390R K392MT394W, AA) 
EFATMAVMAPRTLVLLLSGALALTQTWAGDIQMTQSPSSLSASVGDRVTITCRASQDVNTA
VAWYQQKPGKAPKLLIYSASFLYSGVPSRFSGSRSGTDFTLTISSLQPEDFATYYCQQHYTTP
PTFGQGTKVEIKGGSGGGSGGGSGGGSGGGSGEVQLVESGGGLVQPGGSLRLSCAASGFNI
KDTYIHWVRQAPGKGLEWVARIYPTNGYTRYADSVKGRFTISADTSKNTAYLQMNSLRAE
DTAVYYCSRWGGDGFYAMDYWGQGTLVTVSSAAEPKSSDKTHTCPPCPAPELLGGPSVFL
FPPKPKDTLMISRTPEVTCVVVDVSHEDPEVKFNWYVDGVEVHNAKTKPREEQYNSTYRV
VSVLTVLHQDWLNGKEYKCKVSNKALPAPIEKTISKAKGQPREPQVYTLPPSRDELTKNQV
 
SLICLVKGFYPSDIAVEWESNGQPENRYMTWPPVLDSDGSFFLYSKLTVDKSRWQQGNVFS
CSVMHEALHNHYTQKSLSLSPGK*GS
135. SEQ ID NO: 153 (CL #719_scFv_FD5_Fc_IGG1_CH-A_L351Y_S400E_F405A_Y407V) -> see above
   CL_#716_Fc_IGG1_CH-B_T366I_N390R_K392M_T394W> 
GAATTCGCCACCATGGCCGTGATGGCTCCTAGAACCCTGGTGCTGCTGCTGTCTGGAGC
T
CTGGCTCTGACTCAGACCTGGGCTGGAGAGCCCAAGAGCAGCGATAAGACCCACACCT
GC
CCTCCCTGTCCAGCTCCAGAACTGCTGGGAGGACCTAGCGTGTTCCTGTTCCCCCTAAG
CCAAAAGACACTCTGATGATTTCCAGGACTCCCGAGGTGACCTGCGTGGTGGTGGACGT
G
TCTCACGAGGACCCCGAAGTGAAGTTCAACTGGTACGTGGATGGCGTGGAAGTGCATA
AT
GCTAAGACAAAACCAAGAGAGGAACAGTACAACTCCACTTATCGCGTCGTGAGCGTGC
TG
ACCGTGCTGCACCAGGACTGGCTGAACGGGAAGGAGTATAAGTGCAAAGTCAGTAATA
AG
GCCCTGCCTGCTCCAATCGAAAAAACCATCTCTAAGGCCAAAGGCCAGCCAAGGGAGC
CC
CAGGTGTACACACTGCCACCCAGCAGAGACGAACTGACCAAGAACCAGGTGTCCCTGA
TC
TGTCTGGTGAAAGGCTTCTATCCTAGTGATATTGCTGTGGAGTGGGAATCAAATGGACA
G
CCAGAGAACAGATACATGACCTGGCCTCCAGTGCTGGACAGCGATGGCAGCTTCTTCCT
G
TATTCCAAGCTGACAGTGGATAAATCTCGATGGCAGCAGGGGAACGTGTTTAGTTGTTC
A
GTGATGCATGAAGCCCTGCACAATCATTACACTCAGAAGAGCCTGTCCCTGTCTCCCGG
C
AAATGAGGATCC
136. SEQ ID NO: 154 (CL_#719_scFv_FD5_Fc_IGG1_CH-A_L351Y_S400E_F405A_Y407V) 
EFATMAVMAPRTLVLLLSGALALTQTWAGEPKSSDKTHTCPPCPAPELLGGPSVFLFPPKPK
DTLMISRTPEVTCVVVDVSHEDPEVKFNWYVDGVEVHNAKTKPREEQYNSTYRVVSVLTV
LHQDWLNGKEYKCKVSNKALPAPIEKTISKAKGQPREPQVYTLPPSRDELTKNQVSLICLVK
GFYPSDIAVEWESNGQPENRYMTWPPVLDSDGSFFLYSKLTVDKSRWQQGNVFSCSVMHE
ALHNHYTQKSLSLSPGK*GS
137. SEQ ID NO: 155 (CL_#1102_MM-111) 
GAATTCGCGACCATGGCCGTGATGGCTCCTCGGACACTGGTGCTGCTGCTGAGTGGGGC
T
CTGGCTCTGACACAGACTTGGGCTGGACAGGTGCAGCTGCAGGAGTCCGGAGGAGGAC
TG
GTGAAGCCAGGAGGGTCCCTGAGACTGTCTTGCGCCGCTAGTGGCTTCACCTTTAGCTC
C
TACTGGATGTCTTGGGTGAGGCAGGCACCTGGCAAGGGACTGGAGTGGGTGGCAAACA
TC
AATCGCGACGGATCAGCCAGCTACTATGTGGATAGCGTCAAGGGCAGGTTTACAATTAG
T
CGCGACGATGCTAAAAACTCACTGTACCTGCAGATGAATAGCCTGCGGGCAGAGGATA
CT
GCCGTGTACTATTGCGCCAGAGACAGGGGAGTCGGCTATTTCGATCTGTGGGGGAGAG
GA
ACTCTGGTGACCGTCTCTAGTGCTAGCACCGGAGGCGGGGGATCTGGCGGAGGAGGCA
GT
GGAGGAGGAGGGTCCCAGTCTGCTCTGACACAGCCAGCAAGTGTGTCAGGCAGCCCCG
GG
CAGTCAATCACTATTAGCTGTACTGGCACCTCAAGCGACGTGGGAGGCTACAACTTTGT
C
AGCTGGTATCAGCAGCACCCTGGAAAAGCCCCAAAGCTGATGATCTACGACGTGAGCG
AT
CGACCTTCCGGCGTCTCTGATCGGTTCTCCGGGTCTAAGAGTGGAAATACTGCCTCCCT
G
ATCATTTCTGGGCTGCAGGCTGACGATGAGGCAGACTACTATTGCTCCTCTTATGGAAG
T
TCAAGCACCCATGTGATCTTCGGGGGAGGCACAAAAGTGACTGTCCTGGGCGCAGCCTC
A
GATGCTCACAAAAGCGAGGTGGCACATCGGTTCAAGGACCTGGGGGAGGAAAACTTTA
AA
GCCCTGGTGCTGATTGCATTCGCCCAGTACCTGCAGCAGAGCCCATTTGAGGACCACGT
G
AAGCTGGTCAACGAGGTGACCGAGTTCGCCAAAACATGCGTGGCCGACGAGTCCGCTG
AA
AATTGTGATAAGTCTCTGCATACTCTGTTTGGAGATAAACTGTGCACCGTGGCCACACT
G
CGAGAGACCTACGGCGAAATGGCAGACTGCTGTGCCAAGCAGGAGCCAGAAAGAAAC
GAG
TGCTTCCTGCAGCACAAAGACGATAACCCAAATCTGCCACGACTGGTGCGACCAGAAG
TG
GACGTCATGTGTACTGCTTTCCACGATAATGAGGAAACCTTTCTGAAGAAATACCTGTA
T
GAGATCGCCCGGAGACATCCCTACTTTTATGCACCTGAACTGCTGTTCTTTGCCAAAAG
A
TACAAGGCTGCATTCACCGAGTGCTGTCAGGCCGCTGATAAAGCAGCCTGCCTGCTGCC
A
AAGCTGGACGAGCTGCGAGATGAAGGCAAGGCCTCCTCTGCTAAACAGAGACTGAAGT
 
GT
GCCAGCCTGCAGAAATTCGGCGAGAGGGCTTTTAAGGCTTGGGCAGTGGCACGACTGTC
C
CAGAGATTCCCTAAGGCAGAGTTTGCCGAAGTCTCTAAACTGGTGACTGACCTGACCAA
G
GTGCACACCGAGTGCTGTCATGGCGACCTGCTGGAATGCGCCGACGATCGCGCTGATCT
G
GCAAAGTACATCTGTGAGAACCAGGACAGCATTAGTTCAAAGCTGAAAGAGTGCTGTG
AA
AAACCCCTGCTGGAGAAGAGCCACTGCATCGCAGAGGTGGAAAACGACGAAATGCCCG
CC
GATCTGCCTAGTCTGGCTGCAGACTTCGTGGAGTCAAAAGATGTCTGTAAGAATTACGC
T
GAAGCAAAGGATGTGTTCCTGGGCATGTTTCTGTACGAGTATGCTAGGCGCCACCCAGA
C
TACTCCGTGGTCCTGCTGCTGAGGCTGGCCAAGACCTATGAGACCACACTGGAAAAATG
C
TGTGCCGCTGCAGATCCCCATGAGTGCTATGCCAAAGTGTTCGACGAGTTCAAGCCACT
G
GTCGAGGAACCCCAGAACCTGATTAAGCAGAATTGTGAGCTGTTTGAACAGCTGGGCG
AG
TACAAATTCCAGAACGCCCTGCTGGTGCGCTATACAAAGAAAGTCCCTCAGGTGAGCAC
A
CCAACTCTGGTGGAAGTCTCCAGGAATCTGGGAAAGGTCGGCTCTAAATGCTGTAAGCA
C
CCCGAGGCCAAACGCATGCCTTGCGCTGAAGATTACCTGTCCGTGGTCCTGAACCAGCT
G
TGTGTGCTGCATGAGAAGACCCCAGTCTCTGACCGGGTGACAAAATGCTGTACTGAAAG
T
CTGGTGAATCGACGGCCCTGCTTTAGCGCCCTGGAGGTGGATGAAACATATGTCCCTAA
G
GAGTTCCAGGCTGAAACCTTCACATTTCACGCAGACATCTGTACTCTGTCCGAGAAAGA
A
AGACAGATTAAGAAACAGACCGCCCTGGTCGAGCTGGTGAAGCATAAACCCAAGGCCA
CA
AAAGAACAGCTGAAGGCTGTGATGGACGATTTCGCCGCTTTTGTCGAGAAATGCTGTAA
G
GCTGACGATAAGGAAACTTGCTTTGCAGAGGAAGGGAAGAAACTGGTGGCAGCATCCC
AG
GCTGCACTGGGACTGGCAGCTGCACTGCAGGTCCAGCTGGTGCAGTCTGGCGCCGAGGT
G
AAGAAACCTGGGGAAAGTCTGAAAATCTCCTGTAAGGGCAGTGGGTACTCATTCACCA
GC
TATTGGATTGCCTGGGTGAGGCAGATGCCAGGAAAGGGCCTGGAGTACATGGGACTGA
TC
TATCCTGGCGACAGCGATACAAAATACTCACCAAGCTTTCAGGGCCAGGTCACAATTAG
C
 
GTGGATAAGTCCGTCTCTACTGCCTATCTGCAGTGGAGCTCCCTGAAACCTAGTGACTC
A
GCCGTGTACTTCTGCGCTCGCCACGACGTCGGCTATTGCACAGATCGAACTTGTGCCAA
G
TGGCCAGAGTGGCTGGGAGTGTGGGGACAGGGAACCCTGGTGACAGTCTCTAGTGGGG
GA
GGCGGGTCAAGCGGAGGAGGGTCCGGAGGAGGAGGAAGCCAGTCCGTGCTGACCCAG
CCC
CCTTCTGTCAGTGCCGCTCCTGGCCAGAAGGTGACAATCTCATGCAGCGGGTCCTCTAG
T
AACATTGGAAACAATTACGTGAGCTGGTATCAGCAGCTGCCAGGGACCGCTCCCAAGC
TG
CTGATCTACGATCATACAAATAGACCTGCAGGAGTGCCAGACAGGTTTTCCGGCTCTAA
A
AGTGGGACCTCAGCCAGCCTGGCTATTAGCGGCTTCCGGTCCGAGGACGAAGCAGATT
AC
TATTGTGCCTCCTGGGACTATACACTGTCTGGCTGGGTGTTCGGCGGGGGAACTAAGCT
G
ACCGTCCTGGGGTGAGGATCC
138. SEQ ID NO: 156 (CL_#1102_MM-111, AA) 


















*GS
139. SEQ ID NO: 157 (V1088) 
GAATTCGCGACCATGGCTGTGATGGCTCCTAGAACACTGGTGCTGCTGCTGTCCGGGGC
T
CTGGCTCTGACTCAGACTTGGGCTGGGCAGGTGCAGCTGCAGGAGTCTGGAGGAGGAC
 
TG
GTGAAGCCAGGAGGGTCTCTGCGACTGAGTTGCGCCGCTTCAGGCTTCACCTTTAGCTC
C
TACTGGATGAGCTGGGTCCGCCAGGCACCAGGGAAGGGACTGGAGTGGGTGGCCAACA
TC
AATCGAGACGGGAGCGCTTCCTACTATGTGGATAGCGTCAAGGGAAGGTTTACAATTAG
T
CGCGACGATGCCAAAAACTCACTGTACCTGCAGATGAATAGCCTGCGAGCTGAGGATA
CT
GCAGTCTACTATTGCGCTAGAGACAGGGGCGTGGGGTATTTCGATCTGTGGGGAAGAG
GC
ACTCTGGTGACCGTCTCTAGTGCAAGCACCGGAGGAGGAGGATCAGGCGGAGGAGGCA
GC
GGAGGAGGCGGGTCTCAGAGTGCCCTGACCCAGCCAGCTTCAGTGAGCGGGTCCCCAG
GA
CAGAGCATCACAATTTCCTGTACTGGCACCTCAAGCGACGTCGGAGGCTACAACTTTGT
G
AGCTGGTATCAGCAGCACCCAGGCAAAGCCCCCAAGCTGATGATCTACGACGTCTCCG
AT
CGACCTAGCGGGGTGTCCGATCGGTTCTCTGGAAGTAAATCAGGCAATACCGCCTCTCT
G
ATCATTAGTGGGCTGCAGGCCGACGATGAGGCTGACTACTATTGCTCCTCTTATGGAAG
T
TCAAGCACACATGTGATCTTCGGGGGAGGCACAAAAGTGACTGTCCTGGGAGCAGCCT
CC
GATGCACACAAATCTGAGGTGGCCCATCGGTTCAAGGACCTGGGCGAGGAAAACTTTA
AA
GCCCTGGTGCTGATTGCCTTCGCTCAGTACCTGCAGCAGAGCCCCTTTGAGGACCACGT
G
AAGCTGGTCAACGAGGTGACCGAGTTCGCCAAAACATGCGTGGCAGACGAGTCCGCCG
AA
AATTGTGATAAGTCTCTGCATACTCTGTTTGGAGATAAACTGTGTACCGTGGCCACACT
G
CGGGAGACCTATGGCGAAATGGCTGACTGCTGTGCAAAGCAGGAGCCCGAAAGAAACG
AG
TGCTTCCTGCAGCACAAAGACGATAACCCCAATCTGCCTCGCCTGGTGCGACCTGAAGT
G
GACGTCATGTGTACTGCCTTCCACGATAATGAGGAAACCTTTCTGAAGAAATACCTGTA
T
GAGATCGCTCGGAGACATCCCTACTTTTATGCCCCTGAACTGCTGTTCTTTGCTAAAAGA
TACAAGGCTGCATTCACCGAGTGCTGTCAGGCCGCTGATAAAGCAGCCTGCCTGCTGCC
C
AAGCTGGACGAGCTGAGAGATGAAGGAAGGCTTCCTCTGCAAAACAGAGGCTGAAGT
GT
GCTAGCCTGCAGAAATTCGGCGAGAGGGCCTTCAAGGCATGGGCAGTGGCTCGACTGA
GC
CAGAGATTCCCTAAGGCCGAGTTTGCTGAAGTCTCCAAACTGGTGACTGACCTGACCAA
 
G
GTGCACACAGAGTGCTGTCATGGCGACCTGCTGGAATGCGCCGACGATCGCGCAGATCT
G
GCCAAGTACATCTGTGAGAATCAGGACTCTATTAGTTCAAAGCTGAAAGAGTGCTGTGA
A
AAACCCCTGCTGGAGAAGAGCCACTGCATCGCCGAGGTGGAAAACGACGAAATGCCTG
CT
GATCTGCCAAGTCTGGCTGCAGACTTTGTCGAGTCAAAAGATGTGTGTAAGAATTACGC
A
GAAGCCAAGGATGTGTTCCTGGGCATGTTTCTGTACGAGTATGCAAGGCGCCACCCTGA
C
TACTCCGTGGTCCTGCTGCTGAGGCTGGCTAAGACCTATGAGACCACACTGGAAAAATG
C
TGTGCCGCTGCAGATCCACATGAGTGCTATGCCAAAGTCTTCGACGAGTTCAAGCCACT
G
GTGGAGGAACCCCAGAACCTGATTAAGCAGAATTGTGAGCTGTTTGAACAGCTGGGCG
AG
TACAAATTCCAGAACGCCCTGCTGGTGCGCTATACAAAGAAAGTCCCTCAGGTGAGCAC
A
CCAACTCTGGTGGAAGTCTCCAGGAATCTGGGCAAGGTCGGGTCTAAATGCTGTAAGCA
C
CCCGAGGCTAAACGCATGCCTTGCGCAGAAGATTACCTGTCTGTGGTCCTGAACCAGCT
G
TGTGTGCTGCATGAGAAGACACCAGTCAGTGACCGGGTGACAAAATGCTGTACTGAAA
GT
CTGGTGAATCGACGGCCTTGCTTTTCAGCCCTGGAGGTCGATGAAACTTATGTGCCAAA
G
GAGTTCCAGGCAGAAACCTTCACATTTCACGCCGACATCTGTACACTGTCCGAGAAAGA
A
AGACAGATTAAGAAACAGACTGCCCTGGTCGAGCTGGTGAAGCATAAACCCAAGGCTA
CT
AAAGAACAGCTGAAGGCAGTCATGGACGATTTCGCCGCTTTTGTGGAGAAATGCTGTAA
G
GCAGACGATAAGGAAACCTGCTTCGCCGAGGAAGGCAAGAAACTGGTGGCAGCCTCTC
AG
GCTGCACTGGGGCTGTGAGGATCC
140. SEQ ID NO: 158 (V1088, AA) 
EFATMAVMAPRTLVLLLSGALALTQTWAGQVQLQESGGGLVKPGGSLRLSCAASGFTFSS
YWMSWVRQAPGKGLEWVANINRDGSASYYVDSVKGRFTISRDDAKNSLYLQMNSLRAED
TAVYYCARDRGVGYFDLWGRGTLVTVSSASTGGGGSGGGGSGGGGSQSALTQPASVSGSP
GQSITISCTGTSSDVGGYNFVSWYQQHPGKAPKLMIYDVSDRPSGVSDRFSGSKSGNTASLII
SGLQADDEADYYCSSYGSSSTHVIFGGGTKVTVLGAASDAHKSEVAHRFKDLGEENFKAL
VLIAFAQYLQQSPFEDHVKLVNEVTEFAKTCVADESAENCDKSLHTLFGDKLCTVATLRET
YGEMADCCAKQEPERNECFLQHKDDNPNLPRLVRPEVDVMCTAFHDNEETFLKKYLYEIA
RRHPYFYAPELLFFAKRYKAAFTECCQAADKAACLLPKLDELRDEGKASSAKQRLKCASLQ
 
KFGERAFKAWAVARLSQRFPKAEFAEVSKLVTDLTKVHTECCHGDLLECADDRADLAKYI
CENQDSISSKLKECCEKPLLEKSHCIAEVENDEMPADLPSLAADFVESKDVCKNYAEAKDV
FLGMFLYEYARRHPDYSVVLLLRLAKTYETTLEKCCAAADPHECYAKVFDEFKPLVEEPQN
LIKQNCELFEQLGEYKFQNALLVRYTKKVPQVSTPTLVEVSRNLGKVGSKCCKHPEAKRMP
CAEDYLSVVLNQLCVLHEKTPVSDRVTKCCTESLVNRRPCFSALEVDETYVPKEFQAETFTF
HADICTLSEKERQIKKQTALVELVKHKPKATKEQLKAVMDDFAAFVEKCCKADDKETCFA
EEGKKLVAASQAALGL*GS
141. SEQ ID NO: 159 (V1089, NT) 
GAATTCGCGACCATGGCTGTGATGGCTCCACGAACCCTGGTCCTGCTGCTGTCCGGCGC
A
CTGGCACTGACTCAGACTTGGGCTGGGATGCTCATAAGTCTGAGGTGGCACACAGGTT
C
AAAGATCTGGGCGAGGAAAACTTTAAGGCCCTGGTCCTGATCGCTTTCGCACAGTACCT
G
CAGCAGAGCCCTTTTGAGGACCACGTGAAACTGGTCAACGAGGTGACCGAGTTCGCCA
AG
ACATGCGTGGCTGACGAGTCAGCAGAAAATTGTGATAAAAGCCTGCATACCCTGTTTGG
G
GATAAGCTGTGCACCGTGGCCACACTGAGAGAGACATACGGAGAAATGGCCGACTGCT
GT
GCTAAACAGGAGCCTGAAAGGAACGAGTGCTTCCTGCAGCATAAGGACGATAACCCAA
AT
CTGCCCAGACTGGTGAGGCCAGAAGTGGACGTCATGTGTACTGCTTTCCACGATAATGA
G
GAAACCTTTCTGAAGAAATACCTGTATGAGATCGCCCGGAGACATCCTTACTTTTATGC
A
CCAGAACTGCTGTTCTTTGCCAAACGCTACAAGGCCGCTTTCACCGAGTGCTGTCAGGC
A
GCCGATAAAGCTGCATGCCTGCTGCCCAAGCTGGACGAGCTGCGAGATGAAGGGAAGG
CA
AGCTCCGCCAAACAGCGGCTGAAGTGTGCTAGCCTGCAGAAATTCGGAGAGCGAGCCT
TC
AAGGCATGGGCTGTGGCACGACTGAGTCAGCGATTCCCTAAGGCTGAGTTTGCAGAAGT
C
TCAAAACTGGTGACTGACCTGACCAAGGTGCACACCGAGTGCTGTCATGGCGACCTGCT
G
GAATGCGCCGACGATAGAGCCGATCTGGCTAAGTACATCTGTGAGAATCAGGACTCTAT
T
TCTAGTAAGCTGAAAGAGTGCTGTGAAAAACCCCTGCTGGAGAAGAGCCACTGCATCG
CC
GAGGTGGAAAACGACGAAATGCCTGCTGATCTGCCATCCCTGGCCGCTGACTTCGTGGA
G
TCTAAAGATGTCTGTAAGAATTACGCCGAAGCTAAGGATGTGTTCCTGGGCATGTTTCT
G
TACGAGTATGCTAGGCGCCACCCAGACTACAGCGTGGTCCTGCTGCTGCGGCTGGCCAA
 
A
ACCTATGAGACCACACTGGAAAAGTGCTGTGCAGCCGCTGATCCCCATGAGTGCTATGC
C
AAAGTGTTCGACGAGTTCAAGCCCCTGGTCGAGGAACCTCAGAACCTGATCAAGCAGA
AT
TGTGAGCTGTTTGAACAGCTGGGCGAGTACAAGTTCCAGAACGCCCTGCTGGTGAGATA
T
ACCAAGAAAGTCCCACAGGTGAGTACTCCCACCCTGGTGGAGGTCTCACGCAATCTGG
GA
AAAGTGGGCAGCAAATGCTGTAAGCACCCTGAGGCCAAGCGAATGCCATGCGCTGAAG
AT
TACCTGTCTGTGGTCCTGAACCAGCTGTGTGTGCTGCATGAGAAAACTCCAGTCAGTGA
C
AGGGTGACAAAGTGCTGTACTGAATCCCTGGTGAATCGACGGCCATGCTTTTCTGCCCT
G
GAGGTGGATGAAACATATGTCCCCAAAGAGTTCCAGGCTGAAACATTCACTTTTCACGC
A
GACATCTGTACTCTGTCCGAGAAGGAACGCCAGATTAAGAAACAGACCGCCCTGGTCG
AG
CTGGTGAAGCATAAACCCAAGGCCACAAAAGAACAGCTGAAGGCTGTGATGGACGATT
TC
GCAGCCTTTGTCGAGAAATGCTGTAAGGCAGACGATAAGGAAACTTGCTTTGCCGAGG
AA
GGCAAGAAACTGGTGGCTGCAAGCCAGGCAGCTCTGGGACTGGCAGCAGCTCTGCAGG
TC
CAGCTGGTGCAGTCCGGAGCAGAGGTGAAGAAACCTGGAGAAAGTCTGAAAATCTCCT
GT
AAGGGAAGCGGCTACTCCTTCACCTCTTATTGGATTGCCTGGGTGCGGCAGATGCCAGG
G
AAAGGACTGGAGTACATGGGCCTGATCTATCCCGGGGACAGCGATACAAAGTACAGTC
CT
TCATTTCAGGGCCAGGTCACAATTTCCGTGGATAAAAGCGTCTCCACTGCCTATCTGCA
G
TGGTCAAGCCTGAAGCCCTCTGACAGTGCAGTGTACTTCTGCGCCAGGCACGACGTCGG
C
TATTGCACAGATCGCACTTGTGCCAAGTGGCCTGAGTGGCTGGGAGTGTGGGGACAGG
GA
ACCCTGGTCACAGTCTCCTCTGGCGGAGGAGGCAGTTCAGGAGGAGGCAGCGGAGGAG
GA
GGGTCACAGAGCGTGCTGACACAGCCACCTTCCGTCTCTGCAGCACCAGGACAGAAAG
TG
ACTATCAGTTGCTCAGGAAGCTCCTCTAACATTGGCAACAATTACGTGTCCTGGTATCA
G
CAGCTGCCCGGAACCGCTCCTAAGCTGCTGATCTACGATCACACAAATCGACCAGCAGG
A
GTGCCAGACCGGTTCAGCGGGTCCAAGTCTGGAACTAGTGCATCACTGGCCATTAGCGG
G
 
TTCAGGTCCGAGGACGAAGCTGATTACTATTGCGCATCTTGGGACTATACCCTGAGTGG
A
TGGGTGTTCGGAGGCGGGACTAAGCTGACCGTCCTGGGCTGAGGATCC
142. SEQ ID NO: 160 (V1089, AA) 
EFATMAVMAPRTLVLLLSGALALTQTWAGDAHKSEVAHRFKDLGEENFKALVLIAFAQYL
QQSPFEDHVKLVNEVTEFAKTCVADESAENCDKSLHTLFGDKLCTVATLRETYGEMADCC
AKQEPERNECFLQHKDDNPNLPRLVRPEVDVMCTAFHDNEETFLKKYLYEIARRHPYFYAP
ELLFFAKRYKAAFTECCQAADKAACLLPKLDELRDEGKASSAKQRLKCASLQKFGERAFK
AWAVARLSQRFPKAEFAEVSKLVTDLTKVHTECCHGDLLECADDRADLAKYICENQDSISS
KLKECCEKPLLEKSHCIAEVENDEMPADLPSLAADFVESKDVCKNYAEAKDVFLGMFLYE
YARRHPDYSVVLLLRLAKTYETTLEKCCAAADPHECYAKVFDEFKPLVEEPQNLIKQNCEL
FEQLGEYKFQNALLVRYTKKVPQVSTPTLVEVSRNLGKVGSKCCKHPEAKRMPCAEDYLS
VVLNQLCVLHEKTPVSDRVTKCCTESLVNRRPCFSALEVDETYVPKEFQAETFTFHADICTL
SEKERQIKKQTALVELVKHKPKATKEQLKAVMDDFAAFVEKCCKADDKETCFAEEGKKLV
AASQAALGLAAALQVQLVQSGAEVKKPGESLKISCKGSGYSFTSYWIAWVRQMPGKGLEY
MGLIYPGDSDTKYSPSFQGQVTISVDKSVSTAYLQWSSLKPSDSAVYFCARHDVGYCTDRT
CAKWPEWLGVWGQGTLVTVSSGGGGSSGGGSGGGGSQSVLTQPPSVSAAPGQKVTISCSG
SSSNIGNNYVSWYQQLPGTAPKLLIYDHTNRPAGVPDRFSGSKSGTSASLAISGFRSEDEAD
YYCASWDYTLSGWVFGGGTKLTVLG*GS
143. SEQ ID NO: 161 (CL_#1105_antiHER3_1_HSA-1_337_DSS) 
GAATTCGCGACCATGGCCGTGATGGCTCCTAGAACACTGGTGCTGCTGCTGTCCGGGGC
T
CTGGCTCTGACTCAGACTTGGGCTGGGCAGGTGCAGCTGCAGGAGAGCGGAGGAGGAC
TG
GTGAAACCAGGAGGGTCTCTGCGACTGAGTTGCGCAGCTTCAGGCTTCACCTTTAGCTC
C
TACTGGATGTCCTGGGTCCGACAGGCACCTGGGAAGGGACTGGAATGGGTGGCCAACA
TC
AATCGGGACGGAAGCGCTTCCTACTATGTGGATAGCGTCAAAGGCCGCTTTACCATTAG
T
CGAGACGATGCTAAGAACTCACTGTACCTGCAGATGAATAGCCTGCGCGCTGAGGATA
CA
GCAGTCTACTATTGCGCAAGGGACCGAGGAGTGGATATTTCGATCTGTGGGGACGAG
GC
ACTCTGGTGACCGTCTCTAGTGCCAGCACAGGCGGAGGAGGATCAGGAGGAGGAGGCA
GC
GGAGGAGGCGGGTCTCAGAGTGCCCTGACTCAGCCAGCTTCAGTGAGCGGGTCCCCCG
GA
CAGAGCATCACCATTTCCTGTACCGGCACATCAAGCGACGTCGGAGGCTACAACTTTGT
G
TCCTGGTATCAGCAGCACCCAGGAAAGGCCCCCAAACTGATGATCTACGACGTCTCTGA
T
CGGCCTAGCGGCGTGTCCGATAGATTCTCTGGCAGTAAGTCAGGGAATACTGCCTCTCT
 
G
ATCATTAGTGGCCTGCAGGCCGACGATGAGGCTGACTACTATTGCTCCTCTTATGGGAG
T
TCAAGCACCCATGTGATCTTTGGGGGAGGCACAAAAGTGACTGTCCTGGGGGAGGCT
CC
GATGCACACAAGTCTGAGGTCGCCCATAGGTTCAAAGACCTGGGCGAGGAAAACTTTA
AG
GCCCTGGTGCTGATTGCTTTCGCACAGTACCTGCAGCAGTGCCCATTTGAAGATCACGT
G
AAACTGGTCAACGAGGTGACAGAGTTCGCCAAGACTTGCGTGGCAGACGAGTCCGCCG
AA
AATTGTGATAAATCTCTGCATACACTGTTTGGGGATAAGCTGTGTACCGTGGCCACACT
G
AGAGAGACTTATGGAGAAATGGCTGACTGCTGTGCAAAACAGGAGCCTGAAAGGAACG
AG
TGCTTCCTGCAGCACAAGGACGATAACCCCAATCTGCCTCGACTGGTGCGGCCAGAAGT
G
GACGTCATGTGTACCGCTTTCCACGATAATGAGGAAACATTTCTGAAGAAATACCTGTA
T
GAGATCGCCCGGAGACATCCCTACTTTTATGCTCCTGAACTGCTGTTCTTTGCAAAGCGC
TACAAAGCAGCCTTCACAGAGTGCTGTCAGGCTGCAGATAAGGCCGCTTGCCTGCTGCC
A
AAACTGGACGAGCTGAGGGATGAAGGAAAGCTTCCTCTGCAAAGCAGCGCCTGAAAT
GT
GCATCCCTGCAGAAGTTCGGCGAGCGGGCCTTTAAAGCCTGGGCTGTGGCAAGACTGTC
C
CAGAGGTTCCCCAAGGCCGAGTTTGCTGAAGTCTCTAAGCTGGTGACTGACCTGACCAA
A
GTGCACACTGAGTGCTGTCATGGCGACCTGCTGGAATGCGCCGACGATAGAGCAGATCT
G
GCCAAGTACATCTGTGAGAATCAGGACAGCATTAGTTCAAAGCTGAAAGAGTGCTGTG
AA
AAGCCCCTGCTGGAGAAAAGCCACTGCATTGCTGAGGTGGAAAACGACGAAATGCCTG
CA
GATCTGCCAAGTCTGGCAGCCGACTTCGTCGAGAGCAAGGATGTGTGTAAAAATTATGC
C
GAAGCTAAGGACGTGTTCCTGGGCATGTTTCTGTACGAGTATGCAAGAGCCTGAGGATC
C
144. SEQ ID NO: 162 (CL_#1105_antiHER3_1_HSA-1_337_DSS) 
EFATMAVMAPRTLVLLLSGALALTQTWAGQVQLQESGGGLVKPGGSLRLSCAASGFTFSS
YWMSWVRQAPGKGLEWVANINRDGSASYYVDSVKGRFTISRDDAKNSLYLQMNSLRAED
TAVYYCARDRGVGYFDLWGRGTLVTVSSASTGGGGSGGGGSGGGGSQSALTQPASVSGSP
GQSITISCTGTSSDVGGYNFVSWYQQHPGKAPKLMIYDVSDRPSGVSDRFSGSKSGNTASLII
SGLQADDEADYYCSSYGSSSTHVIFGGGTKVTVLGGGSDAHKSEVAHRFKDLGEENFKAL
VLIAFAQYLQQCPFEDHVKLVNEVTEFAKTCVADESAENCDKSLHTLFGDKLCTVATLRET
 
YGEMADCCAKQEPERNECFLQHKDDNPNLPRLVRPEVDVMCTAFHDNEETFLKKYLYEIA
RRHPYFYAPELLFFAKRYKAAFTECCQAADKAACLLPKLDELRDEGKASSAKQRLKCASLQ
KFGERAFKAWAVARLSQRFPKAEFAEVSKLVTDLTKVHTECCHGDLLECADDRADLAKYI
CENQDSISSKLKECCEKPLLEKSHCIAEVENDEMPADLPSLAADFVESKDVCKNYAEAKDV
FLGMFLYEYARA*GS
145. SEQ ID NO: 163 (CL_#668_HSA-342-585DSS_4D5scFv-Subcloning) 
GAATTCGCCACTATGGCCGTCATGGCACCTAGAACACTGGTCCTGCTGCTGAGCGGGGC
A
CTGGCACTGACACAGACTTGGGCTGGGTCCGTCGTCCTGCTGCTGAGACTGGCTAAGAC
C
TACGAGACCACACTGGAAAAATGCTGTGCCGCTGCAGACCCCCACGAGTGCTATGCCA
AG
GTGTTCGATGAGTTCAAGCCTCTGGTCGAGGAACCACAGAACCTGATCAAGCAGAATTG
T
GAGCTGTTCGAACAGCTGGGCGAGTACAAATTTCAGAACGCCCTGCTGGTGAGGTATAC
A
AAGAAAGTGCCCCAGGTCTCTACACCTACTCTGGTGGAGGTCAGTAGGAATCTGGGCAA
G
GTCGGGTCAAAATGCTGTAAGCACCCAGAGGCCAAACGCATGCCCTGCGCTGAAGACT
AC
CTGTCTGTGGTCCTGAACCAGCTGTGTGTGCTGCATGAGAAGACCCCTGTGAGCGATCG
A
GTCACCAAATGCTGTACAGAAAGCCTGGTGAATCGGAGACCCTGCTTTTCCGCTCTGGA
G
GTGGACGAAACATATGTCCCTAAGGAGTTCAATGCAGAAACCTTCACATTTCACGCCGA
T
ATCTGTACTCTGTCCGAGAAGGAACGCCAGATTAAGAAACAGACCGCCCTGGTGGAGC
TG
GTCAAGCATAAACCAAAGGCTACTAAGGAACAGCTGAAAGCAGTGATGGACGATTTCG
CC
GCTTTTGTCGAGAAATGCTGTAAGGCAGACGATAAGGAAACCTGCTTTGCCGAGGAAG
GC
AAGAAACTGGTGGCAGCCAGCCAGGCTGCACTGGGACTGGGAGGGTCCGGAGGCTCTG
GA
GGAAGTGGAGGGTCAGGAGGCGACATCCAGATGACACAGAGCCCAAGCTCCCTGTCAG
CA
AGCGTGGGCGACCGAGTCACTATTACCTGTCGGGCCTCCCAGGATGTGAATACTGCAGT
C
GCCTGGTACCAGCAGAAACCAGGAAAGGCTCCCAAACTGCTGATCTACTCCGCATCTTT
C
CTGTATAGCGGCGTGCCATCCAGGTTTAGTGGATCACGCAGCGGCACAGACTTCACACT
G
ACTATTTCTAGTCTGCAGCCCGAGGATTTTGCCACTTACTATTGCCAGCAGCACTATACT
ACCCCCCCTACCTTCGGACAGGGCACAAAGGTGGAGATCAAGGGAGGATCTGGAGGAG
 
GA
AGTGGAGGAGGATCAGGAGGAGGAAGCGGAGGAGGCAGCGGAGAGGTGCAGCTGGTC
GAA
TCTGGAGGAGGACTGGTGCAGCCTGGAGGGTCTCTGCGACTGAGTTGTGCCGCTTCAGG
C
TTTAACATCAAGGACACCTACATTCATTGGGTGCGGCAGGCACCTGGGAAGGGACTGG
AG
TGGGTCGCTAGAATCTATCCAACTAATGGGTACACCAGATATGCCGACAGCGTGAAGG
GA
AGGTTCACCATTAGCGCCGATACATCCAAAAACACTGCTTACCTGCAGATGAACAGCCT
G
CGCGCTGAGGATACAGCAGTGTACTATTGCAGTCGATGGGGCGGCGATGGGTTCTACGC
A
ATGGACTACTGGGGACAGGGGACTCTGGTCACCGTCAGCAGCTGAGGATCC
146. SEQ ID NO: 164 (CL_#668_HSA-342-585DSS_4D5scFv-Subcloning, AA) 
EFATMAVMAPRTLVLLLSGALALTQTWAGSVVLLLRLAKTYETTLEKCCAAADPHECYAK
VFDEFKPLVEEPQNLIKQNCELFEQLGEYKFQNALLVRYTKKVPQVSTPTLVEVSRNLGKV
GSKCCKHPEAKRMPCAEDYLSVVLNQLCVLHEKTPVSDRVTKCCTESLVNRRPCFSALEVD
ETYVPKEFNAETFTFHADICTLSEKERQIKKQTALVELVKHKPKATKEQLKAVMDDFAAFV
EKCCKADDKETCFAEEGKKLVAASQAALGLGGSGGSGGSGGSGGDIQMTQSPSSLSASVG
DRVTITCRASQDVNTAVAWYQQKPGKAPKLLIYSASFLYSGVPSRFSGSRSGTDFTLTISSLQ
PEDFATYYCQQHYTTPPTFGQGTKVEIKGGSGGGSGGGSGGGSGGGSGEVQLVESGGGLV
QPGGSLRLSCAASGFNIKDTYIHWVRQAPGKGLEWVARIYPTNGYTRYADSVKGRFTISAD
TSKNTAYLQMNSLRAEDTAVYYCSRWGGDGFYAMDYWGQGTLVTVSS*GS
147. SEQ ID NO 165 (CL_#1105_antiHER3_1_HSA-1_337_DSS> 
GAATTCGCGACCATGGCCGTGATGGCTCCTAGAACACTGGTGCTGCTGCTGTCCGGGGC
T
CTGGCTCTGACTCAGACTTGGGCTGGGCAGGTGCAGCTGCAGGAGAGCGGAGGAGGAC
TG
GTGAAACCAGGAGGGTCTCTGCGACTGAGTTGCGCAGCTTCAGGCTTCACCTTTAGCTC
C
TACTGGATGTCCTGGGTCCGACAGGCACCTGGGAAGGGACTGGAATGGGTGGCCAACA
TC
AATCGGGACGGAAGCGCTTCCTACTATGTGGATAGCGTCAAAGGCCGCTTTACCATTAG
T
CGAGACGATGCTAAGAACTCACTGTACCTGCAGATGAATAGCCTGCGCGCTGAGGATA
CA
GCAGTCTACTATTGCGCAAGGGACCGAGGAGTGGGATATTTCGATCTGTGGGGACGAG
GC
ACTCTGGTGACCGTCTCTAGTGCCAGCACAGGCGGAGGAGGATCAGGAGGAGGAGGCA
GC
GGAGGAGGCGGGTCTCAGAGTGCCCTGACTCAGCCAGCTTCAGTGAGCGGGTCCCCCG
GA
 
CAGAGCATCACCATTTCCTGTACCGGCACATCAAGCGACGTCGGAGGCTACAACTTTGT
G
TCCTGGTATCAGCAGCACCCAGGAAAGGCCCCCAAACTGATGATCTACGACGTCTCTGA
T
CGGCCTAGCGGCGTGTCCGATAGATTCTCTGGCAGTAAGTCAGGGAATACTGCCTCTCT
G
ATCATTAGTGGCCTGCAGGCCGACGATGAGGCTGACTACTATTGCTCCTCTTATGGGAG
T
TCAAGCACCCATGTGATCTTTGGGGGAGGCACAAAAGTGACTGTCCTGGGGGGAGGCT
CC
GATGCACACAAGTCTGAGGTCGCCCATAGGTTCAAAGACCTGGGCGAGGAAAACTTTA
AG
GCCCTGGTGCTGATTGCTTTCGCACAGTACCTGCAGCAGTGCCCATTTGAAGATCACGT
G
AAACTGGTCAACGAGGTGACAGAGTTCGCCAAGACTTGCGTGGCAGACGAGTCCGCCG
AA
AATTGTGATAAATCTCTGCATACACTGTTTGGGGATAAGCTGTGTACCGTGGCCACACT
G
AGAGAGACTTATGGAGAAATGGCTGACTGCTGTGCAAAACAGGAGCCTGAAAGGAACG
AG
TGCTTCCTGCAGCACAAGGACGATAACCCCAATCTGCCTCGACTGGTGCGGCCAGAAGT
G
GACGTCATGTGTACCGCTTTCCACGATAATGAGGAAACATTTCTGAAGAAATACCTGTA
T
GAGATCGCCCGGAGACATCCCTACTTTTATGCTCCTGAACTGCTGTTCTTTGCAAAGCGC
TACAAAGCAGCCTTCACAGAGTGCTGTCAGGCTGCAGATAAGGCCGCTTGCCTGCTGCC
A
AAACTGGACGAGCTGAGGGATGAAGGAAAGCTTCCTCTGCAAAGCAGCGCCTGAAAT
GT
GCATCCCTGCAGAAGTTCGGCGAGCGGGCCTTTAAAGCCTGGGCTGTGGCAAGACTGTC
C
CAGAGGTTCCCCAAGGCCGAGTTTGCTGAAGTCTCTAAGCTGGTGACTGACCTGACCAA
A
GTGCACACTGAGTGCTGTCATGGCGACCTGCTGGAATGCGCCGACGATAGAGCAGATCT
G
GCCAAGTACATCTGTGAGAATCAGGACAGCATTAGTTCAAAGCTGAAAGAGTGCTGTG
AA
AAGCCCCTGCTGGAGAAAAGCCACTGCATTGCTGAGGTGGAAAACGACGAAATGCCTG
CA
GATCTGCCAAGTCTGGCAGCCGACTTCGTCGAGAGCAAGGATGTGTGTAAAAATTATGC
C
GAAGCTAAGGACGTGTTCCTGGGCATGTTTCTGTACGAGTATGCAAGAGCCTGAGGATC
C
148. SEQ ID NO: 166 (CL_#1105_antiHER3_1_HSA-1_337_DSS, AA) 
EFATMAVMAPRTLVLLLSGALALTQTWAGQVQLQESGGGLVKPGGSLRLSCAASGFTFSS
YWMSWVRQAPGKGLEWVANINRDGSASYYVDSVKGRFTISRDDAKNSLYLQMNSLRAED
TAVYYCARDRGVGYFDLWGRGTLVTVSSASTGGGGSGGGGSGGGGSQSALTQPASVSGSP
GQSITISCTGTSSDVGGYNFVSWYQQHPGKAPKLMIYDVSDRPSGVSDRFSGSKSGNTASLII
SGLQADDEADYYCSSYGSSSTHVIFGGGTKVTVLGGGSDAHKSEVAHRFKDLGEENFKAL
VLIAFAQYLQQCPFEDHVKLVNEVTEFAKTCVADESAENCDKSLHTLFGDKLCTVATLRET
YGEMADCCAKQEPERNECFLQHKDDNPNLPRLVRPEVDVMCTAFHDNEETFLKKYLYEIA
RRHPYFYAPELLFFAKRYKAAFTECCQAADKAACLLPKLDELRDEGKASSAKQRLKCASLQ
KFGERAFKAWAVARLSQRFPKAEFAEVSKLVTDLTKVHTECCHGDLLECADDRADLAKYI
CENQDSISSKLKECCEKPLLEKSHCIAEVENDEMPADLPSLAADFVESKDVCKNYAEAKDV
FLGMFLYEYARA*GS
149. SEQ ID NO: 167 (CL_#667_4D5scFv_HSA-342-585DSS-Subcloning) 
GAATTCGCCACTATGGCCGTGATGGCACCTAGAACACTGGTCCTGCTGCTGAGCGGGGC
A
CTGGCACTGACACAGACTTGGGCTGGAGACATTCAGATGACACAGAGCCCAAGCTCCC
TG
TCCGCATCTGTGGGCGACCGAGTCACAATCACTTGCCGGGCCTCCCAGGATGTGAACAC
T
GCTGTCGCATGGTACCAGCAGAAACCAGGGAAGGCTCCCAAACTGCTGATCTACAGTG
CA
TCATTCCTGTATAGTGGCGTGCCATCAAGGTTTAGCGGCTCCCGATCTGGAACCGACTTC
ACCCTGACAATCTCTAGTCTGCAGCCCGAGGATTTTGCCACATACTATTGCCAGCAGCA
C
TATACCACACCCCCTACTTTCGGGCAGGGAACCAAGGTGGAGATCAAGGGAGGGAGCG
GA
GGAGGGTCCGGAGGAGGGTCTGGAGGCGGGAGTGGAGGAGGGTCAGGAGAGGTGCAG
CTG
GTCGAAAGCGGAGGAGGACTGGTGCAGCCTGGAGGCAGCCTGCGACTGTCCTGTGCCG
CT
TCTGGCTTTAACATCAAGGACACCTACATTCATTGGGTGCGGCAGGCACCTGGCAAAGG
A
CTGGAGTGGGTGGCTAGAATCTATCCAACTAATGGATACACCAGATATGCTGACAGCGT
G
AAGGGCAGGTTTACTATCAGTGCTGATACATCAAAGAACACTGCATACCTGCAGATGAA
T
AGCCTGCGCGCCGAGGATACCGCTGTGTACTATTGTAGCCGATGGGGGGGAGACGGCTT
C
TACGCCATGGATTATTGGGGACAGGGCACCCTGGTGACAGTCTCAAGCGGAGGGAGTG
GA
GGCTCAGGAGGAAGCGGAGGGTCCGGAGGCTCTGTGGTCCTGCTGCTGAGACTGGCTA
AG
ACCTACGAGACTACCCTGGAAAAATGCTGTGCAGCCGCTGACCCCCACGAGTGCTATGC
A
AAGGTGTTCGATGAGTTCAAGCCTCTGGTCGAGGAACCACAGAACCTGATCAAGCAGA
AT
 
TGTGAGCTGTTCGAACAGCTGGGCGAGTACAAGTTTCAGAACGCCCTGCTGGTGAGGTA
T
ACAAAGAAAGTGCCCCAGGTCAGCACTCCTACCCTGGTGGAGGTCTCCAGGAATCTGG
GG
AAGGTCGGATCTAAGTGCTGTAAACACCCAGAGGCAAAACGCATGCCCTGCGCCGAAG
AC
TACCTGTCCGTGGTCCTGAATCAGCTGTGTGTGCTGCATGAGAAGACCCCTGTGTCTGAT
CGAGTCACCAAATGCTGTACAGAAAGTCTGGTGAACCGGAGACCCTGCTTTTCTGCCCT
G
GAGGTGGACGAAACATATGTCCCTAAGGAGTTCAATGCCGAAACATTCACTTTTCACGC
T
GATATCTGTACACTGTCCGAGAAGGAACGCCAGATTAAGAAACAGACTGCTCTGGTGG
AG
CTGGTCAAGCATAAACCAAAGGCAACCAAGGAACAGCTGAAAGCCGTGATGGACGATT
TC
GCAGCCTTTGTCGAGAAGTGCTGTAAAGCCGACGATAAGGAAACTTGTTTCGCCGAGGA
A
GGCAAAAAACTGGTCGCAGCATCACAGGCAGCACTGGGACTGTGAGGATCC
150. SEQ ID NO: 168 (CL_#667_4D5scFv_HSA-342-585DSS-Subcloning) 
EFATMAVMAPRTLVLLLSGALALTQTWAGDIQMTQSPSSLSASVGDRVTITCRASQDVNTA
VAWYQQKPGKAPKLLIYSASFLYSGVPSRFSGSRSGTDFTLTISSLQPEDFATYYCQQHYTTP
PTFGQGTKVEIKGGSGGGSGGGSGGGSGGGSGEVQLVESGGGLVQPGGSLRLSCAASGFNI
KDTYIHWVRQAPGKGLEWVARIYPTNGYTRYADSVKGRFTISADTSKNTAYLQMNSLRAE
DTAVYYCSRWGGDGFYAMDYWGQGTLVTVSSGGSGGSGGSGGSGGSVVLLLRLAKTYET
TLEKCCAAADPHECYAKVFDEFKPLVEEPQNLIKQNCELFEQLGEYKFQNALLVRYTKKVP
QVSTPTLVEVSRNLGKVGSKCCKHPEAKRMPCAEDYLSVVLNQLCVLHEKTPVSDRVTKC
CTESLVNRRPCFSALEVDETYVPKEFNAETFTFHADICTLSEKERQIKKQTALVELVKHKPK
ATKEQLKAVMDDFAAFVEKCCKADDKETCFAEEGKKLVAASQAALGL*GS
151. SEQ ID NO: 169 (CL_#1105_antiHER3_1_HSA-1_337_DSS> 
GAATTCGCGACCATGGCCGTGATGGCTCCTAGAACACTGGTGCTGCTGCTGTCCGGGGC
T
CTGGCTCTGACTCAGACTTGGGCTGGGCAGGTGCAGCTGCAGGAGAGCGGAGGAGGAC
TG
GTGAAACCAGGAGGGTCTCTGCGACTGAGTTGCGCAGCTTCAGGCTTCACCTTTAGCTC
C
TACTGGATGTCCTGGGTCCGACAGGCACCTGGGAAGGGACTGGAATGGGTGGCCAACA
TC
AATCGGGACGGAAGCGCTTCCTACTATGTGGATAGCGTCAAAGGCCGCTTTACCATTAG
T
CGAGACGATGCTAAGAACTCACTGTACCTGCAGATGAATAGCCTGCGCGCTGAGGATA
CA
GCAGTCTACTATTGCGCAAGGGACCGAGGAGTGGGATATTTCGATCTGTGGGGACGAG
GC
 
ACTCTGGTGACCGTCTCTAGTGCCAGCACAGGCGGAGGAGGATCAGGAGGAGGAGGCA
GC
GGAGGAGGCGGGTCTCAGAGTGCCCTGACTCAGCCAGCTTCAGTGAGCGGTCCCCCG
GA
CAGAGCATCACCATTTCCTGTACCGGCACATCAAGCGACGTCGGAGGCTACAACTTTGT
G
TCCTGGTATCAGCAGCACCCAGGAAAGGCCCCCAAACTGATGATCTACGACGTCTCTGA
T
CGGCCTAGCGGCGTGTCCGATAGATTCTCTGGCAGTAAGTCAGGGAATACTGCCTCTCT
G
ATCATTAGTGGCCTGCAGGCCGACGATGAGGCTGACTACTATTGCTCCTCTTATGGGAG
T
TCAAGCACCCATGTGATCTTTGGGGGAGGCACAAAAGTGACTGTCCTGGGGGGAGGCT
CC
GATGCACACAAGTCTGAGGTCGCCCATAGGTTCAAAGACCTGGGCGAGGAAAACTTTA
AG
GCCCTGGTGCTGATTGCTTTCGCACAGTACCTGCAGCAGTGCCCATTTGAAGATCACGT
G
AAACTGGTCAACGAGGTGACAGAGTTCGCCAAGACTTGCGTGGCAGACGAGTCCGCCG
AA
AATTGTGATAAATCTCTGCATACACTGTTTGGGGATAAGCTGTGTACCGTGGCCACACT
G
AGAGAGACTTATGGAGAAATGGCTGACTGCTGTGCAAAACAGGAGCCTGAAAGGAACG
AG
TGCTTCCTGCAGCACAAGGACGATAACCCCAATCTGCCTCGACTGGTGCGGCCAGAAGT
G
GACGTCATGTGTACCGCTTTCCACGATAATGAGGAAACATTTCTGAAGAAATACCTGTA
T
GAGATCGCCCGGAGACATCCCTACTTTTATGCTCCTGAACTGCTGTTCTTTGCAAAGCGC
TACAAAGCAGCCTTCACAGAGTGCTGTCAGGCTGCAGATAAGGCCGCTTGCCTGCTGCC
A
AAACTGGACGAGCTGAGGGATGAAGGGAAAGCTTCCTCTGCAAAGCAGCGCCTGAAAT
GT
GCATCCCTGCAGAAGTTCGGCGAGCGGGCCTTTAAAGCCTGGGCTGTGGCAAGACTGTC
C
CAGAGGTTCCCCAAGGCCGAGTTTGCTGAAGTCTCTAAGCTGGTGACTGACCTGACCAA
A
GTGCACACTGAGTGCTGTCATGGCGACCTGCTGGAATGCGCCGACGATAGAGCAGATC
TG
GCCAAGTACATCTGTGAGAATCAGGACAGCATTAGTTCAAAGCTGAAAGAGTGCTGTG
AA
AAGCCCCTGCTGGAGAAAAGCCACTGCATTGCTGAGGTGGAAAACGACGAAATGCCTG
CA
GATCTGCCAAGTCTGGCAGCCGACTTCGTCGAGAGCAAGGATGTGTGTAAAAATTATGC
C
GAAGCTAAGGACGTGTTCCTGGGCATGTTTCTGTACGAGTATGCAAGAGCCTGAGGATC
C
152. SEQ ID NO: 170 (CL_#1105_antiHER3_1_HSA-1_337_DSS, aa) 
EFATMAVMAPRTLVLLLSGALALTQTWAGQVQLQESGGGLVKPGGSLRLSCAASGFTFSS
YWMSWVRQAPGKGLEWVANINRDGSASYYVDSVKGRFTISRDDAKNSLYLQMNSLRAED
TAVYYCARDRGVGYFDLWGRGTLVTVSSASTGGGGSGGGGSGGGGSQSALTQPASVSGSP
GQSITISCTGTSSDVGGYNFVSWYQQHPGKAPKLMIYDVSDRPSGVSDRFSGSKSGNTASLII
SGLQADDEADYYCSSYGSSSTHVIFGGGTKVTVLGGGSDAHKSEVAHRFKDLGEENFKAL
VLIAFAQYLQQCPFEDHVKLVNEVTEFAKTCVADESAENCDKSLHTLFGDKLCTVATLRET
YGEMADCCAKQEPERNECFLQHKDDNPNLPRLVRPEVDVMCTAFHDNEETFLKKYLYEIA
RRHPYFYAPELLFFAKRYKAAFTECCQAADKAACLLPKLDELRDEGKASSAKQRLKCASLQ
KFGERAFKAWAVARLSQRFPKAEFAEVSKLVTDLTKVHTECCHGDLLECADDRADLAKYI
CENQDSISSKLKECCEKPLLEKSHCIAEVENDEMPADLPSLAADFVESKDVCKNYAEAKDV
FLGMFLYEYARA*GS
153. SEQ ID NO: 171 (CL_#1106_HSA-342_585_DSS_antiHER2_2, nt) 
GAATTCGCGACCATGGCTGTGATGGCTCCTCGAACCCTGGTGCTGCTGCTGTCCGGCGC
T
CTGGCTCTGACTCAGACTTGGGCTGGCTCCGTGGTGCTGCTGCTGAGGCTGGCCAAAAC
C
TACGAGACCACACTGGAAAAGTGCTGTGCCGCTGCAGACCCTCACGAGTGCTATGCTAA
A
GTCTTCGATGAGTTCAAGCCCCTGGTGGAGGAACCTCAGAACCTGATCAAACAGAATTG
T
GAGCTGTTCGAACAGCTGGGCGAGTACAAGTTTCAGAACGCCCTGCTGGTGCGCTATAC
C
AAGAAAGTGCCACAGGTCAGCACTCCCACCCTGGTGGAGGTCTCCAGAAATCTGGGGA
AA
GTGGGATCTAAATGCTGTAAGCACCCTGAGGCAAAGAGGATGCCATGCGCCGAAGACT
AC
CTGAGCGTGGTCCTGAACCAGCTGTGTGTGCTGCATGAGAAAACCCCAGTGTCCGATCG
C
GTCACCAAGTGCTGTACAGAAAGCCTGGTGAACCGGAGACCATGCTTCTCCGCCCTGGA
G
GTCGACGAAACATACGTGCCCAAGGAGTTTAATGCTGAAACATTCACTTTCCACGCAGA
T
ATCTGTACCCTGAGCGAGAAGGAACGACAGATTAAGAAACAGACAGCCCTGGTGGAGC
TG
GTCAAGCATAAACCCAAGGCCACCAAAGAACAGCTGAAGGCTGTCATGGACGATTTCG
CC
GCTTTTGTGGAGAAATGCTGTAAGGCAGACGATAAGGAAACATGCTTCGCCGAGGAAG
GC
AAGAAACTGGTGGCAGCATCCCAGGCTGCACTGGGACTGGGAGGGAGTGGACAGGTGC
AG
CTGGTCCAGAGCGGAGCAGAGGTGAAGAAACCCGGCGAATCACTGAAAATCAGCTGTA
 
AG
GGGTCCGGATACTCTTTACTAGTTATTGGATTGCCTGGGTGCGGCAGATGCCTGGCAA
A
GGGCTGGAGTACATGGGGCTGATCTATCCCGGAGACTCTGATACAAAGTACTCACCTAG
C
TTCCAGGGCCAAGTGACTATTAGCGTCGACAAATCCGTGTCTACCGCTTATCTGCAGTG
G
AGCTCCCTGAAGCCTAGTGATTCAGCTGTCTACTTTTGCGCACGACACGACGTGGGCTA
T
TGCACCGATCGGACATGTGCCAAGTGGCCAGAGTGGCTGGGAGTGTGGGGACAGGGAA
CT
CTGGTGACCGTCTCTAGTGGAGGCGGGGGATCAAGCGGAGGAGGATCTGGAGGAGGAG
GC
AGCCAGTCCGTCCTGACTCAGCCACCTTCTGTGAGTGCAGCTCCAGGACAGAAGGTGAC
C
ATCTCATGCAGCGGCTCCTCTAGTAACATTGGGAACAATTACGTGAGCTGGTATCAGCA
G
CTGCCAGGAACAGCTCCCAAACTGCTGATCTACGACCATACTAATAGGCCTGCAGGCGT
G
CCAGATCGCTTCTCCGGCTCTAAGAGTGGGACATCAGCAAGCCTGGCCATTTCCGGCTT
T
AGATCTGAGGACGAAGCCGATTACTATTGTGCTAGTTGGGACTATACTCTGTCAGGGTG
G
GTCTTCGGGGGAGGCACAAAGCTGACTGTGCTGGGATGAGGATCC
154. SEQ ID NO: 172 (CL_#1106_HSA-342_585_DSS_antiHER2_2, AA) 
EFATMAVMAPRTLVLLLSGALALTQTWAGSVVLLLRLAKTYETTLEKCCAAADPHECYAK
VFDEFKPLVEEPQNLIKQNCELFEQLGEYKFQNALLVRYTKKVPQVSTPTLVEVSRNLGKV
GSKCCKHPEAKRMPCAEDYLSVVLNQLCVLHEKTPVSDRVTKCCTESLVNRRPCFSALEVD
ETYVPKEFNAETFTFHADICTLSEKERQIKKQTALVELVKHKPKATKEQLKAVMDDFAAFV
EKCCKADDKETCFAEEGKKLVAASQAALGLGGSGQVQLVQSGAEVKKPGESLKISCKGSG
YSFTSYWIAWVRQMPGKGLEYMGLIYPGDSDTKYSPSFQGQVTISVDKSVSTAYLQWSSLK
PSDSAVYFCARHDVGYCTDRTCAKWPEWLGVWGQGTLVTVSSGGGGSSGGGSGGGGSQS
VLTQPPSVSAAPGQKVTISCSGSSSNIGNNYVSWYQQLPGTAPKLLIYDHTNRPAGVPDRFS
GSKSGTSASLAISGFRSEDEADYYCASWDYTLSGWVFGGGTKLTVLG*GS
155. SEQ ID NO: 173 (HER2_scFv) 
QVQLVQSGAEVKKPGESLKISCKGSGYSFTSYWIAWVRQMPGKGLEYMGLIYPGDSDTKY
SPSFQGQVTISVDKSVSTAYLQWSSLKPSDSAVYFCARHDVGYCTDRTCAKWPEWLGVWG
QGTLVTVSSGGGGSSGGGSGGGGSQSVLTQPPSVSAAPGQKVTISCSGSSSNIGNNYVSWY
QQLPGTAPKLLIYDHTNRPAGVPDRFSGSKSGTSASLAISGFRSEDEADYYCASWDYTLSG
WVFGGGTKLTVLG
156. SEQ ID NO: 174 (HER3_scFv)

QVQLQESGGGLVKPGGSLRLSCAASGFTFSSYWMSWVRQAPGKGLEWVANINRDGSASY
YVDSVKGRFTISRDDAKNSLYLQMNSLRAEDTAVYYCARDRGVGYFDLWGRGTLVTVSS
ASTGGGGSGGGGSGGGGSQSALTQPASVSGSPGQSITISCTGTSSDVGGYNFVSWYQQHPG
KAPKLMIYDVSDRPSGVSDRFSGSKSGNTASLIISGLQADDEADYYCSSYGSSSTHVIFGGGT
KVTVLG

## Claims

1. A heteromultimer comprising:
a first polypeptide construct that comprises a first transporter polypeptide and at least one first cargo molecule, optionally wherein the first cargo molecule is a cargo polypeptide, optionally wherein the first polypeptide construct comprises two first cargo polypeptides; and
a second polypeptide construct that comprises a second transporter polypeptide;
and
wherein said first and second transporter polypeptides are obtained by segmentation of an albumin polypeptide having the sequence of SEQ ID NO:1 at a segmentation site, and
wherein the location of the segmentation site is:
(a) between residues 339 and 340 of SEQ ID NO:1; or
(b) between residues 300 and 301 of SEQ ID NO:1; or
(c) between residues 364 and 365 of SEQ ID NO:1; or
(d) between residues 441 and 442 of SEQ ID NO:1; or
(e) between residues 171 and 172 of SEQ ID NO:1; or
(f) between residues 281 and 282 of SEQ ID NO:1; or
(g) after residue 83 and before residue 85 of SEQ ID NO:1 and residue 84 is deleted;
wherein the numbering of residues begins with the first residue after the signal
sequence,
and wherein said first transporter polypeptide is different from said second transporter polypeptide, and said transporter polypeptides self-assemble to form a quasi-native structure of the monomeric form of said albumin polypeptide.

2. The heteromultimer of claim 1, wherein:
at least one of the first and second transporter polypeptides further comprises a linker, optionally wherein the linker is a GGGGS linker.

3. The heteromultimer of claim 1 or claim 2, wherein:
(a) said first transporter polypeptide has a sequence comprising SEQ ID NO:39, and wherein said second transporter polypeptide has a sequence comprising SEQ ID NO:40;
(b) said first transporter polypeptide has a sequence comprising SEQ ID NO:41, and wherein said second transporter polypeptide has a sequence comprising SEQ ID NO:42;
(c) said first transporter polypeptide has a sequence comprising SEQ ID NO:43, and wherein said second transporter polypeptide has a sequence comprising SEQ ID NO:44;
(d) said first transporter polypeptide has a sequence comprising SEQ ID NO:45, and wherein said second transporter polypeptide has a sequence comprising SEQ ID NO:46 thereof;
(e) said first transporter polypeptide has a sequence comprising SEQ ID NO:47, and wherein said second transporter polypeptide has a sequence comprising SEQ ID NO:48;
(f) said first transporter polypeptide has a sequence comprising SEQ ID NO:49, and wherein said second transporter polypeptide has a sequence comprising SEQ ID NO:50;
(g) said first transporter polypeptide has a sequence comprising SEQ ID NO:51, and wherein said second transporter polypeptide has a sequence comprising SEQ ID NO:52;
optionally wherein the first and second transporter polypeptide sequence lacks a signal sequence.

4. The heteromultimer of any one of claims 1 to 3, wherein:
(a) said second polypeptide construct further comprises at least one second cargo molecule, optionally wherein the second cargo molecule is a cargo polypeptide, optionally wherein the second polypeptide construct further comprises two second cargo polypeptides, optionally wherein at least one first cargo polypeptide and/or at least one second cargo polypeptide is an antigen-binding polypeptide construct, optionally an antigen-binding polypeptide construct that binds CD3, CD19, CD20, HER2, or HER3; or
(b) said second polypeptide construct further comprises at least one second cargo polypeptide, optionally wherein:
(i) said first cargo polypeptide is an antigen-binding polypeptide construct that binds CD3 and said second cargo polypeptide is an antigen-binding polypeptide construct that binds to CD19 or CD20, or
(ii) said first cargo polypeptide is an antigen-binding polypeptide construct that binds HER2 and said second cargo polypeptide is an antigen-binding polypeptide construct that binds HER3; or
(c) said at least one first cargo polypeptide is fused to said first transporter polypeptide with a linker, optionally wherein said at least one second cargo polypeptide is fused to said second transporter polypeptide with a linker.

5. The heteromultimer according to any one of claims 1 to 3, wherein said heteromultimer binds to FcRn.

6. The heteromultimer according to claim 1, wherein:
the C- and N- termini introduced at the segmentation site are fused to GGGGS peptide sequence.

7. The heteromultimer of any one of claims 1 to 3, wherein:
(a) the first polypeptide construct comprises at least one first cargo polypeptide that binds a target antigen, and the second polypeptide construct comprises at least one second cargo polypeptide that is a toxin moiety,
(b) the first polypeptide construct comprises at least one first cargo polypeptide that binds a target antigen, and wherein said target antigen is at least one of a-chain (CD25) of IL-2R, Amyloid beta, anti-EpCAM, anti-CD3, CD16, CD20, CD22, CD23, CD3, CD4, CD52, CD80, CTLA-4, EGFR, EpCAM, F protein of RSV, G250, glycoprotein IIB/IIIa R, HER2, HER3, IGF1R, EGFR, HSP90, IgE antibody, IL-12, IL-23, IL-lb, IL-5, IL-6, RANKL, TNF alpha, TNFR, TRAIL,VEGF-A, glucagon receptor, GLP receptor, and LDL receptor, or
(c) at least one cargo polypeptide is an enzyme, hormone, therapeutic polypeptide, antigen, chemotoxin, radiotoxin, cytokine or variant or fragment thereof.

8. The heteromultimer of any one of claims 3 to 5, wherein the quasi-native monomeric structure has a thermal stability that is comparable to that of albumin, optionally wherein the thermal stability is measured by determining the melting temperature (Tm) of the quasi-native monomeric structure.

9. The heteromultimer of any one of claims 1 to 8, wherein the activity of the cargo polypeptide is:
(a) selectively altered on fusion to the transporter polypeptide relative to its unfused format, or
(b) selectively attenuated on fusion to the transporter polypeptide relative to its unfused format.

10. A host cell comprising nucleic acid encoding the heteromultimer of any one of claims 1 to 11, optionally wherein:
(a) the nucleic acid encoding the first polypeptide construct and the nucleic acid encoding the second polypeptide construct are present in a single vector, or
(b) the nucleic acid encoding the first polypeptide construct protein and the nucleic acid encoding the second polypeptide construct protein are present in separate vectors.

11. A pharmaceutical composition comprising the heteromultimer of any one of claims 1 to 9 and an adjuvant.

12. The pharmaceutical composition of claim 11 for use in a method of treating cancer.

13. The heteromultimer of any one of claims 1 to 9 for use in a method of treating cancer by inhibiting growth of a tumor or by shrinking a tumor, wherein the method comprises contacting the tumor with an effective amount of the heteromultimer.

## Patentansprüche

1. Heteromultimer, umfassend:
ein erstes Polypeptidkonstrukt, das ein erstes Transporter-Polypeptid und mindestens ein erstes Cargo-Molekül umfasst, wobei das erste Cargo-Molekül optional ein Cargo-Polypeptid ist, wobei das erste Polypeptidkonstrukt optional zwei erste Cargo-Polypeptide umfasst; und
ein zweites Polypeptidkonstrukt, das ein zweites Transporter-Polypeptid umfasst; und
wobei das erste und das zweite Transporter-Polypeptid durch Segmentierung eines Albuminpolypeptids mit der Sequenz SEQ ID NO: 1 an einer Segmentierungsstelle erhalten werden; und
wobei die Position der Segmentierungsstelle:
(a) zwischen den Resten 339 und 340 der SEQ ID NO: 1 ist; oder
(b) zwischen den Resten 300 und 301 der SEQ ID NO: 1 ist; oder
(c) zwischen den Resten 364 und 365 der SEQ ID NO: 1 ist; oder
(d) zwischen den Resten 441 und 442 der SEQ ID NO: 1 ist; oder
(e) zwischen den Resten 171 und 172 der SEQ ID NO: 1 ist; oder
(f) zwischen den Resten 281 und 282 der SEQ ID NO: 1 ist; oder
(g) hinter Rest 83 und vor Rest 85 der SEQ ID NO: 1 ist, und wobei Rest 84 gelöscht ist;
wobei die Nummerierung der Reste mit dem ersten Rest nach der Signalsequenz beginnt;
und wobei sich das erste Transporter-Polypeptid von dem zweiten Transporter-Polypeptid unterscheidet, und wobei die Transporter-Polypeptide sich selbst organisieren, um eine quasi-native Struktur der monomeren Form des Albuminpolypeptids zu bilden.

2. Heteromultimer nach Anspruch 1, wobei:
mindestens das erste und/oder das zweite Transporter-Polypeptid ferner einen Linker umfasst, wobei der Linker optional ein GGGGS Linker ist.

3. Heteromultimer nach Anspruch 1 oder 2, wobei:
(a) das erste Transporter-Polypeptid eine Sequenz aufweist, welche die SEQ ID NO: 39 umfasst, und wobei das zweite Transporter-Polypeptid eine Sequenz aufweist, welche die SEQ ID NO: 40 umfasst;
(b) das erste Transporter-Polypeptid eine Sequenz aufweist, welche die SEQ ID NO: 41 umfasst, und wobei das zweite Transporter-Polypeptid eine Sequenz aufweist, welche die SEQ ID NO: 42 umfasst;
(c) das erste Transporter-Polypeptid eine Sequenz aufweist, welche die SEQ ID NO: 43 umfasst, und wobei das zweite Transporter-Polypeptid eine Sequenz aufweist, welche die SEQ ID NO: 44 umfasst;
(d) das erste Transporter-Polypeptid eine Sequenz aufweist, welche die SEQ ID NO: 45 umfasst, und wobei das zweite Transporter-Polypeptid eine Sequenz aufweist, welche die SEQ ID NO: 46 umfasst;
(e) das erste Transporter-Polypeptid eine Sequenz aufweist, welche die SEQ ID NO: 47 umfasst, und wobei das zweite Transporter-Polypeptid eine Sequenz aufweist, welche die SEQ ID NO: 48 umfasst;
(f) das erste Transporter-Polypeptid eine Sequenz aufweist, welche die SEQ ID NO: 49 umfasst, und wobei das zweite Transporter-Polypeptid eine Sequenz aufweist, welche die SEQ ID NO: 50 umfasst;
(g) das erste Transporter-Polypeptid eine Sequenz aufweist, welche die SEQ ID NO: 51 umfasst, und wobei das zweite Transporter-Polypeptid eine Sequenz aufweist, welche die SEQ ID NO: 52 umfasst;
wobei der Sequenz des ersten und zweiten Polypeptids optional eine Signalsequenz fehlt.

4. Heteromultimer nach einem der Ansprüche 1 bis 3, wobei:
(a) das zweite Polypeptidkonstrukt ferner mindestens ein zweites Cargo-Molekül umfasst, wobei das zweite Cargo-Molekül optional ein Cargo-Polypeptid ist, wobei das zweite Polypeptidkonstrukt optional ferner zwei zweite Cargo-Polypeptide umfasst, wobei optional mindestens eine erstes Cargo-Polypeptid und/oder mindestens ein zweites Cargo-Polypeptid ein antigenbindendes Polypeptidkonstrukt ist, optional ein antigenbindendes Polypeptidkonstrukt, das an CD3, CD19, CD20, HER2 oder HER3 bindet; oder
(b) das zweite Polypeptidkonstrukt ferner mindestens ein zweites Cargo-Polypeptid umfasst, wobei optional:
(i) das erste Cargo-Polypeptid ein antigenbindendes Polypeptidkonstrukt ist, das an CD3 bindet, und wobei das zweite Cargo-Polypeptid ein antigenbindendes Polypeptidkonstrukt ist, das an CD19 oder CD20 bindet; oder
(ii) das erste Cargo-Polypeptid ein antigenbindendes Polypeptidkonstrukt ist, das an HER2 bindet, und wobei das zweite Cargo-Polypeptid ein antigenbindendes Polypeptidkonstrukt ist, das an HER3 bindet; oder
(c) das mindestens eine erste Cargo-Polypeptid mit einem Linker an das erste Transporter-Polypeptid kondensiert ist, wobei das mindestens eine zweite Cargo-Polypeptid mit einem Linker an das zweite Transporter-Polypeptid kondensiert ist.

5. Heteromultimer nach einem der Ansprüche 1 bis 3, wobei das Heteromultimer an FcRn bindet.

6. Heteromultimer nach Anspruch 1, wobei:
die an der Segmentierungsstelle eingefügten C- und N-Termini an die GGGGS Peptidsequenz kondensiert sind.

7. Heteromultimer nach einem der Ansprüche 1 bis 3, wobei:
(a) das erste Polypeptidkonstrukt mindestens ein erstes Cargo-Polypeptid umfasst, das an ein Zielantigen bindet, und wobei das zweite Polypeptidkonstrukt mindestens ein zweites Cargo-Polypeptid umfasst, das einen Toxinanteil ist;
(b) das erste Polypeptidkonstrukt mindestens ein erstes Cargo-Polypeptid umfasst, das an ein Zielantigen bindet, und wobei das Zielantigen mindestens eines der folgenden ist: eine a-Kette (CD25) von IL-2R, Beta-Amyloid, anti-EpCAM, anti-CD3, CD16, CD20, CD22, CD23, CD3, CD4, CD52, CD80, CTLA-4, EGFR, EpCAM, F-Protein von RSV, G250, Glycoprotein IIB/IIIa R, HER2, HER3, IGF1R, EGFR, HSP90, IgE Antikörper, IL-12, IL-23, IL-1b, IL-5, IL-6, RANKL, TNF alpha, TNFR, TRAIL, VEGF-A, Glucagonrezeptor, GLP-Rezeptor und LDL-Rezeptor; oder
(c) das mindestens eine Cargo-Polypeptid ein Enzym, ein Hormon, ein therapeutisches Polypeptid, ein Antigen, ein Chemotoxin, ein Radiotoxin, ein Zytokin oder eine Variante oder ein Fragment davon ist.

8. Heteromultimer nach einem der Ansprüche 3 bis 5, wobei die quasi-native monomere Struktur eine thermische Stabilität aufweist, die mit der von Albumin vergleichbar ist, wobei die thermische Stabilität optional durch Bestimmung der Schmelztemperatur (Tm) der quasi-nativen monomeren Struktur gemessen wird.

9. Heteromultimer nach einem der Ansprüche 1 bis 8, wobei die Aktivität des Cargo-Polypeptids:
(a) selektiv verändert wird bei Kondensation des Transporter-Polypeptids im Verhältnis zu dessen unkondensierten Form; oder
(b) selektiv abgeschwächt wird bei Kondensation des Transporter-Polypeptids im Verhältnis zu dessen unkondensierten Form.

10. Wirtszelle, umfassend Nukleinsäure, die für das Heteromultimer nach einem der Ansprüche 1 bis 11 codiert, wobei optional:
(a) die für das erste Polypeptidkonstrukt codierende Nukleinsäure und die für das zweite Polypeptidkonstrukt codierende Nukleinsäure in einem einzelnen Vektor vorhanden sind; oder
(b) die für das erste Polypeptidkonstrukt codierende Nukleinsäure und die für das zweite Polypeptidkonstrukt codierende Nukleinsäure in separaten Vektor vorhanden sind.

11. Pharmazeutische Zusammensetzung, die das Heteromultimer nach einem der Ansprüche 1 bis 9 und einen Hilfsstoff umfasst.

12. Pharmazeutische Zusammensetzung nach Anspruch 11 zur Verwendung in einem Verfahren zur Krebsbehandlung.

13. Heteromultimer nach einem der Ansprüche 1 bis 9 zur Verwendung in einem Verfahren zur Krebsbehandlung durch Hemmung des Wachstums eines Tumors oder durch Schrumpfen eines Tumors, wobei das Verfahren das Inkontaktbringen des Tumors mit einer wirksamen Menge des Heteromultimers umfasst.

## Revendications

1. Hétéromultimère comprenant :
une première construction de polypeptide qui comprend un premier polypeptide transporteur et au moins une première molécule cargo, éventuellement dans lequel la première molécule cargo est un polypeptide cargo, éventuellement dans lequel la première construction de polypeptide comprend deux premiers polypeptides cargos ; et
une deuxième construction de polypeptide qui comprend un deuxième polypeptide transporteur ;
et
dans lequel ledit premier et deuxième polypeptide transporteur sont obtenus par segmentation d'un polypeptide d'albumine ayant la séquence de SEQ ID NO : 1 au niveau d'un site de segmentation, et
dans lequel l'emplacement du site de segmentation est :
(a) entre les résidus 339 et 340 de SEQ ID NO : 1 ; ou
(b) entre les résidus 300 et 301 de SEQ ID NO : 1 ; ou
(c) entre les résidus 364 et 365 de SEQ ID NO : 1 ; ou
(d) entre les résidus 441 et 442 de SEQ ID NO : 1 ; ou
(e) entre les résidus 171 et 172 de SEQ ID NO : 1 ; ou
(f) entre les résidus 281 et 282 de SEQ ID NO : 1 ; ou
(g) après le résidu 83 et avant le résidu 85 de SEQ ID NO : 1 et le résidu 84 est supprimé ;
dans lequel la numérotation des résidus commence avec le premier résidu après la séquence signal,
et dans lequel ledit premier peptide transporteur est différent dudit deuxième polypeptide transporteur, et lesdits polypeptides transporteurs s'auto-assemblent pour former une structure quasi-native de la forme monomère dudit polypeptide d'albumine.

2. Hétéromultimère selon la revendication 1, dans lequel :
au moins un parmi le premier et le deuxième polypeptide transporteur comprend en outre un lieur, éventuellement dans lequel le lieur est un lieur GGGGS.

3. Hétéromultimère selon la revendication 1 ou la revendication 2, dans lequel :
(a) ledit premier polypeptide transporteur a une séquence comprenant SEQ ID NO : 39 et dans lequel ledit deuxième polypeptide transporteur a une séquence comprenant SEQ ID NO : 40 ;
(b) ledit premier polypeptide transporteur a une séquence comprenant SEQ ID NO : 41 et dans lequel ledit deuxième polypeptide transporteur a une séquence comprenant SEQ ID NO : 42 ;
(c) ledit premier polypeptide transporteur a une séquence comprenant SEQ ID NO : 43 et dans lequel ledit deuxième polypeptide transporteur a une séquence comprenant SEQ ID NO : 44 ;
(d) ledit premier polypeptide transporteur a une séquence comprenant SEQ ID NO : 45 et dans lequel ledit deuxième polypeptide transporteur a une séquence comprenant SEQ ID NO : 46 ;
(e) ledit premier polypeptide transporteur a une séquence comprenant SEQ ID NO : 47 et dans lequel ledit deuxième polypeptide transporteur a une séquence comprenant SEQ ID NO : 48 ;
(f) ledit premier polypeptide transporteur a une séquence comprenant SEQ ID NO : 49 et dans lequel ledit deuxième polypeptide transporteur a une séquence comprenant SEQ ID NO : 50 ;
(g) ledit premier polypeptide transporteur a une séquence comprenant SEQ ID NO : 51 et dans lequel ledit deuxième polypeptide transporteur a une séquence comprenant SEQ ID NO : 52 ;
éventuellement dans lequel la séquence du premier et du deuxième polypeptide transporteur ne comprend pas de séquence signal.

4. Hétéromultimère selon l'une quelconque des revendications 1 à 3, dans lequel :
(a) ladite deuxième construction de polypeptide comprend en outre au moins une deuxième molécule cargo, éventuellement dans lequel la deuxième molécule cargo est un polypeptide cargo, éventuellement dans lequel la deuxième construction de polypeptide comprend en outre deux deuxièmes polypeptides cargos, éventuellement dans lequel au moins un premier polypeptide cargo et/ou au moins un deuxième polypeptide cargo sont une construction de polypeptide de liaison à l'antigène, éventuellement une construction de polypeptide de liaison à l'antigène qui se lie à CD3, CD19, CD20, HER2 ou HER3 ; ou
(b) ladite deuxième construction de polypeptide comprend en outre au moins un deuxième polypeptide cargo, éventuellement dans lequel :
(i) ledit premier polypeptide cargo est une construction de polypeptide de liaison à l'antigène qui se lie à CD3 et ledit deuxième polypeptide cargo est une construction de polypeptide de liaison à l'antigène qui se lie à CD19 ou CD20, ou
(ii) ledit premier polypeptide cargo est une construction de polypeptide de liaison à l'antigène qui se lie à HER2 et ledit deuxième polypeptide cargo est une construction de polypeptide de liaison à l'antigène qui se lie à HER3 ; ou
(c) ledit au moins un premier polypeptide cargo est fusionné audit premier polypeptide transporteur avec un lieur, éventuellement dans lequel ledit au moins un deuxième polypeptide cargo est fusionné audit deuxième polypeptide transporteur avec un lieur.

5. Hétéromultimère selon l'une quelconque des revendications 1 à 3, dans lequel ledit hétéromultimère se lie à FcRn.

6. Hétéromultimère selon la revendication 1, dans lequel :
les terminaisons C et N introduites au niveau du site de segmentation sont fusionnées à une séquence peptidique GGGGS.

7. Hétéromultimère selon l'une quelconque des revendications 1 à 3, dans lequel :
(a) la première construction de polypeptide comprend au moins un premier polypeptide cargo qui se lie à un antigène cible, et la deuxième construction de polypeptide comprend au moins un deuxième polypeptide cargo qui est une fraction de toxine,
(b) la première construction de polypeptide comprend au moins un premier polypeptide cargo qui se lie à un antigène cible, et dans lequel ledit antigène cible est au moins un parmi la chaîne a (CD25) d'IL-2R, la bêta-amyloïde, anti-EpCAM, anti-CD3, CD16, CD20, CD22, CD23, CD3, CD4, CD52, CD80, CTLA-4, EGFR, EpCAM, la protéine F du VRS, G250, la glycoprotéine IIB/IIIa R, HER2, HER3, IGF1R, EGFR, HSP90, l'anticorps IgE, IL-12, IL-23, IL-1b, IL-5, IL-6, RANKL, TNF alpha, TNFR, TRAIL, VEGF-A, le récepteur de glucagon, le récepteur de GLP et le récepteur de LDL, ou
(c) au moins un polypeptide cargo est une enzyme, une hormone, un polypeptide thérapeutique, un antigène, une chimiotoxine, une radiotoxine, une cytokine ou une variante ou un fragment de ceux-ci.

8. Hétéromultimère selon l'une quelconque des revendications 3 à 5, dans lequel la structure monomère quasi-native a une stabilité thermique qui est comparable à celle de l'albumine, éventuellement dans lequel la stabilité thermique est mesurée en déterminant la température de fusion (Tm) de la structure monomère quasi-native.

9. Hétéromultimère selon l'une quelconque des revendications 1 à 8, dans lequel l'activité du polypeptide cargo est :
(a) modifiée sélectivement lors de la fusion au polypeptide transporteur par rapport à son format non fusionné, ou
(b) atténuée sélectivement lors de la fusion au polypeptide transporteur par rapport à son format non fusionné.

10. Cellule hôte comprenant un acide nucléique codant l'hétéromultimère selon l'une quelconque des revendications 1 à 11, éventuellement dans laquelle :
(a) l'acide nucléique codant la première construction de polypeptide et l'acide nucléique codant la deuxième construction de polypeptide sont présents dans un vecteur unique, ou
(b) l'acide nucléique codant la première construction de polypeptide et l'acide nucléique codant la deuxième construction de polypeptide sont présents dans des vecteurs séparés.

11. Composition pharmaceutique comprenant l'hétéromultimère selon l'une quelconque des revendications 1 à 9 et un adjuvant.

12. Composition pharmaceutique selon la revendication 11 pour une utilisation dans un procédé de traitement du cancer.

13. Hétéromultimère selon l'une quelconque des revendications 1 à 9 pour une utilisation dans un procédé de traitement du cancer par inhibition de la croissance d'une tumeur ou par rétrécissement d'une tumeur, le procédé comprenant la mise en contact de la tumeur avec une quantité efficace de l'hétéromultimère.
